(19) Europäisches Patentamt
European Patent Office
Office européen des brevets

(11) EP 2 354 249 A1

(12) EUROPEAN PATENT APPLICATION

(43) Date of publication:
**10.08.2011 Bulletin 2011/32**

(51) Int Cl.:
***C12Q 1/68*** *(2006.01)* ***C07H 21/00*** *(2006.01)*

(21) Application number: **10174640.2**

(22) Date of filing: **23.06.2004**

(84) Designated Contracting States:
**AT BE BG CH CY CZ DE DK EE ES FI FR GB GR HU IE IT LI LU MC NL PL PT RO SE SI SK TR**

(30) Priority:
**23.06.2003 US 602494**
**23.06.2003 US 603138**
**06.05.2004 EP 04090175**
**27.02.2004 EP 04090072**
**03.10.2003 US 679062**

(62) Document number(s) of the earlier application(s) in accordance with Art. 76 EPC:
**04777046.6 / 1 636 386**

(27) Previously filed application:
**23.06.2004 PCT/US2004/020336**

(71) Applicant: **Epigenomics AG**
**10178 Berlin (DE)**

(72) Inventors:
- **Lofton-Day, Cathy**
  **Seattle, WA 98103 (US)**
- **Model, Fabian**
  **12683, Berlin (DE)**
- **Sledziewski, Andrew**
  **Shoreline, WA 98177 (US)**
- **Rujan, Tamas**
  **79104, Freiburg (DE)**
- **Lewin, Jörn**
  **10115, Berlin (DE)**
- **Distler, Jürgen**
  **12163, Berlin (DE)**

Remarks:
- The complete document including Reference Tables and the Sequence Listing can be downloaded from the EPO website
- This application was filed on 31-08-2010 as a divisional application to the application mentioned under INID code 62.

(54) **Methods and nucleic acids for analyses of colorectal cell proliferative disorders**

(57) The invention provides methods, nucleic acids and kits for detecting, or for detecting and distinguishing between or among colorectal cell proliferative disorders. The invention discloses genomic sequences the methylation patterns of which have utility for the improved detection of and differentiation between said class of disorders, thereby enabling the improved diagnosis and treatment of patients.



Printed by Jouve, 75001 PARIS (FR)

## Description

CROSS-REFERENCE TO RELATED APPLICATIONS

This application claims the benefit of priority to: U.S. Patent Application No. 10/679,062, filed 03 October 2003, which is a continuation-in-part of U.S. Patent Application No. 10/603,138, filed 23 June, 2003; to U.S. Application No. 10/602,494, filed 23 June 2003; to European Patent Application No. EP 04090175.3, filed 06 May 2004; and to European Patent Application No. EP 04090072.2, filed 27 February 2004; all of which are incorporated herein by reference in their entirety.

FIELD OF THE INVENTION

The present invention relates to genomic DNA sequences that exhibit altered CpG methylation patterns in disease states relative to normal. Particular embodiments provide methods, nucleic acids, nucleic acid arrays and k its useful for detecting, or for detecting and differentiating between or among colorectal cell proliferative disorders.

SEQUENCE LISTING

A Sequence Listing, pursuant to 37 C.F.R. § 1.52(e)(5), has been provided on compact disc (1 of 1) as a 4.17 MB text file, entitled "Sequence Listing for 47675-75.txt," and which is incorporated by reference herein in its entirety.

BACKGROUND

The etiology of pathogenic states is known to involve modified methylation patterns of individual genes or of the genome. 5-methylcytosine, in the context of CpG dinucleotide sequences, is the most frequent covalently modified base in the DNA of eukaryotic cells, and plays a role in the regulation of transcription, genetic imprinting, and tumorigenesis. The identification and quantification of 5-methylcytosine sites in a specific specimen, or between or among a plurality of specimens, is thus of considerable interest, not only in research, but particularly for the molecular diagnoses of various diseases.

*Correlation of aberrant DNA methylation with cancer.* Aberrant DNA methylation within CpG 'islands' is characterized by *hyper*- or *hypomethylation* of CpG dinucleotide sequences leading to abrogation or overexpression of a broad spectrum of genes, and is among the earliest and most common a Iterations found in, and correlated with human malignancies. Additionally, abnormal methylation has been shown to occur in CpG-rich regulatory elements in intronic and coding parts of genes for certain tumors. In colon cancer, for example, aberrant DNA methylation constitutes one of the most prominent alterations and inactivates many tumor suppressor genes such as p14ARF, p16INK4a, THBS1, MINT2, and MINT31 and DNA mismatch repair genes such as hMLH1.

In contrast to the specific hypermethylation of tumor suppressor genes, an overall hypomethylation of DNA can be observed in tumor cells. This decrease in global methylation can be detected early, far before the development of frank tumor formation. A correlation between hypomethylation and increased gene expression has been determined for many oncogenes.

In the United States the annual incidence of colorectal cancer is approximately 150,000, with 56,600 individuals dying form colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (*e.g.*, sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HICI, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β -catenin mutations. Furthermore, a lterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported. In addition, using comparative genomic hybridization several alterations were found in liver metastasis that were unique to metastatic lesions (-9q, -11q, and -17q).

Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001).

*Multifactorial approach.* Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* If colon cancer screening specificity can be increased, the problem of false positive test results leading to unnecessary colonoscopic examination would be reduced leading to cost savings and improved safety.

In view of the incidence of colon cancer and disadvantages associated with current colorectal cell proliferative disorder screening methods there is a substantial need in the art for improved methods for the early detection of colon cell proliferative disorders, in particular colon cancer, to be used in addition to or as a substitute for currently available tests.

## SUMMARY OF THE INVENTION

Various aspects of the present invention provide efficient and unique panels of genes, whereby methylation analysis of one or a combination of the members of the panel enables the detection of cell proliferative disorders of the prostate with a particularly high sensitivity, specificity and/or predictive value. The inventive colorectal cancer testing methods have particular utility for the screening of at-risk populations. The inventive methods have advantages over prior art methods (including the industry standard FOBT), because of improved sensitivity, specificity and likely patient compliance.

The present invention provides novel methods for detecting or distinguishing between colorectal cell proliferative disorders. Said methods are most preferably utilised for detecting or detecting and distinguishing between one or more of the following: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon normal tissue, body fluids and non-colon cancer tissue.

In a further embodiment of the invention the method according to the invention may be used for the detection of aerodigestive cell proliferative disorders.

In one embodiment the invention provides a method for detecting and/or for detecting and distinguishing between or among colorectal cell proliferative disorders in a subject. Said method comprises the following steps: i) contacting genomic DNA isolated from blood plasma, blood serum, whole blood, isolated blood cells, cells isolated from the blood obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence; and ii) detecting, or detecting and distinguishing between or among c olorectal cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

The method is novel as no methods currently exist that enable the detection of colon cancer by analysis of bodily fluids, with a sensitivity and specificity high enough for use in a commercially available and regulatory body approved assay. Current methods used to detect and diagnose colorectal cell proliferative disorders include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that patient compliance for a single body fluid based test will be higher than the triplicate analysis of stool currently recommended for FOBT.

A particular embodiment the method comprises the use of one or more genes or genomic sequences selected from the group consisting of: SEQ ID NOS: 1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 as markers for the differentiation, detection and distinguishing of colorectal cell proliferative disorders.

Said use of the gene may be enabled by means of any analysis of the expression of the gene, by means of mRNA expression analysis or protein expression analysis. However, in the most preferred embodiment of the invention, the detection, differentiation and distinguishing of colorectal cell proliferative disorders is enabled by means of analysis of the *methylation status* of one or more genes or genomic sequences selected from the group consisting of SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30, DUX4, and their promoter or regulatory elements.

The invention provides a method for the analysis of biological samples for features associated with the development of colorectal cell proliferative disorders, the method characterised in that at least one nucleic acid, or a fragment thereof, from the group consisting of SEQ ID NO: to SEQ ID NO:535 is/are contacted with a reagent or series of reagents capable of distinguishing between methylated and non methylated CpG dinucleotides within the genomic sequence, or sequences of interest.

The present invention provides a method for ascertaining genetic and/or epigenetic parameters of genomic DNA. The method has utility for the improved diagnosis, treatment and monitoring of colorectal cell proliferative disorders, more specifically by enabling the improved identification of and differentiation between subclasses of said disorder. The invention presents improvements over the state of the art in that it enables a highly specific classification of colorectal cell proliferative disorders, thereby allowing for improved and informed treatment of patients.

Preferably, the source of the test sample is selected from the group consisting of cells or cell lines, histological slides, biopsies, paraffin-embedded tissue, bodily fluids, ejaculate, stool, urine, blood, and combinations thereof. Preferably, the source is selected from the group consisting of stool, blood plasma, blood serum, whole blood, isolated blood cells, cells isolated from the blood obtained from the subject.

Specifically, the present invention provides a method for detecting colorectal cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NOS: 1 to 64, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NOS:304 to SEQ ID NO:535 and SEQ ID NOS:65 to SEQ ID NO:88, and contiguous regions thereof corresponding to the target sequence.

Additional embodiments provide a method for the detection of colorectal cell proliferative disorders, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NOS:304 to SEQ ID NO:535 and SEQ ID NOS:65 to SEQ ID NO:88, and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence selected from the group consisting of SEQ ID NOS:1 to SEQ ID NO:64, or an average, or a value reflecting an average methylation state of a plurality of CpG dinucleotide sequences thereof. Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:304 to SEQ ID NO:535 and SEQ ID NOS:65 to SEQ ID NO:88, and complements thereof; hybridizing at least one nucleic acid molecule, bound to a solid phase, comprising a contiguous sequence at least 9 nucleotides in length that is complementary to, or hybridizes under moderately stringent or stringent conditions 6 a" sequence selected from the group consisting of SEQ ID NOS:304 to SEQ ID NO:535 and SEQ m NOS:65 to SEQ ID NO:88, and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:304 to SEQ ID NO:535 and SEQ ID NOS:65 to SEQ ID NO:88, and complements thereof, and extending at least one such hybridized nucleic acid molecule by at least one nucleotide base; and sequencing of the amplificate.

Further embodiments provide a method for the analysis of colorectal cell proliferative disorders, comprising: obtaining a biological sample having subject genomic DNA; extracting the genomic DNA; contacting the genomic DNA, or a fragment thereof, comprising one or more sequences selected from the group consisting of SEQ ID NOS:1 to SEQ ID NO:64 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NOS:1 to SEQ ID NO:64, or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NOS:1 to SEQ ID NO:64.

## BRIEF DESCRIPTION OF THE DRAWINGS

Figures 1, 5, 9, 13, 16, 20, 24, 28 and 32 show ranked matrices of data obtained according to EXAMPLES 1 and 2, and according to CpG methylation differences between the two classes of tissues, using a suitable algorithm. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. The p-values for the individual CpG positions are shown on the right side. The p-values are the probabilities that the observed distribution occurred by chance in the data set.

Figures 2, 6, 10, 17, 21, 25, 29 and 33 show the uncorrected multivariate LogReg test results for each comparison. Each individual genomic region of interest is represented as a point, the dotted line represents the cut off point for the 25% false discovery rate.

Figures 3, 7, 11, 14, 18, 22, 26, 30 and 34 show ranked matrices of data, obtained according to EXAMPLES 1 and 2, of the accuracy of the genewise linear support vector machine cross validations between the two classes of tissues, for the best performing markers. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. Accuracy values for each individual genomic region of interest are shown on the right side.

Figures 4, 8, 12, 15, 18, 23, 27, 31 and 35 show the accuracy of the genewise linear support vector machine cross validation for all genomic regions of each classification. The accuracy of each genomic region is represented as black squares, the specificity as unfilled diamonds, and the sensitivity as unfilled squares. The accuracy as measured on the X-axis shows the fraction of correctly classified samples.

Figure 36 shows the level of methylation determined by different MSP MethyLight™ assays and HeavyMethyl™ MethyLight™ assays. The Y-axis shows the degree of methylation. Tumor samples are represented by white points, and normal colon tissue samples by black points. A significantly higher degree of methylation was observed in tumor samples than in healthy tissue samples.

Figure 37 shows the Receiver Operating Characteristic curve (ROC curve) of the SEQ ID NO:35 -MSP-MethylLight™-Assay for adenocarcinomas according to EXAMPLE 2. The AUC for the MSP-MethylLight™-Assay is: 0.94.

Figure 38 shows the Receiver Operating Characteristic curve (ROC curve) of the SEQ ID NO:35 -HM-MethylLight™-Assay for Adenocarcinoma according to EXAMPLE 3. The AUC for the HM-MethylLight™-Assay is: 0.91.

Figure 39 shows the level of methylation determined by a SEQ ID NO:35 - HeavyMethyl™ MethyLight™ assay according to EXAMPLE 3, testing an additional set of colon samples (25 adenocarcinoma, 33 normals, and 13 adenomas). The Y-axis shows the degree of methylation within the region of the SEQ ID NO:35 gene investigated. Adenocarcinoma samples are represented by white squares, and normal colon tissue samples by black diamonds. A significantly higher degree of methylation was observed in tumor samples than in healthy tissue samples. The level of significance as measured using a t-test was 0.00424.

Figure 40 shows the Receiver Operating Characteristic curve (ROC curve) of the SEQ ID NO:35 -HM-MethylLight™-Assay for Adenocarcinoma and Adenoma according to EXAMPLE 3 (additional sets of samples). The area under an ROC curve (AUC) is a measure for the accuracy of a diagnostic test. The AUC for the HM-MethylLight™-Assay is 0.81.

Figure 41 shows the Receiver Operating Characteristic curve (ROC curve) of the SEQ ID NO:35 -HM-MethylLight™-Assay for Adenocarcinoma only according to EXAMPLE 2 (additional sets of samples). The area under an ROC curve (AUC) is a measure for the accuracy of a diagnostic test. The AUC for the HM-Methyl-Light-Assay is: 0.844.

Figure 42 shows the Receiver Operating Characteristic curve (ROC curve) of the SEQ ID NO:35 -HM-MethylLight™-Assay for Adenenomas according to EXAMPLE 3 (additional sets of samples). The area under an ROC curve (AUC) is a measure for the accuracy of a diagnostic test. The AUC for the HM-MethylLight™-Assay is: 0.748.

Figure 43 shows the level of methylation in different tumor and healthy tissues determined by a SEQ ID NO 35 -HeavyMethyl™ MethyLight™ assay according to example 4. The Y-axis shows the degree of methylation within the region of the SEQ ID NO:35 gene investigated. Besides the colon cancer samples only one of the two breast cancer tissues were methylated.

Figure 44 shows the level of methylation in different breast cancer tissues determined by a SEQ ID NO:35 -HeavyMethyl™ MethyLight™ assay according to EXAMPLE 4. Only one was methylated.

Figure 45 shows the level of methylation in serum samples determined by a SEQ ID NO:35 -HeavyMethyl™ MethyLight™ assay according to EXAMPLE 4. The Y-axis shows the degree of methylation within the region of the SEQ ID NO:35 gene investigated.

Figure 46 shows the ROC curve of the SEQ ID NO:34 -MSP-Methyl-Light ™-Assay according to EXAMPLE 9. The AUC is: 0.84.

Figure 47 shows the ROC curve of the SEQ ID NO:29 -MSP-Methyl-Light ™-Assay according to EXAMPLE 10. The AUC is: 0.80.

Figure 48 shows the regression plot of the percentage methylation within SEQ ID NO:35 calculated in each sample using the MSP and HeavyMethyl™ variants of the MethyLight™ assay.

Figure 49 shows the ROC curve of the SEQ ID NO:29 -MSP-MethylLight ™-Assay according to EXAMPLE 8 (first sample set). The AUC is: 0.93.

Figure 50 shows the ROC curve of the SEQ ID NO:29 -MSP-MethylLight ™-Assay according to EXAMPLE 8 (second sample set). The AUC is: 1.

Figure 51 shows the ROC curve of the SEQ ID NO:39 -MSP-MethylLight ™-Assay according to EXAMPLE 9. The AUC is: 0.94.

Figures 52 to 59 show the amplification of stool samples according to example 10. The X-axis shows the cycle number of the polymerase chain reaction, the Y-axis shows the delta Rn, which is the fluorescence intensity over background, the horizontal line (A) represents the threshold level of the calibration curve (B), if the curve reaches above (A) methylation has been detected.

Figures 60 to 71 show the PCR amplification of samples according to example 11.Each individual sample is annotated on the x-axis as 'n' denoting normal, or 't' denoting tumour. A bronchial tissue sample was accidentally analysed in some assays, these are annotated with an arrow, and are irrelevant in the context of the present invention. The Y-axis shows the level of methylation ('methylation rate ') in the sample. The methylation rate {methylated/(methylated + unmethylated) are shown for the fragments corresponding to the primer pairs in Table 4.

Figures 72 to 86 show the PCR amplification of samples according to assays 1 to 15 of example 14 respectively. The X-axis shows the cycle number of the polymerase chain reaction, the Y-axis shows the fluorescence intensity over background.

Figure 72 shows the amplification curve of assay 1.

Figure 73 shows the amplification curve of assay 2.

Figure 74 shows the amplification curve of assay 3.

Figure 75 shows the amplification curve of assay 4.

Figure 76 shows the amplification curve of assay 5.

Figure 77 shows the amplification curve of assay 6.

Figure 78 shows the amplification curve of assay 7.

Figure 79 shows the amplification curve of assay 8.

Figure 80 shows the amplification curve of assay 9.

Figure 81 shows the amplification curve of assay 10.

Figure 82 shows the amplification curve of assay 11.

Figure 83 shows the amplification curve of assay 12.

Figure 84 shows the amplification curve of assay 13.

Figure 85 shows the amplification curve of assay 14.

Figure 86 shows the amplification curve of assay 15.

Figures 87 to 95 show the class prediction performance of quadratic SVMs trained on the data set according to example 14. Circled points are the support vectors defining the borderline (white) between the lighter and darker shaded areas.

Figure 87 shows an SVM trained using Assays 12 and 9.

Figure 88 shows an SVM trained using Assays 12 and 11.

Figure 89 shows an SVM trained using Assays 12 and 15.

Figure 90 shows an SVM trained using Assays 7 and 6 .

Figure 91 shows an SVM trained using Assays 11 and 5 .

Figure 92 shows an SVM trained using Assays 7 and 5 .
Figure 93 shows an SVM trained using Assays 6 and 4.
Figure 94 shows an SVM trained using Assays 12 and 7 .
Figure 95 shows an SVM trained using Assays 12 and 6 .
Figure 96 shows a ranked matrices of data obtained according to Examples 1 and 2 according to CpG methylation differences between the two classes of tissues, (aerodigestive cancer on the left and healthy aerodigestive tissues on the right) using an algorithim. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.

DETAILED DESCRIPTION OF THE INVENTION

Definitions:

The term "Observed/Expected Ratio" ("O/E Ratio") refers to the frequency of CpG dinucleotides within a particular DNA sequence, and corresponds to the [number of CpG sites / (number of C bases x number of G bases)] x band length for each fragment.
The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.
The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."
The term "hemi-methylation" or "hemimethylation" refers to the methylation state of a palindromic CpG methylation site, where only a single cytosine in one of the two CpG dinucleotide sequences of the palindromic CpG methylation site is methylated (e.g., 5'-CC$^M$GG-3' (top strand): 3'-GGCC-5' (bottom strand)).
The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).
The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.
The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.
The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.
"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).
"Epigenetic parameters" are, in particular, cytosine methylations. Further epigenetic parameters include, for example, the acetylation of histones which, however, cannot be directly analyzed using the described method but which, in turn, correlate with the DNA methylation.
The term "bisulfite reagent" refers to a reagent comprising bisulfite, disulfite, hydrogen sulfite or combinations thereof, useful as disclosed herein to distinguish between methylated and unmethylated CpG dinucleotide sequences.
The term "Methylation assay" refers to any assay for determining the methylation state of one or more CpG dinucleotide sequences within a sequence of DNA.
The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.
The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68˚C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60˚C in 2.5 x SSC buffer, followed by several washing steps at 37˚C in a low buffer concentration, and remains stable). Moderately stringent conditions, as defined herein, involve including washing in 3x SSC at 42˚C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NOS:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

<u>Overview:</u>

The present invention provides for molecular genetic markers that have novel utility for the analysis of methylation patterns associated with the development of colorectal cell proliferative disorders. Said markers may be used for detecting or distinguishing between colorectal cell proliferative disorders, thereby providing improved means for the classification and treatment of said disorders. In an alternative embodiment said molecular genetic markers may be used for the detection of aerodigestive cell proliferative disorders.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.,* Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The p resent invention provides for the use of the bisulfite technique, in combination with one or more methylation assays, for determination of the methylation status of CpG dinuclotide sequences within sequences from the group consisting of SEQ ID NOS:1 to SEQ ID NO:64. According to the present invention, determination of the methylation status of CpG dinuclotide sequences within sequences from the group consisting of SEQ ID NOS:1 to SEQ ID NO:64 has diagnostic and prognostic utility.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA.* COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight*™. The MethyLight™ assay is a high-throughput quantitative methylation assay that utilizes fluorescence-based real-time PCR (TaqMan ™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; e.g., with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical MethyLight ™-based kit) for MethyLight™ analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA.* The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.,* as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences According to SEQ ID NO:1 to SEQ ID NO:64, and Non-naturally Occuring Treated Variants Thereof According to S E ID N OS:304 to S EQ ID N OS:535 and S EQ ID NOS:65 TO SEQ ID NO:88, were Determined to have Novel Utility for the Detection, Classification and/or Treatment of Colorectral Cell Proliferative Disorders

In one embodiment the invention provides a method for detecting and/or for detecting and distinguishing between or among colon cell proliferative disorders in a subject. Said method comprises the following steps: i) contacting genomic DNA isolated from bodily fluids obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and ii) detecting, or detecting and distinguishing between or among colon cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Body fluids are the preferred source of the DNA; particularly preferred are blood plasma, blood serum, whole blood, isolated blood cells and cells isolated from the blood.

The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analysed in order to determine the methylation state of one or more target gene sequences (prior to the treatment) associated with the development of colorectal carcinoma. It is particularly preferred that the target region comprises, or hybridizes under stringent conditions to at least 16 contiguous nucleotides of at least one gene or sequence selected from the group consisting the genes and sequences as listed in Table 1. It is further preferred that the sequences of said genes in Table 3 as described in the accompanying sequence listing are analysed. The method of analysis may be selected from those known in the art, including those listed herein. Particularly preferred are MethyLight, MSP and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: (IDBISEQFIRST) to SEQ ID NO: and (IDBISEQLAST) and sequences complementary thereto.

Aberrant methylation, more preferably hypermethylation of one or more genes or genomic sequences taken from the group consisting of: SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 are associated with the presence of colorectal carcinoma.

Analysis of one or a plurality of the sequences enables for the first time detecting, or detecting and distinguishing between or among colon cell proliferative disorders afforded with a sensitivity of greater than or equal to 80% and a specificity of greater than or equal to 80%. Sensitivity is calculated as:{detected colon neoplasia/all colon neoplasia); and specificity is calculated as (non-detected negatives/total negatives).

Preferably, the sensitivity is from about 75% to about 96%, or from about 80% to about 90%, or from about 80% to about 85%. Perferably, the specificity is is from about 75% to about 96%, or from about 80% to about 90%, or from about 80% to about 85%.

Colon neoplasia can be defined as all colon malignancies or colon malignancies and large (> 1 cm adenomas), or subsets thereof. Negatives can be defined as any disease other than colon malignancy and adenomas, or healthy individuals.

In one embodiment the method discloses the use of one or more genes or genomic sequences selected from the group consisting SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 as markers for the differentiation, detection and distinguishing of colon cell proliferative disorders.

Said use of the gene may be enabled by means of any analysis of the expression of the gene, by means of mRNA expression analysis or protein expression analysis. However, in the most preferred embodiment of the invention, the detection, differentiation and distinguishing of colon cell proliferative disorders is enabled by means of analysis of the methylation status of one or more genes or genomic sequences selected from the group consisting SEQ ID NOS: 1-3, 14, 16, 21, 22, 23, 44-46, 48, 50, 59-64, C OX7B, C STS, E YA3, FTH1, RNF4, S OX 21, R NF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 and their promoter or regulatory elements. Methods for the methylation analysis of genes are described herein.

Aberrant levels of mRNA expression of the genes, genomic sequences or genes regulated by genomic sequences according to SEQ ID NOS: 1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 according to Table 3 are associated with colorectal carcinoma.

Accordingly, increased or decreased levels of expression of said genes or sequences are associable with the development of colorectal carcinoma and other colorectal cell proliferative disorders.

To detect the presence of mRNA encoding a gene or genomic sequence in a detection system for colon cancer, a sample is obtained from a patient. The sample can be a tissue biopsy sample or a sample of blood, plasma, serum or the like. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2d ed., 1989). Detection of the resulting duplex is usually accomplished by the use of labelled probes. Alternatively, the probe may be unlabeled, but may be detectable by specific binding with a ligand which is labelled, either directly or indirectly. Suitable labels and methods for labelling probes and ligands are known in the art, and include, for example, radioactive labels which may b e incorporated by known methods (e.g., nick translation or kinasing),biotin, fluorescent groups, chemiluminescent groups (e.g., dioxetanes, particularly triggered dioxetanes), enzymes, antibodies, and the like.

In order to increase the sensitivity of the he detection in a sample of m RNA transcribed from the gene or genomic sequence, the technique of reverse transcription/polymerisation chain reaction c an be used to amplify cDNA transcribed from the mRNA. The method of reverse transcription /PCR is well known in the art (for example, see Watson and Fleming, *supra*).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference).

The present invention may also be described in certain embodiments as a kit for use in detecting a colon cancer disease state through testing of a biological sample. A representative kit may comprise one or more nucleic acid segments that selectively hybridise to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridisation, amplification or detection reactions. Preferred kit components include reagents for reverse transcription-PCR, in situ hybridisation, Northern analysis and/or RPA

The present invention further provides for methods to detect the presence of the polypeptide encoded by said genes or gene sequences in a sample obtained from a patient.

Aberrant levels of polypeptide expression of the polypeptides encoded by the genes, genomic sequences or genes regulated by genomic sequences according to SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30 and DUX4 according to Table 3 are associated with colorectal carcinoma.

Accordingly over or under expression of said polypeptides are associable with the development of colorectal carcinoma and other colorectal cell proliferative disorders.

Any method known in the art for detecting proteins can be used. Such methods include, but are not limited to immunodiffusion, immunoelectrophoresis, immunochemical methods, binder-ligand assays, immunohistochemical techniques, agglutination and complement assays. (*e.g.*, see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled protein or derivative thereof.

Certain embodiments of the present invention comprise the use of antibodies specific to the polypeptide encoded by the genes or genomic sequences of the group consisting of SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1,

ADCY9, BCL-5, BCOR, TAFII28, HOMEOBOX PROTEIN SIX6, GSK-3 BETA, ELK-L, HLA-F, TGFBR2, AR, RNF30 and DUX4.

Such antibodies may be useful for diagnostic and prognostic applications in detecting the disease state, by comparing a patient's levels of colon disease marker expression to expression of the same markers in normal individuals. In certain embodiments production of monoclonal or polyclonal antibodies can be induced by the use of the coded polypeptide as antigene. Such antibodies may in turn be used to detect expressed proteins as markers for human disease states. The levels of such proteins present in the peripheral blood or tissue sample of a patient may be quantified by conventional methods. Antibody-protein binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive protein binding immunoassays are well known in the art. Antibodies employed in such assays may be unlabeled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like for use in radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

One approach for preparing antibodies to a protein is the selection and preparation of an amino acid sequence of all or part of the protein, chemically synthesising the sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In addition to the embodiments above wherein the methylation analysis of genes or genomic sequences selected from the group SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1,ADCY9, BCL-5, BCOR, TAFII28, HOMEOBOX PROTEIN SIX6, GSK-3 BETA, ELK-L, HLA-F, TGFBR2, AR, RNF30 and DUX4 according to Table 3 are analysed, the invention presents two further panels of genes with novel utility for the detection of colorectal cancer.

In a first further embodiment the present invention is based upon the analysis of methylation levels within one or more genes or genomic sequences taken from the group consisting of SEQ ID NOS:1-3, 14, 16, 21, 22, 23, 44-46, 48, 50, 59-64, COX7B, CST5, EYA3, FTH1, RNF4, SOX 21, RNF4, ALX 4, ONC2, NGFR, DLX-5, HVIAAT, BTF3 Homolog 1, ADCY9, BCL-5, BCOR,TAFII28, HOMEOBOX PROTEIN SIX6, GSK-3 BETA, ELK-L, HLA-F, TGFBR2, AR, RNF30, DUX4, SMAD7, GTMBP, EYA4 and TCF1-ALPHA according to Table 3 and/or their regulatory sequences.

It is further preferred that the sequences of said genes or genomic sequences are as according to Table 3.

In a further embodiment the present invention is based upon the analysis of methylation levels within two or more genes or genomic sequences taken from the group consisting all genes and genomic sequences according to Table 3 and/or their regulatory sequences. It is further preferred that the sequences of said genes or genomic sequences are as according to SEQ ID NOS:1 to SEQ ID NO:64.

Particular embodiments of the present invention provide a novel application of the analysis of methylation levels and/or patterns within said sequences that enables a precise detection, characterisation and/or treatment of colorectal cell proliferative disorders. Early detection of colorectal cell proliferative disorders is directly linked with disease prognosis, and the disclosed method thereby enables the physician and patient to make better and more informed treatment decisions. The sequences of the group consisting SEQ ID NOS: 20, 41, 35, 3, 39, 33 & 27 have further utility for the detection of arodigestive cell proliferative disorders.

## FURTHER IMPROVEMENTS

The present invention provides novel uses for genomic sequences selected from the group consisting of SEQ ID NOS:1 to SEQ ID NO:64. Additional embodiments provide modified variants of SEQ ID NOS:1 to SEQ ID NO:64, as well as oligonucleotides and/or PNA-oligomers for analysis of cytosine methylation patterns within SEQ ID NOS:1 to SEQ ID NO:64.

An objective of the invention comprises analysis of the methylation state of one or more CpG dinucleotides within at least one of the genomic sequences selected from the group consisting of SEQ ID NOS:1 to SEQ ID NO: 64 and sequences complementary thereto.

The disclosed invention provides treated nucleic acids, derived from genomic SEQ ID NOS:1 to SEQ ID NO: 64, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NOS:304 to SEQ ID NO:535 and SEQ ID NOS:65 to SEQ ID NO:88, wherein said sequence comprises at least one CpG, TpA or CpA dinucleotide and sequences complementary thereto. The sequences of SEQ ID NOS:304 to SEQ ID NO:535 and SEQ ID NOS:65 to SEQ ID NO:88 provide non-naturally occurring modified versions of the nucleic acid according to SEQ ID NOS:1 to SEQ ID NO:64, wherein the modification of each genomic sequence results in the synthesis of a nucleic acid having a sequence that is unique and distinct from said genomic sequence as follows. For each sense strand genomic DNA, *e.g.*, SEQ ID NO: 1, four converted versions are disclosed. A first version wherein "C"→"T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (i.e. antisense strand), wherein "C"→"T," but "CpG" remains "CpG" (i.e., corresponds to case where, for all "C" residues of CpG dinucleotide sequences are methylated and are thus not converted). The 'upmethylated' converted sequences of SEQ ID NOS:1 to SEQ ID NO:64 correspond to SEQ ID NOS:65 to 76 and SEQ ID NOS:304 to SEQ ID NO:419. A third chemically converted version of each genomic sequences is provided, wherein "C"→"T" for all "C" residues, including those of "CpG" dinucleotide sequences (i.e., corresponds to case where, for the genomic sequences, all "C" residues of CpG dinucleotide sequences are *un*methylated); a final chemically converted version of each sequence, discloses the complement of the disclosed genomic DNA sequence (i.e. antisense strand), wherein "C"→"T" for all "C" residues, including those of "CpG" dinucleotide sequences (i.e., corresponds to case where, for the complement (antisense strand) of each genomic sequence, all "C" residues of CpG dinucleotide sequences are unmethylated). The 'downmethylated' converted sequences of SEQ ID NOS:1 to SEQ ID NO:64 correspond to SEQ ID NOS: 77 to 88 and SEQ ID NOS:420 to SEQ ID NO:535.

Significantly, heretofore, the nucleic acid sequences and molecules according SEQ ID NOS:1-7, 9-11, 14-23, 27, 31, 32, 40-42, 44-46, 48, 50-55 and 59-64 were not implicated in or connected with the detection, classification or treatment of colorectal cell proliferative disorders, or aerodigestive cell proliferative disorders..

In an alternative preferred embodiment, such analysis comprises the use of an oligonucleotide or oligomer for detecting the cytosine methylation state within genomic or treated (chemically modified) D NA, according to SEQ ID NOS:1 toSEQ ID NO:535. S aid oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NOS: 65 to SEQ ID NO: 88 and SEQ ID NOS:304 to SEQ ID NO:535 and/or sequences complementary thereto, or to a genomic sequence according to SEQ ID NOS:1 to SEQ ID NO:64 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NOS:1 to SEQ ID NO:535, or to the complements thereof. The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 9 8%, or 100% identical to the sequence, or to a portion thereof of SEQ ID NOS:1 to SEQ ID NO:535, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NOS:1 to SEQ ID NO:64 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1 °C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, e.g., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

$$n \text{ to } (n + (X\text{-}1));$$

where n=1, 2, 3,...(Y-(X-1));
where Y equals the length (nucleotides or base pairs) of SEQ ID NO:1 (2,280);
where X equals the common length (in nucleotides) of each oligonucleotide in the set (e.g., X=20 for a set of consecutively overlapping 20-mers); and
where the number (Z) of consecutively overlapping oligomers of length X for a given SEQ ID NO of length Y is equal to Y (X-1). For example Z= 2,280-19= 2,261 for either sense or antisense sets of SEQ ID NO: 1, where X=20.

Preferably, the set is limited to those oligomers that comprise at least one CpG, TpG or CpA dinucleotide.
Examples of inventive 20-mer oligonucleotides include the following set of 2,261 oligomers (and the antisense set complementary thereto), indicated by polynucleotide positions with reference to SEQ ID NO: 1:

1-20, 2-21, 3-22, 4-23, 5-24, ......2259-2278, 2260-2279 and 2261-2280.

Preferably, the set is limited to those oligomers that comprise at least one CpG, TpG or CpA dinucleotide.
Likewise, examples of inventive 25-mer oligonucleotides include the following set of 2,256 oligomers (and the antisense set complementary thereto), indicated by polynucleotide positions with reference to SEQ ID NO:1:

1-25, 2-26, 3-27, 4-28, 5-29, ......2254-2278, 2255-2279 and 2256-2280.

Preferably, the set is limited to those oligomers that comprise at least one CpG, TpG or CpA dinucleotide.
The present invention encompasses, for *each* of SEQ ID NOS:1 to SEQ ID NO:535 (sense and antisense), multiple consecutively overlapping sets of oligonucleotides or modified oligonucleotides of length X, where, e.g., X= 9, 10, 17, 20, 22, 23, 25, 27, 30 or 35 nucleotides.
The oligonucleotides or oligomers according to the present invention constitute effective tools useful to ascertain genetic and epigenetic parameters of the genomic sequence corresponding to SEQ ID NOS:1 to SEQ ID NO:64. Preferred sets of such oligonucleotides or modified oligonucleotides of length X are those consecutively overlapping sets of oligomers corresponding to SEQ ID NOS:1 to SEQ ID NO:535 (and to the complements thereof). Preferably, said oligomers comprise at least one CpG, TpG or CpA dinucleotide.
Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.
The oligonucleotides of the invention can also be modified bye hemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly p referred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.
The oligonucleotide may also comprise at least one art-recognized modified sugar and/or base moiety, or may comprise a modified backbone or non-natural internucleoside linkage.
The oligonucleotides or oligomers according to particular embodiments of the present invention are typically used in 'sets,' which contain at least one oligomer for analysis of each of the CpG dinucleotides of genomic sequences SEQ ID NOS:1 to SEQ ID NO:64 and sequences complementary thereto, or to the corresponding CpG. TpG or CpA dinucleotide within a sequence of the treated nucleic acids according to SEQ ID NOS:304 to SEQ ID NO:535 and sequences complementary thereto. However, it is anticipated that for economic or other factors it may be preferable to analyze a limited selection of the CpG dinucleotides within said sequences, and the content of the set of oligonucleotides is altered accordingly.
Therefore, in particular embodiments, the present invention provides a set of at least two (2) (oligonucleotides and/or PNA-oligomers) useful for detecting the cytosine methylation state in treated genomic DNA (SEQ ID NOS:304 to SEQ ID NO:535), or in genomic DNA (SEQ ID NOS:1 to SEQ ID NO:64 and sequences complementary thereto).

These probes enable diagnosis, classification and/or therapy of genetic and epigenetic parameters of colorectal cell proliferative disorders. The set of oligomers may also be used for detecting single nucleotide polymorphisms (SNPs) in treated genomic DNA (SEQ ID NOS:304 to SEQ ID NO:535), or in genomic DNA (SEQ ID NOS:1 to SEQ ID NO:64 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NOS: to SEQ ID NO:535 and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.*, each analyte) can be uniquely bound by a unique label and thus detected (each label is directly counted, resulting in a digital read-out of each molecular species in the mixture).

It is particularly preferred that the oligomers according to the invention are utilised for at least one of: detection of; detection and differentiation between or among subclasses of; diagnosis of; prognosis of; treatment of; monitoring of; and treatment and monitoring of colorectal cell proliferative disorders. This is enabled by use of said sets for the detection or detection and differentiation of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferrred are those sets of oligomer that comprise at least two oligonucleotides selected from one of the following sets of oligonucleotides:

SEQ ID NOS:89-285, 14424 - 14453;
SEQ ID NOS:89 - 109, 113 - 223, 227 - 293, 14424 - 14453;
SEQ ID NOS:89-109, 113-161, 164-223, 227-285, 287-293, 14424 - 14453;
SEQ ID NOS:89, 90, 126-135, 147-151, 224-226, 253-256, 261-267, 283-285;
SEQ ID NOS:89-161, 164-293, 14424 - 14453;
SEQ ID NOS:89-109, 113-299, 14424 - 14453;
SEQ ID NOS:89-109, 113-293, 296-299, 14424 - 14453;
SEQ ID NOS:1-12,15-20, 22, 25-36, 38-49, 51-58;
SEQ NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003, 1010 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102,1102,1103,1103-1114,1114,1115, 1115-1119,1119,1120,1120-1141,1141,1142, 1142;
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 8115 - 832, 832, 833, 833 - 836, 836, 837, 837 - 872, 872, 873, 873 - 876, 876, 877, 877, 878, 878, 879, 879 - 882, 882, 883, 883 - 885, 890 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003, 1010 - 1016, 1016, 1017, 1017, 1018, 1018, 1019, 1019 - 1028, 1028, 1029, 1029 - 1034, 1034, 1035, 1035 - 1046, 1046, 1047, 1047 - 1058,1058,1059,1059,1060,1060,1061,1061,1062,1062,1063,1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095,1095-1102,1102,1103,1103-1114,1114,1115, 1115-1119,1119,1120,1120- 1124, 1129 - 1141, 1141, 1142, 1142;

SEQ ID NOS:738 - 740, 810 - 814, 814, 815, 815 - 829, 854 - 865, 1004 - 1006, 1006, 1007, 1007 - 1009, 1062, 1062, 1063, 1063 - 1069, 1078, 1078, 1079, 1079 - 1084, 1084, 1085, 1085, 1086, 1086, 1087, 1087 - 1091, 1121 - 1124;

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142 - 1145, 1145, 1146, 1146, 1147, 1147, 1148, 1148 - 1151, 1151, 1152, 1152 - 1154, 1154, 1155, 1155, 1156, 1156, 1157, 1157, 1158, 1158, 1159, 1159;

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 885, 890 - 892, 892, 893, 893 - 896, 896, 897, 897 - 909, 909, 910, 910,911,911,912,912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142;

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 91 S, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 9 99, 9 99, 1000, 1000, 1001, 1 001, 1002, 1002, 1003, 1 003 - 1 006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142 - 1145, 1145, 1146, 1146, 1147, 1147, 1148, 1148 - 1151, 1151, 1152, 1152;

and SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142, 1145, 1145, 1146, 1146, 1147, 1147, 1148, 1148 - 1151, 1151, 1152, 1152.

In one embodiment of the method, at least one of colorectal carcinoma tissue or colon adenomas is distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NOS:1-12, 15-20, 22, 25-36. 38-49, 51-58 and complements thereof. This is preferrably achieved by use of a set consisting of at least one oligonucleotide, and more preferably at least two selected from one of the groups consisting of, or by use of a set comprising of at least two oligonucleotides selected from one of the groups consisting of:

SEQ ID NOS:89-28S & 14424 - 14453; and

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095,1095-1102, 1102,1103,1103-1114,1114, 1115,1115-1119,1119,1120,1120-1124.

In one embodiment of the method, colorectal carcinoma is distinguished from at least one tissue selected from the group consisting of non-colon healthy tissue, peripheral blood lymphocytes and non-colon cancer. In one embodiment this is achieved by analysis of the methylation status of at least one target sequence comprising, or hybridizing under stringent conditions to at least 16 contiguous nucleotides of a sequence selected from the group consisting SEQ ID NOS:1-23, 26-36, 38-43, 45-49, 51-58. This is preferrably achieved by use of a set consisting of at least one oligonucleotide, and more preferably at least two oligonucleotides selected from one of the groups consisting of:

SEQ ID NOS:89 - 109, 113 - 223, 227 - 293, 14424-14453 ; and

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003, 1010 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142.

In one embodiment of the method, colorectal carcinoma is distinguished from at least one tissue selected from the group consisting of inflammatory colon tissue, normal colon tissue, non-colon healthy tissue, peripheral blood lymphocytes, colon adenomas and non-colon cancer tissue. This is achieved by analysis of the methylation status of at least one target sequence comprising, or hybridizing under stringent conditions to at least 16 contiguous nucleotides of a sequence selected from the group consisting SEQ ID NO: 1-3, 5-13, 15-23, 26-36, 38-43, 45-49, 51-58 and complements thereof. This is preferably achieved by use of a set consisting of at least one oligonucleotide, and more preferably at least two by use of a set of oligomers comprising at least two oligonucleotides selected from one of the groups consisting of:

SEQ ID NOS:89 - 109, 113 - 161, 164 - 223, 227 - 285, 287 - 293, 14424-14453; and
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 885, 890 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003, 1010 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1124, 1129-1141,1141,1142,1142.

In one embodiment of the method, the colorectal carcinoma is distinguished from colon adenomas. This is achieved by analysis of the methylation status of at least one target sequence comprising, or hybridizing under stringent conditions to at least 16 contiguous nucleotides of a sequence selected from the group consisting SEQ ID NO: 11, 25, 27, 38, 40, 45, 53 and complements thereof. This is preferrably achieved by use of a set consisting of at least one oligonucleotide, and more preferably at least two oligonucleotides selected from one of the groups consisting of:

SEQ ID NOS:89, 90, 126 - 135, 147 - 151, 224 - 226, 253 - 256, 261 - 267, 283 - 285; and
SEQ ID NOS:738 - 740, 810 - 814, 814, 815, 815 - 829, 854 - 865, 1004 - 1006, 1006, 1007, 1007 - 1009, 1062, 1062, 1063, 1063 - 1069, 1078, 1078, 1079, 1079 - 1084, 1084, 1085, 1085, 1086, 1086, 1087, 1087 - 1091, 1121 - 1124.

In one embodiment of the method, at least one of colorectal carcinoma tissue, or colon adenomas is distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NO: 1-64 and complements thereof. This is preferably achieved by use of a set consisting of at least one oligonucleotide, and more preferably at least two oligomers comprising at least two oligonucleotides selected from one of the groups consisting of:

SEQ ID NOS: 89 - 303, 14424-14453; and
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103,1103-1114,1114,1115,1115-1119,1119, 1120, 1120 - 1141, 1141, 1142, 1142 - 1145, 1145, 1146, 1146, 1147, 1147, 1148, 1148 - 1151, 1151, 1152, 1152 - 1154, 1154, 1155, 1155, 1156, 1156, 1157, 1157, 1158, 1158, 1159, 1159.

In one embodiment of the method, colorectal carcinoma tissue is distinguished from at least one of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NOS: 1-3, 5-23, 25-36, 38-49, 51-58 and complements thereof. This is preferably achieved by use of a set consisting of at least one oligonucleotide, and more preferably at least two oligomers comprising at least two oligonucleotides selected from one of the groups consisting of:

SEQ ID NOS: 89 - 161, 164 - 293, 14424-14453; and
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 885, 890 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114,1114,1115,1115-1119,1119,1120, 1120 - 1141, 1141, 1142, 1142.

In one embodiment of the method, at least one of colorectal carcinoma tissue, or colon adenomas is distinguished from at least one tissue selected from the group consisting of inflammatory colon tissue, normal colon tissue, non-colon healthy tissue, peripheral blood lymphocytes, and non-colon cancer tissue. This is achieved by analysis of the methylation status of at least one target sequence comprising, or hybridizing under stringent conditions to at least 16 contiguous nucleotides of a sequence selected from the group consisting SEQ ID NO: 1-36, 38-43, 45-58 and complements thereof. This is preferably achieved by use of a set consisting of at least one oligonucleotide, and more preferably at least two oligomers comprising at least two oligonucleotides selected from one of the groups consisting of:

SEQ IDNOS: 89 - 109, 113 - 299, 14424-14453; and
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 9 99, 9 99, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142 - 1145, 1145, 1146, 1146, 1147, 1147, 1148, 1148 - 1151, 1151, 1152, 1152.

In one embodiment of the method, tissues originating from the colon are distinguished from tissues of non-colon origin by use of a set of oligomers comprising at least two oligonucleotides selected from one of the groups consisting of:

SEQ ID NOS: 89 - 303, 14424-14453; and
SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 999, 999, 1000, 1000, 1001, 1001, 1002, 1002, 1003, 1003 - 1006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142 - 1145,1145,1146,1146,1147,1147,1148,1148 - 1151, 1151, 1152, 1152 - 1154, 1154, 1155, 1155, 1156, 1156, 1157, 1157, 1158, 1158, 1159, 1159.

In one embodiment of the method, cell proliferative disorders are distinguished from healthy tissues by use of a set of oligomers comprising at least two oligonucleotides selected from one of the groups consisting of:

SEQ ID NOS:89 - 109, 113 - 293, 296 - 299, 14424-14453; and

SEQ ID NOS:681 - 683, 683, 684, 684, 685, 685, 686, 686, 687, 687, 688, 688 - 691, 691, 692, 692 - 695, 695, 696, 696 - 699, 699, 700, 700 - 709, 709, 710, 710 - 725, 725, 726, 726, 727, 727, 728, 728 - 760, 760, 761, 761, 762, 762, 763, 763 - 777, 784, 784, 785, 785, 786, 786, 787, 787 - 802, 802, 803, 803 - 814, 814, 815, 815 - 832, 832, 833, 833 - 836, 836, 837, 837 - 872, 872, 873, 873 - 876, 876, 877, 877, 878, 878, 879, 879 - 882, 882, 883, 883 - 892, 892, 893, 893 - 896, 896, 897, 897 - 909, 909, 910, 910, 911, 911, 912, 912 - 915, 915, 916, 916 - 921, 921 - 925, 925, 926, 926 - 939, 939, 940, 940, 941, 941, 942, 942 - 944, 944 - 957, 957, 958, 958, 959, 959, 960, 960, 961, 961, 962, 962 - 971, 971, 972, 972, 973, 973, 974, 974, 975, 975, 976, 976 - 979, 979, 980, 980, 981, 981 - 988, 988, 989, 989 - 994, 994, 995, 995 - 998, 998, 9 99, 9 99, 1000, 1 000, 1001, 1 001, 1002, 1002, 1003, 1003 - 1 006, 1006, 1007, 1007 - 1016, 1016, 1017, 1017, 1018, 1018, 1019, 1019 - 1028, 1028, 1029, 1029 - 1034, 1034, 1035, 1035 - 1046, 1046, 1047, 1047 - 1058, 1058, 1059, 1059, 1060, 1060, 1061, 1061, 1062, 1062, 1063, 1063 - 1070, 1070, 1071, 1071, 1072, 1072, 1073, 1073, 1074, 1074, 1075, 1075 - 1078, 1078, 1079, 1079 - 1084, 1084, 1085, 1085, 1086, 1086, 1087, 1087 - 1092, 1092, 1093, 1093, 1094, 1094, 1095, 1095 - 1102, 1102, 1103, 1103 - 1114, 1114, 1115, 1115 - 1119, 1119, 1120, 1120 - 1141, 1141, 1142, 1142, 1145, 1145, 1146, 1146, 1147, 1147, 1148, 1148 - 1151, 1151, 1152,1152.

In a further embodiment of the method aerodigestive cell proliferative disorders of the group consisting lung, liver, pancreas, bile duct, stomach, and colon cancers are detected. This is achieved by analysis of the methylation status of at least This is achieved by analysis of the methylation status of at least one target sequence comprising, or hybridizing under stringent conditions to at least 16 contiguous nucleotides of a sequence selected from the group consisting SEQ ID NO: 20, 41, 35, 3, 39, 33 and complements thereof.The present invention further provides a method for ascertaining genetic and/or epigenetic parameters of the genomic sequences according to SEQ ID NOS:1 to SEQ ID NO:64 within a subject by analyzing cytosine methylation and single nucleotide polymorphisms. Said method comprising contacting a nucleic acid comprising one or more of SEQ ID NOS:1 to SEQ ID NO:64 in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step,* a sample of the tissue to be analysed is obtained. The source may be any suitable source, such as cell lines, histological slides, biopsies, paraffin-embedded tissue, bodily fluids, stool, colonic effluent, urine, blood plasma, blood serum, whole blood, isolated blood cells, cells isolated from the blood and all possible combinations thereof. It is preferred that said sources of DNA are stool or bodily fluids selected from the group consisting colonic effluent , urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NOS:304 to SEQ ID NO:535 and sequences complementary thereto.

In an alternate embodiment of the method, the methylation status of preselected C pG positions within the nucleic acid sequences comprising one or more of SEQ ID NOS:1 to SEQ ID NO:64 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NOS:304 to SEQ ID NO: 535 and sequences complementary thereto, wherein the base sequence of said oligomers comprises at least one CpG dinucleotide.

In a further preferred embodiment of the method, the MSP primers are selected from the group consisting SEQ ID NOS:2754, 2757, 2759, 2767, 2768, 2772, 2776, 2779, 2784, 2785, 2786, 2789, 2791, 2792, 2794, 2797, 2798, 2801, 2805, 2806, 2809, 2811, 2815, 2817, 2824, 2830, 2832, 2833, 2834, 2838, 2840, 2843, 2846, 2850, 2851, 2853, 2855, 2859, 2866, 2867, 2870, 2871, 2876, 2878, 2881, 2885, 2886, 2892, 2893, 2895, 2896, 2898, 2904, 2912, 2915, 2917, 2919, 2922, 2923, 2929, 2931, 2939, 2941, 2944, 2946, 2948, 2950, 2955, 2956, 2957, 2960, 2962, 2965, 2966, 2967, 2968, 2969, 2971, 2975, 2976, 2977, 2978, 2979, 2980, 2981, 2984, 2988, 2989, 2990, 2992, 2994, 2997, 2999, 3001, 3002, 3004, 3006, 3007, 3008, 3010, 3013, 3014, 3016, 3017, 3018, 3020, 3022, 3025, 2752, 2755, 2760, 2762, 2763, 2764, 2765, 2769, 2771, 2773, 2775, 2777, 2780, 2781, 2782, 2783, 2787, 2788, 2790, 2793, 2795, 2796, 2800, 2802, 2803, 2804, 2808, 2810, 2812, 2814, 2816, 2818, 2820, 2821, 2823, 2825, 2827, 2828, 2829, 2831, 2835, 2836, 2837, 2839, 2841, 2842, 2844, 2845, 2847, 2849, 2852, 2854, 2856, 2858, 2860, 2861, 2862, 2863, 2864, 2865, 2868, 2869, 2872, 2873, 2874, 2875, 2877, 2879, 2880, 2882, 2883, 2884, 2887, 2888, 2889, 2890, 2891, 2894, 2897, 2899, 2901, 2902, 2903, 2905, 2907, 2909, 2911, 2914, 2916, 2918, 2920, 2924, 2926, 2927, 2928, 2930, 2932, 2933, 2934, 2935, 2936, 2937, 2938, 2942, 2945, 2947, 2949, 2951, 2953, 2954, 2958, 2959, 2961, 2963, 2970, 2972, 2973, 2974, 2982, 2985, 2987, 2991, 2993, 2995, 2996, 2998, 3000, 3003, 3005, 3011, 3019, 3021, 3023, 3026, 3027, 3028, 3032, 3033, 3036, 3037, 3038, 3039, 3041, 3042, 3043, 3044, 3047, 3048, 3049, 3052, 3055, 3059, 3061, 3064, 3065, 3068, 3069, 3071, 3072, 3075, 3076, 3080, 3083, 3084, 3085, 3086, 3091, 3093, 3096, 3097, 3100, 3104, 3109, 3110, 3113, 3115, 3117, 3118, 3123, 3126, 3127, 3130, 3134, 3135, 3136, 3138, 3139, 3144, 3146, 3147, 3149, 3150, 3155, 3029, 3031, 3034, 3040, 3045, 3050, 3051, 3053, 3054, 3056, 3057, 3060, 3063, 3066, 3073, 3078, 3081, 3088, 3089, 3090, 3092, 3094, 3098, 3101, 3103, 3105, 3107, 3108, 3111, 3114, 3116, 3119, 3121, 3122, 3124, 3128, 3131, 3133, 3137, 3140, 3142, 3145, 3148, 3151, 3152, 3153, 3157, 3159, 3165, 3166, 3167, 3170, 3171, 3172, 3174, 3175, 3176, 3179, 3180, 3181, 3184, 3185, 3186, 3192, 3194, 3198, 3201, 3202, 3203, 3204, 3206, 3209, 3212, 3213, 3215, 3216, 3217, 3218, 3221, 3224, 3226, 3228, 3229, 3230, 3232, 3233, 3234, 3235, 3237, 3238, 3239, 3245, 3247, 3248, 3249, 3160, 3162, 3163, 3164, 3168, 3169, 3173, 3178, 3182, 3183, 3187, 3189, 3190, 3195, 3196, 3197, 3199, 3205, 3207, 3210, 3214, 3219, 3222, 3223, 3225, 3227, 3236, 3240, 3242, 3243, 3244, 3246, 3250, 3252, 3256, 3261, 3264, 3268, 3269, 3270, 3273, 3275, 3279, 3280, 3281, 3282, 3283, 3284, 3285, 3286, 3290, 3292, 3295, 3297, 3301, 3304, 3305, 3306, 3307, 3308, 3310, 3311, 3312, 3315, 3319, 3320, 3324, 3327, 3331, 3333, 3334, 3339, 3348, 3351, 3354, 3358, 3360, 3364, 3367, 3368, 3374, 3376, 3253, 3255, 3257, 3259, 3260, 3262, 3265, 3266, 3267, 3271, 3274, 3276, 3278, 3287, 3289, 3291, 3293, 3298, 3300, 3302, 3309, 3313, 3314, 3316, 3318, 3321, 3322, 3325, 3328, 3330, 3332, 3335, 3337, 3338, 3340, 3342, 3343, 3344, 3345, 3346, 3347, 3349, 3350, 3352, 3353, 3355, 3356, 3357, 3359, 3361, 3363, 3365, 3369, 3371, 3372, 3373, 3377, 3380, 3383, 3386, 3388, 3389, 3390, 3391, 3392, 3395, 3396, 3400, 3401, 3403, 3406, 3408, 3413, 3414, 3417, 3420, 3423, 3428, 3432, 3433, 3434, 3436, 3439, 3440, 3442, 3443, 3445, 3447, 3453, 3455, 3458, 3463, 3466, 3469, 3470, 3471, 3472, 3473, 3474, 3475, 3477, 3478, 3480, 3481, 3483, 3484, 3487, 3490, 3494, 3495, 3496, 3497, 3499, 3500, 3504, 3505, 3508, 3512, 3514, 3515, 3516, 3521, 3525, 3529, 3533, 3534, 3537, 3538, 3540, 3541, 3542, 3543, 3544, 3547, 3548, 3553, 3557, 3558, 3559, 3560, 3562, 3563, 3566, 3571, 3574, 3575, 3577, 3580, 3583, 3584, 3585, 3586, 3592, 3594, 3595, 3597, 3598, 3599, 3601, 3603, 3604, 3607, 3608, 3612, 3619, 3621, 3622, 3626, 3631, 3632, 3637, 3638, 3639, 3640, 3642, 3643, 3644, 3646, 3647, 3649, 3650, 3652, 3656, 3657, 3660, 3663, 3668, 3670, 3673, 3676, 3677, 3678, 3683, 3688, 3690, 3694, 3697, 3700, 3702, 3703, 3704, 3708, 3404, 3409, 3411, 3415, 3418, 3419, 3421, 3424, 3426, 3427, 3429, 3431, 3435, 3437, 3438, 3441, 3444, 3446, 3448, 3450, 3452, 3454, 3456, 3459, 3461, 3462, 3464, 3467, 3476, 3479, 3482, 3485, 3488, 3489, 3491, 3492, 3493, 3501, 3503, 3506, 3509, 3510, 3511, 3513, 3517, 3518, 3519, 3520, 3522, 3524, 3526, 3527, 3528, 3530, 3531, 3535, 3536, 3545, 3549, 3551, 3552, 3554, 3555, 3556, 3561, 3564, 3565, 3568, 3569, 3570, 3573, 3576, 3578, 3581, 3587, 3588, 3589, 3590, 3591, 3593, 3596, 3600, 3602, 3605, 3606, 3609, 3611, 3613, 3614, 3615, 3616, 3617, 3618, 3623, 3624, 3625, 3627, 3629, 3630, 3633, 3634, 3635, 3636, 3641, 3645, 3651, 3653, 3654, 3655, 3658, 3661, 3662, 3665, 3666, 3667, 3669, 3671, 3674, 3679, 3681, 3684, 3686, 3689, 3691, 3692, 3693, 3695, 3696, 3698, 3701, 3705, 3706, 3707, 3709, 3710, 3714, 3715, 3717, 3718, 3722, 3723, 3729, 3730, 3732, 3735, 3739, 3740, 3741, 3743, 3744, 3745, 3746, 3749, 3753, 3754, 3755, 3756, 3711, 3713, 3719, 3721, 3724, 3725, 3726, 3727, 3728, 3731, 3733, 3736, 3737, 3738, 3742, 3747, 3748, 3750, 3752, 3757, 3758, 3759, 3760, 3761, 3764, 3767, 3771, 3773, 3775, 3776, 3777, 3781, 3785, 3786, 3787, 3790, 3791, 3793, 3796, 3797, 3798, 3799, 3805, 3809, 3814, 3817, 3818, 3822, 3823, 3828, 3833, 3836, 3844, 3847, 3850, 3851, 3853, 3856, 3857, 3862, 3873, 3876, 3877, 3880, 3885, 3887, 3889, 3890, 3892, 3893, 3895, 3896, 3899, 3902, 3903, 3905, 3906, 3907, 3908, 3916, 3923, 3926, 3927, 3930, 3935, 3936, 3938, 3939, 3940, 3944, 3945, 3949, 3950, 3951, 3954, 3955, 3957, 3960, 3965, 3970, 3971, 3978, 3979, 3982, 3983, 3984, 3985, 3762, 3765, 3768, 3770, 3772, 3774, 3778, 3779, 3780, 3782, 3784, 3788, 3792, 3794, 3800, 3801, 3802, 3803, 3804, 3806, 3807, 3808, 3810, 3812, 3813, 3815, 3819, 3820, 3821, 3824, 3826, 3827, 3829, 3831, 3832, 3834, 3837, 3838, 3839, 3840, 3842, 3845, 3848, 3852, 3854, 3858, 3860, 3861, 3863, 3864, 3865, 3866, 3867, 3868, 3869, 3870, 3871, 3872, 3874, 3875, 3878, 3881, 3883, 3884, 3886, 3888, 3891, 3894, 3897, 3898, 3900, 3901, 3904, 3909, 3911, 3914, 3915, 3917, 3919, 3920, 3921, 3922, 3924, 3928, 3931, 3933, 3934, 3937, 3941, 3942, 3943, 3947, 3948, 3952, 3958, 3959, 3961, 3962, 3963, 3964, 3966, 3968, 3972, 3974, 3975, 3976, 3980, 3981, 3986, 3987, 3990, 3993, 3994, 3997, 4000, 4003, 4004, 4006, 4012, 4015, 4017, 4020, 4024, 4027, 4029, 4031, 4032, 4033, 4037, 4038, 4040, 4042, 4047, 4048, 4050, 4051, 4052, 4054, 4055, 4058, 4063, 4066, 4069, 4070, 4072, 4073, 4076, 4078, 4079, 4082, 4083, 4084, 4085, 4086, 4090,4093, 4094, 4095, 4096, 4099, 4100, 4104, 4106, 4113, 4116, 4119, 4120, 4124, 4127, 4130, 4134, 4135, 4136, 4138, 4142, 4145, 4146, 4149, 4150, 4152, 4153, 4159, 4160, 4161, 4163, 4168, 4169, 4171, 4173, 4176, 4180, 4181, 4185, 4189, 4191, 4192, 4194, 4197, 4198, 4199, 4201, 4206, 4207, 4208, 4213, 4219, 4220, 4223, 4224, 4225, 4228, 4231, 4232, 4233, 4237, 4238, 4242, 4243, 4246, 4247, 4251, 4254, 4255, 4256, 4258, 3988, 3991, 3995, 3998, 4001, 4005, 4007, 4009, 4010, 4011, 4013, 4016, 4018, 4019, 4021, 4022, 4023, 4025, 4028, 4034, 4035, 4039, 4043, 4045, 4046, 4049, 4056, 4059, 4061, 4064, 4065, 4067, 4068, 4071, 4074, 4075, 4077, 4080, 4087, 4088, 4089, 4092, 4098, 4101, 4102, 4105, 4108, 4109, 4110, 4111, 4112, 4114, 4115, 4117, 4121, 4122, 4123, 4125, 4128, 4129, 4131, 4132, 4133, 4137, 4139, 4141, 4144, 4148, 4151, 4154, 4156, 4157, 4162, 4165, 4166, 4167, 4170, 4172, 4174, 4177, 4179, 4182, 4184, 4186, 4188, 4190, 4195, 4196, 4202, 4204, 4205, 4209, 4211, 4214, 4216, 4217, 4218, 4221, 4226, 4229, 4234, 4236, 4239, 4241, 4244, 4245, 4248, 4249, 4252, 4257, 4259, 4260, 4261, 4262, 4266, 4267, 4268, 4271, 4272, 4275, 4276, 4279, 4281, 4282, 4284, 4285, 4287, 4289, 4290, 4292, 4295, 4297, 4299, 4301, 4306, 4310, 4316, 4317, 4318, 4321, 4323, 4325, 4328, 4334, 4335, 4337, 4341, 4342, 4343, 4344, 4348, 4350, 4351, 4352, 4354, 4357, 4358, 4359, 4364, 4365, 4366, 4370, 4371, 4372, 4373, 4376, 4378, 4381, 4383, 4384, 4386, 4388, 4396, 4397, 4399, 4400, 4403, 4405, 4407, 4409, 4412, 4415, 4416, 4417, 4424, 4426, 4429, 4433, 4435, 4437, 4442, 4443, 4447, 4450, 4451, 4452, 4455, 4457, 4460, 4461, 4462, 4465, 4466, 4469, 4470, 4474, 4478, 4481, 4484, 4488, 4491, 4494, 4495, 4497, 4498, 4504, 4506, 4509, 4510, 4511, 4514, 4516, 4518, 4520, 4521, 4523, 4525, 4528, 4530, 4533, 4534, 4535, 4538, 4540, 4542, 4545, 4547, 4548, 4553, 4554, 4555, 4556, 4557, 4562, 4564, 4567, 4568, 4569, 4579, 4580, 4582, 4584, 4587, 4589, 4593, 4596, 4597, 4598, 4600, 4601, 4602, 4605, 4606, 4607, 4609, 4610, 4613, 4614, 4616, 4618, 4621, 4624, 4626, 4627, 4628, 4631, 4632, 4636, 4639, 4641, 4644, 4645, 4648, 4263, 4265, 4269, 4270, 4273, 4274, 4277, 4280, 4283, 4286, 4288, 4291, 4293, 4296, 4298, 4300, 4302, 4303, 4304, 4305, 4307, 4308, 4309, 4311, 4313, 4315, 4319, 4322, 4324, 4326, 4327, 4330, 4331, 4332, 4333, 4336, 4338, 4340, 4345, 4347, 4349, 4353, 4355, 4360, 4361, 4362, 4367, 4368, 4369, 4374, 4375, 4377, 4379, 4382, 4385, 4387, 4389, 4390, 4391, 4392, 4393, 4394, 4395, 4398, 4401, 4406, 4408, 4410, 4411, 4413, 4418, 4420, 4422, 4423, 4425, 4427, 4428, 4430, 4432, 4434, 4436, 4438, 4440, 4441, 4444, 4446, 4448, 4453, 4454, 4456, 4458, 4463, 4464, 4467, 4471, 4472, 4473, 4475, 4476, 4477, 4479, 4480, 4482, 4485, 4486, 4487, 4489, 4490, 4492, 4496, 4499, 4501, 4502, 4503, 4505, 4507, 4508, 4512, 4515, 4517, 4519, 4522, 4524, 4529, 4531, 4536, 4537, 4539, 4541, 4543, 4546, 4549, 4551, 4558, 4559, 4560, 4561, 4563, 4565, 4570, 4571, 4572, 4573, 4574, 4575, 4576, 4578, 4581, 4583, 4585, 4586, 4588, 4590, 4592, 4595, 4599, 4603, 4611, 4612, 4615, 4617, 4619, 4620, 4622, 4630, 4633, 4634, 4635, 4638, 4640, 4642, 4643, 4646, 4647, 4649, 4652, 4655, 4659, 4661, 4663, 4664, 4665, 4668, 4671, 4674, 4675, 4676, 4678, 4680, 4683, 4686, 4690, 4696, 4700, 4703, 4704, 4706, 4709, 4711, 4714, 4715, 4717, 4718, 4719, 4720, 4721, 4726, 4727, 4730, 4732, 4738, 4742, 4745, 4749, 4750, 4753, 4755, 4759, 4763, 4768, 4769, 4772, 4773, 4776, 4778, 4779, 4782, 4784, 4789, 4790, 4794, 4797, 4801, 4805, 4807, 4813, 4814, 4819, 4821, 4822, 4827, 4829, 4833, 4834, 4835, 4837, 4838, 4841, 4842, 4843, 4844, 4847, 4849, 4850, 4852, 4853, 4859, 4860, 4861, 4865, 4867, 4868, 4869, 4870, 4873, 4876, 4877, 4878, 4879, 4883, 4885, 4889, 4891, 4892, 4894, 4895, 4897, 4898, 4903, 4906, 4911, 4912, 4918, 4919, 4920, 4924, 4927, 4929, 4932, 4934, 4941, 4942, 4945, 4948, 4949, 4950, 4650, 4653, 4656, 4658, 4660, 4662, 4666, 4667, 4669, 4670, 4672, 4677, 4681, 4684, 4685, 4687, 4688, 4689, 4691, 4693, 4694, 4695, 4697, 4699, 4701, 4702, 4705, 4707, 4708, 4716, 4722, 4724, 4728, 4731, 4733, 4735, 4736, 4737, 4739, 4741, 4744, 4746, 4748, 4751, 4754, 4756, 4758, 4760, 4761, 4764, 4766, 4767, 4770, 4771, 4774, 4777, 4780, 4783, 4785, 4786, 4787, 4788, 4791, 4792, 4793, 4795, 4798, 4800, 4802, 4804, 4806, 4808, 4810, 4811, 4812, 4815, 4816, 4817, 4818, 4820, 4823, 4824, 4825, 4828, 4830, 4832, 4836, 4839, 4840, 4845, 4846, 4848, 4851, 4854, 4855, 4856, 4857, 4858, 4862, 4864, 4871, 4872, 4874, 4875, 4881, 4882, 4884, 4886, 4887, 4890, 4893, 4896, 4899, 4900, 4901, 4902, 4904, 4905, 4907, 4908, 4909, 4910, 4913, 4915, 4917, 4921, 4922, 4923, 4925, 4926, 4928, 4930, 4935, 4936, 4937, 4938, 4940, 4943, 4944, 4946, 4947, 4951, 4952, 4953, 4954, 4956, 4957, 4960, 4963, 4967, 4970, 4971, 4972, 4976, 4977, 4985, 4986, 4987, 4989, 4991, 4992, 4997, 4999, 5002, 5003, 5004, 5006, 5010, 5013, 5014, 5015, 5017, 5018, 5019, 5020, 5021, 5024, 5025, 5027, 5031, 5032, 5033, 5037, 5040, 5041, 5042, 5043, 4958, 4961, 4964, 4966, 4968, 4969, 4973, 4974, 4975, 4978, 4979, 4980, 4981, 4982, 4983, 4984, 4988, 4990, 4993, 4995, 4996, 4998, 5000, 5005, 5007, 5009, 5011, 5016, 5022, 5026, 5028, 5030, 5034, 5036, 5038, 5039, 5044, 5047, 5049, 5050, 5052, 5054, 5055, 5057, 5060, 5061, 5064, 5045, 5048, 5051, 5053, 5056, 5058, 5062, 5065, 5066, 5069, 5073, 5077, 5080, 5083, 5087, 5093, 5096, 5099, 5100, 5103, 5104, 5106, 5111, 5116, 5119, 5120, 5125, 5128, 5132, 5134, 5135, 5137, 5139, 5140, 5142, 5144, 5148, 5149, 5150, 5155, 5157, 5158, 5160, 5166, 5168, 5170, 5171, 5173, 5176, 5177, 5179, 5184, 5187, 5188, 5190, 5193, 5195, 5198, 5201, 5204, 5208, 5211, 5215, 5216, 5217, 5220, 5221, 5222, 5224, 5226, 5229, 5230, 5231, 5232, 5234, 5237, 5242, 5244, 5245, 5246, 5247, 5249, 5250, 5252, 5254, 5255, 5256, 5261, 5262, 5264, 5265, 5273, 5274, 5276, 5278, 5279, 5281, 5284, 5285, 5286, 5287, 5288, 5289, 5292, 5296, 5300, 5303, 5304, 5307, 5308, 5309, 5313, 5315, 5317, 5318, 5322, 5323, 5326, 5329, 5330, 5333, 5336, 5337, 5341, 5343, 5348, 5349, 5352, 5354, 5355, 5358, 5359, 5361, 5362, 5363, 5364, 5365, 5367, 5369, 5370, 5372, 5375, 5377, 5379, 5382, 5385, 5387, 5393, 5394, 5395, 5398, 5400, 5404, 5407, 5408, 5409, 5412, 5413, 5414, 5416, 5418, 5421, 5424, 5426, 5428, 5431, 5432, 5433, 5434, 5435, 5437, 5440, 5441, 5443, 5448, 5450, 5451, 5454, 5455, 5458, 5460, 5461, 5464, 5468, 5470, 5471, 5474, 5475, 5477, 5482, 5484, 5485, 5487, 5490, 5491, 5492, 5494, 5496, 5498, 5499, 5500, 5501, 5502, 5503, 5504, 5506, 5509, 5510, 5512, 5516, 5517, 5518, 5524, 5525, 5526, 5528, 5531, 5533, 5534, 5535, 5536, 5537, 5540, 5542, 5544, 5545, 5546, 5549, 5550, 5552, 5553, 5559, 5561, 5566, 5569, 5572, 5573, 5575, 5577, 5578, 5579, 5580, 5581, 5582, 5588, 5593, 5595, 5596, 5598, 5600, 5601, 5603, 5604, 5605, 5608, 5614, 5615, 5616, 5619, 5622, 5624, 5625, 5628, 5629, 5633, 5634, 5637, 5638, 5639, 5643, 5644, 5645, 5647, 5648, 5649, 5650, 5654, 5662, 5664, 5067, 5070, 5072, 5074, 5075, 5076, 5078, 5081, 5084, 5086, 5088, 5090, 5091, 5094, 5097, 5101, 5105, 5107, 5109, 5110, 5112, 5114, 5115, 5117, 5121, 5123, 5124, 5126, 5129, 5131, 5136, 5143, 5145, 5147, 5151, 5153, 5156, 5159, 5161, 5162, 5163, 5164, 5165, 5167, 5169, 5172, 5174, 5175, 5180, 5181, 5182, 5183, 5185, 5186, 5189, 5191, 5194, 5196, 5199, 5200, 5202, 5203, 5205, 5207, 5209, 5210, 5212, 5214, 5218, 5219, 5223, 5225, 5227, 5233, 5235, 5238, 5239, 5240, 5241, 5248, 5251, 5253, 5257, 5259, 5260, 5263, 5266, 5268, 5269, 5270, 5271, 5272, 5280, 5282, 5290, 5291, 5293, 5295, 5297, 5299, 5301, 5306, 5310, 5311, 5312, 5314, 5316, 5319, 5321, 5324, 5327, 5331, 5332, 5334, 5338, 5339, 5340, 5342, 5344, 5346, 5347, 5351, 5353, 5356, 5360, 5366, 5368, 5371, 5373, 5376, 5378, 5380, 5383, 5388, 5389, 5391, 5392, 5396, 5399, 5401, 5403, 5405, 5410, 5415, 5417, 5419, 5420, 5422, 5425, 5427, 5429, 5436, 5438, 5442, 5444, 5445, 5446, 5447, 5449, 5452, 5456, 5457, 5459, 5462, 5465, 5466, 5467, 5469, 5472, 5478, 5480, 5481, 5486, 5488, 5493, 5495, 5497, 5505, 5507, 5508, 5511, 5513, 5515, 5519, 5521, 5522, 5527, 5529, 5532, 5538, 5539, 5541, 5543, 5548, 5551, 5554, 5555, 5556, 5557, 5558, 5560, 5562, 5563, 5564, 5565, 5567, 5568, 5570, 5571, 5574, 5576, 5583, 5585, 5586, 5587, 5589, 5591, 5592, 5594, 5597, 5599, 5602, 5606, 5609, 5611, 5612, 5618, 5620, 5623, 5626, 5627, 5630, 5632, 5635, 5640, 5642, 5646, 5651, 5652, 5656, 5657, 5659, 5660, 5661, 5663, 5665, 5667, 5670, 5672, 5673, 5676, 5678, 5679, 5681, 5682, 5683, 5686, 5687, 5688, 5690, 5694, 5697, 5698, 5699, 5700, 5701, 5704, 5708, 5709, 5711, 5713, 5714, 5717, 5721, 5723, 5724, 5727, 5728, 5733, 5734, 5735, 5736, 5739, 5740, 5741, 5742, 5743, 5668, 5671, 5674, 5677, 5680, 5684, 5689, 5691, 5693, 5696, 5702, 5705, 5707. 5710, 5712, 5715, 5718, 5720, 5722, 5725, 5729, 5730, 5731, 5732, 5737, 5744, 5747, 5750, 5753, 5759, 5762, 5763, 5770, 5775, 5777, 5778, 5780, 5782, 5785, 5787, 5791, 5792, 5793, 5794, 5797, 5801, 5803, 5808, 5810, 5812, 5814, 5816, 5817, 5824, 5832, 5833, 5834, 5835, 5838, 5840, 5841, 5844, 5849, 5853, 5854, 5857, 5859, 5860, 5862, 5863, 5865, 5868, 5874, 5882, 5883, 5885, 5887, 5894, 5895, 5896, 5897, 5902, 5904, 5906, 5909, 5912, 5915, 5918, 5919, 5920, 5922, 5923, 5926, 5927, 5928, 5930, 5932, 5933, 5934, 5935, 5939, 5940, 5946, 5947, 5948, 5949, 5952, 5954, 5955, 5956, 5958, 5959, 5961, 5962, 5964, 5965, 5968, 5970, 5971, 5972, 5973, 5977, 5978, 5980, 5983, 5986, 5987, 5992, 5993, 5996, 5997, 6000, 6002, 6003, 6004, 6005, 6006, 6008, 6009, 6010, 6011, 6012, 6013, 6017, 6018, 6019, 6020, 6025, 6027, 6030, 6033, 6035, 6036, 6039, 6041, 6042, 6045, 6049, 6050, 6051, 6055, 6059, 6063, 6064, 6065, 6067, 6070, 6072, 6073, 6075, 6077, 6078, 6081, 5745, 5748, 5751, 5754, 5756, 5758, 5760, 5764, 5766, 5767, 5768, 5769, 5771, 5773, 5774, 5776, 5779, 5781, 5783, 5786, 5788, 5789, 5790, 5795, 5796, 5798, 5800, 5802, 5804, 5806, 5807, 5809, 5811, 5813, 5818, 5820, 5821, 5822, 5823, 5825, 5826, 5827, 5828, 5830, 5831, 5836, 5837, 5839, 5842, 5843, 5845, 5846, 5847, 5848, 5850, 5852, 5855, 5856, 5858, 5861, 5864, 5866, 5867, 5870, 5871, 5872, 5873, 5875, 5876, 5877, 5878, 5879, 5880, 5881, 5884, 5886, 5888, 5889, 5890, 5891, 5892, 5893, 5898, 5899, 5900, 5903, 5905, 5907, 5910, 5913, 5916, 5921, 5924, 5929, 5931, 5936, 5937, 5938, 5942, 5943, 5944, 5945, 5950, 5953, 5957, 5960, 5963, 5966, 5969, 5975, 5976, 5981, 5984, 5988, 5990, 5991, 5994, 5998, 5999, 6001, 6007, 6014, 6015, 6016, 6021, 6023, 6024, 6026, 6028, 6031, 6032, 6034, 6037, 6038, 6040, 6043, 6046, 6048, 6053, 6054, 6056, 6058, 6060, 6062, 6066, 6068, 6074, 6076, 6079, 6083, 6084, 6085, 6089, 6090, 6093, 6096, 6098, 6100, 6106, 6110, 6111, 6114, 6118, 6121, 6127, 6130, 6133, 6135, 6137, 6138, 6141, 6143, 6145, 6148, 6150, 6151, 6152, 6153, 6154, 6156, 6159, 6160, 6162, 6163, 6166, 6167, 6168, 6169, 6170, 6171, 6172, 6175, 6177, 6180, 6183, 6187, 6188, 6190, 6193, 6194, 6195, 6196, 6198, 6201, 6202, 6204, 6207, 6211, 6212, 6214, 6215, 6216, 6218, 6219, 6220, 6222, 6225, 6226, 6230, 6235, 6239, 6241, 6242, 6243, 6244, 6245, 6247, 6250, 6252, 6254, 6259, 6260, 6261, 6264, 6265, 6268, 6270, 6273, 6275, 6278, 6280, 6282, 6286, 6287, 6288, 6291, 6293, 6298, 6299, 6300, 6302, 6303, 6305, 6307, 6308, 6312, 6313, 6314, 6315, 6316, 6317, 6318, 6320, 6321, 6323, 6325, 6327, 6328, 6334, 6335, 6337, 6340, 6341, 6344, 6348, 6350, 6353, 6354, 6357, 6360, 6362, 6364, 6368, 6372, 6374, 6377, 6378, 6383, 6386, 6392, 6394, 6398, 6400, 6404, 6405, 6409, 6410, 6411, 6417, 6418, 6424, 6426, 6429, 6431, 6432, 6433, 6435, 6436, 6437, 6438, 6440, 6442, 6445, 6446, 6448, 6449, 6452, 6455, 6457, 6458, 6461, 6462, 6463, 6464, 6465, 6470, 6473, 6475, 6478, 6480, 6481, 6484, 6485, 6486, 6489, 6491, 6492, 6494, 6498, 6502, 6504, 6505, 6507, 6511, 6513, 6514, 6086, 6088, 6091, 6094, 6097, 6099, 6101, 6103, 6105, 6108, 6112, 6115, 6116, 6117, 6119, 6120, 6122, 6123, 6125, 6126, 6128, 6131, 6132, 6134, 6136, 6139, 6142, 6144, 6146, 6149, 6155, 6157, 6161, 6164, 6173, 6174, 6176, 6178, 6181, 6182, 6184, 6186, 6189, 6191, 6192, 6197, 6199, 6200, 6205, 6206, 6208, 6210, 6213, 6221, 6223, 6224, 6227, 6229, 6231, 6232, 6233, 6234, 6236, 6237, 6238, 6240, 6246, 6248, 6251, 6253, 6255, 6256, 6257, 6258, 6262, 6266, 6269, 6271, 6272, 6274, 6276, 6277, 6281, 6283, 6284, 6285, 6289, 6292, 6294, 6295, 6296, 6297, 6301, 6304, 6306, 6309, 6310, 6311, 6319, 6322, 6324, 6326, 6329, 6330, 6331, 6333, 6336, 6338, 6342, 6345, 6347, 6349, 6351, 6355, 6356, 6358, 6359, 6361, 6365, 6367, 6369, 6371, 6373, 6375, 6376, 6379, 6381, 6382, 6384, 6387, 6388, 6389, 6390, 6393, 6395, 6397, 6399, 6401, 6402, 6403, 6406, 6407, 6412, 6413, 6414, 6415, 6416, 6419, 6420, 6421, 6422, 6423, 6425, 6427, 6428, 6434, 6441, 6443, 6444, 6447, 6450, 6451, 6453, 6459, 6460, 6466, 6467, 6468, 6469, 6471, 6472, 6474, 6476, 6479, 6482, 6487, 6490, 6493, 6496, 6497, 6499, 6500, 6501, 6503, 6506, 6508, 6510, 6512, 6515, 6518, 6521, 6522, 6523, 6524, 6528, 6532, 6535, 6539, 6549, 6560, 6561, 6564, 6566, 6567, 6570, 6573, 6576, 6577, 6578, 6579, 6583, 6584, 6585, 6586, 6587, 6590, 6591, 6594, 6596, 6597, 6603, 6605, 6608, 6609, 6610, 6611, 6612, 6615, 6625, 6630, 6632, 6633, 6634, 6638, 6639, 6642, 6643, 6644, 6647, 6650, 6655, 6657, 6658, 6659, 6661, 6663, 6664, 6665, 6669, 6671, 6674, 6677, 6678, 6679, 6680, 6681, 6682, 6686, 6687, 6688, 6689, 6690, 6691, 6693, 6695, 6696, 6697, 6699, 6701, 6703, 6704, 6705, 6706, 6707, 6708, 6710, 6711, 6712, 6713, 6717, 6718, 6720, 6721, 6722, 6725, 6727, 6732, 6734, 6735, 6739, 6741, 6745, 6748, 6751, 6755, 6759, 6762, 6764, 6767, 6770, 6772, 6773, 6774, 6777, 6778, 6782, 6785, 6786, 6787, 6790, 6791, 6794, 6795, 6796, 6798, 6800, 6802, 6803, 6806, 6811, 6814, 6815, 6818, 6824, 6829, 6834, 6835, 6842, 6516, 6519, 6525, 6526, 6527, 6529, 6531, 6533, 6536, 6537, 6538, 6540, 6541, 6542, 6543, 6544, 6545, 6546, 6547, 6548, 6550, 6551, 6552, 6553, 6554, 6556, 6558, 6562, 6563, 6565, 6568, 6571, 6572, 6575, 6580, 6582, 6588, 6589, 6592, 6595, 6598, 6599, 6600, 6601, 6602, 6604, 6606, 6607, 6613, 6614, 6617, 6618, 6619, 6620, 6621, 6623, 6624, 6627, 6628, 6629, 6631, 6635, 6640, 6641, 6645, 6646, 6649, 6651, 6652, 6653, 6656, 6660, 6662, 6666, 6668, 6670, 6672, 6675, 6676, 6683, 6685, 6692, 6694, 6698, 6700, 6702, 6709, 6714, 6715, 6716, 6719, 6723, 6726, 6728, 6730, 6731, 6736, 6738, 6740, 6742, 6744, 6746, 6747, 6749, 6752, 6754, 6756, 6757, 6758, 6760, 6761, 6765, 6769, 6775, 6779, 6781, 6783, 6784, 6789, 6792, 6797, 6799, 6801, 6804, 6805, 6807, 6809, 6810, 6812, 6816, 6817, 6819, 6821, 6822, 6825, 6827, 6828, 6830, 6831, 6832, 6833,6836,6837,6838,6839,6840,6841,6843,

Detection: SEQ ID NOS:6517, 6520, 6530, 6534, 6555, 6557, 6559, 6569, 6574, 6581, 6593, 6616, 6622, 6626, 6636, 6637, 6648, 6654, 6667, 6673, 6684, 6724, 6729, 6733, 6737, 6743, 6750, 6753, 6763, 6766, 6768, 6771, 6776, 6780, 6788, 6793, 6808, 6813, 6820, 6823, 6826, 6844, 6848, 6850, 6852, 6854, 6857, 6859, 6861, 6862, 6865, 6868, 6870, 6871, 6872, 6874, 6875, 6876, 6880, 6883, 6886, 6887, 6888, 6890, 6891, 6894, 6896, 6897, 6898, 6899, 6900, 6901, 6902, 6904, 6905, 6906, 6910, 6912, 6917, 6920, 6845, 6847, 6849, 6851, 6853, 6855, 6858, 6860, 6863, 6866, 6869, 6873, 6877, 6879, 6881, 6882, 6884, 6889, 6892, 6893, 6895, 6907, 6908, 6909, 6911, 6913, 6915, 6916, 6918, 6921, 6924, 6927, 6928, 6933, 6935, 6937, 6939, 6940, 6943, 6946, 6947, 6948, 6949, 6951, 6953, 6954, 6955, 6959, 6961, 6964, 6965, 6968, 6969, 6972, 6973, 6976, 6979, 6980, 6922, 6925, 6929, 6930, 6931, 6932, 6934, 6936, 6942, 6944, 6945, 6950, 6952, 6956, 6957, 6958, 6960, 6962, 6963, 6966, 6967, 6970, 6971, 6974, 6975, 6977, 6981, 6983, 6986, 6992, 6995, 6998, 7002, 7006, 7013, 7017, 7022, 7024, 7027, 7030, 7032, 7036, 7037, 7040, 7050, 7052, 7055, 7056, 7058, 7059, 7060, 7064, 7067, 7072, 7074, 7076, 7077, 7084, 7085, 7086, 7087, 7088, 7089, 7090, 7091, 7096, 7097, 7100, 7102, 7109, 7110, 7113, 7114, 7115, 7118, 7121, 7123, 7124, 7131, 7133, 7135, 7136, 7138, 7139, 7140, 7144, 7146, 7148, 7149, 7158, 7159, 7160, 7163, 7165, 7166, 7168, 7172, 7175, 7178, 7180, 7181, 7183, 7185, 7186, 7187, 7192, 7194, 7199, 7202, 7203, 7204, 7205, 7207, 7208, 7213, 7214, 7216, 7220, 7221, 7227, 7228, 7230, 7233, 7235, 7237, 7239, 7243, 7245, 7246, 7248, 7249, 7251, 7255, 7256, 7260, 7263, 7264, 7265, 7266, 7268, 7269, 7272, 7276, 7277, 7278, 7279, 7280, 7281, 7283, 7284, 7287, 7291, 7295, 7299, 7302, 7303, 7306, 7309, 7310, 7311, 7314, 7315, 7320, 7321, 7324, 7325, 7329, 7331, 7332, 7334, 7336, 7339, 7340, 7341, 7344, 7350, 7353, 7355, 7358, 7360, 7362, 7372, 7374, 7375, 7378, 7381, 7385, 7387, 7390, 7391, 7392, 7394, 7397, 7401, 7402, 7405, 7410, 7413, 7415, 7416, 7417, 7420, 6984, 6987, 6989, 6990, 6991, 6993, 6996, 6999, 7001, 7003, 7005, 7007, 7009, 7010, 7011, 7012, 7014, 7016, 7018, 7020, 7023, 7025, 7026, 7028, 7029, 7031, 7033, 7034, 7035, 7038, 7039, 7041, 7042, 7043, 7044, 7045, 7046, 7047, 7048, 7049, 7051, 7053, 7054, 7057, 7061, 7062, 7063, 7065, 7066, 7068, 7070, 7071, 7073, 7075, 7078, 7080, 7081, 7082, 7083, 7092, 7093, 7094, 7095, 7098, 7101, 7103, 7105, 7106, 7107, 7108, 7111, 7112, 7116, 7119, 7122, 7125, 7126, 7127, 7128, 7129, 7130, 7132, 7134, 7137, 7141, 7142, 7143, 7147, 7150, 7152, 7154, 7155, 7156, 7157, 7161, 7167, 7170, 7171, 7173, 7174, 7176, 7177, 7179, 7182, 7184, 7188, 7189, 7190, 7191, 7193, 7195, 7196, 7197, 7198, 7200, 7206, 7210, 7211, 7212, 7215, 7217, 7218, 7219, 7222, 7223, 7224, 7225, 7226, 7229, 7231, 7232, 7236, 7240, 7241, 7242, 7244, 7247, 7250, 7253, 7254, 7257, 7258, 7259, 7261, 7262, 7267, 7270, 7271, 7273, 7274, 7275, 7282, 7285, 7289, 7290, 7292, 7293, 7294, 7296, 7297, 7298, 7301, 7304, 7307, 7308, 7312, 7316, 7317, 7318, 7319, 7322, 7326, 7328, 7330, 7333, 7335, 7337, 7342, 7345, 7347, 7348, 7349, 7351, 7354, 7356, 7357, 7359, 7361, 7363, 7364, 7365, 7366, 7367, 7368, 7369, 7370, 7371, 7373, 7376, 7379, 7382, 7383, 7384, 7386, 7388, 7389, 7393, 7395, 7398, 7400, 7403, 7404, 7407, 7408, 7409, 7411, 7419, 7421, 7422, 7425, 7427, 7435, 7438, 7439, 7440, 7442, 7444, 7448, 7449, 7456, 7459, 7462, 7463, 7465, 7466, 7467, 7470, 7471, 7474, 7476, 7477, 7480, 7481, 7485, 7486, 7489, 7490, 7493, 7496, 7500, 7503, 7508, 7512, 7518, 7519, 7520, 7522, 7524, 7525, 7527, 7528, 7529, 7532, 7540, 7542, 7543, 7545, 7549, 7552, 7554, 7423, 7426, 7428, 7430, 7431, 7432, 7433, 7434, 7436, 7445, 7446, 7447, 7450, 7451, 7452, 7453, 7454, 7455, 7457, 7460, 7464, 7468, 7472, 7475, 7478, 7479, 7482, 7483, 7484, 7487, 7488, 7492, 7494, 7495, 7497, 7498, 7499, 7501, 7505, 7506, 7507, 7509, 7510, 7511, 7513, 7515, 7516, 7517, 7521, 7523, 7526, 7530, 7533, 7534, 7535, 7536, 7537, 7538, 7539, 7541, 7544, 7546, 7547, 7548, 7550, 7555, 7558, 7559, 7560, 7565, 7566, 7567, 7570, 7571, 7573, 7574, 7575, 7576, 7577, 7578, 7579, 7580, 7581, 7582, 7585, 7586, 7587, 7588, 7589, 7590, 7596, 7597, 7598, 7600, 7603, 7606, 7609, 7610, 7615, 7618, 7620, 7621, 7623, 7627, 7629, 7633, 7635, 7636, 7643, 7647, 7651, 7655, 7661, 7666, 7672, 7674, 7676, 7677, 7682, 7683, 7685, 7687, 7689, 7690, 7691, 7694, 7699, 7701, 7702, 7704, 7707, 7709, 7711, 7712, 7713, 7714, 7716, 7718, 7720, 7726, 7728, 7729, 7733, 7734, 7735, 7736, 7738, 7740, 7741, 7744, 7746, 7747, 7748, 7749, 7750, 7751, 7752, 7753, 7754, 7755, 7756, 7758, 7556, 7561, 7563, 7568, 7583, 7584, 7591, 7593, 7594, 7595, 7599, 7601, 7604, 7607, 7608, 7611, 7613, 7614, 7616, 7617, 7619, 7622, 7624, 7626, 7628, 7631, 7637, 7638, 7639, 7640, 7641, 7642, 7644, 7645, 7646, 7649, 7650, 7652, 7654, 7656, 7657, 7658, 7659, 7660, 7662, 7663, 7665, 7667, 7669, 7670, 7671, 7673, 7675, 7678, 7679, 7680, 7681, 7684, 7686, 7688, 7692, 7695, 7697, 7698, 7700, 7703, 7705, 7708, 7710, 7715, 7717, 7719, 7721, 7722, 7723, 7724, 7725, 7727, 7730, 7731, 7732, 7737, 7739, 7742, 7743, 7745, 7757, 7759, 7760, 7761, 7764, 7770, 7774, 7775, 7778, 7782, 7786, 7788, 7795, 7797, 7799, 7805, 7807, 7809, 7810, 7811, 7815, 7817, 7819, 7820, 7822, 7823, 7824, 7828, 7832, 7834, 7837, 7839, 7843, 7845, 7851, 7852, 7857, 7860, 7863, 7865, 7868, 7874, 7881, 7885, 7889, 7892, 7894, 7896, 7900, 7902, 7904, 7907, 7909, 7910, 7913, 7917, 7922, 7925, 7926, 7928, 7933, 7934, 7937, 7941, 7945, 7946, 7948, 7950, 7951, 7952, 7953, 7954, 7955, 7956, 7957, 7963, 7964, 7968, 7971, 7972, 7973, 7976, 7979, 7980, 7981, 7982, 7983, 7985, 7986, 7987, 7988, 7991, 7762, 7765, 7767, 7768, 7771, 7773, 7776, 7779, 7781, 7783, 7784, 7785, 7789, 7791, 7792, 7793, 7794, 7796, 7798, 7800, 7802, 7803, 7808, 7812, 7813, 7814, 7816, 7818, 7821, 7825, 7827, 7829, 7830, 7833, 7835, 7836, 7838, 7840, 7841, 7842, 7844, 7846, 7847, 7848, 7849, 7853, 7854, 7855, 7856, 7858, 7861, 7864, 7866, 7869, 7870, 7871, 7872, 7873, 7875, 7877, 7878, 7879, 7880, 7882, 7884, 7886, 7888, 7890, 7897, 7899, 7903, 7905, 7912, 7914, 7916, 7918, 7919, 7920, 7921, 7923, 7929, 7931, 7932, 7936, 7938, 7939, 7940, 7942, 7943, 7944, 7947, 7949, 7958, 7960, 7962, 7965, 7967, 7969, 7970, 7974, 7977, 7989, 7990, 7992, 7995, 7996, 8000, 8003, 8006, 8007, 8010, 8012, 8015, 8016, 8018, 8019, 8026, 8027, 8030, 8034, 8035, 8038, 8039, 8043, 8044, 8046, 8049, 8050, 8052, 8053, 8055, 8056, 8062, 8064, 8066, 8067, 8068, 8069, 8070, 8071, 8074, 8076, 8077, 8078, 8079, 8080, 8081, 7993, 7997, 7999, 8001, 8004, 8005, 8008, 8009, 8011, 8013, 8017, 8020, 8021, 8022, 8023, 8024, 8025, 8028, 8031, 8033, 8036, 8040, 8042, 8047, 8051, 8054, 8057, 8059, 8060, 8061, 8065, 8072, 8073, 8075, 8082, 8083, 8084, 8085, 8087, 8088, 8089, 8090, 8093, 8095, 8098, 8100, 8101, 8102, 8104, 8107, 8110, 8111, 8114, 8115, 8116, 8118, 8122, 8124, 8126, 8127, 8129, 8132, 8134, 8136, 8138, 8142, 8143, 8144, 8146, 8151, 8157, 8160, 8163, 8165, 8168, 8169, 8170, 8173, 8174, 8175, 8176, 8178, 8181, 8185, 8187, 8188, 8189, 8190, 8191, 8193, 8194, 8200, 8203, 8206, 8208, 8209, 8212, 8214, 8216, 8217, 8220, 8222, 8224, 8228, 8230, 8091, 8094, 8096, 8099, 8103, 8105, 8108, 8112, 8113, 8117, 8119, 8121, 8123, 8125, 8128, 8130, 8131, 8133, 8135, 8137, 8139, 8145, 8147, 8148, 8149, 8150, 8152, 8153, 8154, 8155, 8156, 8158, 8161, 8164, 8166, 8167, 8171, 8177, 8179, 8180, 8182, 8183, 8184, 8186, 8192, 8195, 8197, 8198, 8199, 8201, 8202, 8204, 8207, 8210, 8213, 8218, 8221, 8223, 8225, 8227, 8229, 8231, 8232, 8235, 8236, 8237, 8238, 8244, 8245, 8247, 8250, 8251, 8253, 8254, 8255, 8256, 8258, 8259, 8260, 8263, 8267, 8269, 8270, 8273, 8274, 8277, 8280, 8281, 8289, 8292, 8293, 8294, 8295, 8296, 8297, 8298, 8301, 8305, 8306, 8310, 8311, 8312, 8315, 8317, 8318, 8322, 8323, 8324, 8327, 8328, 8329, 8330, 8333, 8336, 8339, 8340, 8347, 8349, 8350, 8352, 8353, 8356, 8359, 8360, 8366, 8369, 8370, 8372, 8374, 8376, 8377, 8379, 8381, 8382, 8383, 8233, 8239, 8240, 8242, 8243, 8246, 8248, 8252, 8257, 8261, 8264, 8266, 8268, 8271, 8275, 8278, 8282, 8284, 8285, 8286, 8287, 8288, 8291, 8299, 8302, 8303, 8304, 8307, 8309, 8313, 8314, 8316, 8319, 8321, 8325, 8326, 8331, 8332, 8334, 8335, 8337, 8341, 8342, 8343, 8344, 8345, 8346, 8348, 8351, 8354, 8357, 8361, 8362, 8363, 8364, 8365, 8367, 8371, 8373, 8375, 8378, 8384, 8387, 8390, 8391, 8397, 8398, 8401, 8402, 8405, 8406, 8409, 8414, 8418, 8419, 8422, 8425, 8428, 8431, 8432, 8433, 8435, 8437, 8440, 8444, 8449, 8452, 8454, 8455, 8457, 8462, 8464, 8466, 8468, 8469, 8472, 8475, 8476, 8478, 8479, 8481, 8484, 8487, 8488, 8490, 8493, 8496, 8497, 8498, 8501, 8505, 8508, 8511, 8513, 8515, 8517, 8518, 8519, 8521, 8522, 8523, 8526, 8528, 8530, 8531, 8532, 8534, 8536, 8541, 8544, 8547, 8551, 8554, 8556, 8558, 8560, 8562, 8563, 8566, 8567, 8568, 8570, 8571, 8574, 8575, 8576, 8577, 8580, 8581, 8585, 8588, 8591, 8594, 8601, 8602, 8603, 8608, 8609, 8613, 8614, 8617, 8618, 8619, 8620, 8623, 8624, 8628, 8629, 8630, 8633, 8634, 8635, 8636, 8638, 8643, 8645, 8648, 8654, 8657, 8658, 8659, 8660, 8661, 8662, 8666, 8670, 8672, 8674, 8676, 8681, 8683, 8686, 8688, 8689, 8691, 8692, 8693, 8695, 8696, 8700, 8701, 8702, 8703, 8704, 8705, 8709, 8716, 8717, 8719, 8722, 8725, 8727, 8728, 8729, 8732, 8733, 8734, 8735, 8736, 8385, 8388, 8392, 8394, 8395, 8399, 8403, 8407, 8410, 8411, 8412, 8413, 8415, 8416, 8420, 8423, 8424, 8426, 8429, 8434, 8436, 8438, 8442, 8445, 8446, 8447, 8448, 8450, 8451, 8456, 8458, 8460, 8461, 8463, 8465, 8467, 8470, 8473, 8474, 8477, 8480, 8482, 8485, 8486, 8489, 8491, 8492, 8494, 8499, 8500, 8502, 8503, 8504, 8506, 8510, 8512, 8514, 8516, 8520, 8524, 8527, 8529, 8533, 8535, 8537, 8539, 8542, 8545, 8546, 8548, 8550, 8552, 8555, 8557, 8564, 8565, 8569, 8572, 8578, 8582, 8583, 8586, 8587, 8589, 8592, 8595, 8596, 8597, 8598, 8599, 8600, 8604, 8606, 8607, 8610, 8612, 8615, 8616, 8621, 8625, 8627, 8631, 8632, 8637, 8639, 8640, 8641, 8642, 8644, 8646, 8647, 8649, 8650, 8651, 8652, 8653, 8655, 8663, 8665, 8667, 8668, 8669, 8671, 8673, 8675, 8677, 8678, 8679, 8680, 8682, 8684, 8685, 8687, 8690, 8694, 8697, 8698, 8699, 8706, 8707, 8708, 8710, 8711, 8712, 8713, 8714, 8715, 8718, 8720, 8723, 8726, 8730, 8737, 8738, 8741, 8744, 8747, 8750, 8751, 8753, 8754, 8755, 8756, 8759, 8760, 8762, 8763, 8766, 8767, 8768, 8769, 8770, 8772, 8775, 8776, 8777, 8779, 8782, 8783, 8784, 8785, 8786, 8787, 8788, 8789, 8790, 8793, 8794, 8796, 8798, 8799, 8801, 8803, 8804, 8806, 8807, 8809, 8812, 8813, 8815, 8820, 8823, 8826, 8829, 8830, 8832, 8835, 8836, 8837, 8842, 8845, 8846, 8847, 8851, 8853, 8856, 8857, 8859, 8861, 8866, 8739, 8878, 8879, 8880, 8884, 8891, 8893, 8895, 8898, 8900, 8901, 8909, 8912, 8739, 8742, 8745, 8748, 8749, 8752, 8757, 8758, 8761, 8764, 8771, 8773, 8778, 8780, 8791, 8797, 8800, 8802, 8805, 8808, 8810, 8814, 8816, 8818, 8819, 8821, 8824, 8827, 8828, 8831, 8833, 8838, 8840, 8841, 8755, 8843, 8844, 8848, 8850, 8852, 8854, 8855, 8863, 8864, 8865, 8867, 8868, 8869, 8870, 8871, 8872, 8873, 8874, 8876, 8877, 8881, 8882, 8883, 8885, 8887, 8888, 8889, 8890, 8892, 8894, 8896, 8897, 8899, 8902, 8785, 8903, 8904, 8905, 8906, 8907, 8908, 8911, 8913, 8917, 8920, 8923, 8924, 8929, 8931, 8934, 8935, 8936, 8914, 8916, 8918, 8921, 8925, 8927, 8928, 8930, 8932, 8933, 8937, 8980, 8984, 8988, 8989, 8998, 9001, 9002, 9005, 9007, 9008, 9012, 9013, 9016, 9019, 9020, 9022, 9023, 9025, 9028, 9029, 9032, 9033, 9034, 9035, 9037, 9038, 9044, 9046, 9047, 9048, 9049, 9054, 8981, 8983, 8985, 8987, 8990, 8992, 8993, 8994, 8995, 8997, 8999, 9003, 9004, 9006, 9009, 9010, 9011, 9014, 9017, 9018, 9021, 9024, 9026, 9030, 9036, 9039, 9041, 9042, 9043, 9045, 9050, 9051, 9052, 9053, 9055, 9058, 9060, 9063, 9066, 9067, 9068, 9069, 9070, 9071, 9074, 9075, 9077, 9078, 9079, 9080, 9082, 9084, 9088, 9089, 9090, 9099, 9103, 9104, 9105, 9107, 9111, 9056, 9061, 9064, 9072, 9081, 9083, 9085, 9086, 9087, 9091, 9092, 9093, 9094, 9095, 9096, 9097, 9098, 9100, 9102, 9106, 9108, 9109, 9110, 9112, 9113, 9116, 9118, 9125, 9126, 9128, 9129, 9130, 9136, 9141, 9143, 9148, 9150, 9153, 9157, 9158, 9159, 9163, 9164, 9165, 9167, 9168, 9171, 9173, 9177, 9180, 9181, 9182, 9183, 9185, 9187, 9188, 9189, 9191, 9192, 9193, 9194, 9197, 9198, 9200, 9206, 9207, 9208, 9209, 9211, 9216, 9218, 9219, 9220, 9221, 9222, 9223, 9226, 9228, 9229, 9230, 9231, 9232, 9233, 9234, 9237, 9238, 9240, 9243, 9246, 9248, 9253, 9256, 9257, 9260, 9263, 9264, 9266, 9269, 9271, 9279, 9280, 9281, 9282, 9283, 9284, 9290, 9114, 9119, 9121, 9122, 9123, 9124, 9127, 9131, 9133, 9134, 9135, 9137, 9138, 9139, 9140, 9142, 9144, 9146, 9147, 9149, 9151, 9152, 9154, 9155, 9156, 9160, 9162, 9166, 9169, 9172, 9174, 9175, 9176, 9178, 9184, 9186, 9190, 9195, 9199, 9201, 9202, 9203, 9204, 9205, 9212, 9214, 9215, 9217, 9224, 9227, 9235, 9239, 9241, 9244, 9247, 9249, 9251, 9252, 9254, 9258, 9261, 9265, 9267, 9270, 9273, 9274, 9275, 9276, 9277, 9278, 9285, 9286, 9287, 9288, 9289, 9291, 9292, 9295, 9297, 9300, 9302, 9303, 9304, 9307, 9308, 9310, 9311, 9313, 9319, 9321, 9323, 9324, 9328, 9333, 9334, 9337, 9338, 9339, 9342, 9344, 9348, 9351, 9352, 9354, 9355, 9357, 9360, 9367, 9371, 9374, 9377, 9379, 9383, 9384, 9385, 9388, 9389, 9390, 9393, 9394, 9398, 9401, 9402, 9406, 9408, 9409, 9410, 9413, 9415, 9416, 9417, 9418, 9419, 9421, 9422, 9425, 9429, 9433, 9434, 9435, 9436, 9437, 9439, 9440, 9446, 9452, 9453, 9454, 9455, 9457, 9458, 9459, 9462, 9466, 9467, 9468, 9470, 9474, 9479, 9482, 9487, 9488, 9489, 9492, 9495, 9497, 9499, 9500, 9504, 9505, 9508, 9513, 9514, 9519, 9522, 9523, 9524, 9526, 9528, 9532, 9533, 9534, 9535, 9537, 9538, 9541, 9542, 9543, 9545, 9546, 9548, 9293, 9296, 9298, 9301, 9305, 9309, 9312, 9314, 9316, 9317, 9318, 9320, 9322, 9325, 9326, 9327, 9329, 9331, 9332, 9335, 9336, 9340, 9345, 9347, 9349, 9353, 9356, 9358, 9361, 9363, 9364, 9365, 9368, 9370, 9372, 9375, 9376, 9378, 9380, 9382, 9386, 9391, 9395, 9397, 9399, 9400, 9403, 9405, 9407, 9411, 9412, 9414, 9420, 9423, 9424, 9426, 9428, 9430, 9431, 9432, 9438, 9441, 9443, 9444, 9445, 9447, 9448, 9449, 9450, 9451, 9456, 9460, 9461, 9464, 9465, 9469, 9471, 9473, 9475, 9477, 9478, 9480, 9483, 9485, 9490, 9493, 9494, 9496, 9498, 9501, 9502, 9503, 9506, 9509, 9511, 9512, 9515, 9516, 9517, 9518, 9520, 9521, 9525, 9527, 9529, 9530, 9536, 9539, 9544, 9547, 9549, 9550, 9551, 9555, 9556, 9557, 9559, 9560, 9563, 9564, 9565, 9566, 9569, 9570, 9573, 9577, 9578, 9579, 9580, 9585, 9586, 9587, 9588, 9591, 9593, 9596, 9552, 9554, 9558, 9561, 9567, 9571, 9574, 9576, 9581, 9583, 9584, 9589, 9592, 9594, 9595, 9598, 9602, 9604, 9605, 9608, 9612, 9613, 9614, 9617, 9618, 9621, 9622, 9625, 9626, 9627, 9632, 9633, 9638, 9640, 9643, 9644, 9645, 9646, 9651, 9654, 9656, 9657, 9658, 9661, 9662, 9665, 9666, 9667, 9668, 9672, 9673, 9675, 9676, 9677, 9680, 9683, 9686, 9688, 9691, 9694, 9698, 9699, 9702, 9705, 9707, 9710, 9711, 9715, 9719, 9720, 9722, 9723, 9726, 9729, 9732, 9733, 9735, 9737, 9738, 9741, 9744, 9745, 9746, 9747, 9748, 9749, 9750, 9754, 9755, 9756, 9758, 9759, 9768, 9771, 9772, 9776, 9777, 9781, 9782, 9784, 9787, 9789, 9790, 9792, 9796, 9797, 9800, 9805, 9806, 9812, 9815, 9818, 9821, 9823, 9825, 9827, 9831, 9832, 9835, 9837, 9839, 9841, 9842, 9844, 9845, 9851, 9853, 9854, 9855, 9857, 9860, 9862, 9863, 9865, 9871, 9873, 9875, 9878, 9879, 9599, 9601, 9603, 9606, 9607, 9609, 9611, 9615, 9619, 9623, 9624, 9628, 9630, 9631, 9634, 9635, 9637, 9639, 9641, 9647, 9649, 9650, 9652, 9653, 9659, 9660, 9663, 9664, 9670, 9671, 9679, 9682, 9684, 9687, 9690, 9692, 9693, 9695, 9697, 9700, 9701, 9703, 9706, 9708, 9712, 9713, 9714, 9716, 9717, 9718, 9721, 9724, 9727, 9730, 9734, 9739, 9742, 9751, 9753, 9757, 9760, 9761, 9762, 9763, 9764, 9765, 9766, 9767, 9769, 9773, 9775, 9778, 9780, 9783, 9786, 9788, 9791, 9793, 9794, 9795, 9798, 9801, 9803, 9804, 9807, 9808, 9809, 9810, 9811, 9813, 9816, 9817, 9819, 9820, 9822, 9826, 9828, 9830, 9833, 9836, 9838, 9840, 9843, 9846, 9847, 9848, 9849, 9850, 9852, 9856, 9858, 9859, 9861, 9864, 9866, 9867, 9868, 9869, 9870, 9872, 9876, 9877, 9880, 9881, 9882, 9886, 9890, 9891, 9894, 9900, 9902, 9904, 9908, 9911, 9912, 9915, 9917, 9919, 9923, 9925, 9928, 9935, 9936, 9940, 9943, 9949, 9953, 9954, 9956, 9957, 9958, 9962, 9963, 9964, 9968, 9969, 9975, 9978, 9981, 9988, 9989, 9990, 9991, 9992, 9995, 9997, 10011, 10012, 10014, 10017, 10018, 10031, 10032, 10033, 10034, 10035, 10037, 10041, 10042, 10046, 10050, 10054, 10058, 10059, 9883, 9885, 9887, 9889, 9892, 9893, 9895, 9896, 9897, 9898, 9899, 9901, 9903, 9905, 9907, 9909, 9913, 9914, 9916, 9918, 9920, 9921, 9922, 9924, 9926, 9929, 9931, 9932, 9933, 9934, 9937, 9938, 9939, 9941, 9942, 9944, 9945, 9946, 9947, 9948, 9950, 9952, 9955, 9959, 9960, 9961, 9966, 9967, 9970, 9972, 9973, 9974, 9976, 9979, 9980, 9982, 9983, 9984, 9985, 9986, 9987, 9993, 9994, 9996; 9998, 9999, 10000, 10001, 10002, 10003, 10004, 10005, 10006, 10007, 10008, 10009, 10010, 10013, 10015, 10016, 10019, 10020, 10021, 10022, 10023, 10025, 10026, 10027, 10028, 10030, 10036, 10038, 10040, 10043, 10044, 10045, 10047, 10048, 10049, 10051, 10053, 10055, 10056, 10057, 10060, 10061, 10064, 10067, 10068, 10070, 10071, 10072, 10074, 10079,10081,10083,10088,10091,10095,10096,10097,10101,10103,10105,10106,10109, 10110, 10111, 10113, 10116, 10118, 10120, 10122, 10125, 10126, 10127, 10131, 10134, 10137, 10138, 10144, 10147, 10149, 10151, 10152, 10155, 10156, 10159, 10160, 10169, 10172, 10174, 10175, 10178, 10181, 10186, 10188, 10189, 10190, 10191, 10192, 10196, 10199, 10203, 10204, 10210, 10211, 10216, 10218, 10221, 10224, 10225, 10226, 10229, 10230, 10231, 10232, 10234, 10239, 10241, 10244, 10252, 10253, 10257, 10258, 10259, 10261, 10266, 10272, 10276, 10062, 10065, 10069, 10075, 10077, 10078, 10080, 10082, 10084, 10086, 10087, 10089, 10090, 10092, 10093,10094,10098,10099,10100, 10102,10104,10107,10112,10114,10117,10119,10121, 10123, 10124, 10129, 10130, 10132, 10133, 10135, 10139, 10140, 10141, 10142, 10143, 10145, 10148,10150,10153,10154,10157,10161,10163,10164,10165,10166,10167, 10170,10173, 10176, 10177, 10179, 10182, 10183, 10184, 10187, 10193, 10194, 10195, 10197, 10198, 10200, 10201, 10202, 10205, 10206, 10207, 10209, 10212, 10213, 10214, 10215, 10219, 10220, 10222, 10223, 10227, 10228, 10233, 10235, 10236, 10237, 10240, 10242, 10243, 10245, 10247, 10248, 10249, 10251, 10254, 10255, 10256, 10260, 10262, 10263, 10264, 10265, 10267, 10268, 10269, 10270, 10271, 10273, 10274, 10275, 10277, 10278, 10279, 10282, 10285, 10287, 10291, 10292, 10297, 10299, 10301, 10304, 10306, 10308, 10310, 10314, 10317, 10318, 10320, 10322, 10327, 10329, 10330, 10333, 10335, 10336, 10337, 10340, 10342, 10348, 10350, 10351, 10354, 10358, 10359, 10360, 10362, 10366, 10369, 10372, 10373, 10374, 10376, 10378, 10383, 10385, 10391, 10398, 10403, 10407, 10410, 10413, 10415, 10419, 10422, 10425, 10427, 10428, 10429, 10430, 10432, 10433, 10434, 10437, 10438, 10439, 10442, 10443, 10445, 10446, 10447, 10448, 10452, 10453, 10454, 10455, 10456, 10460, 10463, 10466, 10467, 10469, 10470, 10473, 10475, 10477, 10480, 10484, 10488, 10490, 10494, 10495, 10497, 10501, 10502, 10503, 10507, 10508, 10510, 10517, 10518, 10520, 10522, 10524, 10525, 10528, 10529, 10533, 10536, 10537, 10540, 10541, 10545, 10547, 10551, 10553, 10556, 10559, 10560, 10563, 10564, 10565, 10568, 10571, 10574, 10576, 10579, 10581, 10583, 10588, 10589, 10590, 10592, 10593, 10596, 10597, 10601, 10602, 10603, 10605, 10607, 10609, 10610, 10612, 10613, 10617, 10618, 10621, 10624, 10627, 10630, 10632, 10634, 10637, 10638, 10639, 10640, 10641, 10643, 10645, 10647, 10653, 10655, 10657, 10659, 10661, 10662, 10663, 10664, 10665, 10666, 10667, 10668, 10669, 10672, 10673, 10674, 10675, 10280, 10283, 10286, 10288, 10290, 10293, 10295, 10298, 10300, 10302, 10305, 10307, 10309, 10311, 10312, 10313, 10315, 10319, 10321, 10323, 10325, 10326, 10328, 10331, 10338, 10341, 10343, 10344, 10345, 10346, 10347, 10349, T0352, I0355, 10356, 10357, 10361, 10363, 10365, 10367, 10370, 10375, 10377, 10379, 10380, 10381, 10382, 10384, 10386, 10387, 10388, 10390, 10392, 10394, 10395, 10399, 10401, 10402, 10404, 10406, 10408, 10411, 10412, 10414, 10416, 10417, 10418, 10420, 10423, 10426, 10431, 10435, 10440, 10444, 10449, 10451, 10457, 10459, 10461, 10462, 10464, 10465, 10468, 10471, 10476, 10478, 10481, 10483, 10485, 10487, 10489,10491, 10492, 10493, 10498, 10499, 0500, 0504, 0506,10509,10511,10513, 0514, 10515,10516,10519,10521,10523,10526,10530,10531, 0532, 0534,10535,10538, 10539, 10542, 0543, 0544, 0546, 0548, 0550, 0552, 0554, 0557, 0558, 0561, 0566, 0569, 10572, 0573, 0577, 0578, 0580, 0582, 0584, 0586, 0594, 0598, 10600, 10604, 10606, 10611, 10614, 10616, 10619, 10622, 10625, 10626, 10628, 10631, 10633, 10635, 10642, 10644, 10646, 10648, 10649, 10650, 10651, 10652, 10654, 10656, 10658, 10660, 10670, 10671, 10676, 10678, 10684, 10679, 10681, 10682, 10683, 10685, 10687, 10688, 10689, 10690, 10691, 10693, 10696, 10699, 10703, 10704, 10709, 10711, 10712, 10713, 10720, 10721, 10722, 10724, 10725, 10726, 10728, 10729, 10734, 10740, 10742, 10743, 10744, 10745, 10746, 10748, 10?49, 10751, 10754, 10758, 10759, 10760, 10764, 10768, 10769, 10772, 10775, 10780, 10781, 10783, 10786, 10791, 10796, 10801, 10808, 10817, 10818, 10819, 10821, 10823, 10826, 10827, 10829, 10830, 10834, 10843, 10844, 10847, 10848, 10849, 10853, 10856, 10858, 10866, 10869, 10872, 10874, 10877, 10878, 10879, 10882, 10884, 10885, 10889, 10892, 10893, 10896, 10900, 10901, 10903, 10905, 10907, 10909, 10910, 10911, 10914, 10916, 10919, 10926, 10930, 10932, 10933, 10934, 10938, 10941, 10942, 10943, 10944, 10946, 10947, 10953, 109SS, 10956, 10958, 10959, 10962, 10967, 10968, 10972, 10975, 10977, 10978, 10981, 10984, 10985, 10988, 10992, 10995, 10997, 10999, 11000, 11001, 11006, 11009, 11013, 11016, 11020, 11025, 11033, 11036, 11040, 11041, 11042, 11043, 11045, 11046, 11048, 11052, 110SS, 11060, 11061, 11062, 11064, 11065, 11067, 11070, 11071, 11072, 11078, 11079, 11081, 11085, 11093, 10694, 10697, 10700, 10702, 10705, 10707, 10710, 10714, 10716, 10717, 10718, 10719, 10723, 10727, 10730, 10732, 10733, 10735, 10737, 10738, 10739, 10741, 10747, 10750, 10752, 107SS, 10757, 10761, 10762, 10763, 10765, 10767, 10770, 10773, 10776, 10778, 10779, 10782, 10784, 10788, 10789, 10790, 10792, 10793, 10794, 10798, 10799, 10800, 10802, 10804, 10805, 10806, 10807, 10809, 10811, 10812, 10813, 10814, 10815, 10816, 10820, 10822, 10824, 10831, 10833, 10835, 10837, 10838, 10839, 10840, 10841, 10842, 10845, 10846, 10850, 10851, 10854, 10857, 10859, 10860, 10861, 10862, 10863, 10864, 10865, 10868, 10870, 10871, 10873, 10875, 10880, 10881, 10883, 10886, 10888, 10890, 10894, 10897, 10899, 10902, 10904, 10906, 10908, 10912, 1091 S, 10917, 10920, 10922, 10924, 10925, 10927, 10928, 10929, 10931, 10935, 10937, 10939, 10945, 10948, 10950, 10951, 10952, 10954, 10957, 10960, 10963, 10965, 10966, 10969, 10970, 10971, 10973, 10976, 10979, 10980, 10982, 10986, 10987, 10989, 10991, 10993, 10996, 10998, 11002, 11004, 11005, 11007, 11010, 11012, 11014, 11015, 11017, 11019, 11021, 11022, 11023, 11026, 11027, 11028, 11029, 11030, 11031, 11032, 11034, 11037, 11039, 11044, 11047, 11049, 11050, 11051, 11053, 11054, 11056, 11058, 11059, 11063, 11066, 11068, 11069, 11073, 11074, 11075, 11076, 11077, 11080, 11082, 11083, 11084, 11086, 11087, 11088, 11089, 11090, 11091, 11092, 11094, 11376, 11381, 11382, 11383, 11384, 11387, 11397, 11401, 11405, 11408, 11414, 11418, 11421, 11422, 11424, 11427, 11429, 11430, 11377, 11379, 11380, 11385, 11386, 11388, 11390, 11391, 11392, 11394, 11395, 11396, 11398, 11399, 11400, 11402, 11403, 11404, 11406, 11409, 11410, 11411, 11412, 11415, 11416, 11417, 11419, 11423, 11425, 11428, 11431, 11432, 11433, 11434, 11438, 11442, 11443, 11447, 11449, 11450, 11452, 11456, 11459, 11463, 11464, 11467, 11468, 11472, 11477, 11480, 11481, 11482, 11483, 11484, 11488, 11489, 11493, 11498, 11502, 11504, 11505, 11506, 11507, 11508, 11509, 11512, 11513, 11514, 11517, 11520, 11522, 11523, 11524, 11526, 11528, 11532, 11534,11435,11439,11444,11445,11446,11448, 11451,11453,11457,11458,11460,11461, 11462, 11465, 11466, 11469, 11473, 11474, 11475, 11476, 11478, 11479, 11485, 11486, 11487, 11490, 11494, 11497, 11499, 11500, 11501, 11503, 11510, 11511, 11515, 11518, 11519, 11521, 11525, 11527, 11529, 11535, 11539, 11540, 11541, 11548, 11550, 11551, 11552, 11555, 11556, 11557, 11560, 11561, 11562, 11568, 11569, 11571, 11572, 11573, 11577, 11536, 11542, 11545, 11546, 11547, 11549, 11553, 11554, 11563, 11566, 11570, 11574, 11578, 11585, 11586, 11590, 11591, 11592, 11595, 11596, 11597, 11600, 11579, 11582, 11587, 11593, 11594, 11598, 11601, 11602, 11606, 11607, 11608, 11609, 11616, 11618, 11622, 11626, 11628, 11632, 11633, 11603, 11610, 11613, 11614, 11615, 11617, 11619, 11623, 11627, 11629, 11634, 11635, 11640, 11641, 11642, 11643, 11647, 11648, 11654, 11655, 11656, 11657, 11658, 11636, 11639, 11644, 11645, 11646, 11649, 11652, 11653, 11659, 11663, 11664, 11665, 11666, 11670, 11671, 11672, 11673, 11677, 11681, 11687, 11690, 11694, 11696, 11697, 11701, 11705, 11709, 11713, 11719, 11723, 11729, 11733, 11739, 11744, 11745, 11748, 11749, 11751, 11755, 11759, 11763, 11770, 11772, 11774, 11776, 11778, 11782, 11785, 11660, 11667, 11674, 11675, 11676, 11678, 11682, 11685, 11686, 11688, 11689, 11691, 11692, 11693, 11695, 11698, 11699, 11700, 11702, 11706, 11710, 11716, 11720, 11724, 11725, 11727, 11728, 11730, 11731, 11732, 11734, 11735, 11736, 11737, 11738, 11740, 11743, 11746, 11747, 11750, 11752, 11756, 11761, 11762, 11764, 11765, 11767, 11768, 11769, 11773, 11775, 11777, 11779, 11783, 11784, 11786, 11790, 11791, 11792, 11787, 11793, 11796, 11800, 11802, 11803, 11805, 11810, 11818, 11819, 11823, 11824, 11827, 11828, 11829, 11833, 11836, 11837, 11839, 11840, 11847, 11850, 11852, 11857, 11860, 11864, 11866, 11797, 11801, 11804, 11806, 11809, 11811, 11814, 11815, 11817, 11820, 11825, 11830, 11835, 11841, 11844, 11845, 11846, 11848, 11851, 11853, 11856, 11858, 11861, 11862, 11863, 11865, 11867, 11871, 11876, 11878, 11882, 11888, 11891, 11894, 11868, 11872, 11875, 11877, 11879, 11883, 11886, 11887, 11889, 11890, 11892, 11893, 11895, 11898, 11902, 11903, 11904, 11908, 11909, 11911, 11912, 11913, 11914, 11915, 11917, 11918, 11922, 11926, 11927, 11930, 11932, 11933, 11937, 11941, 11942, 11946, 11947, 11948, 11949, 11950, 11951, 11952, 11955, 11959, 11968, 11972, 11976, 11978, 11979, 11980, 11981, 11986, 11988, 11989, 11991, 11992, 11995, 11997, 11998, 12001, 12002, 12004, 12005, 11899, 11905, 11910, 11916, 11919, 11923, 11924, 11925, 11928, 11929, 11931, 11934, 11938, 11943, 11953, 11954, 11956, 11960, 11963, 11964, 11967, 11969, 11973, 11977, 1982, 11985, 11987, 11990, 11993, 11994, 11996, 11999, 12000, 12003, 12006, 12011, 12015, 12016, 12017, 12021, 12022, 12023, 12024, 12025, 12026, 12027, 12031, 12034, 12042, 12043, 12045, 12049, 12050, 12051, 12054, 12062, 12063, 12064, 12066, 12067, 12068, 12070, 12072, 12076, 12082, 12085, 12086, 12089, 12090, 12093, 12094, 12095, 12096, 12097, 12098, 12101, 12103, 12107, 12108, 12111, 12112, 12114, 12118, 12119, 12120, 12128, 12007, 12010, 12012, 12020, 12028, 12032, 12033, 12035, 12038, 12039, 12044, 12046, 12052, 12055, 12056, 12057, 12058, 12059, 12060, 12061, 12065, 12069, 12071, 12073, 12077, 12080, 12081, 12084, 12087, 12088, 12091, 12099, 12100, 12102, 12104, 12109, 12110, 12113, 1211 S, 12121, 12122, 12123, 12124, 12125, 12127, 12129, 12130, 12135, 12136, 12141, 12144, 12147, 12148, 12149, 12153, 12159, 12160, 12131, 12134, 12137, 12138, 12139, 12140, 12142, 12143, 12145, 12146, 12150, 12154, 12157, 12158, 12161, 12162, 12163, 12164, 12168, 12171, 12176, 12181, 12187, 12190, 12194, 12196, 12197, 12199, 12201, 12202, 12209, 12213, 12216, 12220, 1222T, 12223, 12225, 12226, 12231, 12233, 12239, 12243, 12244, 12250, 12254, 12256, 12257, 12259, 12266, 12270, 12271, 12272, 12274, 12276, 12277, 12278, 12280, 12284, 12285, 12288, 12289, 12290, 12291, 12295, 12296, 12302, 12308, 12313, 12317, 12321, 12322, 12323, 12324, 12325, 12327, 12328, 12330, 12331, 12332, 12336, 12337, 12339, 12343, 12346, 12349, 12353, 12354, 12355, 12359, 12361, 12363, 12367, 12369, 12370, 12372, 12376, 12377, 12379, 12380, 12381, 12382, 12383, 12385, 12389, 12393, 12398, 12400, 12402, 12403, 12406, 12408, 12409, 12410, 12412, 12415, 12417, 12419, 12422, 12424, 12429, 12430, 12432, 12433, 12435, 12436, 12437, 12441, 12445, 12448, 12449, 12454, 12458, 12459, 12460, 12165, 12169, 12170, 12172, 12175, 12177, 12180, 12182, 12185, 12186, 12188, 12191, 12195, 12198, 12200, 12203, 12206, 12207, 12208, 12210, 12214, 12217, 12222, 12224, 12227, 12228, 12229, 12230, 12232, 12234, 12237, 12238, 12240, 12241, 12242, 12245, 12248, 12249, 12251, 12255, 12258, 12260, 12262, 12263, 12264, 12265, 12267, 12268, 12269, 12273, 12279, 12281, 12286, 12287, 12292, 12297, 12300, 12303, 12306, 12307, 12309, 12312, 12314, 12318, 12326, 12333, 12338, 12340, 12341, 12342, 12345, 12347, 12348, 12352, 12356, 12360, 12362, 12364, 12371, 12373, 123?8, 12386, 12390, 12394, 12397, 12401, 12404, 12405, 12411, 12413, 12418, 12420, 12423, 12425, 12428, 12438, 12439, 12440, 12442, 12443, 12446, 12450, 12453, 12455, 12461, 12465, 12467, 12468, 12472, 12474, 12477, 12480, 12484, 12486, 12488, 12489, 12490, 12493, 12495, 12499, 12501, 12504, 12506, 12462, 12469, 12475, 12478, 12481, 12483, 12485, 12487, 12492, 12494, 12496, 12497, 12498, 12500, 12502, 12505, 12507, 12511, 12517, 12520, 12522, 12523, 12524, 12528, 12530, 12534, 12535, 12536, 12537, 12541, 12542, 12547, 12550, 12551, 12552, 12554, 12555, 12560, 12564, 12565, 12569, 12571, 12574, 12579, 12581, 12586, 12590, 12592, 12593, 12594, 12596, 12600, 12601, 12602, 12604, 12610, 12613, 12614, 12615, 12616, 12620, 12626, 12630, 12508, 12512, 12515, 12518, 12521, 12525, 12529, 12531, 12538, 12543, 12546, 12548, 12549, 12553, 12556, 12559, 12561, 12566, 12570, 12572, 12573, 12575, 12578, 12582, 12585, 12587, 12595, 12597, 12599, 12603, 12605, 12608, 12609, 12611, 12617, 12618, 12621, 12624, 12625, 12627, 12631, 12636, 12637, 12638, 12639, 12643, 12647, 12648, 12652, 12654, 12660, 12664, 12666, 12670, 12672, 12675, 12677, 12680, 12686, 12691, 12692, 12695, 12699, 12700, 12701, 12703, 12705, 12709, 12710, 12714, 12715, 12716, 12718, 12719, 12720, 12724, 12725, 12728, 12732, 12738, 12739, 12743, 12744, 12745, 12747, 12748, 12752, 12757, 12763, 12764, 12766, 12767, 12768, 12771, 12776, 12777, 12781, 12785, 12787, 12788, 12791, 12792, 12796, 12797, 12800, 12804, 12808, 12813, 12815, 12816, 12817, 12822, 12825, 12828, 12831, 12833, 12834, 12837, 12839, 12841, 12842, 12632, 12635, 12640, 12641, 12644, 12649, 12653, 12655, 12658, 12661, 12662, 12663, 12665, 12667, 12671, 12673, 12678, 12679, 12681, 12682, 12683, 12684, 12685, 12687, 12688, 12689, 12690, 12693, 12694, 12696, 12702, 12704, 12706, 12712, 12713, 12717, 12721, 12726, 12729, 12730, 12731, 12733, 12736, 12737, 12740, 12746, 12749, 12750, 12751, 12753, 12754, 127SS, 12756, 12758, 12760, 12761, 12762, 12765, 12769, 12770, 12772, 12775, 12778, 12782, 12786, 12789, 12790, 12793, 12794, 12795, 12798, 12799, 12801, 12805, 12809, 12812, 12814, 12818, 12821, 12823, 12824, 12826, 12827, 12832, 12835, 12836, 12838, 12840, 12843, 12844, 12845, 12849, 12850, 12854, 12857, 12862, 12866, 12867, 12868, 12870, 12872, 12880, 12881, 12884, 12885, 12889, 12890, 12892, 12896, 12898, 12899, 12903, 12907, 12910, 12913, 12919, 12920, 12921, 12923, 12928, 12932, 12938, 12942, 12945, 12948, 12950, 12951, 12953, 12959, 12846, 12851, 12855, 12858, 12861, 12863, 12869, 12871, 12873, 12876, 12877, 12878, 12879, 12882, 12883, 12886, 12887, 12888, 12891, 12893, 12894, 12895, 12900, 12901, 12902, 12904, 12908, 12909, 12911, 12912, 12914, 12917, 12918, 12924, 12927, 12929, 12933, 12936, 12937, 12939, 12943, 12949, 12954, 12957, 12958, 12960, 12961, 12965, 12969, 12975, 12977, 12962, 12966, 12967, 12968, 12970, 12971, 12972, 12973, 12974, 12976, 12978, 12981, 12982, 12986, 12989, 12991, 12992, 12993, 12995, 12999, 13001, 12983, 12987, 12996, 12997, 12998, 13000, 13002, 13005, 13009, 13013, 13017, 13019, 13025, 13029, 13030, 13032, 13033, 13034, 13037, 13040, 13042, 13043, 13044, 13048, 13049, 13051, 13052, 13053, 13057, 13062, 13064, 13065, 13066, 13067, 13072, 1307.3., 13076, 13078, 13082, 13083, 13085, 13087, 13088, 13089, 13092, 13096, 13100, 13101, 13102, 13105, 13108, 13109, 13110, 13114, 13117, 13118, 13124, 13129, 13131, 13132, 13138, 13144, 13146, 13147, 13148, 13152, 13156, 13159, 13164, 13167, 13168, 13170, 13171, 13172, 13173, 13174, 13184, 13185, 13006, 13010, 13014, 13018, 13020, 13021, 13022, 13023, 13024, 13026, 13031, 13035, 13036, 13038, 13039, 13041, 13045, 13050, 13054, 13058, 13061, 13063, 13068, 13069, 13070, 13071, 13074, 13075, 13077, 13079, 13084, 13086, 13090, 13091, 13093, 13097, 13104, 13107, 13111, 13115, 13119, 13121, 13122, 13123, 13125, 13126, 13127, 13128, 13130, 13133, 13137, 13139, 13142, 13143, 13145, 13149, 13153, 131 S7, 13158, 13160, 13163, 13165, 13166, 13169, 13175, 13176, 13177, 13178, 13179, 13180, 13181, 13182, 13183, 13186, 13189, 13190, 13191, 13195, 13196, 13197, 13199, 13200, 13204, 13206, 13192, 13198, 13201, 13207, 13212, 13213, 13226, 13228, 13231, 13235, 13240, 13245, 13247, 13248, 13250, 13254, 13256, 13258, 13264, 13265, 13268, 13269, 13273, 13274, 13277, 13279, 13280, 13281, 13286, 13208, 13211, 13214, 13217, 13220, 13222, 13224, 13225, 13227, 13229, 13232, 13233, 13234, 13237, 13239, 13241, 13242, 13244, 13246, 13249, 13251, 13255, 13257, 13259, 13262, 13263, 13266, 13267, 13270, 13275, 13276, 13282, 13285, 13641, 13645, 13646, 13649, 13650, 13652, 13653, 13654, 13655, 13658, 13659, 13642, 13647, 13648, 13656, 13660, 13661, 13664, 13669, 13670, 13671, 13672, 13673, 13675, 13676, 13677, 13681, 13683, 13685, 13687, 13689, 13691, 13693, 13696, 13665, 13668, 13674, 13678, 13682, 13684, 13686, 13688, 13690, 13692, 13694, 13695, 13697, 13701, 13702, 13703, 13709, 13713, 13714, 13715, 13719, 13721, 13722, 13726, 13728, 13732, 13735, 13740, 13698, 13704, 13705, 13706, 13707, 13708, 13710, 13716, 13717, 13718, 13720, 13723, 13724, 13725, 13727, 13729, 13733, 13734, 13736, 13739, 13741, 13744, 13745, 13749, 13751, 13752, 13753, 13754, 13758, 13762, 13765, 13766, 13768, 13772, 13773, 13774, 13777, 13778, 13779, 13783, 13785, 13790, 13791, 13797, 13802, 13803, 13805, 13806, 13807, 13809, 13812, 13814, 13816, 13817, 13818, 13819, 13820, 13823, 13824, 13826, 13828, 13746, 13755, 13759, 13763, 13764, 13767, 13769, 13775, 13776, 13780, 13784, 13786, 13788, 13789, 13792, 13795, 13796, 13798, 13800, 13804, 13808, 13813, 13815, 13821, 13822, 13829, 13832, 13837, 13841, 13844, 13845, 13846, 13849, 13854, 13855, 13856, 13857, 13861, 13865, 13869, 13871, 13875, 13879, 13880, 13833, 13836, 13838, 13843, 13847, 13850, 13853, 13858, 13862, 13866, 13870, 13872, 13876, 13881, 13882, 13883, 13889, 13894, 13902, 13903, 13910, 13911, 13914, 13915, 13916, 13928, 13929, 13930, 13931, 13935, 13936, 13937, 13944, 13947, 13948, 13950, 13960, 13961, 13962, 13964, 13965, 13966, 13968, 13970, 13971, 13972, 13976, 13978, 13884, 13887, 13890, 13893, 13895, 13898, 13900, 13904, 13905, 13906, 13907, 13908, 13909, 13912, 13913, 13917, 13920, 13921, 13922, 13923, 13924, 13925, 13926, 13932, 13938, 13941, 13942, 13943, 13945, 13946, 13949, 13953, 13954, 13956, 13957, 13958, 13959, 13963, 13967, 13969, 13974, 13975, 13977, 13980, 13981, 13988, 13989, 13990, 13992, 13994, 13995, 13999, 13982, 13985, 13986, 13987, 13991, 13993, 13996, 14000, 14001, 14002, 14003, 14004, 14009, 14014, 14017, 14020, 14024, 14026, 14029, 14035, 14036, 14040, 14043, 14045, 14051, 14057, 14058, 14059, 14063, 14065, 14066, 14067, 14068, 14069, 14070, 14071, 14073, 14074, 14078, 14079, 14080, 14081, 14082, 14083, 14084, 14085, 14087, 14094, 14095, 14100, 14108, 14109, 14113, 14114, 14118, 14120, 14121, 14122, 14130, 14133, 14138, 14141, 14142, 14143, 14147, 14005, 14008, 14010, 14013, 14015, 14018, 14021, 14025, 14027, 14028, 14030, 14033, 14034, 14037, 14038, 14039, 14041, 14042, 14044, 14046, 14049, 14050, 14052, 14055, 14056, 14060, 14064, 14072, 14077, 14086, 14088, 14089, 14090, 14091, 14092, 14093, 14096, 14097, 14098, 14099, 141O1, 14102, 14103, 14104, 14105, 14106, 14107, 14110, 14115, 14123, 14126, 14127, 14132, 14134, 14135, 14136, 14137, 14139, 14140, 14144, 14145, 14148, 14151, 14155, 14157, 14161, 14166, 14171, 14175, 14183, 14187, 14190, 14192, 14194, 14195, 14196, 14200, 14201, 14152, 14156, 14158, 14162, 14165, 14167, 14170, 14172, 14176, 14179, 14180, 14182, 14184, 14185, 14186, 14188, 14189, 14191, 14193, 14197, 14198, 14199, 14202, 14203, 14204, 14210, 14211, 14216, 14217, 14223, 14226, 14228, 14229, 14230, 14233, 14235, 14239, 14241, 14243, 14244, 14250, 14254, 14255, 14256, 14257, 14260, 14261, 14262, 14267, 14268, 14269, 14273, 14274, 14277, 14281, 14284, 14286, 14289, 14292, 14293, 14297, 14299, 14301, 14304, 14305, 14306, 14312, 14316, 14320, 14322, 14324, 14328, 14329, 14331, 14337, 14342, 14344, 14346, 14347, 14349, 14350, 14353, 14356, 14361, 14363, 14364, 14365, 14366, 14205, 14208, 14212, 14215, 14218, 14221, 14224, 14225, 14227, 14231, 14236, 14240, 14242, 14245, 14246, 14247, 14251, 14263, 14266, 14270, 14278, 14282, 14283, 14285, 14287, 14290, 14291, 14294, 14298, 14300, 14302, 14303, 14307, 14310, 14313, 14317, 14323, 14325, 14327, 14330, 14334, 14338, 14340, 14341, 14345, 14348, 14351, 14352, 14354, 14355, 14357, 14358, 14359, 14360, 14367, 14368, 14372, 14373, 14375, 14386, 14369,14374, 14376, 14379, 14380, 14381, 14382, 14383, 14384 and SEQ ID NO:14385.

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker—a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NOS:304 to SEQ ID NO:535 and sequences complementary thereto, wherein the base sequence of said oligonucleotides comprises at least one CpG, TpG or CpA dinucleotide.

In a further preferred embodiment of the method, the *blocking oligonucleotides* are selected from the group consisting of SEQ ID NOS:2114, 2119, 2124, 2125, 2127, 2132, 2133, 2135, 2137, 2138, 2139, 2140, 2141, 2142, 2143, 2144, 2146, 2147, 2148, 2149, 2150, 2151, 2152, 2153, 2154, 2155, 2156, 2157, 2158, 2159, 2160, 2031, 2082, 2087, 2089, 2094, 2095, 2096, 2097, 2099, 2107, 1980, 2018, 2022, 2023, 2026, 1939, 1966, 1973, 1974, 1975, 1926, 1930, 1931, 1932, 1933, 1934, 1903, 1917, 1921, 1834, 1896, 1805, 1819, 1821, 1823, 1824, 1825, 1826, 1827, 1828, 1829, 1770, 1782, 1785, 1786, 1788, 1789, 1790, 1792, 1795, 1796, 1797, 1798, 1799, 1800, 1748, 1759, 1760, 1761, 1762, 1763, 1764, 1765, 1724, 1741, 1597, 1609, 1610, 1611, 1612, 1613, 1614, 1615, 1616, 1617, 1618, 1619, 1620, 1621, 1622, 1623, 1624, 1625, 1626, 1627, 1628, 1629, 1630, 1631, 1633, 1634, 1635, 1636, 1637, 1638, 1639, 1640, 1641, 1642, 1643, 1644, 1645, 1646, 1647, 1648, 1649, 1650, 1651, 1652, 1653, 1654, 1655, 1656, 1657, 1658, 1659, 1660, 1661, 1662, 1663, 1668, 1684, 1685, 1686, 1687, 1688, 1689, 1690, 1691, 1692, 1693, 1694, 1695, 1696, 1697, 1698, 1699, 1700, 1701, 1706, 1712, 1713, 1715, 1716, 1717, 1718, 1719, 1571, 1579, 1581, 1584, 1585, 1586, 1587, 1588, 1591, 1592, 1537, 1555, 1556, 1557, 1558, 1559, 1560, 1561, 1562, 1563, 1564, 1565, 1566, 1452, 1455, 1456, 1457, 1458, 1463, 1514, 1515, 1516, 1517, 1518, 1519, 1524, 1525, 1526, 1527, 1528, 1529, 1531, 1532, 1372, 1423, 1424, 1425, 1427, 1432, 1433, 1434, 1435, 1436, 1437, 1440, 1441, 1442, 1443, 1444, 1445, 1446, 1447, 1343, 1358, 1359, 1360, 1361, 1362, 1363, 1364, 1365, 1366, 1367, 1310, 1315, 1316, 1317, 1318, 1319, 1320, 1321, 1322, 1324, 1325, 1327, 1328, 1330, 1332, 1334, 1337, 1338, 1276, 1290, 1305, 1203, 1228, 1238, 1239, 1240, 1247, 1248, 1255, 1256, 1265, 1268, 1269, 1164, 1171, 1172, 1173, 1174, 1176, 1177, 1178, 1179, 1180, 1181, 1182, 1183, 1184, 1185, 1186, 1187, 1188, 1189, 1190, 1191, 1192, 1193, 1194, 1195, 1196, 1197, 1198, 2725, 2748, 2749, 2750, 2710, 2717, 2718, 2719, 2720, 2721, 2684, 2699, 2703, 2704, 2705, 2706, 2658, 2662, 2679, 2603, 2613, 2617, 2585, 2594, 2595, 2596, 2597, 2598, 2599, 2489, 2497, 2498, 2499, 2500, 2501, 2504, 2505, 2507, 2508, 2510, 2511, 2512, 2513, 2514, 2515, 2519, 2520, 2521, 2522, 2525, 2526, 2527, 2528, 2529, 2530, 2531, 2532, 2534, 2535, 2536, 2537, 2539, 2540, 2541, 2542, 2543, 2544, 2545, 2546, 2547, 2550, 2551, 2552, 2553, 2554, 2558, 2563, 2564, 2572, 2580, 2581, 2472, 2482, 2484, 2408, 2464, 2465, 2466, 2467, 2468, 2339, 2349, 2367, 2370, 2372, 2373, 2374, 2375, 2376, 2377, 2378, 2379, 2380, 2382, 2386, 2387, 2389, 2393, 2394, 2395, 2396, 2397, 2398, 2402, 2403, 2404, 2327, 2333, 2334, 2335, 2305, 2315, 2318, 2260, 2268, 2270, 2271, 2272, 2273, 2274, 2275, 2276, 2277, 2278, 2279, 2280, 2281, 2282, 2283, 2284, 2285, 2286, 2287, 2288, 2289, 2290, 2291, 2292, 2293, 2294, 2295, 2296, 2297, 2298, 2299, 2300, 2301, 2232, 2245, 2246, 2248, 2249, 2250, 2251, 2252, 2254, 2255, 2256, 2210, 2218, 2219, 2221, 2222, 2223, 2227, 2228, 2202, 2204, 2205, 2206, 2164, 2180, 2183, 2187, 2188, 2189, 2190, 2191, 2192, 2193, 2194, 2195, 2196, 2197 and 2198.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NOS:1 to SEQ ID NO:64, and the equivalent positions within SEQ ID NOS:304 to SEQ ID NO:535. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in p referred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, *the fifth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fifth step* of the method comprises sequencing and subsequent sequence analysis of the amplificate generated in the *third step* of the method (Sanger F., et al., Proc Natl Acad Sci USA 74:5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NOS:304 to SEQ ID NO:535 and sequences complementary thereto, wherein the base sequence of said oligomers comprises at least one CpG dinucleotide.

In a further preferred embodiment of the method, the MSP primers are selected from the group consisting of SEQ ID NOS:2754, 2757, 2759, 2767, 2768, 2772, 2776, 2779, 2784, 2785, 2786, 2789, 2791, 2792, 2794, 2797, 2798, 2801, 2805, 2806, 2809, 2811, 2815, 2817, 2824, 2830, 2832, 2833, 2834, 2838, 2840, 2843, 2846, 2850, 2851, 2853, 2855, 2859, 2866, 2867, 2870, 2871, 2876, 2878, 2881, 2885, 2886, 2892, 2893, 2895, 2896, 2898, 2904, 2912, 2915, 2917, 2919, 2922, 2923, 2929, 2931, 2939, 2941, 2944, 2946, 2948, 2950, 2955, 2956, 2957, 2960, 2962, 2965, 2966, 2967, 2968, 2969, 2971, 2975, 2976, 2977, 2978, 2979, 2980, 2981, 2984, 2988, 2989, 2990, 2992, 2994, 2997, 2999, 3001, 3002, 3004, 3006, 3007, 3008, 3010, 3013, 3014, 3016, 3017, 3018, 3020, 3022, 3025, 2752, 2755, 2760, 2762, 2763, 2764, 2765, 2769, 2771, 2773, 2775, 2777, 2780, 2781, 2782, 2783, 2787, 2788, 2790, 2793, 2795, 2796, 2800, 2802, 2803, 2804, 2808, 2810, 2812, 2814, 2816, 2818, 2820, 2821, 2823, 2825, 2827, 2828, 2829, 2831, 2835, 2836, 2837, 2839, 2841, 2842, 2844, 2845, 2847, 2849, 2852, 2854, 2856, 2858, 2860, 2861, 2862, 2863, 2864, 2865, 2868, 2869, 2872, 2873, 2874, 2875, 2877, 2879, 2880, 2882, 2883, 2884, 2887, 2888, 2889, 2890, 2891, 2894, 2897, 2899, 2901, 2902, 2903, 2905, 2907, 2909, 2911, 2914, 2916, 2918, 2920, 2924, 2926, 2927, 2928, 2930, 2932, 2933, 2934, 2935, 2936, 2937, 2938, 2942, 2945, 2947, 2949, 2951, 2953, 2954, 2958, 2959, 2961, 2963, 2970, 2972, 2973, 2974, 2982, 2985, 2987, 2991, 2993, 2995, 2996, 2998, 3000, 3003, 3005, 3011, 3019, 3021, 3023, 3026, 3027, 3028, 3032, 3033, 3036, 3037, 3038, 3039, 3041, 3042, 3043, 3044, 3047, 3048, 3049, 3052, 3055, 3059, 3061, 3064, 3065, 3068, 3069, 3071, 3072, 3075, 3076, 3080, 3083, 3084, 3085, 3086, 3091, 3093, 3096, 3097, 3100, 3104, 3109, 3110, 3113, 3115, 3117, 3118, 3123, 3126, 3127, 3130, 3134, 3135, 3136, 3138, 3139, 3144, 3146, 3147, 3149, 3150, 3155, 3029, 3031, 3034, 3040, 3045, 3050, 3051, 3053, 3054, 3056, 3057, 3060, 3063, 3066, 3073, 3078, 3081, 3088, 3089, 3090, 3092, 3094, 3098, 3101, 3103, 3105, 3107, 3108, 3111, 3114, 3116, 3119, 3121, 3122, 3124, 3128, 3131, 3133, 3137,3140,3142, 3145, 3148,3151,3152, 3153, 3157,3159,3165, 3166, 3167,3170,3171, 3172, 3174, 3175, 3176, 3179, 3180, 3181, 3184, 3185, 3186, 3192, 3194, 3198,3201,3202, 3203, 3204, 3206, 3209, 3212, 3213, 3215, 3216, 3217, 3218, 3221, 3224, 3226, 3228, 3229, 3230, 3232, 3233, 3234, 3235, 3237, 3238, 3239, 3245, 3247, 3248, 3249, 3160, 3162, 3163, 3164, 3168, 3169, 3173, 3178, 3182, 3183, 3187, 3189, 3190, 3195, 3196, 3197,3199,3205, 3207, 3210, 3214, 3219, 3222, 3223, 3225, 3227, 3236, 3240, 3242, 3243, 3244, 3246, 3250, 3252, 3256, 3261, 3264, 3268, 3269, 3270, 3273, 3275, 3279, 3280, 3281, 3282, 3283, 3284, 3285, 3286, 3290, 3292, 3295, 3297, 3301, 3304, 3305, 3306, 3307, 3308, 3310, 3311, 3312, 3315, 3319, 3320, 3324, 3327, 3331, 3333, 3334, 3339, 3348, 3351, 3354, 3358, 3360, 3364, 3367, 3368, 3374, 3376, 3253, 3255, 3257, 3259, 3260, 3262, 3265, 3266, 3267, 3271, 3274, 3276, 3278, 3287, 3289, 3291, 3293, 3298, 3300, 3302, 3309, 3313, 3314, 3316, 3318, 3321, 3322, 3325, 3328, 3330, 3332, 3335, 3337, 3338, 3340, 3342, 3343, 3344, 3345, 3346, 3347, 3349, 3350, 3352, 3353, 3355, 3356, 3357, 3359, 3361, 3363, 3365, 3369, 3371, 3372, 3373, 3377, 3380, 3383, 3386, 3388, 3389, 3390, 3391, 3392, 3395, 3396, 3400, 3401, 3403, 3406, 3408, 3413, 3414, 3417, 3420, 3423, 3428, 3432, 3433, 3434, 3436, 3439, 3440, 3442, 3443, 3445, 3447, 3453, 3455, 3458, 3463, 3466, 3469, 3470, 3471, 3472, 3473, 3474, 3475, 3477, 3478, 3480, 3481, 3483, 3484, 3487, 3490, 3494, 3495, 3496, 3497, 3499, 3500, 3504, 3505, 3508, 3512, 3514, 3515, 3516, 3521, 3525, 3529, 3533, 3534, 3537, 3538, 3540, 3541, 3542, 3543, 3544, 3547, 3548, 3553, 3557, 3558, 3559, 3560, 3562, 3563, 3566, 3571, 3574, 3575, 3577, 3580, 3583, 3584, 3585, 3586, 3592, 3594, 3595, 3597, 3598, 3599, 3601, 3603, 3604, 3607, 3608, 3612, 3619, 3621, 3622, 3626, 3631, 3632, 3637, 3638, 3639, 3640, 3642, 3643, 3644, 3646, 3647, 3649, 3650, 3652, 3656, 3657, 3660, 3663, 3668, 3670, 3673, 3676, 3677, 3678, 3683, 3688, 3690, 3694, 3697, 3700, 3702, 3703, 3704, 3708, 3404, 3409, 3411, 3415, 3418, 3419, 3421, 3424, 3426, 3427, 3429, 3431, 3435, 3437, 3438, 3441, 3444, 3446, 3448, 3450, 3452, 3454, 3456, 3459, 3461, 3462, 3464, 3467, 3476, 3479, 3482, 3485, 3488, 3489, 3491, 3492, 3493, 3501, 3503, 3506, 3509, 3510, 3511, 3513, 3517, 3518, 3519, 3520, 3522, 3524, 3526, 3527, 3528, 3530, 3531, 3535, 3536, 3545, 3549, 3551, 3552, 3554, 3555, 3556, 3561, 3564, 3565, 3568, 3569, 3570, 3573, 3576, 3578, 3581, 3587, 3588, 3589, 3590, 3591, 3593, 3596, 3600, 3602, 3605, 3606, 3609, 3611, 3613, 3614, 3615, 3616, 3617, 3618, 3623, 3624, 3625, 3627, 3629, 3630, 3633, 3634, 3635, 3636, 3641, 3645, 3651, 3653, 3654, 3655, 3658, 3661, 3662, 3665, 3666, 3667, 3669, 3671, 3674, 3679, 3681, 3684, 3686, 3689, 3691, 3692, 3693, 3695, 3696, 3698, 3701, 3705, 3706, 3707, 3709, 3710, 3714, 3715, 3717, 3718, 3722, 3723, 3729, 3730, 3732, 3735, 3739, 3740, 3741, 3743, 3744, 3745, 3746, 3749, 3753, 3754, 3755, 3756, 3711, 3713, 3719, 3721, 3724, 3725, 3726, 3727, 3728, 3731, 3733, 3736, 3737, 3738, 3742, 3747, 3748, 3750, 3752, 3757, 3758, 3759, 3760, 3761, 3764, 3767, 3771, 3773, 3775, 3776, 3777, 3781, 3785, 3786, 3787, 3790, 3791, 3793, 3796, 3797, 3798, 3799, 3805, 3809, 3814, 3817, 3818, 3822, 3823, 3828, 3833, 3836, 3844, 3847, 3850, 3851, 3853, 3856, 3857, 3862, .3873, 3876, 3877, 3880, 3885, 3887, 3889, 3890, 3892, 3893, 3895, 3896, 3899, 3902, 3903, 3905, 3906, 3907, 3908, 3916, 3923, 3926, 3927, 3930, 3935, 3936, 3938, 3939, 3940, 3944, 3945, 3949, 3950, 3951, 3954, 3955, 3957, 3960, 3965, 3970, 3971, 3978, 3979, 3982, 3983, 3984, 3985, 3762, 3765, 3768, 3770, 3772, 3774, 3778, 3779, 3780, 3782, 3784, 3788, 3792, 3794, 3800, 3801, 3802, 3803, 3804, 3806, 3807, 3808, 3810, 3812, 3813, 3815, 3819, 3820, 3821, 3824, 3826, 3827, 3829, 3831, 3832, 3834,3837,3838,3839,3840, 3842, 3845, 3848, 3852, 3854, 3858, 3860, 3861, 3863, 3864, 3865, 3866, 3867, 3868, 3869, 3870, 3871, 3872, 3874, 3875, 3878, 3881, 3883, 3884, 3886, 3888, 3891, 3894, 3897, 3898, 3900, 3901, 3904, 3909, 3911, 3914, 3915, 3917, 3919, 3920, 3921, 3922, 3924, 3928, 3931, 3933,3934,3937,3941,3942,3943, 3947,3948,3952,3958,3959,3961,3962,3963,3964, 3966, 3968, 3972, 3974, 3975, 3976, 3980, 3981, 3986, 3987, 3990, 3993, 3994, 3997, 4000, 4003, 4004, 4006, 4012, 4015, 4017, 4020, 4024, 4027, 4029, 4031, 4032, 4033, 4037, 4038, 4040, 4042, 4047, 4048, 4050, 4051, 4052, 4054, 4055, 4058, 4063, 4066, 4069, 4070, 4072, 4073, 4076, 4078, 4079, 4082, 4083, 4084, 4085, 4086, 4090, 4093, 4094, 4095, 4096, 4099, 4100, 4104, 4106, 4113, 4116, 4119, 4120, 4124, 4127, 4130, 4134, 4135, 4136, 4138, 4142, 4145, 4146, 4149, 4150, 4152, 4153, 4159, 4160, 4161, 4163, 4168, 4169, 4171, 4173, 4176, 4180, 4181, 4185, 4189, 4191, 4192, 4194, 4197, 4198, 4199, 4201, 4206, 4207, 4208, 4213, 4219, 4220, 4223, 4224, 4225, 4228, 4231, 4232, 4233, 4237, 4238, 4242, 4243, 4246, 4247, 4251, 4254, 4255, 4256, 4258, 3988, 3991, 3995, 3998, 4001, 4005, 4007, 4009, 4010, 4011, 4013, 4016, 4018, 4019, 4021, 4022, 4023, 4025, 4028, 4034, 4035, 4039, 4043, 4045, 4046, 4049, 4056, 4059, 4061, 4064, 4065, 4067, 4068, 4071, 4074, 4075, 4077, 4080, 4087, 4088, 4089, 4092, 4098, 4101, 4102, 4105, 4108, 4109, 4110, 4111, 4112, 4114, 4115, 4117, 4121, 4122, 4123, 4125, 4128, 4129, 4131, 4132, 4133, 4137, 4139, 4141, 4144, 4148, 4151, 4154, 4156, 4157, 4162, 4165, 4166, 4167, 4170, 4172, 4174, 4177, 4179, 4182, 4184, 41-86, 4188, 4190, 4195, 4196, 4202, 4204, 4205, 4209, 4211, 4214, 4216, 4217, 4218, 4221, 4226, 4229, 4234, 4236, 4239, 4241, 4244, 4245, 4248, 4249, 4252, 4257, 4259, 4260, 4261, 4262, 4266, 4267, 4268, 4271, 4272, 4275, 4276, 4279, 4281, 4282, 4284, 4285, 4287, 4289, 4290, 4292, 4295, 4297, 4299, 4301, 4306, 4310, 4316, 4317, 4318, 4321, 4323, 4325, 4328, 4334, 4335, 4337, 4341, 4342, 4343, 4344, 4348, 4350, 4351, 4352, 4354, 4357, 4358, 4359, 4364, 4365, 4366, 4370, 4371, 4372, 4373, 4376, 4378, 4381, 4383, 4384, 4386, 4388, 4396, 4397, 4399, 4400, 4403, 4405, 4407, 4409, 4412, 4415, 4416, 4417, 4424, 4426, 4429, 4433, 4435, 4437, 4442, 4443, 4447, 4450, 4451, 4452, 4455, 4457, 4460, 4461, 4462, 4465, 4466, 4469, 4470, 4474, 4478, 4481, 4484, 4488, 4491, 4494, 4495, 4497, 4498, 4504, 4506, 4509, 4510, 4511, 4514, 4516, 4518, 4520, 4521, 4523, 4525, 4528, 4530, 4533, 4534, 4535, 4538, 4540, 4542, 4545, 4547, 4548, 4553, 4554, 4555, 4556, 4557, 4562, 4564, 4567, 4568, 4569, 4579, 4580, 4582, 4584, 4587, 4589, 4593, 4596, 4597, 4598, 4600, 4601, 4602, 4605, 4606, 4607, 4609, 4610, 4613, 4614, 4616, 4618, 4621, 4624, 4626, 4627, 4628, 4631, 4632, 4636, 4639, 4641, 4644, 4645, 4648, 4263, 4265, 4269, 4270, 4273, 4274, 4277, 4280, 4283, 4286, 4288, 4291, 4293, 4296, 4298, 4300, 4302, 4303, 4304, 4305, 4307, 4308, 4309, 4311, 4313, 4315, 4319, 4322, 4324, 4326, 4327, 4330, 4331, 4332, 4333, 4336, 4338, 4340, 4345, 4347, 4349, 4353, 4355, 4360, 4361, 4362, 4367, 4368, 4369, 4374, 4375, 4377, 4379, 4382, 4385, 4387, 4389, 4390, 4391, 4392, 4393, 4394, 4395, 4398, 4401, 4406, 4408, 4410, 4411, 4413, 4418, 4420, 4422, 4423, 4425, 4427, 4428, 4430, 4432, 4434, 4436, 4438, 4440, 4441, 4444, 4446, 4448, 4453, 4454, 4456, 4458, 4463, 4464, 4467, 4471, 4472, 4473, 4475, 4476, 4477, 4479, 4480, 4482, 4485, 4486, 4487, 4489, 4490, 4492, 4496, 4499, 4501, 4502, 4503, 4505, 4507, 4508, 4512, 4515, 4517, 4519, 4522, 4524, 4529, 4531, 4536, 4537, 4539, 4541, 4543, 4546, 4549, 4551, 4558, 4559, 4560, 4561, 4563, 4565, 4570, 4571, 4572, 4573, 4574, 4575, 4576, 4578, 4581, 4583, 4585, 4586, 4588, 4590, 4592, 4595, 4599, 4603, 4611, 4612, 4615, 4617, 4619, 4620, 4622, 4630, 4633, 4634, 4635, 4638, 4640, 4642, 4643, 4646, 4647, 4649, 4652, 4655, 4659, 4661, 4663, 4664, 4665, 4668, 4671, 4674, 4675, 4676, 4678, 4680, 4683, 4686, 4690, 4696, 4700, 4703, 4704, 4706, 4709, 4711, 4714, 4715, 4717, 4718, 4719, 4720, 4721, 4726, 4727, 4730, 4732, 4738, 4742, 4745, 4749, 4750, 4753, 4755, 4759, 4763, 4768, 4769, 4772, 4773, 4776, 4778, 4779, 4782, 4784, 4789, 4790, 4794, 4797, 4801, 4805, 4807, 4813, 4814, 4819, 4821, 4822, 4827, 4829, 4833, 4834, 4835, 4837, 4838, 4841, 4842, 4843, 4844, 4847, 4849, 4850, 4852, 4853, 4859, 4860, 4861, 4865, 4867, 4868, 4869, 4870, 4873, 4876, 4877, 4878, 4879, 4883, 4885, 4889, 4891, 4892, 4894, 4895, 4897, 4898, 4903, 4906, 4911, 4912, 4918, 4919, 4920, 4924, 4927, 4929, 4932, 4934, 4941, 4942, 4945, 4948, 4949, 4950, 4650, 4653, 4656, 4658, 4660, 4662, 4666, 4667, 4669, 4670, 4672, 4677, 4681, 4684, 4685, 4687, 4688, 4689, 4691, 4693, 4694, 4695, 4697, 4699, 4701, 4702, 4705, 4707, 4708, 4716, 4722, 4724, 4728, 4731, 4733, 4735, 4736, 4737, 4739, 4741, 4744, 4746, 4748, 4751, 4754, 4756, 4758, 4760, 4761, 4764, 4766, 4767, 4770, 4771, 4774, 4777, 4780, 4783, 4785, 4786, 4787, 4788, 4791, 4792, 4793, 4795, 4798, 4800, 4802, 4804, 4806, 4808, 4810, 4811, 4812, 4815, 4816, 4817, 4818, 4820, 4823, 4824, 4825, 4828, 4830, 4832, 4836, 4839, 4840, 4845, 4846, 4848, 4851, 4854, 4855, 4856, 4857, 4858, 4862, 4864, 4871, 4872, 4874, 4875, 4881, 4882, 4884, 4886, 4887, 4890, 4893, 4896, 4899, 4900, 4901, 4902, 4904, 4905, 4907, 4908, 4909, 4910, 4913, 4915, 4917, 4921, 4922, 4923, 4925, 4926, 4928, 4930, 4935, 4936, 4937, 4938, 4940, 4943, 4944, 4946, 4947, 4951, 4952, 4953, 4954, 4956, 4957, 4960, 4963, 4967, 4970, 4971, 4972, 4976, 4977, 4985, 4986, 4987, 4989, 4991, 4992, 4997, 4999, 5002, 5003, 5004, 5006, 5010, 5013, 5014, 5015, 5017, 5018, 5019, 5020, 5021, 5024, 5025, 5027, 5031, 5032, 5033, 5037, 5040, 5041, 5042, 5043, 4958, 4961, 4964, 4966, 4968, 4969, 4973, 4974, 4975, 4978, 4979, 4980, 4981, 4982, 4983, 4984, 4988, 4990, 4993, 4995, 4996, 4998, 5000, 5005, 5007, 5009, 5011, 5016, 5022, 5026, 5028, 5030, 5034, 5036, 5038, 5039, 5044, 5047,5049, 5050, 5052, 5054, 5055, 5057, 5060, 5061, 5064, 5045, 5048, 5051, 5053, 5056, 5058, 5062, 5065, 5066, 5069, 5073, 5077, 5080, 5083, 5087, 5093, 5096, 5099, 5100, 5103, 5104, 5106, 5111, 5116, 5119, 5120, 5125, 5128, 5132, 5134, 5135, 5137, 5139, 5140, 5142, 5144, 5148, 5149, 5150, 5155, 5157, 5158,5160, 5166, 5168, 5170, 5171, 5173, 5176, 5177, 5179, 5184, 5187, 5188, 5190, 5193, 5195, 5198, 5201, 5204, 5208, 5211, 5215, 5216, 5217, 5220, 5221, 5222, 5224, 5226, 5229, 5230, 5231, 5232, 5234, 5237, 5242, 5244, 5245, 5246, 5247, 5249, 5250, 5252, 5254, 5255, 5256, 5261, 5262, 5264, 5265, 5273, 5274, 5276, 5278, 5279, 5281, 5284, 5285, 5286, 5287, 5288, 5289, 5292, 5296, 5300, 5303, 5304, 5307, 5308, 5309, 5313, 5315, 5317, 5318, 5322, 5323, 5326, 5329, 5330, 5333, 5336, 5337, 5341, 5343, 5348, 5349, 5352, 5354, 5355, 5358, 5359, 5361, 5362, 5363, 5364, 5365, 5367, 5369, 5370, 5372, 5375, 5377, 5379, 5382, 5385, 5387, 5393, 5394, 5395, 5398, 5400, 5404, 5407, 5408, 5409, 5412, 5413, 5414, 5416, 5418, 5421, 5424, 5426, 5428, 5431, 5432, 5433, 5434, 5435, 5437, 5440, 5441, 5443, 5448, 5450, 5451, 5454, 5455, 5458, 5460, 5461, 5464, 5468, 5470, 5471, 5474, 5475, 5477, 5482, 5484, 5485, 5487, 5490, 5491, 5492, 5494, 5496, 5498, 5499, 5500, 5501, 5502, 5503, 5504, 5506, 5509, 5510, 5512, 5516, 5517, 5518, 5524, 5525, 5526, 5528, 5531, 5533, 5534, 5535, 5536, 5537, 5540, 5542, 5544, 5545, 5546, 5549, 5550, 5552, 5553, 5559, 5561, 5566, 5569, 5572, 5573, 5575, 5577, 5578, 5579, 5580, 5581, 5582, 5588, 5593, 5595, 5596, 5598, 5600, 5601, 5603, 5604, 5605, 5608, 5614, 5615, 5616, 5619, 5622, 5624, 5625, 5628, 5629, 5633, 5634, 5637, 5638, 5639, 5643, 5644, 5645, 5647, 5648, 5649, 5650, 5654, 5662, 5664, 5067, 5070, 5072, 5074, 5075, 5076, 5078, 5081, 5084, 5086, 5088, 5090, 5091, 5094, 5097, 5101, 5105, 5107, 5109, 5110, 5112, 5114, 5115, 5117, 5121, 5123, 5124, 5126, 5129, 5131, 5136, 5143, 5145, 5147, 5151, 5153, 5156, 5159, 5161, 5162, 5163, 5164, 5165, 5167, 5169, 5172, 5174, 5175, 5180, 5181, 5182, 5183, 5185, 5186, 5189, 5191, 5194, 5196, 5199, 5200, 5202, 5203, 5205, 5207, 5209, 5210, 5212, 5214, 5218, 5219, 5223, 5225, 5227, 5233, 5235, 5238, 5239, 5240, 5241, 5248, 5251, 5253, 5257, 5259, 5260, 5263, 5266, 5268, 5269, 5270, 5271, 5272, 5280, 5282, 5290, 5291, 5293, 5295, 5297, 5299, 5301, 5306, 5310, 5311, 5312, 5314, 5316, 5319, 5321, 5324, 5327, 5331, 5332, 5334, 5338, 5339, 5340, 5342, 5344, 5346, 5347, 5351, 5353, 5356, 5360, 5366, 5368, 5371, 5373, 5376, 5378, 5380, 5383, 5388, 5389, 5391, 5392, 5396, 5399, 5401, 5403, 5405, 5410, 5415, 5417, 5419, 5420, 5422, 5425, 5427, 5429, 5436, 5438, 5442, 5444, 5445, 5446, 5447, 5449, 5452, 5456, 5457, 5459, 5462, 5465, 5466, 5467, 5469, 5472, 5478, 5480, 5481, 5486, 5488, 5493, 5495, 5497, 5505, 5507, 5508, 5511, 5513, 5515, 5519, 5521, 5522, 5527, 5529, 5532, 5538, 5539, 5541, 5543, 5548, 5551, 5554, 5555, 5556, 5557, 5558, 5560, 5562, 5563, 5564, 5565, 5567, 5568, 5570, 5571, 5574, 5576, 5583, 5585, 5586, 5587, 5589, 5591, 5592, 5594, 5597, 5599, 5602, 5606, 5609, 5611, 5612, 5618, 5620, 5623, 5626, 5627, 5630, 5632, 5635, 5640, 5642, 5646, 5651, 5652, 5656, 5657, 5659, 5660, 5661, 5663, 5665, 5667, 5670, 5672, 5673, 5676, 5678, 5679, 5681, 5682, 5683, 5686, 5687, 5688, 5690, 5694, 5697, 5698, 5699, 5700, 5701, 5704, 5708, 5709, 5711, 5713, 5714, 5717, 5721, 5723, 5724, 5727, 5728, 5733, 5734, 5735, 5736, 5739, 5740, 5741, 5742, 5743, 5668, 5671, 5674, 5677, 5680, 5684, 5689, 5691, 5693, 5696, 5702, 5705, 5707, 5710, 5712, 5715, 5718, 5720, 5722, 5725, 5729, 5730, 5731, 5732,5737, 5744, 5747, 5750, 5753, 5759, 5762, 5763, 5770, 5775, 5777, 5778, 5780, 5782, 5785, 5787, 5791, 5792, 5793, 5794, 5797, 5801, 5803, 5808, 5810, 5812, 5814, 5816, 5817, 5824, 5832, 5833, 5834, 5835, 5838, 5840, 5841, 5844, 5849, 5853, 5854, 5857, 5859, 5860, 5862, 5863, 5865, 5868, 5874, 5882, 5883, 5885, 5887, 5894, 5895, 5896, 5897, 5902, 5904, 5906, 5909, 5912, 5915, 5918, 5919, 5920, 5922, 5923, 5926, 5927, 5928, 5930, 5932, 5933, 5934, 5935, 5939, 5940, 5946, 5947, 5948, 5949, 5952, 5954, 5955, 5956, 5958, 5959, 5961, 5962, 5964, 5965, 5968,5970,5971,5972, 5973, 5977, 5978, 5980, 5983, 5986, 5987, 5992, 5993, 5996, 5997, 6000, 6002, 6003, 6004, 6005, 6006, 6008, 6009, 6010, 6011, 6012, 6013, 6017, 6018, 6019, 6020, 6025, 6027, 6030, 6033, 6035, 6036, 6039, 6041, 6042, 6045, 6049, 6050, 6051, 6055, 6059, 6063, 6064, 6065, 6067, 6070, 6072, 6073, 6075, 6077, 6078, 6081, 5745, 5748, 5751. 5754, 5756, 5758, 5760, 5764, 5766, 5767, 5768, 5769, 5771, 5773, 5774, 5776, 5779, 5781, 5783, 5786, 5788, 5789, 5790, 5795, 5796, 5798, 5800, 5802, 5804, 5806, 5807,5809, 5811, 5813, 5818, 5820, 5821, 5822, 5823, 5825, 5826, 5827, 5828, 5830, 5831, 5836, 5837, 5839, 5842, 5843, 5845, 5846, 5847, 5848, 5850, 5852, 5855, 5856, 5858, 5861, 5864, 5866, 5867, 5870, 5871, 5872, 5873, 5875, 5876, 5877, 5878, 5879, 5880, 5881, 5884, 5886, 5888, 5889, 5890, 5891, 5892, 5893, 5898, 5899, 5900, 5903, 5905, 5907, 5910, 5913, 5916, 5921, 5924, 5929, 5931, 5936, 5937, 5938, 5942, 5943, 5944, 5945, 5950, 5953, 5957, 5960, 5963, 5966, 5969, 5975, 5976, 5981, 5984, 5988, 5990, 5991, 5994, 5998, 5999, 6001, 6007, 6014, 6015, 6016, 6021, 6023, 6024, 6026, 6028, 6031, 6032, 6034, 6037, 6038, 6040, 6043, 6046, 6048, 6053, 6054, 6056, 6058, 6060, 6062, 6066, 6068, 6074, 6076, 6079, 6083, 6084, 6085, 6089, 6090, 6093, 6096, 6098, 6100, 6106, 6110, 6111, 6114, 6118, 6121, 6127, 6130, 6133, 6135, 6137, 6138, 6141, 6143, 6145, 6148, 6150, 6151, 6152, 6153, 6154, 6156, 6159, 6160, 6162, 6163, 6166, 6167, 6168, 6169, 6170, 6171, 6172, 6175, 6177, 6180, 6183, 6187, 6188, 6190, 6193, 6194, 6195, 6196, 6198, 6201, 6202, 6204, 6207, 6211, 6212, 6214, 6215, 6216, 6218, 6219, 6220, 6222, 6225, 6226, 6230, 6235, 6239, 6241, 6242, 6243, 6244, 6245, 6247, 6250, 6252, 6254, 6259, 6260, 6261, 6264, 6265, 6268, 6270, 6273, 6275, 6278, 6280, 6282, 6286, 6287, 6288, 6291, 6293, 6298, 6299, 6300, 6302, 6303, 6305, 6307, 6308, 6312, 6313, 6314, 6315, 6316, 6317, 6318, 6320, 6321, 6323, 6325, 6327, 6328, 6334, 6335, 6337, 6340, 6341, 6344, 6348, 6350, 6353, 6354, 6357, 6360, 6362, 6364, 6368, 6372, 6374, 6377, 6378, 6383, 6386, 6392, 6394, 6398, 6400, 6404, 6405, 6409, 6410, 6411, 6417, 6418, 6424, 6426, 6429, 6431, 6432, 6433, 6435, 6436, 6437, 6438, 6440, 6442, 6445, 6446, 6448, 6449, 6452, 6455, 6457, 6458, 6461, 6462, 6463, 6464, 6465, 6470, 6473, 6475, 6478, 6480, 6481, 6484, 6485, 6486, 6489, 6491, 6492, 6494, 6498, 6502, 6504, 6505, 6507, 6511, 6513, 6514, 6086, 6088, 6091, 6094, 6097, 6099, 6101, 6103, 6105, 6108, 6112, 6115, 6116, 6117, 6119, 6120, 6122, 6123, 6125, 6126, 6128, 6131, 6132, 6134, 6136, 6139, 6142, 6144, 6146, 6149, 6155, 6157, 6161, 6164, 6173, 6174, 6176, 6178, 6181, 6182, 6184, 6186, 6189, 6191, 6192, 6197, 6199, 6200, 6205, 6206, 6208, 6210, 6213, 6221, 6223, 6224, 6227, 6229, 6231, 6232, 6233, 6234, 6236, 6237, 6238, 6240, 6246, 6248, 6251, 6253, 6255, 6256, 6257, 6258, 6262, 6266, 6269, 6271, 6272, 6274, 6276, 6277, 6281, 6283, 6284, 6285, 6289, 6292, 6294, 6295, 6296, 6297, 6301, 6304, 6306, 6309, 6310, 6311, 6319, 6322, 6324, 6326, 6329, 6330, 6331, 6333, 6336, 6338, 6342, 6345, 6347, 6349, 6351, 6355, 6356, 6358, 6359, 6361, 6365, 6367, 6369, 6371, 6373, 6375, 6376, 6379, 6381, 6382, 6384, 6387, 6388, 6389, 6390, 6393, 6395, 6397, 6399, 6401, 6402, 6403, 6406, 6407, 6412, 6413, 6414, 6415, 6416, 6419, 6420, 6421, 6422, 6423, 6425, 6427, 6428, 6434, 6441, 6443, 6444, 6447, 6450, 6451, 6453, 6459, 6460, 6466, 6467, 6468, 6469, 6471, 6472, 6474, 6476, 6479, 6482, 6487, 6490, 6493, 6496, 6497, 6499, 6500, 6501, 6503, 6506, 6508, 6510, 6512, 6515, 6518, 6521, 6522, 6523, 6524, 6528, 6532, 6535, 6539, 6549, 6560, 6561, 6564, 6566, 6567, 6570, 6573, 6576, 6577, 6578, 6579, 6583, 6584, 6585, 6586, 6587, 6590, 6591, 6594, 6596, 6597, 6603, 6605, 6608, 6609, 6610, 6611, 6612, 6615, 6625, 6630, 6632, 6633, 6634, 6638, 6639, 6642, 6643, 6644, 6647, 6650, 6655, 6657, 6658, 6659, 6661, 6663, 6664, 6665, 6669, 6671, 6674, 6677, 6678, 6679, 6680, 6681, 6682, 6686, 6687, 6688, 6689, 6690, 6691, 6693, 6695, 6696, 6697, 6699, 6701, 6703, 6704, 6705, 6706, 6707, 6708, 6710, 6711, 6712, 6713, 6717, 6718, 6720, 6721, 6722, 6725, 6727, 6732, 6734, 6735, 6739, 6741, 6745, 6748, 6751, 6755, 6759, 6762, 6764, 6767, 6770, 6772, 6773, 6774, 6777, 6778, 6782, 6785, 6786, 6787, 6790, 6791, 6794, 6795, 6796, 6798, 6800, 6802, 6803, 6806, 6811, 6814, 6815, 6818, 6824, 6829, 6834, 6835, 6842, 6516, 6519, 6525, 6526, 6527, 6529, 6531, 6533, 6536, 6537, 6538, 6540, 6541, 6542, 6543, 6544, 6545, 6546, 6547, 6548, 6550, 6551, 6552, 6553, 6554, 6556, 6558, 6562, 6563, 6565, 6568, 6571, 6572, 6575, 6580, 6582, 6588, 6589, 6592, 6595, 6598, 6599, 6600, 6601, 6602, 6604, 6606, 6607, 6613, 6614, 6617, 6618, 6619, 6620, 6621, 6623, 6624, 6627, 6628, 6629, 6631, 6635, 6640, 6641, 6645, 6646, 6649, 6651, 6652, 6653, 6656, 6660, 6662, 6666, 6668, 6670, 6672, 6675, 6676, 6683, 6685, 6692, 6694, 6698, 6700, 6702, 6709, 6714, 6715, 6716, 6719, 6723, 6726, 6728, 6730, 6731, 6736, 6738, 6740, 6742, 6744, 6746, 6747, 6749, 6752, 6754, 6756, 6757, 6758, 6760, 6761, 6765, 6769, 6775, 6779, 6781, 6783, 6784, 6789, 6792, 6797, 6799, 6801, 6804, 6805, 6807, 6809, 6810, 6812, 6816, 6817, 6819, 6821, 6822, 6825, 6827, 6828, 6830, 6831, 6832, 6833, 6836, 6837, 6838, 6839, 6840, 6841, 6843,

Detection: 6517, 6520, 6 530, 6534, 6555, 6557, 6559, 6569, 6574, 6581, 6593, 6616, 6622, 6626, 6636, 6637, 6648, 6654, 6667, 6673, 6684, 6724, 6729, 6733, 6737, 6743, 6750, 6753, 6763, 6766, 6768, 6771, 6776, 6780, 6788, 6793, 6808, 6813, 6820, 6823, 6826, 6844, 6848, 6850, 6852, 6854, 6857, 6859, 6861, 6862, 6865, 6868, 6870, 6871, 6872, 6874, 6875, 6876, 6880, 6883, 6886, 6887, 6888, 6890, 6891, 6894, 6896, 6897, 6898, 6899, 6900, 6901, 6902, 6904, 6905, 6906, 6910, 6912, 6917, 6920, 6845, 6847, 6849, 6851, 6853, 6855, 6858, 6860, 6863, 6866, 6869, 6873, 6877, 6879, 6881, 6882, 6884, 6889, 6892, 6893, 6895, 6907, 6908, 6909, 6911, 6913, 6915, 6916, 6918, 6921, 6924, 6927, 6928, 6933, 6935, 6937, 6939, 6940, 6943, 6946, 6947, 6948, 6949, 6951, 6953, 6954, 6955, 6959, 6961, 6964, 6965, 6968, 6969, 6972, 6973, 6976, 6979, 6980, 6922, 6925, 6929, 6930, 6931, 6932, 6934, 6936, 6942, 6944, 6945, 6950, 6952, 6956, 6957, 6958, 6960, 6962, 6963, 6966, 6967, 6970, 6971, 6974, 6975, 6977, 6981, 6983, 6986, 6992, 6995, 6998, 7002, 7006, 7013, 7017, 7022, 7024, 7027, 7030, 7032, 7036, 7037, 7040, 7050, 7052, 7055, 7056, 7058, 7059, 7060, 7064, 7067, 7072, 7074, 7076, 7077, 7084, 7085, 7086, 7087, 7088, 7089, 7090, 7091, 7096, 7097, 7100, 7102, 7109, 7110, 7113, 7114, 7115, 7118, 7121, 7123, 7124, 7131, 7133, 7135, 7136, 7138, 7139, 7140, 7144, 7146, 7148, 7149, 7158, 7159, 7160, 7163, 7165, 7166, 7168, 7172, 7175, 7178, 7180, 7181, 7183, 7185, 7186, 7187, 7192, 7194, 7199, 7202, 7203, 7204, 7205, 7207, 7208, 7213, 7214, 7216, 7220, 7221, 7227, 7228, 7230, 7233, 7235, 7237, 7239, 7243, 7245, 7246, 7248, 7249, 7251, 7255, 7256, 7260, 7263, 7264, 7265, 7266, 7268, 7269, 7272, 7276, 7277, 7278, 7279, 7280, 7281, 7283, 7284, 7287, 7291, 7295, 7299, 7302, 7303, 7306, 7309, 7310, 7311, 7314, 7315, 7320, 7321, 7324, 7325, 7329, 7331, 7332, 7334, 7336, 7339, 7340, 7341, 7344, 7350, 7353, 7355, 7358, 7360, 7362, 7372, 7374, 7375, 7378, 7381, 7385, 7387, 7390, 7391, 7392, 7394, 7397, 7401, 7402, 7405, 7410, 7413, 7415, 7416, 7417, 7420, 6984, 6987, 6989, 6990, 6991, 6993, 6996, 6999, 7001, 7003, 7005, 7007, 7009, 7010, 7011, 7012, 7014, 7016, 7018, 7020, 7023, 7025, 7026, 7028, 7029, 7031, 7033, 7034, 7035, 7038, 7039, 7041, 7042, 7043, 7044, 7045, 7046, 7047, 7048, 7049, 7051, 7053, 7054, 7057, 7061, 7062, 7063, 7065, 7066, 7068, 7070, 7071, 7073, 7075, 7078, 7080, 7081, 7082, 7083, 7092, 7093, 7094, 7095, 7098, 7101, 7103, 7105, 7106, 7107, 7108, 7111, 7112, 7116, 7119, 7122, 7125, 7126, 7127, 7128, 7129, 7130, 7132, 7134, 7137, 7141, 7142, 7143, 7147, 7150, 7152, 7154, 7155, 7156, 7157, 7161, 7167, 7170, 7171, 7173, 7174, 7176, 7177, 7179, 7182, 7184, 7188, 7189, 7190, 7191, 7193, 7195, 7196, 7197, 7198, 7200, 7206, 7210, 7211, 7212, 7215, 7217, 7218, 7219, 7222, 7223, 7224, 7225, 7226, 7229, 7231, 7232, 7236, 7240, 7241, 7242, 7244, 7247, 7250, 7253, 7254, 7257, 7258, 7259, 7261, 7262, 7267, 7270, 7271, 7273, 7274, 7275, 7282, 7285, 7289, 7290, 7292, 7293, 7294, 7296, 7297, 7298, 7301, 7304, 7307, 7308, 7312, 7316, 7317, 7318, 7319, 7322, 7326, 7328, 7330, 7333, 7335, 7337, 7342, 7345, 7347, 7348, 7349, 7351, 7354, 7356, 7357, 7359, 7361, 7363, 7364, 7365, 7366, 7367, 7368, 7369, 7370, 7371, 7373, 7376, 7379, 7382, 7383, 7384, 7386, 7388, 7389, 7393, 7395, 7398, 7400, 7403, 7404, 7407, 7408, 7409, 7411, 7419, 7421, 7422, 7425, 7427, 7435, 7438, 7439, 7440, 7442, 7444, 7448, 7449, 7456, 7459, 7462, 7463, 7465, 7466, 7467, 7470, 7471, 7474, 7476, 7477, 7480, 7481, 7485, 7486, 7489, 7490, 7493, 7496, 7500, 7503, 7508, 7512, 7518, 7519, 7520, 7522, 7524, 7525, 7527, 7528, 7529, 7532, 7540, 7542, 7543, 7545, 7549, 7552, 7554, 7423, 7426, 7428, 7430, 7431, 7432, 7433, 7434, 7436, 7445, 7446, 7447, 7450, 7451, 7452, 7453, 7454, 7455, 7457, 7460, 7464, 7468, 7472, 7475, 7478, 7479, 7482, 7483, 7484, 7487, 7488, 7492, 7494, 7495, 7497, 7498, 7499, 7501, 7505, 7506, 7507, 7509, 7510, 7511, 7513, 7515, 7516, 7517, 7521, 7523, 7526, 7530, 7533, 7534, 7535, 7536, 7537, 7538, 7539, 7541, 7544, 7546, 7547, 7548, 7550, 7555, 7558, 7559, 7560, 7565, 7566, 7567, 7570, 7571, 7573, 7574, 7575, 7576, 7577, 7578, 7579, 7580, 7581, 7582, 7585, 7586, 7587, 7588, 7589, 7590, 7596, 7597, 7598, 7600, 7603, 7606, 7609, 7610, 7615, 7618, 7620, 7621, 7623, 7627, 7629, 7633, 7635, 7636, 7643, 7647, 7651, 7655, 7661, 7666, 7672, 7674, 7676, 7677, 7682, 7683, 7685, 7687, 7689, 7690, 7691, 7694, 7699, 7701, 7702, 7704, 7707, 7709, 7711, 7712, 7713, 7714, 7716, 7718, 7720, 7726, 7728, 7729, 7733, 7734, 7735, 7736, 7738, 7740, 7741, 7744, 7746, 7747, 7748, 7749, 7750, 7751, 7752, 7753, 7754, 7755, 7756, 7758, 7556, 7561, 7563, 7568, 7583, 7584, 7591, 7593, 7594, 7595, 7599, 7601, 7604, 7607, 7608, 7611, 7613, 7614, 7616, 7617, 7619, 7622, 7624, 7626, 7628, 7631, 7637, 7638, 7639, 7640, 7641, 7642, 7644, 7645, 7646, 7649, 7650, 7652, 7654, 7656, 7657, 7658, 7659, 7660, 7662, 7663, 7665, 7667, 7669, 7670, 7671, 7673, 7675, 7678, 7679, 7680, 7681, 7684, 7686, 7688, 7692, 7695, 7697, 7698, 7700, 7703, 7705, 7708, 7710, 7715, 7717, 7719, 7721, 7722, 7723, 7724, 7725, 7727, 7730, 7731, 7732, 7737, 7739, 7742, 7743, 7745, 7757, 7759, 7760, 7761, 7764, 7770, 7774, 7775, 7778, 7782, 7786, 7788, 7795, 7797, 7799, 7805, 7807, 7809, 7810, 7811, 7815, 7817, 7819, 7820, 7822, 7823, 7824, 7828, 7832, 7834, 7837, 7839, 7843, 7845, 7851, 7852, 7857, 7860, 7863, 7865, 7868, 7874, 7881, 7885, 7889, 7892, 7894, 7896, 7900, 7902, 7904, 7907, 7909, 7910, 7913, 7917, 7922, 7925, 7926, 7928, 7933, 7934, 7937, 7941, 7945, 7946, 7948, 7950, 7951, 7952, 7953, 7954, 7955, 7956, 7957, 7963, 7964, 7968, 7971, 7972, 7973, 7976, 7979, 7980, 7981, 7982, 7983, 7985, 7986, 7987, 7988, 7991, 7?62, 7765, 7767, 7768, 7771, 7773, 7776, 7779, 7781, 7783, 7784, 7785, 7789, 7791, 7792, 7793, 7794, 7796, 7798, 7800, 7802, 7803, 7808, 7812, 7813, 7814, 7816, 7818, 7821, 7825, 7827, 7829, 7830, 7833, 7835, 7836, 7838, 7840, 7841, 7842, 7844, 7846, 7847, 7848, 7849, 7853, 7854, 7855, 7856, 7858, 7861, 7864, 7866, 7869, 7870, 7871, 7872, 7873, 7875, 7877, 7878, 7879, 7880, 7882, 7884, 7886, 7888, 7890, 7897, 7899, 7903, 7905, 7912, 7914, 7916, 7918, 7919, 7920, 7921, 7923, 7929, 7931, 7932, 7936, 7938, 7939, 7940, 7942, 7943, 7944, 7947, 7949, 7958, 7960, 7962, 7965, 7967, 7969, 7970, 7974, 7977, 7989, 7990, 7992, 7995, 7996, 8000, 8003, 8006, 8007, 8010, 8012, 8015, 8016, 8018, 8019, 8026, 8027, 8030, 8034, 8035, 8038, 8039, 8043, 8044, 8046, 8049, 8050, 8052, 8053, 8055, 8056, 8062, 8064, 8066, 8067, 8068, 8069, 8070, 8071, 8074, 8076, 8077, 8078, 8079, 8080, 8081, 7993, 7997, 7999, 8001, 8004, 8005, 8008, 8009, 8011, 8013, 8017, 8020, 8021, 8022, 8023, 8024, 8025, 8028, 8031, 8033, 8036, 8040, 8042, 8047, 8051, 8054, 8057, 8059, 8060, 8061, 8065, 8072, 8073, 8075, 8082, 8083, 8084, 8085, 8087, 8088, 8089, 8090, 8093, 8095, 8098, 8100, 8101, 8102, 8104, 8107, 8110, 8111, 8114, 8115, 8116, 8118, 8122, 8124, 8126, 8127, 8129, 8132, 8134, 8136, 8138, 8142, 8143, 8144, 8146, 8151, 8157, 8160, 8163, 8165, 8168, 8169, 8170, 8173, 8174, 8175, 8176, 8178, 8181, 8185, 8187, 8188, 8189, 8190, 8191, 8193, 8194, 8200, 8203, 8206, 8208, 8209, 8212, 8214, 8216, 8217, 8220, 8222, 8224, 8228, 8230, 8091, 8094, 8096, 8099, 8103, 8105, 8108, 8112, 8113, 8117, 8119, 8121, 8123, 8125, 8128, 8130, 8131, 8133, 8135, 8137, 8139, 8145, 8147, 8148, 8149, 8150, 8152, 8153, 8154, 8155, 8156, 8158, 8161, 8164, 8166, 8167, 8171, 8177, 8179, 8180, 8182, 8183, 8184, 8186, 8192, 8195, 8197, 8198, 8199, 8201, 8202, 8204, 8207, 8210, 8213, 8218, 8221, 8223, 8225, 8227, 8229, 8231, 8232, 8235, 8236, 8237, 8238, 8244, 8245, 8247, 8250, 8251, 8253, 8254, 8255, 8256, 8258, 8259, 8260, 8263, 8267, 8269, 8270, 8273, 8274, 8277, 8280, 8281, 8289, 8292, 8293, 8294, 8295, 8296, 8297, 8298, 8301, 8305, 8306, 8310, 8311, 8312, 8315, 8317, 8318, 8322, 8323, 8324, 8327, 8328, 8329, 8330, 8333, 8336, 8339, 8340, 8347, 8349, 8350, 8352, 8353, 8356, 8359, 8360, 8366, 8369, 8370, 8372, 8374, 8376, 8377, 8379, 8381, 8382, 8383, 8233, 8239, 8240, 8242, 8243, 8246, 8248, 8252, 8257, 8261, 8264, 8266, 8268, 8271, 8275, 8278, 8282, 8284, 8285, 8286, 8287, 8288, 8291, 8299, 8302, 8303, 8304, 8307, 8309, 8313, 8314, 8316, 8319, 8321, 8325, 8326, 8331, 8332, 8334, 8335, 8337, 8341, 8342, 8343, 8344, 8345, 8346, 8348, 8351, 8354, 8357, 8361, 8362, 8363, 8364, 8365, 8367, 8371, 8373, 8375, 8378, 8384, 8387, 8390, 8391, 8397, 8398, 8401, 8402, 8405, 8406, 8409, 8414, 8418, 8419, 8422, 8425, 8428, 8431, 8432, 8433, 8435, 8437, 8440, 8444, 8449, 8452, 8454, 8455, 8457, 8462, 8464, 8466, 8468, 8469, 8472, 8475, 8476, 8478, 8479, 8481, 8484, 8487, 8488, 8490, 8493, 8496, 8497, 8498, 8501, 8505, 8508, 8511, 8513, 8515, 8517, 8518, 8519, 8521, 8522, 8523, 8526, 8528, 8530, 8531, 8532, 8534, 8536, 8541, 8544, 8547, 8551, 8554, 8556, 8558, 8560, 8562, 8563, 8566, 8567, 8568, 8570, 8571, 8574, 8575, 8576, 8577, 8580, 8581, 8585, 8588, 8591, 8594, 8601, 8602, 8603, 8608, 8609, 8613, 8614, 8617, 8618, 8619, 8620, 8623, 8624, 8628, 8629, 8630, 8633, 8634, 8635, 8636, 8638, 8643, 8645, 8648, 8654, 8657, 8658, 8659, 8660, 8661, 8662, 8666, 8670, 8672, 8674, 8676, 8681, 8683, 8686, 8688, 8689, 8691, 8692, 8693, 8695, 8696, 8700, 8701, 8702, 8703, 8704, 8705, 8709, 8716, 8717, 8719, 8722, 8725, 8727, 8728, 8729, 8732, 8733, 8734, 8735, 8736, 8385, 8388, 8392, 8394, 8395, 8399, 8403, 8407, 8410, 8411, 8412, 8413, 8415, 8416, 8420, 8423, 8424, 8426, 8429, 8434, 8436, 8438, 8442, 8445, 8446, 8447, 8448, 8450, 8451, 8456, 8458, 8460, 8461, 8463, 8465, 8467, 8470, 8473, 8474, 8477, 8480, 8482, 8485, 8486, 8489, 8491, 8492, 8494, 8499, 8500, 8502, 8503, 8504, 8506, 8510, 8512, 8514, 8516, 8520, 8524, 8527, 8529, 8533, 8535, 8537, 8539, 8542, 8545, 8546, 8548, 8550, 8552, 8555, 8557, 8564, 8565, 8569, 8572, 8578, 8582, 8583, 8586, 8587, 8589, 8592, 8595, 8596, 8597, 8598, 8599, 8600, 8604, 8606, 8607, 8610, 8612, 8615, 8616, 8621, 8625, 8627, 8631, 8632, 8637, 8639, 8640, 8641, 8642, 8644, 8646, 8647, 8649, 8650, 8651, 8652, 8653, 8655, 8663, 8665, 8667, 8668, 8669, 8671, 8673, 8675, 8677, 8678, 8679, 8680, 8682, 8684, 8685, 8687, 8690, 8694, 8697, 8698, 8699, 8706, 8707, 8708, 8710, 8711, 8712, 8713, 8714, 8715, 8718, 8720, 8723, 8726, 8730, 8737, 8738, 8741, 8744, 8747, 8750, 8751, 8753, 8754, 8755, 8756, 8759, 8760, 8762, 8763, 8766, 8767, 8768, 8769, 8770, 8772, 8775, 8776, 8777, 8779, 8782, 8783, 8784, 8785, 8786, 8787, 8788, 8789, 8790, 8793, 8794, 8796, 8798, 8799, 8801, 8803, 8804, 8806, 8807, 8809, 8812, 8813, 8815, 8820, 8823, 8826, 8829, 8830, 8832, 8835, 8836, 8837, 8842, 8845, 8846, 8847, 8851, 8853, 8856, 8857, 8859, 8861, 8866, 8739, 8878, 8879, 8880, 8884, 8891, 8893, 8895, 8898, 8900, 8901, 8909, 8912, 8739, 8742, 8745, 8748, 8749, 8752, 8757, 8758, 8761, 8764, 8771, 8773, 8778, 8780, 8791, 8797, 8800, 8802, 8805, 8808, 8810, 8814, 8816, 8818, 8819, 8821, 8824, 8827, 8828, 8831, 8833, 8838, 8840, 8841, 8755, 8843, 8844, 8848, 8850, 8852, 8854, 8855, 8863, 8864, 8865, 8867, 8868, 8869, 8870, 8871, 8872, 8873, 8874, 8876, 8877, 8881, 8882, 8883, 8885, 8887, 8888, 8889, 8890, 8892, 8894, 8896, 8897, 8899, 8902, 8785, 8903, 8904, 8905, 8906, 8907, 8908, 8911, 8913, 8917, 8920, 8923, 8924, 8929, 8931, 8934, 8935, 8936, 8914, 8916, 8918, 8921, 8925, 8927, 8928, 8930, 8932, 8933, 8937, 8980, 8984, 8988, 8989, 8998, 9001, 9002, 9005, 9007, 9008, 9012, 9013, 9016, 9019, 9020, 9022, 9023, 9025, 9028, 9029, 9032, 9033, 9034, 9035, 9037, 9038, 9044, 9046, 9047, 9048, 9049, 9054, 8981, 8983, 8985, 8987, 8990, 8992, 8993, 8994, 8995, 8997, 8999, 9003, 9004, 9006, 9009, 9010, 9011, 9014, 9017, 9018, 9021, 9024, 9026, 9030, 9036, 9039, 9041, 9042, 9043, 9045, 9050, 9051, 9052, 9053, 9055, 9058, 9060, 9063, 9066, 9067, 9068, 9069, 9070, 9071, 9074, 9075, 9077, 9078, 9079, 9080, 9082, 9084, 9088, 9089, 9090, 9099, 9103, 9104, 9105, 9107, 9111, 9056, 9061, 9064, 9072, 9081, 9083, 9085, 9086, 9087, 9091, 9092, 9093, 9094, 9095, 9096, 9097, 9098, 9100, 9102, 9106, 9108, 9109, 9110, 9112, 9113, 9116, 9118, 9125, 9126, 9128, 9129, 9130, 9136, 9141, 9143, 9148, 9150, 9153, 9157, 9158, 9159, 9163, 9164, 9165, 9167, 9168, 9171, 9173, 9177, 9180, 9181, 9182, 9183, 9185, 9187, 9188, 9189, 9191, 9192, 9193, 9194, 9197, 9198, 9200, 9206, 9207, 9208, 9209, 9211, 9216, 9218, 9219, 9220, 9221, 9222, 9223, 9226, 9228, 9229, 9230, 9231, 9232, 9233, 9234, 9237, 9238, 9240, 9243, 9246, 9248, 9253, 9256, 9257, 9260, 9263, 9264, 9266, 9269, 9271, 9279, 9280, 9281, 9282, 9283, 9284, 9290, 9114, 9119, 9121, 9122, 9123, 9124, 9127, 9131, 9133, 9134, 9135, 9137, 9138, 9139, 9140, 9142, 9144, 9146, 9147, 9149, 9151, 9152, 9154, 9155, 9156, 9160, 9162, 9166, 9169, 9172, 9174, 9175, 9176, 9178, 9184, 9186, 9190, 9195, 9199, 9201, 9202, 9203, 9204, 9205, 9212, 9214, 9215, 9217, 9224, 9227, 9235, 9239, 9241, 9244, 9247, 9249, 9251, 9252, 9254, 9258, 9261, 9265, 9267, 9270, 9273, 9274, 9275, 9276, 9277, 9278, 9285, 9286, 9287, 9288, 9289, 9291, 9292, 9295, 9297, 9300, 9302, 9303, 9304, 9307, 9308, 9310, 9311, 9313, 9319, 9321, 9323, 9324, 9328, 9333, 9334, 9337, 9338, 9339, 9342, 9344, 9348, 9351, 9352, 9354, 9355, 9357, 9360, 9367, 9371, 9374, 9377, 9379, 9383, 9384, 9385, 9388, 9389, 9390, 9393, 9394, 9398, 9401, 9402, 9406, 9408, 9409, 9410, 9413, 9415, 9416, 9417, 9418, 9419, 9421, 9422, 9425, 9429, 9433, 9434, 9435, 9436, 9437, 9439, 9440, 9446, 9452, 9453, 9454, 9455, 9457, 9458, 9459, 9462, 9466, 9467, 9468, 9470, 9474, 9479, 9482, 9487, 9488, 9489, 9492, 9495, 9497, 9499, 9500, 9504, 9505, 9508, 9513, 9514, 9519, 9522, 9523, 9524, 9526, 9528, 9532, 9533, 9534, 9535, 9537, 9538, 9541, 9542, 9543, 9545, 9546, 9548, 9293, 9296, 9298, 9301, 9305, 9309, 9312, 9314, 9316, 9317, 9318, 9320, 9322, 9325, 9326, 9327, 9329, 9331, 9332, 9335, 9336, 9340, 9345, 9347, 9349, 9353, 9356, 9358, 9361, 9363, 9364, 9365, 9368, 9370, 9372, 9375, 9376, 9378, 9380, 9382, 9386, 9391, 9395, 9397, 9399, 9400, 9403, 9405, 9407, 9411, 9412, 9414, 9420, 9423, 9424, 9426, 9428, 9430, 9431, 9432, 9438, 9441, 9443, 9444, 9445, 9447, 9448, 9449, 9450, 9451, 9456, 9460, 9461, 9464, 9465, 9469, 9471, 9473, 9475, 9477, 9478, 9480, 9483, 9485, 9490, 9493, 9494, 9496, 9498, 9501, 9502, 9503, 9506, 9509, 9511, 9512, 9515, 9516, 9517, 9518, 9520, 9521, 9525, 9527, 9529, 9530, 9536, 9539, 9544, 9547, 9549, 9550, 9551, 9555, 9556, 9557, 9559, 9560, 9563, 9564, 9565, 9566, 9569, 9570, 9573, 9577, 9578, 9579, 9580, 9585, 9586, 9587, 9588, 9591, 9593, 9596, 9552, 9554, 9558, 9561, 9567, 9571, 9574, 9576, 9581, 9583, 9584, 9589, 9592, 9594, 9595, 9598, 9602, 9604, 9605, 9608, 9612, 9613, 9614, 9617, 9618, 9621, 9622, 9625, 9626, 9627, 9632, 9633, 9638, 9640, 9643, 9644, 9645, 9646, 9651, 9654, 9656, 9657, 9658, 9661, 9662, 9665, 9666, 9667, 9668, 9672, 9673, 9675, 9676, 9677, 9680, 9683, 9686, 9688, 9691, 9694, 9698, 9699, 9702, 9705, 9707, 9710, 9711, 9715, 9719, 9720, 9722, 9723, 9726, 9729, 9732, 9733, 9735, 9737, 9738, 9741, 9744, 9745, 9746, 9747, 9748, 9749, 9750, 9754, 9755, 9756, 9758, 9759, 9768, 9771, 9772, 9776, 9777, 9781, 9782, 9784, 9787, 9789, 9790, 9792, 9796, 9797, 9800, 9805, 9806, 9812, 9815, 9818, 9821, 9823, 9825, 9827, 9831, 9832, 9835, 9837, 9839, 9841, 9842, 9844, 9845, 9851, 9853, 9854, 9855, 9857, 9860, 9862, 9863, 9865, 9871, 9873, 9875, 9878, 9879, 9599, 9601, 9603, 9606, 9607, 9609, 9611, 9615, 9619, 9623, 9624, 9628, 9630, 9631, 9634, 9635, 9637, 9639, 9641, 9647, 9649, 9650, 9652, 9653, 9659, 9660, 9663, 9664, 9670, 9671, 9679, 9682, 9684, 9687, 9690, 9692, 9693, 9695, 9697, 9700, 9701, 9703, 9706, 9708, 9712, 9713, 9714, 9716, 9717, 9718, 9721, 9724, 9727, 9730, 9734, 9739, 9742, 9751, 9753, 9757, 9760, 9761, 9762, 9763, 9764, 9765, 9766, 9767, 9769, 9773, 9775, 9778, 9780, 9783, 9786, 9788, 9791, 9793, 9794, 9795, 9798, 9801, 9803, 9804, 9807, 9808, 9809, 9810, 9811, 9813, 9816, 9817, 9819, 9820, 9822, 9826, 9828, 9830, 9833, 9836, 9838, 9840, 9843, 9846, 9847, 9848, 9849, 9850, 9852, 9856, 9858, 9859, 9861, 9864, 9866, 9867, 9868, 9869, 9870, 9872, 9876, 9877, 9880, 9881, 9882, 9886, 9890, 9891, 9894, 9900, 9902, 9904, 9908, 9911, 9912, 9915, 9917, 9919, 9923, 9925, 9928, 9935, 9936, 9940, 9943, 9949, 9953, 9954, 9956, 9957, 9958, 9962, 9963, 9964, 9968, 9969, 9975, 9978, 9981, 9988, 9989, 9990, 9991, 9992, 9995, 9997, 10011, 10012, 10014, 10017, 10018, 10031, 10032, 10033, 10034, 10035, 10037, 10041, 10042, 10046, 10050, 10054, 10058, 10059, 9883, 9885, 9887, 9889, 9892, 9893, 9895, 9896, 9897, 9898, 9899, 9901, 9903, 9905, 9907, 9909, 9913, 9914, 9916, 9918, 9920, 9921, 9922, 9924, 9926, 9929, 9931, 9932, 9933, 9934, 9937, 9938, 9939, 9941, 9942, 9944, 9945, 9946, 9947, 9948, 9950, 9952, 9955, 9959, 9960, 9961, 9966, 9967, 9970, 9972, 9973, 9974, 9976, 9979, 9980, 9982, 9983, 9984, 9985, 9986, 9987, 9993, 9994, 9996, 9998, 9999, 10000, 10001, 10002, 10003, 10004, 10005, 10006, 10007, 10008, 10009, 10010, 10013, 10015, 10016, 10019, 10020, 10021, 10022, 10023, 10025, 10026, 10027, 10028, 10030, 10036, 10038, 10040, 10043, 10044, 10045, 10047, 10048, 10049, 10051, 10053, 10055, 10056, 10057, 10060, 10061, 10064, 10067, 10068, 10070, 10071, 10072, 10074, 10079, 10081, 10083, 10088, 10091, 10095, 10096, 10097, 10101, 10103, 10105, 10106, 10109, 10110, 10111, 10113, 10116, 10118, 10120, 10122, 10125, 10126, 10127, 10131, 10134, 10137, 10138, 10144, 10147, 10149, 10151, 10152, 10155, 10156, 10159, 10160, 10169, 10172, 10174, 10175, 10178, 10181, 10186, 10188, 10189, 10190, 10191, 10192, 10196, 10199, 10203, 10204, 10210, 10211, 10216, 10218, 10221, 10224, 10225, 10226, 10229, 10230, 10231, 10232, 10234, 10239, 10241, 10244, 10252, 10253, 10257, 10258, 10259, 10261, 10266, 10272, 10276, 10062, 10065, 10069, 10075, 10077, 10078, 10080, 10082, 10084, 10086, 10087, 10089, 10090, 10092, 10093, 10094, 10098, 10099, 10100, 10102, 10104, 10107, 10112, 10114, 10117, 10119, 10121, 10123, 10124, 10129, 10130, 10132, 10133, 10135, 10139, 10140, 10141, 10142, 10143, 10145, 10148, 10150, 10153, 10154, 10157, 10161, 10163, 10164, 10165, 10166, 10167, 10170, 10173, 10176, 10177, 10179, 10182, 10183, 10184, 10187, 10193, 10194, 10195, 10197, 10198, 10200, 10201, 10202, 10205, 10206, 10207, 10209, 10212, 10213, 10214, 10215, 10219, 10220, 10222, 10223, 10227, 10228, 10233, 10235, 10236, 10237, 10240, 10242, 10243, 10245, 10247, 10248, 10249, 10251, 10254, 10255, 10256, 10260, 10262, 10263, 10264, 10265, 10267, 10268, 10269, 10270, 10271, 10273, 10274, 10275, 10277, 10278, 10279, 10282, 10285, 10287, 10291, 10292, 10297, 10299, 10301, 10304, 10306, 10308, 10310, 10314, 10317, 10318, 10320, 10322, 10327, 10329, 10330, 10333, 10335, 10336, 10337, 10340, 10342, 10348, 10350, 10351, 10354, 10358, 10359, 10360, 10362, 10366, 10369, 10372, 10373, 10374, 10376, 10378, 10383, 10385, 10391, 10398, 10403, 10407, 10410, 10413, 10415, 10419, 10422, 10425, 10427, 10428, 10429, 10430, 10432, 10433, 10434, 10437, 10438, 10439, 10442, 10443, 10445, 10446, 10447, 10448, 10452, 10453, 10454, 10455, 10456, 10460, 10463, 10466, 10467, 10469, 10470, 10473, 10475, 10477, 10480, 10484, 10488, 10490, 10494, 10495, 10497, 10501, 10502, 10503, 10507, 10508, 10510, 10517, 10518, 10520, 10522, 10524, 10525, 10528, 10529, 10533, 10536, 10537, 10540, 10541, 10545, 10547, 10551, 10553, 10556, 10559, 10560, 10563, 10564, 10565, 10568, 10571, 10574, 10576, 10579, 10581, 10583, 10588, 10589, 10590, 10592, 10593, 10596, 10597, 10601, 10602, 10603, 10605, 10607, 10609, 10610, 10612, 10613, 10617, 10618, 10621, 10624, 10627, 10630, 10632, 10634, 10637, 10638, 10639, 10640, 10641, 10643, 10645, 10647, 10653, 10655, 10657, 10659, 10661, 10662, 10663, 10664, 10665, 10666, 10667, 10668, 10669, 10672, 10673, 10674, 10675, 10280, 10283, 10286, 10288, 10290, 10293, 10295, 10298, 10300, 10302, 10305, 10307, 10309, 10311, 10312, 10313, 10315, 10319, 10321, 10323, 10325, 10326, 10328, 10331, 10338, 10341, 10343, 10344, 10345, 10346, 10347, 10349, 10352, 10355, 10356, 10357, 10361, 10363, 10365, 10367, 10370, 10375, 10377, 10379, 10380, 10381, 10382, 10384, 10386, 10387, 10388, 10390, 10392, 10394, 10395, 10399, 10401, 10402, 10404, 10406, 10408, 10411, 10412, 10414, 10416, 10417, 10418, 10420, 10423, 10426, 10431, 10435, 10440, 10444, 10449, 10451, 10457, 10459, 10461, 10462, 10464, 10465, 10468, 10471, 10476, 10478, 10481, 10483, 10485, 10487, 10489, 10491, 10492, 10493, 10498, 10499, 10500, 10504, 10506, 10509, 10511, 10513, 10514, 10515, 10516, 10519, 10521, 10523, 10526, 10530, 10531, 10532, 10534, 10535, 10538, 10539, 10542, 10543, 10544, 10546, 10548, 10550, 10552, 10554, 10557, 10558, 10561, 10566, 10569, 10572, 10573, 10577, 10578, 10580, 10582, 10584, 10586, 10594, 10598, 10600, 10604, 10606, 10611, 10614, 10616, 10619, 10622, 10625, 10626, 10628, 10631, 10633, 10635, 10642, 10644, 10646, 10648, 10649, 10650, 10651, 10652, 10654, 10656, 10658, 10660, 10670, 10671, 10676, 10678, 10684, 10679, 10681, 10682, 10683, 10685, 10687, 10688, 10689, 10690, 10691, 10693, 10696, 10699, 10703, 10704, 10709, 10711, 10712, 10713, 10720, 10721, 10722, 10724, 10725, 10726, 10728, 10729, 10734, 10740, 10742, 10743, 10744, 10745, 10746, 10748, 10749, 10751, 10754, 10758, 10759, 10760, 10764, 10768, 10769, 10772, 10775, 10780, 10781, 10783, 10786, 10791, 10796, 10801, 10808, 10817, 10818, 10819, 10821, 10823, 10826, 10827, 10829, 10830, 10834, 10843, 10844, 10847, 10848, 10849, 10853, 10856, 10858, 10866, 10869, 10872, 10874, 10877, 10878, 10879, 10882, 10884, 10885, 10889, 10892, 10893, 10896, 10900, 10901, 10903, 10905, 10907, 10909, 10910, 10911, 10914, 10916, 10919, 10926, 10930, 10932, 10933, 10934, 10938, 10941, 10942, 10943, 10944, 10946, 10947, 10953, 10955, 10956, 10958, 10959, 10962, 10967, 10968, 10972, 10975, 10977, 10978, 10981, 10984, 10985, 10988, 10992, 10995, 10997, 10999, 11000, 11001, 11006, 11009, 11013, 11016, 11020, 11025, 11033, 11036, 11040, 11041, 11042, 11043, 11045, 11046, 11048, 11052, 11055, 11060, 11061, 11062, 11064, 11065, 11067, 11070, 11071, 11072, 11078, 11079, 11081, 11085, 11093, 10694, 10697, 10700, 10702, 10705, 10707, 10710, 10714, 10716, 10717, 10718, 10719, 10723, 10727, 10730, 10732, 10733, 10735, 10737, 10738, 10739, 10741, 10747, 10750, 10752, 10755, 10757, 10761, 10762, 10763, 10765, 10767, 10770, 10773, 10776, 10778, 10779, 10782, 10784, 10788, 10789, 10790, 10792, 10793, 10794, 10798, 10799, 10800, 10802, 10804, 10805, 10806, 10807, 10809, 10811, 10812, 10813, 10814, 10815, 10816, 10820, 10822, 10824, 10831, 10833, 10835, 10837, 10838, 10839, 10840, 10841, 10842, 10845, 10846, 10850, 10851, 10854, 10857, 10859, 10860, 10861, 10862, 10863, 10864, 10865, 10868, 10870, 10871, 10873, 10875, 10880, 10881, 10883, 10886, 10888, 10890, 10894, 10897, 10899, 10902, 10904, 10906, 10908, 10912, 10915, 10917, 10920, 10922, 10924, 10925, 10927, 10928, 10929, 10931, 10935, 10937, 10939, 10945, 10948, 10950, 10951, 10952, 10954, 10957, 10960, 10963, 10965, 10966, 10969, 10970, 10971, 10973, 10976, 10979, 10980, 10982, 10986, 10987, 10989, 10991, 10993, 10996, 10998, 11002, 11004, 11005, 11007, 11010, 11012, 11014, 11015, 11017, 11019, 11021, 11022, 11023, 11026, 11027, 11028, 11029, 11030, 11031, 11032, 11034, 11037, 11039, 11044, 11047, 11049, 11050, 11051, 11053, 11054, 11056, 11058, 11059, 11063, 11066, 11068, 11069, 11073, 11074, 11075, 11076, 11077, 11080, 11082, 11083, 11084, 11086, 11087, 11088, 11089, 11090, 11091, 11092, 11094, 11376, 11381, 11382, 11383, 11384, 11387, 11397, 11401, 11405, 11408, 11414, 11418, 11421, 11422, 11424, 11427, 11429, 11430, 11377, 11379, 11380, 11385, 11386, 11388, 11390, 11391, 11392, 11394, 11395, 11396, 11398, 11399, 11400, 11402, 11403, 11404, 11406, 11409, 11410, 11411, 11412, 11415, 11416, 11417, 11419, 11423, 11425, 11428, 11431, 11432, 11433, 11434, 11438, 11442, 11443, 11447, 11449, 11450, 11452, 11456, 11459, 11463, 11464, 11467, 11468, 11472, 11477, 11480, 11481, 11482, 11483, 11484, 11488, 11489, 11493, 11498, 11502, 11504, 11505, 11506, 11507, 11508, 11509, 11512, 11513, 11514, 11517, 11520, 11522, 11523, 11524, 11526, 11528, 11532, 11534, 11435, 11439, 11444, 11445, 11446, 11448, 11451, 11453, 11457, 11458, 11460, 11461, 11462, 11465, 11466, 11469, 11473, 11474, 11475, 11476, 11478, 11479, 11485, 11486, 11487, 11490, 11494, 11497, 11499, 11500, 11501, 11503, 11510, 11511, 11515, 11518, 11519, 11521, 11525, 11527, 11529, 11535, 11539, 11540, 11541, 11548, 11550, 11551, 11552, 11555, 11556, 11557, 11560, 11561, 11562, 11568, 11569, 11571, 11572, 11573, 11577, 11536, 11542, 11545, 11546, 11547, 11549, 11553, 11554, 11563, 11566, 11570, 11574, 11578, 11585, 11586, 11590, 11591, 11592, 11595, 11596, 11597, 11600, 11579, 11582, 11587, 11593, 11594, 11598, 11601, 11602, 11606, 11607, 11608, 11609, 11616, 11618, 11622, 11626, 11628, 11632, 11633, 11603, 11610, 11613, 11614, 11615, 11617, 11619, 11623, 11627, 11629, 11634, 11635, 11640, 11641, 11642, 11643, 11647, 11648, 11654, 11655, 11656, 11657, 11658, 11636, 11639, 11644, 11645, 11646, 11649, 11652, 11653, 11659, 11663, 11664, 11665, 11666, 11670, 11671, 11672, 11673, 11677, 11681, 11687, 11690, 11694, 11696, 11697, 11701, 11705, 11709, 11713, 11719, 11723, 11729, 11733, 11739, 11744, 11745, 11748, 11749, 11751, 11755, 11759, 11763, 11770, 11772, 11774, 11776, 11778, 11782, 11785, 11660, 11667, 11674, 11675, 11676, 11678, 11682, 11685, 11686, 11688, 11689, 11691, 11692, 11693, 11695, 11698, 11699, 11700, 11702, 11706, 11710, 11716, 11720, 11724, 11725, 11727, 11728, 11730, 11731, 11732, 11734, 11735, 11736, 11737, 11738, 11740, 11743, 11746, 11747, 11750, 11752, 11756, 11761, 11762, 11764, 11765, 11767, 11768, 11769, 11773, 11775, 11777, 11779, 11783, 11784, 11786, 11790, 11791, 11792, 11787, 11793, 11796, 11800, 11802, 11803, 11805, 11810, 11818, 11819, 11823, 11824, 11827, 11828, 11829, 11833, 11836, 11837, 11839, 11840, 11847, 11850, 11852, 11857, 11860, 11864, 11866, 11797, 11801, 11804, 11806, 11809, 11811, 11814, 11815, 11817, 11820, 11825, 11830, 11835, 11841, 11844, 11845, 11846, 11848, 11851, 11853, 11856, 11858, 11861, 11862, 11863, 11865, 11867, 11871, 11876, 11878, 11882, 11888, 11891, 11894, 11868, 11872, 11875, 11877, 11879, 11883, 11886, 11887, 11889, 11890, 11892, 11893, 11895, 11898, 11902, 11903, 11904, 11908, 11909, 11911, 11912, 11913, 11914, 11915, 11917, 11918, 11922, 11926, 11927, 11930, 11932, 11933, 11937, 11941, 11942, 11946, 11947, 11948, 11949, 11950, 11951, 11952, 11955, 11959, 11968, 11972, 11976, 11978, 11979, 11980, 11981, 11986, 11988, 11989, 11991, 11992, 11995, 11997, 11998, 12001, 12002, 12004, 12005, 11899, 11905, 11910, 11916, 11919, 11923, 11924, 11925, 11928, 11929, 11931, 11934, 11938, 11943, 11953, 11954, 11956, 11960, 11963, 11964, 11967, 11969, 11973, 11977, 11982, 11985, 11987, 11990, 11993, 11994, 11996, 11999, 12000, 12003, 12006, 12011, 12015, 12016, 12017, 12021, 12022, 12023, 12024, 12025, 12026, 12027, 12031, 12034, 12042, 12043, 12045, 12049, 12050, 12051, 12054, 12062, 12063, 12064, 12066, 12067, 12068, 12070, 12072, 12076, 12082, 12085, 12086, 12089, 12090, 12093, 12094, 12095, 12096, 12097, 12098, 12101, 12103, 12107, 12108, 12111, 12112, 12114, 12118, 12119, 12120, 12128, 12007, 12010, 12012, 12020, 12028, 12032, 12033, 12035, 12038, 12039, 12044, 12046, 12052, 12055, 12056, 12057, 12058, 12059, 12060, 12061, 12065, 12069, 12071, 12073, 12077, 12080, 12081, 12084, 12087, 12088, 12091, 12099, 12100, 12102, 12104, 12109, 12110, 12113, 12115, 12121, 12122, 12123, 12124, 12125, 12127, 12129, 12130, 12135, 12136, 12141, 12144, 12147, 12148, 12149, 12153, 12159, 12160, 12131, 12134, 12137, 12138, 12139, 12140, 12142, 12143, 12145, 12146, 12150, 12154, 12157, 12158, 12161, 12162, 12163, 12164, 12168, 12171, 12176, 12181, 12187, 12190, 12194, 12196, 12197, 12199, 12201, 12202, 12209, 12213, 12216, 12220, 12221, 12223, 12225, 12226, 12231, 12233, 12239, 12243, 12244, 12250, 12254, 12256, 12257, 12259, 12266, 12270, 12271, 12272, 12274, 12276, 12277, 12278, 12280, 12284, 12285, 12288, 12289, 12290, 12291, 12295, 12296, 12302, 12308, 12313, 12317, 12321, 12322, 12323, 12324, 12325, 12327, 12328, 12330, 12331, 12332, 12336, 12337, 12339, 12343, 12346, 12349, 12353, 12354, 12355, 12359, 12361, 12363, 12367, 12369, 12370, 12372, 12376, 12377, 12379, 12380, 12381, 12382, 12383, 12385, 12389, 12393, 12398, 12400, 12402, 12403, 12406, 12408, 12409, 12410, 12412, 12415, 12417, 12419, 12422, 12424, 12429, 12430, 12432, 12433, 12435, 12436, 12437, 12441, 12445, 12448, 12449, 12454, 12458, 12459, 12460, 12165, 12169, 12170, 12172, 12175, 12177, 12180, 12182, 12185, 12186, 12188, 12191, 12195, 12198, 12200, 12203, 12206, 12207, 12208, 12210, 12214, 12217, 12222, 12224, 12227, 12228, 12229, 12230, 12232, 12234, 12237, 12238, 12240, 12241, 12242, 12245, 12248, 12249, 12251, 12255, 12258, 12260, 12262, 12263, 12264, 12265, 12267, 12268, 12269, 12273, 12279, 12281, 12286, 12287, 12292, 12297, 12300, 12303, 12306, 12307, 12309, 12312, 12314, 12318, 12326, 12333, 12338, 12340, 12341, 12342, 12345, 12347, 12348, 12352, 12356, 12360, 12362, 12364, 12371, 12373, 12378, 12386, 12390, 12394, 12397, 12401, 12404, 12405, 12411, 12413, 12418, 12420, 12423, 12425, 12428, 12438, 12439, 12440, 12442, 12443, 12446, 12450, 12453, 12455, 12461, 12465, 12467, 12468, 12472, 12474, 12477, 12480, 12484, 12486, 12488, 12489, 12490, 12493, 12495, 12499, 12501, 12504, 12506, 12462, 12469, 12475, 12478, 12481, 12483, 12485, 12487, 12492, 12494, 12496, 12497, 12498, 12500, 12502, 12505, 12507, 12511, 12517, 12520, 12522, 12523, 12524, 12528, 12530, 12534, 12535, 12536, 12537, 12541, 12542, 12547, 12550, 12551, 12552, 12554, 12555, 12560, 12564, 12565, 12569, 12571, 12574, 12579, 12581, 12586, 12590, 12592, 12593, 12594, 12596, 12600, 12601, 12602, 12604, 12610, 12613, 12614, 12615, 12616, 12620, 12626, 12630, 12508, 12512, 12515, 12518, 12521, 12525, 12529, 12531, 12538, 12543, 12546, 12548, 12549, 12553, 12556, 12559, 12561, 12566, 12570, 12572, 12573, 12575, 12578, 12582, 12585, 12587, 12595, 12597, 12599, 12603, 12605, 12608, 12609, 12611, 12617, 12618, 12621, 12624, 12625, 12627, 12631, 12636, 12637, 12638, 12639, 12643, 12647, 12648, 12652, 12654, 12660, 12664, 12666, 12670, 12672, 12675, 12677, 12680, 12686, 12691, 12692, 12695, 12699, 12700, 12701, 12703, 12705, 12709, 12710, 12714, 12715, 12716, 12718, 12719, 12720, 12724, 12725, 12728, 12732, 12738, 12739, 12743, 12744, 12745, 12747, 12748, 12752, 12757, 12763, 12764, 12766, 12767, 12768, 12771, 12776, 12777, 12781, 12785, 12787, 12788, 12791, 12792, 12796, 12797, 12800, 12804, 12808, 12813, 12815, 12816, 12817, 12822, 12825, 12828, 12831, 12833, 12834, 12837, 12839, 12841, 12842, 12632, 12635, 12640, 12641, 12644, 12649, 12653, 12655, 12658, 12661, 12662, 12663, 12665, 12667, 12671, 12673, 12678, 12679, 12681, 12682, 12683, 12684, 12685, 12687, 12688, 12689, 12690, 12693, 12694, 12696, 12702, 12704, 12706, 12712, 12713, 12717, 12721, 12726, 12729, 12730, 12731, 12733, 12736, 12737, 12740, 12746, 12749, 12750, 12751, 12753, 12754, 12755, 12756, 12758, 12760, 12761, 12762, 12765, 12769, 12770, 12772, 12775, 12778, 12782, 12786, 12789, 12790, 12793, 12794, 12795, 12798, 12799, 12801, 12805, 12809, 12812, 12814, 12818, 12821, 12823, 12824, 12826, 12827, 12832, 12835, 12836, 12838, 12840, 12843, 12844, 12845, 12849, 12850, 12854, 12857, 12862, 12866, 12867, 12868, 12870, 12872, 12880, 12881, 12884, 12885, 12889, 12890, 12892, 12896, 12898, 12899, 12903, 12907, 12910, 12913, 12919, 12920, 12921, 12923, 12928, 12932, 12938, 12942, 12945, 12948, 12950, 12951, 12953, 12959, 12846, 12851, 12855, 12858, 12861, 12863, 12869, 12871, 12873, 12876, 12877, 12878, 12879, 12882, 12883, 12886, 12887, 12888, 12891, 12893, 12894, 12895, 12900, 12901, 12902, 12904, 12908, 12909, 12911, 12912, 12914, 12917, 12918, 12924, 12927, 12929, 12933, 12936, 12937, 12939, 12943, 12949, 12954, 12957, 12958, 12960, 12961, 12965, 12969, 12975, 12977, 12962, 12966, 12967, 12968, 12970, 12971, 12972, 12973, 12974, 12976, 12978, 12981, 12982, 12986, 12989, 12991, 12992, 12993, 12995, 12999, 13001, 12983, 12987, 12996, 12997, 12998, 13000, 13002, 13005, 13009, 13013, 13017, 13019, 13025, 13029, 13030, 13032, 13033, 13034, 13037, 13040, 13042, 13043, 13044, 13048, 13049, 13051, 13052, 13053, 13057, 13062, 13064, 13065, 13066, 13067, 13072, 13073, 13076, 13078, 13082, 13083, 13085, 13087, 13088, 13089, 13092, 13096, 13100, 13101, 13102, 13105, 13108, 13109, 13110, 13114, 13117, 13118, 13124, 13129, 13131, 13132, 13138, 13144, 13146, 13147, 13148, 13152, 13156, 13159, 13164, 13167, 13168, 13170, 13171, 13172, 13173, 13174, 13184, 13185, 13006, 13010, 13014, 13018, 13020, 13021, 13022, 13023, 13024, 13026, 13031, 13035, 13036, 13038, 13039, 13041, 13045, 13050, 13054, 13058, 13061, 13063, 13068, 13069, 13070, 13071, 13074, 13075, 13077, 13079, 13084, 13086, 13090, 13091, 13093, 13097, 13104, 13107, 13111, 13115, 13119, 13121, 13122, 13123, 13125, 13126, 13127, 13128, 13130, 13133, 13137, 13139, 13142, 13143, 13145, 13149, 13153, 13157, 13158, 13160, 13163, 13165, 13166, 13169, 13175, 13176, 13177, 13178, 13179, 13180, 13181, 13182, 13183, 13186, 13189, 13190, 13191, 13195, 13196, 13197, 13199, 13200, 13204, 13206, 13192, 13198, 13201, 13207, 13212, 13213, 13226, 13228, 13231, 13235, 13240, 13245, 13247, 13248, 13250, 13254, 13256, 13258, 13264, 13265, 13268, 13269, 13273, 13274, 13277, 13279, 13280, 13281, 13286, 13208, 13211, 13214, 13217, 13220, 13222, 13224, 13225, 13227, 13229, 13232, 13233, 13234, 13237, 13239, 13241, 13242, 13244, 13246, 13249, 13251, 13255, 13257, 13259, 13262, 13263, 13266, 13267, 13270, 13275, 13276, 13282, 13285, 13641, 13645, 13646, 13649, 13650, 13652, 13653, 13654, 13655, 13658, 13659, 13642, 13647, 13648, 13656, 13660, 13661, 13664, 13669, 13670, 13671, 13672, 13673, 13675, 13676, 13677, 13681, 13683, 13685, 13687, 13689, 13691, 13693, 13696, 13665, 13668, 13674, 13678, 13682, 13684, 13686, 13688, 13690, 13692, 13694, 13695, 13697, 13701, 13702, 13703, 13709, 13713, 13714, 13715, 13719, 13721, 13722, 13726, 13728, 13732, 13735, 13740, 13698, 13704, 13705, 13706, 13707, 13708, 13710, 13716, 13717, 13718, 13720, 13723, 13724, 13725, 13727, 13729, 13733, 13734, 13736, 13739, 13741, 13744, 13745, 13749, 13751, 13752, 13753, 13754, 13758, 13762, 13765, 13766, 13768, 13772, 13773, 13774, 13777, 13778, 13779, 13783, 13785, 13790, 13791, 13797, 13802, 13803, 13805, 13806, 13807, 13809, 13812, 13814, 13816, 13817, 13818, 13819, 13820, 13823, 13824, 13826, 13828, 13746, 13755, 13759, 13763, 13764, 13767, 13769, 13775, 13776, 13780, 13784, 13786, 13788, 13789, 13792, 13795, 13796, 13798, 13800, 13804, 13808, 13813, 13815, 13821, 13822, 13829, 13832, 13837, 13841, 13844, 13845, 13846, 13849, 13854, 13855, 13856, 13857, 13861, 13865, 13869, 13871, 13875, 13879, 13880, 13833, 13836, 13838, 13843, 13847, 13850, 13853, 13858, 13862, 13866, 13870, 13872, 13876, 13881, 13882, 13883, 13889, 13894, 13902, 13903, 13910, 13911, 13914, 13915, 13916, 13928, 13929, 13930, 13931, 13935, 13936, 13937, 13944, 13947, 13948, 13950, 13960, 13961, 13962, 13964, 13965, 13966, 13968, 13970, 13971, 13972, 13976, 13978, 13884, 13887, 13890, 13893, 13895, 13898, 13900, 13904, 13905, 13906, 13907, 13908, 13909, 13912, 13913, 13917, 13920, 13921, 13922, 13923, 13924, 13925, 13926, 13932, 13938, 13941, 13942, 13943, 13945, 13946, 13949, 13953, 13954, 13956, 13957, 13958, 13959, 13963, 13967, 13969, 13974, 13975, 13977, 13980, 13981, 13988, 13989, 13990, 13992, 13994, 13995, 13999, 13982, 13985, 13986, 13987, 13991, 13993, 13996, 14000, 14001, 14002, 14003, 14004, 14009, 14014, 14017, 14020, 14024, 14026, 14029, 14035, 14036, 14040, 14043, 14045, 14051, 14057, 14058, 14059, 14063, 14065, 14066, 14067, 14068, 14069, 14070, 14071, 14073, 14074, 14078, 14079, 14080, 14081, 14082, 14083, 14084, 14085, 14087, 14094, 14095, 14100, 14108, 14109, 14113, 14114, 14118, 14120, 14121, 14122, 14130, 14133, 14138, 14141, 14142, 14143, 14147, 14005, 14008, 14010, 14013, 14015, 14018, 14021, 14025, 14027, 14028, 14030, 14033, 14034, 14037, 14038, 14039, 14041, 14042, 14044, 14046, 14049, 14050, 14052, 14055, 14056, 14060, 14064, 14072, 14077, 14086, 14088, 14089, 14090, 14091, 14092, 14093, 14096, 14097, 14098, 14099, 14101, 14102, 14103, 14104, 14105, 14106, 14107, 14110, 14115, 14123, 14126, 14127, 14132, 14134, 14135, 14136, 14137, 14139, 14140, 14144, 14145, 14148, 14151, 14155, 14157, 14161, 14166, 14171, 14175, 14183, 14187, 14190, 14192, 14194, 14195, 14196, 14200, 14201, 14152, 14156, 14158, 14162, 14165, 14167, 14170, 14172, 14176, 14179, 14180, 14182, 14184, 14185, 14186, 14188, 14189, 14191, 14193, 14197, 14198, 14199, 14202, 14203, 14204, 14210, 14211, 14216, 14217, 14223, 14226, 14228, 14229, 14230, 14233, 14235, 14239, 14241, 14243, 14244, 14250, 14254, 14255, 14256, 14257, 14260, 14261, 14262, 14267, 14268, 14269, 14273, 14274, 14277, 14281, 14284, 14286, 14289, 14292, 14293, 14297, 14299, 14301, 14304, 14305, 14306, 14312, 14316, 14320, 14322, 14324, 14328, 14329, 14331, 14337, 14342, 14344, 14346, 14347, 14349, 14350, 14353, 14356, 14361, 14363, 14364, 14365, 14366, 14205, 14208, 14212, 14215, 14218, 14221, 14224, 14225, 14227, 14231, 14236, 14240, 14242, 14245, 14246, 14247, 14251, 14263, 14266, 14270, 14278, 14282, 14283, 14285, 14287, 14290, 14291, 14294, 14298, 14300, 14302, 14303, 14307, 14310, 14313, 14317, 14323, 14325, 14327, 14330, 14334, 14338, 14340, 14341, 14345, 14348, 14351, 14352, 14354, 14355, 14357, 14358, 14359, 14360, 14367, 14368, 14372, 14373, 14375, 14386, 14369, 14374, 14376, 14379, 14380, 14381, 14382, 14383, 14384, and SEQ ID NO:14385.

Step e) of the method, namely the detection of the specific amplificates indicative of the methylation status of one or more CpG positions according to SEQ ID NOS:1 to SEQ ID NO:64 is carried out by means of *real-time* detection methods as described above, and wherein the sequence of said hybridization probes is selected from the group consisting of SEQ ID NOS:2753, 2756, 2758, 2761, 2766, 2770, 2774, 2778, 2799, 2807, 2813, 2819, 2822, 2826, 2848, 2857, 2900, 2906, 2908, 2910, 2913, 2921, 2925, 2940, 2943, 2952, 2964, 2983, 2986, 3009, 3012, 3015, 3024, 3030, 3035, 3046, 3058, 3062, 3067, 3070, 3074, 3077, 3079, 3082, 3087, 3095, 3099, 3102, 3106, 3112, 3120, 3125, 3129, 3132, 3141, 3143, 3154, 3156, 3158, 3161, 3177, 3188, 3191, 3193, 3200, 3208, 3211, 3220, 3231, 3241, 3251, 3254, 3258, 3263, 3272, 3277, 3288, 3294, 3296, 3299, 3303, 3317, 3323, 3326, 3329, 3336, 3341, 3362, 3366, 3370, 3375, 3379, 3382, 3387, 3394, 3398, 3405, 3407, 3410, 3412, 3416, 3422, 3425, 3430, 3449, 3451, 3457, 3460, 3465, 3468, 3486, 3498, 3502, 3507, 3523, 3532, 3539, 3546, 3550, 3567, 3572, 3579, 3582, 3610, 3620, 3628, 3648, 3659, 3664, 3672, 3675, 3680, 3682, 3685, 3687, 3699, 3712, 3716, 3720, 3734, 3751, 3763, 3766, 3769, 3783, 3789, 3795, 3811, 3816, 3825, 3830, 3835, 3841, 3843, 3846, 3849, 3855, 3859, 3879, 3882, 3910, 3912, 3913, 3918, 3925, 3929, 3932, 3946, 3953, 3956, 3967, 3969, 3973, 3977, 3989, 3992, 3996, 3999, 4002, 4008, 4014, 4026, 4030, 4036, 4041, 4044, 4053, 4057, 4060, 4062, 4081, 4091, 4097, 4103, 4107, 4118, 4126, 4140, 4143, 4147, 4155, 4158, 4164, 4175, 4178, 4183, 4187, 4193, 4200, 4203, 4210, 4212, 4215, 4222, 4227, 4230, 4235, 4240, 4250, 4253, 4264, 4278, 4294, 4312, 4314, 4320, 4329, 4339, 4346, 4356, 4363, 4380, 4402, 4404, 4414, 4419, 4421, 4431, 4439, 4445, 4449, 4459, 4468, 4483, 4493, 4500, 4513, 4526, 4527, 4532, 4544, 4550, 4552, 4566, 4577, 4591, 4594, 4604, 4608, 4623, 4625, 4629, 4637, 4651, 4654, 4657, 4673, 4679, 4682, 4692, 4698, 4710, 4712, 4713, 4723, 4725, 4729, 4734, 4740, 4743, 4747, 4752, 4757, 4762, 4765, 4775, 4781, 4796, 4799, 4803, 4809, 4826, 4831, 4863, 4866, 4880, 4888, 4914, 4916, 4931, 4933, 4939, 4955, 4959, 4962, 4965, 4994, 5001, 5008, 5012, 5023, 5029, 5035, 5046, 5059, 5063, 5068, 5071, 5079, 5082, 5085, 5089, 5092, 5095, 5098, 5102, 5108, 5113, 5118, 5122, 5127, 5130, 5133, 5138, 5141, 5146, 5152, 5154, 5178, 5192, 5197, 5206, 5213, 5228, 5236, 5243, 5258, 5267, 5275, 5277, 5283, 5294, 5298, 5302, 5305, 5320, 5325, 5328, 5335, 5345, 5350, 5357, 5374, 5381, 5384, 5386, 5390, 5397, 5402, 5406, 5411, 5423, 5430, 5439, 5453, 5463, 5473, 5476, 5479, 5483, 5489, 5514, 5520, 5523, 5530, 5547, 5584, 5590, 5607, 5610, 5613, 5617, 5621, 5631, 5636, 5641, 5653, 5655, 5658, 5666, 5669, 5675, 5685, 5692, 5695, 5703, 5706, 5716, 5719, 5726, 5738, 5746, 5749, 5752, 5755, 5757, 5761, 5765, 5772, 5784, 5799, 5805, 5815, 5819, 5829, 5851, 5869, 5901, 5908, 5911, 5914, 5917, 5925, 5941, 5951, 5967, 5974, 5979, 5982, 5985, 5989, 5995, 6022, 6029, 6044, 6047, 6052, 6057, 6061, 6069, 6071, 6080, 6082, 6087, 6092, 6095, 6102, 6104, 6107, 6109, 6113, 6124, 6129, 6140, 6147, 6158, 6165, 6179, 6185, 6203, 6209, 6217, 6228, 6249, 6263, 6267, 6279, 6290, 6332, 6339, 6343, 6346, 6352, 6363, 6366, 6370, 6380, 6385, 6391, 6396, 6408, 6430, 6439, 6454, 6456, 6477, 6483, 6488, 6495, 6509, 6517, 6520, 6530, 6534, 6555, 6557, 6559, 6569, 6574, 6581, 6593, 6616, 6622, 6626, 6636, 6637, 6648, 6654, 6667, 6673, 6684, 6724, 6729, 6733, 6737, 6743, 6750, 6753, 6763, 6766, 6768, 6771, 6776, 6780, 6788, 6793, 6808, 6813, 6820, 6823, 6826, 6846, 6856, 6864, 6867, 6878, 6885, 6903, 6914, 6919, 6923, 6926, 6938, 6941, 6978, 6982, 6985, 6988, 6994, 6997, 7000, 7004, 7008, 7015, 7019, 7021, 7069, 7079, 7099, 7104, 7117, 7120, 7145, 7151, 7153, 7162, 7164, 7169, 7201, 7209, 7234, 7238, 7252, 7286, 7288, 7300, 7305, 7313, 7323, 7327, 7338, 7343, 7346, 7352, 7377, 7380, 7396, 7399, 7406, 7412, 7414, 7418, 7424, 7429, 7437, 7441, 7443, 7458, 7461, 7469, 7473, 7491, 7502, 7504, 7514, 7531, 7551, 7553, 7557, 7562, 7564, 7569, 7572, 7592, 7602, 7605, 7612, 7625, 7630, 7632, 7634, 7648, 7653, 7664, 7668, 7693, 7696, 7706, 7763, 7766, 7769, 7772, 7777, 7780, 7787, 7790, 7801, 7804, 7806, 7826, 7831, 7850, 7859, 7862, 7867, 7876, 7883, 7887, 7891, 7893, 7895, 7898, 7901, 7906, 7908, 7911, 7915, 7924, 7927, 7930, 7935, 7959, 7961, 7966, 7975, 7978, 7984, 7994, 7998, 8002, 8014, 8029, 8032, 8037, 8041, 8045, 8048, 8058, 8063, 8086, 8092, 8097, 8106, 8109, 8120, 8140, 8141, 8159, 8162, 8172, 8196, 8205, 8211, 8215, 8219, 8226, 8234, 8241, 8249, 8262, 8265, 8272, 8276, 8279, 8283, 8290, 8300, 8308, 8320, 8338, 8355, 8358, 8368, 8380,8386, 8389, 8393, 8396, 8400, 8404, 8408, 8417, 8421, 8427, 8430, 8439, 8441, 8443, 8453, 8459, 8471, 8483, 8495, 8507, 8509, 8525, 8538, 8540, 8543, 8549, 8553, 8559, 8561, 8573, 8579, 8584, 8590, 8593, 8605, 8611, 8622, 8626, 8656, 8664, 8721, 8724, 8731, 8740, 8743, 8746, 8765, 8774, 8781, 8792, 8795, 8811, 8817, 8822, 8825, 8834, 8839, 8849, 8858, 8860, 8862, 8875, 8886, 8910, 8915, 8919, 8922, 8926, 8940, 8947, 8956, 8959, 8973, 8982, 8986, 8991, 8996, 9000, 9015, 9027, 9031, 9040, 9057, 9059, 9062, 9065, 9073, 9076, 9101, 9115, 9117, 9120, 9132, 9145, 9161, 9170, 9179, 9196, 9210, 9213, 9225, 9236, 9242, 9245, 9250, 9255, 9259, 9262, 9268, 9272, 9294, 9299, 9306, 9315, 9330, 9341, 9343, 9346, 9350, 9359, 9362, 9366, 9369, 9373, 9381, 9387, 9392, 9396, 9404, 9427, 9442, 9463, 9472, 9476, 9481, 9484, 9486, 9491, 9507, 9510, 9531, 9540, 9553, 9562, 9568, 9572, 9575, 9582, 9590, 9597, 9600, 9610, 9616, 9620, 9629, 9636, 9642, 9648, 9655, 9669, 9674, 9678, 9681, 9685, 9689, 9696, 9704, 9709, 9725, 9728, 9731, 9736, 9740, 9743, 9752, 9770, 9774, 9779, 9785, 9799, 9802, 9814, 9824, 9829, 9834, 9874, 9884, 9888, 9906, 9910, 9927, 9930, 9951, 9965, 9971, 9977, 10024, 10029, 10039, 10052, 10063, 10066, 10073, 10076, 10085, 10108, 10115, 10128, 10136, 10146, 10158, 10162, 10168, 10171, 10180, 10185, 10208, 10217, 10238, 10246, 10250, 10281, 10284, 10289, 10294, 10296, 10303, 10316, 10324, 10332, 10334, 10339, 10353, 10364, 10368, 10371, 10389, 10393, 10396, 10397, 10400, 10405, 10409, 10421, 10424, 10436, 10441, 10450, 10458, 10472, 10474, 10479, 10482, 10486, 10496, 10505, 10512, 10527, 10549, 10555, 10562, 10567, 10570, 10575, 10585, 10587, 10591, 10595, 10599, 10608, 10615, 10620, 10623, 10629, 10636, 10677, 10680, 10686, 10692, 10695, 10698, 10701, 10706, 10708, 10715, 10731, 10736, 10753, 10756, 10766, 10771, 10774, 10777, 10785, 10787, 10795, 10797, 10803, 10810, 10825, 10828, 10832, 10836, 10852, 10855, 10867, 10876, 10887, 10891, 10895, 10898, 10913, 10918, 10921, 10923, 10936, 10940, 10949, 10961, 10964, 10974, 10983, 10990, 10994, 11003, 11008, 11011, 11018, 11024, 11035, 11038, 11057, 11095, 11378, 11389, 11393, 11407, 11413, 11420, 11426, 11436, 11440, 11454, 11470, 11491, 11495, 11530, 11437, 11441, 11455, 11471, 11492, 11496, 11516, 11531, 11533, 11537, 11543, 11558, 11564, 11575, 11538, 11544, 11559, 11565, 11567, 11576, 11580, 11583, 11588, 11581, 11584, 11589, 11599, 11634, 11604, 11611, 11620, 11624, 11630, 11605, 11612, 11621, 11625, 11631, 11637, 11650, 11638, 11651, 11661, 11668, 11679, 11683, 11703, 11707, 11711, 11714, 11717, 11721, 11726, 11741, 11753, 11757, 11766, 11771, 11780, 11662, 11669, 11680, 11684, 11704, 11708, 11712, 11715, 11718, 11722, 11742, 11754, 11758, 11760, 11781, 11788, 11794, 11789, 11795, 11798, 11807, 11812, 11816, 11821, 11831, 11842, 11854, 11799, 11808, 11813, 11822, 11826, 11832, 11834, 11838, 11843, 11849, 11855, 11859, 11869, 11873, 11880, 11884, 11896, 11870, 11874, 11881, 11885, 11897, 11900, 11906, 11920, 11935, 11939, 11944, 11957, 11961, 11965, 11970, 11974, 11983, 11901, 11907, 11921, 11936, 11940, 11945, 11958, 11962, 11966, 11971, 11975, 11984, 12008, 12013, 12018, 12029, 12036, 12040, 12047, 12074, 12078, 12083, 12105, 12116, 12009, 12014, 12019, 12030, 12037, 12041, 12048, 12053, 12075, 12079, 12036, 12092, 12106, 12117, 12126, 12132, 12151, 12155, 12133, 12152, 12156, 12166, 12173, 12178, 12183, 12189, 12192, 12204, 12211, 12218, 12235, 12246, 12252, 12282, 12293, 12298, 12301, 12304, 12310, 12315, 12319, 12334, 12350, 12357, 12365, 12374, 12387, 12391, 12395, 12416, 12426, 12444, 12451, 12456, 12167, 12174, 12179, 12184, 12193, 12205, 12212, 12215, 12219, 12236, 12247, 12253, 12261, 12275, 12283, 12294, 12299, 12305, 12311, 12316, 12320, 12329, 12335, 12344, 12351, 12358, 12366, 12368, 12375, 12384, 12388, 12392, 12396, 12399, 12407, 12414, 12421, 12427, 12431, 12434, 12447, 12452, 12457, 12463, 12470, 12473, 12482, 12464, 12466, 12471, 12476, 12479, 12491, 12503, 12509, 12513, 12516, 12514, 12532, 12539, 12544, 12557, 12562, 12567, 12576, 12583, 12588, 12591, 12606, 12612, 12622, 12628, 12510, 12514, 12519, 12526, 12527, 12533, 12540, 12545, 12558, 12563, 12568, 12577, 12580, 12584, 12589, 12598, 12607, 12619, 12623, 12629, 12633, 12642, 12645, 12650, 12656, 12668, 12674, 12676, 12697, 12707, 12722, 12734, 12741, 12759, 12773, 12779, 12783, 12802, 12806, 12810, 12819, 12829, 12634, 12646, 12651, 12657, 12659, 12669, 12698, 12708, 12711, 12723, 12707, 12727, 12735, 12742, 12774, 12780, 12784, 12803, 12807, 12811, 12820, 12830, 12847, 12852, 12859, 12864, 12874, 12905, 12915, 12925, 12930, 12934, 12940, 12944, 12946, 12955, 12848, 12853, 12856, 12860, 12865, 12875, 12897, 12906, 12916, 12922, 12926, 12931, 12935, 12941, 12930, 12947, 12952, 12956, 12963, 12979, 12964, 12980, 12984, 13003, 12985, 12988, 12990, 12994, 13004, 13007, 13011, 13015, 13027, 13046, 13055, 13059, 13080, 13094, 13098, 13112, 13120, 13134, 13140, 13150, 13154, 13161, 13187, 13008, 13012, 13016, 13028, 13047, 13056, 13060, 13081, 13095, 13099, 13103, 13106, 13113, 13116, 13135, 13136, 13141, 13151, 13155, 13162, 13188, 13193, 13202, 13194, 13203, 13205, 13209, 13215, 13218, 13236, 13252, 13260, 13271, 13283, 13210, 13216, 13219, 13221, 13223, 13230, 13238, 13243, 13253, 13261, 13272, 13278, 13284, 13643, 13644, 13651, 13657, 13662, 13663, 13666, 13679, 13667, 13680, 13699, 13711, 13730, 13737, 13742, 13700, 13712, 13731, 13738, 13743, 13747, 13756, 13760, 13781, 13793, 13801, 13810, 13830, 13748, 13750, 13757, 13761, 13770, 13771, 13782, 13787, 13794, 13799, 13811, 13825, 13827, 13831, 13834, 13839, 13851, 13859, 13863, 13867, 13873, 13877, 13835, 13840, 13842, 13848, 13852, 13860, 13864, 13868, 13874, 13878, 13885, 13886, 13891, 13896, 13901, 13918, 13927, 13933, 13939, 13951, 13886, 13888, 13892, 13897, 13899, 13901, 13919, 13934, 13940, 13952, 13955, 13973, 13979, 13983, 13997, 13984, 13998, 14006, 14011, 14019, 14022, 14031, 14047, 14053, 14061, 14075, 14111, 14116, 14124, 14149, 14007, 14012, 14016, 14023, 14032, 14048, 14054, 14062, 14076, 14112, 14117, 14119, 14125, 14128, 14129, 14131, 14146, 14150, 14153, 14159, 14163, 14168, 14173, 14177, 14154, 14160, 14164, 14169, 14174, 14178, 14181, 14206, 14213, 14219, 14237, 14248, 14252, 14258, 14264, 14271, 14275, 14279, 14295, 14308, 14311, 14314, 14318, 14332, 14335, 14339, 14343, 14207, 14209, 14214, 14220, 14222, 14232, 14234, 14238, 14249, 14253, 14259, 14265, 14272, 14276, 14280, 14288, 14296, 14309, 14315, 14319, 14321, 14326, 14333, 14336, 14362, 14370, 14377, 14371 and SEQ ID NO:14378.

For each genomic sequence listed, the following oligonucleotides as detailed in the sequence listing may be used for a combined MSP-RealTime analysis:

Taqman Real-time single probe detection assays:

SEQ ID NO: 41

left Primer: 2751, 2754, 2757, 2759, 2767, 2768, 2772, 2776, 2779, 2784, 2785, 2786, 2789, 2791, 2792, 2794, 2797, 2798, 2801, 2805, 2806, 2809, 2811, 2815, 2817, 2824, 2830, 2832, 2833, 2834, 2838, 2840, 2843, 2846, 2850, 2851, 2853, 2855, 2859, 2866, 2867, 2870, 2871, 2876, 2878, 2881, 2885, 2886, 2892, 2893, 2895, 2896, 2898, 2904, 2912, 2915, 2917, 2919, 2922, 2923, 2929, 2931, 2939, 2941, 2944, 2946, 2948, 2950, 2955, 2956, 2957, 2960, 2962, 2965, 2966, 2967, 2968, 2969, 2971, 2975, 2976, 2977, 2978, 2979, 2980, 2981, 2984, 2988, 2989, 2990, 2992, 2994, 2997, 2999, 3001, 3002, 3004, 3006, 3007, 3008, 3010, 3013, 3014,3016,3017,3018,3020,3022,3025,

right Primer: 2752, 2755, 2760, 2762, 2763, 2764, 2765, 2769, 2771, 2773, 2775, 2777, 2780, 2781, 2782, 2783, 2787, 2788, 2790, 2793, 2795, 2796, 2800, 2802, 2803, 2804, 2808, 2810, 2812, 2814, 2816, 2818, 2820, 2821, 2823, 2825, 2827, 2828, 2829, 2831, 2835, 2836, 2837, 2839, 2841, 2842, 2844, 2845, 2847, 2849, 2852, 2854, 2856, 2858, 2860, 2861, 2862, 2863, 2864, 2865, 2868, 2869, 2872, 2873, 2874, 2875, 2877, 2879, 2880, 2882, 2883, 2884, 2887, 2888, 2889, 2890, 2891, 2894, 2897, 2899, 2901, 2902, 2903, 2905, 2907, 2909, 2911, 2914, 2916, 2918, 2920, 2924, 2926, 2927, 2928, 2930, 2932, 2933, 2934, 2935, 2936, 2937, 2938, 2942, 2945, 2947, 2949, 2951, 2953, 2954, 2958, 2959, 2961, 2963, 2970, 2972, 2973, 2974, 2982, 2985, 2987, 2991, 2993, 2995, 2996, 2998, 3000, 3003, 3005, 3011, 3019, 3021, 3023,3026,3027,

Detection: 2753, 2756, 2 758, 2761, 2766, 2770, 2774, 2778, 2799, 2807, 2813, 2819, 2822, 2826, 2848, 2857, 2900, 2906, 2908, 2910, 2913, 2921, 2925, 2940, 2943, 2952, 2964, 2983, 2986, 3009, 3012, 3015, 3024,

SEQ ID NO: 5

left Primer: 3028, 3032, 3033, 3036, 3037, 3038, 3039, 3041, 3042, 3043, 3044, 3047, 3048, 3049, 3052, 3055, 3059, 3061, 3064, 3065, 3068, 3069, 3071, 3072, 3075, 3076, 3080, 3083, 3084, 3085, 3086, 3091, 3093, 3096, 3097, 3100, 3104, 3109, 3110, 3113, 3115, 3117, 3118, 3123, 3126, 3127, 3130, 3134, 3135, 3136, 3138, 3139, 3144, 3146, 3147, 3149, 3150, 3155,
right Primer: 3029, 3031, 3034, 3040, 3045, 3050, 3051, 3053, 3054, 3056, 3057, 3060, 3063, 3066, 3073, 3078, 3081, 3088, 3089, 3090, 3092, 3094, 3098, 3101, 3103, 3105, 3107, 3108, 3111, 3114, 3116, 3119, 3121, 3122, 3124, 3128, 3131, 3133, 3137, 3140, 3142, 3145, 3148, 3151, 3152, 3153, 3157,
Detection: 3030, 3035, 3046, 3058, 3062, 3067, 3070, 3074, 3077, 3079, 3082, 3087, 3095, 3099, 3102, 3106, 3112, 3120, 3125, 3129, 3132, 3141, 3143, 3154, 3156, 3158,

SEQ ID NO: 6

left Primer: 3159, 3165, 3166, 3167, 3170, 3171, 3172, 3174, 3175, 3176, 3179, 3180, 3181, 3184, 3185, 3186, 3192, 3194, 3198, 3201, 3202, 3203, 3204, 3206, 3209, 3212, 3213, 3215, 3216, 3217, 3218, 3221, 3224, 3226, 3228, 3229, 3230, 3232, 3233, 3234, 3235, 3237, 3238,3239,3245,3247,3248,3249,
right Primer: 3160, 3162, 3163, 3164, 3168, 3169, 3173, 3178, 3182, 3183, 3187, 3189, 3190, 3195, 3196, 3197, 3199, 3205, 3207, 3210, 3214, 3219, 3222, 3223, 3225. 3227, 3236, 3240,3242,3243,3244,3246,3250,
Detection: 3161, 3177, 3188, 3191, 3193, 3200, 3208, 3211, 3220, 3231, 3241, 3251,

SEQ ID NO: 8

left Primer: 3252, 3256, 3261, 3264, 3268, 3269, 3270, 3273, 3275, 3279, 3280, 3281, 3282, 3283, 3284, 3285, 3286, 3290, 3292, 3295, 3297, 3301, 3304, 3305, 3306, 3307, 3308, 3310, 3311, 3312, 3315, 3319, 3320, 3324, 3327, 3331, 3333, 3334, 3339, 3348, 3351, 3354, 3358,3360,3364,3367,3368,3374,3376,
right Primer: 3253, 3255, 3257, 3259, 3260, 3262, 3265, 3266, 3267, 3271, 3274, 3276, 3278, 3287, 3289, 3291, 3293, 3298, 3300, 3302, 3309, 3313, 3314, 3316, 3318, 3321, 3322, 3325, 3328, 3330, 3332, 3335, 3337, 3338, 3340, 3342, 3343, 3344, 3345, 3346, 3347, 3349, 3350, 3352, 3353, 3355, 3356, 3357, 3359, 3361, 3363, 3365, 3369, 3371, 3372, 3373,
Detection: 3254, 3258, 3 263, 3272, 3277, 3288, 3294, 3296, 3299, 3303, 3317, 3323, 3326,3329,3336,3341,3362,3366,3370,3375,

SEQ ID NO: 16

left Primer: 3377, 3380, 3383, 3386, 3388, 3389, 3390, 3391, 3392, 3395, 3396, 3400, 3401, right Primer: 3378, 3381, 3384, 3385, 3393, 3397, 3399, 3402,
Detection: 3379, 3382, 3387, 3394, 3398,

SEQ ID NO: 42

left Primer: 3403, 3406, 3408, 3413, 3414, 3417, 3420, 3423, 3428, 3432, 3433, 3434, 3436, 3439, 3440, 3442, 3443, 3445, 3447, 3453, 3455, 3458, 3463, 3466, 3469, 3470, 3471, 3472, 3473, 3474, 3475, 3477, 3478, 3480, 3481, 3483, 3484, 3487, 3490, 3494, 3495, 3496, 3497, 3499, 3500, 3504, 3505, 3508, 3512, 3514, 3515, 3516, 3521, 3525, 3529, 3533, 3534, 3537, 3538, 3540, 3541, 3542, 3543, 3544, 3547, 3548, 3553, 3557, 3558, 3559, 3560, 3562, 3563, 3566, 3571, 3574, 3575, 3577, 3580, 3583, 3584, 3585, 3586, 3592, 3594, 3595, 3597, 3598, 3599, 3601, 3603, 3604, 3607, 3608, 3612, 3619, 3621, 3622, 3626, 3631, 3632, 3637, 3638, 3639, 3640, 3642, 3643, 3644, 3646, 3647, 3649, 3650, 3652, 3656, 3657, 3660, 3663, 3668, 3670, 3673, 3676, 3677, 3678, 3683, 3688, 3690, 3694, 3697, 3700, 3702, 3703, 3704, 3708,
right Primer: 3404, 3409, 3411, 3415, 3418, 3419, 3421, 3424, 3426, 3427, 3429, 3431, 3435, 3437, 3438, 3441, 3444, 3446, 3448, 3450, 3452, 3454, 3456, 3459, 3461, 3462, 3464, 3467, 3476, 3479, 3482, 3485, 3488, 3489, 3491, 3492, 3493, 3501, 3503, 3506, 3509, 3510, 3511, 3513, 3517, 3518, 3519, 3520, 3522, 3524, 3526, 3527, 3528, 3530, 3531, 3535, 3536, 3545, 3549, 3551, 3552, 3554, 3555, 3556, 3561, 3564, 3565, 3568, 3569, 3570, 3573, 3576, 3578, 3581, 3587, 3588, 3589, 3590, 3591, 3593, 3596, 3600, 3602, 3605, 3606, 3609, 3611, 3613, 3614, 3615, 3616, 3617, 3618, 3623, 3624, 3625, 3627, 3629, 3630, 3633, 3634, 3635, 3636, 3641, 3645, 3651, 3653, 3654, 3655, 3658, 3661, 3662, 3665, 3666, 3667, 3669, 3671, 3674, 3679, 3681, 3684, 3686, 3689, 3691, 3692, 3693, 3695, 3696, 3698, 3701, 3705, 3706, 3707,3709,
Detection: 3405, 3407, 3410, 3412, 3416, 3422, 3425, 3430, 3449, 3451, 3457, 3460, 3465, 3468, 3486, 3498, 3502, 3507, 3523, 3532, 3539, 3546, 3550, 3567, 3572, 3579, 3582, 3610, 3620, 3628, 3648, 3659, 3664, 3672, 3675, 3680, 3682, 3685, 3687, 3699,

SEQ ID NO: 14

left Primer: 3710, 3714, 3715, 3717, 3718, 3722, 3723, 3729, 3730, 3732, 3735, 3739, 3740, 3741, 3743, 3744, 3745, 3746, 3749, 3753, 3754, 3755, 3756,
right Primer: 3711, 3713, 3719, 3721, 3724, 3725, 3726, 3727, 3728, 3731, 3733, 3736, 3737, 3738, 3742, 3747, 3748, 3750, 3752, 3757, 3758, 3759, 3760,
Detection: 3712, 3716, 3720, 3734, 3751,

SEQ ID NO: 43

left Primer: 3761, 3764, 3767, 3771, 3773, 3775, 3776, 3777, 3781, 3785, 3786, 3787, 3790, 3791, 3793, 3796, 3797, 3798, 3799, 3805, 3809, 3814, 3817, 3818, 3822, 3823, 3828, 3833, 3836, 3844, 3847, 3850, 3851, 3853, 3856, 3857, 3862, 3873, 3876, 3877, 3880, 3885, 3887, 3889, 3890, 3892, 3893, 3895, 3896, 3899, 3902, 3903, 3905, 3906, 3907, 3908, 3916, 3923, 3926, 3927, 3930, 3935, 3936, 3938, 3939, 3940, 3944, 3945, 3949, 3950, 3951, 3954, 3955, 3957, 3960, 3965, 3970, 3971, 3978, 3979, 3982, 3983, 3984, 3985,
right Primer: 3762, 3765, 3768, 3770, 3772, 3774, 3778, 3779, 3780, 3782, 3784, 3788, 3792, 3794, 3800, 3801, 3802, 3803, 3804, 3806, 3807, 3808, 3810, 3812, 3813, 3815, 3819, 3820, 3821, 3824, 3826, 3827, 3829, 3831, 3832, 3834, 3837, 3838, 3839, 3840, 3842, 3845, 3848, 3852, 3854, 3858, 3860, 3861, 3863, 3864, 3865, 3866, 3867, 3868, 3869, 3870, 3871, 3872, 3874, 3875, 3878, 3881, 3883, 3884, 3886, 3888, 3891, 3894, 3897, 3898, 3900, 3901, 3904, 3909, 3911, 3914, 3915, 3917, 3919, 3920, 3921, 3922, 3924, 3928, 3931, 3933, 3934, 3937, 3941, 3942, 3943, 3947, 3948, 3952, 3958, 3959, 3961, 3962, 3963, 3964, 3966, 3968, 3972,3974,3975,3976,3980,3981,3986,
Detection: 3763, 3766, 3 769, 3783, 3789, 3795, 3811, 3816, 3825, 3830, 3835, 3841, 3843, 3846, 3849, 3855, 3859, 3879, 3882, 3910, 3912, 3913, 3918, 3925, 3929, 3932, 3946, 3953,3956,3967,3969,3973,3977,

SEQ ID NO: 15

left Primer: 3987, 3990, 3993, 3994, 3997, 4000, 4003, 4004, 4006, 4012, 4015, 4017, 4020, 4024, 4027, 4029, 4031, 4032, 4033, 4037, 4038, 4040, 4042, 4047, 4048, 4050, 4051, 4052, 4054, 4055, 4058, 4063, 4066, 4069, 4070, 4072, 4073, 4076, 4078, 4079, 4082, 4083, 4084, 4085, 4086, 4090, 4093, 4094, 4095, 4096, 4099, 4100, 4104, 4106, 4113, 4116, 4119, 4120, 4124, 4127, 4130, 4134, 4135, 4136, 4138, 4142, 4145, 4146, 4149, 4150, 4152, 4153, 4159, 4160, 4161, 4163, 4168, 4169, 4171, 4173, 4176, 4180, 4181, 4185, 4189, 4191, 4192, 4194, 4197, 4198, 4199, 4201, 4206, 4207, 4208, 4213, 4219, 4220, 4223, 4224, 4225, 4228, 4231, 4232, 4233, 4237, 4238, 4242, 4243, 4246, 4247, 4251, 4254, 4255, 4256, 4258,
right Primer: 3988, 3991, 3995, 3998, 4001, 4005, 4007, 4009, 4010, 4011, 4013, 4016, 4018, 4019, 4021, 4022, 4023, 4025, 4028, 4034, 4035, 4039, 4043, 4045, 4046, 4049, 4056, 4059, 4061, 4064, 4065, 4067, 4068, 4071, 4074, 4075, 4077, 4080, 4087, 4088, 4089, 4092, 4098, 4101, 4102, 4105, 4108, 4109, 4110, 4111, 4112, 4114, 4115, 4117, 4121, 4122, 4123, 4125, 4128, 4129, 4131, 4132, 4133, 4137, 4139, 4141, 4144, 4148, 4151, 4154, 4156, 4157, 4162, 4165, 4166, 4167, 4170, 4172, 4174, 4177, 4179, 4182, 4184, 4186, 4188, 4190, 4195, 4196, 4202, 4204, 4205, 4209, 4211, 4214, 4216, 4217, 4218, 4221, 4226, 4229, 4234, 4236, 4239, 4241, 4244, 4245, 4248, 4249, 4252, 4257, 4259, 4260, 4261,
Detection: 3989, 3992, 3996, 3999, 4002, 4008, 4014, 4026, 4030, 4036, 4041, 4044, 4053, 4057, 4060, 4062, 4081, 4091, 4097, 4103, 4107, 4118, 4126, 4140, 4143, 4147, 4155, 4158, 4164, 4175, 4178, 4183, 4187, 4193, 4200, 4203, 4210, 4212, 4215, 4222, 4227, 4230, 4235,4240,4250,4253,

SEQ ID NO:4

left Primer: 4262, 4266, 4267, 4268, 4271, 4272, 4275, 4276, 4279, 4281, 4282, 4284, 4285, 4287, 4289, 4290, 4292, 4295, 4297, 4299, 4301, 4306, 4310, 4316, 4317, 4318, 4321, 4323, 4325, 4328, 4334, 4335, 4337, 4341, 4342, 4343, 4344, 4348, 4350, 4351, 4352, 4354, 4357, 4358, 4359, 4364, 4365, 4366, 4370, 4371, 4372, 4373, 4376, 4378, 4381, 4383, 4384, 4386, 4388, 4396, 4397, 4399, 4400, 4403, 4405, 4407, 4409, 4412, 4415, 4416, 4417, 4424, 4426, 4429, 4433, 4435, 4437, 4442, 4443, 4447, 4450, 4451, 4452, 4455, 4457, 4460, 4461, 4462, 4465, 4466, 4469, 4470, 4474, 4478, 4481, 4484, 4488, 4491, 4494, 4495, 4497, 4498, 4504, 4506, 4509, 4510, 4511, 4514, 4516, 4518, 4520, 4521, 4523, 4525, 4528, 4530, 4533, 4534, 4535, 4538, 4540, 4542, 4545, 4547, 4548, 4553, 4554, 4555, 4556, 4557, 4562, 4564, 4567, 4568, 4569, 4579, 4580, 4582, 4584, 4587, 4589, 4593, 4596, 4597, 4598, 4600, 4601, 4602, 4605, 4606, 4607, 4609, 4610, 4613, 4614, 4616, 4618, 4621, 4624, 4626, 4627, 4628, 4631,4632,4636,4639,4641,4644,4645,4648,

right Primer: 4263, 4265, 4269, 4270, 4273, 4274, 4277, 4280, 4283, 4286, 4288, 4291, 4293, 4296, 4298, 4300, 4302, 4303, 4304, 4305, 4307, 4308, 4309, 4311, 4313, 4315, 4319, 4322, 4324, 4326, 4327, 4330, 4331, 4332, 4333, 4336, 4338, 4340, 4345, 4347, 4349, 4353, 4355, 4360, 4361, 4362, 4367, 4368, 4369, 4374, 4375, 4377, 4379, 4382, 4385, 4387, 4389, 4390, 4391, 4392, 4393, 4394, 4395, 4398, 4401, 4406, 4408, 4410, 4411, 4413, 4418, 4420, 4422, 4423, 4425, 4427, 4428, 4430, 4432, 4434, 4436, 4438, 4440, 4441, 4444, 4446, 4448, 4453, 4454, 4456, 4458, 4463, 4464, 4467, 4471, 4472, 4473, 4475, 4476, 4477, 4479, 4480, 4482, 4485, 4486, 4487, 4489, 4490, 4492, 4496, 4499, 4501, 4502, 4503, 4505, 4507, 4508, 4512, 4515, 4517, 4519, 4522, 4524, 4529, 4531, 4536, 4537, 4539, 4541, 4543, 4546, 4549, 4551, 4558, 4559, 4560, 4561, 4563, 4565, 4570, 4571, 4572, 4573, 4574, 4575, 4576, 4578, 4581, 4583, 4585, 4586, 4588, 4590, 4592, 4595, 4599, 4603, 4611, 4612, 4615, 4617, 4619, 4620, 4622, 4630, 4633, 4634, 4635, 4638, 4640, 4642, 4643, 4646, 4647,

Detection: 4264, 4278, 4 294, 4312, 4314, 4320, 4329, 4339, 4346, 4356, 4363, 4380, 4402, 4404, 4414, 4419, 4421, 4431, 4439, 4445, 4449, 4459, 4468, 4483, 4493, 4500, 4513, 4526, 4527, 4532, 4544, 4550, 4552, 4566, 4577, 4591, 4594, 4604, 4608, 4623, 4625, 4629, 4637,

SEQ ID NO: 7

left Primer: 4649, 4652, 4655, 4659, 4661, 4663, 4664, 4665, 4668, 4671, 4674, 4675, 4676, 4678, 4680, 4683, 4686, 4690, 4696, 4700, 4703, 4704, 4706, 4709, 4711, 4714, 4715, 4717, 4718, 4719, 4720, 4721, 4726, 4727, 4730, 4732, 4738, 4742, 4745, 4749, 4750, 4753, 4755, 4759, 4763, 4768, 4769, 4772, 4773, 4776, 4778, 4779, 4782, 4784, 4789, 4790, 4794, 4797, 4801, 4805, 4807, 4813, 4814, 4819, 4821, 4822, 4827, 4829, 4833, 4834, 4835, 4837, 4838, 4841, 4842, 4843, 4844, 4847, 4849, 4850, 4852, 4853, 4859, 4860, 4861, 4865, 4867, 4868, 4869, 4870, 4873, 4876, 4877, 4878, 4879, 4883, 4885, 4889, 4891, 4892, 4894, 4895, 4897, 4898, 4903, 4906, 4911, 4912, 4918, 4919, 4920, 4924, 4927, 4929, 4932, 4934, 4941, 4942,4945,4948,4949,4950,

right Primer: 4650, 4653, 4656, 4658, 4660, 4662, 4666, 4667, 4669, 4670, 4672, 4677, 4681, 4684, 4685, 4687, 4688, 4689, 4691, 4693, 4694, 4695, 4697, 4699, 4701, 4702, 4705, 4707, 4708, 4716, 4722, 4724, 4728, 4731, 4733, 4735, 4736, 4737, 4739, 4741, 4744, 4746, 4748, 4751, 4754, 4756, 4758, 4760, 4761, 4764, 4766, 4767, 4770, 4771, 4774, 4777, 4780, 4783, 4785, 4786, 4787, 4788, 4791, 4792, 4793, 4795, 4798, 4800, 4802, 4804, 4806, 4808, 4810, 4811, 4812, 4815, 4816, 4817, 4818, 4820, 4823, 4824, 4825, 4828, 4830, 4832, 4836, 4839, 4840, 4845, 4846, 4848, 4851, 4854, 4855, 4856, 4857, 4858, 4862, 4864, 4871, 4872, 4874, 4875, 4881, 4882, 4884, 4886, 4887, 4890, 4893, 4896, 4899, 4900, 4901, 4902, 4904, 4905, 4907, 4908, 4909, 4910, 4913, 4915, 4917, 4921, 4922, 4923, 4925, 4926, 4928, 4930, 4935, 4936, 4937, 4938, 4940, 4943, 4944, 4946, 4947, 4951, 4952,

Detection: 4651, 4654, 4 657, 4673, 4679, 4682, 4692, 4698, 4710, 4712, 4713, 4723, 4725, 4729, 4734, 4740, 4743, 4747, 4752, 4757, 4762, 4765, 4775, 4781, 4796, 4799, 4803, 4809, 4826, 4831, 4863, 4866, 4880, 4888, 4914, 4916, 4931, 4933, 4939,

SEQ ID NO: 44

left Primer: 4953,

right Primer: 4954, 4956,

Detection: 4955,

SEQ ID NO: 1

left Primer: 4957, 4960, 4963, 4967, 4970, 4971, 4972, 4976, 4977, 4985, 4986, 4987, 4989, 4991, 4992, 4997, 4999, 5002, 5003, 5004, 5006, 5010, 5013, 5014, 5015, 5017, 5018, 5019, 5020, 5021, 5024, 5025, 5027, 5031, 5032, 5033, 5037, 5040, 5041, 5042, 5043,
right Primer: 4958, 4961, 4964, 4966, 4968, 4969, 4973, 4974, 4975, 4978, 4979, 4980, 4981, 4982, 4983, 4984, 4988, 4990, 4993, 4995, 4996, 4998, 5000, 5005, 5007, 5009, 5011, 5016, 5022, 5026, 5028, 5030, 5034, 5036, 5038, 5039,
Detection: 4959, 4962, 4965, 4994, 5001, 5008, 5012, 5023, 5029, 5035,

SEQ ID NO:2

left Primer: 5044, 5047, 5049, 5050, 5052, 5054, 5055, 5057, 5060, 5061, 5064,
right Primer: 5045, 5048, 5051, 5053, 5056, 5058, 5062, 5065,
Detection: 5046, 5059, 5063,

SEQ ID NO: 45

left Primer: 5066, 5069, 5073, 5077, 5080, 5083, 5087, 5093, 5096, 5099, 5100, 5103, 5104, 5106, 5111, 5116, 5119, 5120, 5125, 5128, 5132, 5134, 5135, 5137, 5139, 5140, 5142, 5144, 5148, 5149, 5150, 5155, 5157, 5158, 5160, 5166, 5168, 5170, 5171, 5173, 5176, 5177, 5179, 5184, 5187, 5188, 5190, 5193, 5195, 5198, 5201, 5204, 5208, 5211, 5215, 5216, 5217, 5220, 5221, 5222, 5224, 5226, 5229, 5230, 5231, 5232, 5234, 5237, 5242, 5244, 5245, 5246, 5247, 5249, 5250, 5252, 5254, 5255, 5256, 5261, 5262, 5264, 5265, 5273, 5274, 5276, 5278, 5279, 5281, 5284, 5285, 5286, 5287, 5288, 5289, 5292, 5296, 5300, 5303, 5304, 5307, 5308, 5309, 5313, 5315, 5317, 5318, 5322, 5323, 5326, 5329, 5330, 5333, 5336, 5337, 5341, 5343, 5348, 5349, 5352, 5354, 5355, 5358, 5359, 5361, 5362, 5363, 5364, 5365, 5367, 5369, 5370, 5372, 5375, 5377, 5379, 5382, 5385, 5387, 5393, 5394, 5395, 5398, 5400, 5404, 5407, 5408, 5409, 5412, 5413, 5414, 5416, 5418, 5421, 5424, 5426, 5428, 5431, 5432, 5433, 5434, 5435, 5437, 5440, 5441, 5443, 5448, 5450, 5451, 5454, 5455, 5458, 5460, 5461, 5464, 5468, 5470, 5471, 5474, 5475, 5477, 5482, 5484, 5485, 5487, 5490, 5491, 5492, 5494, 5496, 5498, 5499, 5500, 5501, 5502, 5503, 5504, 5506, 5509, 5510, 5512, 5516, 5517, 5518, 5524, 5525, 5526, 5528, 5531, 5533, 5534, 5535, 5536, 5537, 5540, 5542, 5544, 5545, 5546, 5549, 5550, 5552, 5553, 5559, 5561, 5566, 5569, 5572, 5573, 5575, 5577, 5578, 5579, 5580, 5581, 5582, 5588, 5593, 5595, 5596, 5598, 5600, 5601, 5603, 5604, 5605, 5608, 5614, 5615, 5616, 5619, 5622, 5624, 5625, 5628, 5629, 5633, 5634, 5637, 5638, 5639, 5643, 5644, 5645, 5647, 5648, 5649, 5650,5654,5662,5664,
right Primer: 5067, 5070, 5072, 5074, 5075, 5076, 5078, 5081, 5084, 5086, 5088, 5090, 5091, 5094, 5097, 5101, 5105, 5107, 5109, 5110, 5112, 5114, 5115, 5117, 5121, 5123, 5124, 5126, 5129, 5131, 5136, 5143, 5145, 5147, 5151, 5153, 5156, 5159, 5161, 5162, 5163, 5164, 5165, 5167, 5169, 5172, 5174, 5175, 5180, 5181, 5182, 5183, 5185, 5186, 5189, 5191, 5194, 5196, 5199, 5200, 5202, 5203, 5205, 5207, 5209, 5210, 5212, 5214, 5218, 5219, 5223, 5225, 5227, 5233, 5235, 5238, 5239, 5240, 5241, 5248, 5251, 5253, 5257, 5259, 5260, 5263, 5266, 5268, 5269, 5270, 5271, 5272, 5280, 5282, 5290, 5291, 5293, 5295, 5297, 5299, 5301, 5306, 5310, 5311, 5312, 5314, 5316, 5319, 5321, 5324, 5327, 5331, 5332, 5334, 5338, 5339, 5340, 5342, 5344, 5346, 5347, 5351, 5353, 5356, 5360, 5366, 5368, 5371, 5373, 5376, 5378, 5380, 5383, 5388, 5389, 5391, 5392, 5396, 5399, 5401, 5403, 5405, 5410, 5415, 5417, 5419, 5420, 5422, 5425, 5427, 5429, 5436, 5438, 5442, 5444, 5445, 5446, 5447, 5449, 5452, 5456, 5457, 5459, 5462, 5465, 5466, 5467, 5469, 5472, 5478, 5480, 5481, 5486, 5488, 5493, 5495, 5497, 5505, 5507, 5508, 5511, 5513, 5515, 5519, 5521, 5522, 5527, 5529, 5532, 5538, 5539, 5541, 5543, 5548, 5551, 5554, 5555, 5556, 5557, 5558, 5560, 5562, 5563, 5564, 5565, 5567, 5568, 5570, 5571, 5574, 5576, 5583, 5585, 5586, 5587, 5589, 5591, 5592, 5594, 5597, 5599, 5602, 5606, 5609, 5611, 5612, 5618, 5620, 5623, 5626, 5627, 5630, 5632, 5635, 5640, 5642, 5646, 5651, 5652, 5656, 5657, 5659, 5660, 5661, 5663, 5665,
Detection: 5068, 5071, 5 079, 5082, 5085, 5089, 5092, 5095, 5098, 5102, 5108, 5113, 5118, 5122, 5127, 5130, 5133, 5138, 5141, 5146, 5152, 5154, 5178, 5192, 5197, 5206, 5213, 5228, 5236, 5243, 5258, 5267, 5275, 5277, 5283, 5294, 5298, 5302, 5305, 5320, 5325, 5328, 5335, 5345, 5350, 5357, 5374, 5381, 5384, 5386, 5390, 5397, 5402, 5406, 5411, 5423, 5430, 5439, 5453, 5463, 5473, 5476, 5479, 5483, 5489, 5514, 5520, 5523, 5530, 5547, 5584, 5590, 5607, 5610, 5613, 5617, 5621, 5631, 5636, 5641, 5653, 5655, 5658, 5666,

SEQ ID NO: 9

left Primer: 5667, 5670, 5672, 5673, 5676, 5678, 5679, 5681, 5682, 5683, 5686, 5687, 5688, 5690, 5694, 5697, 5698, 5699, 5700, 5701, 5704, 5708, 5709, 5711, 5713, 5714, 5717, 5721, 5723, 5724, 5727, 5728, 5733, 5734, 5735, 5736, 5739, 5740, 5741, 5742, 5743,

right Primer: 5668, 5671, 5674, 5677, 5680, 5684, 5689, 5691, 5693, 5696, 5702, 5705, 5707, 5710, 5712, 5715, 5718, 5720, 5722, 5725, 5729, 5730, 5731, 5732, 5737,
Detection: 5669, 5675, 5685, 5692, 5695, 5703, 5706, 5716, 5719, 5726, 5738,

SEQ ID NO: 46

left Primer: 5744, 5747, 5750, 5753, 5759, 5762, 5763, 5770, 5775, 5777, 5778, 5780, 5782, 5785, 5787, 5791, 5792, 5793, 5794, 5797, 5801, 5803, 5808, 5810, 5812, 5814, 5816, 5817, 5824, 5832, 5833, 5834, 5835, 5838, 5840, 5841, 5844, 5849, 5853, 5854, 5857, 5859, 5860, 5862, 5863, 5865, 5868, 5874, 5882, 5883, 5885, 5887, 5894, 5895, 5896, 5897, 5902, 5904, 5906, 5909, 5912, 5915, 5918, 5919, 5920, 5922, 5923, 5926, 5927, 5928, 5930, 5932, 5933, 5934, 5935, 5939, 5940, 5946, 5947, 5948, 5949, 5952, 5954, 5955, 5956, 5958, 5959, 5961, 5962, 5964, 5965, 5968, 5970, 5971, 5972, 5973, 5977, 5978, 5980, 5983, 5986, 5987, 5992, 5993, 5996, 5997, 6000, 6002, 6003, 6004, 6005, 6006, 6008, 6009, 6010, 6011, 6012, 6013, 6017, 6018, 6019, 6020, 6025, 6027, 6030, 6033, 6035, 6036, 6039, 6041, 6042, 6045, 6049, 6050, 6051, 6055, 6059, 6063, 6064, 6065, 6067, 6070, 6072, 6073, 6075, 6077, 6078, 6081,
right Primer: 5745, 5748, 5751, 5754, 5756, 5758, 5760, 5764, 5766, 5767, 5768, 5769, 5771, 5773, 5774, 5776, 5779, 5781, 5783, 5786, 5788, 5789, 5790, 5795, 5796, 5798, 5800, 5802, 5804, 5806, 5807, 5809, 5811, 5813, 5818, 5820, 5821, 5822, 5823, 5825, 5826, 5827, 5828, 5830, 5831, 5836, 5837, 5839, 5842, 5843, 5845, 5846, 5847, 5848, 5850, 5852, 5855, 5856, 5858, 5861, 5864, 5866, 5867, 5870, 5871, 5872, 5873, 5875, 5876, 5877, 5878, 5879, 5880, 5881, 5884, 5886, 5888, 5889, 5890, 5891, 5892, 5893, 5898, 5899, 5900, 5903, 5905, 5907, 5910, 5913, 5916, 5921, 5924, 5929, 5931, 5936, 5937, 5938, 5942, 5943, 5944, 5945, 5950, 5953, 5957, 5960, 5963, 5966, 5969, 5975, 5976, 5981, 5984, 5988, 5990, 5991, 5994, 5998, 5999, 6001, 6007, 6014, 6015, 6016, 6021, 6023, 6024, 6026, 6028, 6031, 6032, 6034, 6037, 6038, 6040, 6043, 6046, 6048, 6053, 6054, 6056, 6058, 6060, 6062, 6066, 6068, 6074, 6076,6079,6083,6084,
Detection: 5746, 5749, 5752, 5755, 5757, 5761, 5765, 5772, 5784, 5799, 5805, 5815, 5819, 5829, 5851, 5869, 5901, 5908, 5911, 5914, 5917, 5925, 5941, 5951, 5967, 5974, 5979, 5982, 5985, 5989, 5995, 6022, 6029, 6044, 6047, 6052, 6057, 6061, 6069, 6071, 6080, 6082,

SEQ ID NO: 10

left Primer: 6085, 6089, 6090, 6093, 6096, 6098, 6100, 6106, 6110, 6111, 6114, 6118, 6121, 6127, 6130, 6133, 6135, 6137, 6138, 6141, 6143, 6145, 6148, 6150, 6151, 6152, 6153, 6154, 6156, 6159, 6160, 6162, 6163, 6166, 6167, 6168, 6169, 6170, 6171, 6172, 6175, 6177, 6180, 6183, 6187, 6188, 6190, 6193, 6194, 6195, 6196, 6198, 6201, 6202, 6204, 6207, 6211, 6212, 6214, 6215, 6216, 6218, 6219, 6220, 6222, 6225, 6226, 6230, 6235, 6239, 6241, 6242, 6243, 6244, 6245, 6247, 6250, 6252, 6254, 6259, 6260, 6261, 6264, 6265, 6268, 6270, 6273, 6275, 6278, 6280, 6282, 6286, 6287, 6288, 6291, 6293, 6298, 6299, 6300, 6302, 6303, 6305, 6307, 6308, 6312, 6313, 6314, 6315, 6316, 6317, 6318, 6320, 6321, 6323, 6325, 6327, 6328, 6334, 6335, 6337, 6340, 6341, 6344, 6348, 6350, 6353, 6354, 6357, 6360, 6362, 6364, 6368, 6372, 6374, 6377, 6378, 6383, 6386, 6392, 6394, 6398, 6400, 6404, 6405, 6409, 6410, 6411, 6417, 6418, 6424, 6426, 6429, 6431, 6432, 6433, 6435, 6436, 6437, 6438, 6440, 6442, 6445, 6446, 6448, 6449, 6452, 6455, 6457, 6458, 6461, 6462, 6463, 6464, 6465, 6470, 6473, 6475, 6478, 6480, 6481, 6484, 6485, 6486, 6489, 6491, 6492, 6494, 6498, 6502, 6504, 6505, 6507, 6511,6513,6514,
right Primer: 6086, 6088, 6091, 6094, 6097, 6099, 6101, 6103, 6105, 6108, 6112, 6115, 6116, 6117, 6119, 6120, 6122, 6123, 6125, 6126, 6128, 6131, 6132, 6134, 6136, 6139, 6142, 6144, 6146, 6149, 6155, 6157, 6161, 6164, 6173, 6174, 6176, 6178, 6181, 6182, 6184, 6186, 6189, 6191, 6192, 6197, 6199, 6200, 6205, 6206, 6208, 6210, 6213, 6221, 6223, 6224, 6227, 6229, 6231, 6232, 6233, 6234, 6236, 6237, 6238, 6240, 6246, 6248, 6251, 6253, 6255, 6256, 6257, 6258, 6262, 6266, 6269, 6271, 6272, 6274, 6276, 6277, 6281, 6283, 6284, 6285, 6289, 6292, 6294, 6295, 6296, 6297, 6301, 6304, 6306, 6309, 6310, 6311, 6319, 6322, 6324, 6326, 6329, 6330, 6331, 6333, 6336, 6338, 6342, 6345, 6347, 6349, 6351, 6355, 6356, 6358, 6359, 6361, 6365, 6367, 6369, 6371, 6373, 6375, 6376, 6379, 6381, 6382, 6384, 6387, 6388, 6389, 6390, 6393, 6395, 6397, 6399, 6401, 6402, 6403, 6406, 6407, 6412, 6413, 6414, 6415, 6416, 6419, 6420, 6421, 6422, 6423, 6425, 6427, 6428, 6434, 6441, 6443, 6444, 6447, 6450, 6451, 6453, 6459, 6460, 6466, 6467, 6468, 6469, 6471, 6472, 6474, 6476, 6479, 6482, 6487, 6490, 6493, 6496, 6497, 6499, 6500, 6501, 6503, 6506, 6508, 6510, 6512,
Detection: 6087, 6092, 6 095, 6102, 6104, 6107, 6109, 6113, 6124, 6129, 6140, 6147, 6158, 6165, 6179, 6185, 6203, 6209, 6217, 6228, 6249, 6263, 6267, 6279, 6290, 6332, 6339, 6343, 6346, 6352, 6363, 6366, 6370, 6380, 6385, 6391, 6396, 6408, 6430, 6439, 6454, 6456, 6477,6483,6488,6495,6509,

SEQ ID NO: 11

left Primer: 6515, 6518, 6521, 6522, 6523, 6524, 6528, 6532, 6535, 6539, 6549, 6560, 6561, 6564, 6566, 6567, 6570, 6573, 6576, 6577, 6578, 6579, 6583, 6584, 6585, 6586, 6587, 6590, 6591, 6594, 6596, 6597, 6603, 6605, 6608, 6609, 6610, 6611, 6612, 6615, 6625, 6630, 6632, 6633, 6634, 6638, 6639, 6642, 6643, 6644, 6647, 6650, 6655, 6657, 6658, 6659, 6661, 6663, 6664, 6665, 6669, 6671, 6674, 6677, 6678, 6679, 6680, 6681, 6682, 6686, 6687, 6688, 6689, 6690, 6691, 6693, 6695, 6696, 6697, 6699, 6701, 6703, 6704, 6705, 6706, 6707, 6708, 6710, 6711, 6712, 6713, 6717, 6718, 6720, 6721, 6722, 6725, 6727, 6732, 6734, 6735, 6739, 6741, 6745, 6748, 6751, 6755, 6759, 6762, 6764, 6767, 6770, 6772, 6773, 6774, 6777, 6778, 6782, 6785, 6786, 6787, 6790, 6791, 6794, 6795, 6796, 6798, 6800, 6802, 6803, 6806, 6811, 6814, 6815, 6818, 6824, 6829, 6834, 6835, 6842,
right Primer: 6516, 6519, 6525, 6526, 6527, 6529, 6531, 6533, 6536, 6537, 6538, 6540, 6541, 6542, 6543, 6544, 6545, 6546, 6547, 6548, 6550, 6551, 6552, 6553, 6554, 6556, 6558, 6562, 6563, 6565, 6568, 6571, 6572, 6575, 6580, 6582, 6588, 6589, 6592, 6595, 6598, 6599, 6600, 6601, 6602, 6604, 6606, 6607, 6613, 6614, 6617, 6618, 6619, 6620, 6621, 6623, 6624, 6627, 6628, 6629, 6631, 6635, 6640, 6641, 6645, 6646, 6649, 6651, 6652, 6653, 6656, 6660, 6662, 6666, 6668, 6670, 6672, 6675, 6676, 6683, 6685, 6692, 6694, 6698, 6700, 6702, 6709, 6714, 6715, 6716, 6719, 6723, 6726, 6728, 6730, 6731, 6736, 6738, 6740, 6742, 6744, 6746, 6747, 6749, 6752, 6754, 6756, 6757, 6758, 6760, 6761, 6765, 6769, 6775, 6779, 6781, 6783, 6784, 6789, 6792, 6797, 6799, 6801, 6804, 6805, 6807, 6809, 6810, 6812, 6816, 6817, 6819, 6821, 6822, 6825, 6827, 6828, 6830, 6831, 6832, 6833, 6836, 6837, 6838, 6839, 6840, 6841, 6843,
Detection: 6517, 6520, 6 530, 6534, 6555, 6557, 6559, 6569, 6574, 6581, 6593, 6616, 6622, 6626, 6636, 6637, 6648, 6654, 6667, 6673, 6684, 6724, 6729, 6733, 6737, 6743, 6750, 6753, 6763, 6766, 6768, 6771, 6776, 6780, 6788, 6793, 6808, 6813, 6820, 6823, 6826,

SEQ ID NO: 12

left Primer: 6844, 6848, 6850, 6852, 6854, 6857, 6859, 6861, 6862, 6865, 6868, 6870, 6871, 6872, 6874, 6875, 6876, 6880, 6883, 6886, 6887, 6888, 6890, 6891, 6894, 6896, 6897, 6898, 6899, 6900, 6901, 6902, 6904, 6905, 6906, 6910, 6912, 6917, 6920,
right Primer: 6845, 6847, 6849, 6851, 6853, 6855, 6858, 6860, 6863, 6866, 6869, 6873, 6877, 6879, 6881, 6882, 6884, 6889, 6892, 6893, 6895, 6907, 6908, 6909, 6911, 6913, 6915, 6916,6918,
Detection: 6846, 6856, 6864, 6867, 6878, 6885, 6903, 6914, 6919,

SEQ ID NO: 13

left Primer: 6921, 6924, 6927, 6928, 6933, 6935, 6937, 6939, 6940, 6943, 6946, 6947, 6948, 6949, 6951, 6953, 6954, 6955, 6959, 6961, 6964, 6965, 6968, 6969, 6972, 6973, 6976, 6979,6980,
right Primer: 6922, 6925, 6929, 6930, 6931, 6932, 6934, 6936, 6942, 6944, 6945, 6950, 6952, 6956, 6957, 6958, 6960, 6962, 6963, 6966, 6967, 6970, 6971, 6974, 6975, 6977, 6981,
Detection: 6923, 6926, 6938, 6941, 6978, 6982,

SEQ ID NO:3

left Primer: 6983, 6986, 6992, 6995, 6998, 7002, 7006, 7013, 7017, 7022, 7024, 7027, 7030, 7032, 7036, 7037, 7040, 7050, 7052, 7055, 7056, 7058, 7059, 7060, 7064, 7067, 7072, 7074, 7076, 7077, 7084, 7085, 7086, 7087, 7088, 7089, 7090, 7091, 7096, 7097, 7100, 7102, 7109, 7110, 7113, 7114, 7115, 7118, 7121, 7123, 7124, 7131, 7133, 7135, 7136, 7138, 7139, 7140, 7144, 7146, 7148, 7149, 7158, 7159, 7160, 7163, 7165, 7166, 7168, 7172, 7175, 7178, 7180, 7181, 7183, 7185, 7186, 7187, 7192, 7194, 7199, 7202, 7203, 7204, 7205, 7207, 7208, 7213, 7214, 7216, 7220, 7221, 7227, 7228, 7230, 7233, 7235, 7237, 7239, 7243, 7245, 7246, 7248, 7249, 7251, 7255, 7256, 7260, 7263, 7264, 7265, 7266, 7268, 7269, 7272, 7276, 7277, 7278, 7279, 7280, 7281, 7283, 7284, 7287, 7291, 7295, 7299, 7302, 7303, 7306, 7309, 7310, 7311, 7314, 7315, 7320, 7321, 7324, 7325, 7329, 7331, 7332, 7334, 7336, 7339, 7340, 7341, 7344, 7350, 7353, 7355, 7358, 7360, 7362, 7372, 7374, 7375, 7378, 7381, 7385, 7387, 7390, 7391, 7392, 7394, 7397, 7401, 7402, 7405, 7410, 7413, 7415, 7416, 7417, 7420,
right Primer: 6984, 6987, 6989, 6990, 6991, 6993, 6996, 6999, 7001, 7003, 7005, 7007, 7009, 7010, 7011, 7012, 7014, 7016, 7018, 7020, 7023, 7025, 7026, 7028, 7029, 7031, 7033, 7034, 7035, 7038, 7039, 7041, 7042, 7043, 7044, 7045, 7046, 7047, 7048, 7049, 7051, 7053, 7054, 7057, 7061, 7062, 7063, 7065, 7066, 7068, 7070, 7071, 7073, 7075, 7078, 7080, 7081, 7082, 7083, 7092, 7093, 7094, 7095, 7098, 7101, 7103, 7105, 7106, 7107, 7108, 7111, 7112, 7116, 7119, 7122, 7125, 7126, 7127, 7128, 7129, 7130, 7132, 7134, 7137, 7141, 7142, 7143, 7147, 7150, 7152, 7154, 7155, 7156, 7157, 7161, 7167, 7170, 7171, 7173, 7174, 7176, 7177, 7179, 7182, 7184, 7188, 7189, 7190, 7191, 7193, 7195, 7196, 7197, 7198, 7200, 7206, 7210, 7211, 7212, 7215, 7217, 7218, 7219, 7222, 7223, 7224, 7225, 7226, 7229, 7231, 7232, 7236, 7240, 7241, 7242, 7244, 7247, 7250, 7253, 7254, 7257, 7258, 7259, 7261, 7262, 7267, 7270, 7271, 7273, 7274, 7275, 7282, 7285, 7289, 7290, 7292, 7293, 7294, 7296, 7297, 7298, 7301, 7304, 7307, 7308, 7312, 7316, 7317, 7318, 7319, 7322, 7326, 7328, 7330, 7333, 7335, 7337, 7342, 7345, 7347, 7348, 7349, 7351, 7354, 7356, 7357, 7359, 7361, 7363, 7364, 7365, 7366, 7367, 7368, 7369, 7370, 7371, 7373, 7376, 7379, 7382, 7383, 7384, 7386, 7388, 7389, 7393, 7395, 7398, 7400, 7403, 7404, 7407, 7408, 7409, 7411, 7419, 7421,
Detection: 6985, 6988, 6 994, 6997, 7000, 7004, 7008, 7015, 7019, 7021, 7069, 7079, 7099, 7104, 7117, 7120, 7145, 7151, 7153, 7162, 7164, 7169, 7201, 7209, 7234, 7238, 7252, 7286, 7288, 7300, 7305, 7313, 7323, 7327, 7338, 7343, 7346, 7352, 7377, 7380, 7396, 7399, 7406, 7412, 7414, 7418,

SEQ ID NO: 47

left Primer: 7422, 7425, 7427, 7435, 7438, 7439, 7440, 7442, 7444, 7448, 7449, 7456, 7459, 7462, 7463, 7465, 7466, 7467, 7470, 7471, 7474, 7476, 7477, 7480, 7481, 7485, 7486, 7489, 7490, 7493, 7496, 7500, 7503, 7508, 7512, 7518, 7519, 7520, 7522, 7524, 7525, 7527, 7528, 7529, 7532, 7540, 7542, 7543, 7545, 7549, 7552, 7554,
right Primer: 7423, 7426, 7428, 7430, 7431, 7432, 7433, 7434, 7436, 7445, 7446, 7447, 7450, 7451, 7452, 7453, 7454, 7455, 7457, 7460, 7464, 7468, 7472, 7475, 7478, 7479, 7482, 7483, 7484, 7487, 7488, 7492, 7494, 7495, 7497, 7498, 7499, 7501, 7505, 7506, 7507, 7509, 7510, 7511, 7513, 7515, 7516, 7517, 7521, 7523, 7526, 7530, 7533, 7534, 7535, 7536, 7537, 7538, 7539, 7541, 7544, 7546, 7547, 7548, 7550,
Detection: 7424, 7429, 7437, 7441, 7443, 7458, 7461, 7469, 7473, 7491, 7502, 7504, 7514, 7531, 7551, 7553,

SEQ ID NO: 18

left Primer: 7555, 7558, 7559, 7560, 7565, 7566, 7567, 7570, 7571, 7573, 7574, 7575, 7576, 7577, 7578, 7579, 7580, 7581, 7582, 7585, 7586, 7587, 7588, 7589, 7590, 7596, 7597, 7598, 7600, 7603, 7606, 7609, 7610, 7615, 7618, 7620, 7621, 7623, 7627, 7629, 7633, 7635, 7636, 7643, 7647, 7651, 7655, 7661, 7666, 7672, 7674, 7676, 7677, 7682, 7683, 7685, 7687, 7689, 7690, 7691, 7694, 7699, 7701, 7702, 7704, 7707, 7709, 7711, 7712, 7713, 7714, 7716, 7718, 7720, 7726, 7728, 7729, 7733, 7734, 7735, 7736, 7738, 7740, 7741, 7744, 7746, 7747, 7748, 7749, 7750, 7751, 7752, 753, 7754, 7755, 7756, 7758,
right Primer: 7556, 7561, 7563, 7568, 7583, 7584, 7591, 7593, 7594, 7595, 7599, 7601, 7604, 7607, 7608, 7611, 7613, 7614, 7616, 7617, 7619, 7622, 7624, 7626, 7628, 7631, 7637, 7638, 7639, 7640, 7641, 7642, 7644, 7645, 7646, 7649, 7650, 7652, 7654, 7656, 7657, 7658, 7659, 7660, 7662, 7663, 7665, 7667, 7669, 7670, 7671, 7673, 7675, 7678, 7679, 7680, 7681, 7684, 7686, 7688, 7692, 7695, 7697, 7698, 7700, 7703, 7705, 7708, 7710, 7715, 7717, 7719, 7721, 7722, 7723, 7724, 7725, 7727, 7730, 7731, 7732, 7737, 7739, 7742, 7743, 7745, 7757, 7759, 7760,
Detection: 7557, 7562, 7 564, 7569, 7572, 7592, 7602, 7605, 7612, 7625, 7630, 7632, 7634, 7648, 7653, 7664, 7668, 7693, 7696, 7706,

SEQ ID NO: 20

left Primer: 7761, 7764, 7770, 7774, 7775, 7778, 7782, 7786, 7788, 7795, 7797, 7799, 7805, 7807, 7809, 7810, 7811, 7815, 7817, 7819, 7820, 7822, 7823, 7824, 7828, 7832, 7834, 7837, 7839, 7843, 7845, 7851, 7852, 7857, 7860, 7863, 7865, 7868, 7874, 7881, 7885, 7889, 7892, 7894, 7896, 7900, 7902, 7904, 7907, 7909, 7910, 7913, 7917, 7922, 7925, 7926, 7928, 7933, 7934, 7937, 7941, 7945, 7946, 7948, 7950, 7951, 7952, 7953, 7954, 7955, 7956, 7957, 7963, 7964, 7968, 7971, 7972, 7973, 7976, 7979, 7980, 7981, 7982, 7983, 7985, 7986, 7987, 7988, 7991,
right Primer: 7762, 7765, 7767, 7768, 7771, 7773, 7776, 7779, 7781, 7783, 7784, 7785, 7789, 7791, 7792, 7793, 7794, 7796, 7798, 7800, 7802, 7803, 7808, 7812, 7813, 7814, 7816, 7818, 7821, 7825, 7827, 7829, 7830, 7833, 7835, 7836, 7838, 7840, 7841, 7842, 7844, 7846, 7847, 7848, 7849, 7853, 7854, 7855, 7856, 7858, 7861, 7864, 7866, 7869, 7870, 7871, 7872, 7873, 7875, 7877, 7878, 7879, 7880, 7882, 7884, 7886, 7888, 7890, 7897, 7899, 7903, 7905, 7912, 7914, 7916, 7918, 7919, 7920, 7921, 7923, 7929, 7931, 7932, 7936, 7938, 7939, 7940, 7942, 7943, 7944, 7947, 7949, 7958, 7960, 7962, 7965, 7967, 7969, 7970, 7974, 7977, 7989, 7990,

Detection: 7763, 7766, 7 769, 7772, 7777, 7780, 7787, 7790, 7801, 7804, 7806, 7826, 7831, 7850, 7859, 7862, 7867, 7876, 7883, 7887, 7891, 7893, 7895, 7898, 7901, 7906, 7908, 7911, 7915, 7924, 7927, 7930, 7935, 7959, 7961, 7966, 7975, 7978, 7984,

SEQ ID NO: 48

left Primer: 7992, 7995, 7996, 8000, 8003, 8006, 8007, 8010, 8012, 8015, 8016, 8018, 8019, 8026, 8027, 8030, 8034, 8035, 8038, 8039, 8043, 8044, 8046, 8049, 8050, 8052, 8053, 8055, 8056, 8062, 8064, 8066, 8067, 8068, 8069, 8070, 8071, 8074, 8076, 8077, 8078, 8079, 8080, 8081,
right Primer: 7993, 7997, 7999, 8001, 8004, 8005, 8008, 8009, 8011, 8013, 8017, 8020, 8021, 8022, 8023, 8024, 8025, 8028, 8031, 8033, 8036, 8040, 8042, 8047, 8051, 8054, 8057, 8059, 8060, 8061, 8065, 8072, 8073, 8075, 8082, 8083, 8084, 8085, 8087, 8088, 8089
Detection: 7994, 7998, 8 002, 8014, 8029, 8032, 8037, 8041, 8045, 8048, 8058, 8063, 8086,

SEQ ID NO: 50

left Primer: 8090, 8093, 8095, 8098, 8100, 8101, 8102, 8104, 8107, 8110, 8111, 8114, 8115, 8116, 8118, 8122, 8124, 8126, 8127, 8129, 8132, 8134, 8136, 8138, 8142, 8143, 8144, 8146, 8151, 8157, 8160, 8163, 8165, 8168, 8169, 8170, 8173, 8174, 8175, 8176, 8178, 8181, 8185, 8187, 8188, 8189, 8190, 8191, 8193, 8194, 8200, 8203, 8206, 8208, 8209, 8212, 8214, 8216,8217,8220,8222,8224,8228,8230,
right Primer: 8091, 8094, 8096, 8099, 8103, 8105, 8108, 8112, 8113, 8117, 8119, 8121, 8123, 8125, 8128, 8130, 8131, 8133, 8135, 8137, 8139, 8145, 8147, 8148, 8149, 8150, 8152, 8153, 8154, 8155, 8156, 8158, 8161, 8164, 8166, 8167, 8171, 8177, 8179, 8180, 8182, 8183, 8184, 8186, 8192, 8195, 8197, 8198, 8199, 8201, 8202, 8204, 8207, 8210, 8213, 8218, 8221, 8223,8225,8227,8229,8231,
Detection: 8092, 8097, 8106, 8109, 8120, 8140, 8141, 8159, 8162, 8172, 8196, 8205, 8211,8215,8219,8226,

SEQ ID NO:19

left Primer: 8232, 8235, 8236, 8237, 8238, 8244, 8245, 8247, 8250, 8251, 8253, 8254, 8255, 8256, 8258, 8259, 8260, 8263, 8267, 8269, 8270, 8273, 8274, 8277, 8280, 8281, 8289, 8292, 8293, 8294, 8295, 8296, 8297, 8298, 8301, 8305, 8306, 8310, 8311, 8312, 8315, 8317, 8318, 8322, 8323, 8324, 8327, 8328, 8329, 8330, 8333, 8336, 8339, 8340, 8347, 8349, 8350, 8352, 8353, 8356, 8359, 8360, 8366, 8369, 8370, 8372, 8374, 8376, 8377, 8379, 8381, 8382, 8383,
right Primer: 8233, 8239, 8240, 8242, 8243, 8246, 8248, 8252, 8257, 8261, 8264, 8266, 8268, 8271, 8275, 8278, 8282, 8284, 8285, 8286, 8287, 8288, 8291, 8299, 8302, 8303, 8304, 8307, 8309, 8313, 8314, 8316, 8319, 8321, 8325, 8326, 8331, 8332, 8334, 8335, 8337, 8341, 8342, 8343, 8344, 8345, 8346, 8348, 8351, 8354, 8357, 8361, 8362, 8363, 8364, 8365, 8367, 8371,8373,8375,8378,
Detection: 8234, 8241, 8 249, 8262, 8265, 8272, 8276, 8279, 8283, 8290, 8300, 8308, 8320,8338,8355,8358,8368,8380,

SEQ ID NO: 17

left Primer: 8384, 8387, 8390, 8391, 8397, 8398, 8401, 8402, 8405, 8406, 8409, 8414, 8418, 8419, 8422, 8425, 8428, 8431, 8432, 8433, 8435, 8437, 8440, 8444, 8449, 8452, 8454, 8455, 8457, 8462, 8464, 8466, 8468, 8469, 8472, 8475, 8476, 8478, 8479, 8481, 8484, 8487, 8488, 8490, 8493, 8496, 8497, 8498, 8501, 8505, 8508, 8511, 8513, 8515, 8517, 8518, 8519, 8521, 8522, 8523, 8526, 8528, 8530, 8531, 8532, 8534, 8536, 8541, 8544, 8547, 8551, 8554, 8556, 8558, 8560, 8562, 8563, 8566, 8567, 8568, 8570, 8571, 8574, 8575, 8576, 8577, 8580, 8581, 8585, 8588, 8591, 8594, 8601, 8602, 8603, 8608, 8609, 8613, 8614, 8617, 8618, 8619, 8620, 8623, 8624, 8628, 8629, 8630, 8633, 8634, 8635, 8636, 8638, 8643, 8645, 8648, 8654, 8657, 8658, 8659, 8660, 8661, 8662, 8666, 8670, 8672, 8674, 8676, 8681, 8683, 8686, 8688, 8689, 8691, 8692, 8693, 8695, 8696, 8700, 8701, 8702, 8703, 8704, 8705, 8709, 8716, 8717, 8719, 8722, 8725, 8727, 8728, 8729, 8732, 8733, 8734, 8735, 8736,
right Primer: 8385, 8388, 8392, 8394, 8395, 8399, 8403, 8407, 8410, 8411, 8412, 8413, 8415, 8416, 8420, 8423, 8424, 8426, 8429, 8434, 8436, 8438, 8442, 8445, 8446, 8447, 8448, 8450, 8451, 8456, 8458, 8460, 8461, 8463, 8465, 8467, 8470, 8473, 8474, 8477, 8480, 8482, 8485, 8486, 8489, 8491, 8492, 8494, 8499, 8500, 8502, 8503, 8504, 8506, 8510, 8512, 8514, 8516, 8520, 8524, 8527, 8529, 8533, 8535, 8537, 8539, 8542, 8545, 8546, 8548, 8550, 8552, 8555, 8557, 8564, 8565, 8569, 8572, 8578, 8582, 8583, 8586, 8587, 8589, 8592, 8595, 8596, 8597, 8598, 8599, 8600, 8604, 8606, 8607, 8610, 8612, 8615, 8616, 8621, 8625, 8627, 8631, 8632, 8637, 8639, 8640, 8641, 8642, 8644, 8646, 8647, 8649, 8650, 8651, 8652, 8653, 8655, 8663, 8665, 8667, 8668, 8669, 8671, 8673, 8675, 8677, 8678, 8679, 8680, 8682, 8684, 8685, 8687, 8690, 8694, 8697, 8698, 8699, 8706, 8707, 8708, 8710, 8711, 8712, 8713, 8714, 8715, 8718, 8720, 8723, 8726, 8730, 8737,
Detection: 8386, 8389, 8 393, 8396, 8400, 8404, 8408, 8417, 8421, 8427, 8430, 8439, 8441, 8443, 8453, 8459, 8471, 8483, 8495, 8507, 8509, 8525, 8538, 8540, 8543, 8549, 8553, 8559, 8561, 8573, 8579, 8584, 8590, 8593, 8605, 8611, 8622, 8626, 8656, 8664, 8721, 8724, 8731,

SEQ ID NO: 23

left Primer: 8738, 8741, 8744, 8747, 8750, 8751, 8753, 8754, 8755, 8756, 8759, 8760, 8762, 8763, 8766, 8767, 8768, 8769, 8770, 8772, 8775, 8776, 8777, 8779, 8782, 8783, 8784, 8785, 8786, 8787, 8788, 8789, 8790, 8793, 8794, 8796, 8798, 8799, 8801, 8803, 8804, 8806, 8807, 8809, 8812, 8813, 8815, 8820, 8823, 8826, 8829, 8830, 8832, 8835, 8836, 8837, 8842, 8845, 8846, 8847, 8851, 8853, 8856, 8857, 8859, 8861, 8866, 8739, 8878, 8879, 8880, 8884, 8891,8893,8895,8898,8900,8901,8909,8912,
right Primer: 8739, 8742, 8745, 8748, 8749, 8752, 8757, 8758, 8761, 8764, 8771, 8773, 8778, 8780, 8791, 8797, 8800, 8802, 8805, 8808, 8810, 8814, 8816, 8818, 8819, 8821, 8824, 8827, 8828, 8831, 8833, 8838, 8840, 8841, 8755, 8843, 8844, 8848, 8850, 8852, 8854, 8855, 8863, 8864, 8865, 8867, 8868, 8869, 8870, 8871, 8872, 8873, 8874, 8876, 8877, 8881, 8882, 8883, 8885, 8887, 8888, 8889, 8890, 8892, 8894, 8896, 8897, 8899, 8902, 8785, 8903, 8904, 8905,8906,8907,8908,8911,
Detection: 8740, 8743, 8746, 8765, 8774, 8781, 8792, 8795, 8811, 8817, 8822, 8825, 8834, 8839, 8849, 8858, 8860, 8862, 8875, 8886, 8910,

SEQ ID NO: 21

left Primer: 8913, 8917, 8920, 8923, 8924, 8929, 8931, 8934, 8935, 8936,
right Primer: 8914, 8916, 8918, 8921, 8925, 8927, 8928, 8930, 8932, 8933, 8937,
Detection: 8915, 8919, 8922, 8926,

SEQ ID NO: 32

left Primer: 8938, 8944, 8945, 8948, 8952, 8953, 8954, 8957, 8958, 8961, 8962, 8965, 8967,8969,8970,8971,8972,8977,
right Primer: 8939, 8941, 8942, 8943, 8946, 8949, 8950, 8951, 8955, 8960, 8963, 8964, 8966,8968,8974,8975,8976,8978,8979,
Detection: 8940, 8947, 8956, 8959, 8973,

SEQ ID NO: 33

left Primer: 8980, 8984, 8988, 8989, 8998, 9001, 9002, 9005, 9007, 9008, 9012, 9013, 9016, 9019, 9020, 9022, 9023, 9025, 9028, 9029, 9032, 9033, 9034, 9035, 9037, 9038, 9044, 9046,9047,9048,9049,9054,
right Primer: 8981, 8983, 8985, 8987, 8990, 8992, 8993, 8994, 8995, 8997, 8999, 9003, 9004, 9006, 9009, 9010, 9011, 9014, 9017, 9018, 9021, 9024, 9026, 9030, 9036, 9039, 9041, 9042,9043,9045,9050,9051, 9052,9053,
Detection: 8982, 8986, 8991, 8996, 9000, 9015, 9027, 9031, 9040,

SEQ ID NO: 34

left Primer: 9055, 9058, 9060, 9063, 9066, 9067, 9068, 9069, 9070, 9071, 9074, 9075, 9077, 9078, 9079, 9080, 9082, 9084, 9088, 9089, 9090, 9099, 9103, 9104, 9105, 9107, 9111,
right Primer: 9056, 9061, 9064, 9072, 9081, 9083, 9085, 9086, 9087, 9091, 9092, 9093, 9094, 9095, 9096, 9097, 9098, 9100, 9102, 9106, 9108, 9109, 9110, 9112,
Detection: 9057, 9059, 9062, 9065, 9073, 9076, 9101,

SEQ ID NO: 24

left Primer: 9113, 9116, 9118, 9125, 9126, 9128, 9129, 9130, 9136, 9141, 9143, 9148, 9150, 9153, 9157, 9158, 9159, 9163, 9164, 9165, 9167, 9168, 9171, 9173, 9177, 9180, 9181, 9182, 9183, 9185, 9187, 9188, 9189, 9191, 9192, 9193, 9194, 9197, 9198, 9200, 9206, 9207, 9208, 9209, 9211, 9216, 9218, 9219, 9220, 9221, 9222, 9223, 9226, 9228, 9229, 9230, 9231, 9232, 9233, 9234, 9237, 9238, 9240, 9243, 9246, 9248, 9253, 9256, 9257, 9260, 9263, 9264, 9266, 9269, 9271, 9279, 9280, 9281, 9282, 9283, 9284, 9290,
right Primer: 9114, 9119, 9121, 9122, 9123, 9124, 9127, 9131, 9133, 9134, 9135, 9137, 9138, 9139, 9140, 9142, 9144, 9146, 9147, 9149, 9151, 9152, 9154, 9155, 9156, 9160, 9162, 9166, 9169, 9172, 9174, 9175, 9176, 9178, 9184, 9186, 9190, 9195, 9199, 9201, 9202, 9203, 9204, 9205, 9212, 9214, 9215, 9217, 9224, 9227, 9235, 9239, 9241, 9244, 9247, 9249, 9251, 9252, 9254, 9258, 9261, 9265, 9267, 9270, 9273, 9274, 9275, 9276, 9277, 9278, 9285, 9286, 9287,9288,9289,9291,
Detection: 9115, 9117, 9120, 9132, 9145, 9161, 9170, 9179, 9196, 9210, 9213, 9225, 9236, 9242, 9245, 9250, 9255, 9259, 9262, 9268, 9272,

SEQ ID NO: 35

left Primer: 9292, 9295, 9297, 9300, 9302, 9303, 9304, 9307, 9308, 9310, 9311, 9313, 9319, 9321, 9323, 9324, 9328, 9333, 9334, 9337, 9338, 9339, 9342, 9344, 9348, 9351, 9352, 9354, 9355, 9357, 9360, 9367, 9371, 9374, 9377, 9379, 9383, 9384, 9385, 9388, 9389, 9390, 9393, 9394, 9398, 9401, 9402, 9406, 9408, 9409, 9410, 9413, 9415, 9416, 9417, 9418, 9419, 9421, 9422, 9425, 9429, 9433, 9434, 9435, 9436, 9437, 9439, 9440, 9446, 9452, 9453, 9454, 9455, 9457, 9458, 9459, 9462, 9466, 9467, 9468, 9470, 9474, 9479, 9482, 9487, 9488, 9489, 9492, 9495, 9497, 9499, 9500, 9504, 9505, 9508, 9513, 9514, 9519, 9522, 9523, 9524, 9526, 9528, 9532, 9533, 9534, 9535, 9537, 9538, 9541, 9542, 9543, 9545, 9546, 9548,
right Primer: 9293, 9296, 9298, 9301, 9305, 9309, 9312, 9314, 9316, 9317, 9318, 9320, 9322, 9325, 9326, 9327, 9329, 9331, 9332, 9335, 9336, 9340, 9345, 9347, 9349, 9353, 9356, 9358, 9361, 9363, 9364, 9365, 9368, 9370, 9372, 9375, 9376, 9378, 9380, 9382, 9386, 9391, 9395, 9397, 9399, 9400, 9403, 9405, 9407, 9411, 9412, 9414, 9420, 9423, 9424, 9426, 9428, 9430, 9431, 9432, 9438, 9441, 9443, 9444, 9445, 9447, 9448, 9449, 9450, 9451, 9456, 9460, 9461, 9464, 9465, 9469, 9471, 9473, 9475, 9477, 9478, 9480, 9483, 9485, 9490, 9493, 9494, 9496, 9498, 9501, 9502, 9503, 9506, 9509, 9511, 9512, 9515, 9516, 9517, 9518, 9520, 9521, 9525, 9527, 9529, 9530, 9536, 9539, 9544, 9547, 9549, 9550,
Detection: 9294, 9299, 9306, 9315, 9330, 9341, 9343, 9346, 9350, 9359, 9362, 9366, 9369, 9373, 9381, 9387, 9392, 9396, 9404, 9427, 9442, 9463, 9472, 9476, 9481, 9484, 9486, 9491, 9507, 9510, 9531, 9540,

SEQ ID NO: 25

left Primer: 9551, 9555, 9556, 9557, 9559, 9560, 9563, 9564, 9565, 9566, 9569, 9570, 9573, 9577, 9578, 9579, 9580, 9585, 9586, 9587, 9588, 9591, 9593, 9596,
right Primer: 9552, 9554, 9558, 9561, 9567, 9571, 9574, 9576, 9581, 9583, 9584, 9589, 9592, 9594, 9595,
Detection: 9553, 9562, 9568, 9572, 9575, 9582, 9590, 9597,

SEQ ID NO: 36

left Primer: 9598, 9602, 9604, 9605, 9608, 9612, 9613, 9614, 9617, 9618, 9621, 9622, 9625, 9626, 9627, 9632, 9633, 9638, 9640, 9643, 9644, 9645, 9646, 9651, 9654, 9656, 9657, 9658, 9661, 9662, 9665, 9666, 9667, 9668, 9672, 9673, 9675, 9676, 9677, 9680, 9683, 9686, 9688, 9691, 9694, 9698, 9699, 9702, 9705, 9707, 9710, 9711, 9715, 9719, 9720, 9722, 9723, 9726, 9729, 9732, 9733, 9735, 9737, 9738, 9741, 9744, 9745, 9746, 9747, 9748, 9749, 9750, 9754, 9755, 9756, 9758, 9759, 9768, 9771, 9772, 9776, 9777, 9781, 9782, 9784, 9787, 9789, 9790, 9792, 9796, 9797, 9800, 9805, 9806, 9812, 9815, 9818, 9821, 9823, 9825, 9827, 9831, 9832, 9835, 9837, 9839, 9841, 9842, 9844, 9845, 9851, 9853, 9854, 9855, 9857, 9860, 9862, 9863,9865,9871,9873,9875,9878,9879,
right Primer: 9599, 9601, 9603, 9606, 9607, 9609, 9611, 9615, 9619, 9623, 9624, 9628, 9630, 9631, 9634, 9635, 9637, 9639, 9641, 9647, 9649, 9650, 9652, 9653, 9659, 9660, 9663, 9664, 9670, 9671, 9679, 9682, 9684, 9687, 9690, 9692, 9693, 9695, 9697, 9700, 9701, 9703, 9706, 9708, 9712, 9713, 9714, 9716, 9717, 9718, 9721, 9724, 9727, 9730, 9734, 9739, 9742, 9751, 9753, 9757, 9760, 9761, 9762, 9763, 9764, 9765, 9766, 9767, 9769, 9773, 9775, 9778, 9780, 9783, 9786, 9788, 9791, 9793, 9794, 9795, 9798, 9801, 9803, 9804, 9807, 9808, 9809, 9810, 9811, 9813, 9816, 9817, 9819, 9820, 9822, 9826, 9828, 9830, 9833, 9836, 9838, 9840, 9843, 9846, 9847, 9848, 9849, 9850, 9852, 9856, 9858, 9859, 9861, 9864, 9866, 9867, 9868, 9869,9870,9872,9876,9877,9880,9881,
Detection: 9600, 9610, 9 616, 9620, 9629, 9636, 9642, 9648, 9655, 9669, 9674, 9678, 9681, 9685, 9689, 9696, 9704, 9709, 9725, 9728, 9731, 9736, 9740, 9743, 9752, 9770, 9774, 9779, 9785, 9799, 9802, 9814, 9824, 9829, 9834, 9874,

SEQ ID NO: 28

left Primer: 9882, 9886, 9890, 9891, 9894, 9900, 9902, 9904, 9908, 9911, 9912, 9915, 9917, 9919, 9923, 9925, 9928, 9935, 9936, 9940, 9943, 9949, 9953, 9954, 9956, 9957, 9958, 9962, 9963, 9964, 9968, 9969, 9975, 9978, 9981, 9988, 9989, 9990, 9991, 9992, 9995, 9997, 10011, 10012, 10014, 10017, 10018, 10031, 10032, 10033, 10034, 10035, 10037, 10041, 10042, 10046, 10050, 10054, 10058, 10059,
right Primer: 9883, 9885, 9887, 9889, 9892, 9893, 9895, 9896, 9897, 9898, 9899, 9901, 9903, 9905, 9907, 9909, 9913, 9914, 9916, 9918, 9920, 9921, 9922, 9924, 9926, 9929, 9931, 9932, 9933, 9934, 9937, 9938, 9939, 9941, 9942, 9944, 9945, 9946, 9947, 9948, 9950, 9952, 9955, 9959, 9960, 9961, 9966, 9967, 9970, 9972, 9973, 9974, 9976, 9979, 9980, 9982, 9983, 9984, 9985, 9986, 9987, 9993, 9994, 9996, 9998, 9999, 10000, 10001, 10002, 10003, 10004, 10005, 10006, 10007, 10008, 10009, 10010, 10013, 10015, 10016, 10019, 10020, 10021, 10022, 10023, 10025, 10026, 10027, 10028, 10030, 10036, 10038, 10040, 10043, 10044, 10045, 10047, 10048, 10049, 10051, 10053, 10055, 10056, 10057, 10060,
Detection: 9884, 9888, 9906, 9910, 9927, 9930, 9951, 9965, 9971, 9977, 10024, 10029, 10039, 10052,

SEQ ID NO: 37

left Primer: 10061, 10064, 10067, 10068, 10070, 10071, 10072, 10074, 10079, 10081, 10083, 10088, 10091, 10095, 10096, 10097, 10101, 10103, 10105, 10106, 10109, 1110, 1111, 10113, 10116, 10118, 10120, 10122, 10125, 10126, 10127, 10131, 10134, 10137, 10138, 10144, 10147, 10149, 10151, 10152, 10155, 10156, 10159, 10160, 10169, 10172, 10174, 10175, 10178, 10181, 10186, 10188, 10189, 10190, 10191, 10192, 10196, 10199, 10203, 10204, 10210, 10211, 10216, 10218, 10221, 10224, 10225, 10226, 10229, 10230, 10231, 10232, 10234, 10239, 10241, 10244, 10252, 10253, 10257, 10258, 10259, 10261, 10266, 10272, 10276,
right Primer: 10062, 10065, 10069, 10075, 10077, 10078, 10080, 10082, 10084, 10086, 10087, 10089, 10090, 10092, 10093, 10094, 10098, 10099, 10100, 10102, 10104, 10107, 10112, 10114, 10117, 10119, 10121, 10123, 10124, 10129, 10130, 10132, 10133, 10135, 10139, 10140, 10141, 10142, 10143, 10145, 10148, 10150, 10153, 10154, 10157, 10161, 10163, 10164, 10165, 10166, 10167, 10170, 10173, 10176, 10177, 10179, 10182, 10183, 10184, 10187, 10193, 10194, 10195, 10197, 10198, 10200, 10201, 10202, 10205, 10206, 10207, 10209, 10212, 10213, 10214, 10215, 10219, 10220, 10222, 10223, 10227, 10228, 10233, 10235, 10236, 10237, 10240, 10242, 10243, 10245, 10247, 10248, 10249, 10251, 10254, 10255, 10256, 10260, 10262, 10263, 10264, 10265, 10267, 10268, 10269, 10270, 10271, 10273, 10274, 10275, 10277, 10278,
Detection: 10063, 10066, 10073, 10076, 10085, 10108, 10115, 10128, 10136, 10146, 10158, 10162, 10168, 10171, 10180, 10185, 10208, 10217, 10238, 10246, 10250,

SEQ ID NO: 26

left Primer: 10279, 10282, 10285, 10287, 10291, 10292, 10297, 10299, 10301, 10304, 10306, 10308, 10310, 10314, 10317, 10318, 10320, 10322, 10327, 10329, 10330, 10333, 10335, 10336, 10337, 10340, 10342, 10348, 10350, 10351, 10354, 10358, 10359, 10360, 10362, 10366, 10369, 10372, 10373, 10374, 10376, 10378, 10383, 10385, 10391, 10398, 10403, 10407, 10410, 10413, 10415, 10419, 10422, 10425, 10427, 10428, 10429, 10430, 10432, 10433, 10434, 10437, 10438, 10439, 10442, 10443, 10445, 10446, 10447, 10448, 10452, 10453, 10454, 10455, 10456, 10460, 10463, 10466, 10467, 10469, 10470, 10473, 10475, 10477, 10480, 10484, 10488, 10490, 10494, 10495, 10497, 10501, 10502, 10503, 10507, 10508, 10510, 10517, 10518, 10520, 10522, 10524, 10525, 10528, 10529, 10533, 10536, 10537, 10540, 10541, 10545, 10547, 10551, 10553, 10556, 10559, 10560, 10563, 10564, 10565, 10568, 10571, 10574, 10576, 10579, 10581, 10583, 10588, 10589, 10590, 10592, 10593, 10596, 10597, 10601, 10602, 10603, 10605, 10607, 10609, 10610, 10612, 10613, 10617, 10618, 10621, 10624, 10627, 10630, 10632, 10634, 10637, 10638, 10639, 10640, 10641, 10643, 10645, 10647, 10653, 10655, 10657, 10659, 10661, 10662, 10663, 10664, 10665, 10666, 10667, 10668, 10669, 10672, 10673, 10674, 10675,
right Primer: 10280, 10283, 10286, 10288, 10290, 10293, 10295, 10298, 10300, 10302, 10305, 10307, 10309, 10311, 10312, 10313, 10315, 10319, 10321, 10323, 10325, 10326, 10328, 10331, 10338, 10341, 10343, 10344, 10345, 10346, 10347, 10349, 10352, 10355, 10356, 10357, 10361, 10363, 10365, 10367, 10370, 10375, 10377, 10379, 10380, 10381, 10382, 10384, 10386, 10387, 10388, 10390, 10392, 10394, 10395, 10399, 10401, 10402, 10404, 10406, 10408, 10411, 10412, 10414, 10416, 10417, 10418, 10420, 10423, 10426, 10431, 10435, 10440, 10444, 10449, 10451, 10457, 10459, 10461, 10462, 10464, 10465, 10468, 10471, 10476, 10478, 10481, 10483, 10485, 10487, 10489, 10491, 10492, 10493, 10498, 10499, 10500, 10504, 10506, 10509, 10511, 10513, 10514, 10515, 10516, 10519, 10521, 10523, 10526, 10530, 10531, 10532, 10534, 10535, 10538, 10539, 10542, 10543, 10544, 10546, 10548, 10550, 10552, 10554, 10557, 10558, 10561, 10566, 10569, 10572, 10573, 10577, 10578, 10580, 10582, 10584, 10586, 10594, 10598, 10600, 10604, 10606, 10611, 10614, 10616, 10619, 10622, 10625, 10626, 10628, 10631, 10633, 10635, 10642, 10644, 10646, 10648, 10649, 10650, 10651, 10652, 10654, 10656, 10658, 10660, 10670, 10671, 10676,
Detection: 10281, 10284, 10289, 10294, 10296, 10303, 10316, 10324, 10332, 10334, 10339, 10353, 10364, 10368, 10371, 10389, 10393, 10396, 10397, 10400, 10405, 10409, 10421, 10424, 10436, 10441, 10450, 10458, 10472, 10474, 10479, 10482, 10486, 10496, 10505, 10512, 10527, 10549, 10555, 10562, 10567, 10570, 10575, 10585, 10587, 10591, 10595, 10599, 10608, 10615, 10620, 10623, 10629, 10636, 10677,

SEQ ID NO: 38

left Primer: 10678, 10684,
right Primer: 10679, 10681, 10682, 10683, 10685, 10687, 10688, 10689,
Detection: 10680, 10686,

SEQ ID NO: 39

left Primer: 10690,
right Primer: 10691,
Detection: 10692,

SEQ ID NO: 27

left Primer: 10693, 10696, 10699, 10703, 10704, 10709, 10711, 10712, 10713, 10720, 10721, 10722, 10724, 10725, 10726, 10728, 10729, 10734, 10740, 10742, 10743, 10744, 10745, 10746, 10748, 10749, 10751, 10754, 10758, 10759, 10760, 10764, 10768, 10769, 10772, 10775, 10780, 10781, 10783, 10786, 10791, 10796, 10801, 10808, 10817, 10818, 10819, 10821, 10823, 10826, 10827, 10829, 10830, 10834, 10843, 10844, 10847, 10848, 10849, 10853, 10856, 10858, 10866, 10869, 10872, 10874, 10877, 10878, 108?9, 10882, 10884, 10885, 10889, 10892, 10893, 10896, 10900, 10901, 10903, 10905, 10907, 10909, 10910, 10911, 10914, 10916, 10919, 10926, 10930, 10932, 10933, 10934, 10938, 10941, 10942, 10943, 10944, 10946, 10947, 10953, 10955, 10956, 10958, 10959, 10962, 10967, 10968, 10972, 10975, 10977, 10978, 10981, 10984, 10985, 10988, 10992, 10995, 10997, 10999, 11000, 11001, 11006, 11009, 11013, 11016, 11020, 11025, 11033, 11036, 11040, 11041, 11042, 11043, 11045, 11046, 11048, 11052, 11055, 11060, 11061, 11062, 11064, 11065, 11067, 11070, 11071, 11072, 11078, 11079, 11081, 11085, 11093,
right Primer: 10694, 10697, 10700, 10702, 10705, 10707, 10710, 10714, 10716, 10717, 10718, 10719, 10723, 10727, 10730, 10732, 10733, 10735, 10737, 10738, 10739, 10741, 10747, 10750, 10752, 10755, 10757, 10761, 10762, 10763, 10765, 10767, 10770, 10773, 10776, 10778, 10779, 10782, 10784, 10788, 10789, 10790, 10792, 10793, 10794, 10798, 10799, 10800, 10802, 10804, 10805, 10806, 10807, 10809, 10811, 10812, 10813, 10814, 10815, 10816, 10820, 10822, 10824, 10831, 10833, 10835, 10837, 10838, 10839, 10840, 10841, 10842, 10845, 10846, 10850, 10851, 10854, 10857, 10859, 10860, 10861, 10862, 10863, 10864, 10865, 10868, 10870, 10871, 10873, 10875, 10880, 10881, 10883, 10886, 10888, 10890, 10894, 10897, 10899, 10902, 10904, 10906, 10908, 10912, 10915, 10917, 10920, 10922, 10924, 10925, 10927, 10928, 10929, 10931, 10935, 10937, 10939, 10945, 10948, 10950, 10951, 10952, 10954, 10957, 10960, 10963, 10965, 10966, 10969, 10970, 10971, 10973, 10976, 10979, 10980, 10982, 10986, 10987, 10989, 10991, 10993, 10996, 10998, 11002, 11004, 11005, 11007, 11010, 11012, 11014, 11015, 11017, 11019, 11021, 11022, 11023, 11026, 11027, 11028, 11029, 11030, 11031, 11032, 11034, 11037, 11039, 11044, 11047, 11049, 11050, 11051, 11053, 11054, 11056, 11058, 11059, 11063, 11066, 11068, 11069, 11073, 11074, 11075, 11076, 11077, 11080, 11082, 11083, 11084, 11086, 11087, 11088, 11089, 11090, 11091, 11092, 11094,
Detection: 10695, 10698, 10701, 10706, 10708, 10715, 10731, 10736, 10753, 10756, 10766, 10771, 10774, 10777, 10785, 10787, 10795, 10797, 10803, 10810, 10825, 10828, 10832, 10836, 10852, 10855, 10867, 10876, 10887, 10891, 10895, 10898, 10913, 10918, 10921, 10923, 10936, 10940, 10949, 10961, 10964, 10974, 10983, 10990, 10994, 11003, 11008, 11011, 11018, 11024, 11035, 11038, 11057, 11095,

SEQ ID NO: 40

left Primer: 11376, 11381, 11382, 11383, 11384, 11387, 11397, 11401, 11405, 11408, 11414, 11418, 11421, 11422, 11424, 11427, 11429, 11430,
right Primer: 11377, 11379, 11380, 11385, 11386, 11388, 11390, 11391, 11392, 11394, 11395, 11396, 11398, 11399, 11400, 11402, 11403, 11404, 11406, 11409, 11410, 11411, 11412, 11415, 11416, 11417, 11419, 11423, 11425, 11428, 11431, 11432, 11433,
Detection: 11378, 11389, 11393, 11407, 11413, 11420, 11426,

Light cycler dual probe assays:

SEQ ID NO: 41

left Primer: 11434, 11438, 11442, 11443, 11447, 11449, 11450, 11452, 11456, 11459, 11463, 11464, 11467, 11468, 11472, 11477, 11480, 11481, 11482, 11483, 11484, 11488, 11489, 11493, 11498, 11502, 11504, 11505, 11506, 11507, 11508, 11509, 11512, 11513, 11514, 11517, 11520, 11522, 11523, 11524, 11526, 11528, 11532, 11534,
right Primer: 11435, 11439, 11444, 11445, 11446, 11448, 11451, 11453, 11457, 11458, 11460, 11461, 11462, 11465, 11466, 11469, 11473, 11474, 11475, 11476, 11478, 11479, 11485, 11486, 11487, 11490, 11494, 11497, 11499, 11500, 11501, 11503, 11510, 11511, 11515, 11518, 11519, 11521, 11525, 11527, 11529,
Detection: 11436, 11440, 11454, 11470, 11491, 11495, 11530,
Anchor: 11437, 11441, 11455, 11471, 11492, 11496, 11516, 11531, 11533,

SEQ ID NO: 5

left Primer: 11535, 11539, 11540, 11541, 11548, 11550, 11551, 11552, 11555, 11556, 11557, 11560, 11561, 11562, 11568, 11569, 11571, 11572, 11573, 11577,
right Primer: 11536, 11542, 11545, 11546, 11547, 11549, 11553, 11554, 11563, 11566, 11570, 11574,
Detection: 11537, 11543, 11558, 11564, 11575,
Anchor: 11538, 11544, 11559, 11565, 11567, 11576,

SEQ ID NO: 6

left Primer: 11578, 11585, 11586, 11590, 11591, 11592, 11595, 11596, 11597, 11600, right Primer: 11579, 11582, 11587, 11593, 11594, 11598, 11601,
Detection: 11580, 11583, 11588,
Anchor: 11581, 11584, 11589, 11599,

SEQ ID NO: 8

left Primer: 11602, 11606, 11607, 11608, 11609, 11616, 11618, 11622, 11626, 11628, 11632, 11633,
right Primer: 11603, 11610, 11613, 11614, 11615, 11617, 11619, 11623, 11627, 11629, 11634,
Detection: 11604, 11611, 11620, 11624, 11630,
Anchor: 11605, 11612, 11621, 11625, 11631,

SEQ ID NO: 16

left Primer: 11635, 11640, 11641, 11642, 11643, 11647, 11648, 11654, 11655, 11656, 11657, 11658,
right Primer: 11636, 11639, 11644, 11645, 11646, 11649, 11652, 11653,
Detection: 11637, 11650,
Anchor: 11638, 11651,

132

SEQ ID NO: 42

left Primer: 11659, 11663, 11664, 11665, 11666, 11670, 11671, 11672, 11673, 11677, 11681, 11687, 11690, 11694, 11696, 11697, 11701, 11705, 11709, 11713, 11719, 11723, 11729, 11733, 11739, 11744, 11745, 11748, 11749, 11751, 11755, 11759, 11763, 11770, 11772, 11774, 11776, 11778, 11782, 11785,
right Primer: 11660, 11667, 11674, 11675, 11676, 11678, 11682, 11685, 11686, 11688, 11689, 11691, 11692, 11693, 11695, 11698, 11699, 11700, 11702, 11706, 11710, 11716, 11720, 11724, 11725, 11727, 11728, 11730, 11731, 11732, 11734, 11735, 11736, 11737, 11738, 11740, 11743, 11746, 11747, 11750, 11752, 11756, 11761, 11762, 11764, 11765, 11767, 11768, 11769, 11773,11775,11777,11779, 11783,11784,
Detection: 11661, 11668, 11679, 11683, 11703, 11707, 11711, 11714, 11717, 11721, 11726, 11741, 11753, 11757, 11766, 11771, 11780,
Anchor: 11662, 11669, 11680, 11684, 11704, 11708, 11712, 11715, 11718, 11722, 11742, 11754, 11758, 11760, 11781,

SEQ ID NO: 14

left Primer: 11786, 11790, 11791, 11792,
right Primer: 11787, 11793,
Detection: 11788, 11794,
Anchor: 11789, 11795,

SEQ ID NO: 43

left Primer: 11796, 11800, 11802, 11803, 11805, 11810, 11818, 11819, 11823, 11824, 11827, 11828, 11829, 11833, 11836, 11837, 11839, 11840, 11847, 11850, 11852, 11857, 11860, 11864, 11866,
right Primer: 11797, 11801, 11804, 11806, 11809, 11811, 11814, 11815, 11817, 11820, 11825, 11830, 11835, 11841, 11844, 11845, 11846, 11848, 11851, 11853, 11856, 11858, 11861, 11862, 11863, 11865,
Detection: 11798, 11807, 11812, 11816, 11821, 11831, 11842, 11854,
Anchor: 11799, 11808, 11813, 11822, 11826, 11832, 11834, 11838, 11843, 11849, 11855, 11859,

SEQ ID NO: 15

left Primer: 11867, 11871, 11876, 11878, 11882, 11888, 11891, 11894,
right Primer: 11868, 11872, 11875, 11877, 11879, 11883, 11886, 11887, 11889, 11890, 11892,11893,11895,
Detection: 11869, 11873, 11880, 11884, 11896,
Anchor: 11870, 11874, 11881, 11885, 11897,

SEQ ID NO:4

left Primer: 11898, 11902, 11903, 11904, 11908, 11909, 11911, 11912, 11913, 11914, 11915, 11917, 11918, 11922, 11926, 11927, 11930, 11932, 11933, 11937, 11941, 11942, 11946, 11947, 11948, 11949, 11950, 11951, 11952, 11955, 11959, 11968, 11972, 11976, 11978, 11979, 11980, 11981, 11986, 11988, 11989, 11991, 11992, 11995, 11997, 11998, 12001, 12002, 12004, 12005,
right Primer: 11899, 11905, 11910, 11916, 11919, 11923, 11924, 11925, 11928, 11929, 11931, 11934, 11938, 11943, 11953, 11954, 11956, 11960, 11963, 11964, 11967, 11969, 11973, 11977, 11982, 11985, 11987, 11990, 11993, 11994, 11996, 11999, 12000, 12003,
Detection: 11900, 11906, 11920, 11935, 11939, 11944, 11957, 11961, 11965, 11970, 11974, 11983,
Anchor: 11901, 11907, 11921, 11936, 11940, 11945, 11958, 11962, 11966, 11971, 11975, 11984,

SEQ ID NO: 7

left Primer: 12006, 12011, 12015, 12016, 12017, 12021, 12022, 12023, 12024, 12025, 12026, 12027, 12031, 12034, 12042, 12043, 12045, 12049, 12050, 12051, 12054, 12062, 12063, 12064, 12066, 12067, 12068, 12070, 12072, 12076, 12082, 12085, 12086, 12089, 12090, 12093, 12094, 12095, 12096, 12097, 12098, 12101, 12103, 12107, 12108, 12111, 12112, 12114, 12118, 12119, 12120, 12128,
right Primer: 12007, 12010, 12012, 12020, 12028, 12032, 12033, 12035, 12038, 12039, 12044, 12046, 12052, 12055, 12056, 12057, 12058, 12059, 12060, 12061, 12065, 12069, 12071, 12073, 12077, 12080, 12081, 12084, 12087, 12088, 12091, 12099, 12100, 12102, 12104, 12109, 12110, 12113, 12115, 12121, 12122, 12123, 12124, 12125, 12127, 12129,

Detection: 12008, 12013, 12018, 12029, 12036, 12040, 12047, 12074, 12078, 12083, 12105, 12116,
Anchor: 12009, 12014, 12019, 12030, 12037, 12041, 12048, 12053, 12075, 12079, 12036, 12092, 12106, 12117, 12126,

SEQ ID NO:1

left Primer: 12130, 12135, 12136, 12141, 12144, 12147, 12148, 12149, 12153, 12159, 12160,
right Primer: 12131, 12134, 12137, 12138, 12139, 12140, 12142, 12143, 12145, 12146, 12150, 12154, 12157, 12158, 12161, 12162, 12163,
Detection: 12132, 12151, 12155,
Anchor: 12133, 12152, 12156,

SEQ ID NO: 45

left Primer: 12164, 12168, 12171, 12176, 12181, 12187, 12190, 12194, 12196, 12197, 12199, 12201, 12202, 12209, 12213, 12216, 12220, 12221, 12223, 12225, 12226, 12231, 12233, 12239, 12243, 12244, 12250, 12254, 12256, 12257, 12259, 12266, 12270, 12271, 12272, 12274, 12276, 12277, 12278, 12280, 12284, 12285, 12288, 12289, 12290, 12291, 12295, 12296, 12302, 12308, 12313, 12317, 12321, 12322, 12323, 12324, 12325, 12327, 12328, 12330, 12331, 12332, 12336, 12337, 12339, 12343, 12346, 12349, 12353, 12354, 12355, 12359, 12361, 12363, 12367, 12369, 12370, 12372, 12376, 12377, 12379, 12380, 12381, 12382, 12383, 12385, 12389, 12393, 12398, 12400, 12402, 12403, 12406, 12408, 12409, 12410, 12412, 12415, 1241?, 12419, 12422, 12424, 12429, 12430, 12432, 12433, 12435, 12436, 12437, 12441, 12445, 12448, 12449, 12454, 12458, 12459, 12460,
right Primer: 12165, 12169, 12170, 12172, 12175, 12177, 12180, 12182, 12185, 12186, 12188, 12191, 12195, 12198, 12200, 12203, 12206, 12207, 12208, 12210, 12214, 12217, 12222, 12224, 12227, 12228, 12229, 12230, 12232, 12234, 12237, 12238, 12240, 12241, 12242, 12245, 12248, 12249, 12251, 12255, 12258, 12260, 12262, 12263, 12264, 12265, 12267, 12268, 12269, 12273, 12279, 12281, 12286, 12287, 12292, 12297, 12300, 12303, 12306, 12307, 12309, 12312, 12314, 12318, 12326, 12333, 12338, 12340, 12341, 12342, 12345, 12347, 12348, 12352, 12356, 12360, 12362, 12364, 12371, 12373, 12378, 12386, 12390, 12394, 12397, 12401, 12404, 12405, 12411, 12413, 12418, 12420, 12423, 12425, 12428, 12438, 12439, 12440, 12442, 12443, 12446, 12450, 12453, 12455,
Detection: 12166, 12173, 12178, 12183, 12189, 12192, 12204, 12211, 12218, 12235, 12246, 12252, 12282, 12293, 12298, 12301, 12304, 12310, 12315, 12319, 12334, 12350, 12357, 12365, 12374, 12387, 12391, 12395, 12416, 12426, 12444, 12451, 12456,
Anchor: 12167, 12174, 12179, 12184, 12193, 12205, 12212, 12215, 12219, 12236, 12247, 12253, 12261, 12275, 12283, 12294, 12299, 12305, 12311, 12316, 12320, 12329, 12335, 12344, 12351, 12358, 12366, 12368, 12375, 12384, 12388, 12392, 12396, 12399, 12407, 12414, 12421, 12427, 12431, 12434, 12447, 12452, 12457,

SEQ ID NO: 9

left Primer: 12461, 12465, 12467, 12468, 12472, 12474, 12477, 12480, 12484, 12486, 12488, 12489, 12490, 12493, 12495, 12499, 12501, 12504, 12506,
right Primer: 12462, 12469, 12475, 12478, 12481, 12483, 12485, 12487, 12492, 12494, 12496, 12497, 12498, 12500, 12502, 12505,
Detection: 12463, 12470, 12473, 12482,
Anchor: 12464, 12466, 12471, 12476, 12479, 12491, 12503,

SEQ ID NO: 46

left Primer: 12507, 12511, 12517, 12520, 12522, 12523, 12524, 12528, 12530, 12534, 12535, 12536, 12537, 12541, 12542, 12547, 12550, 12551, 12552, 12554, 12555, 12560, 12564, 12565, 12569, 12571, 12574, 12579, 12581, 12586, 12590, 12592, 12593, 12594, 12596, 12600, 12601, 12602, 12604, 12610, 12613, 12614, 12615, 12616, 12620, 12626, 12630,
right Primer: 12508, 12512, 12515, 12518, 12521, 12525, 12529, 12531, 12538, 12543, 12546, 12548, 12549, 12553, 12556, 12559, 12561, 12566, 12570, 12572, 12573, 12575, 12578, 12582, 12585, 12587, 12595, 12597, 12599, 12603, 12605, 12608, 12609, 12611, 12617, 12618, 12621, 12624, 12625, 12627,
Detection: 12509, 12513, 12516, 12514, 12532, 12539, 12544, 12557, 12562, 12567, 12576, 12583, 12588, 12591, 12606, 12612, 12622, 12628,
Anchor: 12510, 12514, 12519, 12526, 12527, 12533, 12540, 12545, 12558, 12563, 12568, 12577, 12580, 12584, 12589, 12598, 12607, 12619, 12623, 12629,

SEQ ID NO: 10

left Primer: 12631, 12636, 12637, 12638, 12639, 12643, 12647, 12648, 12652, 12654, 12660, 12664, 12666, 12670, 12672, 12675, 12677, 12680, 12686, 12691, 12692, 12695, 12699, 12700, 12701, 12703, 12705, 12709, 12710, 12714, 12715, 12716, 12718, 12719, 12720, 12724, 12725, 12728, 12732, 12738, 12739, 12743, 12744, 12745, 12747, 12748, 12752, 12757, 12763, 12764, 12766, 12767, 12768, 12771, 12776, 12777, 12781, 12785, 12787, 12788, 12791, 12792, 12796, 12797, 12800, 12804, 12808, 12813, 12815, 12816, 12817, 12822, 12825, 12828, 12831, 12833, 12834, 12837, 12839, 12841, 12842,
right Primer: 12632, 12635, 12640, 12641, 12644, 12649, 12653, 12655, 12658, 12661, 12662, 12663, 12665, 12667, 12671, 12673, 12678, 12679, 12681, 12682, 12683, 12684, 12685, 12687, 12688, 12689, 12690, 12693, 12694, 12696, 12702, 12704, 12706, 12712, 12713, 12717, 12721, 12726, 12729, 12730, 12731, 12733, 12736, 12737, 12740, 12746, 12749, 12750, 12751, 12753, 12754, 12755, 12756, 12758, 12760, 12761, 12762, 12765, 12769, 12770, 12772, 12775, 12778, 12782, 12786, 12789, 12790, 12793, 12794, 12795, 12798, 12799, 12801, 12805, 12809, 12812, 12814, 12818, 12821, 12823, 12824, 12826, 12827, 12832, 12835, 12836, 12838, 12840, 12843, 12844,
Detection: 12633, 12642, 12645, 12650, 12656, 12668, 12674, 12676, 12697, 12707, 12722, 12734, 12741, 12759, 12773, 12779, 12783, 12802, 12806, 12810, 12819, 12829,
Anchor: 12634, 12646, 12651, 12657, 12659, 12669, 12698, 12708, 12711, 12723, 12707, 12727, 12735, 12742, 12774, 12780, 12784, 12803, 12807, 12811, 12820, 12830,

SEQ ID NO: 11

left Primer: 12845, 12849, 12850, 12854, 12857, 12862, 12866, 12867, 12868, 12870, 12872, 12880, 12881, 12884, 12885, 12889, 12890, 12892, 12896, 12898, 12899, 12903, 12907, 12910, 12913, 12919, 12920, 12921, 12923, 12928, 12932, 12938, 12942, 12945, 12948, 12950, 12951, 12953, 12959,
right Primer: 12846, 12851, 12855, 12858, 12861, 12863, 12869, 12871, 12873, 12876, 12877, 12878, 12879, 12882, 12883, 12886, 12887, 12888, 12891, 12893, 12894, 12895, 12900, 12901, 12902, 12904, 12908, 12909, 12911, 12912, 12914, 12917, 12918, 12924, 12927, 12929, 12933, 12936, 12937, 12939, 12943, 12949, 12954, 12957, 12958, 12960,
Detection: 12847, 12852, 12859, 12864, 12874, 12905, 12915, 12925, 12930, 12934, 12940, 12944, 12946, 12955,
Anchor: 12848, 12853, 12856, 12860, 12865, 12875, 12897, 12906, 12916, 12922, 12926, 12931, 12935, 12941, 12930, 12947, 12952, 12956,

SEQ ID NO: 12

left Primer: 12961, 12965, 12969, 12975, 12977,
right Primer: 12962, 12966, 12967, 12968, 12970, 12971, 12972, 12973, 12974, 12976, 12978, 12981,
Detection: 12963, 12979,
Anchor: 12964, 12980,

SEQ ID NO: 13

left Primer: 12982, 12986, 12989, 12991, 12992, 12993, 12995, 12999, 13001, right Primer: 12983, 12987, 12996, 12997, 12998, 13000, 13002,
Detection: 12984, 13003,
Anchor: 12985, 12988, 12990, 12994, 13004,

SEQ ID NO:3

left Primer: 13005, 13009, 13013, 13017, 13019, 13025, 13029, 13030, 13032, 13033, 13034, 13037, 13040, 13042, 13043, 13044, 13048, 13049, 13051, 13052, 13053, 13057, 13062, 13064, 13065, 13066, 13067, 13072, 13073, 13076, 13078, 13082, 13083, 13085, 13087, 13088, 13089, 13092, 13096, 13100, 13101, 13102, 13105, 13108, 13109, 13110, 13114, 13117, 13118, 13124, 13129, 13131, 13132, 13138, 13144, 13146, 13147, 13148, 13152, 3156, 3159, 13164, 13167, 13168, 13170, 13171, 13172, 13173, 13174, 13184, 13185,
right Primer: 13006, 13010, 13014, 13018, 13020, 13021, 13022, 13023, 13024, 13026, 13031, 13035, 13036, 13038, 13039, 13041, 13045, 13050, 13054, 13058, 13061, 13063, 13068, 13069, 13070, 13071, 13074, 13075, 13077, 13079, 13084, 13086, 13090, 13091, 13093, 13097, 13104, 13107, 13111, 13115, 13119, 13121, 13122, 13123, 13125, 13126, 13127, 13128, 13130, 13133, 13137, 13139, 13142, 13143, 13145, 13149, 13153, 13157, 13158, 13160, 13163, 13165, 13166, 13169, 13175, 13176, 13177, 13178, 13179, 13180, 13181, 13182, 13183, 13186, 13189, 13190,
Detection: 13007, 13011, 13015, 13027, 13046, 13055, 13059, 13080, 13094, 13098, 13112, 13120, 13134, 13140, 13150, 13154, 13161, 13187,
Anchor: 13008, 13012, 13016, 13028, 13047, 13056, 13060, 13081, 13095, 13099, 13103, 13106, 13113, 13116, 13135, 13136, 13141, 13151, 13155, 13162, 13188,

SEQ ID NO: 47

left Primer: 13191, 13195, 13196, 13197, 13199, 13200, 13204, 13206,
right Primer: 13192, 13198, 13201,
Detection: 13193, 13202,
Anchor: 13194, 13203, 13205,

SEQ ID NO: 18

left Primer: 13207, 13212, 13213, 13226, 13228, 13231, 13235, 13240, 13245, 13247, 13248, 13250, 13254, 13256, 13258, 13264, 13265, 13268, 13269, 13273, 13274, 13277, 13279, 13280, 13281, 13286,
right Primer: 13208, 13211, 13214, 13217, 13220, 13222, 13224, 13225, 13227, 13229, 13232, 13233, 13234, 13237, 13239, 13241, 13242, 13244, 13246, 13249, 13251, 13255, 13257, 13259, 13262, 13263, 13266, 13267, 13270, 13275, 13276, 13282, 13285,
Detection: 13209, 13215, 13218, 13236, 13252, 13260, 13271, 13283,
Anchor: 13210, 13216, 13219, 13221, 13223, 13230, 13238, 13243, 13253, 13261, 13272, 13278, 13284,

SEQ ID NO: 32

left Primer: 13641, 13645, 13646, 13649, 13650, 13652, 13653, 13654, 13655, 13658, 13659,
right Primer: 13642, 13647, 13648, 13656,
Detection: 13643,
Anchor: 13644, 13651, 13657,

SEQ ID NO: 33

left Primer: 13660,
right Primer: 13661,
Detection: 13662,
Anchor: 13663,

SEQ ID NO: 34

left Primer: 13664, 13669, 13670, 13671, 13672, 13673, 13675, 13676, 13677, 13681, 13683, 13685, 13687, 13689, 13691, 13693, 13696,
right Primer: 13665, 13668, 13674, 13678, 13682, 13684, 13686, 13688, 13690, 13692, 13694, 13695,
Detection: 13666, 13679,
Anchor: 13667, 13680,

SEQ ID NO: 24

left Primer: 13697, 13701, 13702, 13703, 13709, 13713, 13714, 13715, 13719, 13721, 13722, 13726, 13728, 13732, 13735, 13740,
right Primer: 13698, 13704, 13705, 13706, 13707, 13708, 13710, 13716, 13717, 13718, 13720, 13723, 13724, 13725, 13727, 13729, 13733, 13734, 13736, 13739, 13741, 13744,

Detection: 13699, 13711, 13730, 13737, 13742,
Anchor: 13700, 13712, 13731, 13738, 13743,

SEQ ID NO: 35

left Primer: 13745, 13749, 13751, 13752, 13753, 13754, 13758, 13762, 13765, 13766, 13768, 13772, 13773, 13774, 13777, 13778, 13779, 13783, 13785, 13790, 13791, 13797, 13802, 13803, 13805, 13806, 13807, 13809, 13812, 13814, 13816, 13817, 13818, 13819, 13820, 13823, 13824, 13826, 13828,
right Primer: 13746, 13755, 13759, 13763, 13764, 13767, 13769, 13775, 13776, 13780, 13784, 13786, 13788, 13789, 13792, 13795, 13796, 13798, 13800, 13804, 13808, 13813, 13815, 13821, 13822, 13829,
Detection: 13747, 13756, 13760, 13781, 13793, 13801, 13810, 13830,
Anchor: 13748, 13750, 13757, 13761, 13770, 13771, 13782, 13787, 13794, 13799, 13811, 13825, 13827, 13831,

SEQ ID NO: 36

left Primer: 13832, 13837, 13841, 13844, 13845, 13846, 13849, 13854, 13855, 13856, 13857, 13861, 13865, 13869, 13871, 13875, 13879, 13880,
right Primer: 13833, 13836, 13838, 13843, 13847, 13850, 13853, 13858, 13862, 13866, 13870, 13872, 13876, 13881, 13882,
Detection: 13834, 13839, 13851, 13859, 13863, 13867, 13873, 13877,
Anchor: 13835, 13840, 13842, 13848, 13852, 13860, 13864, 13868, 13874, 13878,

SEQ ID NO: 28

left Primer: 13883, 13889, 13894, 13902, 13903, 13910, 13911, 13914, 13915, 13916, 13928, 13929, 13930, 13931, 13935, 13936, 13937, 13944, 13947, 13948, 13950, 13960, 13961, 13962, 13964, 13965, 13966, 13968, 13970, 13971, 13972, 13976, 13978,
right Primer: 13884, 13887, 13890, 13893, 13895, 13898, 13900, 13904, 13905, 13906, 13907, 13908, 13909, 13912, 13913, 13917, 13920, 13921, 13922, 13923, 13924, 13925, 13926, 13932, 13938, 13941, 13942, 13943, 13945, 13946, 13949, 13953, 13954, 13956, 13957, 13958, 13959, 13963, 13967, 13969, 13974, 13975, 13977, 13980,
Detection: 13885, 13886, 13891, 13896, 13901, 13918, 13927, 13933, 13939, 13951,
Anchor: 13886, 13888, 13892, 13897, 13899, 13901, 13919, 13934, 13940, 13952, 13955, 13973, 13979,

SEQ ID NO: 37

left Primer: 13981, 13988, 13989, 13990, 13992, 13994, 13995, 13999,
right Primer: 13982, 13985, 13986, 13987, 13991, 13993, 13996, 14000, 14001, 14002, 14003,
Detection: 13983, 13997,
Anchor: 13984, 13998,

SEQ ID NO: 26

left Primer: 14004, 14009, 14014, 14017, 14020, 14024, 14026, 14029, 14035, 14036, 14040, 14043, 14045, 14051, 14057, 14058, 14059, 14063, 14065, 14066, 14067, 14068, 14069, 14070, 14071, 14073, 14074, 14078, 14079, 14080, 14081, 14082, 14083, 14084, 14085, 14087, 14094, 14095, 14100, 14108, 14109, 14113, 14114, 14118, 14120, 14121, 14122, 14130, 14133, 14138, 14141, 14142, 14143, 14147,
right Primer. 14005, 14008, 14010, 14013, 14015, 14018, 14021, 14025, 14027, 14028, 14030, 14033, 14034, 14037, 14038, 14039, 14041, 14042, 14044, 14046, 14049, 14050, 14052, 14055, 14056, 14060, 14064, 14072, 14077, 14086, 14088, 14089, 14090, 14091, 14092, 14093, 14096, 14097, 14098, 14099, 14101, 14102, 14103, 14104, 14105, 14106, 14107, 14110, 14115, 14123, 14126, 14127, 14132, 14134, 14135, 14136, 14137, 14139, 14140, 14144, 14145, 14148,
Detection: 14006, 14011, 14019, 14022, 14031, 14047, 14053, 14061, 14075, 14111, 14116, 14124, 14149,
Anchor: 14007, 14012, 14016, 14023, 14032, 14048, 14054, 14062, 14076, 14112, 14117, 14119, 14125, 14128, 14129, 14131, 14146, 14150,

SEQ ID NO: 27

left Primer: 14151, 14155, 14157, 14161, 14166, 14171, 14175, 14183, 14187, 14190, 14192, 14194, 14195, 14196, 14200, 14201,
right Primer: 14152, 14156, 14158, 14162, 14165, 14167, 14170, 14172, 14176, 14179, 14180, 14182, 14184, 14185, 14186, 14188, 14189, 14191, 14193, 14197, 14198, 14199, 14202, 14203,
Detection: 14153, 14159, 14163, 14168, 14173, 14177,
Anchor: 14154, 14160, 14164, 14169, 14174, 14178, 14181,

SEQ ID NO: 31

left Primer: 14204, 14210, 14211, 14216, 14217, 14223, 14226, 14228, 14229, 14230, 14233, 14235, 14239, 14241, 14243, 14244, 14250, 14254, 14255, 14256, 14257, 14260, 14261, 14262, 14267, 14268, 14269, 14273, 14274, 14277, 14281, 14284, 14286, 14289, 14292, 14293, 14297, 14299, 14301, 14304, 14305, 14306, 14312, 14316, 14320, 14322, 14324, 14328, 14329, 14331, 14337, 14342, 14344, 14346, 14347, 14349, 14350, 14353, 14356, 14361, 14363, 14364, 14365, 14366,
right Primer: 14205, 14208, 14212, 14215, 14218, 14221, 14224, 14225, 14227, 14231, 14236, 14240, 14242, 14245, 14246, 14247, 14251, 14263, 14266, 14270, 14278, 14282, 14283, 14285, 14287, 14290, 14291, 14294, 14298, 14300, 14302, 14303, 14307, 14310, 14313, 14317, 14323, 14325, 14327, 14330, 14334, 14338, 14340, 14341, 14345, 14348, 14351, 14352, 14354, 14355, 14357, 14358, 14359, 14360, 14367,
Detection: 14206, 14213, 14219, 14237, 14248, 14252, 14258, 14264, 14271, 14275, 14279, 14295, 14308, 14311, 14314, 14318, 14332, 14335, 14339, 14343,
Anchor: 14207, 14209, 14214, 14220, 14222, 14232, 14234, 14238, 14249, 14253, 14259, 14265, 14272, 14276, 14280, 14288, 14296, 14309, 14315, 14319, 14321, 14326, 14333, 14336, 14362,

SEQ ID NO: 40

left Primer: 14368, 14372, 14373, 14375, 14386,
right Primer: 14369, 14374, 14376, 14379, 14380, 14381, 14382, 14383, 14384, 14385, Detection: 14370, 14377,
Anchor: 14371, 14378,

In an alternative most preferred embodiment of the method, the subsequent amplification of d) is carried out in the presence of *blocking oligonucleotides,* as described above. Said *blocking oligonucleotides* comprising a sequence having a length of at least 9 nucleotides which hybridizes to a treated nucleic acid sequence according to one of SEQ ID NOS:304 to SEQ ID NO:535 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NOS:1 to SEQ ID NO:64 is carried out by means of real-time detection methods as described above.

In a further preferred embodiment of the method the sequence(s) of the blocking oligonucleotides are selected from the group consisting of SEQ ID NOS:2114, 2119, 2124, 2125, 2127, 2132, 2133, 2135, 2137, 2138, 2139, 2140, 2141, 2142, 2143, 2144, 2146, 2147, 2148, 2149, 2150, 2151, 2152, 2153, 2154, 2155, 2156, 2157, 2158, 2159, 2160, 2031, 2082, 2087, 2089, 2094, 2095, 2096, 2097, 2099, 2107, 1980, 2018, 2022, 2023, 2026, 1939, 1966, 1973, 1974, 1975, 1926, 1930, 1931, 1932, 1933, 1934, 1903, 1917, 1921, 1834, 1896, 1805, 1819, 1821, 1823, 1824, 1825, 1826, 1827, 1828, 1829, 1770, 1782, 1785, 1786, 1788, 1789, 1790, 1792, 1795, 1796, 1797, 1798, 1799, 1800, 1748, 1759, 1760, 1761, 1762, 1763, 1764, 1765, 1724, 1741, 1597, 1609, 1610, 1611, 1612, 1613, 1614, 1615, 1616, 1617, 1618, 1619, 1620, 1621, 1622, 1623, 1624, 1625, 1626, 1627, 1628, 1629, 1630, 1631, 1633, 1634, 1635, 1636, 1637, 1638, 1639, 1640, 1641, 1642, 1643, 1644, 1645, 1646, 1647, 1648, 1649, 1650, 1651, 1652, 1653, 1654, 1655, 1656, 1657, 1658, 1659, 1660, 1661, 1662, 1663, 1668, 1684, 1685, 1686, 1687, 1688, 1689, 1690, 1691, 1692, 1693, 1694, 1695, 1696, 1697, 1698, 1699, 1700, 1701, 1706, 1712, 1713, 1715, 1716, 1717, 1718, 1719, 1571, 1579, 1581, 1584, 1585, 1586, 1587, 1588, 1591, 1592, 1537, 1555, 1556, 1557, 1558, 1559, 1560, 1561, 1562, 1563, 1564, 1565, 1566, 1452, 1455, 1456, 1457, 1458, 1463, 1514, 1515, 1516, 1517, 1518, 1519, 1524, 1525, 1526, 1527, 1528, 1529, 1531, 1532, 1372, 1423, 1424, 1425, 1427, 1432, 1433, 1434, 1435, 1436, 1437, 1440, 1441, 1442, 1443, 1444, 1445, 1446, 1447, 1343, 1358, 1359, 1360, 1361, 1362, 1363, 1364, 1365, 1366, 1367, 1310, 1315, 1316, 1317, 1318, 1319, 1320, 1321, 1322, 1324, 1325, 1327, 1328, 1330, 1332, 1334, 1337, 1338, 1276, 1290, 1305, 1203, 1228, 1238, 1239, 1240, 1247, 1248, 1255, 1256, 1265, 1268, 1269, 1164, 1171, 1172, 1173, 1174, 1176, 1177, 1178, 1179, 1180, 1181, 1182, 1183, 1184, 1185, 1186, 1187, 1188, 1189, 1190, 1191, 1192, 1193, 1194, 1195, 1196, 1197, 1198, 2725, 2748, 2749, 2750, 2710, 2717, 2718, 2719, 2720, 2721, 2684, 2699, 2703, 2704, 2705, 2706, 2658, 2662, 2679, 2603, 2613, 2617, 2585, 2594, 2595, 2596, 2597, 2598, 2599, 2489, 2497, 2498, 2499, 2500, 2501, 2504, 2505, 2507, 2508, 2510, 2511, 2512, 2513, 2514, 2515, 2519, 2520, 2521, 2522, 2525, 2526, 2527, 2528, 2529, 2530, 2531, 2532, 2534, 2535, 2536, 2537, 2539, 2540, 2541, 2542, 2543, 2544, 2545, 2546, 2547, 2550, 2551, 2552, 2553, 2554, 2558, 2563, 2564, 2572, 2580, 2581, 2472, 2482, 2484, 2408, 2464, 2465, 2466, 2467, 2468, 2339, 2349, 2367, 2370, 2372, 2373, 2374, 2375, 2376, 2377, 2378, 2379, 2380, 2382, 2386, 2387, 2389, 2393, 2394, 2395, 2396, 2397, 2398, 2402, 2403, 2404, 2327, 2333, 2334, 2335, 2305, 2315, 2318, 2260, 2268, 2270, 2271, 2272, 2273, 2274, 2275, 2276, 2277, 2278, 2279, 2280, 2281, 2282, 2283, 2284, 2285, 2286, 2287, 2288, 2289, 2290, 2291, 2292, 2293, 2294, 2295, 2296, 2297, 2298, 2299, 2300, 2301, 2232, 2245, 2246, 2248, 2249, 2250, 2251, 2252, 2254, 2255, 2256, 2210, 2218, 2219, 2221, 2222, 2223, 2227, 2228, 2202, 2204, 2205, 2206, 2164, 2180, 2183, 2187, 2188, 2189, 2190, 2191, 2192, 2193, 2194, 2195, 2196, 2197 and 2198.

Step e) of the method, namely the detection of the specific amplificates indicative of the methylation status of one or more CpG positions according to SEQ ID NOS:1 to SEQ ID NO:64 is carried out by means of real-time detection methods as described above, and wherein the sequence of said hybridization probes is selected from the group consisting of SEQ ID NOS:1162, 1163, 1201, 1202, 1208, 1211, 1217, 1274, 1288, 1275, 1289, 1299, 1308, 1309, 1341, 1342, 1347, 1351, 1354, 1372, 1423, 1424, 1425, 1427, 1432, 1433, 1434, 1435, 1436, 1437, 1440, 1441, 1442, 1443, 1444, 1445, 1446, 1447, 1370, 1371, 1400, 1450, 1461, 1451, 1462, 1467, 1470, 1473, 1475, 1535, 1536, 1542, 1544, 1569, 1570, 1583, 1590, 1595, 1666, 1704, 1596, 1601, 1603, 1667, 1705, 1722, 1723, 1727, 1735, 1746, 1747, 1768, 1769, 1773, 1803, 1804, 1807, 1810, 1832, 1833, 1836, 1867, 1871, 1901, 1902, 1908, 1911, 1924, 1925, 1937, 1938, 1943, 1946, 1949, 1956, 1957, 1978, 1979, 1986, 1992, 1998, 2001, 2003, 2010, 2020, 2025, 2029, 2030, 2113, 2163, 2201, 2209, 2231, 2259, 2304, 2326, 2338, 2348, 2351, 2369, 2407, 2471, 2488, 2557, 2584, 2602, 2616, 2657, 2661, 2664, 2671, 2683, 2709 and SEQ ID NO: 2724.

For each genomic sequence listed, the following oligonucleotides as detailed in the sequence listing may be used for a combined Blocking oligonucleotide-RealTime analysis:

Lightcycler dual probe detection assays:

SEQ ID NO: 41

left Primer: 1161, 1165, 1166, 1167,
right Primer: 1160, 1168, 1169, 1170, 1175,
Blocker: 1164, 1171, 1172, 1173, 1174, 1176, 1177, 1178, 1179, 1180, 1181, 1182, 1183, 1184, 1185, 1186, 1187, 1188, 1189, 1190, 1191, 1192, 1193, 1194, 1195, 1196, 1197, 1198,
Detection: 1162,
Anchor: 1163,

SEQ ID NO: 6

left Primer: 1200, 1204, 1205, 1206, 1207, 1209, 1210, 1212, 1213, 1214, 1215, 1216, 1218,
right Primer: 1199, 1219, 1220, 1221, 1222, 1223, 1224, 1225, 1226, 1227, 1229, 1230, 1231, 1232, 1233, 1234, 1235, 1236, 1237, 1241, 1242, 1243, 1244, 1245, 1246, 1249, 1250, 1251, 1252, 1253, 1254, 1257, 1258, 1259, 1260, 1261, 1262, 1263, 1264, 1266, 1267, 1270, 1271,
Blocker: 1203, 1228, 1238, 1239, 1240, 1247, 1248, 1255, 1256, 1265, 1268, 1269, Detection: 1201,
Anchor: 1202, 1208, 1211, 1217,

SEQ ID NO: 14

left Primer: 1273, 1277, 1278, 1279, 1280, 1281, 1282, 1283, 1287, 1291, 1292, 1293, 1294, 1295, 1296, 1297, 1298, 1300, 1301, 1302, 1303,
right Primer: 1272, 1284, 1285, 1286, 1304,
Blocker: 1276, 1290, 1305,
Detection: 1274, 1288,
Anchor: 1275, 1289, 1299,

SEQ ID NO: 7

left Primer: 1307, 1311,
right Primer: 1306, 1312, 1313, 1314, 1323, 1326, 1329, 1331, 1333, 1335, 1336,
Blocker: 1310, 1315, 1316, 1317, 1318, 1319, 1320, 1321, 1322, 1324, 1325, 1327, 1328, 1330, 1332, 1334, 1337, 1338,

Detection: 1308,
Anchor: 1309,

SEQ ID NO: 44

left Primer: 1340, 1344, 1345, 1346, 1348, 1349, 1350, 1352, 1353, 1355,
right Primer: 1339, 1356, 1357,
Blocker: 1343, 1358, 1359, 1360, 1361, 1362, 1363, 1364, 1365, 1366, 1367, Detection: 1341,
Anchor: 1342, 1347, 1351, 1354,

SEQ ID NO:1

left Primer: 1369, 1373, 1374, 1375, 1376, 1377, 1378, 1379, 1380, 1381, 1382, 1383, 1384, 1385, 1386, 1387, 1388, 1389, 1390, 1391, 1392, 1393, 1394, 1395, 1396, 1397, 1398, 1399, 1401, 1402, 1403, 1404, 1405, 1406, 1407, 1408, 1409, 1410, 1411, 1412, 1413, 1414, 1415,1416,1417,1418,1419,1420,1421,
right Primer: 1368, 1422, 1426, 1428, 1429, 1430, 1431, 1438, 1439,
Blocker: 1372, 1423, 1424, 1425, 1427, 1432, 1433, 1434, 1435, 1436, 1437, 1440, 1441, 1442, 1443, 1444, 1445, 1446, 1447,
Detection: 1370,
Anchor: 1371, 1400,

SEQ ID NO: 9

left Primer: 1449, 1453, 1454, 1460, 1464, 1465, 1466, 1468, 1469, 1471, 1472, 1474, 1476, 1477, 1478, 1479, 1480, 1481, 1482, 1483, 1484, 1485, 1486, 1487, 1488, 1489, 1490, 1491, 1492, 1493, 1494, 1495, 1496, 1497, 1498, 1499, 1500, 1501, 1502, 1503, 1504, 1505, 1506, 1507, 1508, 1509, 1510,
right Primer: 1448, 1459, 1511, 1512, 1513, 1520, 1521, 1522, 1523, 1530, Blocker: 1452, 1455, 1456, 1457, 1458, 1463, 1514, 1515, 1516, 1517, 1518, 1519, 1524, 1525, 1526, 1527, 1528, 1529, 1531, 1532,
Detection: 1450, 1461,
Anchor: 1451, 1462, 1467, 1470, 1473, 1475,

SEQ ID NO: 46

left Primer: 1534, 1538, 1539, 1540, 1541, 1543, 1545, 1546, 1547, 1548, 1549, 1550, 1551, 1552, 1553, 1554,
right Primer: 1533,
Blocker: 1537, 1555, 1556, 1557, 1558, 1559, 1560, 1561, 1562, 1563, 1564, 1565, 1566,
Detection: 1535,
Anchor: 1536, 1542, 1544,

SEQ ID NO: 10

left Primer: 1568, 1572, 1573,
right Primer: 1567, 1574, 1575, 1576, 1577, 1578, 1580, 1582, 1589,
Blocker: 1571, 1579, 1581, 1584, 1585, 1586, 1587, 1588, 1591, 1592,
Detection: 1569,
Anchor: 1570, 1583, 1590,

SEQ ID NO: 12

left Primer: 1594, 1598, 1599, 1600, 1602, 1604, 1605, 1606, 1607, 1665, 1669, 1670, 1671, 1672, 1673, 1674, 1675, 1676, 1677, 1678, 1679, 1680, 1681, 1682, 1703, 1707, 1708,
right Primer: 1593, 1608, 1632, 1664, 1683, 1702, 1709, 1710, 1711, 1714,
Blocker: 1597, 1609, 1610, 1611, 1612, 1613, 1614, 1615, 1616, 1617, 1618, 1619, 1620, 1621, 1622, 1623, 1624, 1625, 1626, 1627, 1628, 1629, 1630, 1631, 1633, 1634, 1635, 1636, 1637, 1638, 1639, 1640, 1641, 1642, 1643, 1644, 1645, 1646, 1647, 1648, 1649, 1650, 1651, 1652, 1653, 1654, 1655, 1656, 1657, 1658, 1659, 1660, 1661, 1662, 1663, 1668, 1684, 1685, 1686, 1687, 1688. 1689, 1690, 1691, 1692, 1693, 1694, 1695, 1696, 1697, 19698, 1699, 1700, 1701, 1706, 1712, 1713, 1715, 1716, 1717, 1718, 1719,

Detection: 1595, 1666, 1704,
Anchor: 1596, 1601, 1603, 1667, 1705,

SEQ ID NO: 13

left Primer: 1721, 1725, 1726, 1728, 1729, 1730, 1731, 1732, 1733, 1734, 1736, 1737, right Primer: 1720, 1738, 1739, 1740, 1742, 1743,
Blocker: 1724, 1741,
Detection: 1722,
Anchor: 1723, 1727, 1735,

SEQ ID NO:3

left Primer: 1745, 1749, 1750, 1751, 1752, 1753, 1754, 1755, 1758,
right Primer: 1744, 1756, 1757,
Blocker: 1748, 1759, 1760, 1761, 1762, 1763, 1764, 1765,
Detection: 1746,
Anchor: 1747,

SEQ ID NO: 18

left Primer: 1767, 1771, 1772, 1774, 1775, 1776, 1777, 1778,
right Primer: 1766, 1779, 1780, 1781, 1783, 1784, 1787, 1791, 1793, 1794,
Blocker: 1770, 1782, 1785, 1786, 1788, 1789, 1790, 1792, 1795, 1796, 1797, 1798, 1799, 1800,
Detection: 1768,
Anchor: 1769, 1773,

SEQ ID NO: 17

left Primer: 1802, 1806, 1808, 1809, 1811, 1812, 1813, 1814,
right Primer: 1801, 1815, 1816, 1817, 1818, 1820, 1822,
Blocker: 1805, 1819, 1821, 1823, 1824, 1825, 1826, 1827, 1828, 1829,
Detection: 1803,
Anchor: 1804, 1807, 1810,

SEQ ID NO: 21

left Primer: 1831, 1835, 1837, 1838, 1839, 1840, 1841, 1842, 1843, 1844, 1845, 1846, 1847, 1848, 1849, 1850, 1851, 1852, 1853, 1854, 1855, 1856, 1857, 1858, 1859, 1860, 1861, 1862, 1863, 1864, 1865, 1866, 1868, 1869, 1870, 1872, 1873, 1874, 1875, 1876, 1877, 1878, 1879, 1880, 1881, 1882, 1883, 1884, 1887, 1889, 1890, 1891, 1895,
right Primer: 1830, 1885, 1886, 1888, 1892, 1893, 1894, 1897, 1898,
Blocker: 1834, 1896,
Detection: 1832,
Anchor: 1833, 1836, 1867, 1871,

SEQ ID NO: 35

left Primer: 1900, 1904, 1905, 1906,
right Primer: 1899, 1907, 1909, 1910, 1912, 1913, 1914, 1915, 1916, 1918, 1919, 1920,
Blocker: 1903, 1917, 1921,
Detection: 1901,
Anchor: 1902, 1908, 1911,

SEQ ID NO: 25

left Primer: 1923,
right Primer: 1922, 1927, 1928, 1929,

Blocker: 1926, 1930, 1931, 1932, 1933, 1934,
Detection: 1924,
Anchor: 1925,

SEQ ID NO: 26

left Primer: 1936, 1940, 1941, 1942, 1944, 1945, 1947, 1948, 1950, 1951, 1952,
right Primer: 1935, 1953, 1954, 1955, 1958, 1959, 1960, 1961, 1962, 1963, 1964, 1965, 1967,1968,1969,1970,1971,1972,
Blocker: 1939, 1966, 1973, 1974, 1975,
Detection: 1937,
Anchor: 1938, 1943, 1946, 1949, 1956, 1957,

SEQ ID NO: 38

left Primer: 1977, 1981, 1982, 1983, 1984, 1985, 1987, 1988, 1989, 1990, 1991, 1993, 1994, 1995, 1996, 1997, 1999, 2000, 2002, 2004, 2005, 2006, 2007, 2008, 2009, 2011, 2012,
right Primer: 1976, 2013, 2014, 2015, 2016, 2017, 2019, 2021, 2024,
Blocker: 1980, 2018, 2022, 2023, 2026,
Detection: 1978,
Anchor: 1979, 1986, 1992, 1998, 2001, 2003, 2010, 2020, 2025,

SEQ ID NO: 27

left Primer: 2028, 2032, 2033, 2034, 2035, 2036, 2037, 2038, 2039, 2040, 2041, 2042, 2043, 2044, 2045, 2046, 2047, 2048, 2049, 2050, 2051, 2052, 2053, 2054, 2055, 2056, 2057, 2058, 2059, 2060, 2061, 2062, 2063, 2064, 2065, 2066, 2067, 2068, 2069, 2070, 2071, 2072, 2073,2074,2075,2076,2077,
right Primer: 2027, 2078, 2079, 2080, 2081, 2083, 2084, 2085, 2486, 2088, 2090, 2091, 2092, 2093, 2098, 2100, 2101, 2102, 2103, 2104, 2105, 2106, 2108, 2109, 2110,
Blocker: 2031, 2082, 2087, 2089, 2094, 2095, 2096, 2097, 2099, 2107,
Detection: 2029,
Anchor: 2030,

Taqman Real-time single probe detection assays:

SEQ ID NO: 41

left Primer: 2112, 2115, 2116, 2117,
right Primer: 2111, 2118, 2120, 2121, 2122, 2123, 2126, 2128, 2129, 2130, 2131, 2134, 2136,2145,
Blocker: 2114, 2119, 2124, 2125, 2127, 2132, 2133, 2135, 2137, 2138, 2139, 2140, 2141, 2142, 2143, 2144, 2146, 2147, 2148, 2149, 2150, 2151, 2152, 2153, 2154, 2155, 2156, 2157,2158,2159,2160,
Detection: 2113,

SEQ ID NO: 6

left Primer: 2162, 2165, 2166, 2167, 2168, 2169, 2170, 2171, 2172, 2173,
right Primer: 2161, 2174, 2175, 2176, 2177, 2178, 2179, 2181, 2182, 2184, 2185, 2186,
Blocker: 2164, 2180, 2183, 2187, 2188, 2189, 2190, 2191, 2192, 2193, 2194, 2195, 2196,2197,2198,
Detection: 2163,

SEQ ID NO: 8

left Primer: 2199, 2203,
right Primer: 2200,
Blocker: 2202, 2204, 2205, 2206,
Detection: 2201,

SEQ ID NO: 14

left Primer: 2208, 2211, 2212, 2213, 2214,
right Primer: 2207, 2215, 2216, 2217, 2220, 2224, 2225, 2226,
Blocker: 2210, 2218, 2219, 2221, 2222, 2223, 2227, 2228,
Detection: 2209,

SEQ ID NO: 9

left Primer: 2230, 2233, 2234, 2235, 2236, 2237, 2238, 2239, 2240, 2241, 2242, 2243, 2244,
right Primer: 2229, 2247, 2253,
Blocker: 2232, 2245, 2246, 2248, 2249, 2250, 2251, 2252, 2254, 2255, 2256,
Detection: 2231,

SEQ ID NO: 46

left Primer: 2257, 2264, 2265, 2266, 2267, 2269,
right Primer: 2258, 2261, 2262, 2263,
Blocker: 2260, 2268, 2270, 2271, 2272, 2273, 2274, 2275, 2276, 2277, 2278, 2279, 2280, 2281, 2282, 2283, 2284, 2285, 2286, 2287, 2288, 2289, 2290, 2291, 2292, 2293, 2294, 2295, 2296, 2297, 2298, 2299, 2300, 2301,
Detection: 2259,

SEQ ID NO: 13

left Primer: 2303, 2306, 2307, 2308, 2309, 2310, 2311, 2312, 2313,
right Primer: 2302, 2314, 2316, 2317, 2319, 2320, 2321, 2322, 2323,
Blocker: 2305, 2315, 2318,
Detection: 2304,

SEQ ID NO: 18

left Primer: 2325, 2328, 2329, 2330,
right Primer: 2324, 2331, 2332,
Blocker: 2327, 2333, 2334, 2335,
Detection: 2326,

SEQ ID NO: 20

left Primer: 2337, 2340, 2341, 2342, 2343, 2344, 2345, 2346, 2366, 2368, 2371, 2381, 2383, 2384, 2385, 2388, 2390, 2391, 2392, 2399, 2400, 2401,
right Primer: 2336, 2347, 2350, 2352, 2353, 2354, 2355, 2356, 2357, 2358, 2359, 2360, 2361,2362,2363,2364,2365,
Blocker: 2339, 2349, 2367, 2370, 2372, 2373, 2374, 2375, 2376, 2377, 2378, 2379, 2380, 2382, 2386, 2387, 2389, 2393, 2394, 2395, 2396, 2397, 2398, 2402, 2403, 2404,
Detection: 2338, 2348, 2351, 2369,

SEQ ID NO: 48

left Primer: 2406, 2409, 2410, 2411, 2412, 2413, 2414, 2415, 2416, 2417, 2418, 2419, 2420, 2421, 2422, 2423, 2424, 2425, 2426, 2427, 2428, 2429, 2430, 2431, 2432, 2433, 2434, 2435, 2436, 2437, 2438, 2439, 2440, 2441, 2442, 2443, 2444, 2445, 2446, 2447, 2448, 2449, 2450, 2451, 2452, 2453, 2454, 2455, 2456, 2457, 2458, 2459, 2460, 2461, 2462, 2463,
right Primer: 2405,
Blocker: 2408, 2464, 2465, 2466, 2467, 2468,
Detection: 2407,

SEQ ID NO: 22

left Primer: 2469, 2483, 2485,

right Primer: 2470, 2473, 2474, 2475, 2476, 2477, 2478, 2479, 2480, 2481,
Blocker: 2472, 2482, 2484,
Detection: 2471,

SEQ ID NO: 19

left Primer: 2486, 2494, 2495, 2496, 2502, 2503, 2506, 2509, 2516, 2517, 2518, 2523, 2524,2533,2538,2548,2549,2556,
right Primer: 2487, 2490, 2491, 2492, 2493, 2555, 2559, 2560, 2561, 2562, 2565, 2566, 2567, 2568, 2569, 2570, 2571, 2573, 2574, 2575, 2576, 2577, 2578, 2579,
Blocker: 2489, 2497, 2498, 2499, 2500, 2501, 2504, 2505, 2507, 2508, 2510, 2511, 2512, 2513, 2514, 2515, 2519, 2520, 2521, 2522, 2525, 2526, 2527, 2528, 2529, 2530, 2531, 2532, 2534, 2535, 2536, 2537, 2539, 2540, 2541, 2542, 2543, 2544, 2545, 2546, 2547, 2550, 2551, 2552, 2553, 2554, 2558, 2563, 2564, 2572, 2580, 2581,
Detection: 2488, 2557,

SEQ ID NO: 17

left Primer: 2583, 2586, 2587, 2588, 2589, 2590, 2591,
right Primer: 2582, 2592, 2593,
Blocker: 2585, 2594, 2595, 2596, 2597, 2598, 2599,
Detection: 2584,

SEQ ID NO: 21

left Primer: 2600, 2612, 2615, 2618, 2619, 2620, 2621, 2622, 2623, 2624, 2625, 2626, 2627, 2628, 2629, 2630, 2631, 2632, 2633, 2634, 2635, 2636, 2637, 2638, 2639, 2640, 2641, 2642,2643,2644,2645,2646,2647,
right Primer: 2601, 2604, 2605, 2606, 2607, 2608, 2609, 2610, 2611, 2614, 2648, 2649, 2650, 2651, 2652, 2653, 2654,
Blocker: 2603, 2613, 2617,
Detection: 2602, 2616,

SEQ ID NO: 36

left Primer: 2655, 2659, 2676, 2677, 2678, 2680,
right Primer: 2656, 2660, 2663, 2665, 2666, 2667, 2668, 2669, 2670, 2672, 2673, 2674, 2675,
Blocker: 2658, 2662, 2679,
Detection: 2657, 2661, 2664, 2671,

SEQ ID NO: 26

left Primer: 2682, 2685, 2686, 2687, 2688, 2689,
right Primer: 2681, 2690, 2691, 2692, 2693, 2694, 2695, 2696, 2697, 2698, 2700, 2701, 2702,
Blocker: 2684, 2699, 2703, 2704, 2705, 2706,
Detection: 2683,

SEQ ID NO: 38

left Primer: 2708, 2711, 2712, 2713, 2714, 2715,
right Primer: 2707, 2716,
Blocker: 2710, 2717, 2718, 2719, 2720, 2721,
Detection: 2709,

SEQ ID NO: 27

left Primer: 2723, 2726, 2727, 2728, 2729, 2730, 2731, 2732, 2733, 2734, 2735, 2736, 2737,2738,2739,2740,2741,2742,2743,2745,

right Primer: 2722, 2744, 2746, 2747,
Blocker: 2725, 2748, 2749, 2750,
Detection: 2724,

Additional embodiments of the invention provide a method for the analysis of the methylation status of genomic DNA according to the invention (SEQ ID NOS:1 to SEQ ID NO:64, and complements thereof) without the need for pretreatment.

In *the first step* of such additional embodiments, the genomic DNA sample is isolated from tissue or cellular sources. Preferably, such sources include cell lines, histological slides, body fluids, or tissue embedded in paraffin. In the *second step,* the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis.

In a preferred embodiment, the DNA may be cleaved prior to the treatment, and this may be by any means standard in the state of the art, in particular with methylation-sensitive restriction endonucleases.

In the *third step,* the DNA is then digested with one or more methylation sensitive restriction enzymes. The digestion is carried out such that hydrolysis of the DNA at the restriction site is informative of the methylation status of a specific CpG dinucleotide.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the presence, absence or subclass of colorectal cell proliferative disorder is deduced based upon the methylation state of at least one CpG dinucleotide sequence of SEQ ID NOS:1 to SEQ ID NO:64 , or an average, or a value reflecting an average methylation state of a plurality of CpG dinucleotide sequences of SEQ ID NOS:1 to SEQ ID NO:64. In an alternative embodiment the presence or absence of an aerodigestive cell proliferative disorder selected from the group consisting is deduced based upon the methylation state of at least one CpG dinucleotide sequence of SEQ ID NOS:20, 41, 35, 3, 39, 33 , or an average, or a value reflecting an average methylation state of a plurality of CpG dinucleotide sequences of SEQ ID NOS:20, 41, 35, 3, 39, 33.

Diagnostic Assays for colorectal cell proliferative disorders

The present invention enables diagnosis of events which are disadvantageous to patients or individuals in which important genetic and/or epigenetic parameters within one or more of SEQ ID NOS: 1 to SEQ ID NO:64 may be used as markers. Said parameters obtained by means of the present invention may be compared to another set of genetic and/or epigenetic parameters, the differences serving as the basis for a diagnosis and/or prognosis of events which are disadvantageous to patients or individuals.

More specifically the present invention enables the screening of at-risk populations for the early detection of colorectal carcinomas. Furthermore, specific embodiments of the present invention enable the detection of aerodigestive cancers and thus are additionally suitable for this purpose.Further embodiments of the method may also be used as alternatives to cytological screening for the differentation of colorectal carcinomas from other colon cell proliferative disorders, e.g. colon polyps.

Specifically, the present invention provides for diagnostic cancer assays based on measurement of differential methylation of one or more CpG dinucleotide sequences of SEQ ID NOS:1 to SEQ ID NO:64, or of subregions thereof that comprise such a CpG dinucleotide sequence. Typically, such assays involve obtaining a tissue sample from a test tissue, performing an assay to measure the methylation status of at least one of one or more CpG dinucleotide sequences of SEQ ID NOS:1 to SEQ ID NO:64 derived from the tissue sample, relative to a control sample, or a known standard and making a diagnosis or prognosis based thereon.

In particular preferred embodiments, inventive oligomers are used to assess the CpG dinucleotide methylation status, such as those based on SEQ ID NOS:1 to SEQ ID NO:535, or arrays thereof, as well as in kits based thereon and useful for the diagnosis and/or prognosis of colorectal cell proliferative disorders.

Kits

Moreover, an additional aspect of the present invention is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NOs:1 to SEQ ID NO:535; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™ , COBRA, and nucleic acid sequencing. However, a kit along the lines of the present invention can also contain only part of the aforementioned components.

While the present invention has been described with specificity in accordance with certain of its preferred embodiments, the following examples serve only to illustrate the invention and are not intended to limit the invention within the principles and scope of the broadest interpretations and equivalent configurations thereof.

## EXAMPLES

Samples were received either as frozen tissue or extracted genomic DNA. DNA samples were extracted using lysis buffer from Qiagen and the Roche magnetic separation kit for genomic DNA isolation. DNA samples were also extracted using Qiagen Genomic Tip-100 columns, as well as the MagnaPure device and Roche reagents. All samples were quantitated using spectrophotometric or fluorometric techniques and on agarose gels for a subset of samples.

Bisulfite treatment and mPCR

Total genomic DNA of all samples was bisulfite treated converting unmethylated cytosines to uracil. Methylated cytosines remained conserved. Bisulfite treatment was performed with minor modifications according to the protocol described in Olek et al. (1996). In order to avoid processing all samples with the same biological background together resulting in a potential process-bias in the data later on, the samples were randomly grouped into processing batches. For bisulfite treatment we created batches of 50 samples randomized for sex, diagnosis, and tissue. Per DNA sample two independent bisulfite reactions were performed. After bisulfitation 10 ng of each DNA sample was used in subsequent mPCR reactions containing 6-8 primer pairs.

Each reaction contained the following:

0.4 mM each dNTPS
1 Unit Taq Polymerase
2.5 μl PCR buffer
3.5 mM MgC12
80 nM Primerset (12-16 primers)
11.25 ng DNA (bisulfite treated)

Further details of the primers are shown in TABLE 1.

Forty cycles were carried out as follows: Denaturation at 95˚C for 15 min, followed by annealing at 55˚C for 45 sec., primer elongation at 65˚C for 2 min. A final elongation at 65˚C was carried out for 10 min.

1.1.2 Hybridization

All PCR products from each individual sample were then hybridised to glass slides carrying a pair of immobilised oligonucleotides for each CpG position under analysis. Each of these detection oligonucleotides was designed to hybridise to the bisulphite converted sequence around one CpG site which was either originally unmethylated (TG) or methylated (CG). See Table 2 for further details of all hybridisation oligonucleotides used (both informative and non-informative.) Hybridisation conditions were selected to allow the detection of the single nucleotide differences between the TG and CG variants.

5 μl volume of each multiplex PCR product was diluted in 10 x Ssarc buffer (10 x Ssarc:230 ml 20 x SSC, 180 ml sodium lauroyl sarcosinate solution 20%, dilute to 1000 ml with dH2O). The reaction mixture was then hybridised to the detection oligonucleotides as follows. Denaturation at 95˚C, cooling down to 10 ˚C, hybridisation at 42˚C overnight followed by washing with 10 x Ssarc and dH2O at 42˚C.

Further details of the hybridisation oligonucleotides are shown in TABLE 2.

Fluorescent signals from each hybridised oligonucleotide were detected u sing g enepix scanner and software. Ratios for the two signals (from the CG oligonucleotide and the TG oligonucleotide used to analyse each CpG position) were calculated based on comparison of intensity of the fluorescent signals.

The samples were processed in batches of 80 samples randomized for sex, diagnosis, tissue, and bisulphite batch For each bisulfite treated DNA sample 2 hybridizations were performed. This means that for each sample a total number of 4 chips were processed.

Data analysis methods

Analysis of the chip data:

From raw hybridization intensities to methylation ratios;

The log methylation ratio (log(CG/TG)) at each CpG position is determined according to a standardized preprocessing pipeline that includes the following steps:

For each spot the median background pixel intensity is subtracted from the median foreground pixel intensity (this gives a good estimate of background corrected hybridization intensities):
For both CG and TG detection oligonucleotides of each CpG position the background corrected median of the 4 redundant spot intensities is taken;
For each chip and each CpG position the log(CG/TG) ratio is calculated;
For each sample the median of log(CG/TG) intensities over the redundant chip repetitions is taken.

This ratio has the property that the hybridization noise has approximately constant variance over the full range of possible methylation rates (Huber et al., 2002).

Principle Component Analysis

The principle component analysis (PCA) projects measurement vectors (e.g. chip data, methylation profiles on several CpGs etc.) onto a new coordinate system. The new coordinate axes are referred to as principal components. The first principal component spans the direction of the largest variance of the data. Subsequent components are ordered by decreasing variance and are orthogonal to each other. Different CpG positions contribute with different weights to the extension of the data cloud along different components. PCA is an unsupervised technique, i.e., it does not take into account the labels of the data points (for further details see e.g. Ripley (1996)).

PCA is typically used to project high dimensional data (in our case methylation-array data) onto lower dimensional subspaces in order to visualize or extract features with high variance from the data. In the present report we use 2 dimensional projections for statistical quality control of the data. We investigate the effect of different process parameters on the chip data and exclude that changing process parameters cause large alterations in the measurement values.

A robust version of PCA is used to detect single outlier chips and exclude them from further analysis (Model et al., 2002).

Hypothesis testing

The main task is to identify markers that show significant differences in the average degree of methylation between two classes. A significant difference is detected when the nullhypothesis that the average methylation of the two classes is identical can be rejected with $p<0.05$. Because we apply this test to a whole set of potential markers we have to correct the p-values for multiple testing. This was done by applying the False Discovery Rate (FDR) method (Dudoit et al., 2002).

For testing the null hypothesis that the methylation levels in the two classes are identical we used the likelihood ratio test for logistic regression models (Venables and Ripley, 2 002). The logistic regression model for a single marker is a linear combination of methylation measurements from all CpG positions in the respective genomic region of interest (ROI). A significant p-value for a marker means that this ROI has some systematic correlation to the question of interest as given by the two classes. However, at least formally it makes no statement about the actual predictive power of the marker.

Class prediction by supervised learning

In order to give a reliable estimate of how well the CpG ensemble of a selected marker can differentiate between different tissue classes we can determine its prediction accuracy by classification. For that purpose we calculate a methylation profile based prediction function using a certain set of tissue samples with their class label. This step is called training and it exploits the prior knowledge represented by the data labels. The prediction accuracy of that function is then tested by cross-validation or on a set of independent samples. As a method of choice, we use the support vector machine (SVM) algorithm (Duda (2001), Christiannini (2000)) to learn the prediction function. If not stated otherwise, for this report the risk associated with false positive or false negative classifications are set to be equal relative to the respective class sizes. It follows that the learning algorithm obtains a class prediction function with the objective to optimize accuracy on an independent test sample set. Therefore sensitivity and specificity of the resulting classifier can be expected to be approximately equal.

Estimating the performance of the tissue class prediction: Cross Validation

With limited sample size the cross-validation method provides an effective and reliable estimate for the prediction accuracy of a discriminator function and therefore in addition to the significance of the markers we provide cross-validation accuracy, sensitivity and specificity estimates. For each classification task, the samples were partitioned into 5 groups of approximately equal size. Then the learning algorithm was trained on 4 of these 5 sample groups. The predictor obtained by this method was then tested on the remaining group of independent test samples. The number of correct positive and negative classifications was counted over 5 runs for the learning algorithm for all possible choices of the independent test group without using any knowledge obtained from the previous runs. This procedure was repeated on up to 10 random permutations of the sample set. Note that the above-described cross-validation procedure evaluates accuracy, sensitivity and specificity using practically all possible combinations of training and independent test sets. It therefore gives a better estimate of the prediction performance than simply splitting the samples into one training sample set and one independent test set.

Results

Figures 1, 5, 9, 13, 16, 20, 24, 28 and 32 show ranked matrices of data obtained according to Examples 1 and 2 according to CpG methylation differences between the two classes of tissues, using an algorithim. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. On the right side p values for the individual CpG positions are shown. The p values are the probabilities that the observed distribution occurred by chance in the data set.

Figure 96 shows a ranked matrices of data obtained according to Examples 1 and 2 according to CpG methylation differences between the two classes of tissues, (aerodigestive cancer on the left and healthy aerodigestive tissues on the right) using an algorithim. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.

Figures 2, 6, 10, 17, 21, 25, 29 and 33 show the uncorrected multivariate LogReg test results for each comparison. Each individual genomic region of interest is represented as a point, the dotted line represents the cut off point for the 25% false discovery rate.

Figures 3, 7, 11, 14, 18, 22, 26, 30 and 34 show ranked matrices of data obtained according to Examples 1 and 2 of the accuracy of the genewise linear support vector machine cross validations between the two classes of tissues, for the best performing markers. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. On the right side accuracy values for each individual genomic region of interest are shown.

Figures 4, 8, 12, 15, 18, 23, 27, 31 and 35 shows the accuracy of the genewise linear support vector machine cross validation for all genomic regions of each classification. The accuracy of each genomic region is represented as black squares, the specificity as unfilled diamonds, the sensitivity as unfilled squares. The accuracy as measured on the X-axis shows the fraction of correctly classified samples.

Colon Normal vs. Colorectal Cancer

In the first comparison 102 colorectal carcinoma samples were compared to 73 samples from normal colon, including colon polyps and colon inflammatory disorders.

Figure 1 shows the multivariate test results of the top 12 performing markers using the conservative Bonferroni corrected LogReg test.

Figure 2 shows the uncorrected multivariate LogReg test results for each comparison. Each individual genomic region of interest is represented as a point, the dotted line represents the cut off point for the 25% false discovery rate.

Figure 3 shows the accuracy of the top 12 performing markers.
Figure 4 shows the accuracy of the genewise linear support vector machine cross validation for all genomic regions of each classification.

The following genomic markers were evaluated as being significant:

SEQ ID NOS:1-12,15-20, 22, 25-36, 38-49, 51-58, and complements thereof.

Other Tissues vs. Colorectal Cancer

In this classification 73 colorectal carcinoma samples were compared to an 'other tissue' class consisting of 140 samples from non-colorectal carcinomas, peripheral blood lymphocytes and other normal tissues of non-colorectal origin. These markers therefore enable the detection of colorectal carcinoma cells in', for example, body fluids such as serum.
Figure 5 shows the multivariate test results of the top 12 performing markers using the conservative Bonferroni corrected LogReg test.
Figure 6 shows the uncorrected multivariate L ogReg test results for each comparison. Each individual genomic region of interest is represented as a point, the dotted line represents the cut off point for the 25% false discovery rate.
Figure 7 shows the accuracy of the top 12 performing markers.
Figure 8 shows the accuracy of the genewise linear support vector machine cross validation for all genomic regions of each classification.
The following genomic markers were evaluated as being significant:

SEQ ID NOS:1-23, 26-36, 38-43, 45-49, 51-58 and complements thereof.

Colon Normal and Other Tissue vs. Colon Cancer

In this classification 73 colorectal carcinoma samples were compared to 242 colon normal and 'other tissue' samples. The colon normal class consisted of healthy colon, colon polyps and inflammatory disorder colon tissue samples, the 'other tissues' consisted of samples from non-colorectal carcinomas, peripheral blood lymphocytes and other normal tissues of non-colorectal origin.
Figure 9 shows the multivariate test results of the top 12 performing markers using the conservative Bonferroni corrected LogReg test.
Figure 10 shows the uncorrected multivariate LogReg test results for each comparison. Each individual genomic region of interest is represented as a point, the dotted line represents the cut off point for the 25% false discovery rate.
Figure 11 shows the accuracy of the top 12 performing markers.
Figure 12 shows the accuracy of the genewise linear support vector machine cross validation for all genomic regions of each classification.
The following genomic markers were evaluated as being significant:

SEQ ID NOS:1-3, 5-13, 15-23, 26-36, 38-43, 45-49, 51-58, and complements thereof.

Polyps vs. Colorectal Cancer

In this classification 73 colorectal carcinoma samples were compared to 51 colon polyp samples.
Figure 13 shows the multivariate test results of the top performing markers using the conservative Bonferroni corrected LogReg test.
Figure 14 shows the accuracy of the top 12 performing markers.
Figure 15 shows the accuracy of the genewise linear support vector machine cross validation for all genomic regions of each classification.
The following genomic markers were evaluated as being significant:

SEQ ID NOS:11, 25, 27, 38, 40, 45, 53, and complements thereof.

Colon normal vs. Colorectal cell proliferative disorder

In this calssification 124 colorectal cell proliferative disorder samples (consisting of colon polyps and colorectal carcinoma) were compared to 51 'normal colon' samples consisting of both healthy colon samples and colon tissue of inflammatory disorders.

Figure 16 shows the multivariate test results of the top 12 performing markers using the conservative Bonferroni corrected LogReg test.

Figure 17 shows the uncorrected multivariate LogReg test results for each comparison. Each individual genomic region of interest is represented as a point, the dotted line represents the cut off point for the 25% false discovery rate.

Figure 18 shows the accuracy of the top 12 performing markers.

Figure 19 shows the accuracy of the genewise linear support vector machine cross validation for all genomic regions of each classification.

The following genomic markers were evaluated as being significant:

SEQ ID NOS:1-58, and complements thereof.

<u>Colon Normal vs. Colorectal Cancer</u>

In this classification 73 colorectal carcinoma samples were compared to 51 'normal colon' samples consisting of both healthy colon samples and colon tissue of inflammatory disorders.

Figure 20 shows the multivariate test results of the top 12 performing markers using the conservative Bonferroni corrected LogReg test.

Figure 21 shows the uncorrected multivariate LogReg test results for each comparison. Each individual genomic region of interest is represented as a point, the dotted line represents the cut off point for the 25% false discovery rate.

Figure 22 shows the accuracy of the top 12 performing markers.

Figure 23 shows the accuracy of the genewise linear support vector machine cross validation for all genomic regions of each classification.

The following genomic markers were evaluated as being significant:

SEQ ID NOS:1-3, 5-23, 25-36, 38-49, 51-58, and complements thereof.

<u>Colon Normal and Other Tissues vs. Colorectal cell proliferative Disorder</u>

In this classification 124 colorectal cell proliferative disorder samples (consisting of colon polyps and colorectal carcinoma) were compared to a class consisting of 'colon normal' and 'other tissue' samples. The colon normal samples consisted of both healthy colon samples and colon tissue of inflammatory disorders, the 'other tissue' samples consisted of samples from on-colorectal carcinomas, peripheral blood lymphocytes and other normal tissues of non-colorectal origin.

Figure 24 shows the multivariate test results of the top 12 performing markers using the conservative Bonferroni corrected LogReg test.

Figure 25 shows the uncorrected multivariate LogReg test results for each comparison. Each individual genomic region of interest is represented as a point, the dotted line represents the cut off point for the 25% false discovery rate.

Figure 26 shows the accuracy of the top 12 performing markers.

Figure 27 shows the accuracy of the genewise linear support vector machine cross validation for all genomic regions of each classification.

The following genomic markers were evaluated as being significant:

SEQ ID NOS:1-36, 38-43, 45-58, and complements thereof.

<u>Other Tissue vs. Colon Tissue</u>

The following comparison was carried out in order to identify markers capable of discerning elevated levels of free floating colon DNA, especially in bodily fluids as a marker of tumor progression. In this classification the 'colon tissue' class consisted of samples from colorectal carcinoma, colon polyps, colon tissue of inflammatory disorders and healthy colon tissue. The 'other tissue' class consisted of samples from non-colorectal carcinomas, peripheral blood lymphocytes and other normal tissues of non-colorectal origin.

Figure 28 shows the multivariate test results of the top 12 performing markers using the conservative Bonferroni corrected LogReg test.

Figure 29 shows the uncorrected multivariate LogReg test results for each comparison. Each individual genomic region of interest is represented as a point, the dotted line represents the cut off point for the 25% false discovery rate.

Figure 30 shows the accuracy of the top 12 performing markers.

Figure 31 shows the accuracy of the genewise linear support vector machine cross validation for all genomic regions of each classification.

The following genomic markers were evaluated as being significant:

SEQ ID NOS:1-58, and complements thereof.

Normal Tissue vs. Cell Proliferative Disorder Tissue

In this classification the gene panel was assessed for its ability to accurately discriminate cell proliferative disorder samples from both colorectal carcinoma, colon polyps and non-colon origin cancers from 'normal tissues', namely healthy colon samples, colon tissue of inflammatory disorders, peripheral blood lymphocytes, other normal tissues of non-colorectal origin.

Figure 32 shows the multivariate test results of the top 12 performing markers using the conservative Bonferroni corrected LogReg test.

Figure 33 shows the uncorrected multivariate LogReg test results for each comparison. Each individual genomic region of interest is represented as a point, the dotted line represents the cut off point for the 25% false discovery rate.

Figure 34 shows the accuracy of the top 12 performing markers.

Figure 35 shows the accuracy of the genewise linear support vector machine cross validation for all genomic regions of each classification.

The following genomic markers were evaluated as being significant:

SEQ ID NOS:1-36, 38-43, 45-49, 51-58, and complements thereof.

Aerodigestive cancer v. healthy aerodigestive tissues

In this classification the term 'aerodigestive' describes a sample set of tissues consisting lung, liver, pancreas, bile duct, stomach, and colon. Figure 96 shows a ranked matrices of data obtained according to Examples 1 and 2 according to CpG methylation differences between the two classes of tissues, (Cancerous tissues on the left of the matrix were compared to normal tissues on the right of the matrix.) using an algorithim. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.The following genomic markers were considered significant SEQ ID NOS: 20,41, 35, 3, 39 & 33.

The following examples describe the analysis of the methylation status of the genomic sequences SEQ ID NO: 35, SEQ ID NO:34 , SEQ ID NO:39, SEQ ID NO:29 in healthy and sick colorectal cell proliferative disorder samples. The initial link between said genes and colorectal cell proliferative disorders was initially carried by means of hybridisation analysis as described in EXAMPLE 1. The sequences were then selected from the larger set of genes analysed in said example, and the correlation between methylation status and colorectal cell proliferative disorder states was validated by analysis of samples using other methylation analysis techniques, namely the MSP- MethyLight™ and HeavyMethyl™ MethyLight™ assays. Please note that the term 'MethyLight™' is used to describe real time PCR analysis of bisulfite treated DNA using probes of both the Taqman™ (single probe) and Lightcycler™ (dual probe) technologies.

**EXAMPLE 2**

Analysis of methylation within colon cancer using an MSP- MethyLight assay (SEQ ID NO 35) DNA was extracted from 33 colon adenocarcinoma samples and 43 colon normal adjacent tissues using a Qiagen extraction kit. The DNA from each sample was treated using a bisulfite solution (hydrogen sulfite, disulfite) according to the agarose-bead method (Olek et al 1996). The treatment is such that all non methylated cytosines within the sample are converted to thymidine. Conversely, 5-methylated cytosines within the sample remain unmodified.

The methylation status was determined with a MSP-MethyLight assay designed for the CpG island of interest and a control fragment from the *beta* actin gene (Eads et al., 2001). The CpG island assay covers CpG sites in both the primers and the Taqman style probe, while the control gene does not. The control gene is used as a measure of total DNA concentration, and the CpG island assay (methylation assay) determines the methylation levels at that site.

*Methods:* The SEQ ID NO 35 gene CpG island assay was performed using the following primers and probes:

Forward Primer: CGGAGGGTACGGAGATTACG (SEQ ID NO:14387);
Reverse Primer: CGACGACGCGCGAAA (SEQ ID NO:14388); and
Probe: CGAAACCCTAAATATCCCGAATAACGCCG (SEQ ID NO: 14389).The corresponding control assay was performed using the following primers and probes:

Primer: TGGTGATGGAGGAGGTTTAGTAAGT (SEQ ID NO:14390);
Primer: AACCAATAAAACCTACTCCTCCCTTAA (SEQ ID NO:14391); and Probe: ACCACCACCCAACACACAATAACAAACACA (SEQ ID NO: 14392)

The reactions were run in triplicate on each DNA sample with the following assay *conditions:Reaction solution:* (900 nM primers; 300 nM probe; 3.5 mM Magnesium Chloride; 1 unit of taq polymerase; 200 μM dNTPs; 7μl of DNA, in a final reaction volume of 20 μl);*Cycling conditions:* (95˚C for 10 minutes; then 50 cycles of: 95˚C for 15 seconds; 60˚C for 1 minute).

The data was analysed using a PMR calculation previously described in the literature (Eads et al *2001).Results.* The mean PMR for normal samples was 0.15, with a standard deviation of 0.18. The mean PMR for tumour samples was 17.98, with a standard deviation of 18.18. The overall difference in methylation levels between tumour and normal samples is significant in a t-test (p=0.00000312). The results are shown in Figure 36. A Receiver Operating Characteristic curve (ROC curve) of the assay was also determined. A ROC is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975). The AUC for the MSP-MethylLight™-Assay is: 0.94 (Figure 37).

## EXAMPLE 3

Methylation of SEQ ID NO 35 within colon cancer was analysed using a HeavyMethyl MethyLight™ assay. The same DNA samples were also used to analyse methylation of the CpG island with a HeavyMethyl MethvLieht™ (or HM MethyLight™) assay, also referred to as the HeavyMethyl™ assay. The methylation status was determined with a HM MethyLight™ assay designed for the CpG island of interest and the same control gene assay described above. The CpG island assay covers CpG sites in both the blockers and the Taqman™ style probe, while the control gene does not.

*Methods.* The CpG island assay (methylation assay) was performed using the following primers and probes:

Forward Primer: GGTGATTGTTTATTGTTATGGTTTG (SEQ ID NO:14393)
Reverse Primer: CCCCTCAACCTAAAAACTACAAC (SEQ ID NO: 14394)
Forward Blocker: GTTATGGTTTGTGATTTTGTGTGGG (SEQ ID NO: 14395)
Reverse Blocker: AAACTACAACCACTCAAATCAACCCA (SEQ ID NO: 14396)
Probe: AAAATTACGACGACGCCACCCGAAA (SEQ ID NO: 14397)

The reactions were each run in triplicate on each DNA sample with the following assay conditions:

*Reaction solution:* (400 nM primers; 400 nM probe; 10μM both blockers; 3.5 mM magnesium chloride; 1x ABI Taqman buffer; 1 unit of ABI TaqGold polymerase; 200μM dNTPs; and 7μl of DNA, in a final reaction volume of 20 μl); *Cycling conditions:* (95˚C for 10 minutes); (95˚C for 15 seconds, 64˚C for 1 minute (2 cycles)); (95˚C for 15 seconds, 62˚C for 1 minute (2 cycles); (95˚C for 15 seconds, 60˚C for 1 minute (2 cycles)); and (95˚C for 1 seconds, 58˚C for 1 minute, 60˚C for 40 seconds (41 cycles)).

*Results.* The mean PMR for normal samples was 1.12 with a standard deviation of 1.45. The mean PMR for tumour samples was 38.23 with a standard deviation of 33.22. The overall difference in methylation levels between tumour and normal samples is significant in a t-test (p=0.000000326). The results are shown in Figure 36.

A ROC curve of the assay was also determined. The AUC for the MSP-Methyl-Light-Assay is 0.91 (Figure 38)

The assay was tested on an additional set of colon samples (25 adenocarcinoma, 33 normals, and 13 adenomas). The results showed a significant difference again (Figure 39). The ROC are shown in Figure 40-42.

The MSP and HeavyMethyl variants of the MethyLight assay were determined to be equivalent for the analysis of methylation in SEQ ID NO:35. Figure 48 shows the regression plot of the percentage methylation detected in each sample using the two methods.

## EXAMPLE 4

The SEQ ID NO:35 -HeavyMethyl-MethyLight-assay was also tested against a panel of other tissues (Figure 43). Besides the colon cancer samples only one of the two breast cancer tissues were methylated. However, on a panel of 21 additional breast tumours (different stages), only one was methylated (Figure 44). So the marker is specific for colon tumour samples. All primers, probes, blockers and reaction conditions were identical to those used in the analysis of the colon cancer samples (Example 3).

**EXAMPLE 5**

Twelve of the colon tissues analysed by real-time PCR also had paired serum taken before surgery. We extracted DNA from 1 ml of that serum using a Qiagen UltraSens□ DNA extraction kit, bisulfite treated the DNA sample, and ran the SEQ ID NO:35 - HeavyMethyl-MethyLight™-assay on those samples. The control gene did not amplify for three of the cancer serum samples and three of the normal serum samples, so we can conclude that the sample preparation did not work in these cases. In the other cases, there was evidence of higher methylation in the cancer samples than the normal samples (Figure 45).

**EXAMPLE 6**

Analysis of methylation within colon cancer using a SEQ ID NO:34 -MSP-MethyLight™ Assay. The colon cancer samples described in Example 2 were also analysed using a SEQ ID NO:34 -MSP- MethyLight™ Assay with a Taqman® style probe. The sample preparation was carried out as described above (Example 1). The assay was performed using the following primers and probes:

Forward Primer: TGGGATTAAGATTTTCGGTTAGTTTC (SEQ ID NO:14398)
Reverse Primer: CACTACAACGCTACGCGACTAAA (SEQ ID NO:14399)
Probe: TCGACGTTACCCAAACGAATCACATAAAAAAC (SEQ ID NO: 14400) The corresponding control assay was performed as described above (EXAMPLE 2)

The reactions were run in triplicate on each DNA sample with the following assay conditions:

*Reaction solution:* (900 nM primers; 300 nM probe; 3.5 mM magnesium chloride; 1 units of taq polymerase; 200 μM dNTPs, 5μM blocker; and 7, μl of DNA, in a final reaction volume of 20 μl);
*Cycling conditions:* 95˚C for 10 minutes; (95˚C for 15 seconds, 60˚C for 1 minute) 50 cycles
The data was analysed using a PMR calculation previously described in the literature (Eads et al 2001).
*Results.* The results are shown in Figure 36. The mean PMR for normal samples was 3.93, with a standard deviation of 3.57. The mean PMR for tumour samples was 23.06, with a standard deviation of 20.23 The overall difference in methylation levels between tumour and normal samples is significant in a t-test (p=0.000003063). The ROC curve of the assay is shown in Figure 46. The AUC is 0.84.
This was further confirmed using a SEQ ID NO:34 -HeavyMethyl MethyLight™ assay, using dual Lightcycler™ probes.
*Methods.* The CpG island assay (methylation assay) was performed using the following primers and probes:

Forward Primer: TGGATAGGAGTTGGGATTAAGATTTT (SEQ ID NO:14401)
Reverse Primer: CTTATTACAATTTAAAAAAAAAATTCACTACAA (SEQ ID NO:14402);
Blocker: AAATTCACTACAACACTACACAACTAAATTCAACATTAC (SEQ ID NO:14403);
Probe: TTTTCGTATTTTTTTTCGGGTTATTACGTTTT-Fluor (SEQ ID NO: 14404);
Probe: LC640-ATGTGATTCGTTTGGGTAACGTCGA-Phos (SEQ ID NO: 14405. The reactions were each run in triplicate on each DNA sample with the following assay conditions:

*Reaction conditions:* 500 nM primers; 10 μM blocker; 250 nM probes; 4 mM Magnesium Chloride
*Cycling profile:* 95˚C denaturation for 10 minutes; 50 cycles: 95˚C 10 seconds, 57˚C 30 seconds, 72˚C 20 seconds

**EXAMPLE 7**

Analysis of methylation within colon cancer using a SEQ ID NO:29 -MSP-MethyLight™ Assay. The colon cancer samples described in Example 2 were also a nalysed using a SEQ ID NO:29 -MSP- MethyLight™ Assay with a Taqman® style probe. The sample preparation was carried out as described above (Example 2). The assay was performed using the following primers and probes:

Forward Primer: TTTTTTTTTCGGACGTCGTTG (SEQ ID NO: 14406)
Reverse Primer: CCTCTACATACGCCGCGAAT (SEQ ID NO:14407)
Probe: AATTACCGAAAACATCGACCGA (SEQ ID NO: 14408)

The reactions were run in triplicate on each DNA sample with the following assay conditions:

*Reaction solution:* (900 nM primers; 300 nM probe; 3.5 mM magnesium chloride; 1 units of taq polymerase; 200 μM dNTPs, 5 μM blocker; and 7 μl of DNA, in a final reaction volume of 20 μl);
*Cycling conditions:* 95˚C for 10 minutes; (95˚C for 15 seconds, 60˚C for 1 minute) 50 cycles
The corresponding control assay was performed as described above (EXAMPLE 2).
The data was analysed using a PMR calculation previously described in the literature (Eads et al 2001).
*Results.* The results are shown in Figure 36. The mean PMR for normal samples was 3.04, with a standard deviation of 4.21. The mean PMR for tumour samples was 21.38, with a standard deviation of 24.08 The overall difference in methylation levels between tumour and normal samples is significant in a t-test (p=0.0000101973). The ROC curve of the assay is shown in Figure 47. The AUC is 0.80.
This was further confirmed using a SEQ ID NO:29 -HeavyMethyl MethyLight™ assay (using dual labeled Lightcycler™ probes.
*Methods.* The CpG island assay (methylation assay) was performed using the following primers and probes:

Forward Primer: GTAGGGTTATTGTTTGGGTTAATAAAT (SEQ ID NO: 14409)
Reverse Primer: TAAAAAAAAAAAAAAAACTCCTCTACATAC (SEQ ID NO:14410)
Blocker: AACTCCTCTACATACACCACAAATAAATT (SEQ ID NO: 14411)
Probe: CGAAAACATCGACCGAACAACG-Fluor (SEQ ID NO: 14412)
Probe: LC640-GTCCGAAAAAAAAAAAACGAACTCC-Phos (SEQ ID NO: 14413)

The reactions were each run in triplicate on each DNA sample with the following assay conditions:

Reaction conditions: Forward primer: 600 nM; Reverse primer: 30 nM; Blocker: 10 μ M; Probes: 500 nM; Taq polymerase: 0.1 U/μl; dNTPs: 0.2mM each; Magnesium Chloride: 4 mM;BSA: 0.25 mg/ml; Roche buffer with no $MgCl_2$: 1x
Cycling conditions: 95˚C denaturation for 10 minutes; 50 cycles: 95˚C for 10 seconds, 57˚C for 25 seconds, 72˚C for 10 seconds

**EXAMPLE 8**

Analysis of methylation within colon cancer using a SEQ ID NO:29 -MSP-MethyLight™ Assay. An additional assay for SEQ ID NO:29 was tested on colon samples. The assay was tested on two sets of tissues, each with 12 colon adenocarcinomas and 12 normal adjacent tissue samples.
The sample preparation was carried out as described above (Example 2). The assay was performed using the following primers and probes:

Forward Primer: GGACGTTTTTTATCGAAGGCG (SEQ ID NO:14414)
Reverse Primer: GCCACCCAACCGCGA (SEQ ID NO:14415)
Probe: ACCCGAAATCACGCGCGAAAAA (SEQ ID NO: 14416)

The reactions were run in triplicate on each DNA sample with the following assay conditions:

*Reaction solution:* (900 nM primers; 300 nM probe; 3.5 mM magnesium chloride; 1 units of taq polymerase; 200 μM dNTPs, 5 μM blocker; and 7 μl of DNA, in a final reaction volume of 20 μl);
*Cycling conditions:* 95˚C for 10 minutes; (95˚C for 15 seconds, 60˚C for 1 minute) 50 cycles. The corresponding control assay was performed as described above (Example 2)

The data was analysed using a PMR calculation previously described in the literature (Eads et al 2001). In both cases, SEQ ID NO:29 was significantly more methylated in the cancer samples. The ROC curves of the assays are shown in Figures 49 and 50. The AUC are 0.93 and 1.

**EXAMPLE 9**

Analysis of methylation within colon cancer using a SEQ ID NO:39 -MSP-MethyLight™ Assay

The colon cancer samples described in Example 2 were also analysed using a SEQ ID NO:39 -MSP- MethyLight™ Assay. The sample preparation was carried out as described above (EXAMPLE 2). The assay was performed using the following primers and probes:

Forward Primer: GACGGATTTTTTTTTAACGTTTTTTC (SEQ ID NO:14417)
Reverse Primer: AAATAAAATACCACCTCCGCGA (SEQ ID NO:14418)
Probe: GCTCCTCGCGAAATACTCACCCCG (SEQ ID NO:14419)

The reactions were run in triplicate on each DNA sample with the following assay conditions:

*Reaction solution:* (900 nM primers; 300 nM probe; 3.5 mM magnesium chloride; 1 units of taq polymerase; 200 μM dNTPs, 5 μM blocker; and 7 μl of DNA, in a final reaction volume of 20 μl);
*Cycling conditions:* 95˚C for 10 minutes; (95˚C for 15 seconds, 60˚C for 1 minute) 50 cycles.
The corresponding control assay was performed as described above (EXAMPLE 2).
The data was analysed using a PMR calculation previously described in the literature (Eads et al 2001).
*Results.* The results are shown in Figure 36. The mean PMR for normal samples was 2.25, with a standard deviation of 2.42. The mean PMR for tumour samples was 25.67, with a standard deviation of 17.57 The overall difference in methylation levels between tumour and normal samples is significant in a t-test (p=0.00000000118). The ROC curve of the assay is shown in Figure 52. The AUC is 0.94
This was further confirmed using a SEQ ID NO:39 -HeavyMethyl MethyLight™ assay, using dual Lightcycler probes using Lightcycler style dual probe technology.
*Methods.* The CpG island assay (methylation assay) was performed using the following primers and probes:

Forward Primer: GTTAGTTAGTTAATTTTTTAAATAGATTAGTAG (SEQ ID NO: 14420)
Reverse Primer: CAAAAAAACAAATAAAATACCACCTCC (SEQ ID NO:14421 )
Blocker: CCTCCACAAAACTCACTCCTCACAAAATAC (SEQ ID NO:14422)
Probe: red640 TTTCGTTTTGTATGGTAGATACGGGGTGA- phosphate (SEQ ID NO:14423)
Probe: ATTAATGGTTTTATAAGACGGATTTTTTTTTAACGT- fluorescine (SEQ ID NO: 14424)

The reactions were each run in triplicate on each DNA sample with the following assay conditions:

Reaction conditions: Forward primer: 600 nM; Reverse primer: 300 nM; Blocker: 10 μM; Probes: 500 nM; Taq polymerase: 0.1 U/μl; dNTPs: 0.2 mM each; Magnesium Chloride: 4 mM; BSA: 0.25 mg/ml; Roche buffer with no $MgCl_2$: 1x
Cycling conditions: 95˚C denaturation for 10 minutes; 50 cycles: 95˚C for 10 seconds, 57˚C for 25 seconds, 72˚C for 10 seconds.

Experiment 10: Stool analysis of methylation markers

The following experiments were run in order to establish the feasibility of methylation markers (for the detection of colon cell proliferative disorders) from DNA derived from stool samples. Accordingly, a series of samples were prepared each comprising either stool only (control) or stool with the addition of a controlled amount of cells from the colon cancer cell line SW 620 (25,000 cell equivalent -0.15 ug) the prepared samples were then frozen. Three different stool samples were used. As a positive control a further sample was prepared comprising peripheral blood lymphocytes (PBLs) and a controlled amount of cells from the colon cancer cell line.
The samples were as follows:

Class 1: Stool sample No. 1
Class 2: Stool sample No. 1 + 0.15 ug
Class 3: Stool sample No. 2
Class 4: Stool sample No. 2 + 0.15 ug
Class 5: Stool sample No. 3
Class 6: Stool sample No. 3 + 0.15 ug
Class 7: PBS + 25,000 cell equivalents (0.15 ug)
Class 8: $H_2O$

Multiple samples were prepared in each class. 300 ng of stool DNA was present in each sample. Each sample was then analysed for methylation using an MSP TaqMan assay for the gene EPHB65 as follows:

Primer Forward: TTTTTTTTTCGGACGTCGTTG (SEQ ID NO:14455)

Primer Reverse: CCTCTACATACGCCGCGAAT (SEQ ID NO: 14456)
Probe: AATTACCGAAAACATCGACCGA (SEQ ID NO:14457)

Cycling conditions were: The reaction conditions for TaqMan:

| | | |
|---|---|---|
| Initial denaturation | 95oC | 10min |
| Denaturation | 95oC | 15sec |
| Annealing/ Extension | 60oC | 60sec |

50 cycles

Amplification curves of the PCR reaction were according to figures 52 to 58.

Class 1: Figure 52
Class 2: Figure 53
Class 3: Figure 54
Class 4: Figure 55
Class 5: Figure 56
Class 6: Not applicable (see below)
Class 7: Figure 57
Class 8: Figure 58

The X-axis shows the cycle number of the polymerase chain reaction, the Y-axis shows the delta Rn, which is the fluorescence intensity over background, the horizontal line (A) represents the threshold level of the calibration curve (B), if the curve reaches above (A) methylation has been detected.

As can be seen, methylated colon cancer DNA could be detected in classes 2 & 5. Class 6 was not analysed as methylated DNA was detected in the stool sample (class 5).

In order to determine the absolute sensitivity of methylated DNA detection in stool samples stool sample No. 2 was spiked with varying amounts of SW 620 DNA. Five classes of samples were analysed using the MSP assays above:

- Class B:25,000 cell equivalents in 250 mg stool
- Class C:2,500 cell equivalents in 250 mg stool
- Class D:250 cell equivalents in 250 mg stool
- Class E:25 cell equivalents in 250 mg stool
- Class F:0 cell equivalents in 250 mg stool

The amplification curves can be seen in figure 59. The X-axis shows the cycle number of the polymerase chain reaction, the Y-axis shows the delta Rn, which is the fluorescence intensity over background, the horizontal line (A) represents the threshold level of the calibration curves (B-F), if the curves reach above (A) methylation has been detected.Curves B, C, D; E; & F are the amplification curves of classes B, C, D, E & F respectively. Thus it can be seen that methylated DNA can be detected in stool samples at levels of 25 cells per 250 mg stool.

**Example 11:**

**Real time analysis of methylation sensitive restriction.**

DNA was prepared from from ten colon tumor samples and ten normal adjacent tissue samples. DNA isolation was performed using standard methods known to the expert and quantified by UV measurement.

A digestion reaction was performed with the following reagents:

1 μg genomic DNA;
25 U enzyme (10U/μl);
Y+/Tango Buffer
By incubation 37˚C.

The following steps were performed for all samples in parallel. 1 μg of DNA was dissolved in 190μl Y+/Tango Buffer (Fermentas) in 0,2ml PCR tubes. 95 μl of the reaction solution (half of the overall volume) was pipetted into a clean PCR tube as a control. To the remaining reagent in the first tube 1,25µl HpaII and 1,25µl Hin6I restriction endunuclease (both from Fermentas)were added.

To the second tube 2,5µl of a mixture of glycerol and water (y:v =1:1) was added. After incubating all 40 tubes at 37°C in an Eppendorff Thermocycler for five hours. Then another aliquot of restriction enzyme, resp. water-glycerol mix, was added to each tube and the incubation was extended for ten hours. After this period of time the restriction enzymes were inactivated by heating at 65°C for 10 minutes.

Subsequently, dilutions of each tube were assembled in 96well plates by transferring 20µl from each tube into the plates and adding 80µl water.

Finally, both the restriction product and the control of each sample were analyzed by Quantitative Real Time PCR. In this example 12µl of a SybrGreen containing PCR mastermix containing one of the primers according to table 4 was pipetted into a 96well Taqman plate followed by 8µl of the corresponding dilutions of restricted sample and blank (each in duplicate).

Real Time PCR was performed on a ABI 7700 instrument applying the following temperature profile: 15 minutes at 95°C; 50 cycles of: 15 seconds at 95°C, 45 seconds at 65°C, 75 seconds at 72°C; 15 seconds at 95°C followed by 50°C for one hour.

Using the described primers and conditions amplification is observed only for methylated copies of the analysed sequences (according to table 4) when methylation sensitive restriction has been performed prior to PCR, since unmethylated copies are digested during this step. The amount of methylated DNA after restriction is normalized against the total amount of DNA present before the restriction as obtained by Real Time quantitation of the blank of the same sample. The equation

$$\Delta Ct = Ct_{blank} - Ct_{restricted}$$

represents a measure for the relative amount of unmethylated DNA present in a DNA sample. A value of zero means that the DNA is completely methylated. A value of 0.5 corresponds to approximately 50% methylation in case of an optimised PCR reactions whereas larger values correspond to low methylation levels. The relative amount of methylated sequence present in a sample would be given by

$$(2) \qquad M = E^{-\Delta Ct}$$

where E is the PCR efficiency. The PCR efficiency is easily obtained by methods known to the expert.

Table 4

| Gene | Forward primer | Reverse Primer | Results Figure |
|---|---|---|---|
| SEQ ID NO: 8 | GACGGGGGTTCTGGACTTAC (SEQ ID NO: 1458) | CCAGTCCCTGGATGTTGCT (SEQ ID NO: 14459) | 60 |
| SEQ ID NO: 5 | GCTAAGCCGGAGTCACCAAG (SEQ ID NO: 1460) | CGCTGAGACCGAGAAGAAG (SEQ ID NO:14461) | 61 |
| SEQ ID NO: 10 | GCCGACGAGACCAAGGAG (SEQ ID NO: 14462) | ATGAGCAGCGTGAAGACGA (SEQ ID NO: 14463) | 62 |
| SEQ ID NO: 62 | CGTGCCATCCCAAGAGTTT (SEQ ID NO: 14464) | ATGACCCTGGCTTTGGTGT (SEQ ID NO: 14465) | 63 |
| SEQ ID NO: 12 | CGCAGTAAGAGGCGACAAC (SEQ ID NO:14466) | AAGGGGTCTACGGGCACT (SEQ ID NO: 14467) | 64 |
| SEQ ID NO: 11 | TCTGGAAGGAGGACGTGAA (SEQ ID NO: 14468) | AAGGGGTCTACGGGCACT (SEQ ID NO: 14469) | 65 |
| SEQ ID NO: 61 | GTTGAAGTCGCCCAGGAAC (SEQ ID NO:14470) | AGTGGGGCACCGACGTAA (SEQ ID NO: 14471) | 66 |
| SEQ ID NO: 46 | CCATTTTACTTGGGGTGTGG (SEQ ID NO: 14472) | CTGGAGCTGTCAGGAGAAAAG (SEQ ID NO: 14473) | 67 |

(continued)

| Gene | Forward primer | Reverse Primer | Results Figure |
|---|---|---|---|
| SEQ ID NO: 64 | GTCGCCCCCTACCCTTCT (SEQ ID NO: 14474) | CCCAAGACCTTAACCCAAGTC (SEQ ID NO: 14475) | 68 |
| SEQ ID NO: 63 | CTCCAAACGCGATACCCTCT (SEQ ID NO: 14476) | GTGACTCCTCCCCTTCCTTC (SEQ ID NO: 14477) | 69 |
| SEQ ID NO: 20 | GCCTTAACTGCTGGGGTCTT (SEQ ID NO: 14478) | GATGGAGATGGCTGAAGTGG (SEQ ID NO: 14479) | 70 |
| SEQ ID NO: 45 | GTCCATCGGTTTCGTCTCC (SEQ ID NO: 14480) | CCACCGTGTAGAAGTTGTCGT (SEQ ID NO: 14481) | 71 |

Results are shown in Figures 60 - 71. Each individual sample is annotated on the x-axis as 'n' denoting normal, or 't' denoting tumour. A bronchial tissue sample was accidentally analysed in some assays, these are annotated with an arrow, and are irrelevant in the context of the present invention. The Y-axis shows the level of methylation ('methylation rate ') in the sample. The methylation rate {methylated/(methylated + unmethylated) are shown for the fragments corresponding to the primer pairs in Table 4. Formula (2) above was used for the calculation assuming a PCR efficiency of E = 2.

The data demonstrate that for several fragments the majority of the tumor samples show significantly higher methylation rates than the corresponding normal adjacent tissues.

**Example 12**

mRNA expression analysis

1. RNA samples

Total RNA from normal colon (n=1) and colon cancer (adenocarcinoma; n=1) was obtained from Ambion (Huntingdon, United Kingdom). Each RNA sample was derived from a single donor. Total cellular RNA was prepared using the ToTALLY RNA Kit (Ambion, Huntingdon, United Kingdom) and Dnase-treatment was performed to minimize contamination. RNA integrity was assessed before and after 14 hours incubation at 37˚C on an Agilent Bioanalyzer (Agilent Technologies, Inc, Palo Alto, CA).

2. Microarray Experiments

Target preparation and labeling, microarray hybridization, washing, and scanning was performed according to the manufacturer's protocol (Affymetrix, Inc., Santa Clara, CA). Briefly, 10 μg of total RNA were reverse transcribed using a T7-linked oligo-dT primer, followed by second strand synthesis. Biotin-labeled cRNA targets were prepared from double stranded cDNA using T7 RNA polymerase and biotinylated ribonucleotides (UTP, CTP). Fragmentation was performed at 94˚C for 35 minutes. 15 μg of the fragmented biotinylated cRNA were mixed with MES buffer (2-(N-Morpholino)ethanesulfonic acid) containing 0.5 mg/ml acetylated bovine serum albumin and hybridised to Affymetrix GeneChip HG_U133A arrays (Santa Clara, CA) for 16 hours at 45˚C. Microarrays were stained using streptavidin phycoerythrin conjugates (Molecular Probes, Inc., Eugene, OR). For signal amplification biotinylated anti-streptavidin antibodies (Vector Laboratories, Burlingame, CA) were used.

Arrays were scanned on an Affymetrix GeneChip Scanner. The expression values for each probe set (representing a gene) were calculated from the raw data using Bioconductor R packages (http://www.bioconductor.org, see below).

3. Data Analysis

The raw expression data obtained from the Affymetrix scanner (the so called cel-files) were background corrected and normalized using the default settings of the RMA normalization procedure (Irizarry RA, Hobbs B, Collin F, Beazer-Barclay YD, Antonellis KJ, Scherf U, Speed TP: Exploration, normalization, and summaries of high density oligonucleotide array probe level data. Biostatistics. 2003 Apr;4(2):249-64) as implemented in the Bioconductor R packages, release 1.4 (Gautier L, Cope L, Bolstad BM, Irizarry RA: affy--analysis of Affymetrix GeneChip data at the probe level. Bioinformatics. 2004 Feb 12;20(3):307-150). The raw data processing results in signal intensity values for each of the 22.293 probe sets available on the Affymetrix GeneChip HG_U133A chip.

To identify differentially expressed probe sets, the fold change of the normalized signal intensities between colon normal tissue and colon tumour tissue was calculated. Genes with a fold change bigger than 2 (or smaller than 0.5) were identified as differentially expressed. Differentially expressed genes are shown in Table 5, as can be seen hypermethylation does not correlate with underexpression in all cases.

Table 5

| Affymetrix Probe ID | Gene SEQ ID NO: | log-fold change | fold-change | up/down in cancer |
|---|---|---|---|---|
| 208944_at | 37 | -1,0 | 2,0 | down |
| 209945_s_at | 27 | 1,2 | 2,3 | up |
| 214211_at | 54 | -1,3 | 2,5 | down |
| 203140_at | 16 | -1,8 | 3,4 | down |
| SEQ ID NO: 3497_at | 15 | -1,4 | 2,6 | down |

**Example 13**

TPEF(TMEFF2) methylation analysis in serum samples.

In the following example 3 different sample sets of serum from colorectal cancer patients and healthy colon individuals were analysed using MSP assay ’4’ and HM assay ’6’ of the gene TMEFF2. Healthy colon was defined as being verified by colonoscopy as having no colon malignancy or polyps present. The assays were run on the LightCycler platform (Roche Diagnostics), using two different versions of the instrument for comparative purposes, namely versions ’1.2’ and ’2.0’. They are referred to below as ’LC 1.2’ and ’LC 2.0’ respectively.

**Sample sets**

Sample set one:

30 colorectal cancers, 30 colonoscopy verified as no colon malignancy or polyps Sample set two:
15 colorectal cancers, 15 colonoscopy verified as no colon malignancy or polyps Sample set three:
30 colorectal cancers, 30 colonoscopy verified as no colon malignancy or polyps In each case approximately 1.8 mls of serum equivalent was analysed per PCR.

Reaction conditions

Assay ’4’ was performed as below.
Forward primer: agggcgttttgttggcg (SEQ ID NO: 14482)
Reverse Primer: ctacaacctaaacgacgcgct (SEQ ID NO: 14483)
Taqman Probe: atcggcgttttagcgtgcggg (FAM/BHQ1 labelled) (SEQ ID NO: 14484)

Lightcycler program:

| | | | |
|---|---|---|---|
| activation: | 95˚10 mins | | |
| 3 cycles | 95˚ 15 secs | (20˚/sec) | |
| | 66˚ 45secs | (20˚/sec) | detection |
| 3 cycles | 95˚ 15 secs | (20˚/sec) | |
| | 64˚ 45secs | (20˚/sec) | detection |
| 50 cycles | 95˚ 15 secs | (20˚/sec) | |
| | 62˚ 45secs | (20˚/sec) | detection |

**Results**

Assay '4'

Sample set one:

AUC=0.95 [0.86, 0.99] (95% confidence intervals) LC 2.0

Sample set two:

AUC=0.65 [0.44,0.80] LC 2.0
AUC=0.57 [0.37, 0.75] LC 1.2

Sample set three:

AUC=0.83 [0.71, 0.92] LC 2.0
AUC=0.83 [0.71, 0.92] LC 1.2

Assay '6'

Forward primer: aaaaaaaaaaaactcctctacatac (SEQ ID NO: 14485)
Reverse Primer: ggttattgtttgggttaataaatg (SEQ ID NO: 14486)
Blocker oligonucleotide: acatacaccacaaataaattaccaaaaacatcaaccaa-pho (SEQ ID NO: 14487)
Probes: tttttttttcggacgtcgtt-fluo (SEQ ID NO: 14488)and red640-tcggtcgatgttttcggtaa-pho (SEQ ID NO: 14489)

| **LightCycler 1.2 program:** | | | | |
|---|---|---|---|---|
| activation: | 95˚C | 10min | | |
| | | | | |
| 55 cycles: | 95˚C | 10 sec | (20˚C/s) | |
| | 56˚C | 30 sec | (20˚C/s) | detection |
| | 72˚C | 10 sec | (20˚C/s) | |

Sample set two:

AUC=0.70 [0.51,0.85] LC 2.0

Sample set three:

AUC= 0.69 [0.55, 0.80] LC 2.0
AUC= 0.71 [0.53, 0.83] LC 1.2 (Some samples were lost, approximately 20 cancer serum samples, 20 healthy colon serum samples were analysed)

**Example 14**

MSP analysis of the genes according to Table 6 (below).

In the following analysis the methylation status of a selection of the genes according to Table 3 were analysed by means of methylation specific amplification using the primers according to Table 6 (below).

The study was run on approximately 140 samples from colon, breast and liver carcinoma, normal tissue (peripheral blood lymphocytes, healthy colon and healthy tissue adjacent to colon carcinoma), colon polyps and other colon diseases. The positive class consisted of Colon Cancer and Colon polyps. The negative class was PBL (peripheral blood lymphocytes), Colon-Normal, Colon-NAT and Colon-Inflammatory. For AUC values in brackets (see table 7, below) Breast-Cancer and Liver-Cancer were also included in the negative class.

Genomic DNA was analyzed using the MSP technique after bisulfite conversion. Total genomic DNA of all samples was bisulfite treated converting unmethylated cytosines to uracil. Methylated cytosines remained conserved. Bisulfite treatment was performed with minor modifications according to the protocol described in Olek et al. (1996).

The sequence of interest was then amplified by means of methylation specific primers, the amplificate is then detected by means of methylation specific Taqman probes.

**Table 6**: Oligonucleotides for MSP Taqman.

| Assay number | Gene/Genomic location | Forward primer sequence | Probe | Reverse primer sequence | Amplification curve |
|---|---|---|---|---|---|
| 1 | Transcription factor BTF3 Homolog 1 | Acggcgttttggttcgt | cgacttaataacgacgtacgcgaaccc | gtcatctaacaacgacctaactaacg | Figure 72 |
| 2 | RING FINGER PROTEIN 4 | ctctaaaccgcgaaaactccg | cgcgtttgttcgggtttcggg | gggatgagaggttgttgacgttc | Figure 73 |
| 3 | TUMOR NECROSIS FACTOR RECEPTOR SUPERFAMILY MEMBER 16 PRECURSOR | agggcgttttgttggcg | atcggcgttttagcgtgcggg | ctacaacctaaacgacgcgct | Figure 74 |
| 4 | Region of chromosome 14q31.1 | gaggtttcggttcgaggttc | acgtcacaaaaaaaaaacccacgtaataataacgaa | ctttactacaacgaaaacgccg | Figure 75 |
| 5 | Chondroitin sulfate proteoglycan 2 (versican) (CPSG2) | gattaaaaaatttgtttttattcggtcg | cgcggaagtagagtaggcgggcg | tccgcttcgaaaacctcga | Figure 76 |
| 6 | EYA 4 | caaaatccgtcgacgataaaac | tcgggttggggttcgtattcgg | acgtagttataggttttgatagttttaggga | Figure 77 |
| 7 | MOTHERS AGAINST DECAPENTAPLEGIC HOMOLOG 7 (SMAD 7) | gaacgcgaaaaaattaacatctcg | tttcgtttttatcgtggtgggtatgttcgtgttta | tttcgggattgttgttggtgttt | Figure 78 |
| 8 | ENDOPLASMIN PRECURSOR (94 KDA GLUCOSE-REGULATED PROTEIN) (GRP94) (GP96 HOMOLOG) (TUMOR REJECTION ANTIGEN 1) | catcaatatcctaatcataacctactatctatcg | taggtttagtcgcgcgttcgcga | gagatggggaatttttgacgtc | Figure 79 |

| | | | | | |
|---|---|---|---|---|---|
| 9 | Region of chromosome 6q25.1 | cttaaaataataacgcgaaatataacgct | cgaaaaacgatcgaaatcccgactttc | tttgcggaaagacgtcgtt | Figure 80 |
| 10 | B-CELL LYMPHOMA 6 PROTEIN (BCL-6) (ZINC FINGER PROTEIN 51) (LAZ-3 PROTEIN) (BCL-5) | ccaaaaatacgcgaccacg | ctaaaccactaatattcgacgtaattctccgtatttac | cgtttttcgtattcggaaagac | Figure 81 |
| 11 | HOMEOBOX PROTEIN ARISTALESS-LIKE 4 | tacgtgtgaggggttcgc | ccaatcgaaactatccaaatacgataaccga | ccaataaaaattcgtaaaaaaaaacg | Figure 82 |
| 12 | TRANSMEMBRANE PROTEIN WITH EGF-LIKE AND TWO FOLLISTATIN-LIKE DOMAINS 2 | cggttagtcggaggcgc | cgaacgaaacaacctaaacgaaaaccccg | ccaaaataaccaatcgacccg | Figure 83 |
| 13 | Hypothetical protein-leucine rich repeat | gggattaagattttcggttagtttcg | caaacgaatcacataaaaaacgtaataacccgaa | aacgctacgcgactaaattcga | Figure 84 |
| 14 | Candidate tumor suppressor 8p22 | gggttagggtgggtcgc | cgtcggcgtgggcgatgtc | gactcccgccccaacg | Figure 85 |
| 15 | VESICULAR INHIBITORY AMINO ACID TRANSPORTER (GABA AND GLYCINE TRANSPORTER) (VESICULAR GABA TRANSPORTER) (HVIAAT) | cggagggtacggagattacg | cgaaaccctaaatatcccgaataacgccg | cgacgacgcgcgaaa | Figure 86 |

Reaction Conditions for Taqman PCR program:

Denaturation at 95˚C for 10 minutes.

50 cycles: Denaturation at 95˚C for 15 seconds.
Annealing at 62˚C for 1 minute. Except Assay 15, which has an annealing temp of 60˚C.

Amplification curves for each assay are shown in figures 72 to 86 (according to Table 6).

The technical performance of a series of alternative assays was validated by testing each of the following using sperm DNA as the template:
Gene SEQ ID NO: 34 (MSP assay)
Primers: cgtacgaacgcaacaaccg (SEQ ID NO: 14535) & gttcggcgttgtttaggc (SEQ ID NO: 14536)

Probes: gcatcttccaacaccgtcccg (SEQ ID NO: 14537) — fluo & LC640-ctccgcatccttccccgaa-p (SEQ ID NO: 14538) (Lightcycler probes)

Lightcycler program:
95˚10 mins

55 cycles 95˚15 secs (20˚/sec)
62˚45 secs (20˚/sec) detection
72˚15 secs (20˚/sec)

Gene SEQ ID NO: 28 (MSP assay)
Primers: ccgaacaaacgtaatacgcg (SEQ ID NO: 14539) & acggcgtgaaggagcg (SEQ ID NO: 14540)
Probes: tacgcgcggtagtcgtgcgc (SEQ ID NO: 14541) (Taqman probe FAM/BHQ1 labelled)

Lightcycler program:
95˚10 mins

55 cycles 95˚15 secs (20˚/sec)
60˚45secs (20˚/sec) detection

Gene SEQ ID NO: 10 (MSP assay)
Primers : cgacaaaatcctcatcgcg (SEQ ID NO: 14542) & gtcgtttagcgttgggaagc (SEQ ID NO: 14543)
Probes: tacgtacgagtttcgtacgcgtattatttcgtcg (SEQ ID NO: 14544) (Taqman probe FAM/BHQ1 labelled)

Lightcycler program:
95˚10 mins

55 cycles 95˚15 secs (20˚/sec)
62˚45secs (20˚/sec) detection

Gene SEQ ID NO: 9 (MSP assay)
Primers: acttaaaaatcgccgaatcg (SEQ ID NO: 14545) & agttttatagttaattagggtagttgtcgtcg (SEQ ID NO: 14546)
Probes: tcctatcatcgctcacgaacg (SEQ ID NO: 14547) — fluo & LC640-cgacaacgactatccttactattataa-p (SEQ ID NO: 14548) (Lightcycler probes)

LC program:
95˚10 mins

55 cycles 95˚15 secs (20˚/sec)
60˚30 secs (20˚/sec) detection
72˚15 secs (20˚/sec)

Gene SEQ ID NO: 17 (HeavyMethyl™ assay)

| | | | |
|---|---|---|---|
| **primer:** | | | |
| Ggtgttgtttattttagagagtt (SEQ ID NO: 14549) | | | |
| Cctaacccctatccttc (SEQ ID NO: 14550) | | | |
| **blocker:** | | | |
| ccttcaccaccttcccaacactacaacac-pho (SEQ ID NO: 14551) | | | |
| **probes:** | | | |
| tcggcgtcggtcgttagg-fluo (SEQ ID NO: 14552) | | | |

(continued)

| | | | |
|---|---|---|---|
| **primer:** | | | |
| red640-cgcgggagtagttagatgcgtc-pho (SEQ ID NO: 14553) | | | |
| | | | |
| **LightCycler program:** | | | |
| activation: | 95°C | 10min | |
| | | | |
| 55 cycles: | 95°C | 10 sec | (20°C/s) |
| | **56°C** | 30 sec | (20°C/s) |
| | 72°C | 10 sec | (20°C/s) |
| | | | |
| melting curve: | 95°C | 10sec | 20 |
| | 40°C | 10se | 20 |
| | 70°C | 0sec | 0,1 |
| | | | |
| cooling: | 40°C | 5 sec | |

Gene SEQ ID NO: 7 (HeavyMethyl™ assay)

| | | | | |
|---|---|---|---|---|
| **Primer :** | | | | |
| Tgagagagagagggttgaaa (SEQ ID NO: 14554) | | | | |
| Tctaaataacaaaatacctccatt (SEQ ID NO: 14555) | | | | |
| **probes:** | | | | |
| CGaccCGccaacCGac-fluo (SEQ ID NO: 14556) | | | | |
| LCred640-CGcCGaaaCGCGctc-p (SEQ ID NO: 14557) | | | | |
| **blocker:** | | | | |
| ccattaccaacacaacccaccaaccaa-p (SEQ ID NO: 14558) | 14558) | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Gene SEQ ID NO: 8 (HeavyMethyl™assays)

| | | | | |
|---|---|---|---|---|
| **primer:** | | | | |
| Aaaaaaaaaaaactcctctacatac (SEQ ID NO: 14559) | | | | |
| Ggttattgtttgggttaataaatg (SEQ ID NO: 14560) | | | | |
| **blocker:** | | | | |
| acatacaccacaaataaattaccaaaaacatcaaccaa-pho (SEQ ID NO: 14561) | | | | |
| **probes:** | | | | |
| tttttttttcggacgtcgtt-fluo (SEQ ID NO: 14562) | | | | |
| red640-tcggtcgatgttttcggtaa-pho (SEQ ID NO: 14563) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

| | | | | |
|---|---|---|---|---|
| **primer:** | | | | |
| Aaaaaaaaaaaactcctctacatac (SEQ ID NO: 14564) | | | | |
| Ggttattgtttgggttaataaatg (SEQ ID NO: 14565) | | | | |
| **blocker:** | | | | |
| ctctacataCAcCACAaataaattacCAaaaacatC-p (SEQ ID NO: 14566) | | | | |
| **probes:** | | | | |
| CGCGaataaattacCGaaaacatc-fluo (SEQ ID NO: 14567) | | | | |
| LCred640-accgaacaacgacgtcc-p (SEQ ID NO: 14568) | | | | |
| | | | | |
| **LightCycler program**: | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |

(continued)

| | | | | |
|---|---|---|---|---|
| **primer:** | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

| | | | | |
|---|---|---|---|---|
| **primer:** | | | | |
| gaggtgttagaggagtagtag (SEQ ID NO: 14569) | | | | |
| Tccccctacaacctaaa (SEQ ID NO: 14570) | | | | |
| **blocker:** | | | | |
| Acctaaacaacacactcccacacactaaaacaccaat (SEQ ID NO: 14571) | | | | |
| **probes:** | | | | |
| Cgagtcggcgcggga-fluo (SEQ ID NO: 14572) | | | | |
| Lcred640-agggcgttttgttggcgatc-p (SEQ ID NO: 14573) | | | | |
| | | | | |
| **LightCycler program:** | | | | |
| activation: | 95°C | 10min | | |
| | | | | |
| 55 cycles: | 95°C | 10 sec | (20°C/s) | |
| | **58°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Gene SEQ ID NO: 9 (HeavyMethyl™ assays)

| | | | | |
|---|---|---|---|---|
| **Primer:** | | | | |
| ctatccatatacctaactaattaataac (SEQ ID NO: 14574) | | | | |
| gtaaagtttttaatttttattagtattagtat (SEQ ID NO: 14575) | | | | |
| Blocker | | | | |
| ctaattaataacacttaaaacacaattataaacaccaccatac-pho (SEQ ID NO: 14576) | | | | |
| Probe: | | | | |
| Red640-atttagttaaagtttatgtcgattatagttacgttagttt-pho (SEQ ID NO: 14577) | | | | |
| gtgaacggtttcgtcgggag-fluo (SEQ ID NO: 14578) | | | | |
| | | | | |
| **LightCycler program:** | | | | |
| activation: | 95°C | 10min | | |
| | | | | |
| 55 cycles: | 95°C | 10 sec | (20°C/s) | |
| | 56°C | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |
| | | | | |
| gttttattgtatgatgaattgga (SEQ ID NO: 14579) | | | | |
| caccaaaaaaatattaaccac (SEQ ID NO: 14580) | | | | |
| Blocker: | | | | |

(continued)

| | | | | |
|---|---|---|---|---|
| **Primer:** | | | | |
| Ttaaccacaacacaaataaaaccaaacaaattatccata-pho (SEQ ID NO: 14581) | | | | |
| Probe: | | | | |
| Gtttttcgcgttcgtcgtttatttt-fluo (SEQ ID NO: 14582) | | | | |
| Red640-ttgggtcgacgagattaaggagg-pho (SEQ ID NO: 14583) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Gene SEQ ID NO: 5 (HeavyMethyl™ assay)

| | | | | |
|---|---|---|---|---|
| **primer:** | | | | |
| Gagaggtttattaaggtaggg (SEQ ID NO: 14584) | | | | |
| Ctactaccaaaaataaaaaattattaac (SEQ ID NO: 14585) | | | | |
| **blocker:** | | | | |
| Aaaattattaacatccaacacatttatccaaacttcaaa (SEQ ID NO: 14586) | | | | |
| **probes:** | | | | |
| gtttaggggtttgtagagaagttcga-fluo (SEQ ID NO: 14587) | | | | |
| red640-tcggataaacgcgtcgg-pho (SEQ ID NO: 14588) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10c | 20 | |
| | 40°C | 10sec | 20 | |
| | 70°C | 0sec | 0,1 | |
| | | | | |
| cooling: | 40°C | 5 sec | | |

Gene 6480 (HeavyMethyl™ assay)

| | | | | |
|---|---|---|---|---|
| **Primer:** | | | | |
| gagttgagagaatttttaaatttt (SEQ ID NO: 14589) | | | | |
| Tcttctaaaacaaaaaacacc (SEQ ID NO: 14590) | | | | |
| **Blocker:** | | | | |
| Caccactacaatccaaacaacccaaa-pho (SEQ ID NO: 14591) | | | | |
| **Probes:** | | | | |
| Ggacgtcgagcgttgggtat-fluo (SEQ ID NO: 14592) | | | | |
| Red640-aggacgcgtagttggacggt-pho (SEQ ID NO: 14593) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |

(continued)

| Primer: | | | | |
|---|---|---|---|---|
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Gene SEQ ID NO: 28 (HeavyMethyl™ assay)

| Primer : | | | |
|---|---|---|---|
| Gaaggaagggaaagtttt (SEQ ID NO: 14594) | | | |
| Taaaaaaaacactaccctac (SEQ ID NO: 14595) | | | |
| | | | |
| **Blocker:** | | | |
| ctaccacaataaaaacccaaaacactccttcacacca-p (SEQ I D NO : 14596) | | | |
| | | | |
| **Probe** | | | |
| acccgaaacgctccttcacg-fluo (SEQ ID NO: 14597) | | | |
| red640-cgtaaacaaacgaaacgacgactacg-Pho (SEQ I D NO: 14598) | | | |
| | | | |
| | | | |
| **LC program Roche:** | | | |
| denat at 95°C | | | |
| 195°C | 10min | | |
| 55 cycles: | | | |
| denat at 95°C | 10 sec | (20°C/s) | |
| annealing 56°C | 30 sec | (20°C/s) | detection |
| extension 72°C | 10 sec | (20°C/s) | |
| | | | |
| melting | | | |
| 95°C | 10sec | 20 | |
| 35°C | 20sec | 20 | |
| 95°C | 0sec | 0,1 | |

Gene SEQ ID NO: 1 (HeavyMethyl™ assay)

| **oligos:** | | | | |
|---|---|---|---|---|
| Primer: | | | | |
| Cacaaactatctataaaaaattaatctc (SEQ ID NO: 14599) | | | | |

(continued)

| | | | | |
|---|---|---|---|---|
| **oligos:** | | | | |
| Gattgttgttggtgtttgttatt (SEQ ID NO: 14600) | | | | |
| Blocker: | | | | |
| ctcaaacacaaaaaaattaacatctcatctcttaaaccactttcaat-pho (SEQ I D NO: 14601) | | | | |
| Probes: | | | | |
| TCgTCTCTTAAACCgCTTTCgA-fluo (SEQ ID NO: 14602) | | | | |
| Red640-CCTCAAAATAAACACgAACATACCC-pho (SEQ ID NO: 14603) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Gene SEQ ID NO: 34 (HeavyMethyl™ assay)

| | | | | |
|---|---|---|---|---|
| **Primer:** | | | | |
| Atacaaaatacaaaaataccttct (SEQ ID NO: 14604) | | | | |
| Atttggttttattttggttatg (SEQ ID NO: 14605) | | | | |
| Blocker: | | | | |
| accttctcacaaatcaactaaaacacacacaacacc-pho (SEQ I D NO : 14606) | | | | |
| Probes: | | | | |
| LCRed640-tggatttagggcgtcgcg-pho (SEQ ID NO: 14607) | | | | |
| cgttcgtttattaagcggcga—fluo (SEQ ID NO: 14608) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |

(continued)

| **Primer:** | | | | |
|---|---|---|---|---|
| melting curve: | 95˚C | 10sec | 20 | |
| | 40˚C | 10sec | 20 | |
| | 70˚C | 0sec | 0,1 | |
| | | | | |
| cooling: | 40˚C | 5 sec | | |

Results

Sensitivity and specificity were computed with SVM and 10 fold cross validation Table 7

| Gene/Genomic location | AUC | Sensitivity | Specificity |
|---|---|---|---|
| Transcription factor BTF3 Homolog 1 | 0.57 (0.56) | 0.15 | 0.99 |
| RING FINGER PROTEIN 4 | 0.54 (0.55) | 0.91 | 0.09 |
| TUMOR NECROSIS FACTOR RECEPTOR SUPERFAMILY MEMBER 16 PRECURSOR | 0.82 (0.82) | 0.74 | 0.90 |
| Region of chromosome 14q31.1 | 0.83 (0.84) | 0.69 | 0.94 |
| Chondroitin sulfate proteoglycan 2 (versican) (CPSG2) | 0.92 (0.88) | 0.83 | 0.96 |
| EYA 4 | 0.83 (0.84) | 0.71 | 0.94 |
| MOTHERS AGAINST DECAPENTAPLEGIC HOMOLOG 7 (SMAD 7) | 0.54 (0.52) | 0.25 | 0.91 |
| ENDOPLASMIN PRECURSOR (94 KDA GLUCOSE-REGULATED PROTEIN) (GRP94) (GP96 HOMOLOG) (TUMOR REJECTION ANTIGEN 1) | 0.6 (0.6) | 0.11 | 0.99 |
| Region of chromosome 6q25.1 | 0.59 (0.57) | 0.1 | 0.99 |
| B-CELL LYMPHOMA 6 PROTEIN (BCL-6) (ZINC FINGER PROTEIN 51) (LAZ-3 PROTEIN) (BCL-5) | 0.62 (0.62) | 0.11 | 0.98 |
| HOMEOBOX PROTEIN ARISTALESS-LIKE 4 | 0.78 (0.75) | 0.74 | 0.95 |
| TRANSMEMBRANE PROTEIN WITH EGF-LIKE AND TWO FOLLISTATIN-LIKE DOMAINS 2 | 0.88 (0.88) | 0.84 | 0.94 |
| Hypothetical protein-leucine rich repeat | 0.73 (0.72) | 0.79 | 0.38 |
| Candidate tumor suppressor 8p22 | 0.79 (0.84) | 0.64 | 0.76 |
| VESICULAR INHIBITORY AMINO ACID TRANSPORTER (GABA AND GLYCINE TRANSPORTER) (VESICULAR GABA TRANSPORTER) (HVIAAT) | 0.77 (0.77) | 0.78 | 0.44 |

Sensitivity and specificity of multiple gene (panel) assays.

Panel: Assay 9 , Assay 12
Sensitivity: 0.88421052631579
Specificity: 0.972972972972973
Panel: Assay 11 , Assay 12
Sensitivity: 0.88421052631579
Specificity: 0.97027027027027
Panel: Assay 12 , Assay 15
Sensitivity: 0.9
Specificity: 0.95
Panel: Assay 6 , Assay 7
Sensitivity: 0.87
Specificity: 0.973333333333333
Panel: Assay 5 , Assay 11
Sensitivity: 0.894736842105263
Specificity: 0.941333333333333
Panel: Assay 5 , Assay 7
Sensitivity: 0.894736842105263
Specificity: 0.938666666666667
Panel: Assay 4 , Assay 6
Sensitivity: 0.857894736842105
Specificity: 0.971428571428571
Panel: Assay 7 , Assay 12
Sensitivity: 0.905263SEQ ID NO: 94737
Specificity: 0.921621621621622
Panel : Assay 6, Assay 12
Sensitivity: 0.842105263SEQ ID NO: 95
Specificity: 0.978378378378378
Panel: Assay 5 , Assay 9
Sensitivity: 0.842105263SEQ ID NO: 95
Specificity: 0.973333333333333
Panel: Assay 3 , Assay 15
Sensitivity: 0.857142857142857
Specificity: 0.953424657534247
Panel: Assay 1 , Assay 12
Sensitivity: 0.842105263SEQ ID NO: 95
Specificity: 0.967567567567568
Panel: Assay 5 , Assay 15
Sensitivity: 0.857H2857142857
Specificity: 0.945205479452055
Panel: SEQ ID NO: 17.2 , Assay 5
Sensitivity: 0.81764705882353
Specificity: 0.966233766233766
Panel: Assay 3 , Assay 12
Sensitivity: 0.8
Specificity: 0.983783783783784
Panel: Assay 6 , Assay 11
Sensitivity: 0.83SEQ ID NO: 947368421
Specificity: 0.952
Panel: Assay 5 , Assay 12
Sensitivity: 0.8105263SEQ ID NO: 9474
Specificity: 0.972972972972973
Panel: Assay 3 , Assay 11
Sensitivity: 0.83SEQ ID NO: 947368421
Specificity: 0.949333333333333
Panel: Assay 3 , Assay 5
Sensitivity: 0.823529411764706
Specificity: 0.955844155844156
Panel: Assay 3 , SEQ ID NO: 17.2

Sensitivity: 0.8
Specificity: 0.974025974025974

Class prediction by supervised learning

In order to give a reliable estimate of how well the CpG ensemble of a selected marker set can differentiate between different tissue classes we determined its prediction accuracy by classification. For that purpose we calculated a methylation profile based prediction function. This step is called training and it exploits the prior knowledge represented by the data labels. The prediction accuracy of that function is then tested by cross-validation or on a set of independent samples. As a method of choice, we used the support vector machine (SVM) algorithm (Duda (2001), Christiannini (2000)) to learn the prediction function. If not stated otherwise, for this report the risk associated with false positive or false negative classifications are set to be equal relative to the respective class sizes. It follows that the learning algorithm obtains a class prediction function with the objective to optimize accuracy on an independent test sample set. Therefore sensitivity and specificity of the resulting classifier can be expected to be approximately equal.Figures 87 to 95 show the performance of quadratic SVMs trained on the full data set using the following assays.

Figure 87 Assays 12 and 9
Figure 88 : Assays 12 and 11
Figure 89 : Assays 12 and 15
Figure 90 : Assays 7 and 6
Figure 91 : Assays 11 and 5
Figure 92 : Assays 7 and 5
Figure 93 : Assays 6 and 4
Figure 94 : Assays 12 and 7
Figure 95 : Assays 12 and 6

**Example 15**

HeavyMethyl analysis.

In the following, methylation of the genes/genomic sequences according to Gene SEQ ID Nos: 7, 8 & 17 was analysed in 15 colorectal cancer samples (referred to as 'colon' in Table 7), and eight peripheral blood lymphocyte samples referred to as 'PBL' in Table 7).

Total genomic DNA of all samples was bisulfite treated converting unmethylated cytosines to uracil. Methylated cytosines remained conserved. Bisulfite treatment was performed with minor modifications according to the protocol described in Olek et al. (1996).

The sequence of interest was then amplified by means of primers specific for bisulfite treated DNA and a blocking oligonucleotide (in order to suppress amplification of non-methylated DNA). The 3'OH terminus of the blocking oligonucleotide was "capped" by a phosphate g roup to prevent polymerase from extending the primer during PCR reaction. T he amplificate is then detected by means of methylation specific LightCycler™ probes, wherein one probe was labelled with 5'red640 and had a capped 3'OH terminus and the other was labelled with 3'Fluorescein.

Genomic SEQ ID N0:8
Primers: aaaaaaaaaaaactcctctacatac (SEQ ID NO: 14609) & ggttattgtttgggttaataaatg (SEQ ID NO: 14610)
Blocking oligonucleotide: acatacaccacaaataaattaccaaaaacatcaaccaa (SEQ ID NO: 14611+14612)
Probes: ttttttttttcggacgtcgtt (SEQ ID NO: 14613)
(Fluorescein) & tcggtcgatgttttcggtaa (red640) (SEQ ID NO: 14614)

Genomic SEQ ID NO : 7
Primers: ggtttgggatggttgt(SEQ ID NO: 14615) &
tctaaataacaaaatacctccatt (SEQ ID NO: 14616)
Blocking oligonucleotides: ccattaccaacacaacccaccaaccaa (SEQ ID NO: 14617)
Probes: ggtcgcggtgagagagagag (Fluorescein) & (SEQ IDNO: 14618) tgaaattagagcgcgtttcgg (red640) (SEQ ID NO: 14619)

Genomic SEQ ID NO:17
Primers: ggtgttgtttattttagagagtt (SEQ ID NO: 14620) & cctaacccctatccttc (SEQ ID NO: 14621)
Blocking oligonucleotides: ccttcaccaccttcccaacactacaacac (SEQ ID NO: 14622) Probes: tggggtttcggcgaagtaa (Fluorescein) (SEQ ID NO: 14623) & tttttcggcgtcggtcgt (red640) (SEQ ID NO: 14624)

The reactions were performed in a total volume of 20 μl using a LightCycler device (Roche Diagnostics). The real time PCR reaction mix contained 10 μl of template DNA, 2 μl of FastStart LightCycler reaction mix for hybridization probes (Roche Diagnostics, Penzberg),

Table 7: Results of HeavyMethyl assays

| Tissue | Crossing points | | Crossing points | | Calculated Concentration DNA undiluted (pg/10 μ1l) |
|---|---|---|---|---|---|
| | Genomic SEQ ID NO: 3 | Genomic SEQ ID NO: 12 | Genomic SEQ ID NO: 4 (undiluted) | Genomic SEQ ID NO: 4 (diluted) | |
| Colon | 36,73 | 38,22 | 39,58 | | 5,855 |
| Colon | 33,1>51 | >51 | | | 386,9 |
| Colon | 39,8 | 42,84 | | | 57,5 |
| Colon | 39 , 74 | 38,75 | 32,54 | 33,89 | 482,6 |
| Colon | | >51 | | | 132,55 |
| Colon | | 38,82 | | | 29,955 |
| Colon | 32,08 | 33,9 | 33,43 | 35,43 | 816 |
| Colon | 37,03 | 36,91 | | | 284,35 |
| Colon | 38,07 | 46,54 | | | 0 |
| Colon | 35,22 | 37,81 | 36,8 | 37,03 | 298,35 |
| Colon | 33,66 | 36,19 | 33,23 | 34,01 | 1355 |
| Colon | 36,78 | 37,46 | 40,71 | | 879 |
| Colon | | 37,23 | | | 57,5 |
| Colon | | | | | 1669 |
| colon | 34,88 | 34,71 | 34,21 | 34,49 | 880,5 |
| PBL | | | | | 716,5 |
| PBL | | | | | 796 |
| PBL | | | | | 584,5 |
| PBL | 38,55. | 38,55. | | | 1813 |
| PBL | | | | | 5470 |
| PBL | | 49,04 | | | 761 |
| PBL | | | | | 625 |
| PBL | | 39,75 | | | 9835 |
| M 100pg | 36,34 | 37,29 | 34,95 | 34,51 | 583,5 |
| M 100pg | 36,06 | 37,86 | 34,64 | 34,09 | 428,3 |
| uMRoche 100ng | | 34,48 | | | |
| uMPhi 100ng | | | | | |
| Water | | | | | |

Bold type figures indicate those classified as positive because the amplification curves were flat.

## Example 15

Serum analysis of the genes according to SEQ ID Nos: 7, 30, 28 & 8.

The assays as described above in example 13 (MSP) & 14 (HeavyMethyl) were then applied to a set of serum samples consisting 32 colorectal carcinoma serum samples, and compared to a set consisting of 54 normal serum samples, 11 serum samples from benign colon lesions and 6 serum samples from inflammatory colon disease. 130 ul of serum was used per assay and the results were quantitatively reported as positive or negative.

Table 6

| Marker or combination of markers | Sensitivity | Specificity |
|---|---|---|
| SEQ ID NO: 8 (HeavyMethyl) | 25 | 92.9 |
| SEQ ID NO: 8 (MSP) | 40.6 | 94.3 |
| SEQ ID NO: 30 (MSP) | 21.9 | 97.1 |
| SEQ ID NO: 28 (MSP) | 37.5 | 77.6 |
| SEQ ID NO: 7 (MSP) | 15.6 | 98.3 |
| SEQ ID NO: 8, 8 & 30 | 50 | 95.7 |
| SEQ ID NO: 8, 30 & 28 | 59.4 | 78.9 |

**FURTHER TABLES**

TABLE 1.

| ***Gene:*** | ***Primer:*** | ***Amplificate Length:*** |
|---|---|---|
| (SEQ ID NO: 16) | TTGGAGTTAAAGTATTTGGTAAGA (SEQ IDNO: 536) AAAACCACCTTCAAACCC (SEQ ID NO: 537) | 442 |
| (SEQ ID NO: 4) | ATCCTCCACACTCTTCCTCTAT (SEQ ID NO: 538) GAAATTAGGTTTGGTTTTGTTT (SEQ ID NO: 539) | 140 |
| (SEQ ID NO: 4) | GAGATTTTGGGAGGGGTAG (SEQ ID NO: 540) AACTCTATCCTTTTCCCTCTTC (SEQ ID NO: 541) | 486 |
| (SEQ ID NO: 56) | TGTTGGTTGTTGTTGTTGTT (SEQ ID NO: 542) CTTTCTACCCATCCCAAAA (SEQ ID NO: 543) | 319 |
| (SEQ ID NO: 27) | TAAGTGATAAAGGAAGGAAGGA (SEQ ID NO: 544) CCTTCAAACCCCAAACAA (SEQ ID NO: 545) | 243 |
| (SEQ ID NO: 31) | TTGTTGTTTTTAGGGGTTTTGG (SEQ ID NO: 546) TCCTTCCCATTCTCCAAATATC (SEQ ID NO: 547) | 947 |

(continued)

| Gene: | Primer: | Amplificate Length: |
|---|---|---|
| (SEQ ID NO: 32) | TCAACTACCATCAACTTCCTTA (SEQ ID NO: 548) AATTTATTTTTAGTGTTGTAGTGGG (SEQ ID NO: 549) | 491 |
| (SEQ ID NO: 33) | GAAAGGAGAGGTTAAAGGTTG (SEQ ID NO: 550) AACTCACTTAACTCCAATCCC (SEQ ID NO: 551) | 696 |
| (SEQ ID NO: 34) | GGATAGGAGTTGGGATTAAGAT (SEQ ID NO: 552) AAATCTTTTTCAACACCAAAAT (SEQ ID NO: 553) | 414 |
| (SEQ ID NO: 24) | TCCAATAAACACAAACCTAAATC (SEQ ID NO: 554) ATATGGGATTGATGGAAGATAG (SEQ ID NO: 555) | 471 |
| (SEQ ID NO: 35) | GGAAGAGGTGATTAAATGGAT (SEQ ID NO: 556) CCCAAAAATCAAACAACAA (SEQ ID NO: 557) | 226 |
| (SEQ ID NO: 57) | ATTTGGGAAAGAGGGAAAG (SEQ ID NO: 558) TAAAAACTCTAAACCCCATCC (SEQ ID NO: 559) | 300 |
| (SEQ ID NO: 25) | CCCTACCCACCAATATACC (SEQ ID NO: 560) AGATTTGGGGAAGAAGTTGTA (SEQ ID NO: 561) | 278 |
| (SEQ ID NO: 36) . | TAAAAGAAGGATTTTTGATTGG (SEQ ID NO: 562) CATCTTATTTACCTCCCTCCC (SEQ ID NO: 563) | 528 |
| (SEQ ID NO: 28) | TTTTAGATTGAGGTTTTAGGGT (SEQ ID NO: 564) ATCCATTCTACCTCCTTTTTCT (SEQ ID NO: 565) | 497 |
| (SEQ ID NO: 37) | GTAATTTGAAGAAAGTTGAGGG (SEQ ID NO: 566) CCAACAACTAAACAAAACCTCT (SEQ ID NO: 567) | 296 |
| (SEQ ID NO: 26) | AGTAAATAGTGGGTGAGTTATGAA (SEQ ID NO: 568) GAAAAACCTCTAAAAACTACTCTCC (SEQ ID NO: 569) | 607 |
| (SEQ ID NO: 38) | GTTAGTATGTTTGGGGGTAAAT (SEQ ID NO: 570) ATAAATAACACCTTCCACCCTA (SEQ ID NO: 571) | 435 |

(continued)

| Gene: | Primer: | Amplificate Length: |
|---|---|---|
| (SEQ ID NO: 39) | TTTGTATTAGGTTGGAAGTGGT (SEQ ID NO: 572) CCCAAATAAATCAACAACAACA (SEQ ID NO: 573) | 286 |
| (SEQ ID NO: 29) | TTGTTTGGGTTAATAAATGGA (SEQ ID NO: 574) CTTCTCTCTTCTCCCCTCTC (SEQ ID NO: 575) | 295 |
| (SEQ ID NO: 40) | AATATAGGGAGGTTTAGGGTTT (SEQ ID NO: 576) TAACCATACATTTCTCATCCAA (SEQ ID NO: 577) | 424 |
| (SEQ ID NO: 41) | TTTTGGGGAATATAGGGTTT (SEQ ID NO: 578) TTCTCACATTTCTAACCACTTCT (SEQ ID NO: 579) | 425 |
| (SEQ ID NO: 5) | CTCCTCCTTCCAACAAAAA (SEQ ID NO: 580) GTTTAGAGGTTTTGGGATGATT (SEQ ID NO: 581) | 487 |
| (SEQ ID NO: 5) | TGAATAGGGTGATATTTTAGTTAGG (SEQ ID NO: 582) ATAAATCATCCCAAAACCTCTA (SEQ ID NO: 583) | 497 |
| (SEQ ID NO: 6) | ATTTGGTTATTGGTTGAAGGTA (SEQ ID NO: 584) AATTTTTAATTTCTCAACACCTCT (SEQ ID NO: 585) | 361 |
| (SEQ ID NO: 6) | GAAGAGGTGTTGAGAAATTAAAA (SEQ ID NO: 586) CCCACCCTAACTTACCATAAA (SEQ ID NO: 587) | 462 |
| (SEQ ID NO: 8) | CAATTCCCCTTATTTCTCTAAA (SEQ ID NO: 588) AATTAGTTATGGTGTTGTGGGA (SEQ ID NO: 589) | 339 |
| (SEQ ID NO: 8) | TTCTATTAAAACCCAACTCCTC (SEQ ID NO: 590) ATAAGGGGAATTGTTGTAGGTT (SEQ ID NO: 591) | 395 |
| (SEQ ID NO: 42) | TACCATTCTTTCCTAAACATCC (SEQ ID NO: 592) GGGTTGGTGGAGAGGTAG (SEQ ID NO: 593) | 148 |
| (SEQ ID NO: 14) | GAAGATGAGAGGAGGTTGAGA (SEQ ID NO: 594) CCACACCACCTACTACAAAAT (SEQ ID NO: 595) | 500 |

(continued)

| Gene: | Primer: | Amplificate Length: |
|---|---|---|
| (SEQ ID NO: 15) | AACAAACCTCCTCCAAATTC (SEQ ID NO: 596) TGTTGGTAGGTATTGGTGATT (SEQ ID NO: 597) | 365 |
| (SEQ ID NO: 15) | TCCCCACTTAAAATAAACAAAT (SEQ ID NO: 598) GTGAATTTGGAGGAGGTTT (SEQ ID NO: 599) | 375 |
| (SEQ ID NO: 7) | GGGGTTGATATTGTTTTTAGAG (SEQ ID NO: 600) CCCCTCCTTCCTTAAATCT (SEQ ID NO: 601) | 328 |
| (SEQ ID NO: 44) | TTTTAGAAGGGGTTGGTTTAG (SEQ ID NO: 602) ACTACCTAACTCTCCCCACAA (SEQ ID NO: 603) | 343 |
| (SEQ ID NO: 44) | TTGTGGGGAGAGTTAGGTAGT (SEQ ID NO: 604) TAACCCAAATATCATAAAACCC (SEQ ID NO: 605) | 411 |
| (SEQ ID NO: 1) | AGATGGATATTTTGTTGGTGTT (SEQ ID NO: 606) TACACAATTATACCTTTCAAACAAT (SEQ ID NO: 607) | 250 |
| (SEQ ID NO: 1) | CCATACAAATATCCTAAATAAAACC (SEQ ID NO: 608) TTGTTGGAAGAATTGATAGTGTT (SEQ ID NO: 609) | 482 |
| (SEQ ID NO: 1) | AGGGAGTTAAGTAAGGGGTTAG (SEQ ID NO:610) AACACCAACAAAATATCCATCT (SEQ ID NO: 611) | 442 |
| (SEQ ID NO: 2) | TGGAATTTTAGGGTTAGTAGGG (SEQ ID NO: 612) CAAATAAACCAAACCACTATCA (SEQ ID NO: 613) | 499 |
| (SEQ ID NO: 2) | TTGGGATTTTTAGGTGGTTT (SEQ ID NO: 614) TTCACTTTCCCTACTAACCCTA (SEQ ID NO: 615) | 492 |
| (SEQ ID NO: 45) | TGGGTTATTTGGTGAGTATTGT (SEQ ID NO: 616) CTTACCCCCACCCAACTA (SEQ ID NO: 617) | 461 |
| (SEQ ID NO: 46) | TTCACATTACATTAACCCATTTA (SEQ ID NO: 618) TTGGAGTTGTTAGGAGAAAAGT (SEQ ID NO: 619) | 452 |

(continued)

| Gene: | Primer: | Amplificate Length: |
|---|---|---|
| (SEQ ID NO: 10) | CCTTCCTTAAAAACCTCAAAAC (SEQ ID NO: 620) GTAAAGAATGGTAGAGGATAGGAT (SEQ ID NO: 621) | 436 |
| (SEQ ID NO: 10) | CTTACTACCCAACCTCTTTCAC (SEQ ID NO: 622) TGGAAGGATAGAGAATTTTGTT (SEQ ID NO: 623) | 444 |
| (SEQ ID NO: 11) | ATCCCATCTCTCAACTCCTACT (SEQ ID NO: 624) TGATTTATTTTGATGTGTGGTT (SEQ ID NO: 625) | 452 |
| (SEQ ID NO: 11) | TATTTAAGGATTTTGGAAGGAG (SEQ ID NO: 626) TCATCTCATTTTATCTCTACAACC (SEQ ID NO: 627) | 349 |
| (SEQ ID NO: 13) | AACAAATTCCCAACACACC (SEQ ID NO: 628) TTTTGGAAGATGGTTTATTTTT (SEQ ID NO: 629) | 476 |
| (SEQ ID NO: 13) | TTTTTAATATGGAGGTAAGGGA (SEQ ID NO: 630) AAATTCCCAACACACCAAC (SEQ ID NO: 631) | 279 |
| (SEQ ID NO: 3) | CTTCTCCAAAATCAACCAACT (SEQ ID NO: 632) TTTGTGTTATTAGTAGGTGAGAGG (SEQ ID NO: 633) | 254 |
| (SEQ ID NO: 3) | TTAGAAGTTGGAGGGTGAAAT (SEQ ID NO: 634) CTTCCTACCTTAAACCCTTACC (SEQ ID NO: 635) | 450 |
| (SEQ ID NO: 18) | TCTAACTCCTCACAAATTCCTAA (SEQ ID NO: 636) GTAGTGTAATAGGGAAAGGGG (SEQ ID NO: 637) | 498 |
| (SEQ ID NO: 48) | TAAAATTCCCTCTTACCCTAAA (SEQ ID NO: 638) TAGTAAGGATTGTAGAAGGGGG (SEQ ID NO: 639) | 396 |
| (SEQ ID NO: 48) | TAGTAAGGATTGTAGAAGGGGG (SEQ ID NO: 640) CCTCAAACCCTAAAAATAACC (SEQ ID NO: 641) | |
| (SEQ ID NO: 58) | GGAGAGGAGTGTTTGTAGAAGA (SEQ ID NO: 642) CAATCTCCCCTAAATCCTAAT (SEQ ID NO: 643) | 369 |

(continued)

| Gene: | Primer: | Amplificate Length: |
|---|---|---|
| (SEQ ID NO: 22) | TAGTAGTTTGAAGAAGGGGAAG (SEQ ID NO: 644) AAACATTCCTAAAATCACAAAAA (SEQ ID NO: 645) | 373 |
| (SEQ ID NO: 19) | TTTATTTGGGTATGATTAGGTTTT (SEQ ID NO: 646) | 426 |
| | CTAAAAACACCACCCCCT (SEQ ID NO: 647) | |
| (SEQ ID NO: 19) | ACAAACCAAAATCTTACTTCCTA (SEQ ID NO: 648) GAATGGAGGGGAAATGTTA (SEQ ID NO: 649) | 458 |
| (SEQ ID NO: 17) | AAAACTCCTCCCCTCTATAAAT (SEQ ID NO: 650) TTGGAGAGATGTGTTGGTTAG (SEQ ID NO: 651) | 492 |
| (SEQ ID NO: 17) | AATCCTAACCAACACATCTCTC (SEQ ID NO: 652) AGGGGATTTTAAGGTGATTAGT (SEQ ID NO: 653) | 482 |
| (SEQ ID NO: 23) | CTCCCCATCCATCTTATTTTA (SEQ ID NO: 654) ATTGTTTGGGTGATAGTGAAGT (SEQ ID NO: 655) | 489 |
| (SEQ ID NO: 21) | TAAGTTTTTGGAGGAAGAGTTT (SEQ ID NO: 656) AAAATACTCCCTATAATTCCCC (SEQ ID NO: 657) | 370 |
| (SEQ ID NO: 21) | TTTCTCTAACCAAACACCTAAAA (SEQ ID NO: 658) AGAAATTAGTAGAGGAGGGAGG (SEQ ID NO: 659) | 398 |
| (SEQ ID NO: 43) | ATCTAATCCCTCTCCTAACTCC (SEQ ID NO: 660) TTTGTTTTGGAATTTAGGTTTT (SEQ ID NO: 661) | 441 |
| (SEQ ID NO: 9) | TCCACAAAACTCTCCTACTAAAA (SEQ ID NO: 662) GGAAGGTTGGGTAGATATAGG (SEQ ID NO: 663) | 186 |
| (SEQ ID NO: 12) | TTGGTAGAGTTGAAAGGAGATAG (SEQ ID NO: 664) AAAAACATTCCCTAAAAATTCC (SEQ ID NO: 665) | 402 |
| (SEQ ID NO: 47) | ATAGAATGGTTAGGGGGTATTT (SEQ ID NO: 666) TACAAATATCAACCTCTCTCCC (SEQ ID NO: 667) | 484 |

(continued)

| Gene: | Primer: | Amplificate Length: |
|---|---|---|
| (SEQ ID NO: 20) | GGTGGGGTATAATAGTAGGGAT (SEQ ID NO: 668) CTTCCCCTCTTTCATTTTATTT (SEQ ID NO: 669) | 448 |
| (SEQ ID NO: 50) | GAGGAATTGGTATTGAAAGAAA (SEQ ID NO: 670) CTAATCCACCCTCCATAAAAC (SEQ ID NO: 671) | 426 |
| (SEQ ID NO: 50) | CTCCAATTCTCCTCCCTATATC (SEQ ID NO: 672) TAATTTTTGAGGTTGGGAAA (SEQ ID NO: 673) | 425 |

TABLE 2.

| *No: | Gene | Oligo: |
|---|---|---|
| 1 | (SEQ ID NO: 41) | TGGACGTAGGAAAGCGA (SEQ ID NO: 681) |
| 2 | (SEO ID NO: 41) | GATGTAGGAAAGTGAGA (SEQ ID NO: 682) |
| 3 | (SEQ ID NO: 41) | ATTTACGGGAGTTTTATCGT (SEQ ID NO: 683) |
| 4 | (SEQ ID NO: 41) | ATTTATGGGAGTTTTATTGT (SEQ ID NO: 684) |
| 5 | (SEQ ID NO: 41) | ATTAGTTCGGGTCGCGT (SEQ ID NO: 685) |
| 6 | (SEQ ID NO: 41) | ATTAGTTTGGGTTGTGT (SEQ ID NO: 686) |
| 7 | (SEQ ID NO: 41) | TATACGAAAGGGAGGCGG (SEQ ID NO: 687) |
| 8 | (SEQ ID NO: 41) | TATATGAAAGGGAGGTGG (SEQ ID NO: 688) |
| 9 | (SEQ ID NO: 41) | GGCGTGTCGTTAGTTTTA (SEQ ID NO: 689) |
| 10 | (SEQ ID NO: 41) | GGTGTGTTGTTAGTTTTATA (SEQ ID NO: 690) |
| 11 | (SEQ ID NO: 41) | TTCGATTGACGTTAGCGA (SEQ ID NO: 691) |
| 12 | (SEQ ID NO: 41) | TTTGATTGATGTTAGTGA (SEQ ID NO: 692) |
| 13 | (SEQ ID NO: 41) | TTTCGAGTTTGACGGT (SEQ ID NO: 693) |
| 14 | (SEQ ID NO: 41 ) | TTTTGAGTTTGATGGTT (SEQ ID NO: 694) |
| 15 | (SEQ ID NO: 41) | TTCGGAGGGCGTATTT (SEQ ID NO: 695) |

(continued)

| *No: | Gene | Oligo: |
|---|---|---|
| 16 | (SEQ ID NO: 41) | TTTGGAGGGTGTATTT (SEQ ID NO: 696) |
| 17 | (SEQ ID NO: 5) | GACGTCGGTACGTAGT (SEQ ID NO: 697) |
| 18 | (SEQ ID NO: 5) | GATGTTGGTATGTAGTAG (SEQ ID NO: 698) |
| 19 | (SEQ ID NO: 5) | TTCGGGGGAATTCGAGT (SEQ ID NO: 699) |
| 20 | (SEQ ID NO: 5) | TTTGGGGGAATTTGAGT (SEQ ID NO: 700) |
| 21 | (SEQ ID NO: 5) | TATTGCGAGGATTCGG (SEQ ID NO: 701) |
| 22 | (SEQ ID NO: 5) | ATTGTGAGGATTTGGT (SEQ ID NO: 702) |
| 23 | (SEQ ID NO: 5) | GTGCGTTCGTAGCGTA (SEQ ID NO: 703) |
| 24 | (SEQ ID NO: 5) | TGTGTTTGTAGTGTAGG (SEQ ID NO: 704) |
| 25 | (SEQ ID NO: 5) | GGACGTCGTTTGTTAG (SEQ ID NO: 705) |
| 26 | (SEQ ID NO: 5) | GGATGTTGTTTGTTAGG (SEQ ID NO: 706) |
| 27 | (SEQ ID NO: 5) | AGAGCGTCGTTTTGTA (SEQ ID NO: 707) |
| 28 | (SEQ ID NO: 5) | AGAGTGTTGTTTTGTAT (SEQ ID NO: 708) |
| 29 | (SEQ ID NO: 5) | TTTCGAGGGTAGGCGAG (SEQ ID NO: 709) |
| 30 | (SEQ ID NO: 5) | TTTTGAGGGTAGGTGAG (SEQ ID NO: 710) |
| 31 | (SEQ ID NO: 5) | TTTCGATTTTAATGCGAA (SEQ ID NO: 711) |
| 32 | (SEQ ID NO: 5) | TTTGATTTTAATGTGAAGT (SEQ ID NO: 712) |
| 33 | (SEQ m NO: 5) | AGGAATTTCGTCGCGA (SEQ ID NO: 713) |
| 34 | (SEQ ID NO: 5) | AGGAATTTTGTTGTGAT (SEQ ID NO: 714) |
| 35 | (SEQ ID NO: 5) | TTTGAGTCGTACGCGT (SEQ ID NO: 715) |
| 36 | (SEQ ID NO: 5) | TTTTGAGTTGTATGTGT (SEQ ID NO: 716) |
| 37 | (SEQ ID NO: 6) | TACGTAGTTGCGCGTT (SEQ ID NO:717) |

(continued)

| *No: | Gene | Oligo: |
|---|---|---|
| 38 | (SEQ ID NO: 51) | GTATGTAGTTGTGTGTT (SEQ ID NO: 674) |
| 39 | (SEQ ID NO: 6) | AATCGGCGGTTAGGAT (SEQ ID NO: 718) |
| 40 | (SEQ ID NO: 6) | GAATTGGTGGTTAGGA (SEQ ID NO: 719) |
| 41 | (SEQ ID NO: 6) | TTTGATCGGGTTTGAG (SEQ ID NO: 720) |
| 42 | (SEQ ID NO: 6) | TTTTGATTGGGTTTGAG (SEQ ID NO: 721 ) |
| 43 | (SEQ ID NO: 51) | TTTGAGTATTCGTAGGAA (SEQ ID NO: 675) |
| 44 | (SEQ ID NO: 6) | TGAGTATTTGTAGGAAGA (SEQ ID NO: 722) |
| 45 | (SEQ ID NO: 6) | AGAGGCGCGGGTTATA (SEQ ID NO: 723) |
| 46 | (SEQ ID NO: 6) | TAGAGGTGTGGGTTAT (SEQ ID NO: 724) |
| 47 | (SEQ ID NO: 6) | TTAGCGGTTAAGTTGCGA (SEQ ID NO: 725) |
| 48 | (SEQ ID NO: 6) | TTAGTGGTTAAGTTGTGA (SEQ ID NO: 726) |
| 49 | (SEQ ID NO: 8) | TTCGTAGAAGAATACGCGTA (SEQ ID NO: 727) |
| 50 | (SEQ ID NO: 8) | TTTGTAGAAGAATATGTGTA (SEQ ID NO: 728) |
| 51 | (SEQ ID NO: 8) | AAACGTTTATCGGTTG (SEQ ID NO: 729) |
| 52 | (SEQ ID NO: 8) | AATGITTATTGGTTGGA (SEQ ID NO: 730) |
| 53 | (SEQ ID NO: 8) | TATCGTAGTTCGTTCGG (SEQ ID NO: 731) |
| 54 | (SEQ ID NO: 8) | ATTGTAGTTTGTTTGGT (SEQ ID NO: 732) |
| 55 | (SEQ ID NO: 16) | TGGTCGGTATATTTCGA (SEQ ID NO: 733) |
| 56 | (SEQ ID NO: 16) | TTGGTTGGTATATTTTGA (SEQ ID NO: 734) |
| 57 | (SEQ ID NO: 16) | GGAGGTTTCGGTTCGA (SEQ ID NO: 735) |
| 58 | (SEQ ID NO: 16) | TGGAGGTTTTGGTTTGA (SEQ ID NO: 736) |
| 59 | (SEQ ID NO: 16) | TTAGCGGTAATAGCGG (SEQ ID NO: 737) |

(continued)

| *No: | Gene | Oligo: |
|---|---|---|
| 60 | (SEQ ID NO: 52) | TATTAGTGGTAATAGTGG (SEQ ID NO: 676) |
| 61 | (SEQ ID NO: 42) | TGCGTAGTAGGCGGTT (SEQ ID NO: 738) |
| 62 | (SEQ ID NO: 53) | TGTGTAGTAGGTGGTTT (SEQ ID NO: 677) |
| 63 | (SEQ ID NO: 42) | TAGGCGGTTGTTCGTA (SEQ ID NO: 739) |
| 64 | (SEQ ID NO: 42) | AGGTGGTTGTTTGTAA (SEQ ID NO: 740) |
| 65 | (SEQ ID NO: 14) | TTGAAGTCGGTACGGT (SEQ ID NO: 1125) |
| 66 | (SEQ ID NO: 14) | TGAAGTTGGTATGGTT (SEQ ID NO: 1126) |
| 67 | (SEQ ID NO: 14) | TGGGACGCGGATATTT (SEQ ID NO: 1127) |
| 68 | (SEQ ID NO: 14) | GTTGGGATGTGGATAT (SEQ ID NO: 1128) |
| 69 | (SEQ ID NO: 43) | GTTCGGGTCGATTCGA (SEQ ID NO: 741) |
| 70 | (SEQ ID NO: 43) | GGTTTGGGTTGATTTGA (SEQ ID NO: 742) |
| 71 | (SEQ ID NO: 43) | TTCGGGATATATTCGATT (SEQ ID NO: 743) |
| 72 | (SEQ ID NO: 43) | TTTTGGGATATATTTGATT (SEQ ID NO: 744) |
| 73 | (SEQ ID NO: 43) | TATTCGAATTGTATTCGT (SEQ ID NO: 745) |
| 74 | (SEQ ID NO: 43) | TTTGAATTGTATTTGTTTAT (SEQ ID NO: 746) |
| 75 | (SEQ ID NO: 15) | TTAAGTTCGATTCGGT (SEQ ID NO: 747) |
| 76 | (SEQ ID NO: 54) | AGTTTGATTTGGTGAAT (SEQ ID NO: 678) |
| 77 | (SEQ ID NO: 15) | TAGTTGTTCGAGAGGG (SEQ ID NO: 748) |
| 78 | (SEQ ID NO: 15) | AGTTGTTTGAGAGGGT (SEQ ID NO: 749) |
| 79 | (SEQ ID NO: 15) | ATAGTATCGAGGTGAGT (SEQ ID NO: 750) |
| 80 | (SEQ ID NO: 15) | ATAGTATTGAGGTGAGTT (SEQ ID NO: 751) |
| 81 | (SEQ ID NO: 15) | TTCGGGATTCGATAAT (SEQ ID NO: 752) |

(continued)

| *No: | Gene | Oligo: |
|---|---|---|
| 82 | (SEQ ID NO: 15) | AGGTTTTGGGATTTGA (SEQ ID NO: 753) |
| 83 | (SEQ ID NO: 15) | TTAGGACGCGGCGATA (SEQ ID NO: 754) |
| 84 | (SEQ ID NO: 15) | AGGATGTGGTGATAGT (SEQ ID NO: 755) |
| 85 | (SEQ ID NO: 15) | TTGTACGTTCGGTATT (SEQ ID NO: 756) |
| 86 | (SEQ ID NO: 15) | TAAGTTGTATGTTTGGTA (SEQ ID NO: 757) |
| 87 | (SEQ ID NO: 4) | GGCGTCGAGGTCGTAG (SEQ ID NO: 758) |
| 88 | (SEQ ID NO: 4) | GGTGTTGAGGTTGTAGT (SEQ ID NO: 759) |
| 89 | (SEQ ID NO: 4) | AGGGTTTCGATTTTCGG (SEQ ID NO: 760) |
| 90 | (SEQ ID NO: 4) | AGGGTTTTGATTTTTGG (SEQ ID NO: 761) |
| 91 | (SEQ ID NO: 4) | TGGAACGTGCGATTGT (SEQ ID NO: 762) |
| 92 | (SEQ ID NO: 4) | TGGAATGTGTGATTGT (SEQ ID NO: 763) |
| 93 | (SEQ ID NO: 4) | TTTTGGCGCGTTTATA (SEQ ID NO: 764) |
| 94 | (SEQ ID NO: 4) | TTGGTGTGTTTATAGATA (SEQ ID NO: 765) |
| 95 | (SEQ ID NO: 4) | AGATTTTTACGATTCGA (SEQ ID NO: 766) |
| 96 | (SEQ ID NO: 4) | TTTTTATGATTTGAAATAGA (SEQ ID NO: 767) |
| 97 | (SEQ ID NO: 4) | AGTATTTTCGCGTGTT (SEQ ID NO: 768) |
| 98 | (SEQ ID NO: 4) | TAGTATTTTTGTGTGTTAA (SEQ ID NO: 769) |
| 99 | (SEQ ID NO: 7) | TTCGTCGGCGGTAGAG (SEQ ID NO: 770) |
| 100 | (SEQ ID NO: 7) | TAGAGTTTGTTGGTGG (SEQ ID NO: 771) |
| 101 | (SEQ ID NO: 7) | GATCGCGGGTACGTTT (SEQ ID NO: 772) |
| 102 | (SEQ ID NO: 7) | ATTGTGGGTATGTTTGT (SEQ ID NO: 773) |
| 103 | (SEQ ID NO: 7) | TTAACGTCGTTGGTTA (SEQ ID NO: 774) |

(continued)

| *No: | Gene | Oligo: |
|---|---|---|
| 104 | (SEQ ID NO: 7) | TGATTAATGTTGTTGGT (SEQ ID NO: 775) |
| 105 | (SEQ ID NO: 7) | TTCGCGCGAAGATTTA (SEQ ID NO: 776) |
| 106 | (SEQ ID NO: 7) | GTTTTTGTGTGAAGATT (SEQ ID NO: 777) |
| 107 | (SEQ ID NO: 44) | TTCGATATCGTGACGG (SEQ ID NO: 778) |
| 108 | (SEQ ID NO: 44) | TTTTGATATTGTGATGGT (SEQ ID NO: 779) |
| 109 | (SEQ ID NO: 44) | AGAATACGGTCGTAGA (SEQ ID NO: 780) |
| 110 | (SEQ ID NO: 44) | TAGAATATGGTTGTAGATA (SEQ ID NO: 781) |
| 111 | (SEQ ID NO: 44) | TATTTTTGCGTACGGG (SEQ ID NO: 782) |
| 112 | (SEQ ID NO: 44) | ATTTTTGTGTATGGGTT (SEQ ID NO: 783) |
| 113 | (SEQ ID NO: 1) | TTACGGTGAAGGCGGA (SEQ ID NO: 784) |
| 114 | (SEQ ID NO: 1) | TTATGGTGAAGGTGGA (SEQ ID NO: 785) |
| 115 | (SEQ ID NO: 1) | TTCGGGATTAATATCGAGAT (SEQ ID NO: 786) |
| 116 | (SEQ ID NO: 1) | TTTGGGATTAATATTGAGAT (SEQ ID NO: 787) |
| 117 | (SEQ ID NO: 1) | TTTCGGTTTTCGTTAAT (SEQ ID NO: 788) |
| 118 | (SEQ ID NO: 1) | TTTGGTTTTTGTTAATTTAG (SEQ ID NO: 789) |
| 119 | (SEQ ID NO: 1) | TGTGCGAAGTTAACGT (SEQ ID NO: 790) |
| 120 | (SEQ ID NO: 1) | TTGTGTGAAGTTAATGT (SEQ ID NO: 791) |
| 121 | (SEQ ID NO: 1) | AAGTTTATCGGCGTTT (SEQ ID NO: 792) |
| 122 | (SEQ ID NO: 1) | AGAAGTTTATTGGTGTTT (SEQ ID NO: 793) |
| 123 | (SEQ ID NO: 1) | ATTTCGGAATTTAAGCGT (SEQ ID NO: 794) |
| 124 | (SEQ ID NO: 1) | TTTGGAATTTAAGTGTTT (SEQ ID NO: 795) |
| 125 | (SEQ ID NO: 1) | TTTTCGCGATTGGAGA (SEQ ID NO: 796) |

(continued)

| *No: | Gene | Oligo: |
|---|---|---|
| 126 | (SEQ ID NO: 1) | GTTTTTGTGATTGGAGA (SEQ ID NO: 797) |
| 127 | (SEQ ID NO:1) | ATTTACGCGTTTTAGG (SEQ ID NO: 798) |
| 128 | (SEQ ID NO:1) | ATGGAATTTATGTGTTTT (SEQ ID NO: 799) |
| 129 | (SEQ ID NO: 1) | ATGTCGCGGTTTTATA (SEQ ID NO: 800) |
| 130 | (SEQ ID NO: 1) | GGATGTTGTGGTTTTAT (SEQ ID NO: 801) |
| 131 | (SEQ ID NO: 2) | AGACGGGGTTTACGAG (SEQ ID NO: 802) |
| 132 | (SEQ ID NO: 2) | AGATGGGGTTTATGAG (SEQ ID NO: 803) |
| 133 | (SEQ ID NO: 2) | TGTCGGTATTAGCGTT (SEQ ID NO: 804) |
| 134 | (SEQ ID NO: 2) | GTGTTGGTATTAGTGTT (SEQ ID NO: 805) |
| 135 | (SEQ ID NO: 2) | TGGTTTACGTTCGGTA (SEQ ID NO: 806) |
| 136 | (SEQ ID NO: 2) | GGTTTATGTTTGGTAGT (SEQ ID NO: 807) |
| 137 | (SEQ ID NO: 2) | TTCGTACGGTTAGGTT (SEQ ID NO: 808) |
| 138 | (SEQ ID NO: 2) | AGTTTTGTATGGTTAGG (SEQ ID NO: 809) |
| 139 | (SEQ ID NO: 45) | ATAGCGATTTCGGCGA (SEQ ID NO: 810) |
| 140 | (SEQ ID NO: 45) | AGTGATTTTGGTGAGA (SEQ ID NO: 811) |
| 141 | (SEQ ID NO: 45) | GGCGTTTTATTTACGAGA (SEQ ID NO: 812) |
| 142 | (SEQ ID NO: 45) | GGGTGTTTTATTTATGAG (SEQ ID NO: 813) |
| 143 | (SEQ ID NO: 45) | ATCGTGGACGGTAACGA (SEQ ID NO: 814) |
| 144 | (SEQ ID NO: 45) | ATTGTGGATGGTAATGA (SEQ ID NO: 815) |
| 145 | (SEQ ID NO: 45) | TTGAGATCGATTCGTT (SEQ ID NO: 816) |
| 146 | (SEQ ID NO: 45) | TGAGATTGATTTGTTTAG (SEQ ID NO: 817) |
| 147 | (SEQ ID NO: 45) | GGCGAGATTCGTACGT (SEQ ID NO: 818) |

(continued)

| *No: | Gene | Oligo: |
|---|---|---|
| 148 | (SEQ ID NO: 45) | GGTGAGATTTGTATGTT (SEQ ID NO: 819) |
| 149 | (SEQ ID NO: 45) | TGACGTTCGTGGTGGA (SEQ ID NO: 820) |
| 150 | (SEQ ID NO: 45) | GATGTTTGTGGTGGAG (SEQ ID NO: 821) |
| 151 | (SEQ ID NO: 45) | GTGATCGATTACGGTA (SEQ ID NO: 822) |
| 152 | (SEQ ID NO: 45) | AGGTGATTGATTATGGT (SEQ ID NO: 823) |
| 153 | (SEQ ID NO: 45) | ATTATTCGTTCGGTGA (SEQ ID NO: 824) |
| 154 | (SEQ ID NO: 45) | TATTATTTGTTTGGTGAG (SEQ ID NO: 825) |
| 155 | (SEQ ID NO: 45) | TATCGTCGTTAAGTGT (SEQ ID NO: 826) |
| 156 | (SEQ ID NO: 45) | TATTATTGTTGTTAAGTGT (SEQ ID NO: 827) |
| 157 | (SEQ ID NO: 45) | TGTAAGCGCGAGAATA (SEQ ID NO: 828) |
| 158 | (SEQ ID NO: 45) | AGTGTAAGTGTGAGAAT (SEQ ID NO: 829) |
| 159 | (SEQ ID NO: 9) | TATAGCGGTTTACGGT (SEQ ID NO: 830) |
| 160 | (SEQ ID NO: 9) | TAGTGGTTTATGGTAGT (SEQ ID NO: 831) |
| 161 | (SEQ ID NO: 9) | AGGGCGATTAGGACGT (SEQ ID NO: 832) |
| 162 | (SEQ ID NO: 9) | AGGGTGATTAGGATGT (SEQ ID NO: 833) |
| 163 | (SEQ ID NO: 46) | TTCGTTAGAGTTCGTAG (SEQ ID NO: 834) |
| 164 | (SEQ ID NO: 46) | TTTGTTAGAGTTTGTAGT (SEQ ID NO: 835) |
| 165 | (SEQ ID NO: 46) | TGAGACGTTTGTCGGT (SEQ ID NO: 836) |
| 166 | (SEQ ID NO: 46) | TGAGATGTTTGTTGGT (SEQ ID NO: 837) |
| 167 | (SEQ ID NO: 46) | GAAAAGTTCGTCGGTT (SEQ ID NO: 838) |
| 168 | (SEQ ID NO: 46) | AGAAAAGTTTGTTGGTT (SEQ ID NO: 839) |
| 169 | (SEQ ID NO: 46) | ATGGCGTAGTCGCGAT (SEQ ID NO: 840) |

(continued)

| *No: | Gene | Oligo: |
|---|---|---|
| 170 | (SEQ ID NO: 46) | TGGTGTAGTTGTGATT (SEQ ID NO: 841) |
| 171 | (SEQ ID NO: 10) | TTTTGACGTCGATGTA (SEQ ID NO: 842) |
| 172 | (SEQ ID NO: 10) | TGATGTTGATGTAGAATT (SEQ ID NO: 843) |
| 173 | (SEQ ID NO: 10) | TTGCGATGTGCGTTTA (SEQ ID NO: 844) |
| 174 | (SEQ ID NO: 10) | TGTGATGTGTGTTTAGT (SEQ ID NO: 845) |
| 175 | (SEQ ID NO: 10) | TGATTACGGCGCGGAT (SEQ ID NO: 846) |
| 176 | (SEQ ID NO: 10) | ATTATGGTGTGGATGG (SEQ ID NO: 847) |
| 177 | (SEQ ID NO: 10) | AGATGGCGACGTCGAA (SEQ ID NO: 848) |
| 178 | (SEQ ID NO: 10) | ATGGTGATGTTGAAGA (SEQ ID NO: 849) |
| 179 | (SEQ ID NO: 10) | TTTAAGCGCGGCGGTA (SEQ ID NO: 850) |
| 180 | (SEQ ID NO: 10) | TTTTAAGTGTGGTGGTA (SEQ ID NO: 851) |
| 181 | (SEQ ID NO: 10) | AGAAACGTAGACGCGA (SEQ ID NO: 852) |
| 182 | (SEQ ID NO: 10) | AATGTAGATGTGATGGA (SEQ ID NO: 853) |
| 183 | (SEQ ID NO: 11) | AGAGACGCGAAAAATT (SEQ ID NO: 854) |
| 184 | (SEQ ID NO: 11) | TAGAGAGATGTGAAAAAT (SEQ ID NO: 855) |
| 185 | (SEQ ID NO: 11) | AGACGAAAGAGTCGTT (SEQ ID NO: 856) |
| 186 | (SEQ ID NO: 11) | AGAGATGAAAGAGTTGT (SEQ ID NO: 857) |
| 187 | (SEQ ID NO: 11) | TTTTAGTTCGAGCGTA (SEQ ID NO: 858) |
| 188 | (SEQ ID NO: 11) | TTAGTTTGAGTGTAGTTA (SEQ ID NO: 859) |
| 189 | (SEQ ID NO: 11) | GACGTGAATTTTCGGAA (SEQ ID NO: 860) |
| 190 | (SEQ ID NO: 11) | AGGATGTGAATTTTTGG (SEQ ID NO: 861) |
| 191 | (SEQ ID NO: 11) | AATGCGTGGTCGTTTT (SEQ ID NO: 862) |

(continued)

| *No: | Gene | Oligo: |
|---|---|---|
| 192 | (SEQ ID NO: 11) | GAAATGTGTGGTTGTT (SEQ ID NO: 863) |
| 193 | (SEQ ID NO: 11) | TTTCGTTTGCGGAATT (SEQ ID NO: 864) |
| 194 | (SEQ ID NO: 11) | TTTTGTTTGTGGAATTG (SEQ ID NO: 865) |
| 195 | (SEQ ID NO: 12) | TGTTCGACGTGATTTT (SEQ ID NO: 866) |
| 196 | (SEQ ID NO: 12) | GTGTTTGATGTGATTTT (SEQ ID NO: 867) |
| 197 | (SEQ ID NO: 12) | TAACGTTTTTTCGGGT (SEQ ID NO: 868) |
| 198 | (SEQ ID NO: 12) | TTAATGTTTTTTTGGGTG (SEQ ID NO: 869) |
| 199 | (SEQ ID NO: 12) | TGTTGATTCGGAAATGA (SEQ ID NO: 870) |
| 200 | (SEQ ID NO: 12) | TTTGTTGATTTGGAAATG (SEQ ID NO: 871) |
| 201 | (SEQ ID NO: 13) | TAAAGTTTCGAAGCGG (SEQ ID NO: 1129) |
| 202 | (SEQ ID NO: 13) | AGTTTTGAAGTGGAGT (SEQ ID NO: 1130) |
| 203 | (SEQ ID NO: 13) | AAGTCGGTAGTTATCGT (SEQ ID NO: 1131) |
| 204 | (SEQ ID NO: 13) | AAGTTGGTAGTTATTGTT (SEQ ID NO: 1132) |
| 205 | (SEQ ID NO: 3) | TTGGAGCGCGAGAAAG (SEQ ID NO: 872) |
| 206 | (SEQ ID NO: 3) | TTGGAGTGTGAGAAAG (SEQ ID NO: 873) |
| 207 | (SEQ ID NO: 3) | TACGTTATCGGTTCGT (SEQ ID NO: 874) |
| 208 | (SEQ ID NO: 3) | TATGTTATTGGTTTGTATT (SEQ ID NO: 875) |
| 209 | (SEQ ID NO: 3) | ATTAGGTTCGTGGGCGT (SEQ ID NO: 876) |
| 210 | (SEQ ID NO: 3) | ATTAGGTTTGTGGGTGT (SEQ ID NO: 877) |
| 211 | (SEQ ID NO: 3) | TGCGGTTTAGAAACGTAG (SEQ ID NO: 878) |
| 212 | (SEQ ID NO: 3) | TGTGGTTTAGAAATGTAG (SEQ ID NO: 879) |
| 213 | (SEQ ID NO: 3) | GAACGGGTTTCGTAGT (SEQ ID NO: 880) |

(continued)

| *No: | Gene | Oligo: |
|---|---|---|
| 214 | (SEQ ID NO: 3) | GGGAATGGGTTTTGTA (SEQ ID NO: 881) |
| 215 | (SEQ ID NO: 3) | TTGCGATAGTCGGCGG (SEQ ID NO: 882) |
| 216 | (SEQ ID NO: 3) | TTGTGATAGTTGGTGG (SEQ ID NO: 883) |
| 217 | (SEQ ID NO: 3) | AAGAACGGACGTGTTT (SEQ ID NO: 884) |
| 218 | (SEQ ID NO: 3) | AGGAAGAATGGATGTG (SEQ ID NO: 885) |
| 219 | (SEQ ID NO: 47) | AAGTTTCGTTTGGGAG (SEQ ID NO: 886) |
| 220 | (SEQ ID NO: 47) | AAAGTTTTGTTTGGGAG (SEQ ID NO: 887) |
| 221 | (SEQ ID NO: 47) | TTGGAAGTCGAAGAGA (SEQ ID NO: 888) |
| 222 | (SEQ ID NO: 47) | TTTGGAAGTTGAAGAGA (SEQ ID NO: 889) |
| 223 | (SEQ ID NO: 18) | TATCGGGTTCGATTTT (SEQ ID NO: 890) |
| 224 | (SEQ ID NO:18) | GGTGTATTGGGTTTGA (SEQ ID NO: 891) |
| 225 | (SEQ ID NO: 20) | TAGGGATTCGCGGAGG (SEQ ID NO: 892) |
| 226 | (SEQ ID NO: 20) | TAGGGATTTGTGGAGG (SEQ ID NO: 893) |
| 227 | (SEQ ID NO: 20) | TTGTCGAGTAATTTTCGT (SEQ ID NO: 894) |
| 228 | (SEQ ID NO: 20) | TGTTGAGTAATTTTTGTT (SEQ ID NO: 895) |
| 229 | (SEQ ID NO: 20) | TATTACGGGCGGAGGG (SEQ ID NO: 896) |
| 230 | (SEQ ID NO: 20) | TATTATGGGTGGAGGG (SEQ ID NO: 897) |
| 231 | (SEQ ID NO: 20) | GACGGTACGTTAGAGG (SEQ ID NO: 898) |
| 232 | (SEQ ID NO: 20) | GATGGTATGTTAGAGGT (SEQ ID NO: 899) |
| 233 | (SEQ ID NO: 20) | TTGGGCGTCGTTATTA (SEQ ID NO: 900) |
| 234 | (SEQ ID NO: 20) | TGGGTGTTGTTATTAGT (SEQ ID NO: 901) |
| 235 | (SEQ ID NO: 20) | TATTAGTTCGGTCGTT (SEQ ID NO: 902) |

(continued)

| *No: | Gene | Oligo: |
|---|---|---|
| 236 | (SEQ ID NO: 20) | AGTTTGGTTGTTAGTTT (SEQ ID NO: 903) |
| 237 | (SEQ ID NO: 20) | TTATTACGTTTAGCGAT (SEQ ID NO: 904) |
| 238 | (SEQ ID NO: 20) | TTTTTATTATGTTTAGTGATA (SEQ ID NO: 905) |
| 239 | (SEQ ID NO: 20) | ATAGCGAGTGCGATAT (SEQ ID NO: 906) |
| 240 | (SEQ ID NO: 48) | AGGGTCGTAGCGGTAG (SEQ ID NO: 907) |
| 241 | (SEQ ID NO: 48) | GAGGGTTGTAGTGGTA (SEQ ID NO: 908) |
| 242 | (SEQ ID NO: 50) | TTAGGTCGGACGTAAG (SEQ ID NO: 1143) |
| 243 | (SEQ ID NO: 50) | GGTTAGGTTGGATGTA (SEQ ID NO: 1144) |
| 244 | (SEQ ID NO: 22) | TAGACGTGGGGTTACGT (SEQ ID NO: 909) |
| 245 | (SEQ ID NO: 22) | TAGATGTGGGGTTATGT (SEQ ID NO: 910) |
| 246 | (SEQ ID NO: 22) | ATTTCGGGGTAGTATCGT (SEQ ID NO: 911) |
| 247 | (SEQ ID NO: 22) | ATTTTGGGGTAGTATTGT (SEQ ID NO: 912) |
| 248 | (SEQ ID NO: 19) | TACGCGCGTTTTAAAA (SEQ ID NO: 913) |
| 249 | (SEQ ID NO: 19) | TTATGTGTGTTTTAAAATG (SEQ ID NO: 914) |
| 250 | (SEQ ID NO: 19) | TACGATATCGTTATATAACGG (SEQ ID NO: 915) |
| 251 | (SEQ ID NO: 19) | TATGATATTGTTATATAATGG (SEQ ID NO: 916) |
| 252 | (SEQ ID NO: 19) | TATAGGTTCGCGGTTT (SEQ ID NO: 917) |
| 253 | (SEQ ID NO: 19) | TATATAGGTTTGTGGTTT (SEQ ID NO: 918) |
| 254 | (SEQ ID NO: 19) | TAGGTGCGCGTTATAT (SEQ ID NO: 919) |
| 255 | (SEQ ID NO: 19) | ATGTAGGTGTGTGTTAT (SEQ ID NO: 920) |
| 256 | (SEQ ID NO: 55) | TACGTTGTTTGGACGAAT (SEQ ID NO: 679) |
| 257 | (SEQ ID NO: 19) | TATGTTGTTTGGATGAAT (SEQ ID NO: 921) |

(continued)

| *No: | Gene | Oligo: |
|---|---|---|
| 258 | (SEQ ID NO: 19) | AAGGAGCGTATTTCGG (SEQ ID NO: 922) |
| 259 | (SEQ ID NO: 19) | AGGAGTGTATTTTGGG (SEQ ID NO: 923) |
| 260 | (SEQ ID NO: 55) | GTCGGATTTCGGAAGT (SEQ ID NO: 680) |
| 261 | (SEQ ID NO: 19) | GTTGGATTTTGGAAGTG (SEQ ID NO: 924) |
| 262 | (SEQ ID NO: 19) | GAAGTGACGCGTTCGT (SEQ ID NO: 925) |
| 263 | (SEQ ID NO: 19) | GAAGTGATGTGTTTGT (SEQ ID NO: 926) |
| 264 | (SEQ ID NO: 19) | TGTTATCGTTGCGCGA (SEQ ID NO: 927) |
| 265 | (SEQ ID NO: 19) | ATGTTATTGTTGTGTGA (SEQ ID NO: 928) |
| 266 | (SEQ ID NO: 17) | TGAAAACGTTTTTCGT (SEQ ID NO: 929) |
| 267 | (SEQ ID NO: 17) | AATGTTTTTTGTAAAGAAA (SEQ ID NO: 930) |
| 268 | (SEQ ID NO: 17) | AGGATTTCGGCGTTAT (SEQ ID NO: 931) |
| 269 | (SEQ ID NO: 17) | AAAGGATTTTGGTGTTA (SEQ ID NO: 932) |
| 270 | (SEQ ID NO: 17) | ATTTATTCGTGCGTTT (SEQ ID NO: 933) |
| 271 | (SEQ ID NO: 17) | TATTTGTGTGTTTAGGG (SEQ ID NO: 934) |
| 272 | (SEQ ID NO: 17) | TTTCGGTGGTTTTCGAA (SEQ ID NO: 935) |
| 273 | (SEQ ID NO: 17) | TTTGGTGGTTTTTGAAG (SEQ ID NO: 936) |
| 274 | (SEQ ID NO: 17) | GGCGTACGGAATTTTA (SEQ ID NO: 937) |
| 275 | (SEQ ID NO: 17) | GGGTGTATGGAATTTTA (SEQ ID NO: 938) |
| 276 | (SEQ ID NO: 17) | TGGACGGAGGTTTCGT (SEQ ID NO: 939) |
| 277 | (SEQ ID NO: 17) | TGGATGGAGGTTTTGT (SEQ ID NO: 940) |
| 278 | (SEQ ID NO: 17) | TGCGGACGGGATAGTT (SEQ ID NO: 941) |
| 279 | (SEQ ID NO: 17) | TGTGGATGGGATAGTT (SEQ ID NO: 942) |

(continued)

| *No: | Gene | Oligo: |
|---|---|---|
| 280 | (SEQ ID NO: 17) | TGATTAGTCGATTCGT (SEQ ID NO: 943) |
| 281 | (SEQ ID NO: 17) | GATGTAGGGATGGAGA (SEQ ID NO: 944) |
| 282 | (SEQ ID NO: 17) | TATCGTGGTTTTTACGTAT (SEQ ID NO: 945) |
| 283 | (SEQ ID NO: 17) | ATATTGTGGTTTTTATGTA (SEQ ID NO: 946) |
| 284 | (SEQ ID NO: 17) | TTTATTCGGTGTTCGA (SEQ ID NO: 947) |
| 285 | (SEQ ID NO: 17) | TATTTGGTGTTTGAGAG (SEQ ID NO: 948) |
| 286 | (SEQ NO: 23) | GAGGCGCGTTATTTTT (SEQ ID NO: 1133) |
| 287 | (SEQ NO: 23) | GGGAGGTGTGTTATTTT (SEQ ID NO: 1134) |
| 288 | (SEQ ID NO: 23) | AACGGTAGTTAGCGATA (SEQ ID NO: 1135) |
| 289 | (SEQ ID NO: 23) | TGAATGGTAGTTAGTGA (SEQ ID NO: 1136) |
| 290 | (SEQ ID NO: 23) | TTTTAACGTTCGCGGA (SEQ ID NO: 1137) |
| 291 | (SEQ ID NO: 23) | AATGTTTGTGGAGGAT (SEQ ID NO: 1138) |
| 292 | (SEQ ID NO: 21) | TTTTTCGCGTATATGTT (SEQ ID NO: 1139) |
| 293 | (SEQ ID NO: 21) | TTTTTTGTGTATATGTTTAGG (SEQ ID NO: 1140) |
| 294 | (SEQ ID NO: 21) | AAGGGCGGTAAGACGG (SEQ ID NO: 1141) |
| 295 | (SEQ ID NO: 21) | AAGGGTGGTAAGATGG (SEQ ID NO: 1142) |
| 296 | (SEQ ID NO: 32) | GGTTTCGTTTAATCGT (SEQ ID NO: 949) |
| 297 | (SEQ ID NO: 32) | GGGTTTTGTTTAATTGTA (SEQ ID NO: 950) |
| 298 | (SEQ ID NO: 32) | GATTCGTATTTCGTAGT (SEQ ID NO: 951) |
| 299 | (SEQ ID NO: 32) | TTTGTATTTTGTAGTGGG (SEQ ID NO: 952) |
| 300 | (SEQ ID NO: 32) | TTCGTATTTAGCGGAT (SEQ ID NO: 953) |
| 301 | (SEQ ID NO: 32) | GGTTTGTATTTAGTGGA (SEQ ID NO: 954) |

(continued)

| *No: | Gene | Oligo: |
|---|---|---|
| 302 | (SEQ ID NO: 32) | TTAATCGGCGGGTTTT (SEQ ID NO: 955) |
| 303 | (SEQ ID NO: 32) | AGTTAATTGGTGGGTT (SEQ ID NO: 956) |
| 304 | (SEQ ID NO: 32) | TATTTTGGCGGGTTGTAT (SEQ ID NO: 957) |
| 305 | (SEQ ID NO: 32) | TATTTTGGTGGGTTGTAT (SEQ ID NO: 958) |
| 306 | (SEQ ID NO: 32) | AAGGTTATCGGTTTAAGA (SEQ ID NO: 959) |
| 307 | (SEQ ID NO: 32) | AAGGTTATTGGTTTAAGA (SEQ ID NO: 960) |
| 308 | (SEQ ID NO: 32) | GGGGGACGACGTTTTTGT (SEQ ID NO: 961) |
| 309 | (SEQ ID NO: 32) | GGGGGATGATGTTTTTGT (SEQ ID NO: 962) |
| 310 | (SEQ ID NO: 33) | TTACGGTTCGGTTATT (SEQ ID NO: 963) |
| 311 | (SEQ ID NO: 33) | AGGTTTTATGGTTTGGT (SEQ ID NO: 964) |
| 312 | (SEQ ID NO: 33) | GACGTCGCGGGGTTAG (SEQ ID NO: 965) |
| 313 | (SEQ ID NO: 33) | TGATGTTGTGGGGTTA (SEQ ID NO: 966) |
| 314 | (SEQ ID NO: 33) | AGGTATTTCGCGATAT (SEQ ID NO: 967) |
| 315 | (SEQ ID NO: 33) | AGGTATTTTGTGATATTTT (SEQ ID NO: 968) |
| 316 | (SEQ ID NO: 33) | GTTTTTCGATTTACGTT (SEQ ID NO: 969) |
| 317 | (SEQ ID NO: 33) | TAGGTTTTTTGATTTATGT (SEQ ID NO: 970) |
| 318 | (SEQ ID NO: 33) | GGTAGTTTCGATTATTTA (SEQ ID NO: 971) |
| 319 | (SEQ ID NO: 33) | GGTAGTTTTGATTATTTA (SEQ ID NO: 972) |
| 320 | (SEQ ID NO: 33) | TAGAGTACGGGGCGGG (SEQ ID NO: 973) |
| 321 | (SEQ ID NO: 33) | TAGAGTATGGGGTGGG (SEQ ID NO: 974) |
| 322 | (SEQ ID NO: 33) | TTGTTTAGCGGATTTTAG (SEQ ID NO: 975) |
| 323 | (SEQ ID NO: 33) | TTGTTTAGTGGATTTTAG (SEQ ID NO: 976) |

(continued)

| *No: | Gene | Oligo: |
|---|---|---|
| 324 | (SEQ ID NO: 33) | TAGGTTCGGTTCGTTAT (SEQ ID NO: 977) |
| 325 | (SEQ ID NO: 33) | TAGGTTTGGTTTGTTATT (SEQ ID NO: 978) |
| 326 | (SEQ ID NO: 33) | TGGTGGTACGTAGTTTGG (SEQ ID NO: 979) |
| 327 | (SEQ ID NO: 33) | TTTGGCGTAGATCGGT (SEQ ID NO: 980) |
| 328 | (SEQ ID NO: 33) | TTTGGTGTAGATTGGT (SEQ ID NO: 981) |
| 329 | (SEQ ID NO: 33) | AGTGTTCGTCGTAGTT (SEQ ID NO: 982) |
| 330 | (SEQ ID NO: 33) | TGAGTGTTTGTTGTAGT (SEQ ID NO: 983) |
| 331 | (SEQ ID NO: 33) | GTGTTTAGCGCGGATT (SEQ ID NO: 984) |
| 332 | (SEQ ID NO: 33) | GGTGTTTAGTGTGGATT (SEQ ID NO: 985) |
| 333 | (SEQ ID NO: 34) | TTCGGTTAGTTTCGTAT (SEQ ID NO: 986) |
| 334 | (SEQ ID NO: 34) | TTTTGGTTAGTTTTGTATT (SEQ ID NO: 987) |
| 335 | (SEQ ID NO: 34) | GATTCGTTTGGGTAACGT (SEQ ID NO: 988) |
| 336 | (SEQ ID NO: 34) | GATTTGTTTGGGTAATGT (SEQ ID NO: 989) |
| 337 | (SEQ ID NO: 34) | GTCGAATTTAGTCGCGT (SEQ ID NO: 990) |
| 338 | (SEQ ID NO: 34) | GTTGAATTTAGTTGTGTA (SEQ ID NO: 991) |
| 339 | (SEQ ID NO: 34) | AATTCGCGAGTTTAGA (SEQ ID NO: 992) |
| 340 | (SEQ ID NO: 34) | AAAAATTTGTGAGTTTAG (SEQ ID NO: 993) |
| 341 | (SEQ ID NO: 24) | AGGGGTTCGATTAGGG (SEQ ID NO: 1145) |
| 342 | (SEQ ID NO: 24) | AGGGGTTTGATTAGGG (SEQ ID NO: 1146) |
| 343 | (SEQ ID NO: 24) | TTAGGTATACGAAAGAGTAT (SEQ ID NO: 1147) |
| 344 | (SEQ ID NO: 24) | TTAGGTATATGAAAGAGTAT (SEQ ID NO: 1148) |
| 345 | (SEQ ID NO: 24) | TGTCGTACGTTATGTT (SEQ ID NO: 1149) |

(continued)

| *No: | Gene | Oligo: |
|---|---|---|
| 346 | (SEQ ID NO: 24) | GGTGTTGTATGTTATGT (SEQ ID NO: 1150) |
| 347 | (SEQ ID NO: 24) | TTGATTGGCGGACGAG (SEQ ID NO: 1151) |
| 348 | (SEQ ID NO: 24) | TTGATTGGTGGATGAG (SEQ ID NO: 1152) |
| 349 | (SEQ ID NO: 35) | TATATATACGTGTGGGTA (SEQ ID NO: 994) |
| 350 | (SEQ ID NO: 35) | TATATATATGTGTGGGTA (SEQ ID NO: 995) |
| 351 | (SEQ ID NO: 35) | TATGTAGTCGCGTAGT (SEQ ID NO: 996) |
| 352 | (SEQ ID NO: 35) | TTTATGTAGTTGTGTAGT (SEQ ID NO: 997) |
| . 353 | (SEQ ID NO: 35) | AGTGTATGCGTAGAAGGT (SEQ ID NO: 998) |
| 354 | (SEQ ID NO: 35) | AGTGTATGTGTAGAAGGT (SEQ ID NO: 999) |
| 355 | (SEQ ID NO: 35) | TTTAGATACGAAATGTTA (SEQ ID NO: 1000) |
| 356 | (SEQ ID NO: 35) | TTTAGATATGAAATGTTA (SEQ ID NO: 1001) |
| 357 | (SEQ ID NO: 35) | AAGTAAGTCGTTGTTGTT (SEQ ID NO: 1002) |
| 358 | (SEQ ID NO: 35) | AAGTAAGTTGTTGTTGTT (SEQ ID NO: 1003) |
| 359 | (SEQ ID NO: 25) | TTTCGTCGGAGGAATT (SEQ ID NO: 1004) |
| 360 | (SEQ ID NO: 25) | GTTTTGTTGGAGGAATT (SEQ ID NO: 1005) |
| 361 | (SEQ ID NO: 25) | ATCGTTTTGTCGGACGG (SEQ ID NO: 1006) |
| 362 | (SEQ ID NO: 25) | ATTGTTTTGTTGGATGG (SEQ ID NO: 1007) |
| 363 | (SEQ ID NO: 25) | TGTCGCGATATATCGA (SEQ ID NO: 1008) |
| 364 | (SEQ ID NO: 25) | TTTGTTGTGATATATTGAT (SEQ ID NO: 1009) |
| 365 | (SEQ ID NO: 36) | AGCGTCGATTAATCGT (SEQ ID NO: 1010) |
| 366 | (SEQ ID NO: 36) | TTAAGTGTTGATTAATTGT (SEQ ID NO: 1011) |
| 367 | (SEQ ID NO: 36) | TTCGGTCGGGTTTAAA (SEQ ID NO: 1012) |

(continued)

| *No: | Gene | Oligo: |
|---|---|---|
| 368 | (SEQ ID NO: 36) | GTTTGGTTGGGTTTAAA (SEQ ID NO: 1013) |
| 369 | (SEQ ID NO: 36) | TAATCGTTAGCGGCGG (SEQ ID NO: 1014) |
| 370 | (SEQ ID NO: 36) | TTAATTGTTAGTGGTGG (SEQ ID NO: 1015) |
| 371 | (SEQ ID NO: 36) | TTAACGGGTGGGTACGT (SEQ ID NO: 1016) |
| 372 | (SEQ ID NO: 36) | TTAATGGGTGGGTATGT (SEQ ID NO: 1017) |
| 373 | (SEQ ID NO: 36) | AGGTCGTTGGTATTCGT (SEQ ID NO: 1018) |
| 374 | (SEQ ID NO: 36) | AGGTTGTTGGTATTTGT (SEQ ID NO: 1019) |
| 375 | (SEQ ID NO: 36) | TTTTCGAGTTTTATCGA (SEQ ID NO: 1020) |
| 376 | (SEQ ID NO: 36) | TTTGAGTTTTATTGAGGT (SEQ ID NO: 1021) |
| 377 | (SEQ ID NO: 36) | ATAGTCGTGGTTTCGT (SEQ ID NO: 1022) |
| 378 | (SEQ ID NO: 36) | ATAGTTGTGGTTTTGTT (SEQ ID NO: 1023) |
| 379 | (SEQ ID NO: 36) | TGACGGGCGTTTTCGA (SEQ ID NO: 1024) |
| 380 | (SEQ ID NO: 36) | GATGGGTGTTTTTGAG (SEQ ID NO: 1025) |
| 381 | (SEQ ID NO: 36) | TAATGAGCGCGTTGTA (SEQ ID NO: 1026) |
| 382 | (SEQ ID NO: 36) | ATGAGTGTGTTGTATTT (SEQ ID NO: 1027) |
| 383 | (SEQ ID NO: 28) | TTGGTTCGGGAAAGGTAA (SEQ ID NO: 1028) |
| 384 | (SEQ ID NO: 28) | TTGGTTTGGGAAAGGTAA (SEQ ID NO: 1029) |
| 385 | (SEQ ID NO: 28) | TTTCGGTGAATCGGAT (SEQ ID NO: 1030) |
| 386 | (SEQ ID NO: 28) | TTTTTGGTGAATTGGAT (SEQ ID NO: 1031) |
| 387 | (SEQ ID NO: 28) | TTCGTAAAGTCGTTGT (SEQ ID NO: 1032) |
| 388 | (SEQ ID NO: 28) | GGTTTTTTGTAAAGTTGT (SEQ ID NO: 1033) |
| 389 | (SEQ ID NO: 28) | GTTTAGTTAGCGGGTTTT (SEQ ID NO: 1034) |

(continued)

| *No: | Gene | Oligo: |
|---|---|---|
| 390 | (SEQ ID NO: 28) | GTTTAGTTAGTGGGTTTT (SEQ ID NO: 1035) |
| 391 | (SEQ ID NO: 28) | GGGCGCGTACGGTTAT (SEQ ID NO: 1036) |
| 392 | (SEQ ID NO: 28) | AGTTGGGTGTGTATGG (SEQ ID NO: 1037) |
| 393 | (SEQ ID NO: 28) | TTATCGCGCGTGGAGG (SEQ ID NO: 1038) |
| 394 | (SEQ ID NO: 28) | TTATTGTGTGTGGAGGA (SEQ ID NO: 1039) |
| 395 | (SEQ ID NO: 37) | AAAACGTGGACGTTTT (SEQ ID NO: 1153) |
| 396 | (SEQ ID NO: 37) | ATTTGGAGCGAGGAATTT (SEQ ID NO: 1154) |
| 397 | (SEQ ID NO: 37) | ATTTGGAGTGAGGAATTT (SEQ ID NO: 1155) |
| 398 | (SEQ ID NO: 37) | TTGAAAGTCGGTTAAAGT (SEQ ID NO: 1156) |
| 399 | (SEQ ID NO: 37) | TTGAAAGTTGGTTAAAGT (SEQ ID NO: 1157) |
| 400 | (SEQ ID NO: 37) | GGTAGTTACGAGAGAGTT (SEQ ID NO: 1158) |
| 401 | (SEQ ID NO: 37) | GGTAGTTATGAGAGAGTT (SEQ ID NO: 11591 |
| 402 | (SEQ ID NO: 26) | GGTGCGCGTAGAGAAT (SEQ ID NO: 1040) |
| 403 | (SEQ ID NO: 26) | GGTGTGTGTAGAGAATA (SEQ ID NO: 1041) |
| 404 | (SEQ ID NO: 26) | TAAGCGTATCGACGTT (SEQ ID NO: 1042) |
| 405 | (SEQ ID NO: 26) | ATTTTAAGTGTATTGATGT (SEQ ID NO: 1043) |
| 406 | (SEQ ID NO: 26) | AAATATCGAACGGGAT (SEQ ID NO: 1044) |
| 407 | (SEQ ID NO: 26) | ATTGAATGGGATTTAGAG (SEQ ID NO: 1045) |
| 408 | (SEQ ID NO: 26) | TTAGAGTTCGAGTTTATA (SEQ ID NO: 1046) |
| 409 | (SEQ ID NO: 26) | TTAGAGTTTGAGTTTATA (SEQ ID NO: 1047) |
| 410 | (SEQ ID NO: 26) | TTAGGCGCGGATTCGT (SEQ ID NO: 1048) |
| 411 | (SEQ ID NO: 26) | TAGGTGTGGATTTGTT (SEQ ID NO: 1049) |

(continued)

| *No: | Gene | Oligo: |
|---|---|---|
| 412 | (SEQ ID NO: 26) | TTCGCGAAGTTACGGG (SEQ ID NO: 1050) |
| 413 | (SEQ ID NO: 26) | TTTGTGAAGTTATGGGT (SEQ ID NO: 1051) |
| 414 | (SEQ ID NO: 26) | TATCGGTTCGGAGTTA (SEQ ID NO: 1052) |
| 415 | (SEQ ID NO: 26) | ATTGGTTTGGAGTTAGA (SEQ ID NO: 1053) |
| 416 | (SEQ ID NO: 26) | AAGTAGCGTCGTTATT (SEQ ID NO: 1054) |
| 417 | (SEQ ID NO: 26) | AAGTAGTGTTGTTATTGA (SEQ ID NO: 1055) |
| 418 | (SEQ ID NO: 26) | GTCGTTCGGAATTCGT (SEQ ID NO: 1056) |
| 419 | (SEQ NO: 26) | AGTTGTTTGGAATTTGT (SEQ ID NO: 1057) |
| 420 | (SEQ ID NO: 26) | TACGTGGTCGAGGGTT (SEQ ID NO: 1058) |
| 421 | (SEQ ID NO: 26) | TATGTGGTTGAGGGTT (SEQ ID NO: 1059) |
| 422 | (SEQ ID NO: 26) | GGAAGTTTCGATGGTTTA (SEQ ID NO: 1060) |
| 423 | (SEQ ID NO: 26) | GGAAGTTTTGATGGTTTA (SEQ ID NO: 1061) |
| 424 | (SEQ ID NO: 38) | GGCGTTGGTATCGTTGA (SEQ ID NO: 1062) |
| 425 | (SEQ ID NO: 38) | GGTGTTGGTATTGTTGA (SEQ ID NO: 1063) |
| 426 | (SEQ ID NO: 38) | TTAAGACGCGTTTTTT (SEQ ID NO: 1064) |
| 427 | (SEQ ID NO: 38) | AAGATGTGTTTTTTGGA (SEQ ID NO: 1065) |
| 428 | (SEQ ID NO: 38) | TTTTGTCGCGGGAATT (SEQ ID NO: 1066) |
| 429 | (SEQ ID NO: 38) | TTTTTGTTGTGGGAATT (SEQ ID NO: 1067) |
| 430 | (SEQ ID NO: 38) | ATACGTAGATTCGGAG (SEQ ID NO: 1068) |
| 431 | (SEQ ID NO: 38) | TATGTAGATTTGGAGGT (SEQ ID NO: 1069) |
| 432 | (SEQ ID NO: 39) | GAAGTGGTCGTTAGTTTT (SEQ ID NO: 1070) |
| 433 | (SEQ ID NO: 39) | GAAGTGGTTGTTAGTTTTT (SEQ ID NO: 1071) |

(continued)

| *No: | Gene | Oligo: |
|---|---|---|
| 434 | (SEQ ID NO: 39) | AAGGAATTCGTTTTGTAA (SEQ ID NO: 1072) |
| 435 | (SEQ ID NO: 39) | AAGGAATTTGTTTTGTAA (SEQ ID NO: 1073) |
| 436 | (SEQ ID NO: 39) | AATGTTTTCGTGATGTTG (SEQ ID NO: 1074) |
| 437 | (SEQ ID NO: 39) | AATGTTTTTGTGATGTTG (SEQ ID NO: 1075) |
| 438 | (SEQ ID NO: 39) | TAAAACGAGGGAGCGT (SEQ ID NO: 1076) |
| 439 | (SEQ ID NO: 39) | AAAATGAGGGAGTGTT (SEQ ID NO: 1077) |
| 440 | (SEQ ID NO: 27) | AGGAGTCGGTTTCGTA (SEQ ID NO: 1078) |
| 441 | (SEQ ID NO: 27) | AGGAGTTGGTTTTGTA (SEQ ID NO: 1079) |
| 442 | (SEQ ID NO: 27) | TAAAGCGCGGATATTT (SEQ ID NO: 1080) |
| 443 | (SEQ ID NO: 27) | GGGTAAAGTGTGGATA (SEQ ID NO: 1081) |
| 444 | (SEQ ID NO: 27) | TTTGAGCGGGTATCGA (SEQ ID NO: 1082) |
| 445 | (SEQ ID NO: 27) | TGAGTGGGTATTGAGT (SEQ ID NO: 1083) |
| 446 | (SEQ ID NO: 27) | TAGAGTCGAGGGGCGG (SEQ ID NO: 1084) |
| 447 | (SEQ NO: 27) | TAGAGTTGAGGGGTGG (SEQ ID NO: 1085) |
| 448 | (SEQ ID NO: 27) | TTTCGAGGGACGGAAG (SEQ ID NO: 1086) |
| 449 | (SEQ ID NO: 27) | TTTTGAGGGATGGAAG (SEQ ID NO: 1087) |
| 450 | (SEQ ID NO: 27) | TATGTTTTCGGCGAAT (SEQ ID NO: 1088) |
| 451 | (SEQ ID NO: 27) | TTTTGGTGAATGGGGA (SEQ ID NO: 1089) |
| 452 | (SEQ ID NO: 27) | ATAGTCGAGGAGTCGT (SEQ ID NO: 1090) |
| 453 | (SEQ ID NO: 27) | AATAGTTGAGGAGTTGT (SEQ ID NO: 1091) |
| 454 | (SEQ ID NO: 29) | ATTTGTTTCGATTAATTT (SEQ ID NO: 1092) |
| 455 | (SEQ ID NO: 29) | ATTTGTTTTGATTAATTT (SEQ ID NO: 1093) |

(continued)

| *No: | Gene | Oligo: |
|---|---|---|
| 456 | (SEQ ID NO: 29) | AATTTGCGAACGTTTGGG (SEQ ID NO: 1094) |
| 457 | (SEQ ID NO: 29) | AATTTGTGAATGTTTGGG (SEQ ID NO: 1095) |
| 458 | (SEQ ID NO: 29) | GTCGATGTTTTCGGTA (SEQ ID NO:1096) |
| 459 | (SEQ ID NO: 29) | GGTTGATGTTTTTGGTA (SEQ ID NO: 1097) |
| 460 | (SEQ ID NO:31) | GAGTTTCGTTATATCGT (SEQ ID NO: 1098) |
| 461 | (SEQ ID NO:31) | GGAGTTTTGTTATATTGT (SEQ ID NO: 1099) |
| 462 | (SEQ ID NO: 31) | TTTTTGCGGTTCGATA (SEQ ID NO: 1100) |
| 463 | (SEQ ID NO:31) | AATTTTTGTGGTTTGATA (SEQ ID NO: 1101) |
| 464 | (SEQ ID NO: 31) | TACGTTAAGGTTAACGTATA (SEQ ID NO: 1102) |
| 465 | (SEQ ID NO:31) | TATGTTAAGGTTAATGTATA (SEQ ID NO: 1103) |
| 466 | (SEQ ID NO:31) | TGTTTCGTCGTTATAAT (SEQ ID NO:1104) |
| 467 | (SEQ ID NO: 31) | GTTTTGTTGTTATAATTAGA (SEQ ID NO: 1105) |
| 468 | (SEQ ID NO: 31) | GGCGTAGGTTACGATT (SEQ ID NO: 1106) |
| 469 | (SEQ ID NO:31) | GGGGTGTAGGTTATGA (SEQ ID NO: 1107) |
| 470 | (SEQ ID NO: 31) | ATTCGTTACGGATCGT (SEQ ID NO: 1108) |
| 471 | (SEQ ID NO: 31) | TTTATTTGTTATGGATTGT (SEQ ID NO: 1109) |
| 472 | (SEQ ID NO:31) | AGTTTTCGGATTCGAA (SEQ ID NO: 1110) |
| 473 | (SEQ ID NO: 31) | AGAGTTTTTGGATTTGA (SEQ ID NO: 1111) |
| 474 | (SEQ ID NO:31) | TATTTCGAGGTAGCGG (SEQ ID NO: 1112) |
| 475 | (SEQ ID NO:31) | TTTGAGGTAGTGGGAT (SEQ ID NO: 1113) |
| 476 | (SEQ ID NO: 31) | GAGAGAAACGGTTTTTGT (SEQ ID NO: 1114) |
| 477 | (SEQ ID NO:31) | GAGAGAAATGGTTTTTGT (SEQ ID NO: 1115) |

(continued)

| *No: | Gene | Oligo: |
|---|---|---|
| 478 | (SEQ ID NO: 31) | GTTTGATGGATGTTTTT (SEQ ID NO: 1116) |
| 479 | (SEQ ID NO:31) | GTACGACGGTAAGGAT (SEQ ID NO: 1117) |
| 480 | (SEQ ID NO:31) | GTATGATGGTAAGGATTA (SEQ ID NO: 1118) |
| 481 | (SEQ ID NO: 31) | AGTTGTTTCGTAGATATT (SEQ ID NO: 1119) |
| 482 | (SEQ ID NO:31) | AGTTGTTTTGTAGATATT (SEQ ID NO: 1120) |
| 483 | (SEQ ID NO: 40) | AGTAAGCGGTTGTATAT (SEQ ID NO: 1121) |
| 484 | (SEQ ID NO: 40) | AAAAGTAAGTGGTTGTAT (SEQ ID NO:1122) |
| 485 | (SEQ ID NO: 40) | AAATTGAGCGTTTATGT (SEQ ID NO: 1123) |
| 486 | (SEQ ID NO: 40) | ATTGAGTGTTTATGTGTA (SEQ ID NO: 1124) |

Table 3: Genes or genomic locus associated with genomic sequences according to SEQ ID NO 1- SEQ ID NO 64.

| Genomic SEQ ID NO: | Bisulfite SEQ ID Nos: | Genbank Ref. ID No:/contig | Description |
|---|---|---|---|
| 1 | 304,305,420 & 421 | AL355481.12.1.12 2435 | Hypothetical protein-leucine rich repeat (Vega gene ID OTTHUMG00013001328) |
| 2 | 306. 307, 422 & 423 | AC027601.25.1.1 92732 | 5 azacytidine induced (Ensembl gene ID 141577) |
| 3 | 308, 309, 424 & 425 | AL831711.4.1.880 1 | known gene, close to ATP/GTP-binding site motif A (P-loop) protein |
| 4 | 310,311,426& 427 | NM_002938 | RING FINGER PROTEIN 4; RNF4 |
| 5 | 312,313,428 & 429 | NM_021926 | HOMEOBOX PROTEIN ARISTALESS-LIKE 4;. ALX 4 |
| 5 | 314, 315, 430 & 431 | NM_004852 | ONE CUT DOMAIN FAMILY MEMBER 2; ONC2 |
| 7 | 316, 317, 432 & 433 | NM_002507 | TUMOR NECROSIS FACTOR RECEPTOR SUPERFAMILY MEMBER 16 PRECURSOR (LOW-AFFINITY NERVE GROWTH FACTOR RECEPTOR) (NGF RECEPTOR) (GP80-LNGFR) (P75 ICD) (LOW AFFINITY NEUROTROPHIN RECEPTOR P75NTR). NGFR |

(continued)

| Genomic SEQ ID NO: | Bisulfite SEQ ID Nos: | Genbank Ref. ID No:/contig | Description |
|---|---|---|---|
| 8 | 318, 319, 434 & 435 | NM_016192 | TRANSMEMBRANE PROTEIN WITH EGF-LIKE AND TWO FOLLISTATIN-LIKE DOMAINS 2; TRANSMEMBRANE PROTEIN TENB2; TOMOREGULIN; PUTATIVE TRANSMEMBRANE PROTEIN WITH EGF-LIKE AND TWO FOLLISTATIN-LIKE DOMAINS 2. |
| 9 | 320,321,436 & 437 | NM_005221 | HOMEOBOX PROTEIN DLX-5 |
| 10 | 322, 323, 438 & 439 | NM_080552 | VESICULAR INHIBITORY AMINO ACID TRANSPORTER (GABA AND GLYCINE TRANSPORTER) VESICULAR GABA TRANSPORTER) (HVIAAT) |
| 11 | 324, 325, 440 & 441 | NM_001208 | Transcription factor BTF3 Homolog 1 |
| 12 | 326, 327, 442 & 443 | NM_005904 | MOTHERS AGAINST DECAPENTAPLEGIC HOMOLOG 7 (SMAD 7) (MOTHERS AGAINST DPP HOMOLOG 7) (SMAD7) (HSMAD7). |
| 13 | 328, 329, 444 & 445 | NM_002146 | HOMEOBOX PROTEIN HOX-B3 (HOX-2G) (HOX-2.7) |
| 14 | 330,331,446 & 447 | NM_025078 | Homo sapiens hypothetical protein FLJ22378 |
| 15 | 332,333,448 & 449 | NM_001116 | ADENYLATE CYCLASE, TYPE IX (EC 4.6.1.1) (ATP PYROPHOSPHATE-LYASE) (ADENYLYL CYCLASE). ADCY9 |
| 16 | 334, 335, 450 & 451 | NM_001706 | B-CELL LYMPHOMA 6 PROTEIN (BCL-6) (ZINC FINGER PROTEIN 51) (LAZ-3 PROTEIN) (BCL-5). |
| 17 | 336, 337, 452 & 453 | NM_014068 | SEEK1 PROTEIN |
| 18 | 338, 339, 454 & 455 | NM_017745 | BCL-6 INTERACTING COREPRESSOR ISOFORM 1 BCOR |
| 19 | 340,341,456 & 457 | NM_005643 | TRANSCRIPTION INITIATION FACTOR TFIID 28 KDA SUBUNIT (TAFII-28) (TAFII28) (TFIID SUBUNIT P30-BETA) |
| 20 | 342, 343, 458 & 459 | NM_007374 | HOMEOBOX PROTEIN SIX6 (SINE OCULIS HOMEOBOX HOMOLOG 6) (OPTIC HOMEOBOX 2) (HOMEODOMAIN PROTEIN OPTX2). |
| 21 | 344,345,460 & 461 | AP003500.2.1.161 078 | Situated between Ensemble gene IDs ENSESTG00002308609 and ENSESTG00002308691 |

(continued)

| Genomic SEQ ID NO: | Bisulfite SEQ ID Nos: | Genbank Ref. ID No:/contig | Description |
|---|---|---|---|
| 22 | 346, 347, 462 & 463 | AC092385.4.1.97 218 | SPIR-2 PROTEIN (FRAGMENT) |
| 23 | 348, 349, 464 & 465 | AC007223.5.1.15 9520 | Situated within Ensemble EST ID ENSESTG00001971415 |
| 24 | 350,351,466 & 467 | NM_005229 | ETS-DOMAIN PROTEIN ELK-1 |
| 25 | 352,353,468 & 469 | NM_000179 | DNA MISMATCH REPAIR PROTEIN MSH6 (MUTS-ALPHA 160 KDA SUBUNIT) (G/T MISMATCH BINDING PROTEIN) (GTBP) (GTMBP) (P160) |
| 26 | 354,355,470 & 471 | NM_005427 | TUMOR PROTEIN P73 (P53-LIKE TRANSCRIPTION FACTOR) (P53-RELATED PROTEIN). |
| 27 | 356,357,472 & 473 | NM_002093 | GLYCOGEN SYNTHASE KINASE-3 BETA (EC 2.7.1.37) (GSK-3 BETA). |
| 28 | 358,359,474 & 475 | NM_006765 | N33 PROTEIN |
| 29 | 360,361,476 & 477 | NM_016192 | TRANSMEMBRANE PROTEIN WITH EGF-LIKE AND TWO FOLLISTATIN-LIKE DOMAINS 2; TRANSMEMBRANE PROTEIN TENB2; TOMOREGULIN; PUTATIVE TRANSMEMBRANE PROTEIN WITH EGF-LIKE AND TWO FOLLISTATIN-LIKE DOMAINS 2. |
| 30 | 362,363,478 & 479 | NM_004429 | EPHRIN-B1 PRECURSOR (EPH-RELATED RECEPTOR TYROSINE KINASE LIGAND 2) (LERK-2) (ELK LIGAND) (ELK-L). |
| 31 | 364, 365, 480 & 481 | NM_018950 | -HLA-F gene for human leukocyte antigen F |
| 32 | 366, 367, 482 & 483 | NM_000038 | ADENOMATOUS POLYPOSIS COLI PROTEIN (APC PROTEIN) |
| 33 | 368, 369, 484 & 485 | NM 000610 | CD44 ANTIGEN PRECURSOR (PHAGOCYTIC GLYCOPROTEIN I) (PGP-1) (HUTCH-I) (EXTRACELLULAR MATRIX RECEPTOR-111) (ECMR-III) (GP90 LYMPHOCYTE HOMING/ ADHESION RECEPTOR) (HERMES ANTIGEN) (HYALURONATE RECEPTOR) (HEPARAN SULFATE PROTEOGLYCAN) (EPICAN) (CDW44) |

(continued)

| Genomic SEQ ID NO: | Bisulfite SEQ ID Nos: | Genbank Ref. ID No:/contig | Description |
|---|---|---|---|
| 34 | 370,371,486 & 487 | NM_004385 | VERSICAN CORE PROTEIN PRECURSOR (LARGE FIBROBLAST PROTEOGLYCAN) (CHONDROITIN SULFATE PROTEOGLYCAN CORE PROTEIN 2) (PG-M) (GLIAL HYALURONATE-BINDING PROTEIN) (GHAP) CSPG2 |
| 35 | 372, 373, 488 & 489 | | EYA 4 EnsEMBL ID ENSG00000112319 |
| 36 | 374, 375, 490 & 491 | NM_000455 | SERINE/THREONINE-PROTEIN KINASE 11 (EC 2.7.1.-) SERINE/ THREONINE- PROTEIN KINASE LKB1) |
| 37 | 376,377,492 & 493 | NM_003242 | Transforming growth factor, beta receptor II (TGFBR2) |
| 38 | 378,379,494 & 495 | NM_001753 | CAVEOLIN-1; CAV 1 |
| 39 | 380,381,496 & 497 | NM_001257 | CADHERIN-13 PRECURSOR (TRUNCATED-CADHERIN) (T-ADHERING (T-CAD) (HEART-CADHERIN) (H-CADHERIN) (P105) |
| 40 | 382, 383, 498 & 499 | NM_000044 | ANDROGEN RECEPTOR (DIHYDROTESTOSTERONE RECEPTOR) AR |
| 41 | 384, 385, 500 & 501 | NM_032546 | RING FINGER PROTEIN 30, RNF 30, |
| 42 | 386, 387, 502 & 503 | NM_033178 | DOUBLE HOMEOBOX, 4; DOUBLE HOMEOBOX PROTEIN 4. DUX4 |
| 43 | 388, 389, 504 & 505 | NM_003573 | LATENT TRANSFORMING GROWTH FACTOR BETA BINDING PROTEIN 4.; LTBP4 |
| 44 | 390, 391, 506 & 507 | | CGI-20 PROTEIN |
| 45 | 392, 393, 508 & 509 | NM_014459 | PROTOCADHERIN 17; PROTOCADHERIN 68.; PCDH 17 |
| 46 | 394,395,510 & 511 | NM_005285 | PROBABLY G PROTEIN-COUPLED RECEPTOR GPR7.; GPR7 |
| 47 | 396, 397, 512 & 513 | NM_016269 | LYMPHOID ENHANCER BINDING FACTOR 1 (LEF-1) (T CELL-SPECIFIC TRANSCRIPTION FACTOR 1-ALPHA) (TCF1-ALPHA) |
| 48 | 398, 399, 514 & 515 | AC 139426.2.1.18 8448 | Situated between EnsEMBL ID ENSESTG00003302072 and EnsEMBL ID ENSESTG00003302068 |

(continued)

| Genomic SEQ ID NO: | Bisulfite SEQ ID Nos: | Genbank Ref. ID No:/contig | Description |
|---|---|---|---|
| 49 | 400, 401, 516 & 517 | NM_005904 | MOTHERS AGAINST DECAPENTAPLEGIC HOMOLOG 7 (SMAD 7) (MOTHERS AGAINST DPP HOMOLOG 7) (SMAD7) (HSMAD7). |
| 50 | 402, 403, 518 & 519 | AL512590.2.1.165 432 | sapiens mRNA for KIAA1529 protein EnsEMBL ID ENSG00000029402 |
| 51 | 404, 405, 520 & 521 | NM_001866 | COX7B |
| 52 | 406,407,522 & 523 | NM_001900 | CST5 |
| 53 | 408, 409, 524 & 525 | NM_001990 | EYES ABSENT HOMOLOG 3. EYA3 |
| 54 | 410,411,526 & 527 | NM_002032 | FERRITIN HEAVY CHAIN (FERRITIN H SUBUNIT). FTH 1. |
| 55 | 412,413,528 & 529 | NM_002938 | RING FINGER PROTEIN 4.; RNF4 |
| 56 | 414, 415, 530 & 531 | | HPP1 EnsEMBL ID ENSG00000144339 |
| 57 | 416, 417, 532 & 533 | NM_000852 | (LUTATHIONE S-TRANSFERASE P EC 2.5.1.18) (GST CLASS-PI) (GSTP1-1) |
| 58 | 418, 419, 534 & 535 | NM_007084 | TRANSCRIPTION FACTOR SOX-21 |
| 59 | 55, 66, 77 & 78 | NM_003299 | |
| 50 | 67, 68, 79 & 80 | NM_025218 | |
| 61 | 59, 70, 81 & 82 | NM_001374 | |
| 52 | 71, 72, 83 & 84 | NM_003714 | |
| 53 | 73, 74, 85 & 86 | NM_002196 | |
| 64 | 75, 76, 87 & 88 | NM_000484 | |

## Claims

**1.** A method for detecting, or for detecting and distinguishing between or among colon cell proliferative disorders in a subject comprising:

i) contacting genomic DNA isolated from blood plasma, blood serum, whole blood, isolated blood cells, cells isolated from the blood obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) detecting, or detecting and distinguishing between or among colon cell proliferative disorders afforded with a sensitivity of greater than or equal to 80% and a specificity of greater than or equal to 80,

wherein the target region comprises, or hybridizes under stringent conditions to at least 16, contiguous nucleotides of the gene or a sequence of ONC2.

**2.** The method of claim 1, wherein at least one of colorectal carcinoma tissue, or colon adenomas is distinguished from at least one tissue selected from the group consisting of inflammatory colon tissue, normal colon tissue, non-colon healthy tissue, peripheral blood lymphocytes, and non-colon cancer tissue, and wherein the target region comprises, or hybridizes under stringent conditions to at least 16 contiguous nucleotides of the sequence SEQ ID NO: 6, and complements thereof.

3. A method for detecting, or for detecting and distinguishing between or among colon cell proliferative disorders in a subject comprising determining the expression level of the gene or sequence of ONC2, in a biological sample isolated from said subject,
wherein said expression level is determined by detecting the presence, absence or level ofmRNA transcribed from said gene or sequence, or wherein said expression level is determined by detecting the presence, absence or level of a polypeptide encoded by said gene or sequence, or wherein said expression is determined by detecting the presence or absence of CpG methylation within said gene or sequence, wherein hypermethylation indicates the presence of a colon cell proliferative disorder.

4. A method for detecting, or for detecting and distinguishing between or among colon cell proliferative disorders in a subject, comprising contacting genomic DNA isolated from a biological sample obtained from the subject with at least one reagent, or series of reagents that distinguishes between methylated and non-methylated CpG dinucleotides within at least one target region of the genomic DNA, wherein the target region comprises, or hybridizes under stringent conditions to a sequence of at least 16, contiguous nucleotides of the gene or a sequence of ONC2, wherein said contiguous nucleotides comprise at least one CpG dinucleotide sequence.

5. A method according to claim 4, wherein the target region comprises, or hybridizes under stringent conditions to a sequence of at least 16, contiguous nucleotides of at least one gene or sequence selected from the group consisting of SEQ ID NOS: 1-3, 14, 16, 21, 22, 23, 44-46, 48, 50, 59-64, 36, COX7B, CST5, EYA3, FTH1, RNF4, SOX 21, RNF4, ALX 4, NGFR, DLX-5, HVIAAT, BTF3 Homolog 1, ADCY9, BCL-5, BCOR, TAFII28, HOMEOBOX PROTEIN SIX6, GSK-3 BETA, ELK-L, HLA-F, TGFBR2, AR, RNF30, DUX4, SMAD7, GTMBP, EYA4, TCF1-ALPHA, TPEF, P73, APC, CD44, CSPG2, CAV 1, H-cadherin and LTBP4, respectively, wherein said contiguous nucleotides comprise at least one CpG dinucleotide sequence.

6. A method according to any of claims 3 to 5, comprising:

a) extracting or otherwise isolating genomic DNA from a biological sample obtained from the subject
b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
c) contacting the treated genomic DNA, or the treated fragment thereof, with an amplification enzyme and at least two primers comprising, in each case a contiguous sequence of at least 9 nucleotides that is complementary to, or hybridizes under moderately stringent or stringent conditions to a sequence selected from the group consisting of SEQ ID NOs: 314, 315, 430, 431, and complements thereof, wherein the treated genomic DNA or the fragment thereof is either amplified to produce at least one amplificate, or is not amplified; and
d) determining, based on a presence or absence of, or on a property of said amplificate, the methylation state of at least one CpG dinucleotide of the sequence SEQ ID NO: 6, or an average, or a value reflecting an average methylation state of a plurality of CpG dinucleotides of the sequence of SEQ ID NO: 6.

7. The method of claim 6, wherein treating the genomic DNA, or the fragment thereof in b), comprises use of a reagent selected from the group consisting of bisulfite, hydrogen sulfite, disulfite, and combinations thereof.

8. The method of claim 6, further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NOs: 314, 315, 430, 431, and complements thereof, wherein said nucleic acid molecule or peptide nucleic acid molecule suppresses amplification of the nucleic acid to which it is hybridized.

9. The method of claim 6, wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NOs: 314, 315, 430, 431, and complements thereof.

10. A method for detecting, or for detecting and distinguishing between or among colon cell proliferative disorders in a subject, comprising:

a) providing, from a subject, a biological sample having subject genomic DNA;
b) extracting, or otherwise isolating the genomic DNA;

c) contacting the genomic DNA of b), or a fragment thereof, comprising at least 16, contiguous nucleotides of a sequence of SEQ ID NO: 6, and sequences that hybridize under stringent conditions thereto, with one or more methylation-sensitive restriction enzymes, wherein the genomic DNA is, with respect to each cleavage recognition motif thereof, either cleaved thereby to produce cleavage fragments, or not cleaved thereby; and
d) determining, based on a presence or absence of, or on property of at least one such cleavage fragment, the methylation state of at least one CpG dinucleotide of the sequence of SEQ ID NO: 6, or an average, or a value reflecting an average methylation state of a plurality of CpG dinucleotides of the sequence of SEQ ID NO: 6.

**11.** A treated nucleic acid derived from genomic SEQ ID NO: 6, wherein the treatment is suitable to convert at least one unmethylated cytosine base of the genomic DNA sequence to uracil or another base that is detectably dissimilar to cytosine in terms of hybridization.

**12.** A nucleic acid, comprising at least 16, contiguous nucleotides of a treated genomic DNA sequence selected from the group consisting of SEQ ID NOs: 314, 315, 430, 431, and sequences complementary thereto, wherein the treatment is suitable to convert at least one unmethylated cytosine base of the genomic DNA sequence to uracil or another base that is detectably dissimilar to cytosine in terms of hybridization.

**13.** An oligomer, comprising a sequence of at least 9 contiguous nucleotides that is complementary to, or hybridizes under moderately stringent or stringent conditions to a treated genomic DNA sequence selected from the group consisting of SEQ ID NOs: 314, 315, 430, 431.

**14.** The nucleic acid of claims 11 to 12 or the oligomer of claim 13, wherein the contiguous base sequence comprises at least one CpG, TpG or CpA dinucleotide sequence.

**15.** Use of a method according to any of claims 1 to 10, a nucleic acid according to any of claims 11 to 14 for the detection and/or distinguishing between colon cell proliferative disorders.

**16.** A kit useful for detecting, or for detecting and differentiating between or among colon cell proliferative disorders of a subject, comprising:

- a methylation-sensitive restriction enzyme; and
- at least one nucleic acid molecule or peptide nucleic acid molecule, comprising a contiguous sequence of at least 16, nucleotides that is complementary to, or hybridizes under moderately stringent or stringent conditions to the sequence of SEQ ID NO: 6, and complements thereof.

**17.** A kit useful for detecting, or for detecting and differentiating between or among colon cell proliferative disorders of a subject, comprising:

- a bisulfite reagent; and
- at least one nucleic acid molecule or peptide nucleic acid molecule, comprising a contiguous sequence of at least 16, nucleotides that is complementary to, or hybridizes under moderately stringent or stringent conditions to the sequence selected from the group consisting of SEQ ID NOs: 314, 315, 430, 431, and complements thereof.

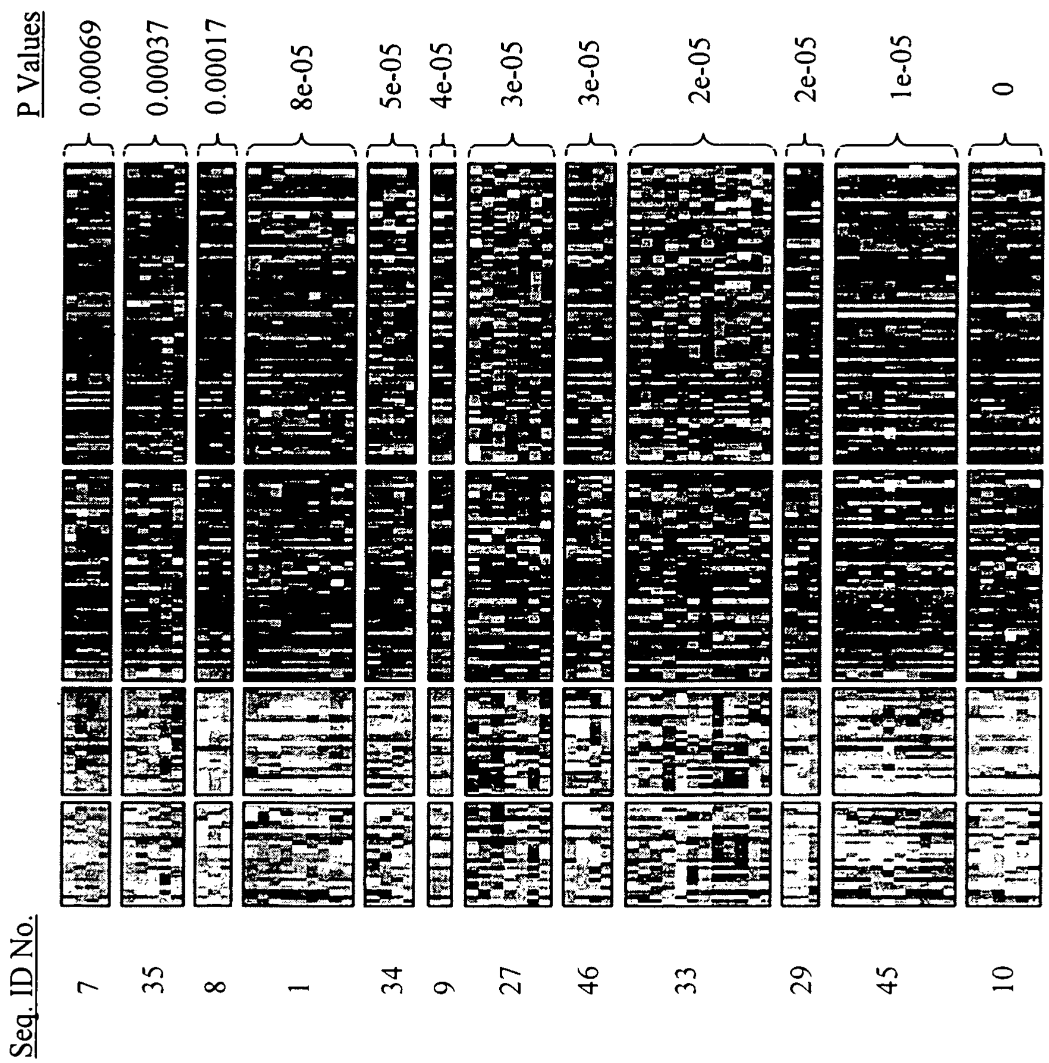

P Values
0.00069
0.00037
0.00017
8e-05
5e-05
4e-05
3e-05
3e-05
2e-05
2e-05
1e-05
0
Seq. ID No.
7
35
8
1
34
9
27
46
33
29
45
10


Figure 1

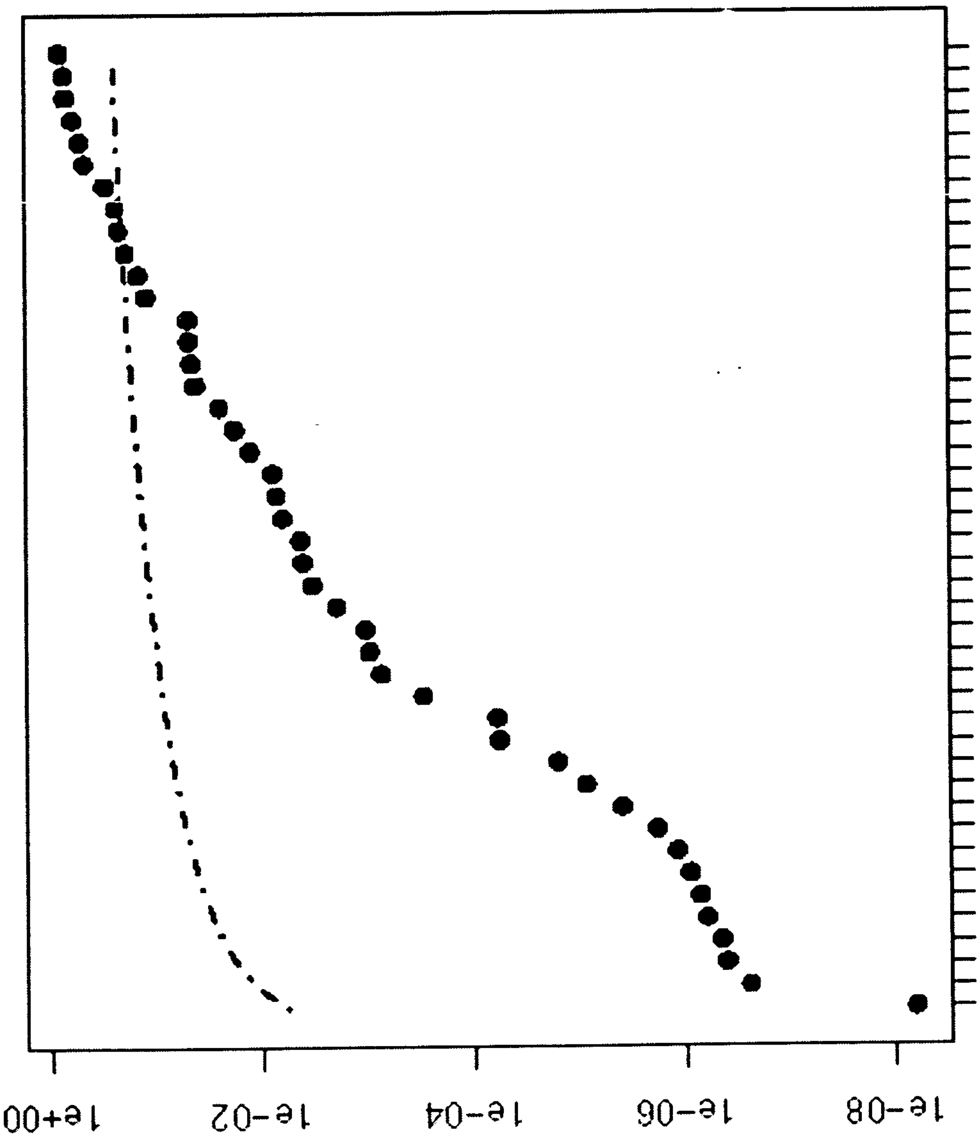


Figure 2

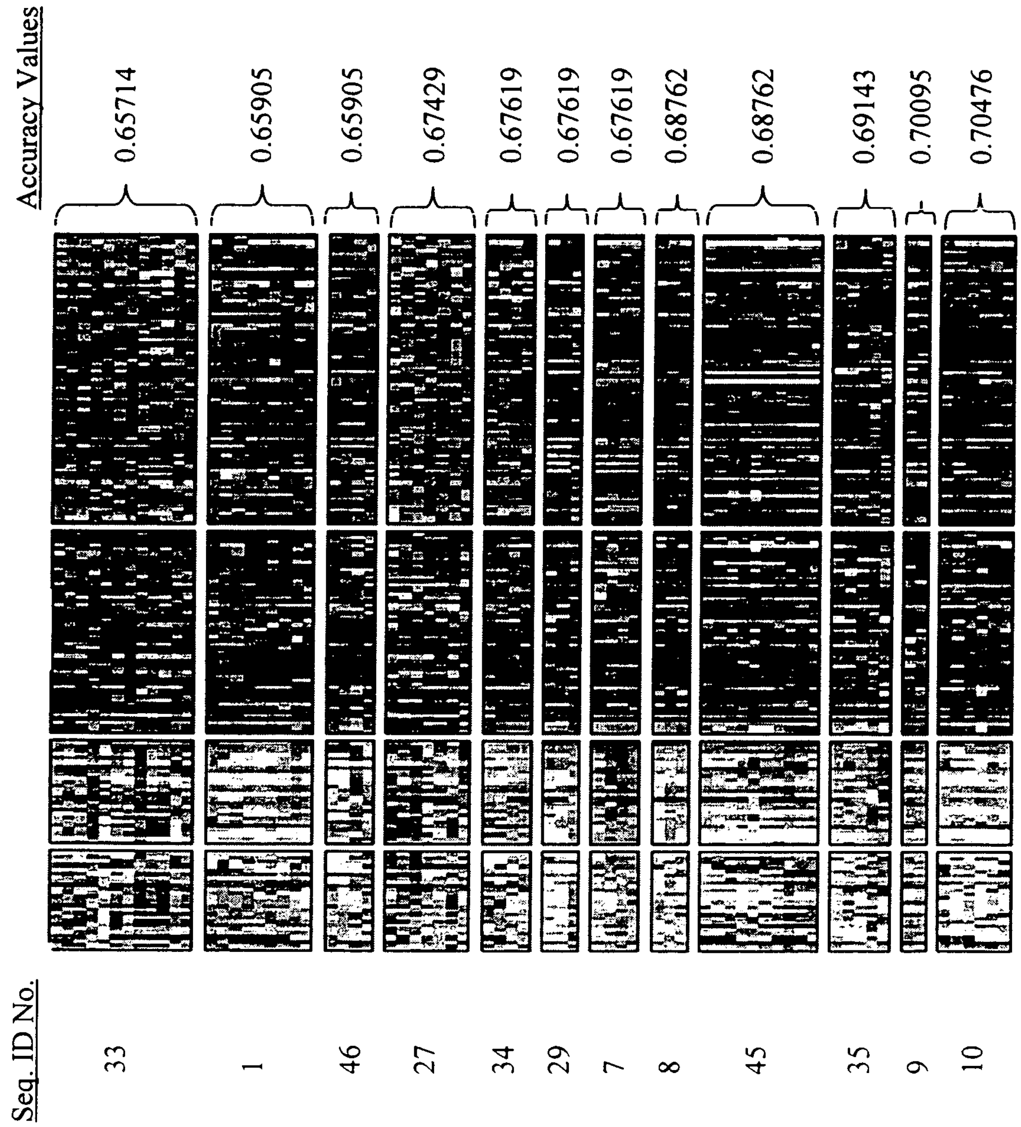
Seq. ID No.
33
1
46
27
34
29
7
8
45
35
9
10
Accuracy Values
0.65714
0.65905
0.65905
0.67429
0.67619
0.67619
0.67619
0.68762
0.68762
0.69143
0.70095
0.70476

Figure 3

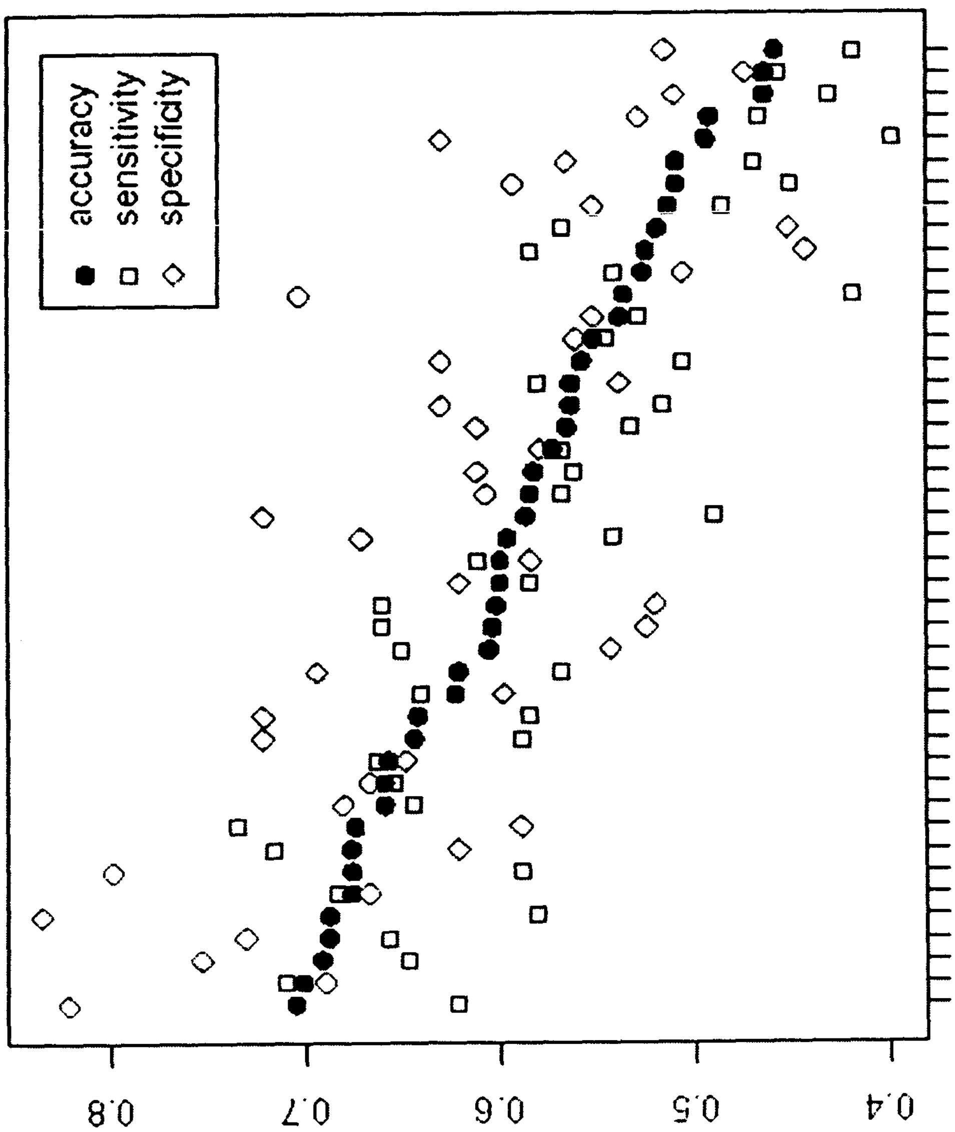


Figure 4

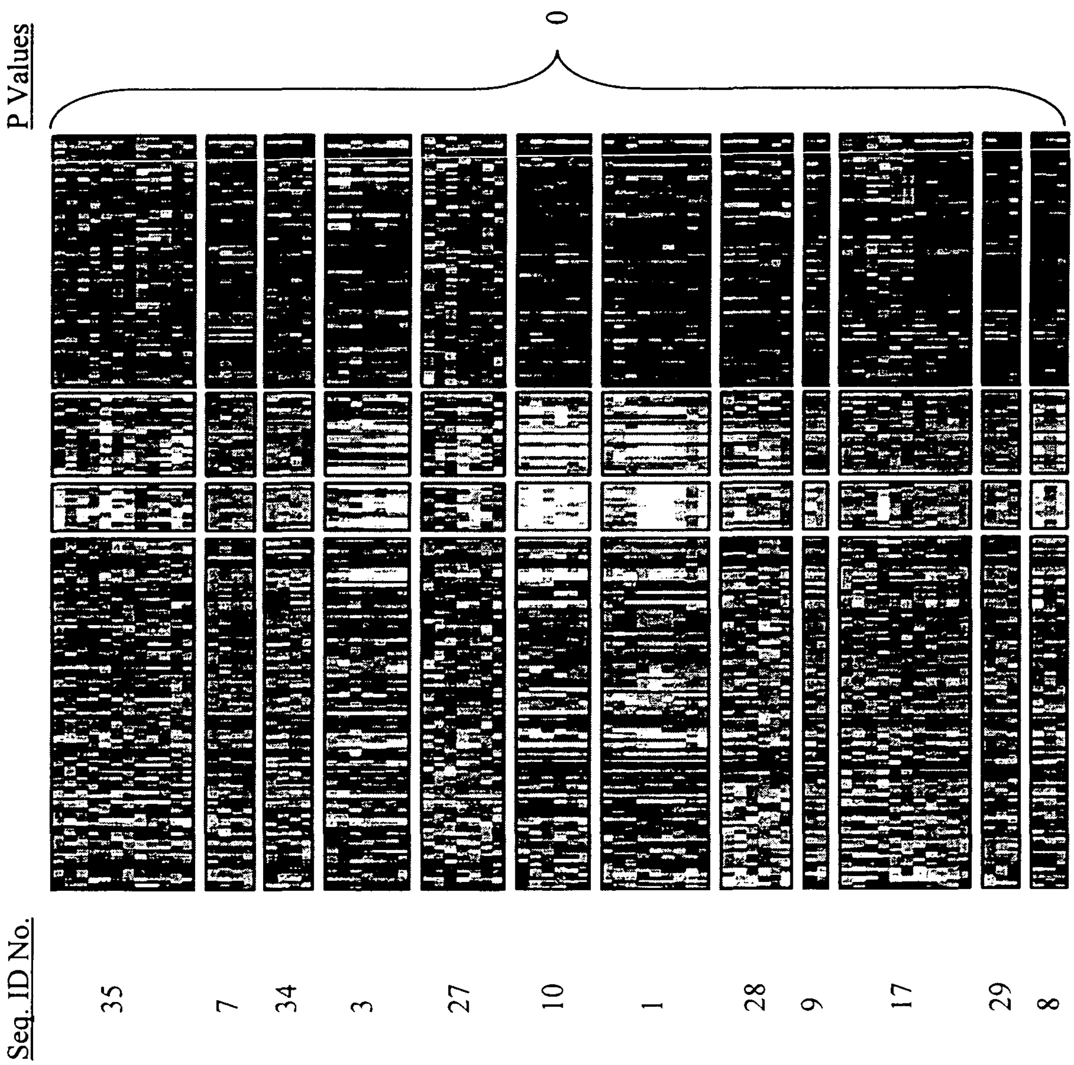


Figure 5

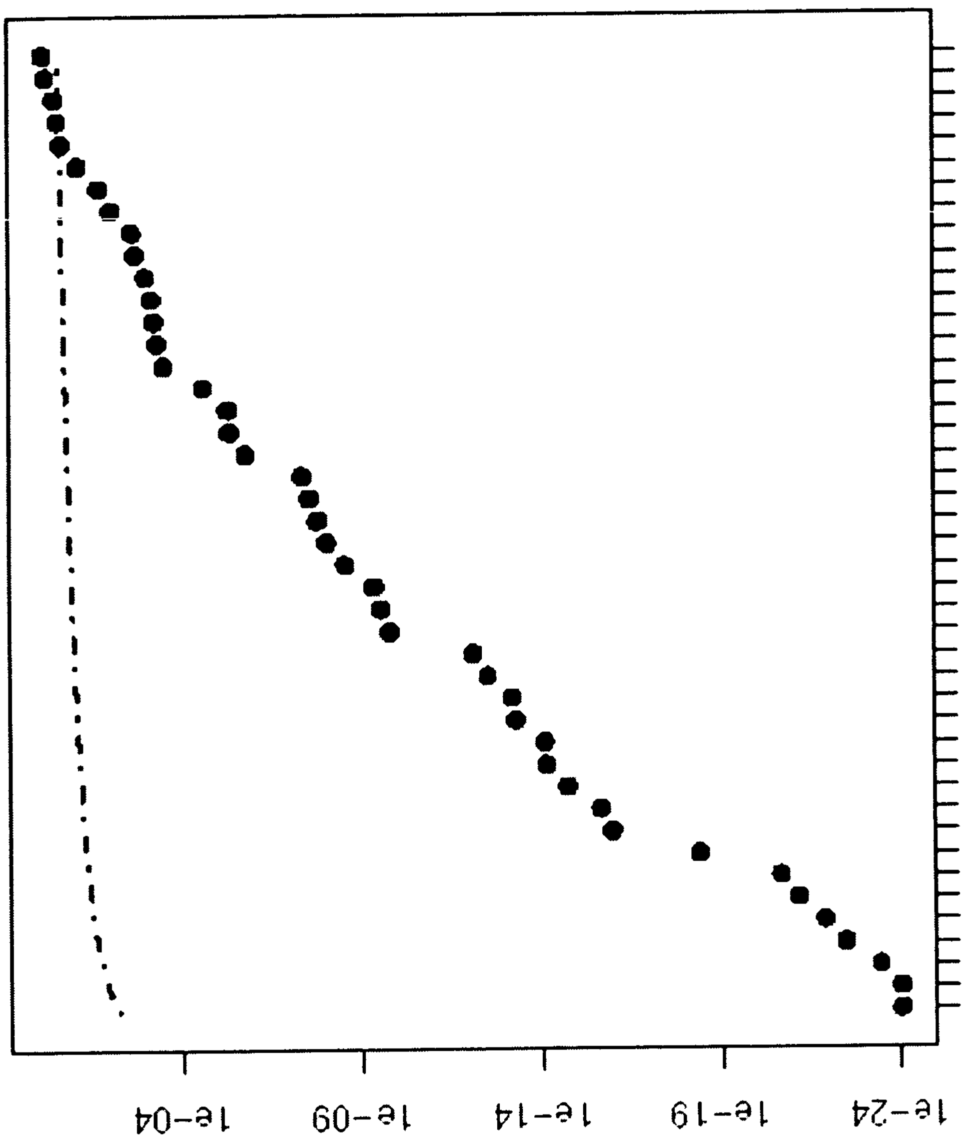


Figure 6

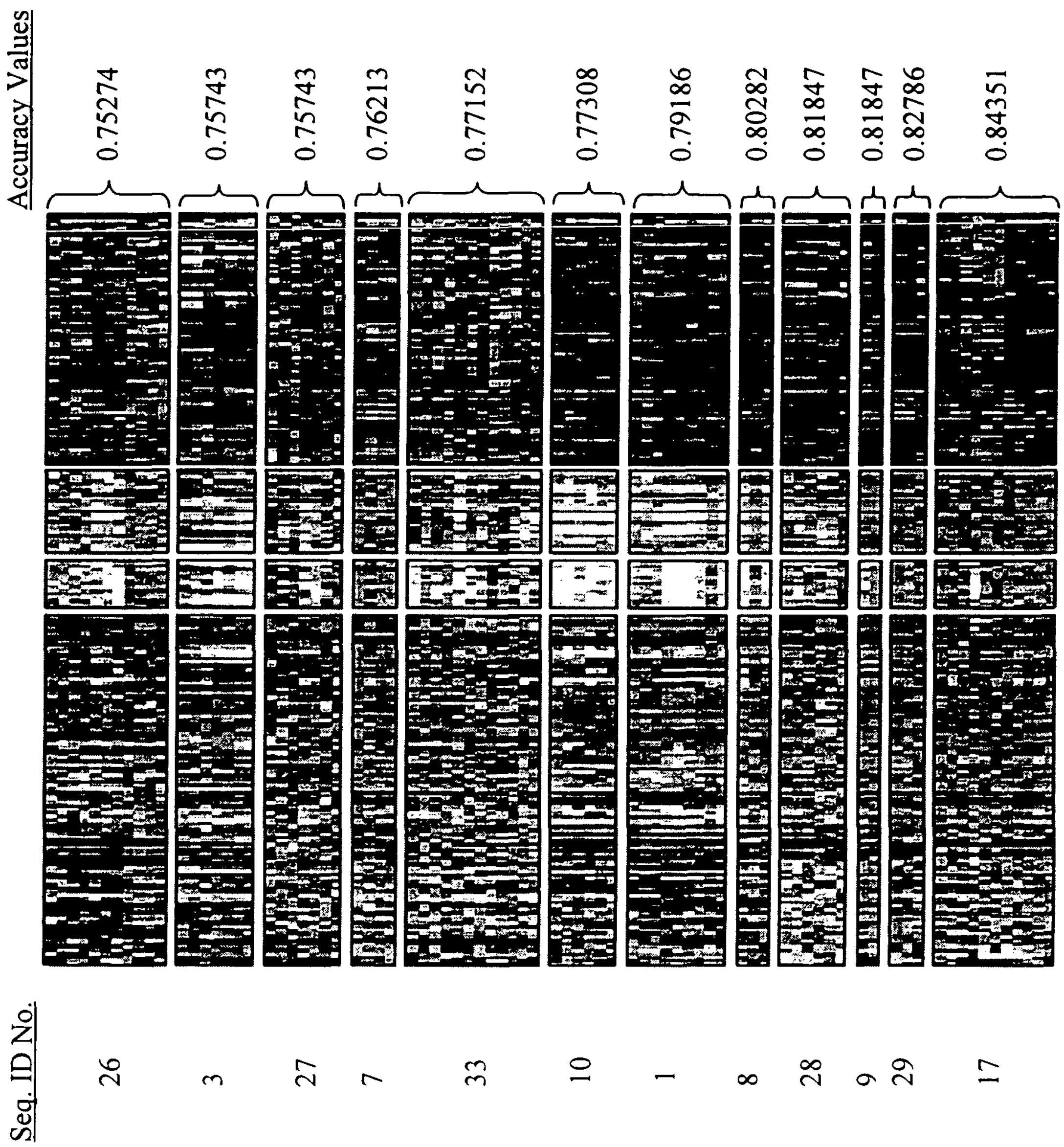
Accuracy Values
0.75274
0.75743
0.75743
0.76213
0.77152
0.77308
0.79186
0.80282
0.81847
0.81847
0.82786
0.84351
Seq. ID No.
26
3
27
7
33
10
1
8
28
9
29
17

Figure 7

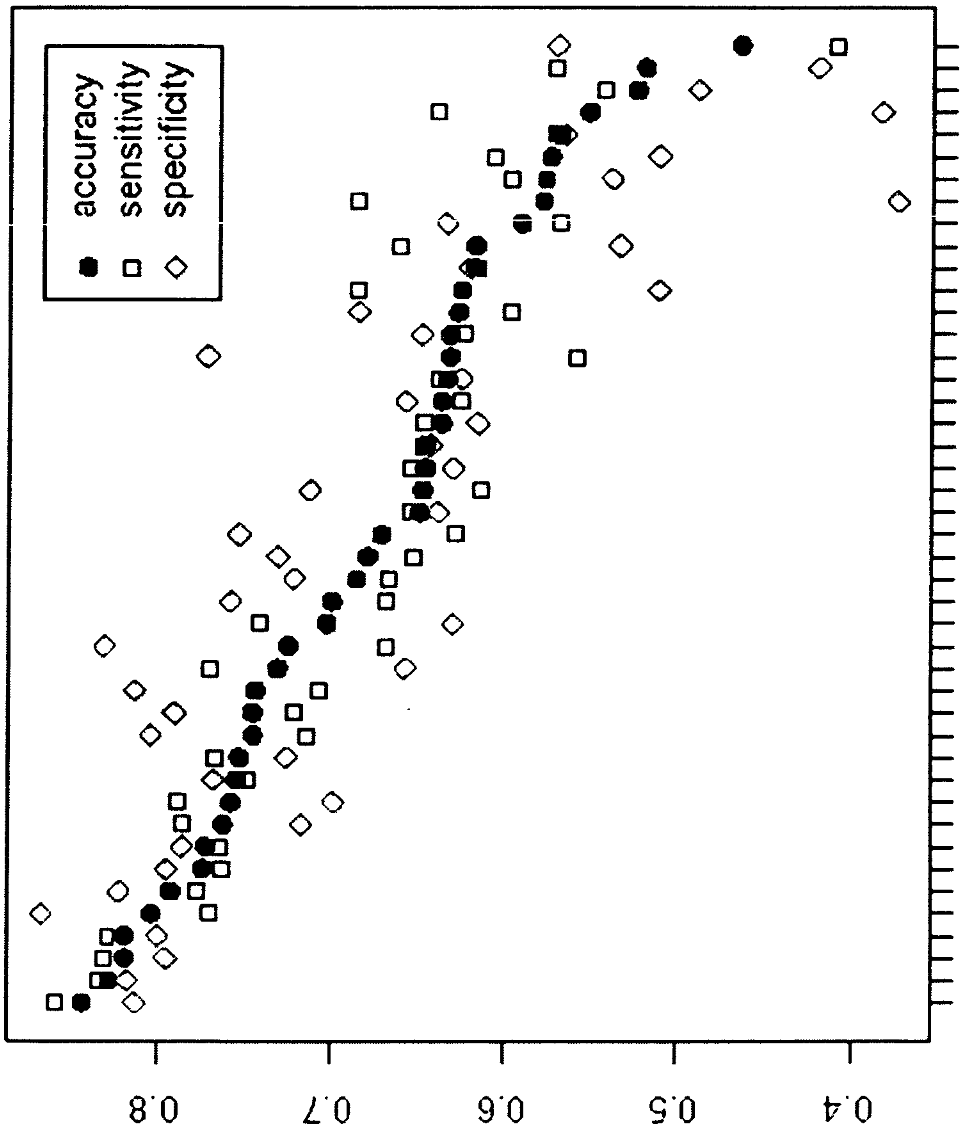


Figure 8

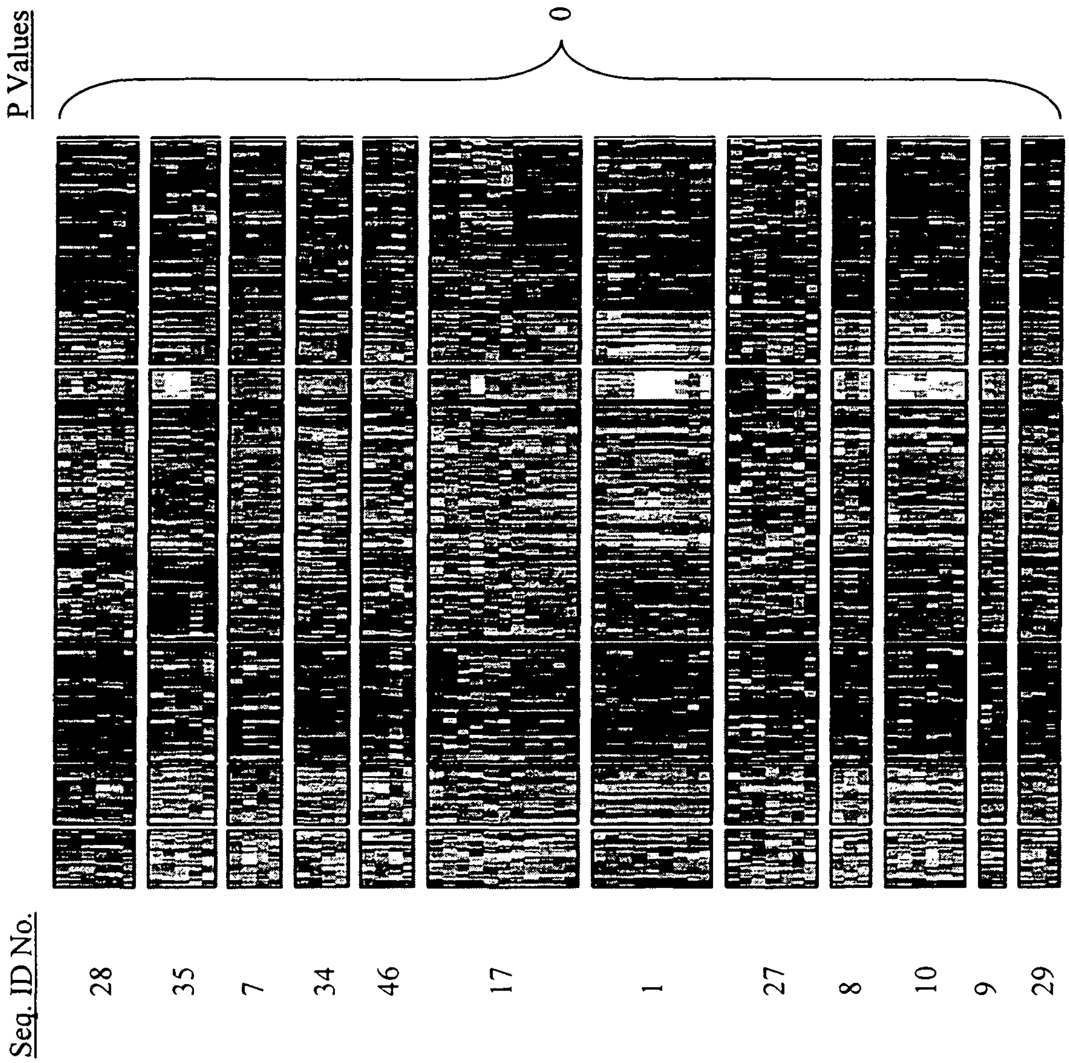


Figure 9

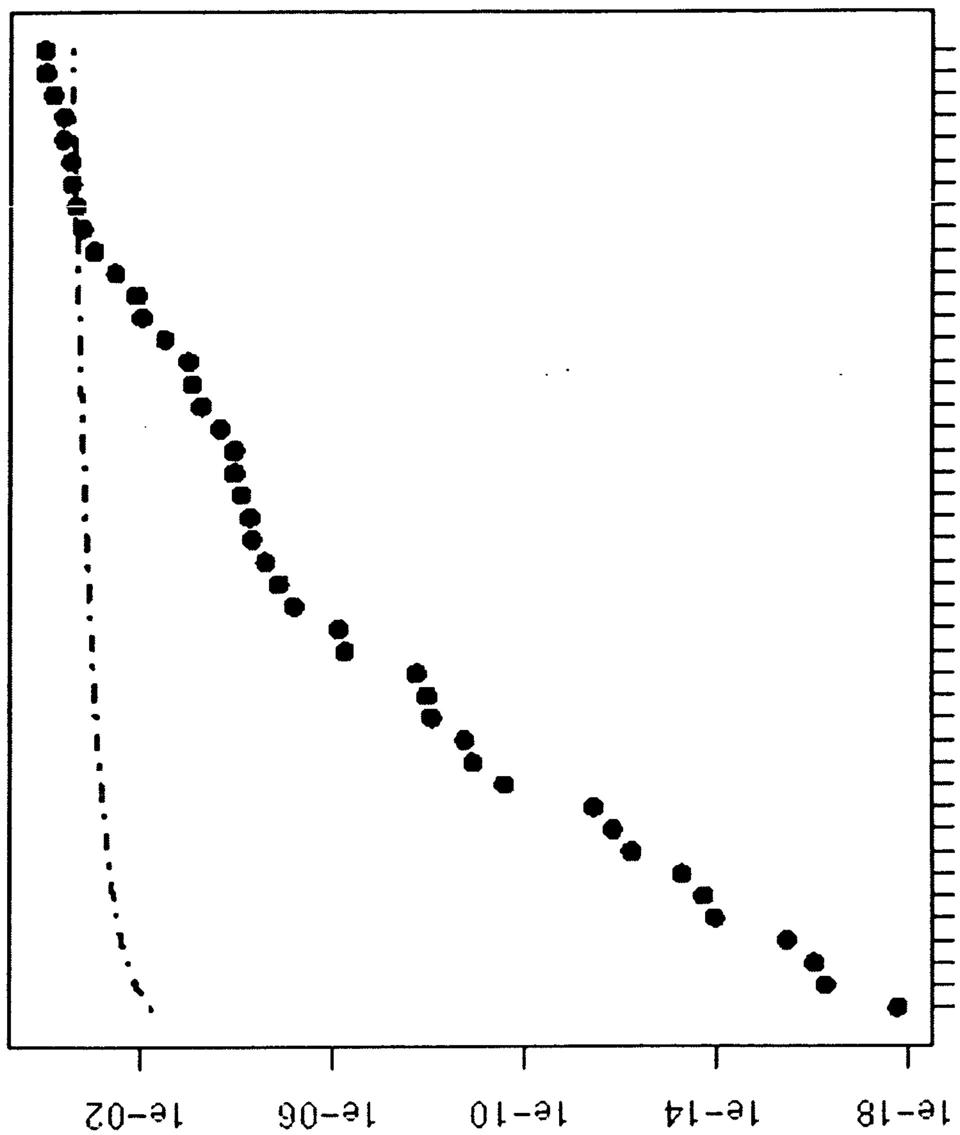


Figure 10

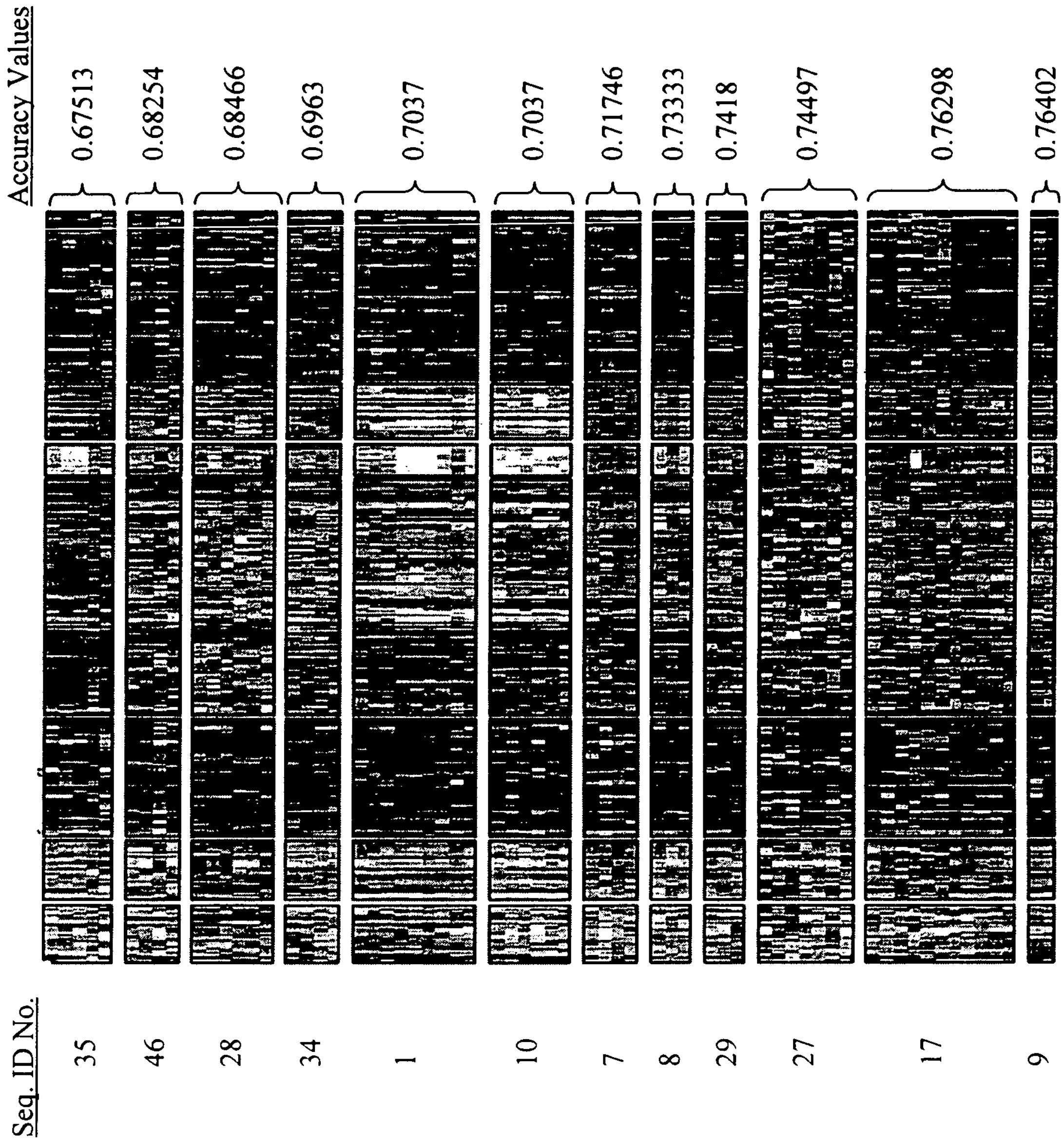
Accuracy Values
Seq. ID No.
35 0.67513
46 0.68254
28 0.68466
34 0.6963
1 0.7037
10 0.7037
7 0.71746
8 0.73333
29 0.7418
27 0.74497
17 0.76298
9 0.76402

Figure 11

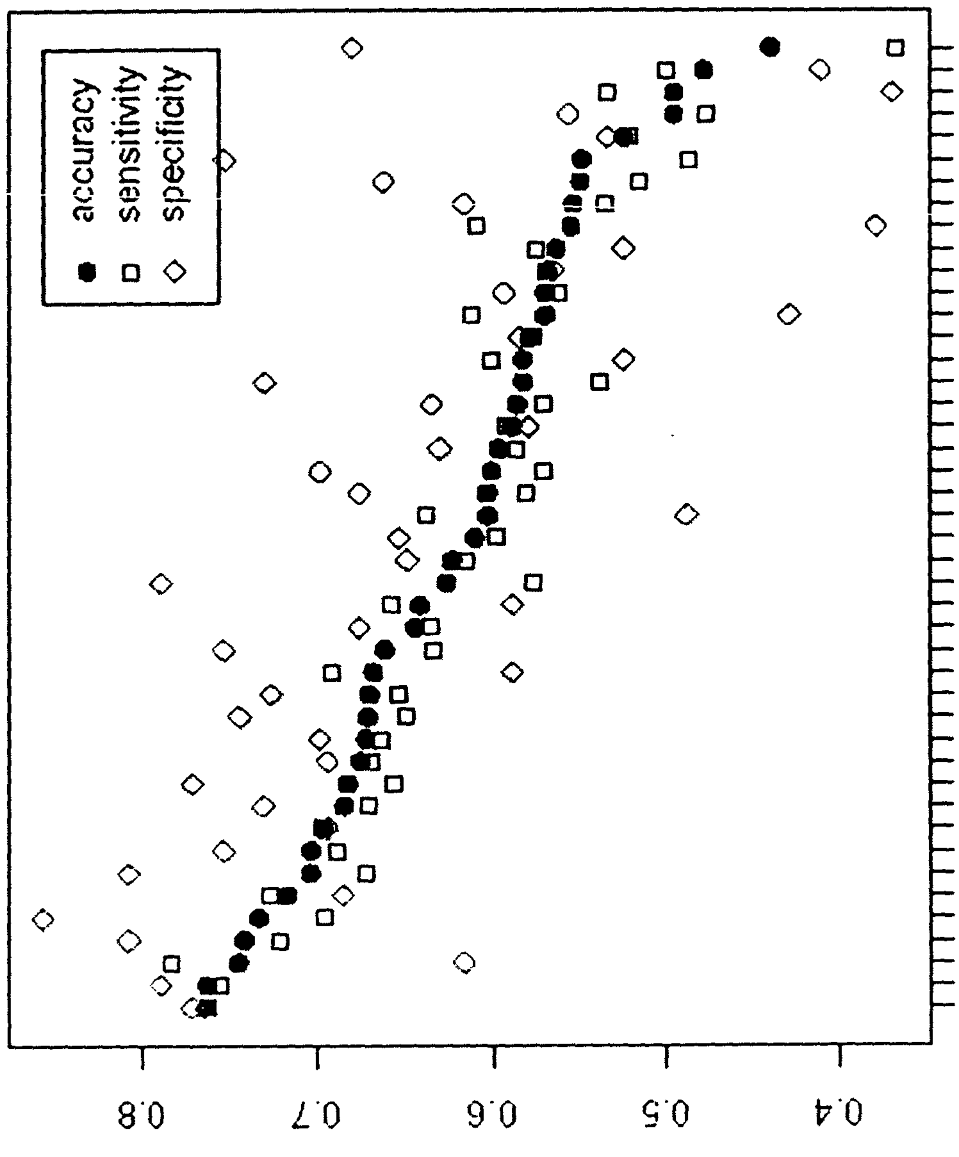


Figure 12

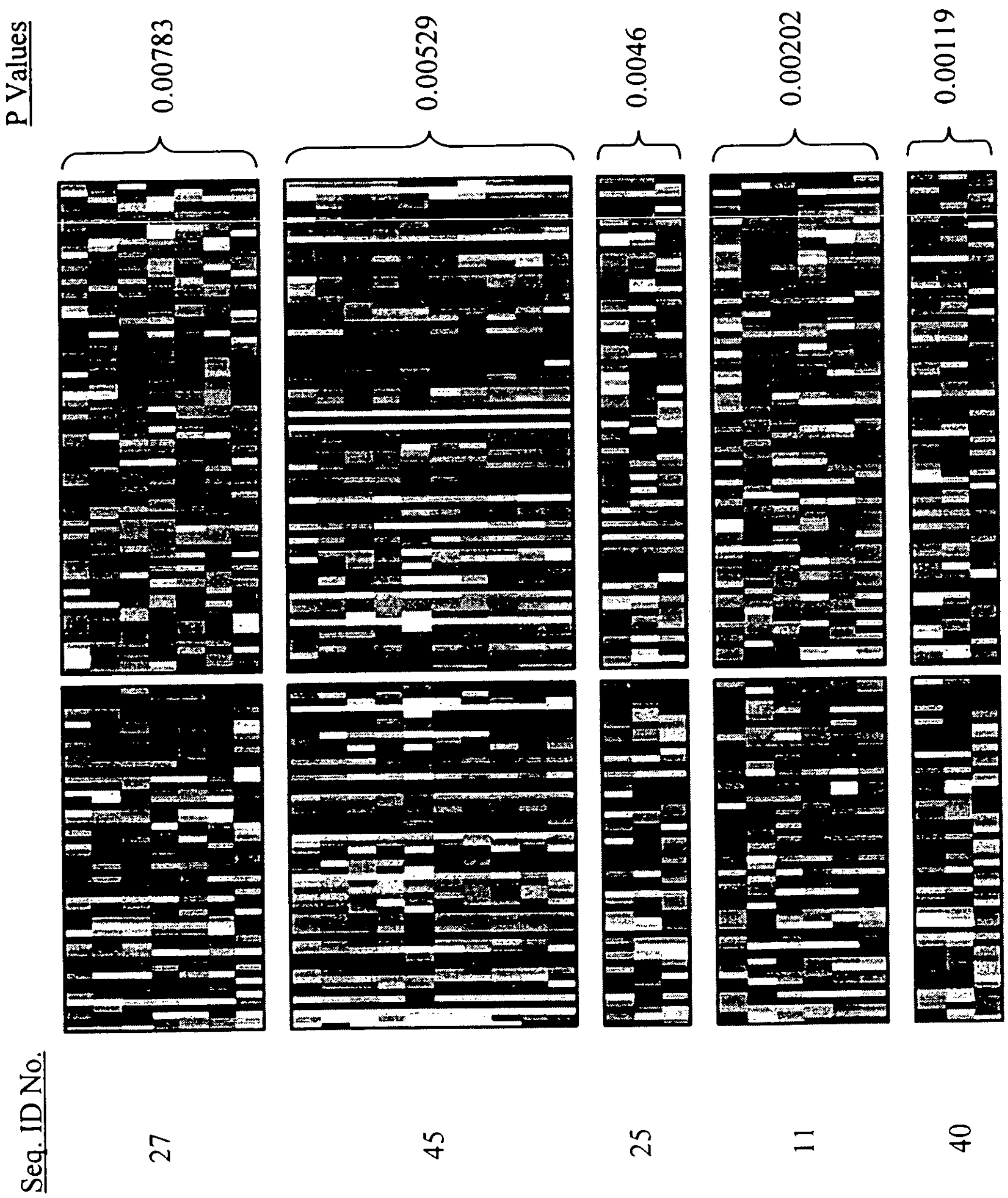
P Values
0.00783
0.00529
0.0046
0.00202
0.00119
Seq. ID No.
27
45
25
11
40

Figure 13

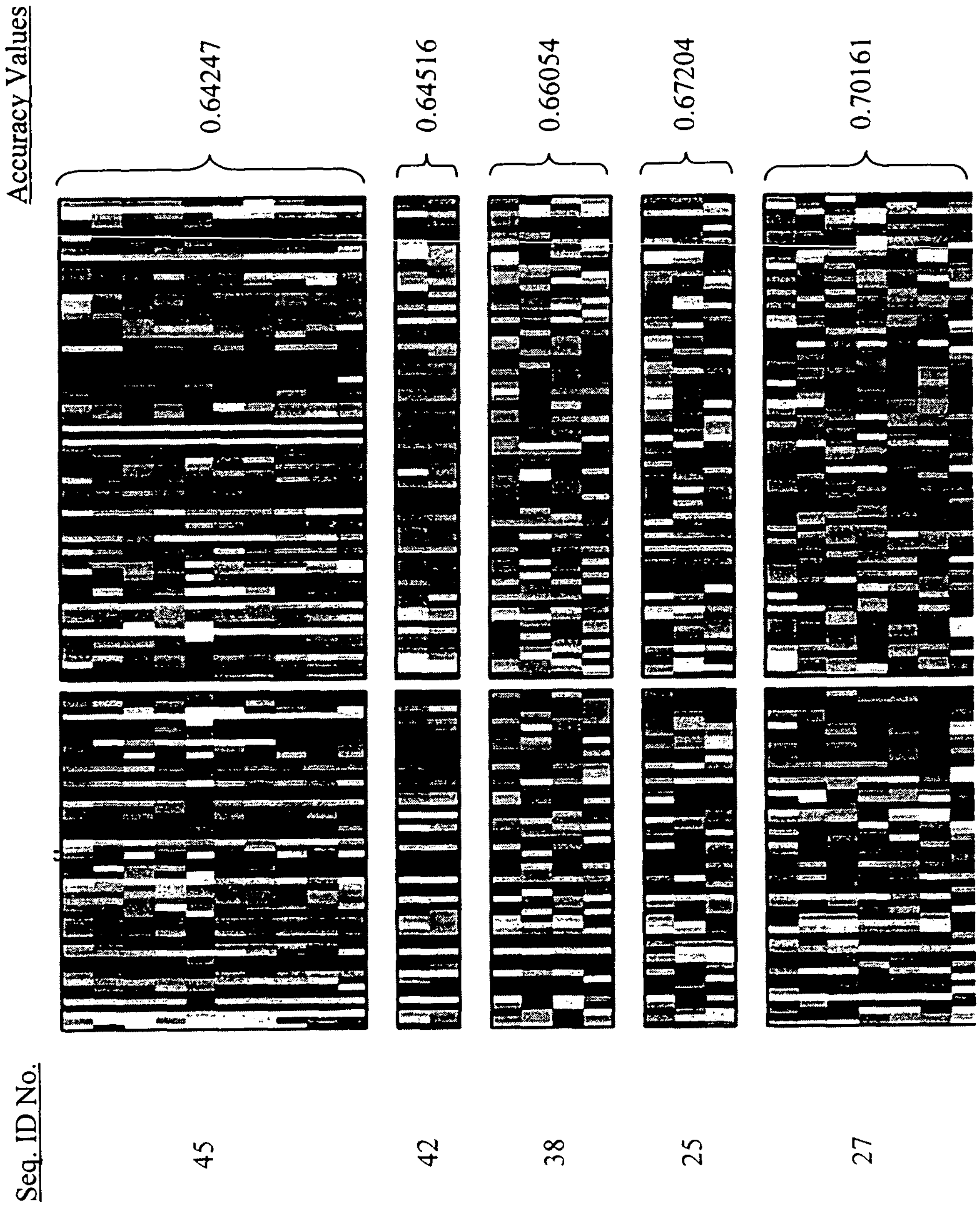
Accuracy Values
0.64247
0.64516
0.66054
0.67204
0.70161
Seq. ID No.
45
42
38
25
27

Figure 14

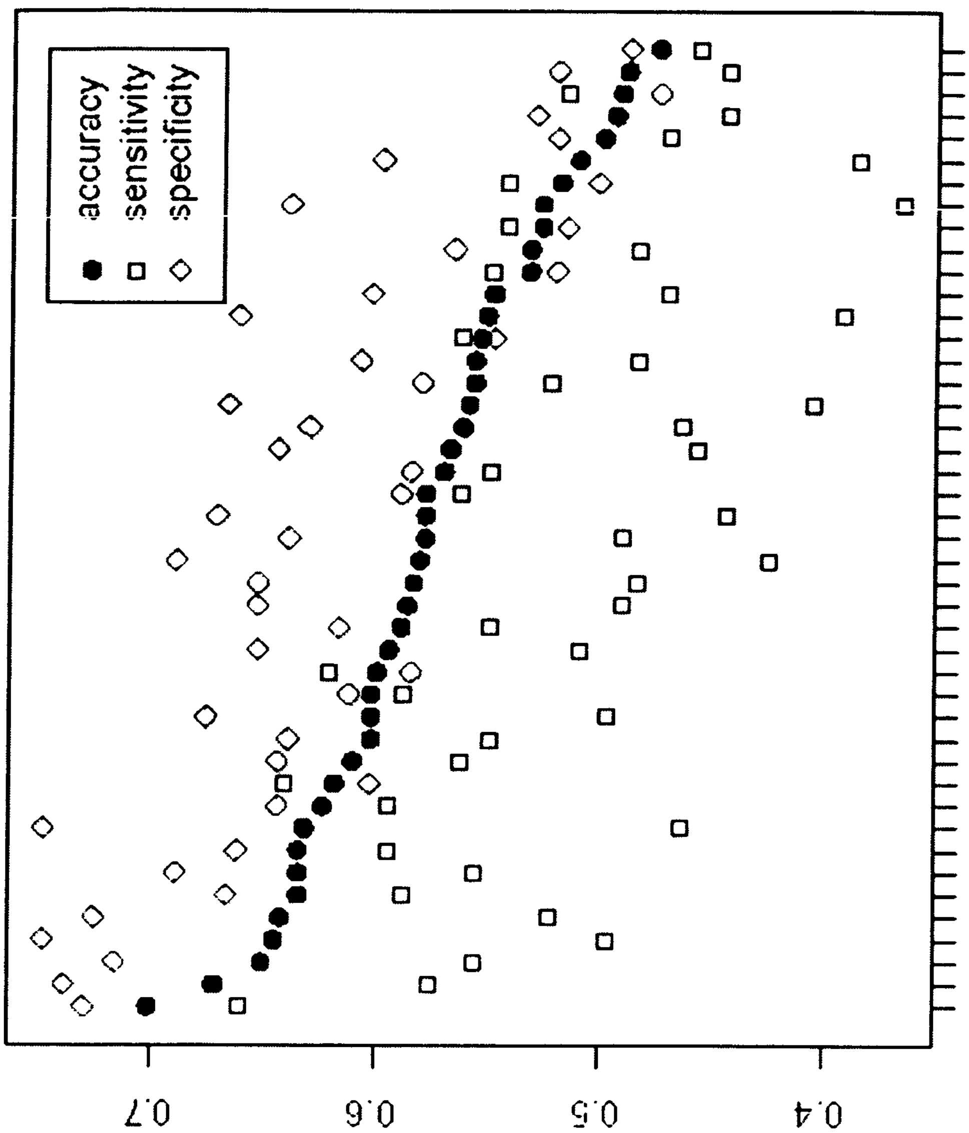


Figure 15

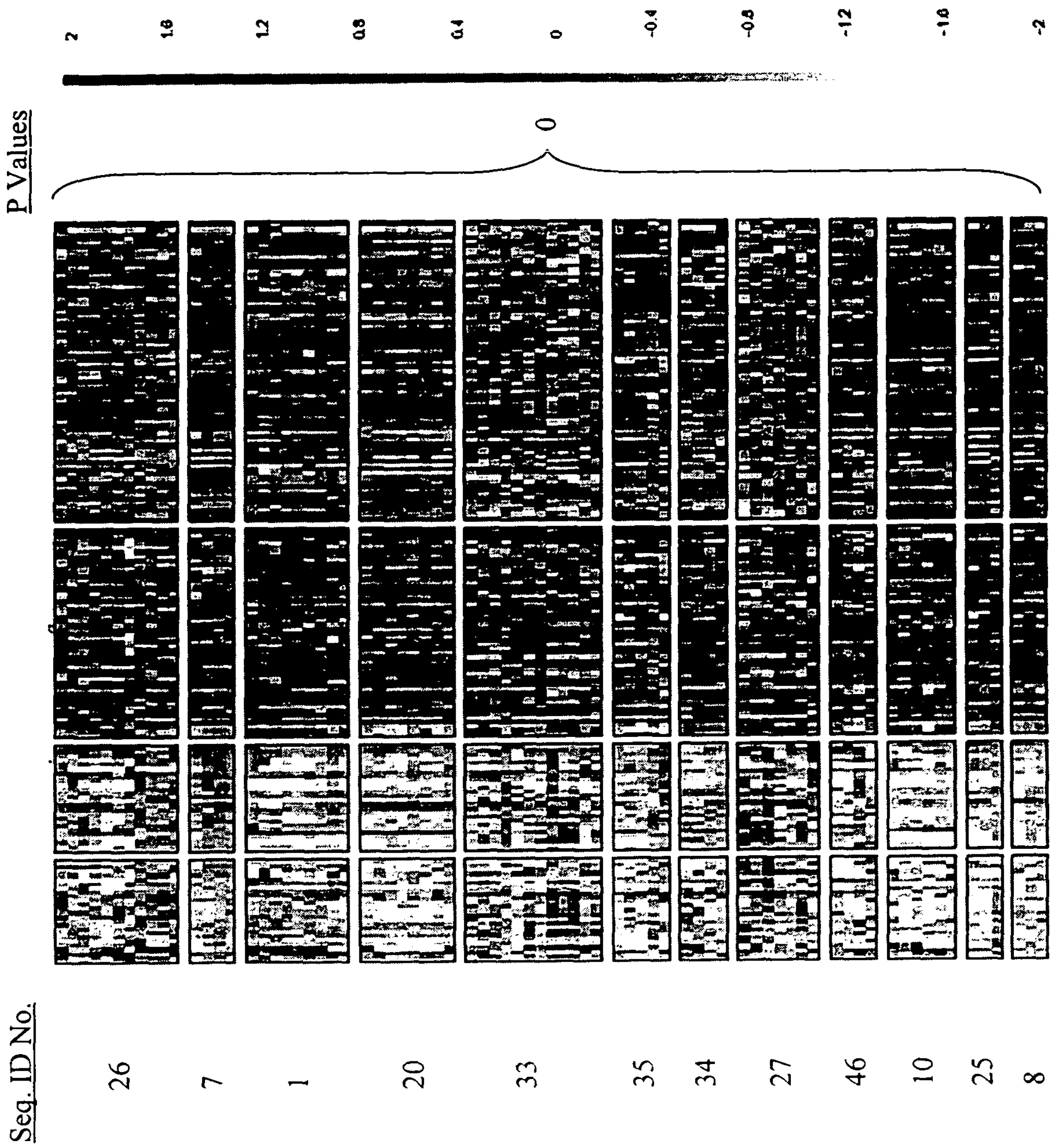
P Values
0
2
1.6
1.2
0.8
0.4
0
-0.4
-0.8
-1.2
-1.6
-2
Seg. ID No.
26
7
1
20
33
35
34
27
46
10
25
8

Figure 16

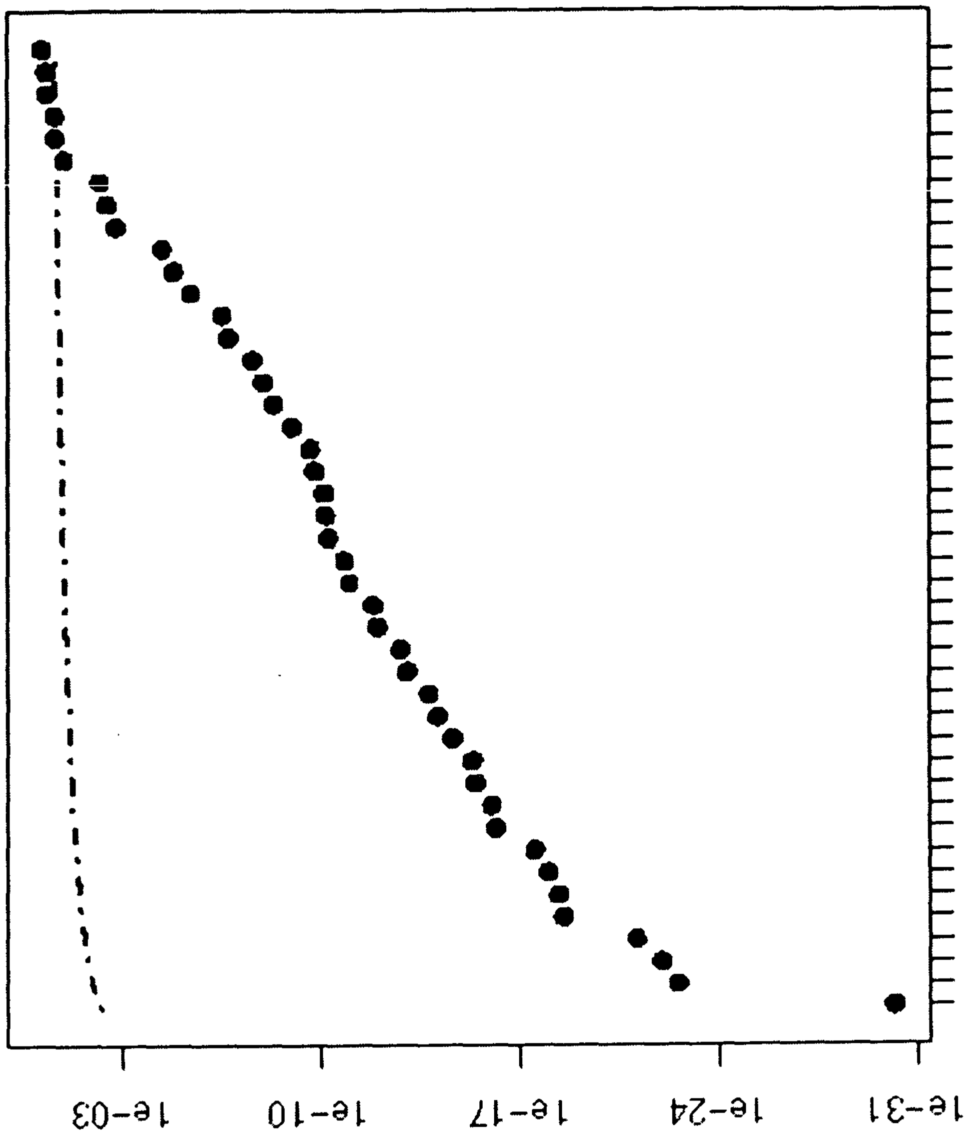


Figure 17

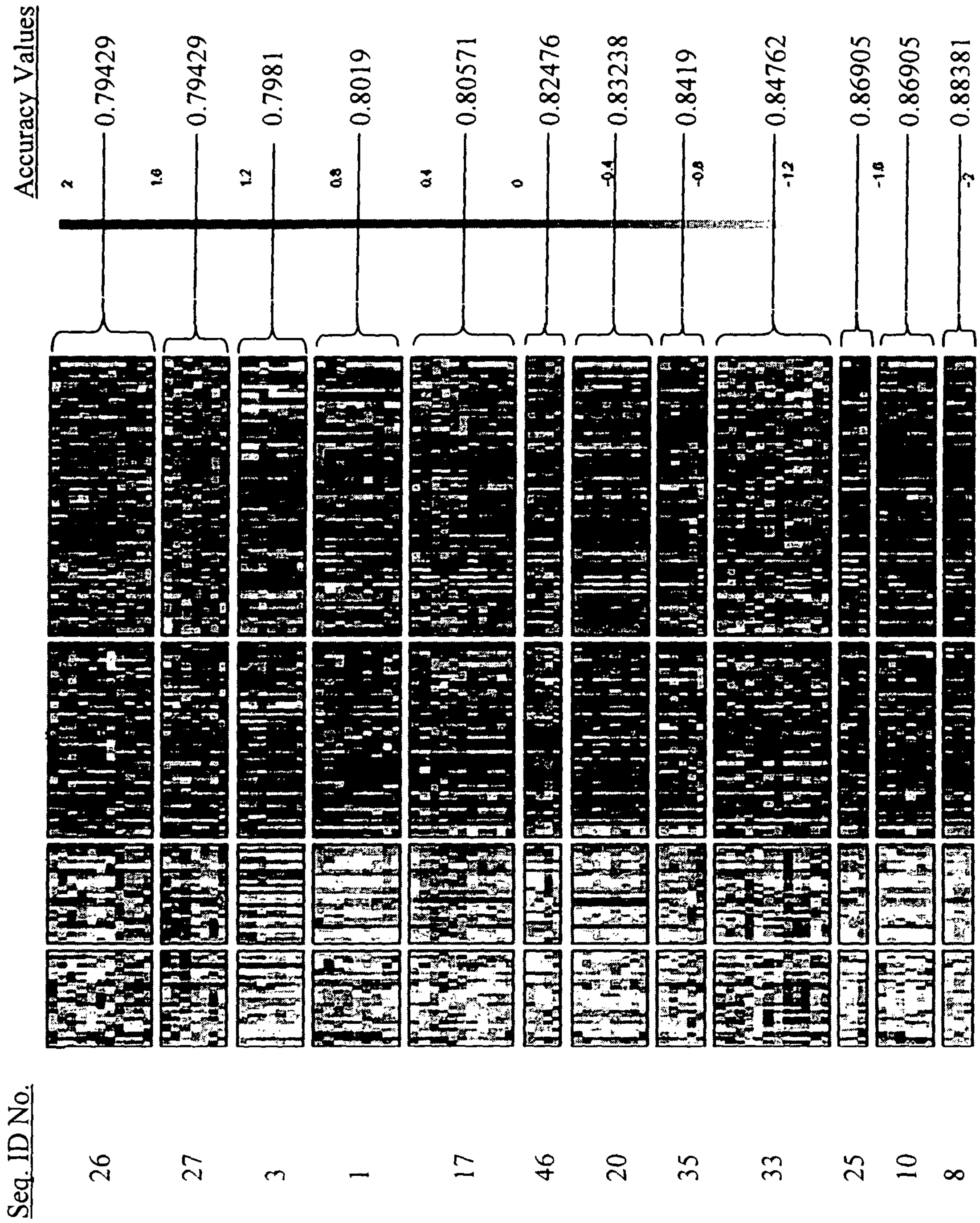
Seq. ID No.
26
27
3
1
17
46
20
35
33
25
10
8
Accuracy Values
0.79429
0.79429
0.7981
0.8019
0.80571
0.82476
0.83238
0.8419
0.84762
0.86905
0.86905
0.88381
2
1.6
1.2
0.8
0.4
0
-0.4
-0.8
-1.2
-1.6
-2

Figure 18

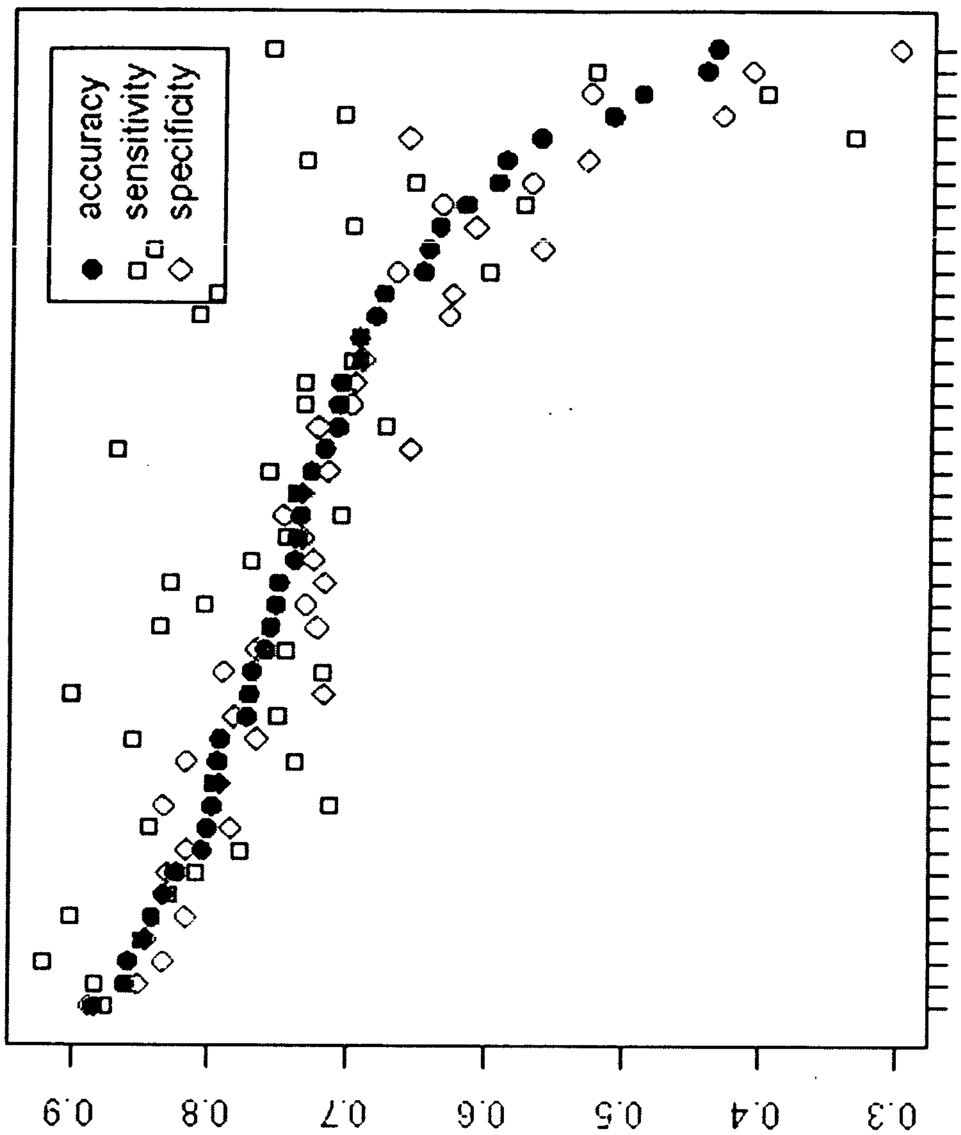


Figure 19

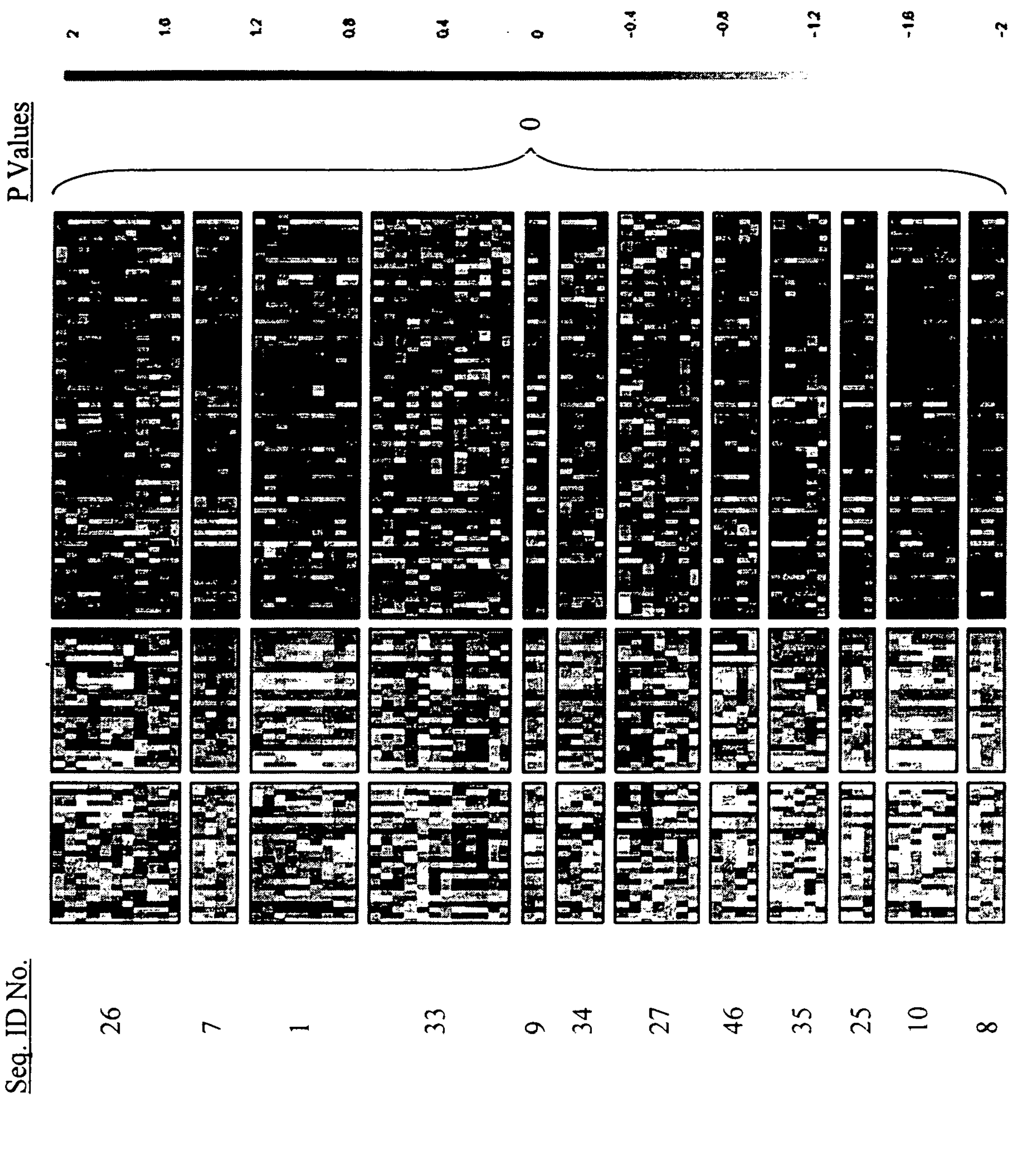
P Values
0
2
1.6
1.2
0.8
0.4
0
-0.4
-0.8
-1.2
-1.6
-2
Seq. ID No.
26
7
1
33
9
34
27
46
35
25
10
8

Figure 20

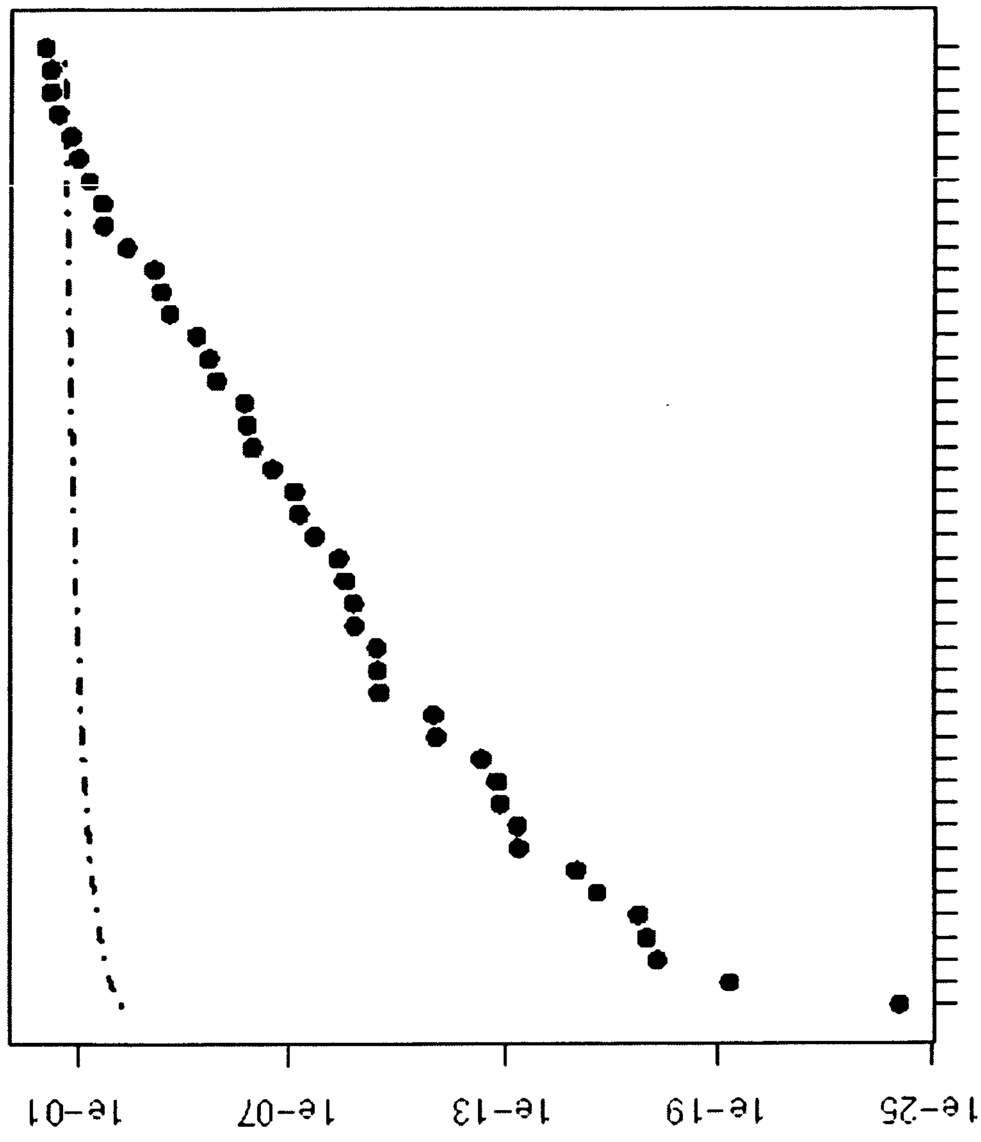


Figure 21

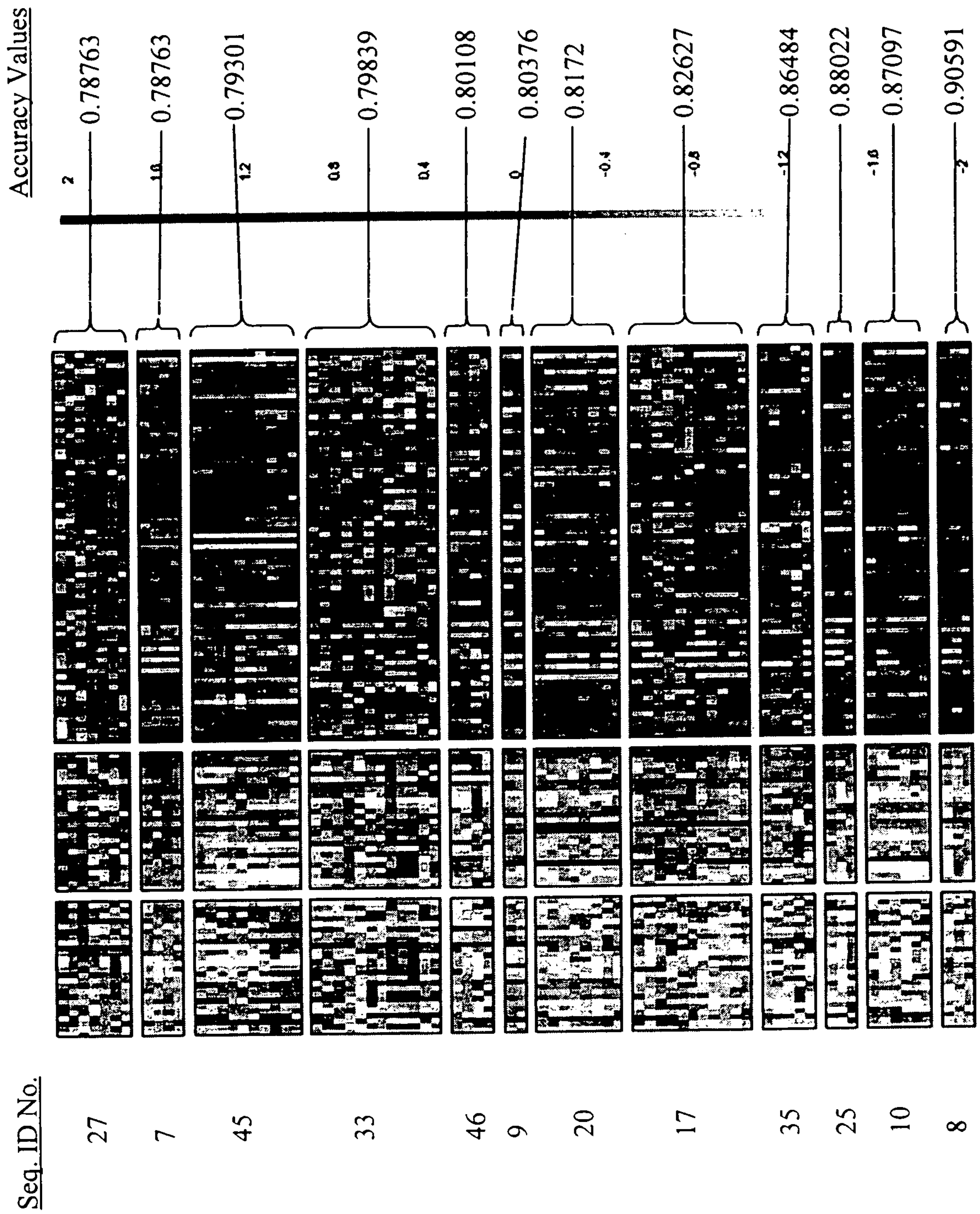
Accuracy Values
0.78763
0.78763
0.79301
0.79839
0.80108
0.80376
0.8172
0.82627
0.86484
0.88022
0.87097
0.90591
2
1.6
1.2
0.8
0.4
0
-0.4
-0.8
-1.2
-1.6
-2
Seq. ID No.
27
7
45
33
46
9
20
17
35
25
10
8

Figure 22

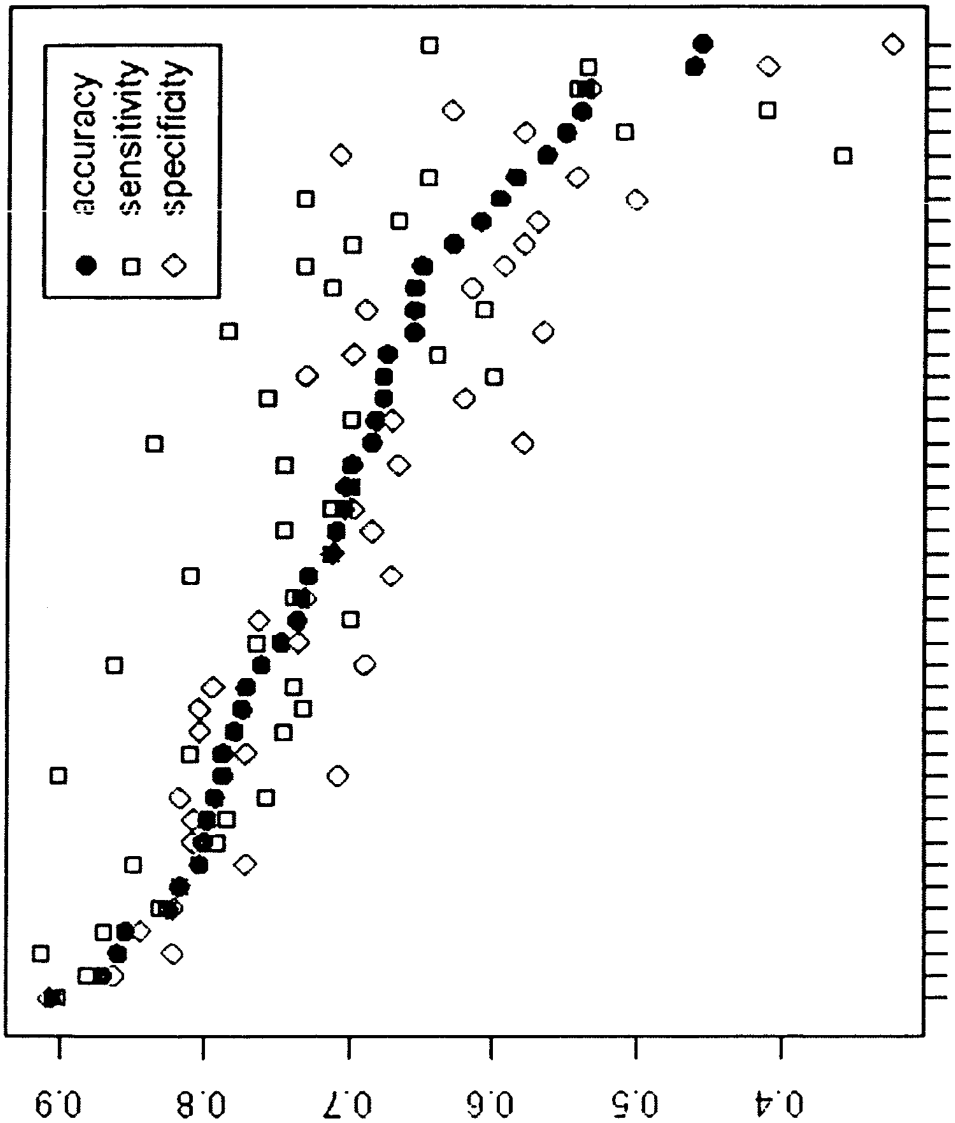


Figure 23

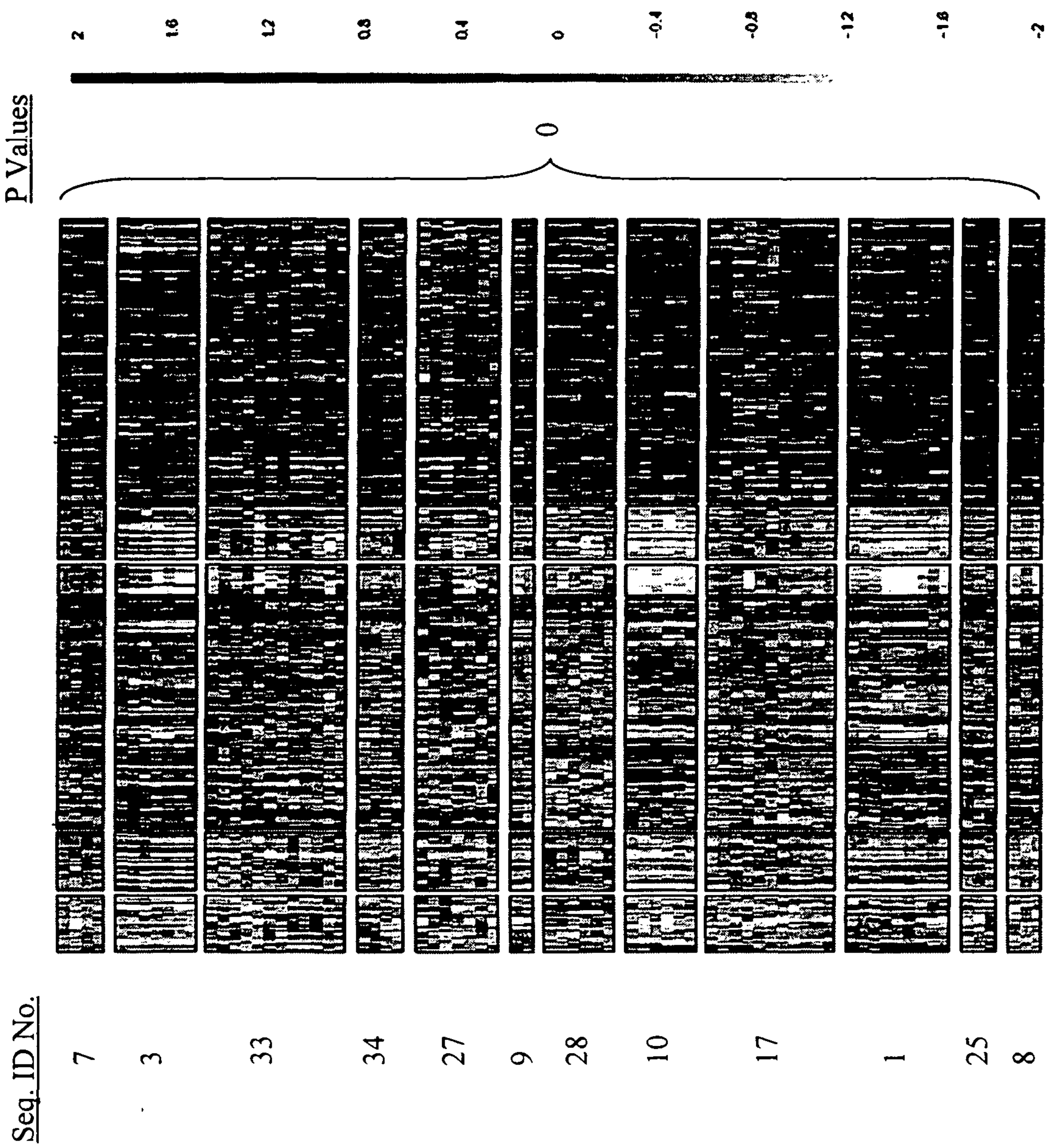
P Values
0
2
1.6
1.2
0.8
0.4
0
-0.4
-0.8
-1.2
-1.6
-2
Seg. ID No.
7
3
33
34
27
9
28
10
17
1
25
8

Figure 24

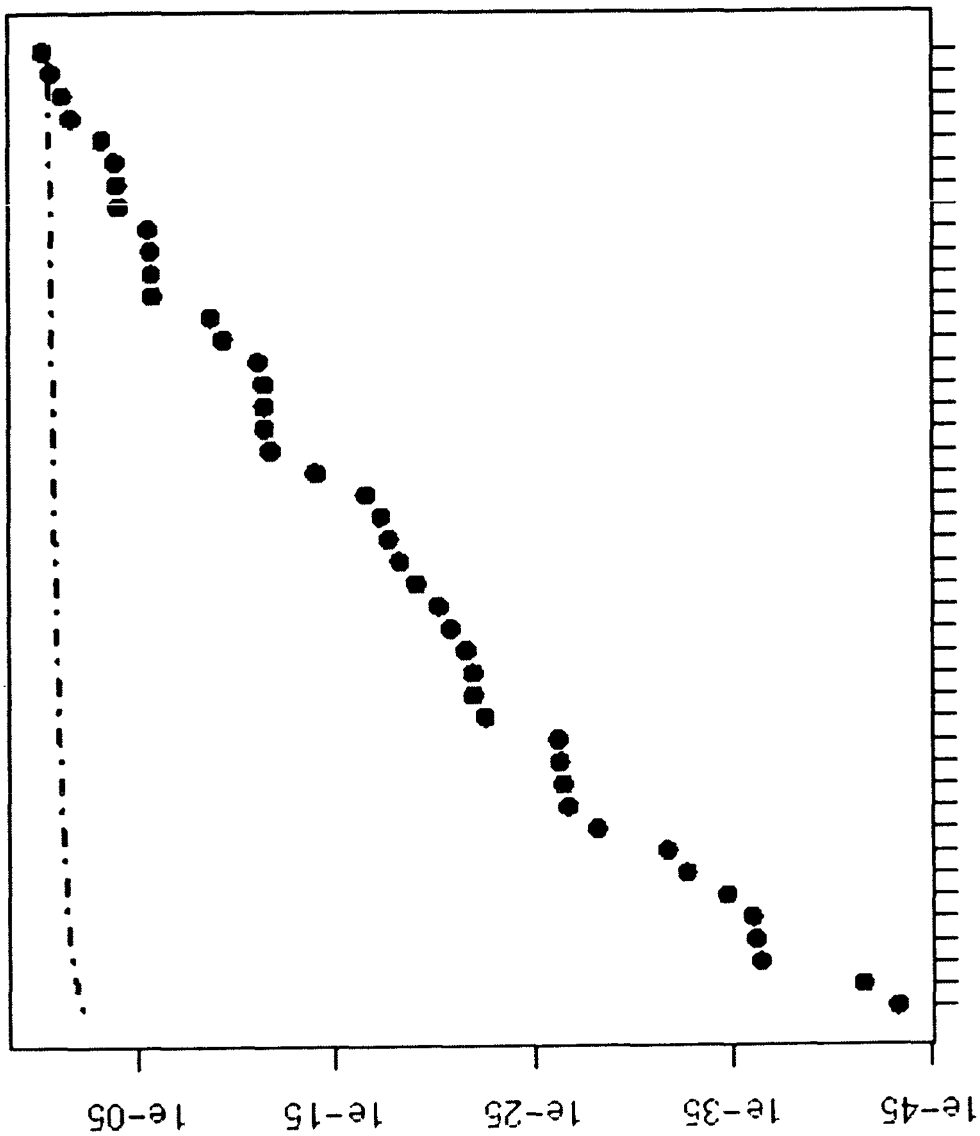


Figure 25

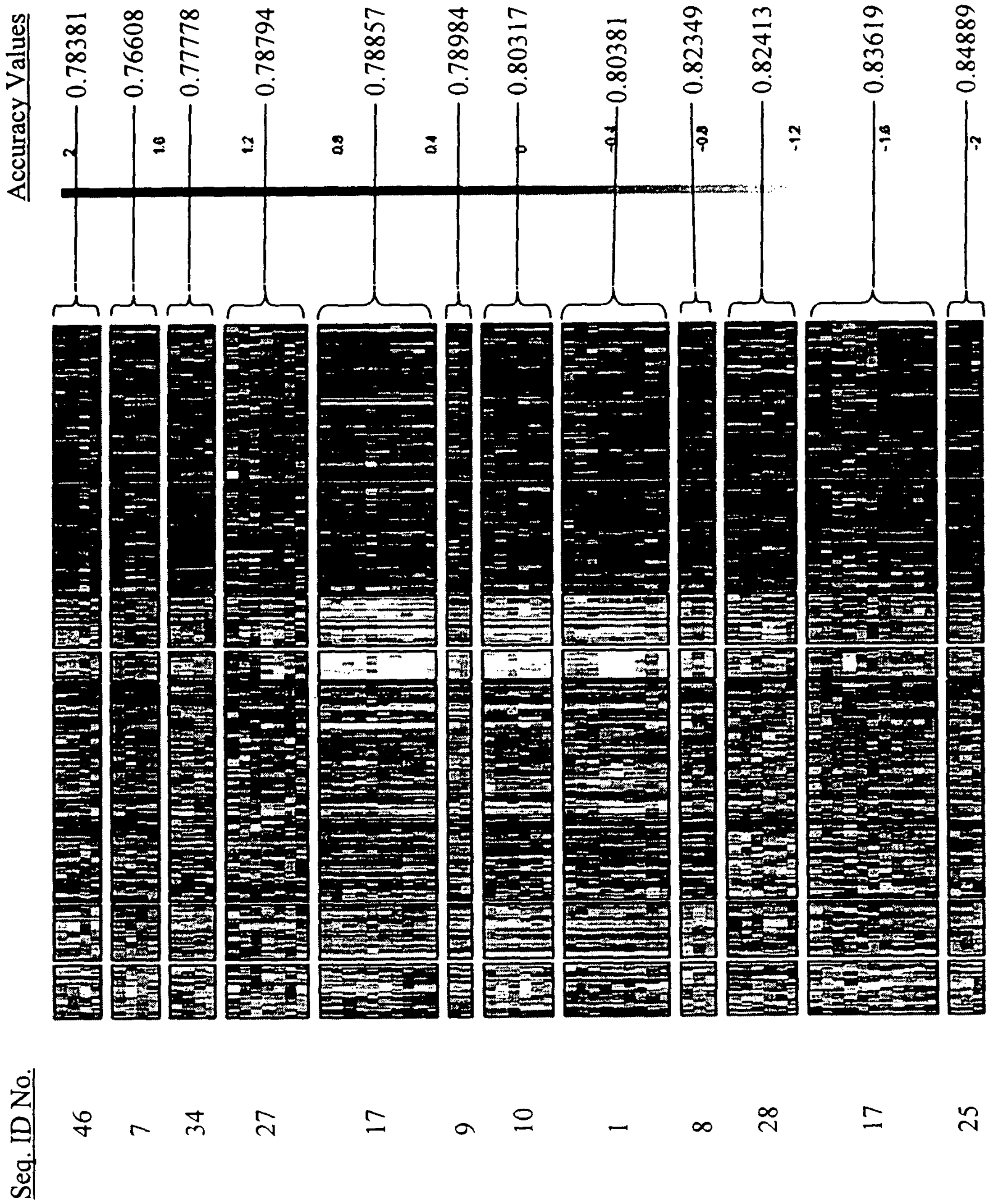
Accuracy Values
0.78381
0.76608
0.77778
0.78794
0.78857
0.78984
0.80317
0.80381
0.82349
0.82413
0.83619
0.84889
2
1.6
1.2
0.8
0.4
0
-0.4
-0.8
-1.2
-1.6
-2
Seq. ID No.
46
7
34
27
17
9
10
1
8
28
17
25

Figure 26

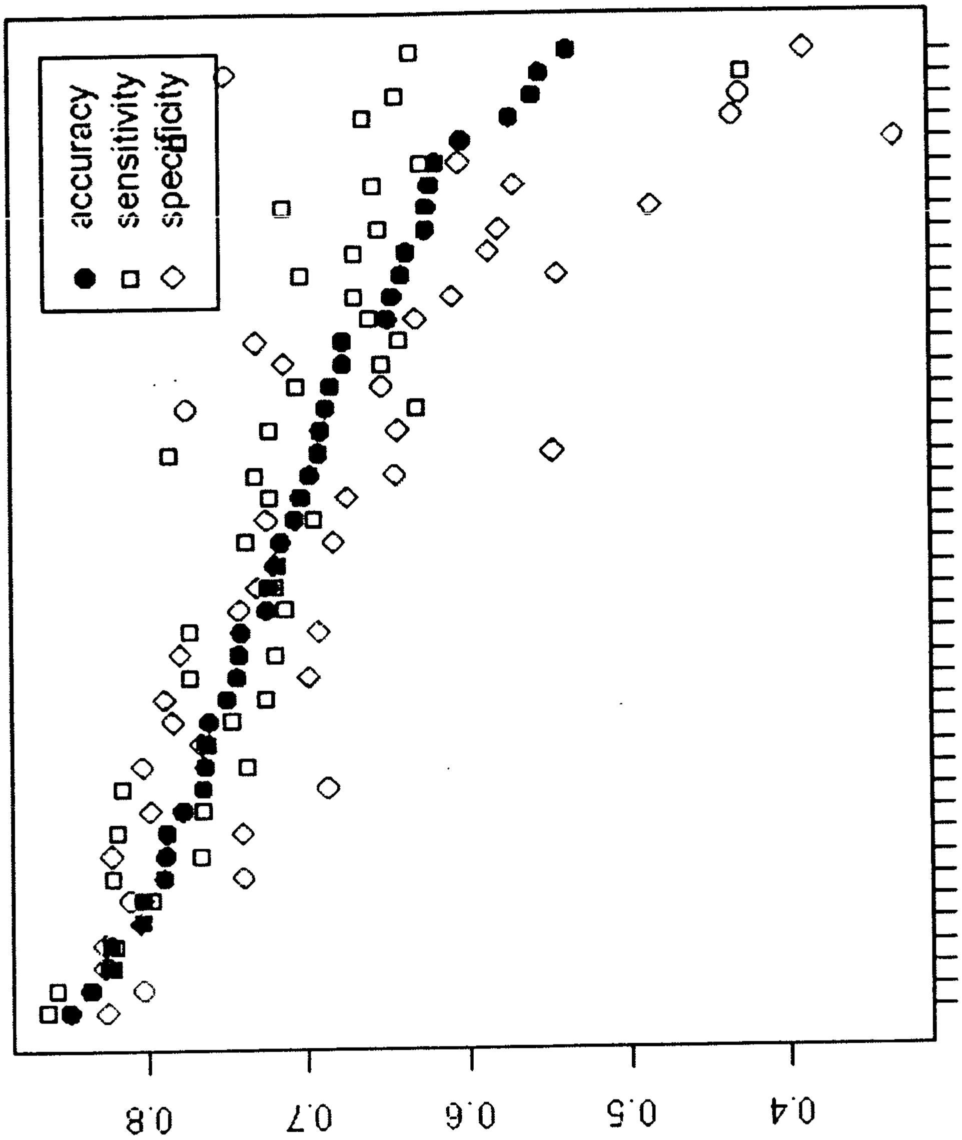


Figure 27

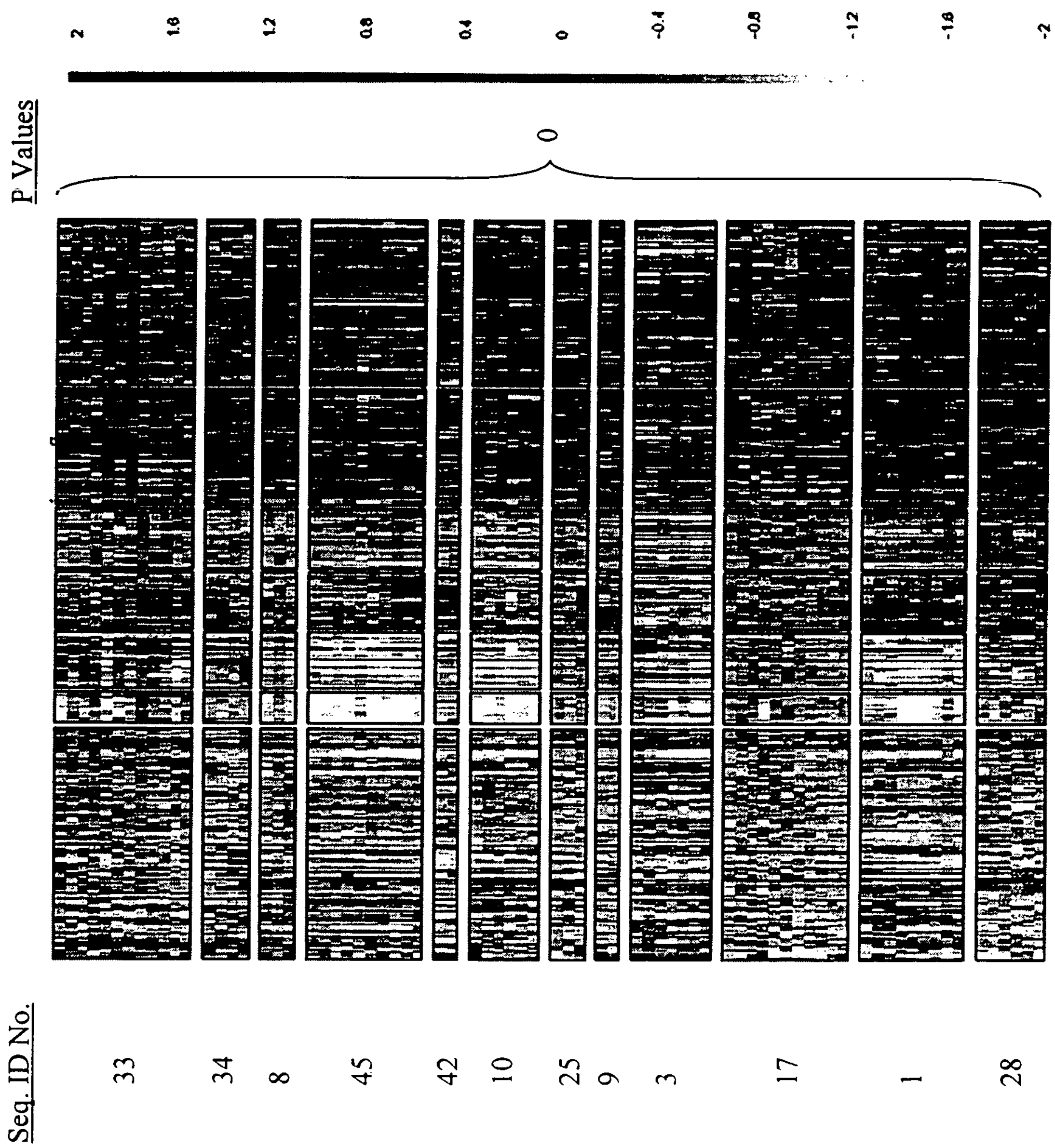
P Values
0
2
1.6
1.2
0.8
0.4
0
-0.4
-0.8
-1.2
-1.6
-2
Seq. ID No.
33
34
8
45
42
10
25
9
3
17
1
28

Figure 28

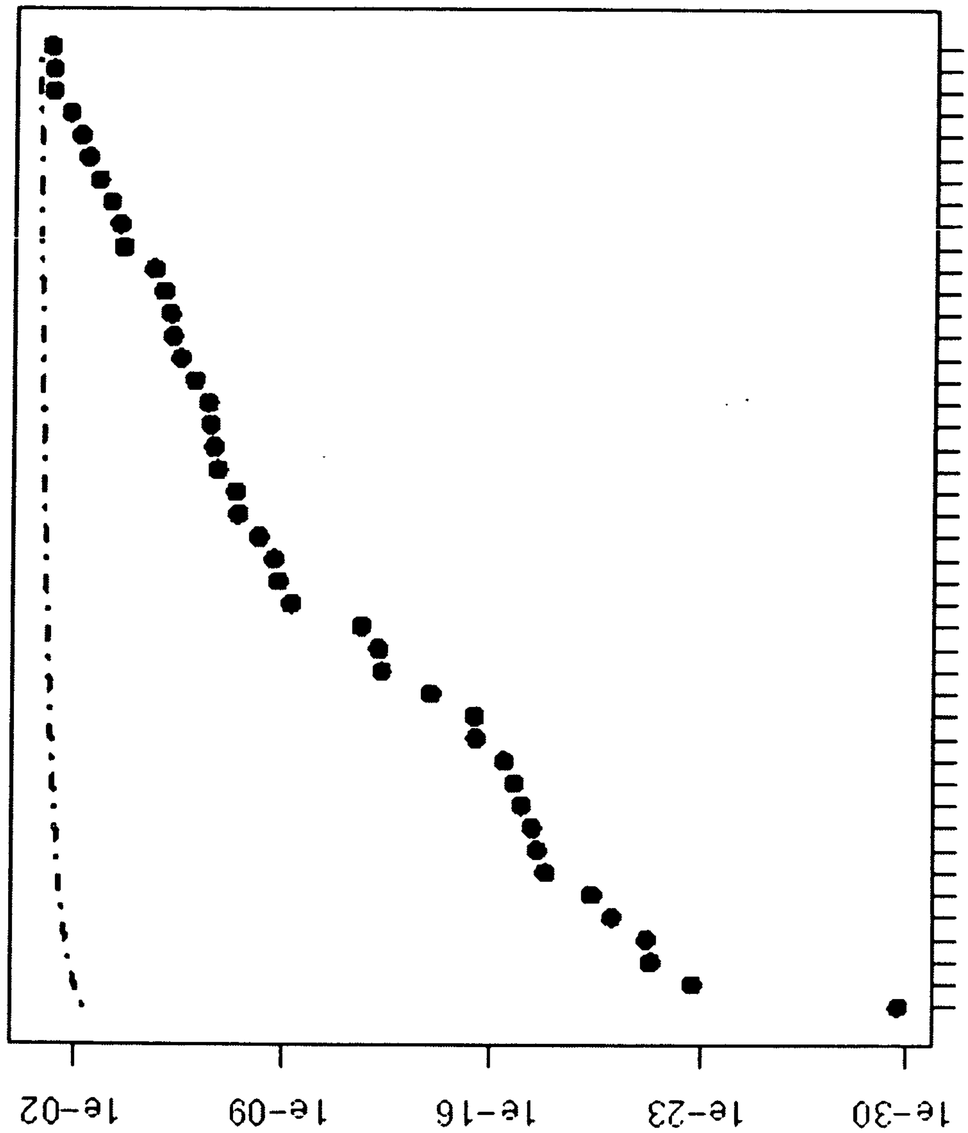


Figure 29

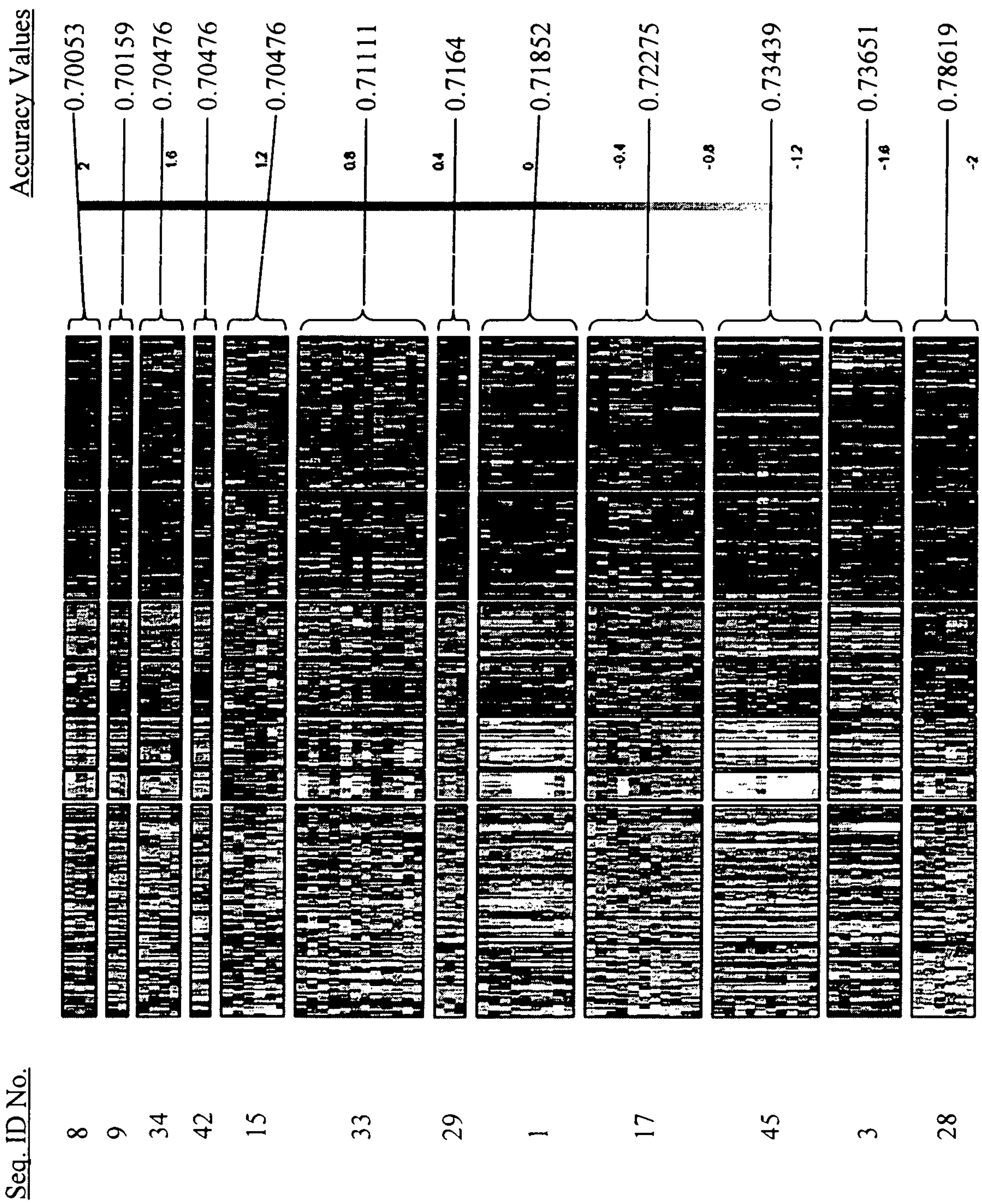
Seq. ID No.
8
9
34
42
15
33
29
1
17
45
3
28
Accuracy Values
0.70053
0.70159
0.70476
0.70476
0.70476
0.71111
0.7164
0.71852
0.72275
0.73439
0.73651
0.78619
2
1.6
1.2
0.8
0.4
0
-0.4
-0.8
-1.2
-1.6
-2

Figure 30

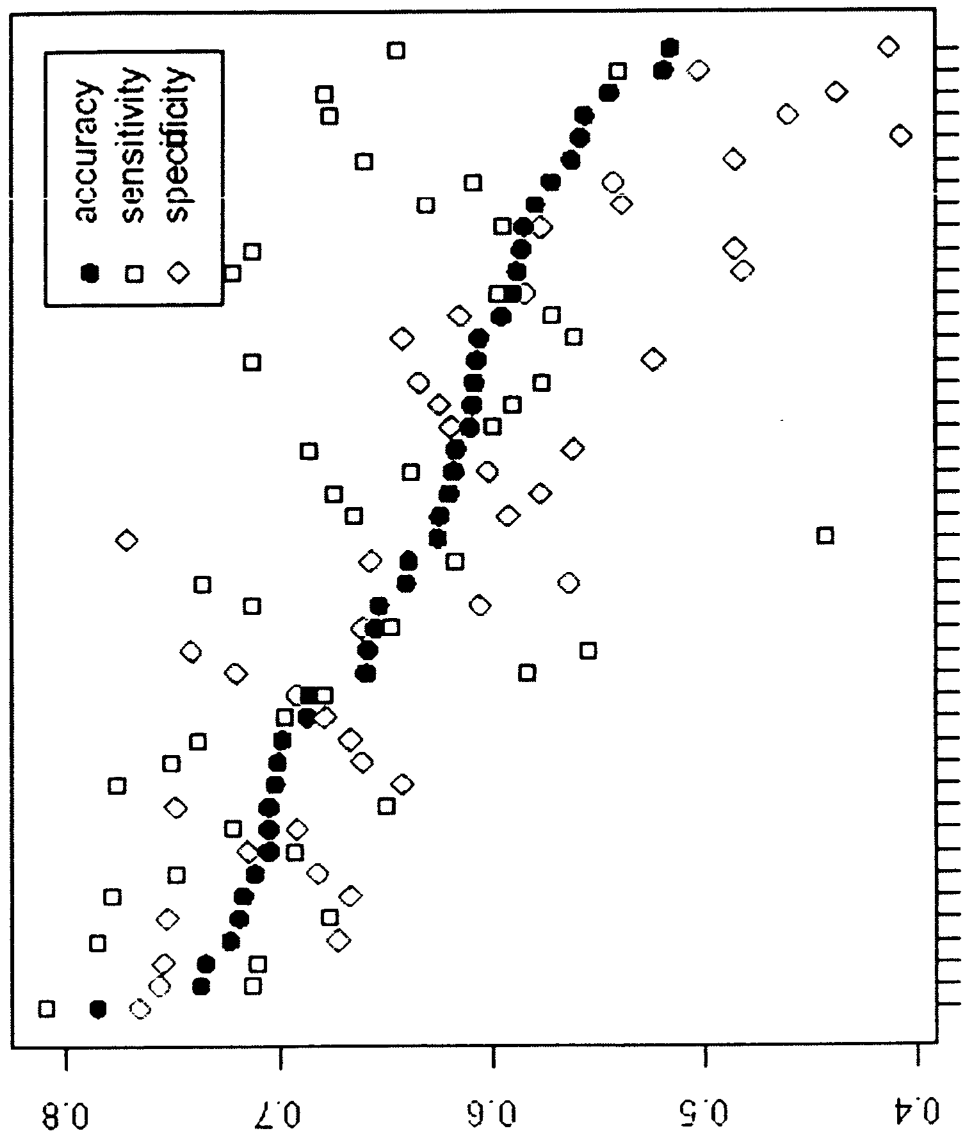


Figure 31

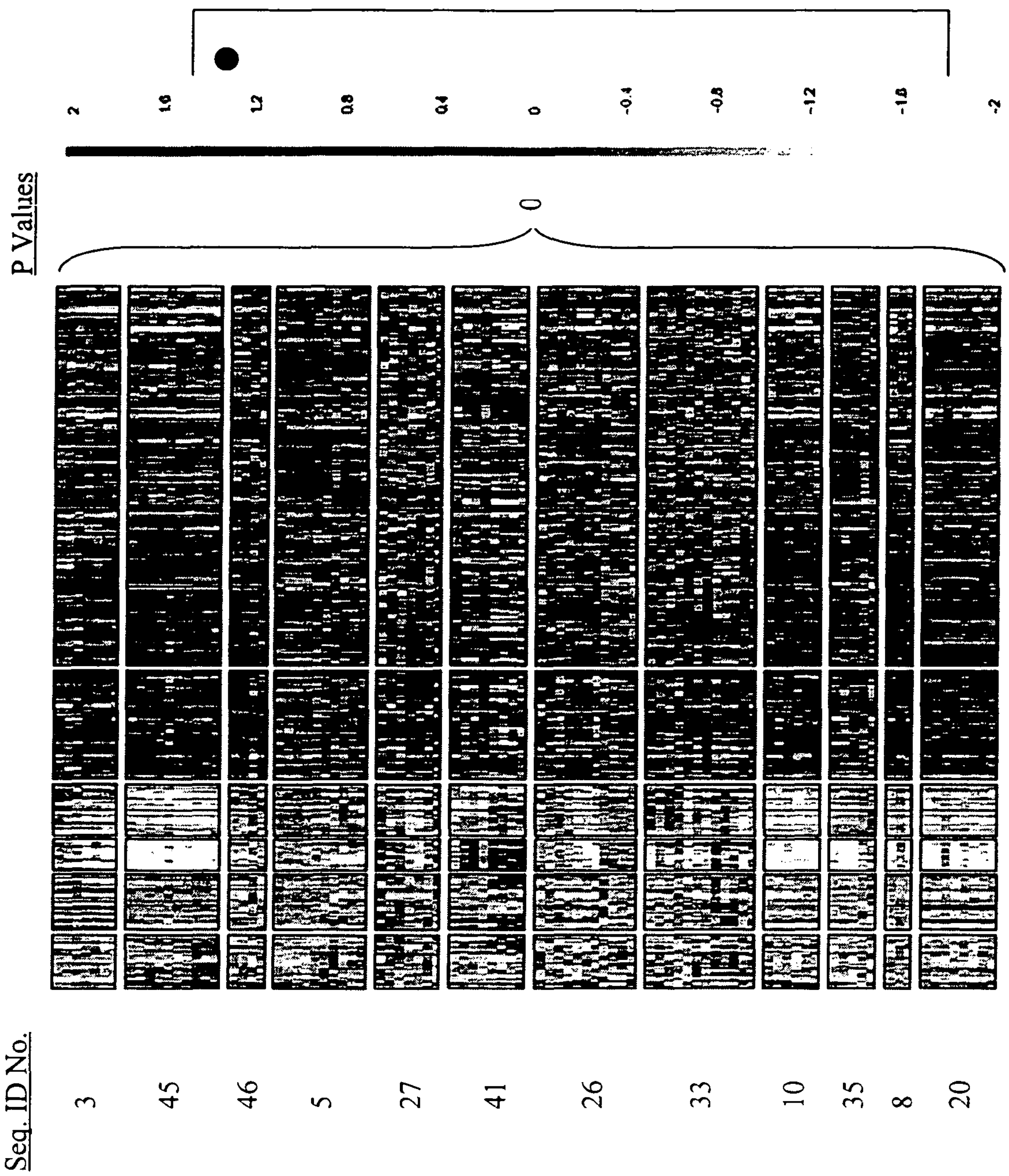
P Values
2
1.6
1.2
0.8
0.4
0
-0.4
-0.8
-1.2
-1.6
-2
0
Seq. ID No.
3
45
46
5
27
41
26
33
10
35
8
20

Figure 32

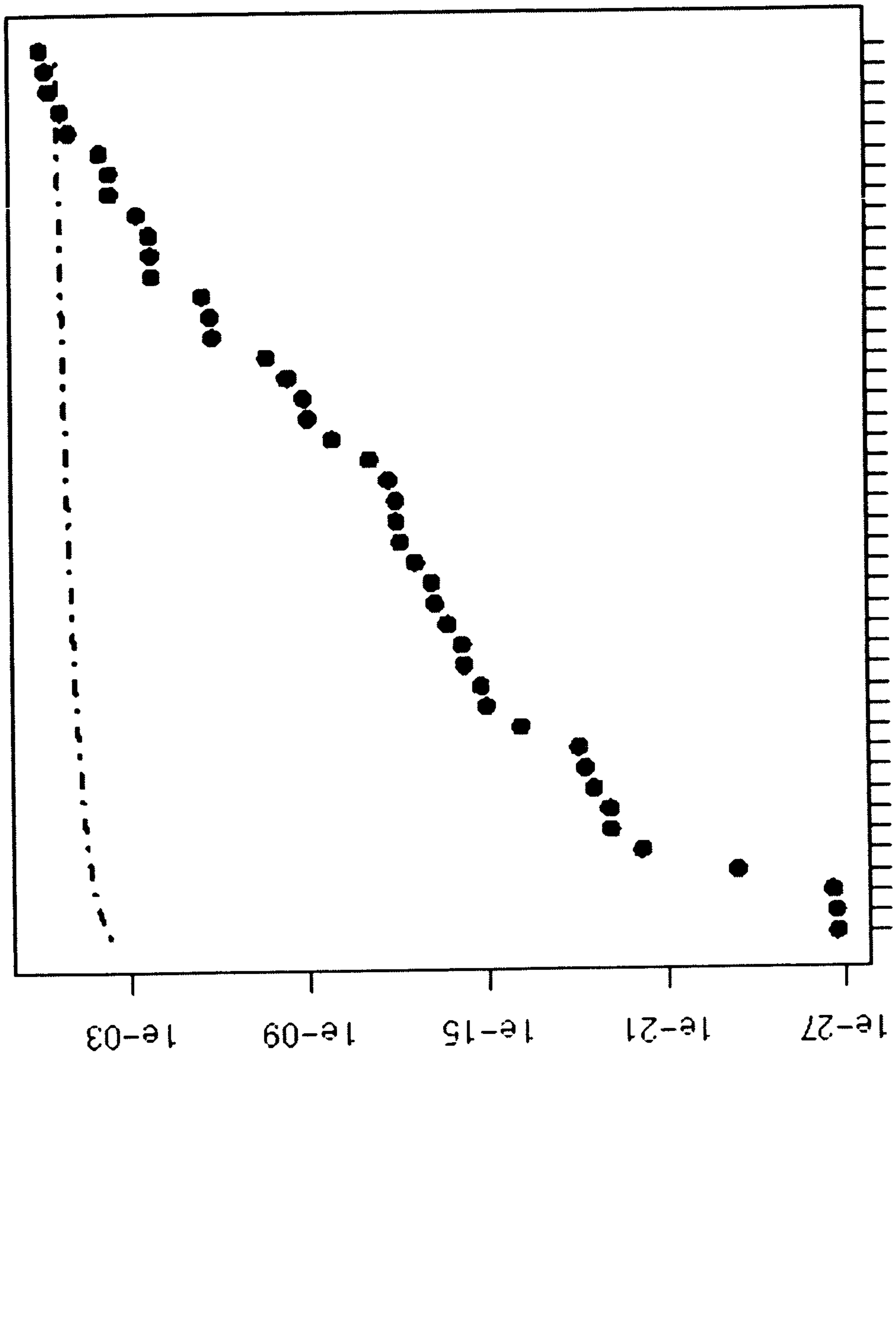


Figure 33

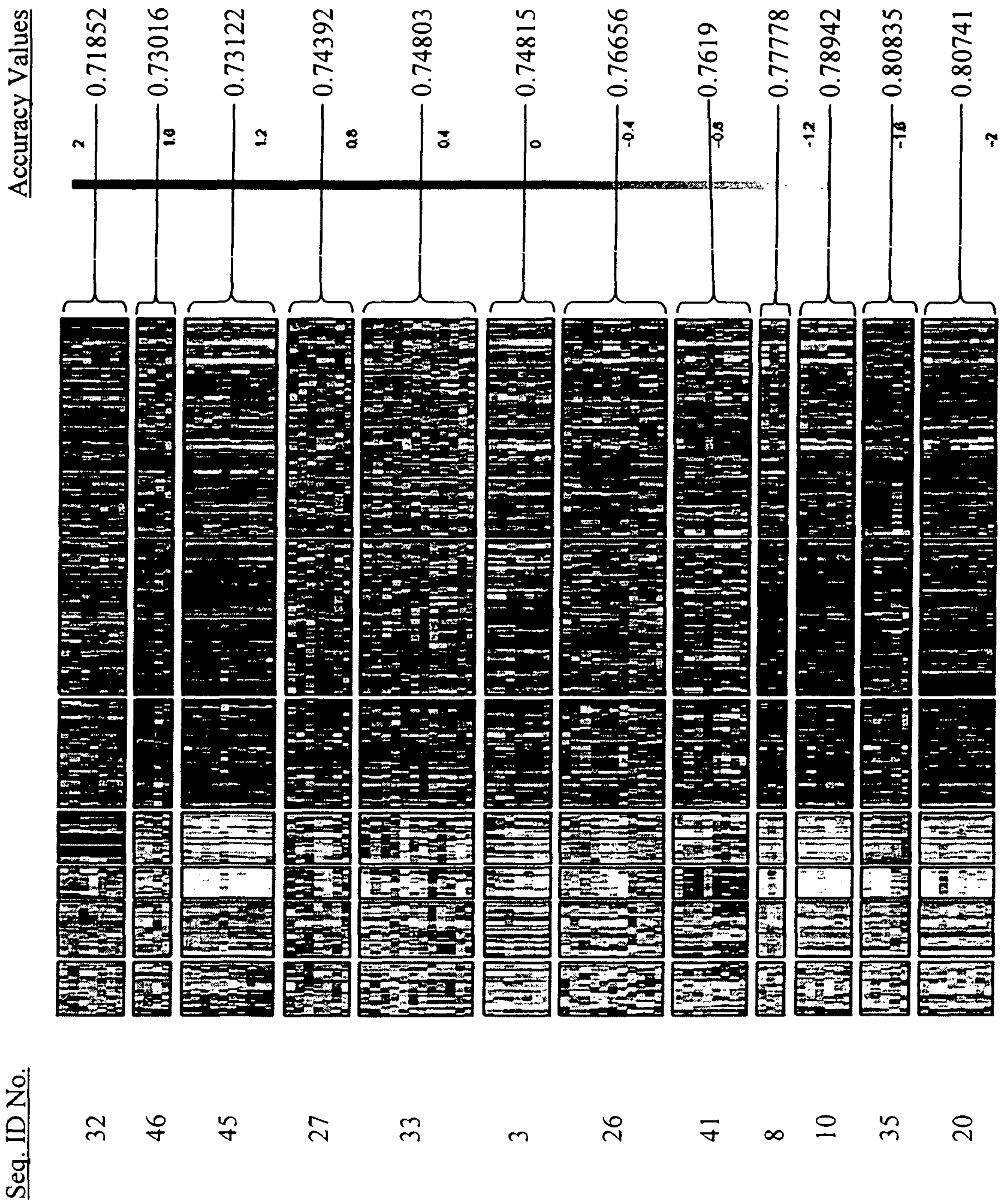
Seq. ID No.
Accuracy Values
32
0.71852
46
0.73016
45
0.73122
27
0.74392
33
0.74803
3
0.74815
26
0.76656
41
0.7619
8
0.77778
10
0.78942
35
0.80835
20
0.80741
2
1.6
1.2
0.8
0.4
0
-0.4
-0.8
-1.2
-1.6
-2

Figure 34

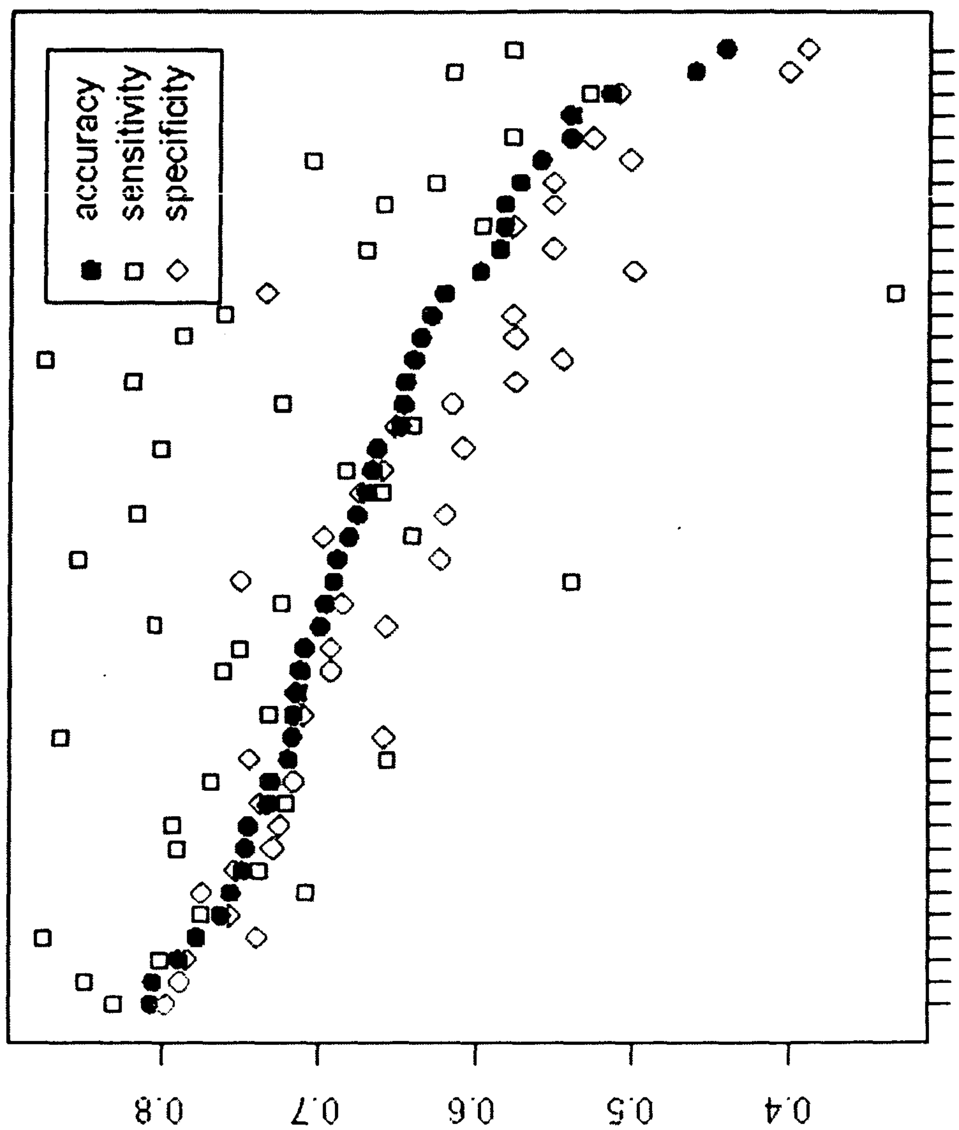


Figure 35

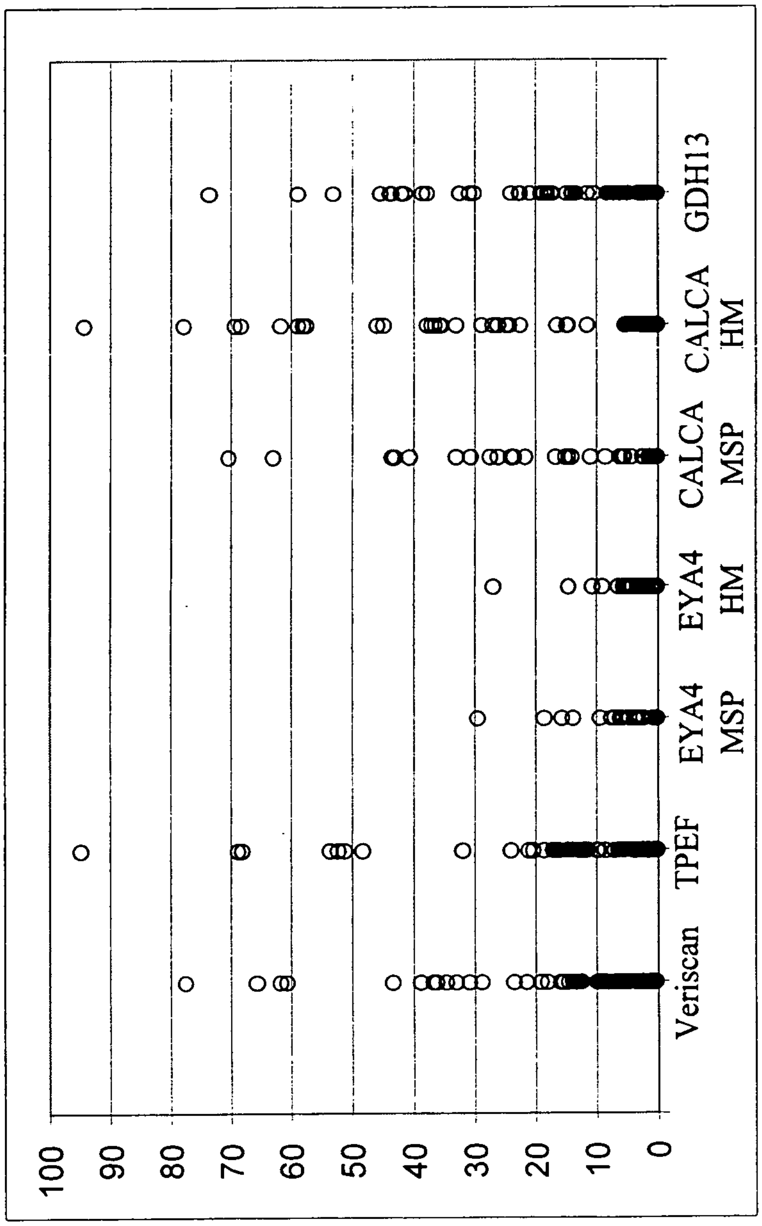
100
90
80
70
60
50
40
30
20
10
0
Veriscan
TPEF
EYA4 MSP
EYA4 HM
CALCA MSP
CALCA HM
GDH13

Figure 36

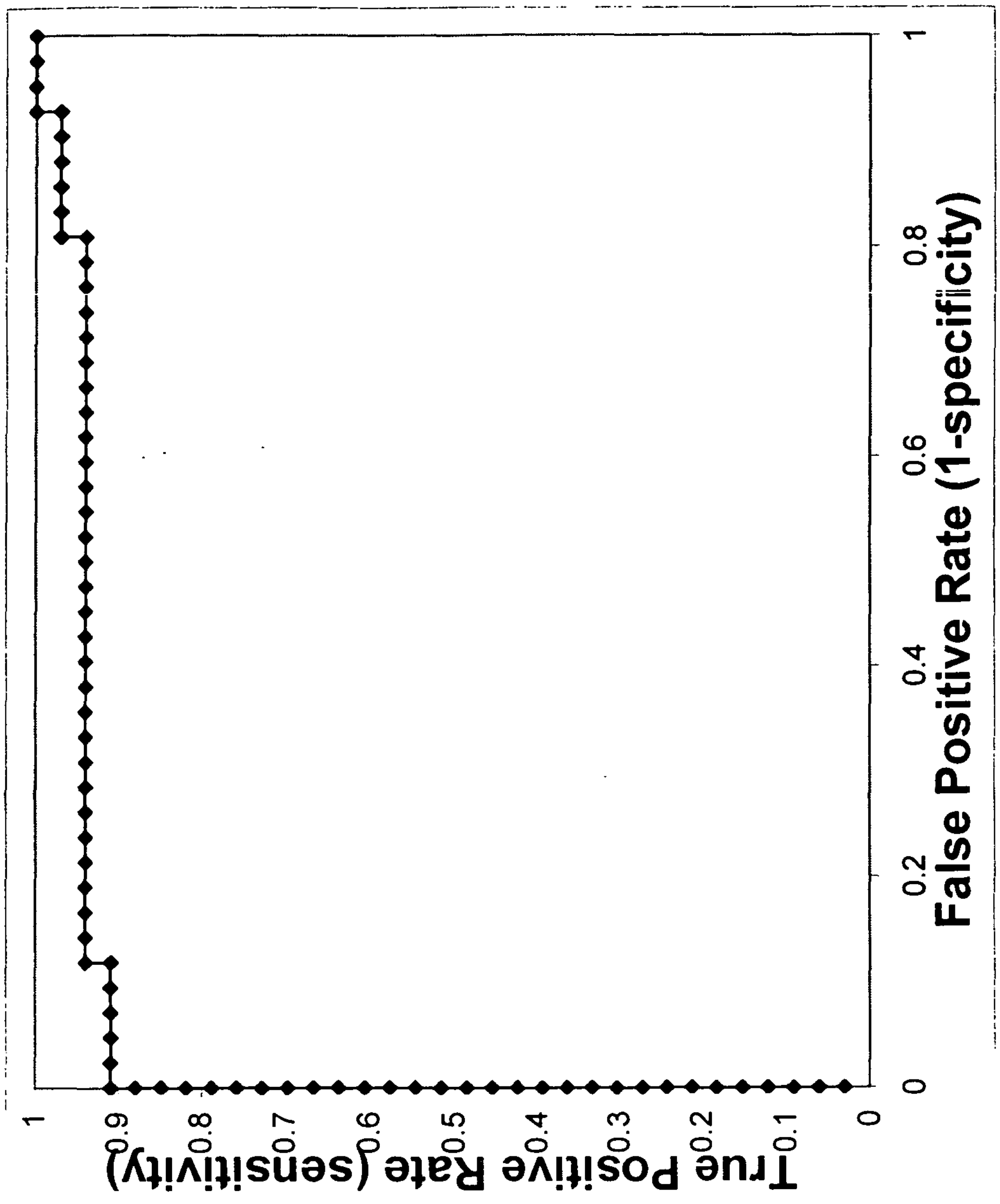
True Positive Rate (sensitivity)
False Positive Rate (1-specificity)
0
0.1
0.2
0.3
0.4
0.5
0.6
0.7
0.8
0.9
1
0
0.2
0.4
0.6
0.8
1

Figure 37

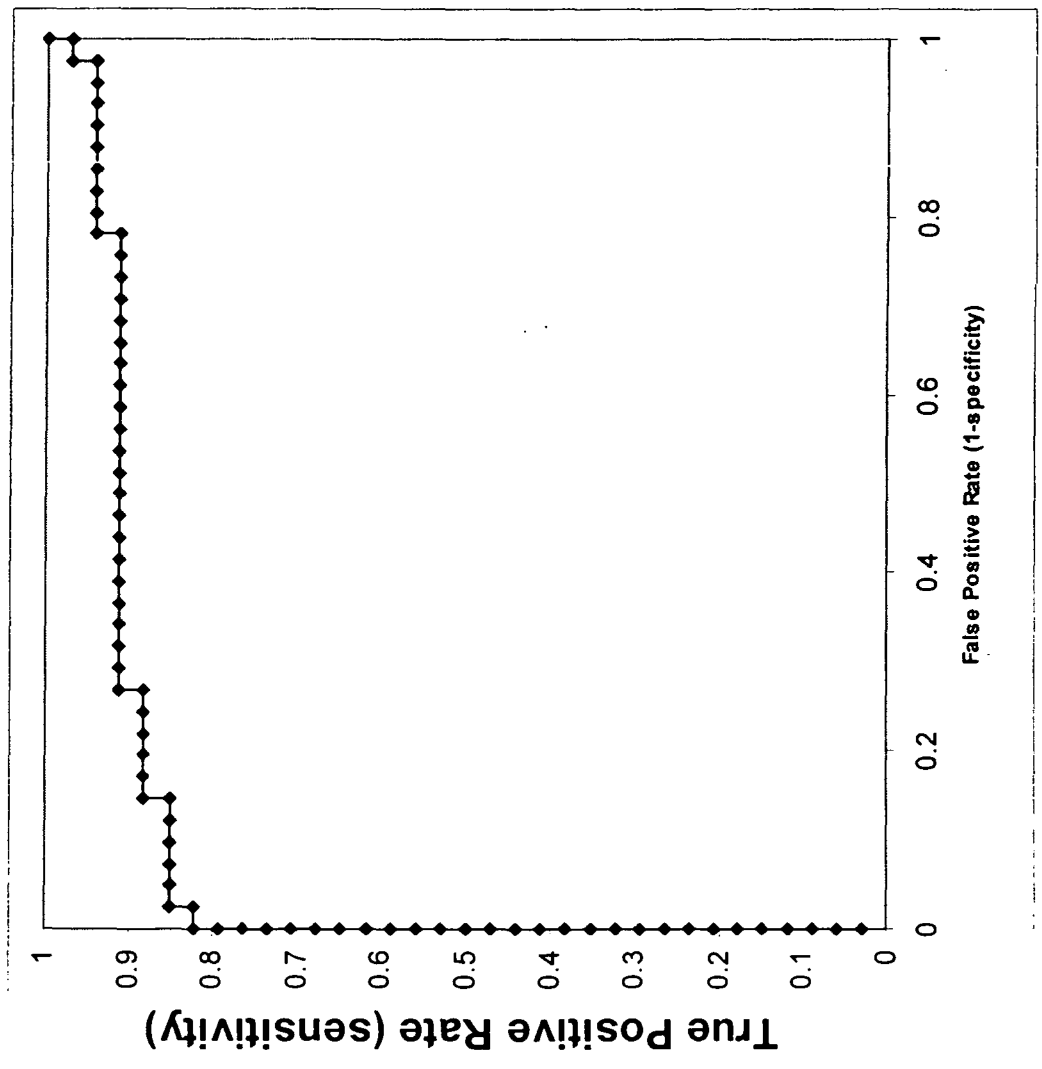
True Positive Rate (sensitivity)
1
0.9
0.8
0.7
0.6
0.5
0.4
0.3
0.2
0.1
0
0
0.2
0.4
0.6
0.8
1
False Positive Rate (1-specificity)

Figure 38

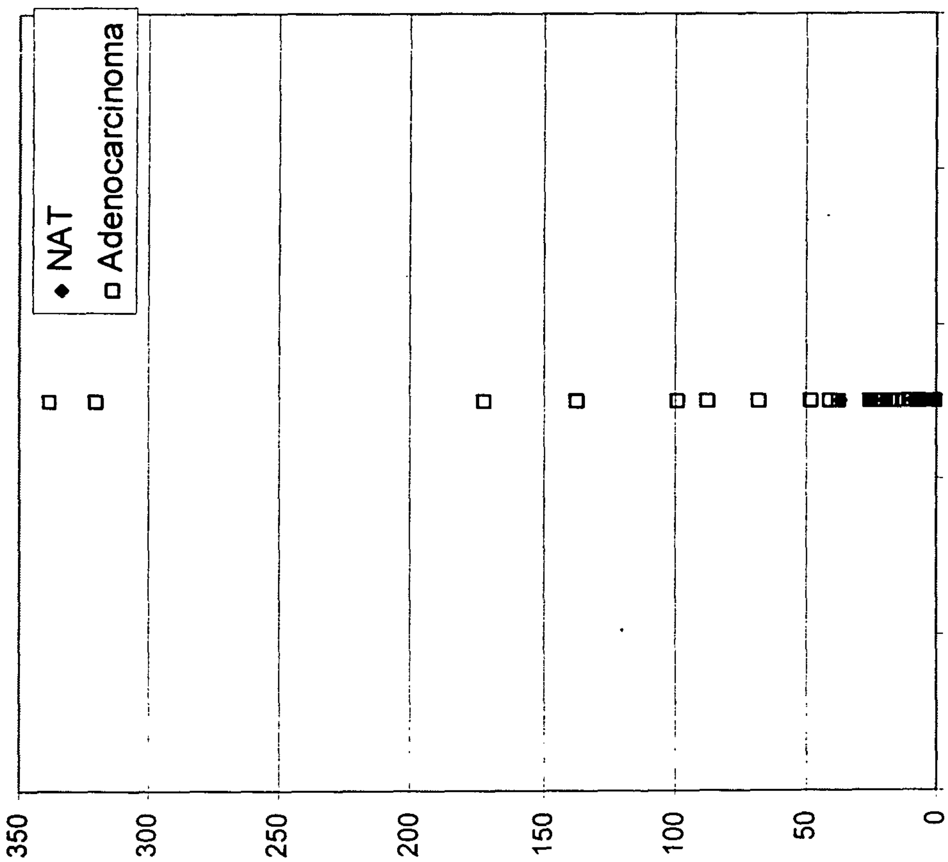


Figure 39

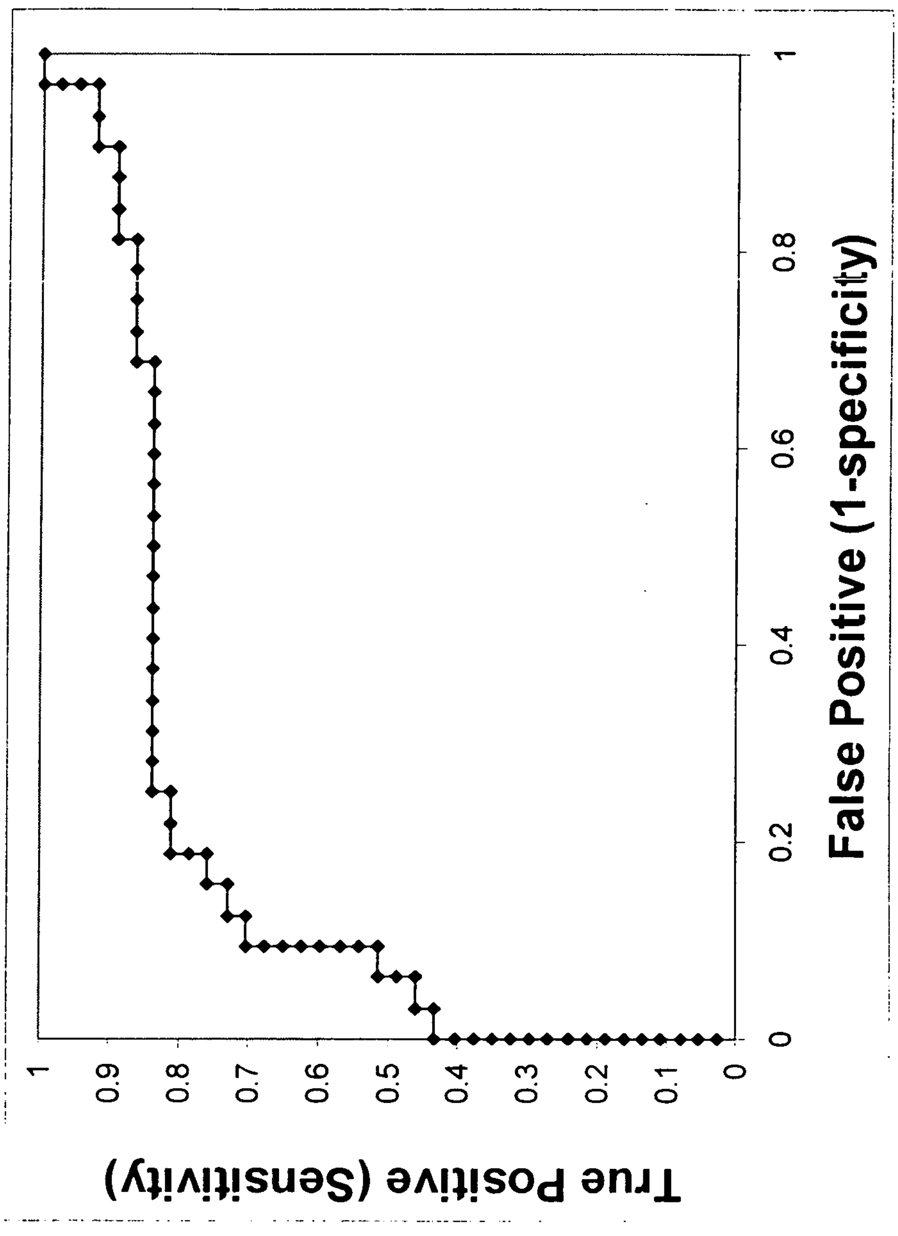
True Positive (Sensitivity)
1
0.9
0.8
0.7
0.6
0.5
0.4
0.3
0.2
0.1
0
0
0.2
0.4
0.6
0.8
1
False Positive (1-specificity)

Figure 40

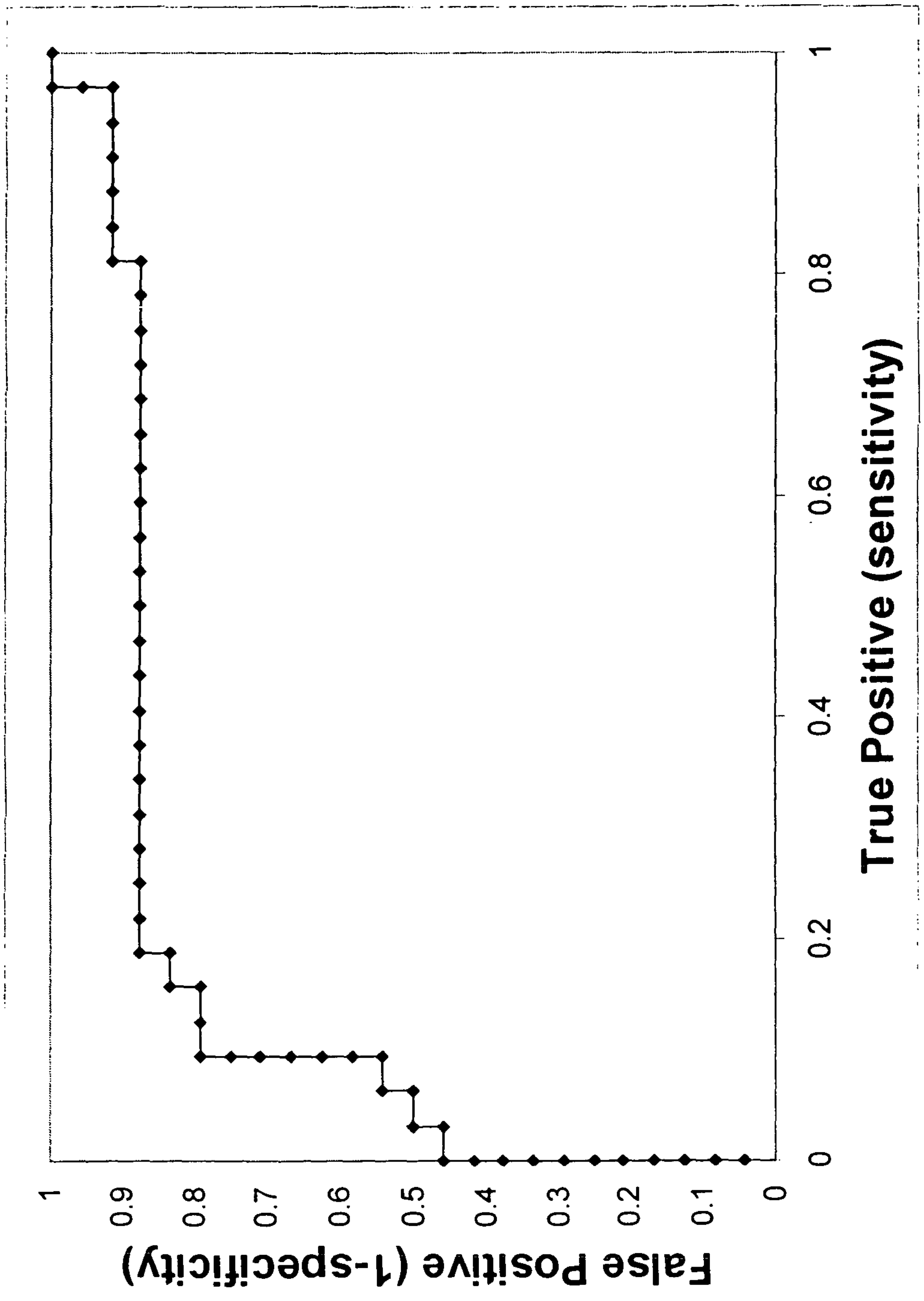
True Positive (sensitivity)
0
0.2
0.4
0.6
0.8
1
False Positive (1-specificity)
0
0.1
0.2
0.3
0.4
0.5
0.6
0.7
0.8
0.9
1

Figure 41

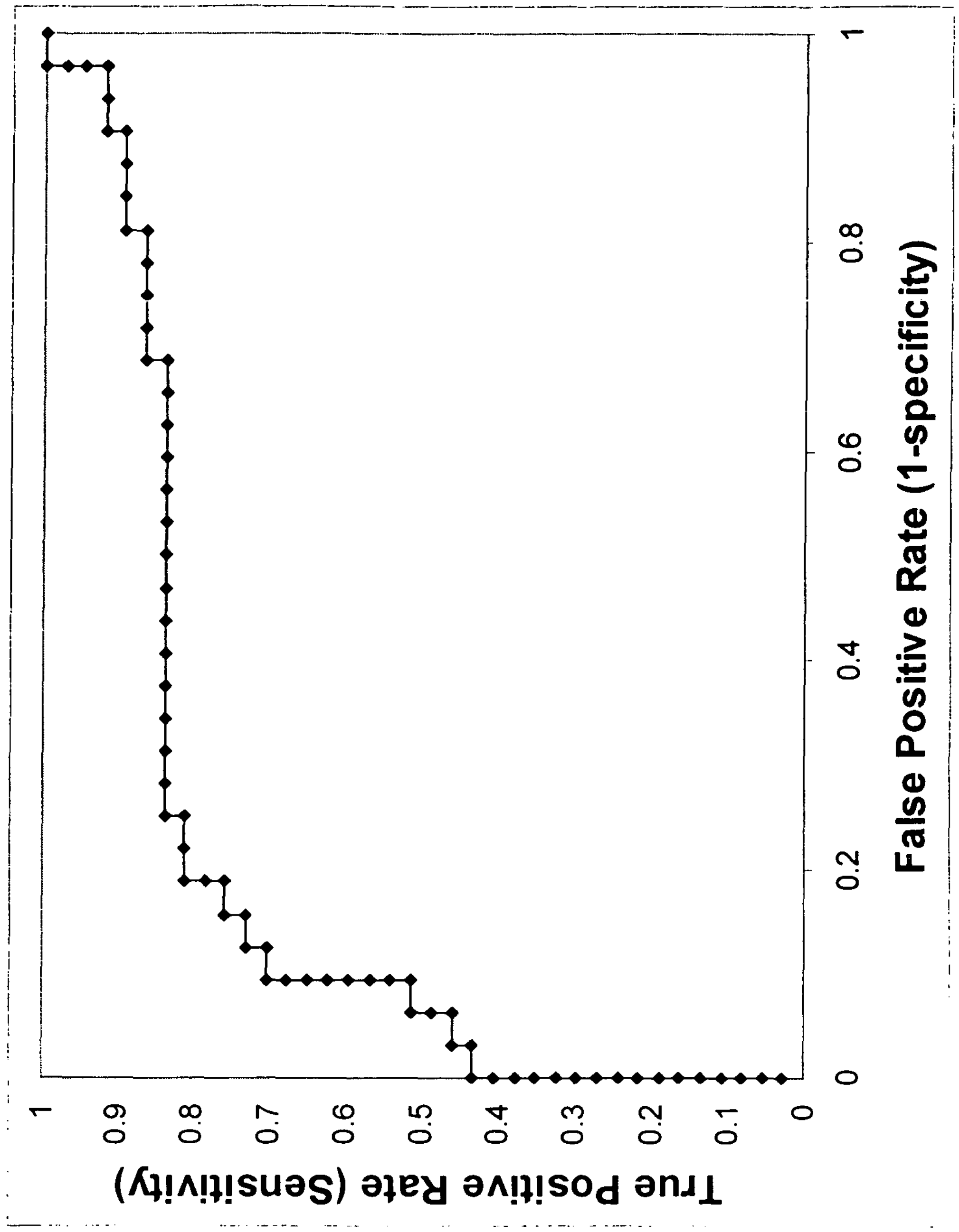
True Positive Rate (Sensitivity)
1
0.9
0.8
0.7
0.6
0.5
0.4
0.3
0.2
0.1
0
0.2
0.4
0.6
0.8
False Positive Rate (1-specificity)

Figure 42

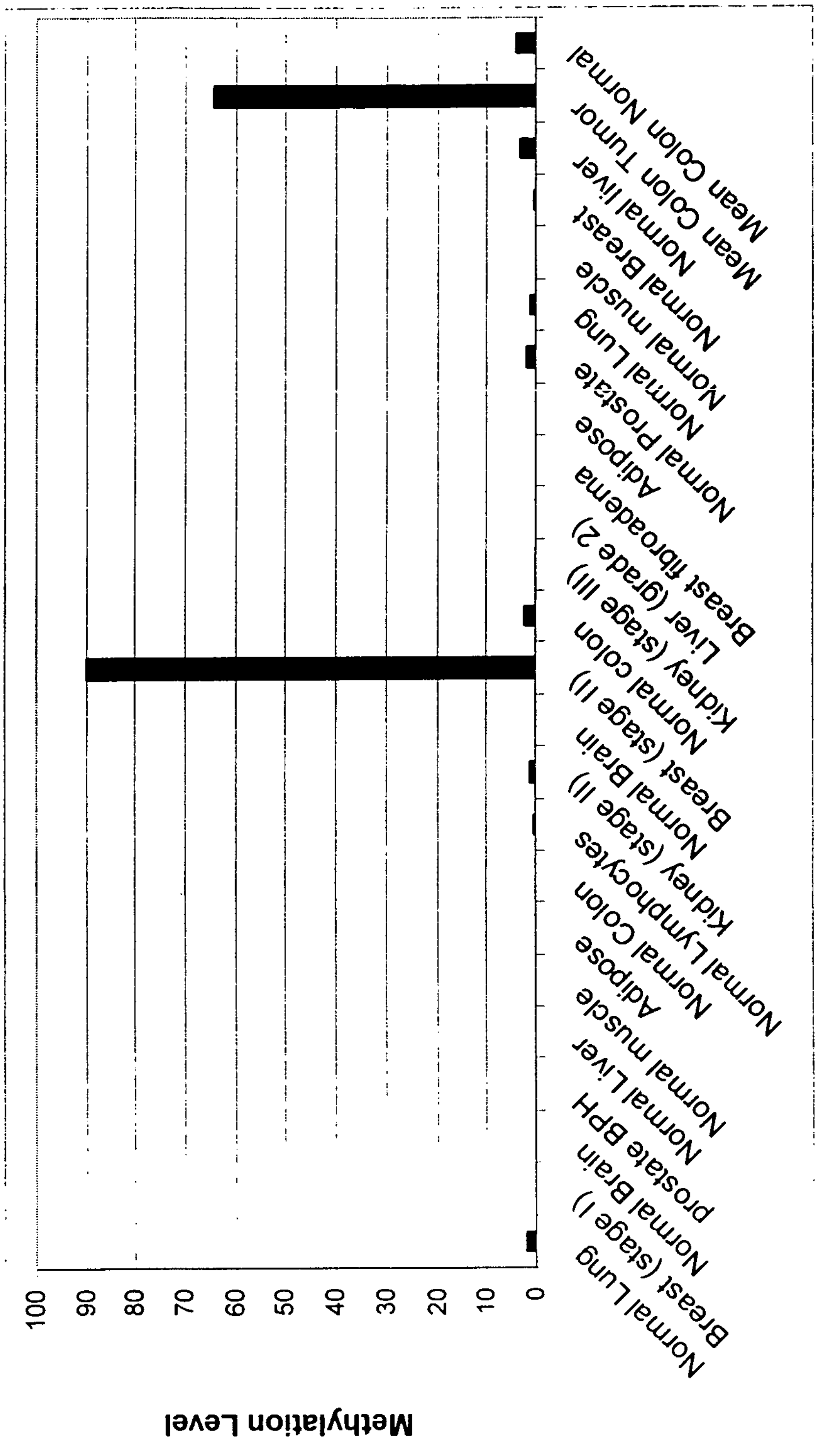
Methylation Level
100
90
80
70
60
50
40
30
20
10
0
Normal Lung
Breast (stage I)
Normal Brain
prostate BPH
Normal Liver
Normal muscle
Adipose
Normal Colon
Normal Lymphocytes
Kidney (stage II)
Normal Brain
Breast (stage II)
Normal colon
Kidney (stage III)
Liver (grade 2)
Breast fibroadema
Adipose
Normal Prostate
Normal Lung
Normal muscle
Normal Breast
Normal liver
Mean Colon Tumor
Mean Colon Normal

Figure 43

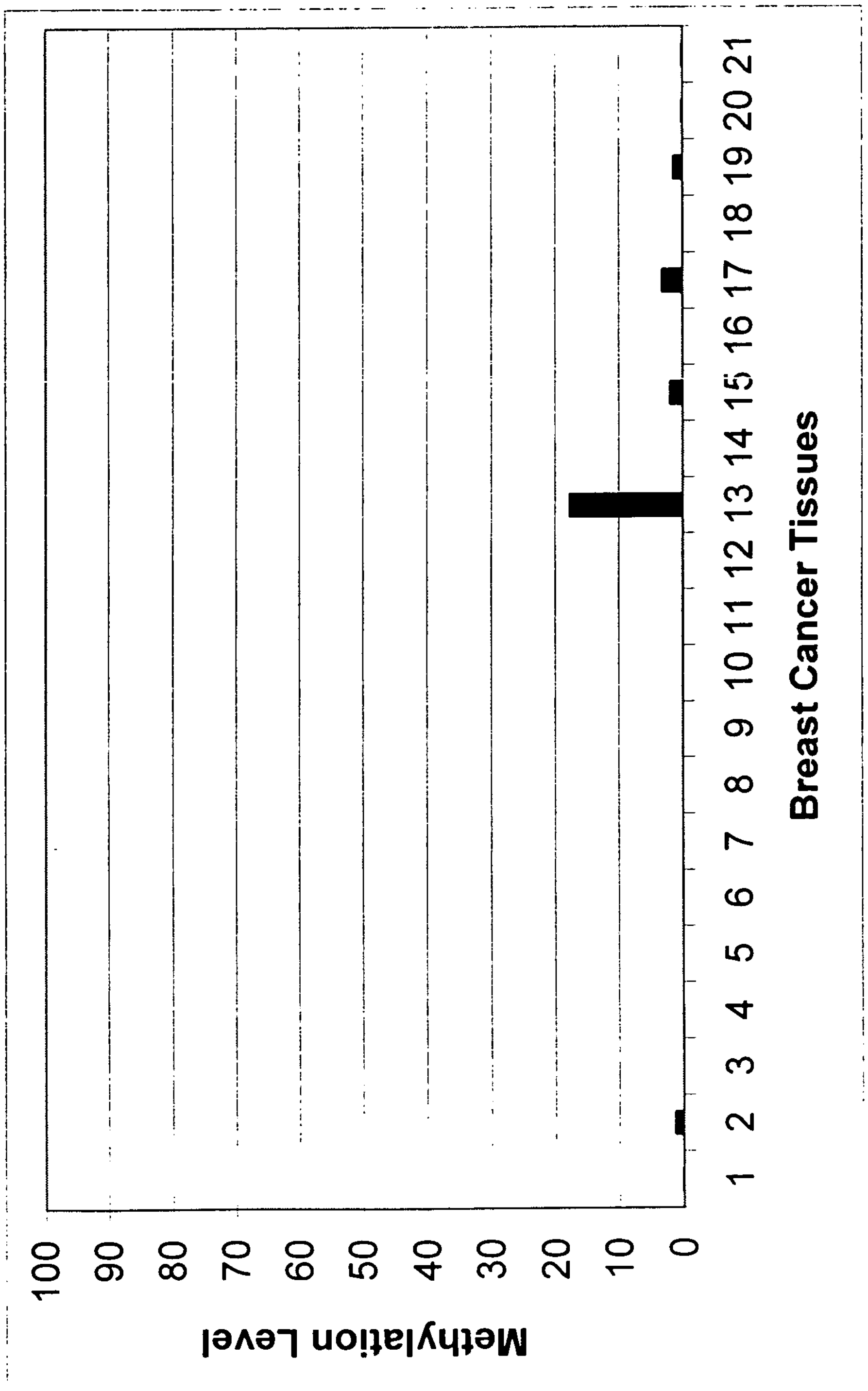
Methylation Level
100
90
80
70
60
50
40
30
20
10
0
1 2 3 4 5 6 7 8 9 10 11 12 13 14 15 16 17 18 19 20 21
Breast Cancer Tissues

Figure 44

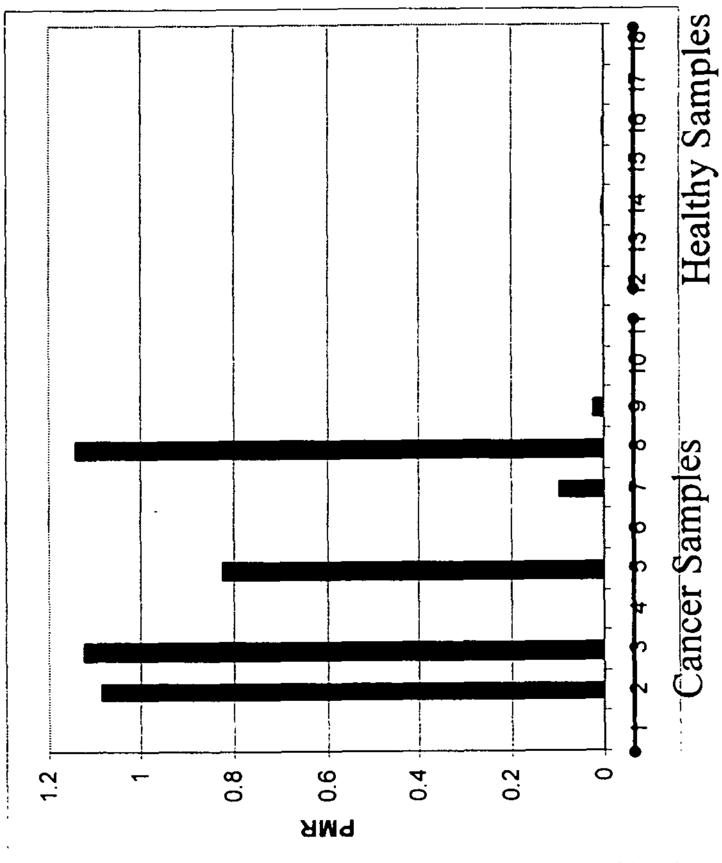
PMR
1.2
1
0.8
0.6
0.4
0.2
0
1 2 3 4 5 6 7 8 9 10 11 12 13 14 15 16 17 18
Cancer Samples
Healthy Samples

Figure 45

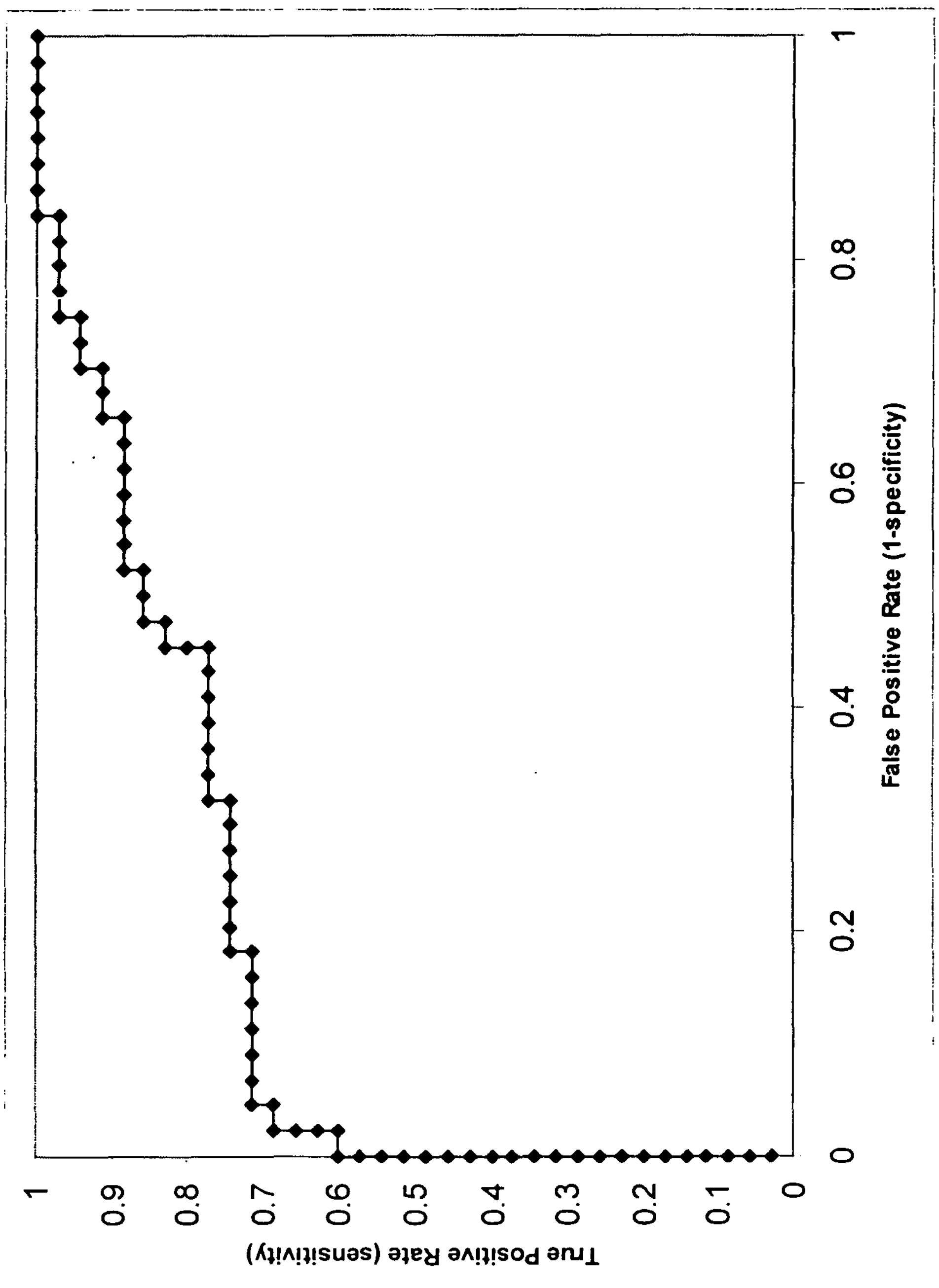
True Positive Rate (sensitivity)
1
0.9
0.8
0.7
0.6
0.5
0.4
0.3
0.2
0.1
0
False Positive Rate (1-specificity)
0
0.2
0.4
0.6
0.8
1

Figure 46

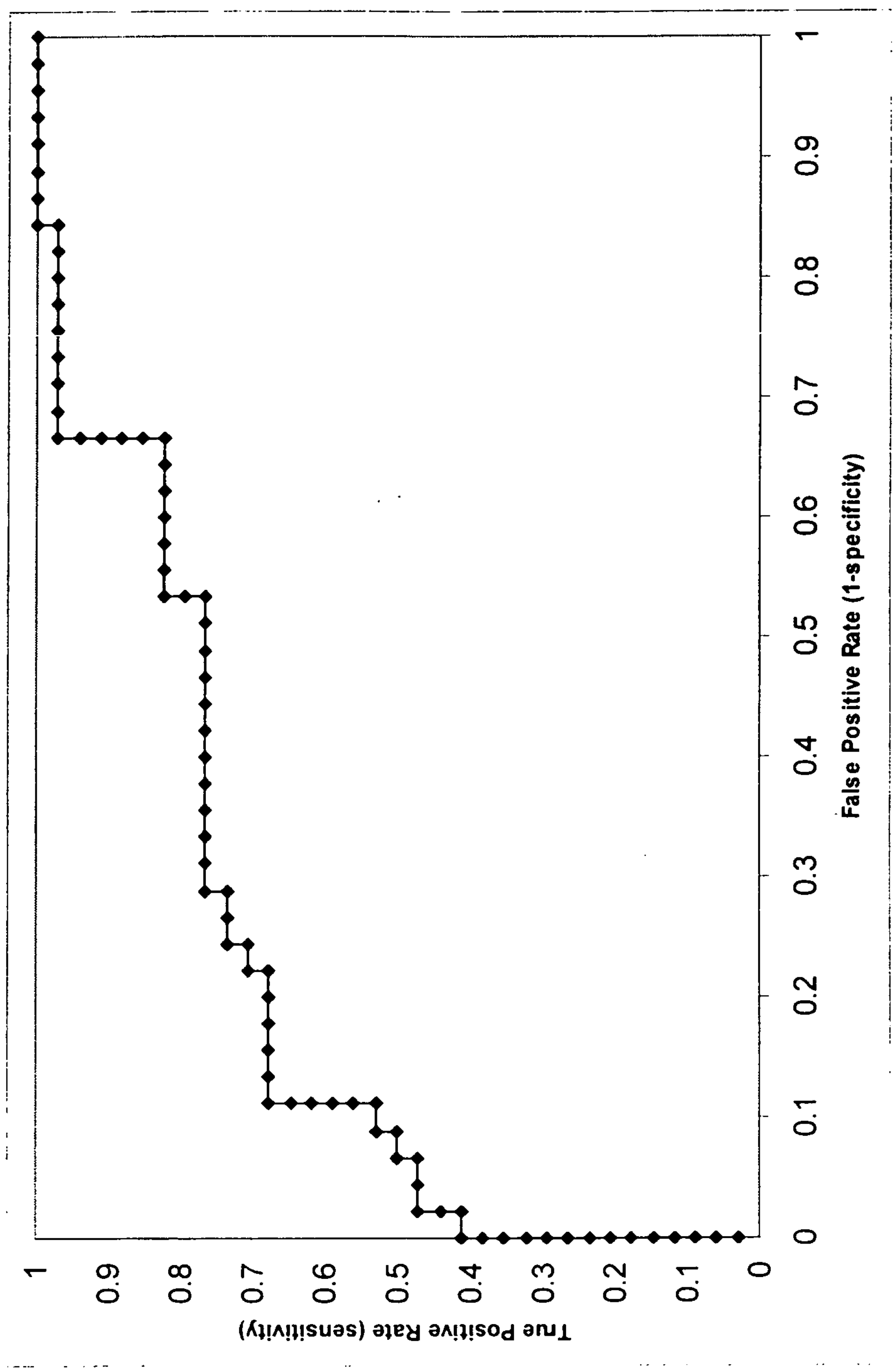
True Positive Rate (sensitivity)
False Positive Rate (1-specificity)
0
0.1
0.2
0.3
0.4
0.5
0.6
0.7
0.8
0.9
1

Figure 47

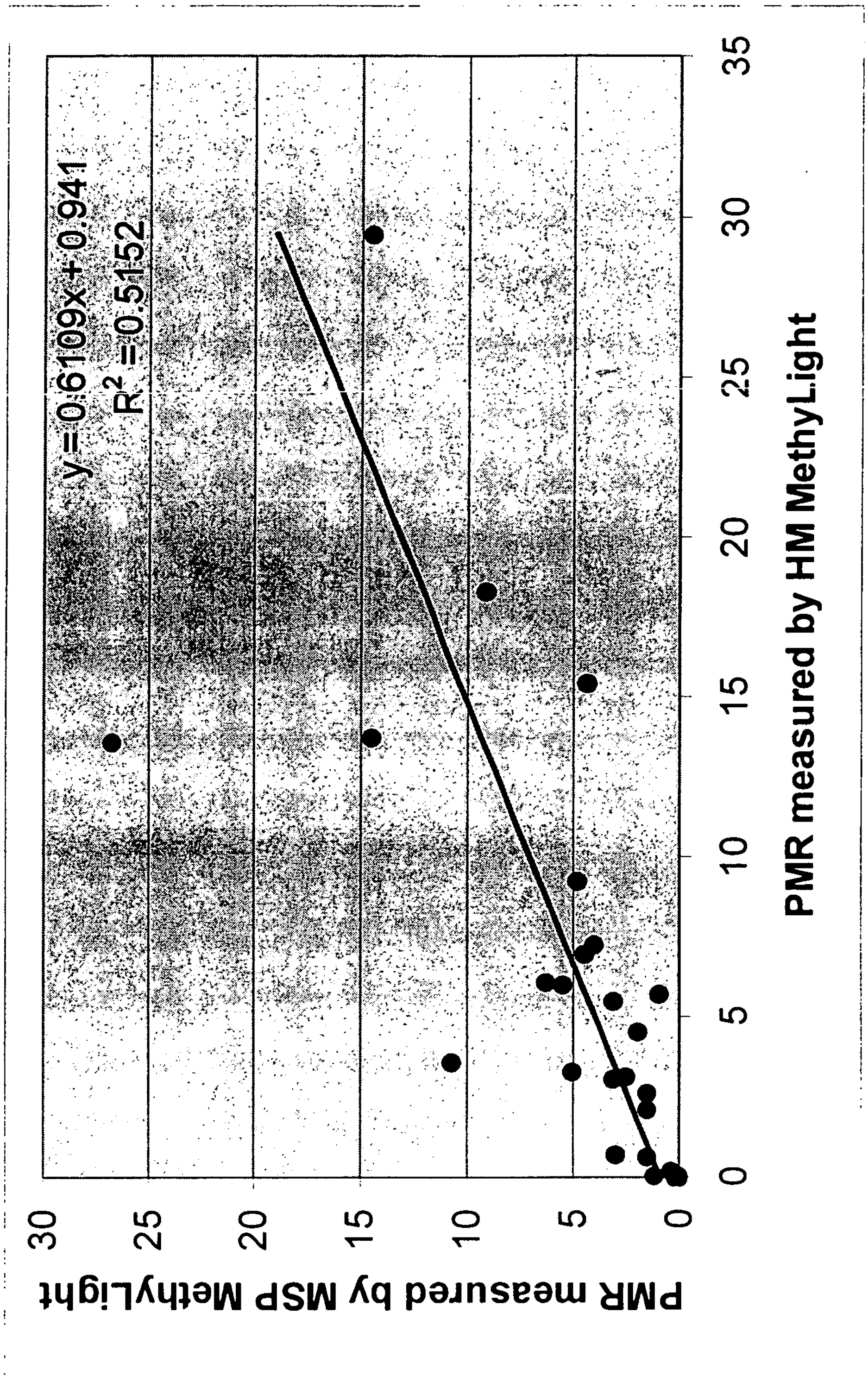
y = 0.6109x + 0.941
R2 = 0.5152
PMR measured by MSP MethyLight
PMR measured by HM MethyLight
0
5
10
15
20
25
30
35

Figure 48

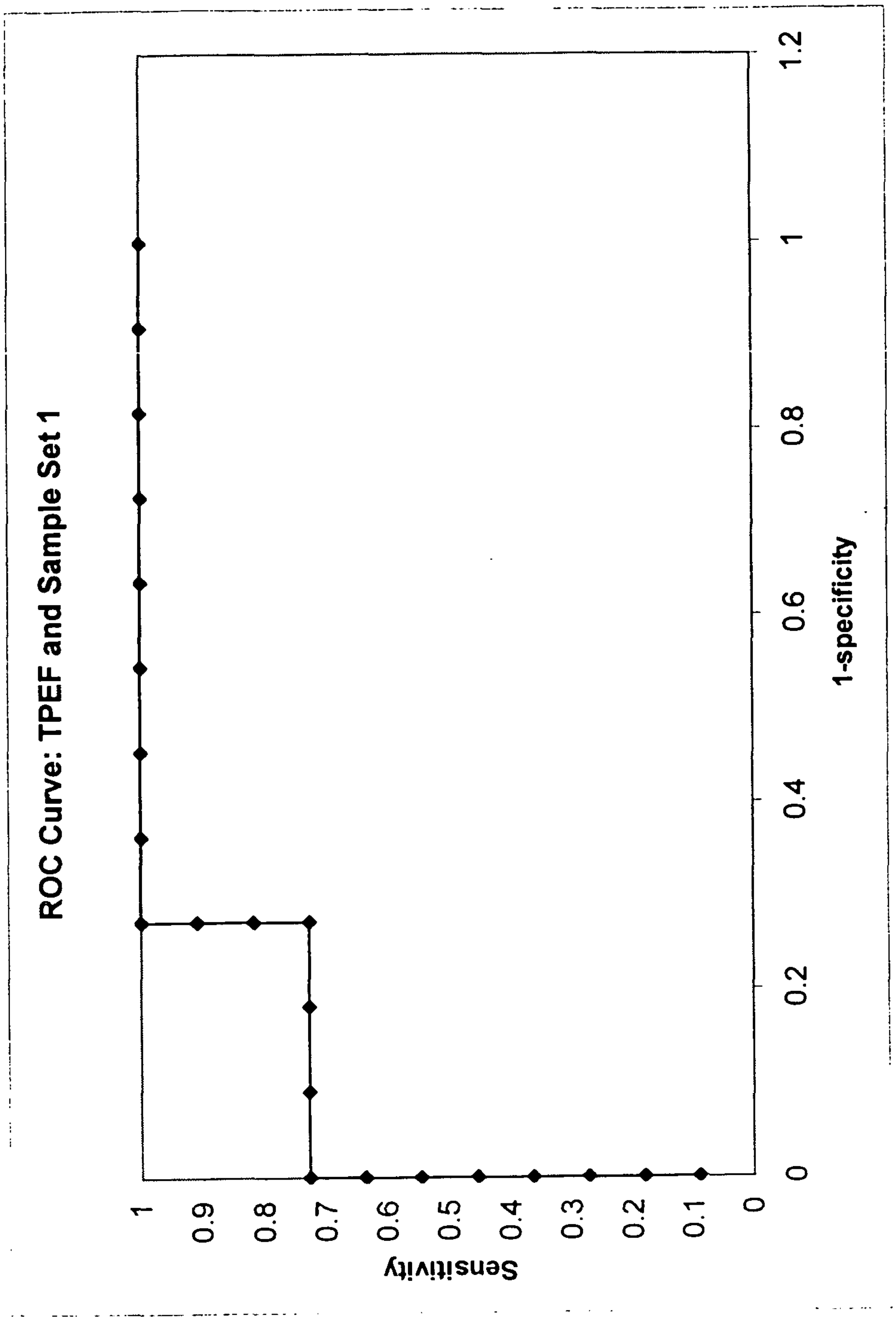
ROC Curve: TPEF and Sample Set 1
Sensitivity
0
0.1
0.2
0.3
0.4
0.5
0.6
0.7
0.8
0.9
1
1-specificity
0
0.2
0.4
0.6
0.8
1
1.2

Figure 49

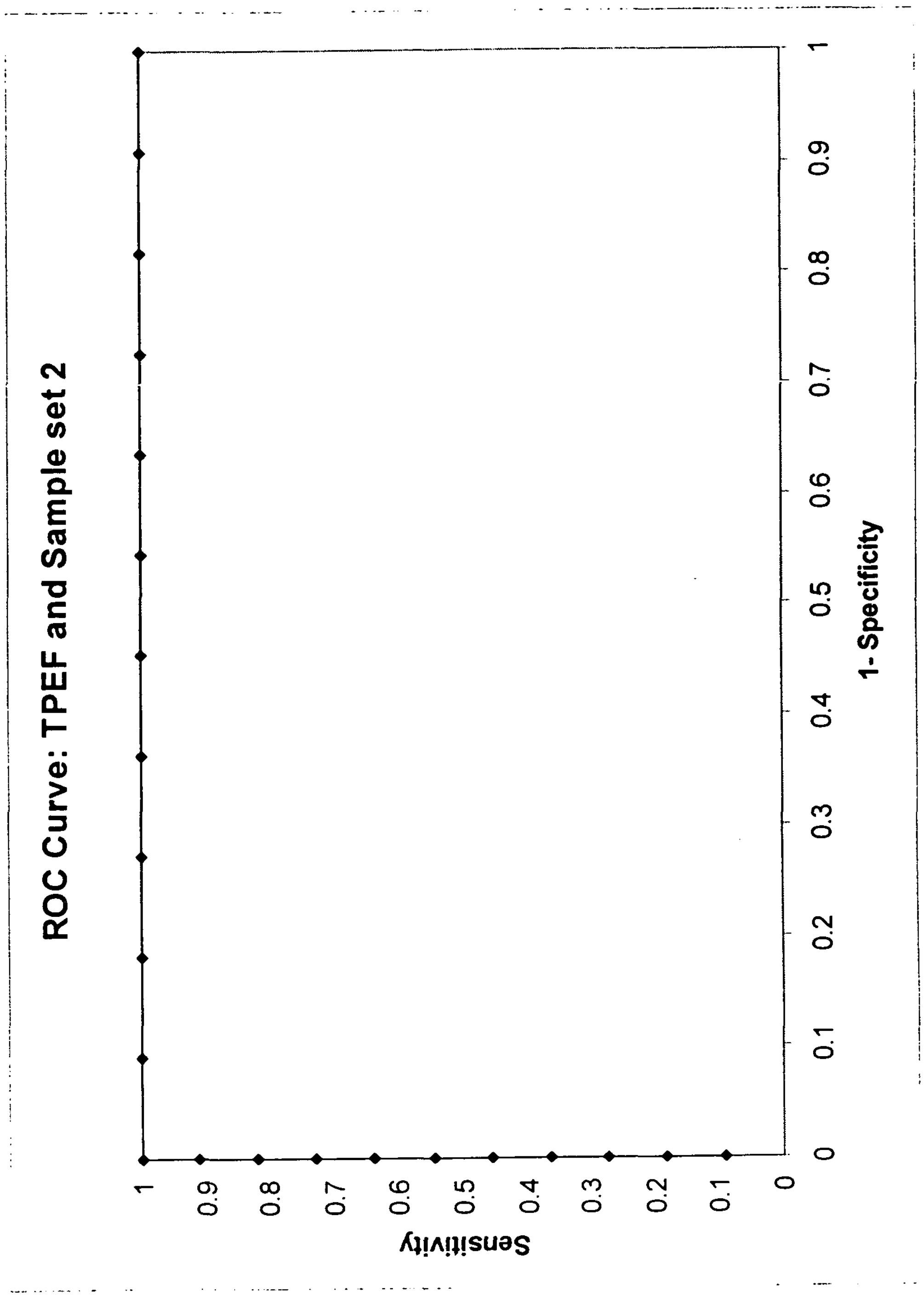
ROC Curve: TPEF and Sample set 2
Sensitivity
1
0.9
0.8
0.7
0.6
0.5
0.4
0.3
0.2
0.1
0
1- Specificity

Figure 50

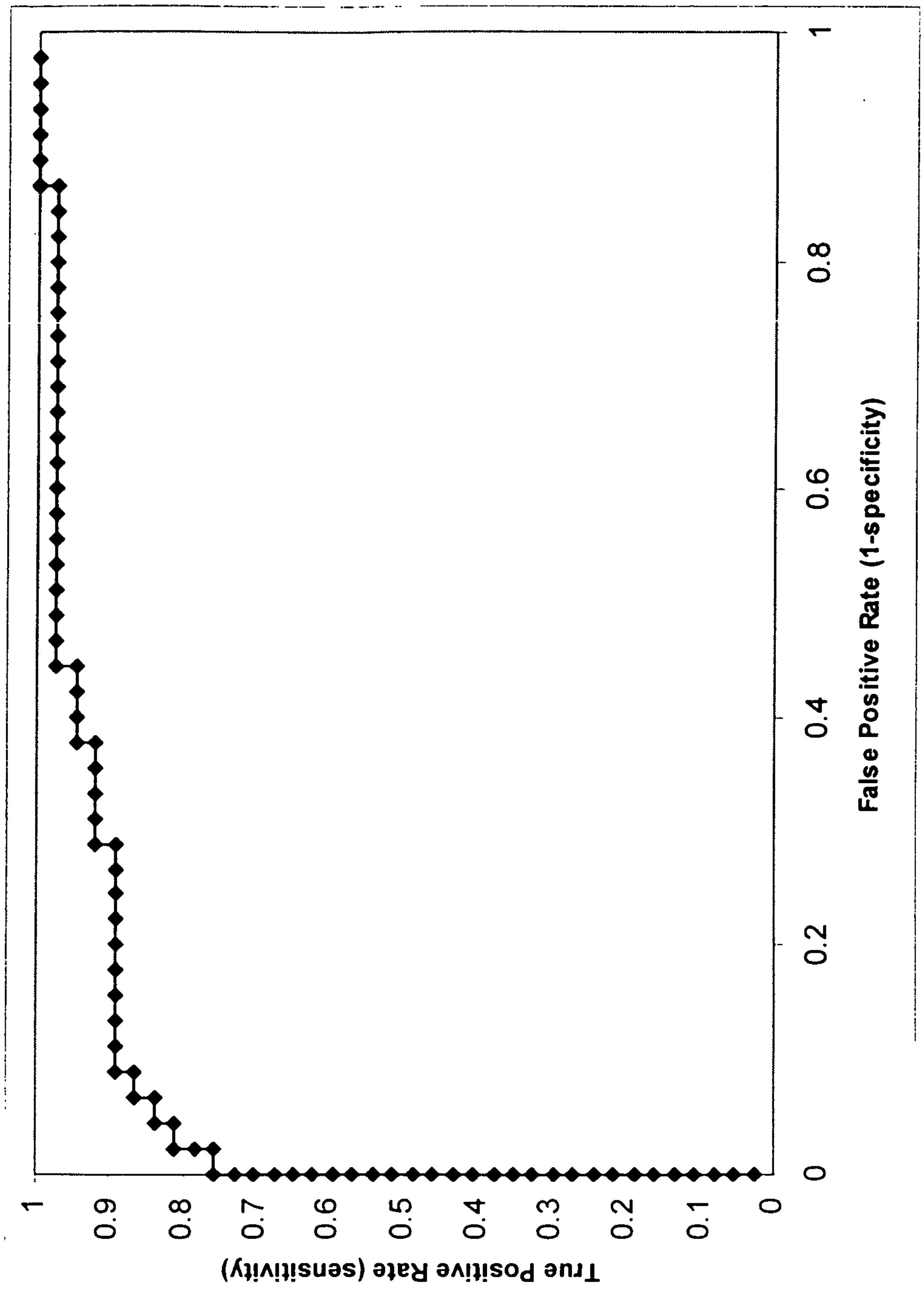
True Positive Rate (sensitivity)
1
0.9
0.8
0.7
0.6
0.5
0.4
0.3
0.2
0.1
0
0
0.2
0.4
0.6
0.8
1
False Positive Rate (1-specificity)

Figure 51

FIGURE 52

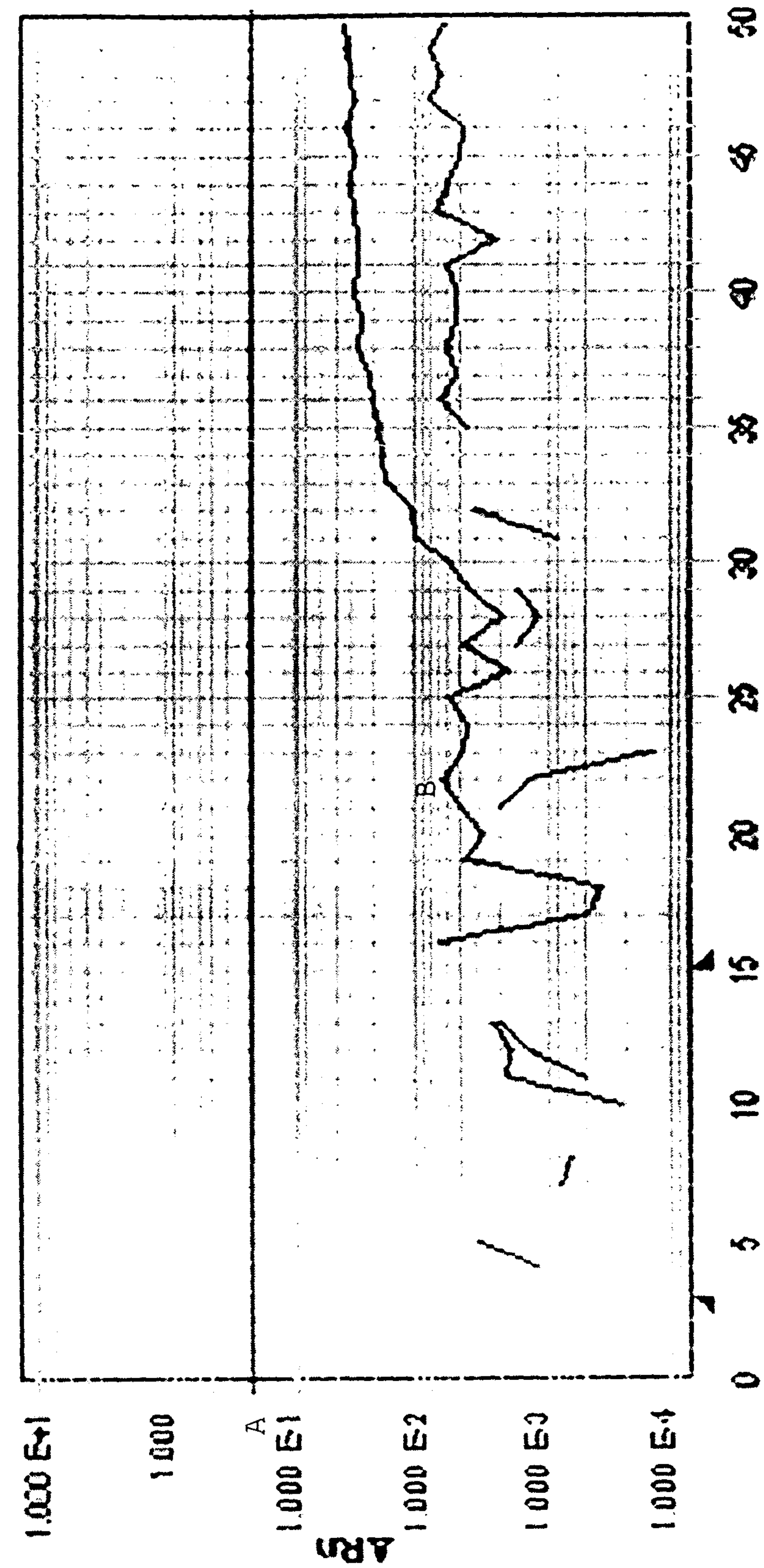

ARS
1.000 E+1
1.000
1.000 E-1
1.000 E-2
1.000 E-3
1.000 E-4
A
B
0
5
10
15
20
25
30
35
40
45
50

FIGURE 53

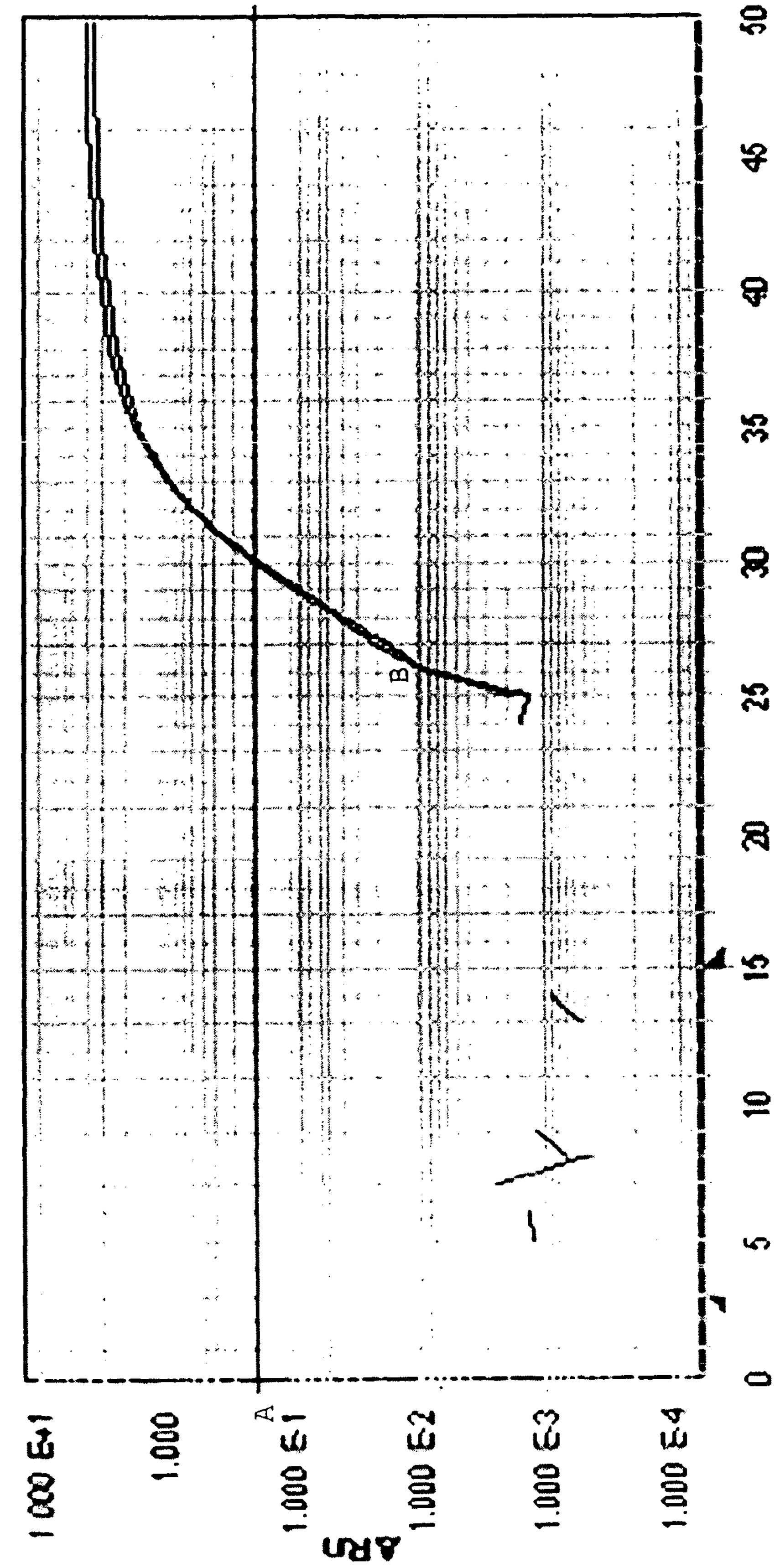
1.000 E+1
1.000
1.000 E-1
1.000 E-2
1.000 E-3
1.000 E-4
ΔRn
A
B
0
5
10
15
20
25
30
35
40
45
50

FIGURE 54

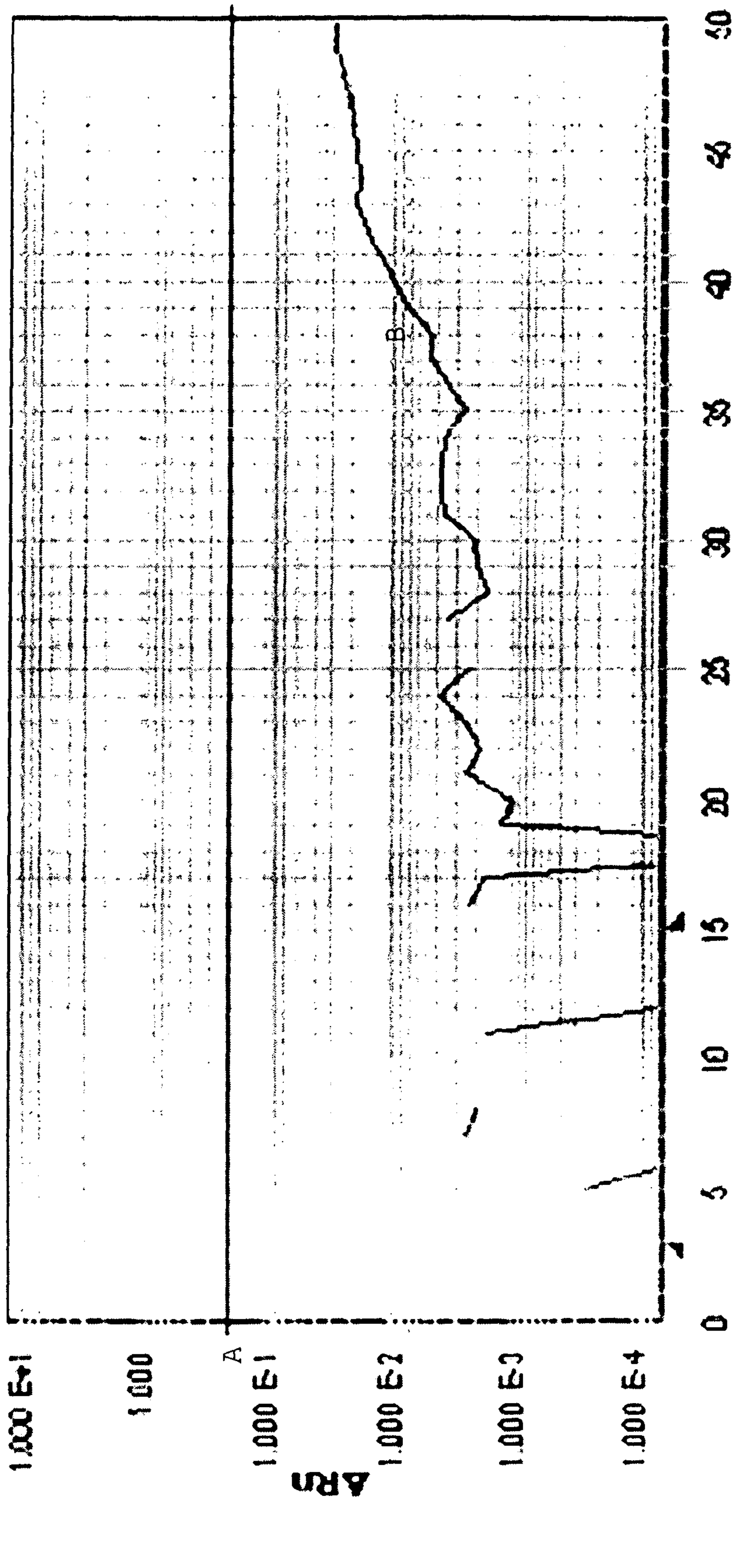
ΔRn
1.000 E+1
1.000
1.000 E-1
1.000 E-2
1.000 E-3
1.000 E-4
A
B
0
5
10
15
20
25
30
35
40
45
50

FIGURE 55

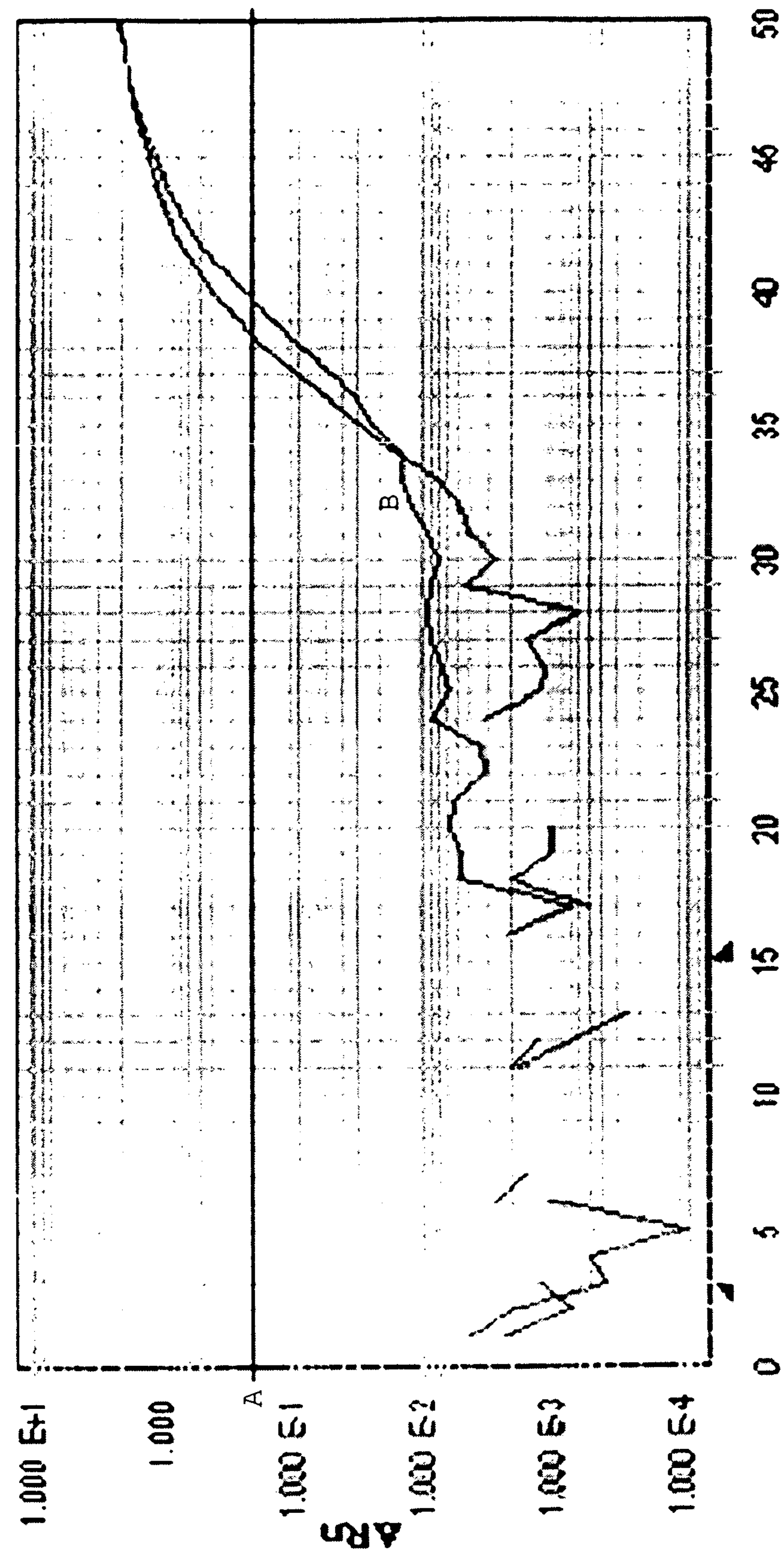
A
B
ΔRn
1.000 E+1
1.000
1.000 E-1
1.000 E-2
1.000 E-3
1.000 E-4
0
5
10
15
20
25
30
35
40
45
50

FIGURE 56

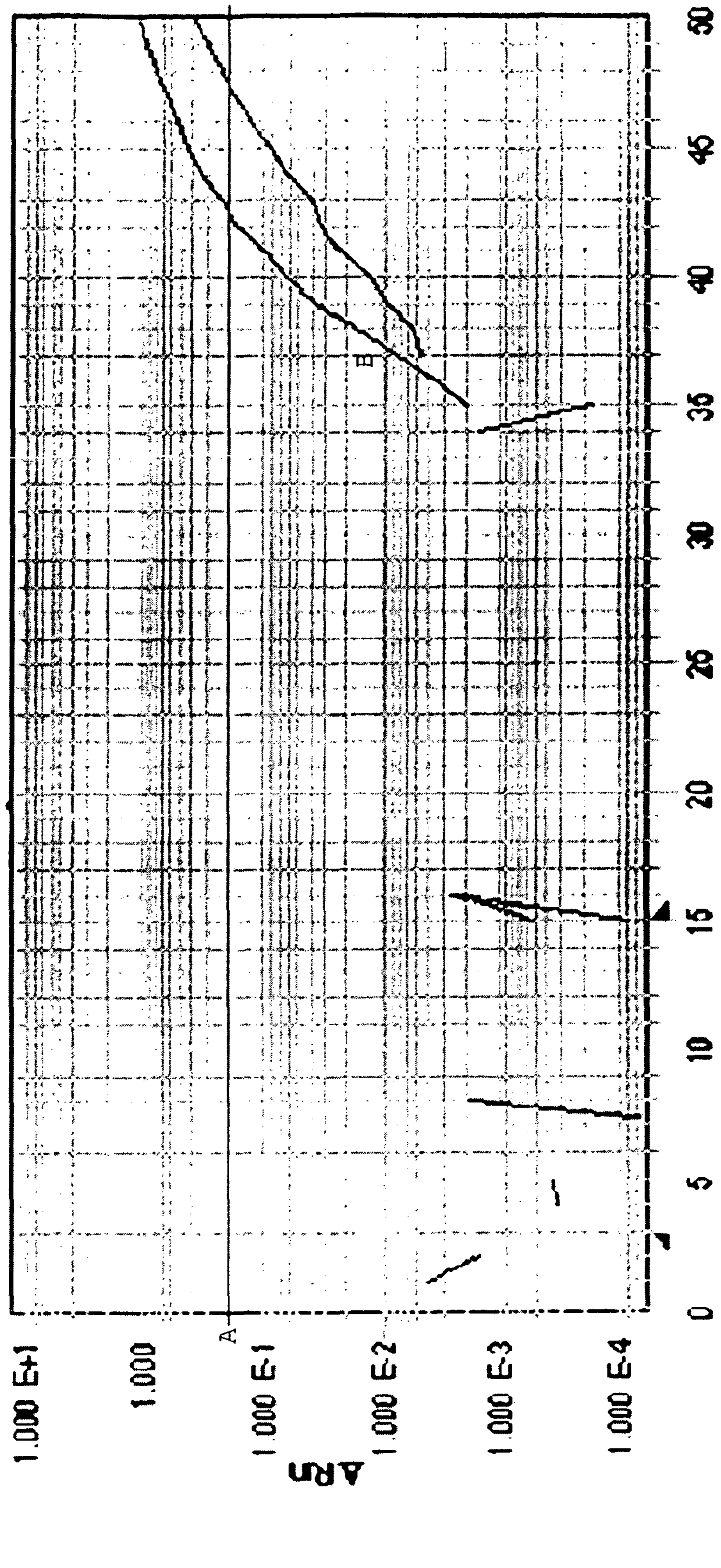
ΔRn
1.000 E+1
1.000
1.000 E-1
1.000 E-2
1.000 E-3
1.000 E-4
A
B
0
5
10
15
20
25
30
35
40
45
50

FIGURE 57

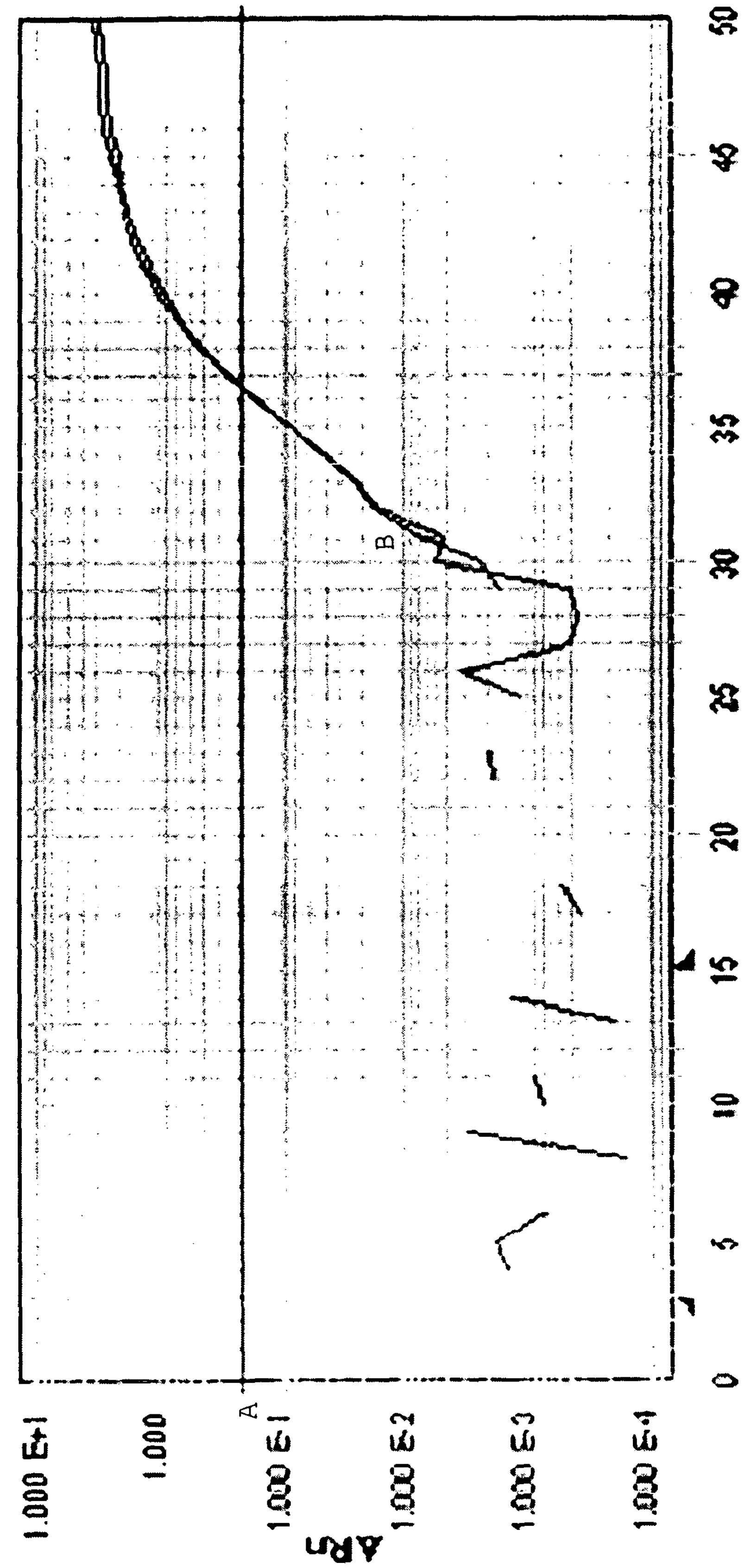
1.000 E+1
1.000
1.000 E-1
1.000 E-2
1.000 E-3
1.000 E-4
ΔRn
A
B
0
5
10
15
20
25
30
35
40
45
50

FIGURE 58

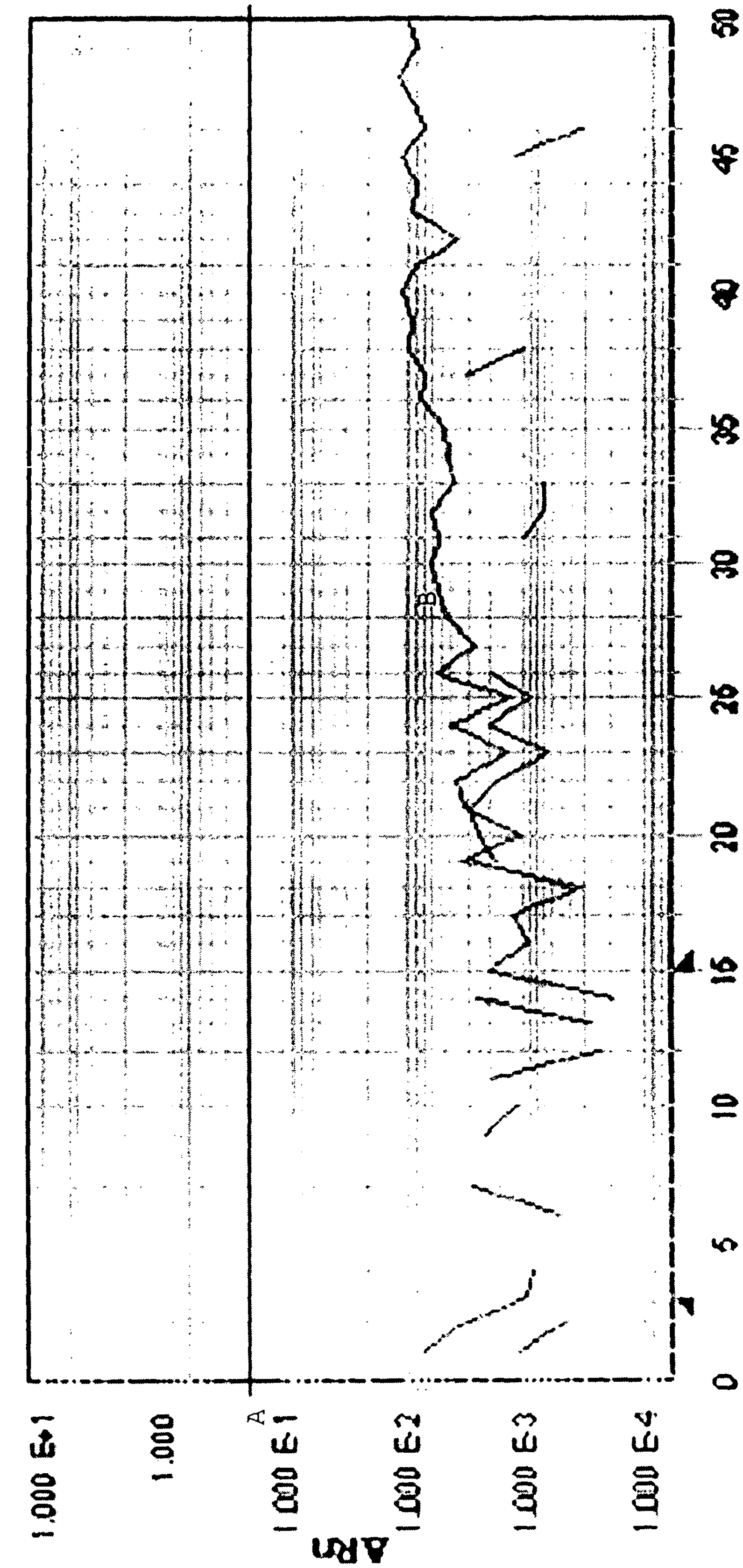
1.000 E+1
1.000
1.000 E-1
1.000 E-2
1.000 E-3
1.000 E-4
ARn
A
B
0
5
10
15
20
25
30
35
40
45
50

Figure 59

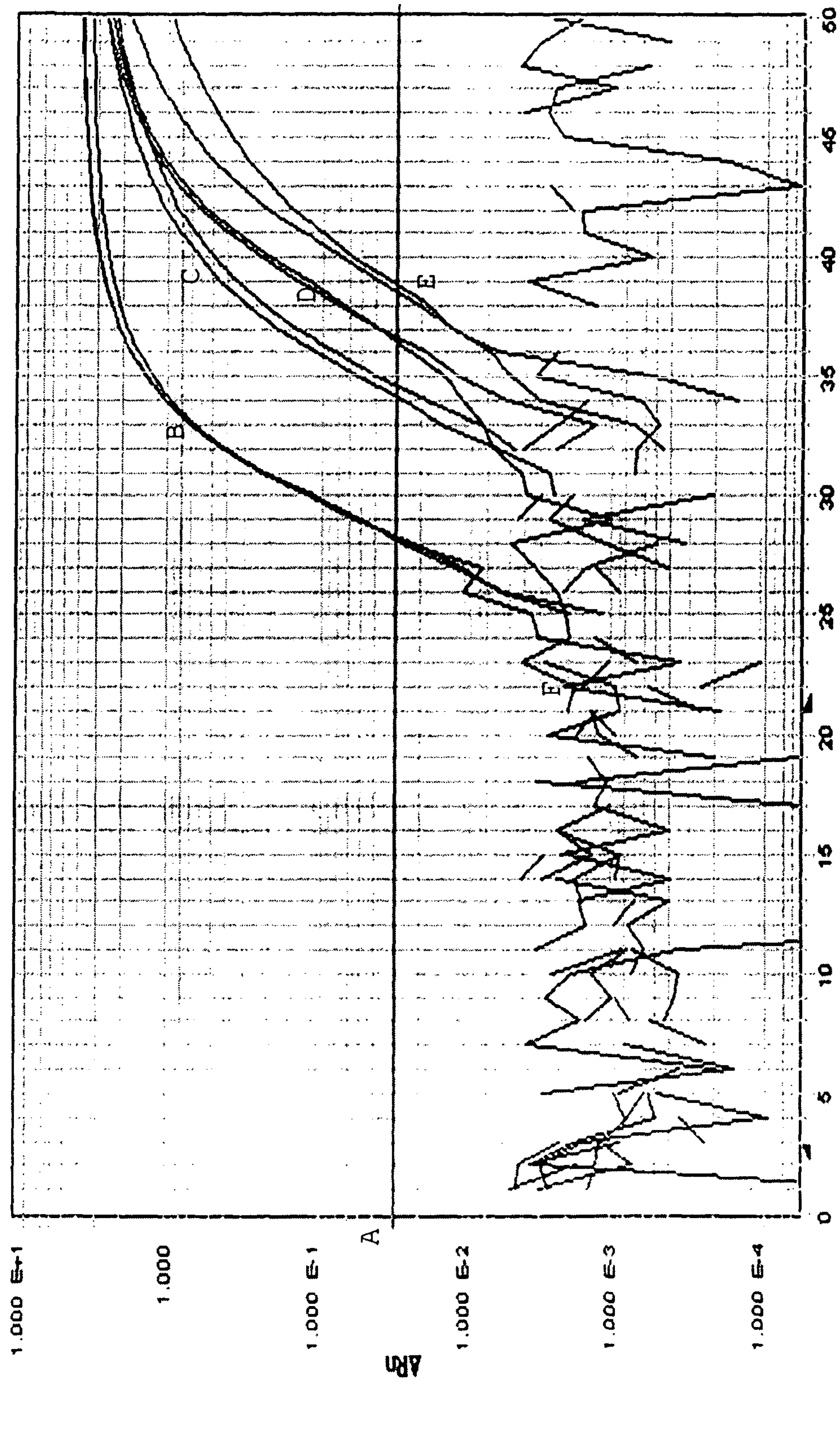
A
B
C
D
E
E
0
5
10
15
20
25
30
35
40
45
50
1.000 E+1
1.000
1.000 E-1
1.000 E-2
1.000 E-3
1.000 E-4
ΔRn

Figure 60

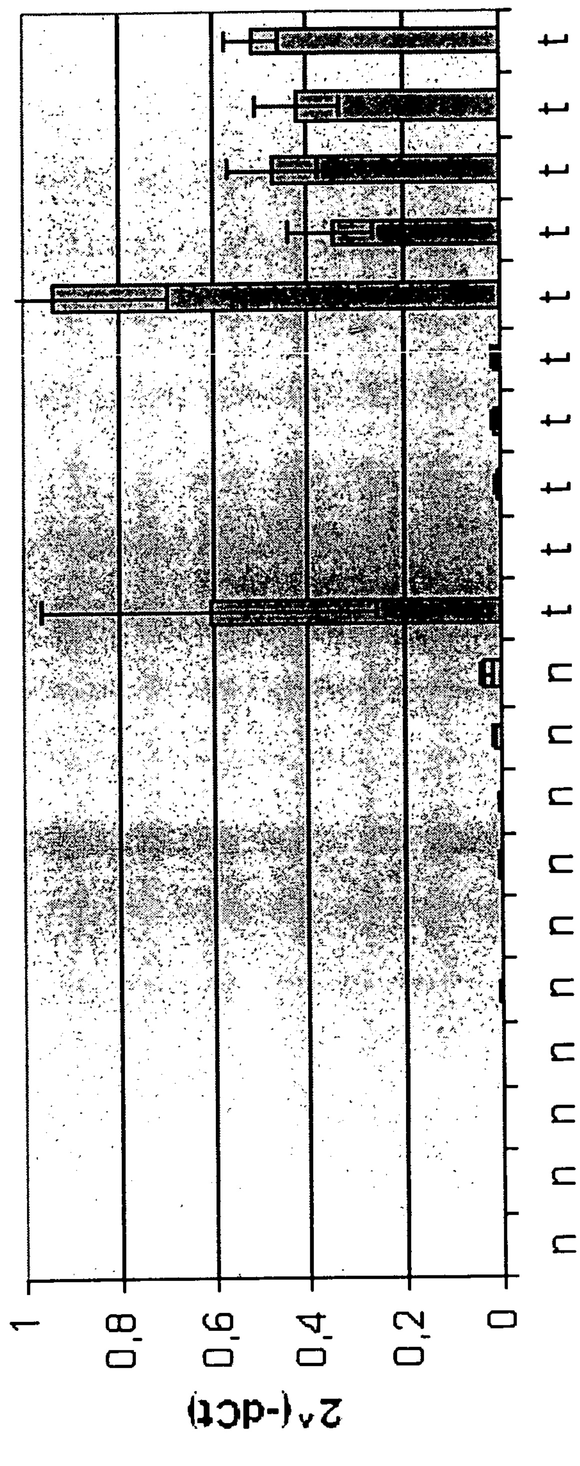

Figure 61

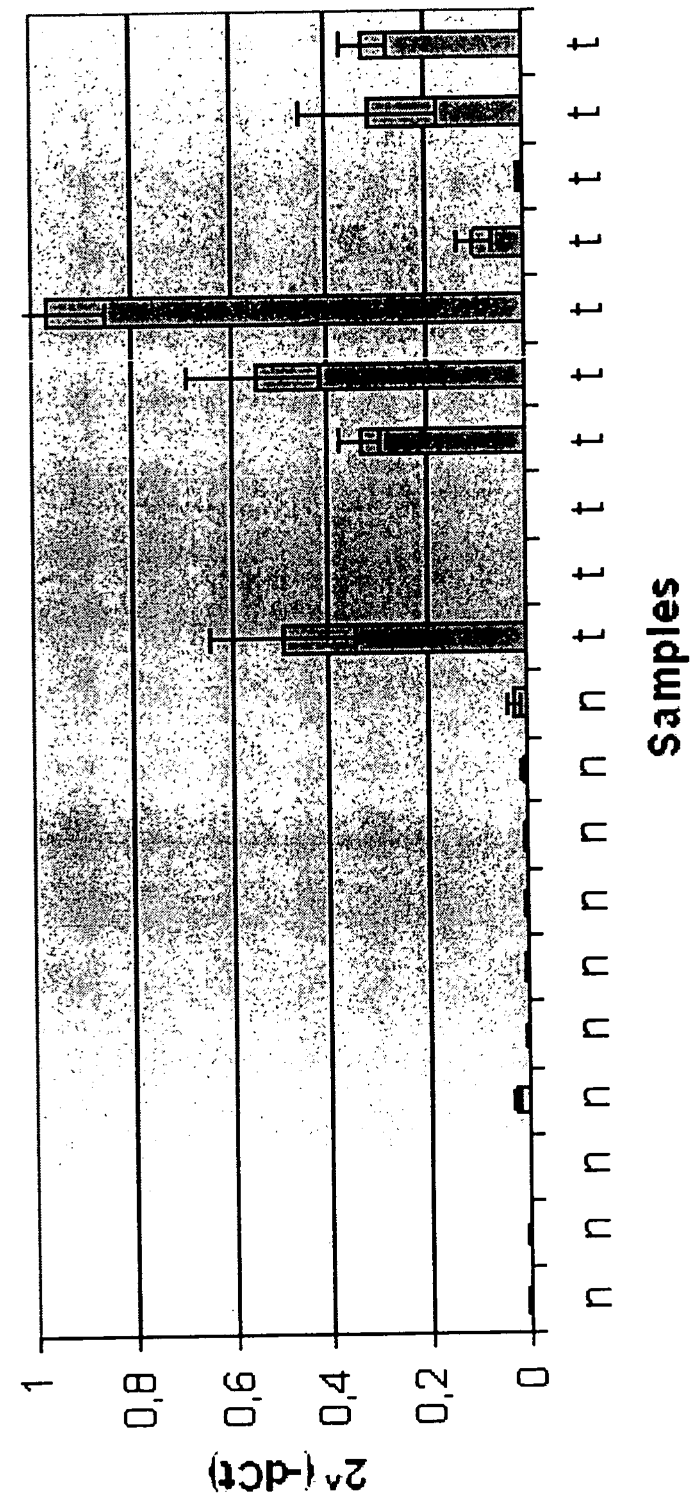

Figure 62

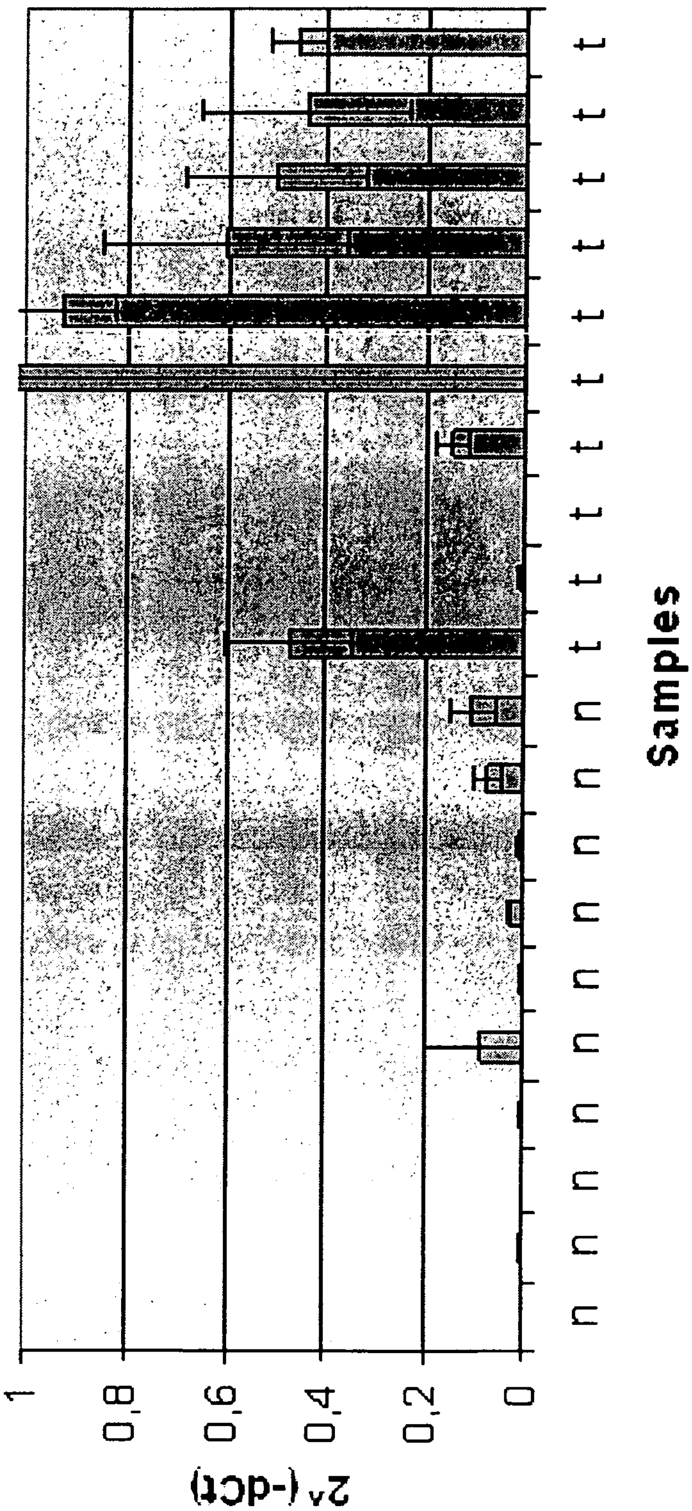

Figure 63

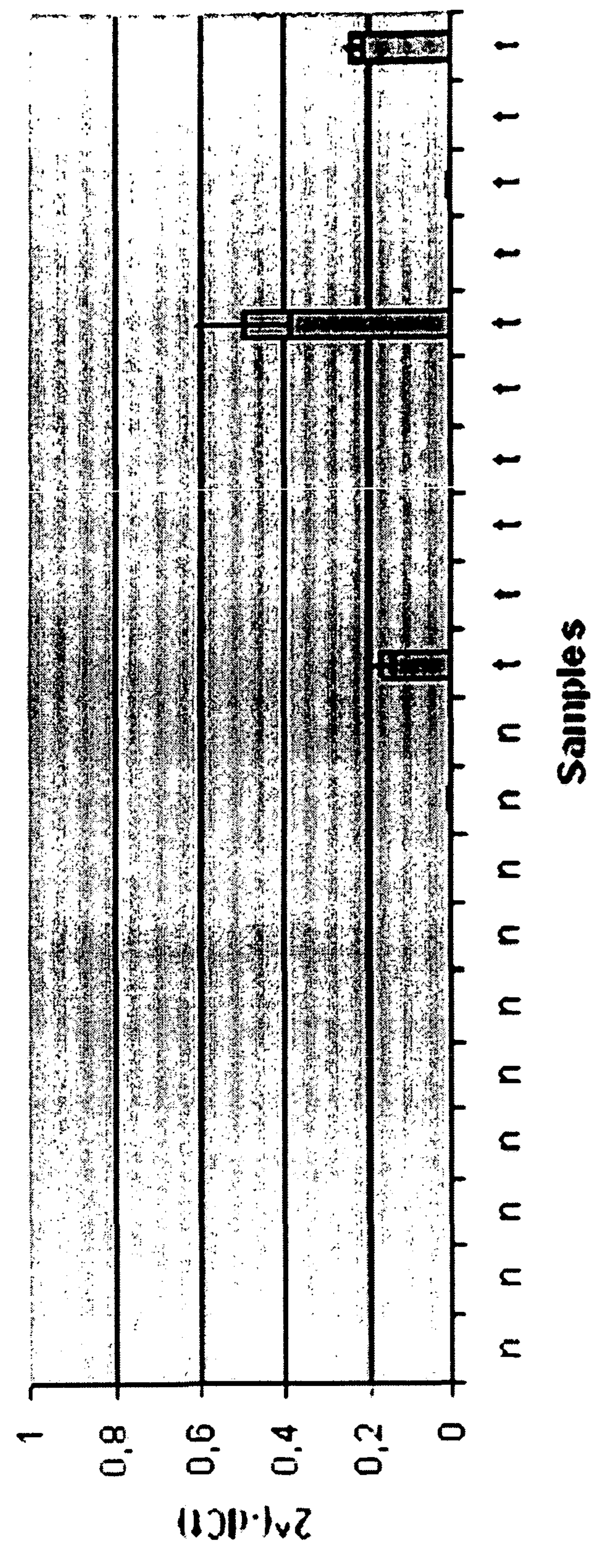
1
0,8
0,6
0,4
0,2
0
Samples
n n n n n n n n n n n n t t t t t t t t t t t t t t t t t t t t t

Figure 64

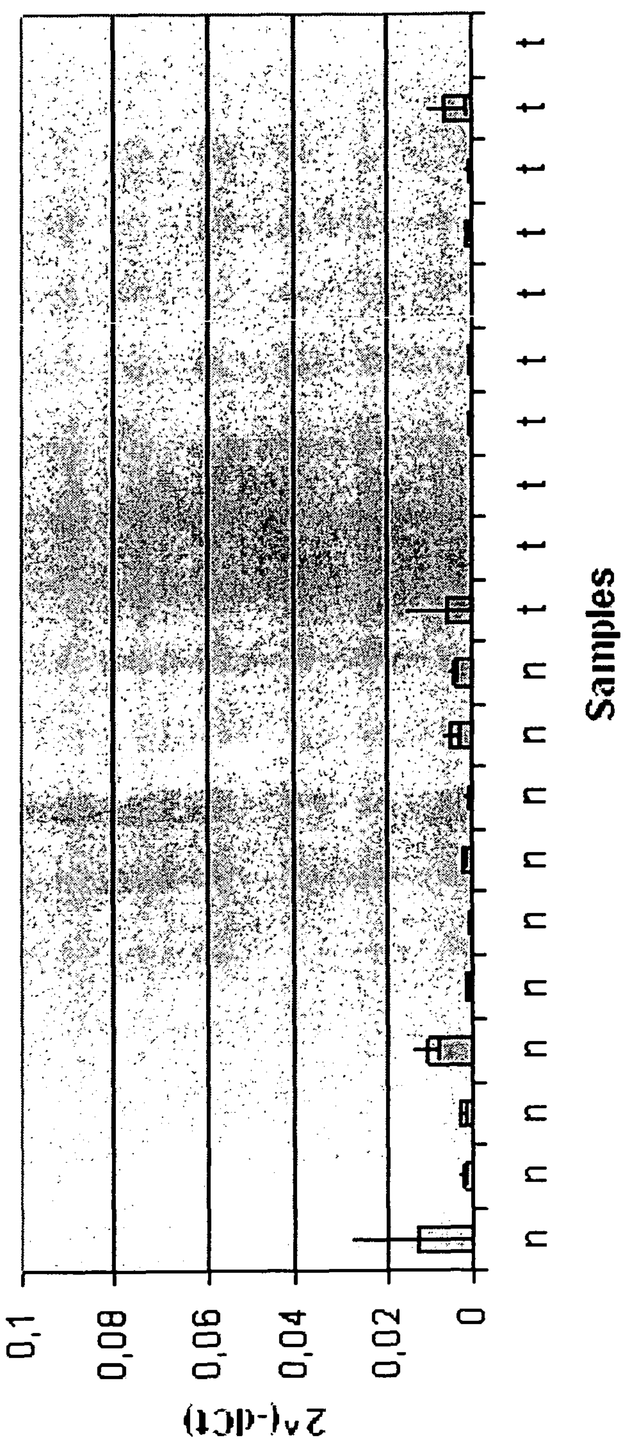

Figure 65

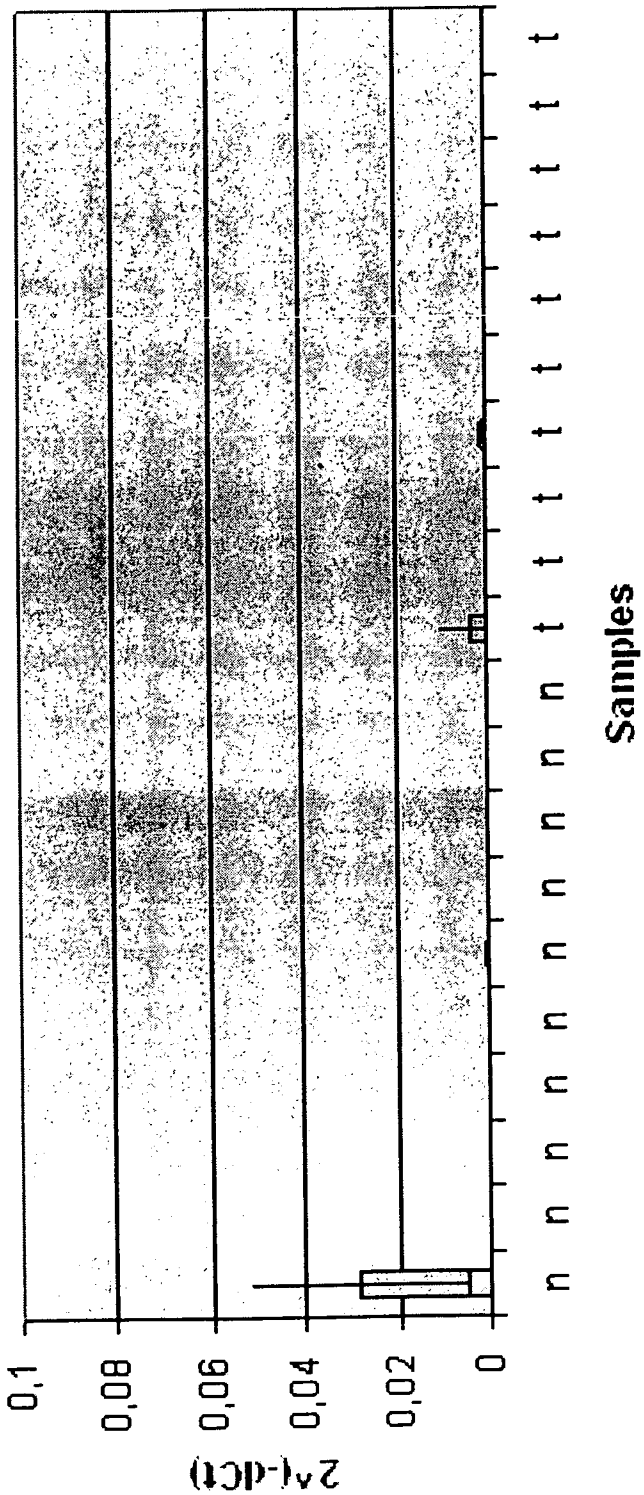

Figure 66

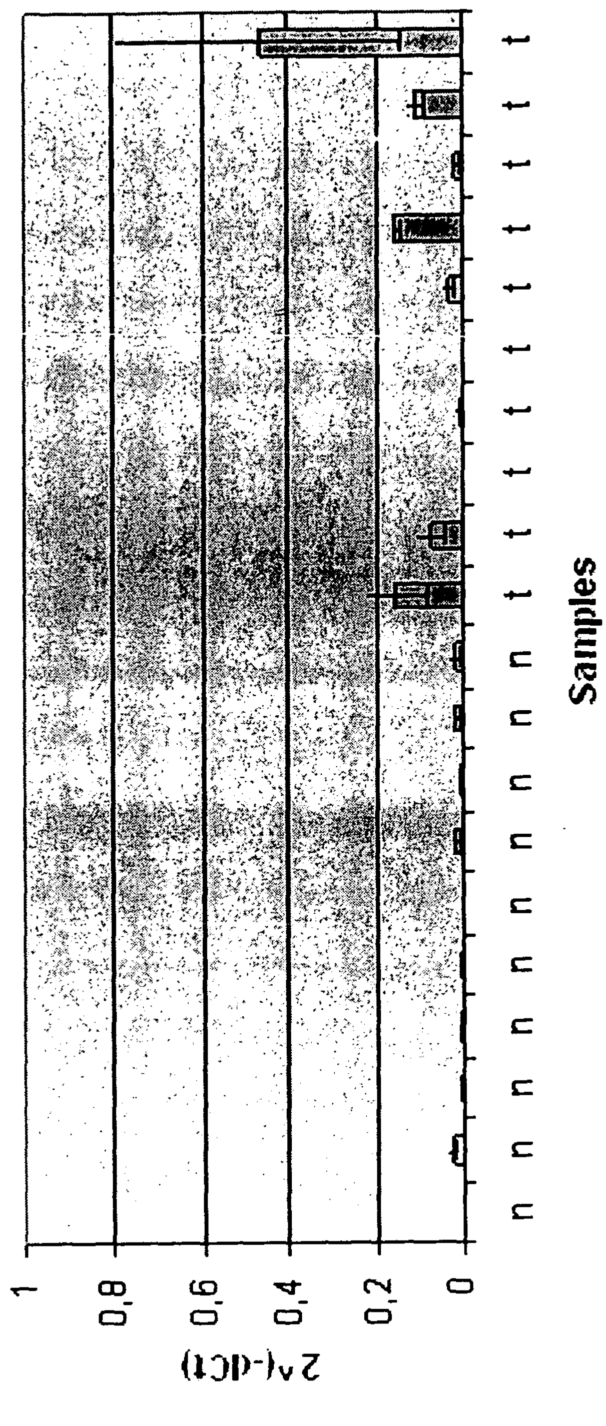

Figure 67

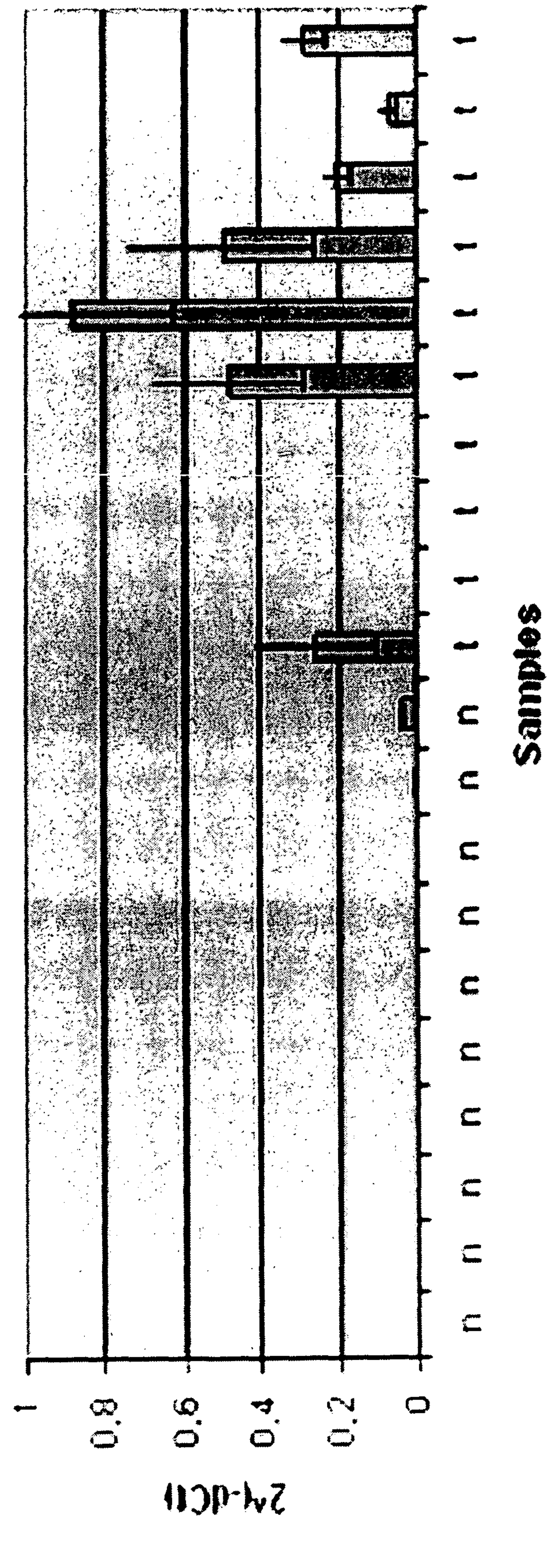

Figure 68

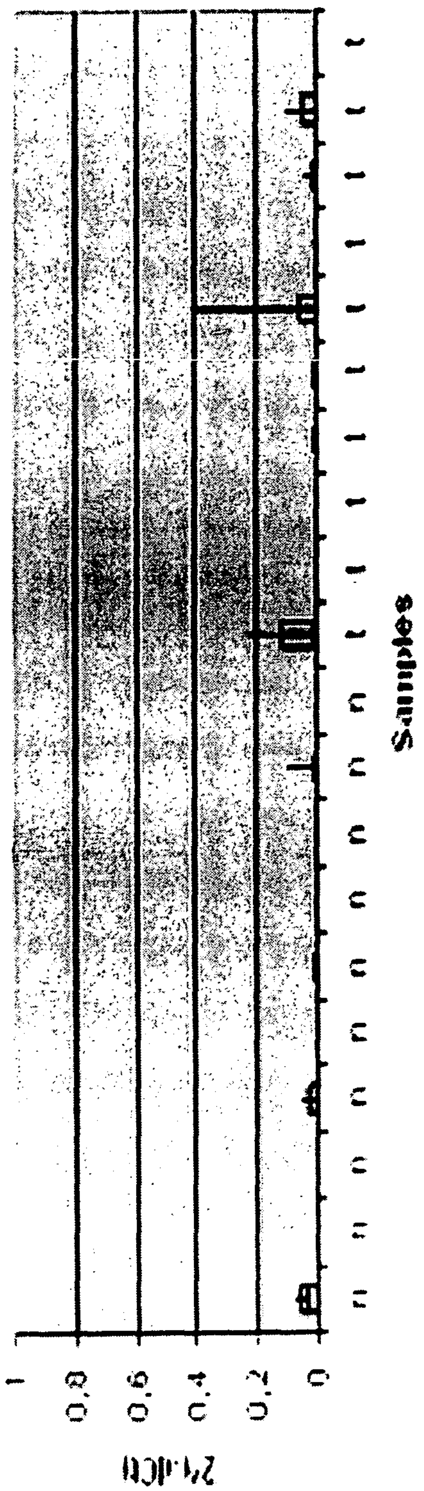

Figure 69

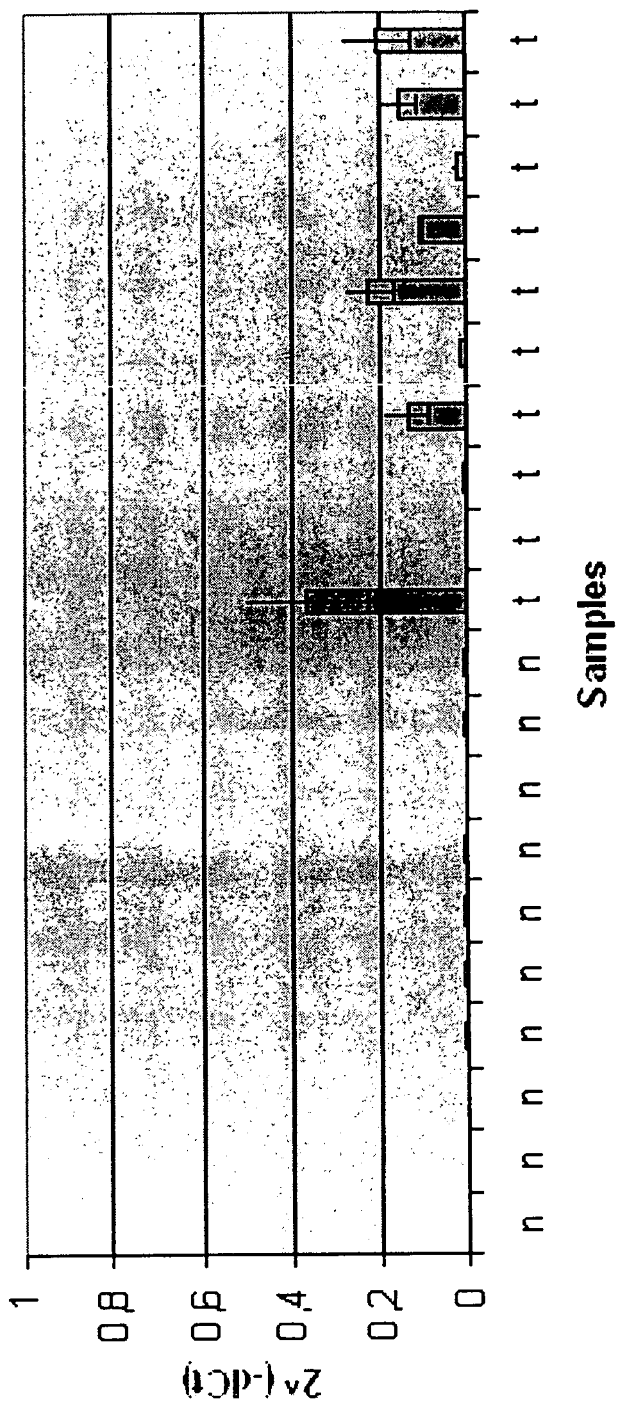

Figure 70

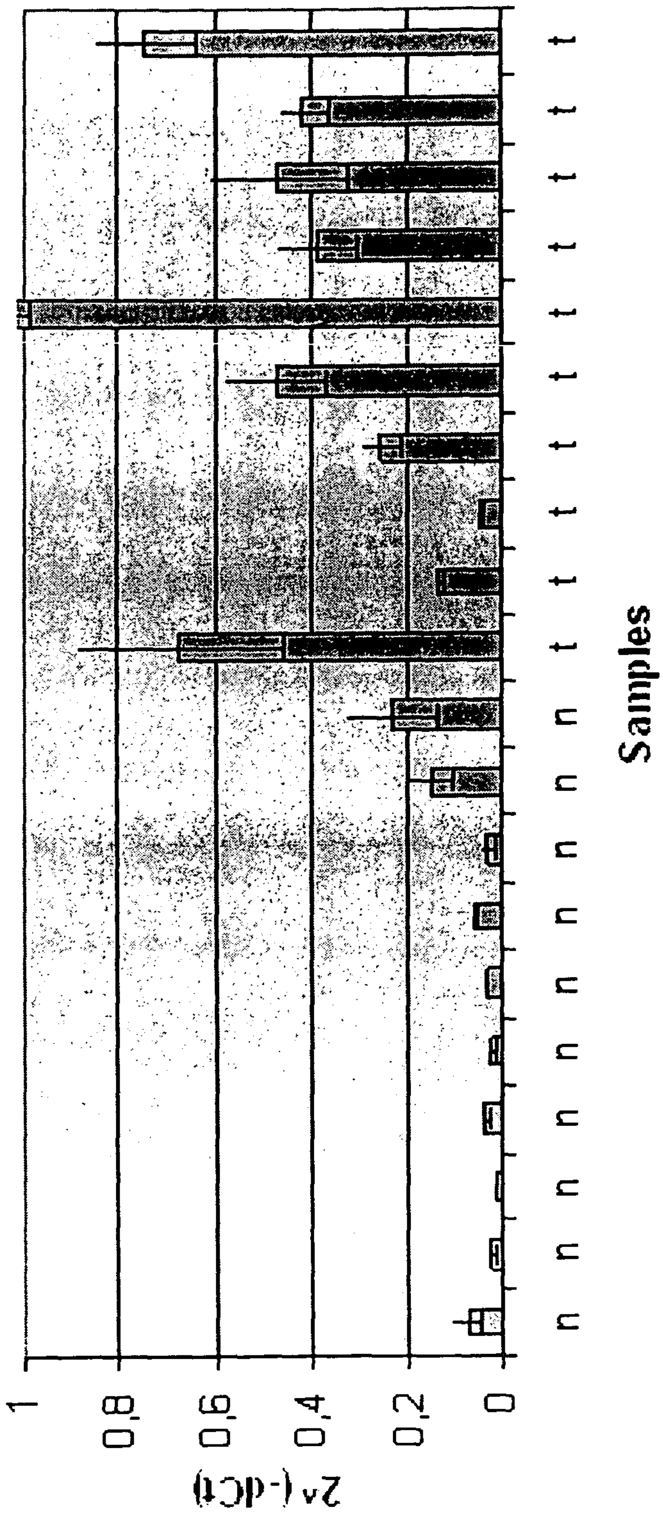

Figure 71

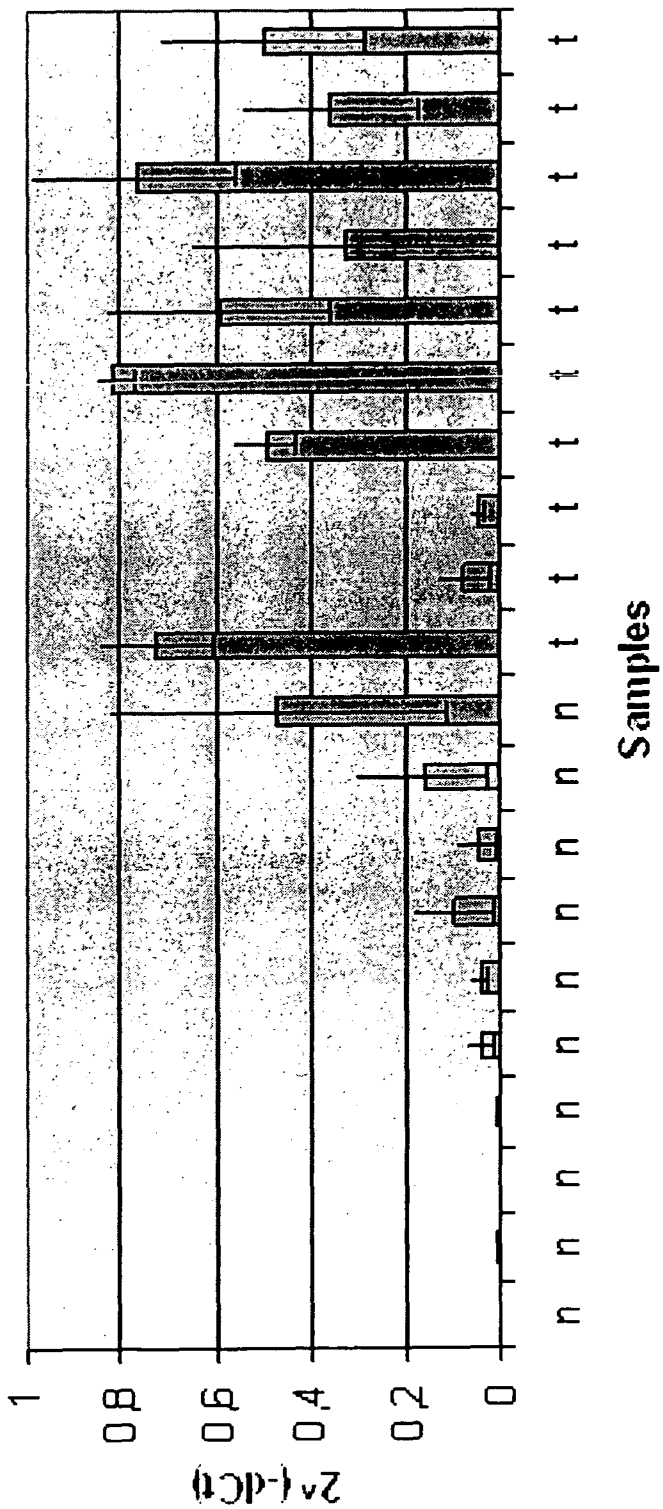

Figure 72

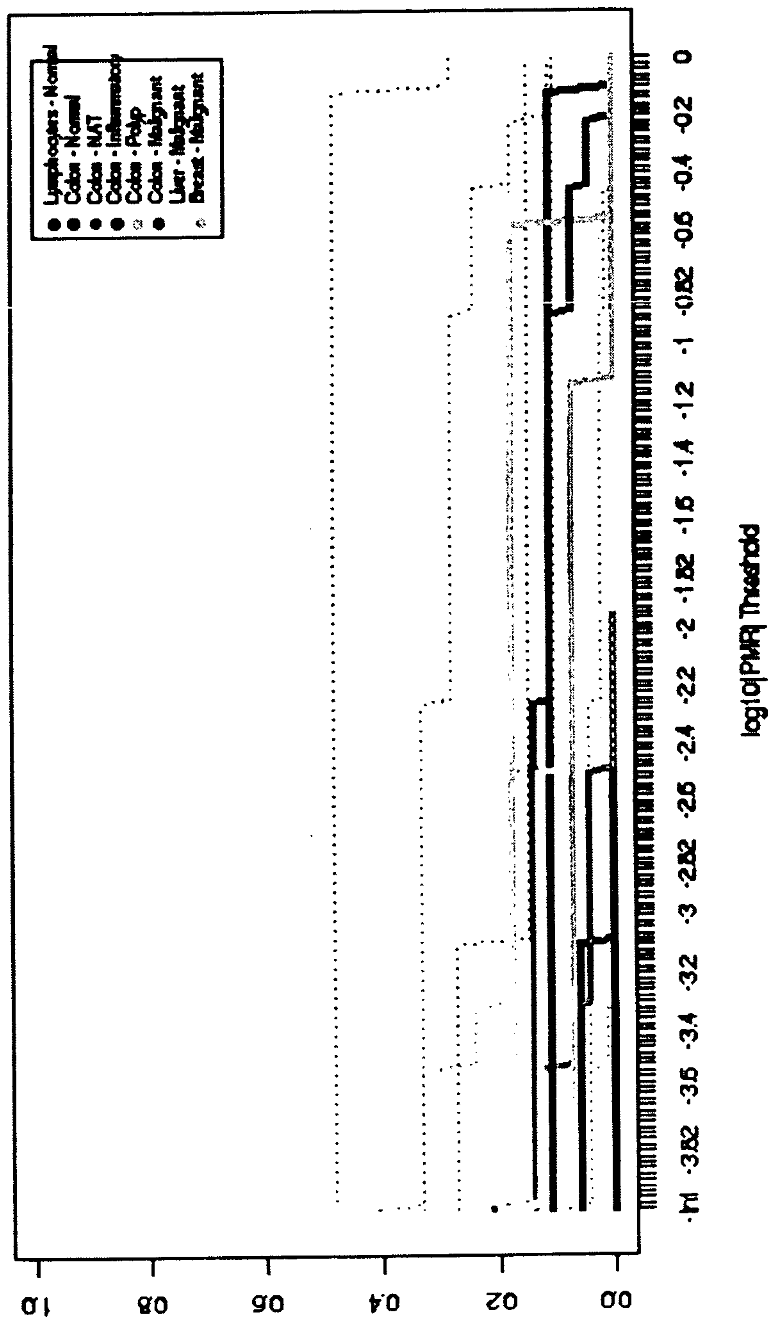
Lymphocytes - Normal
Colon - Normal
Colon - NAT
Colon - Inflammatory
Colon - Polyp
Colon - Malignant
Liver - Malignant
Breast - Malignant
log10(PMR) Threshold
0.0
0.2
0.4
0.6
0.8
1.0

Figure 73

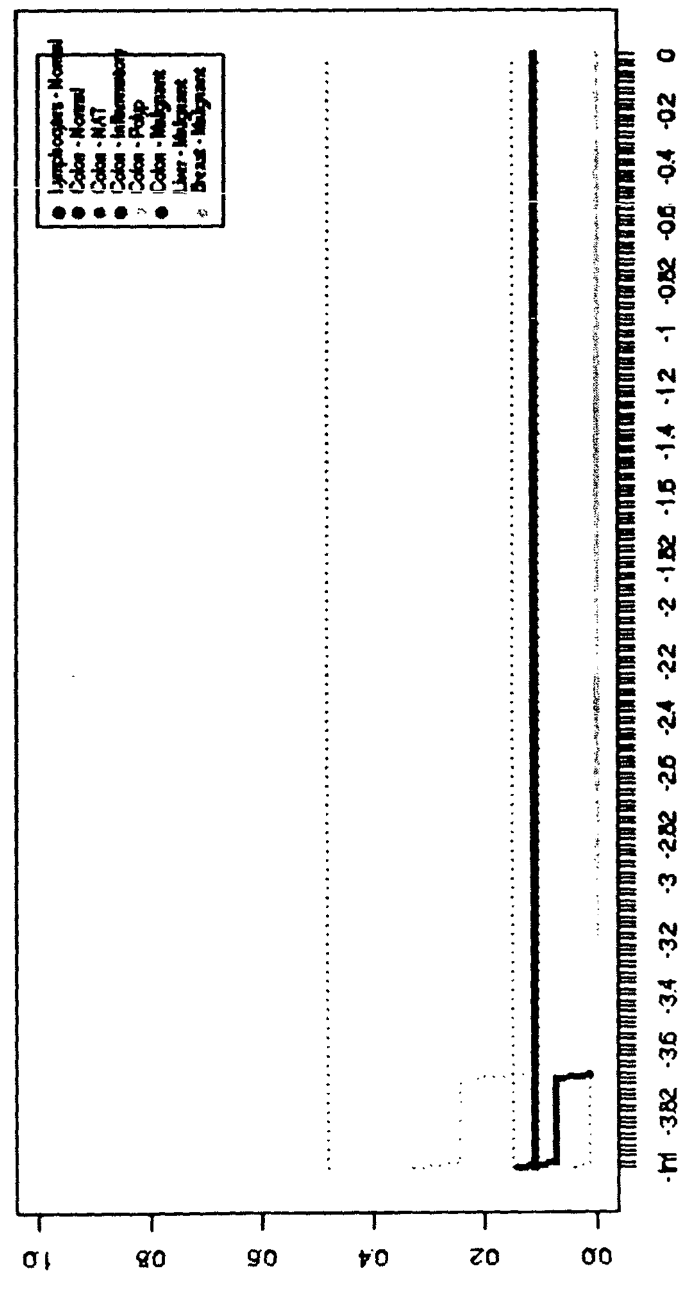
Lymphocytes - Normal
Colon - Normal
Colon - NAT
Colon - Inflammatory
Colon - Polyp
Colon - Malignant
Liver - Malignant
Breast - Malignant

Figure 74

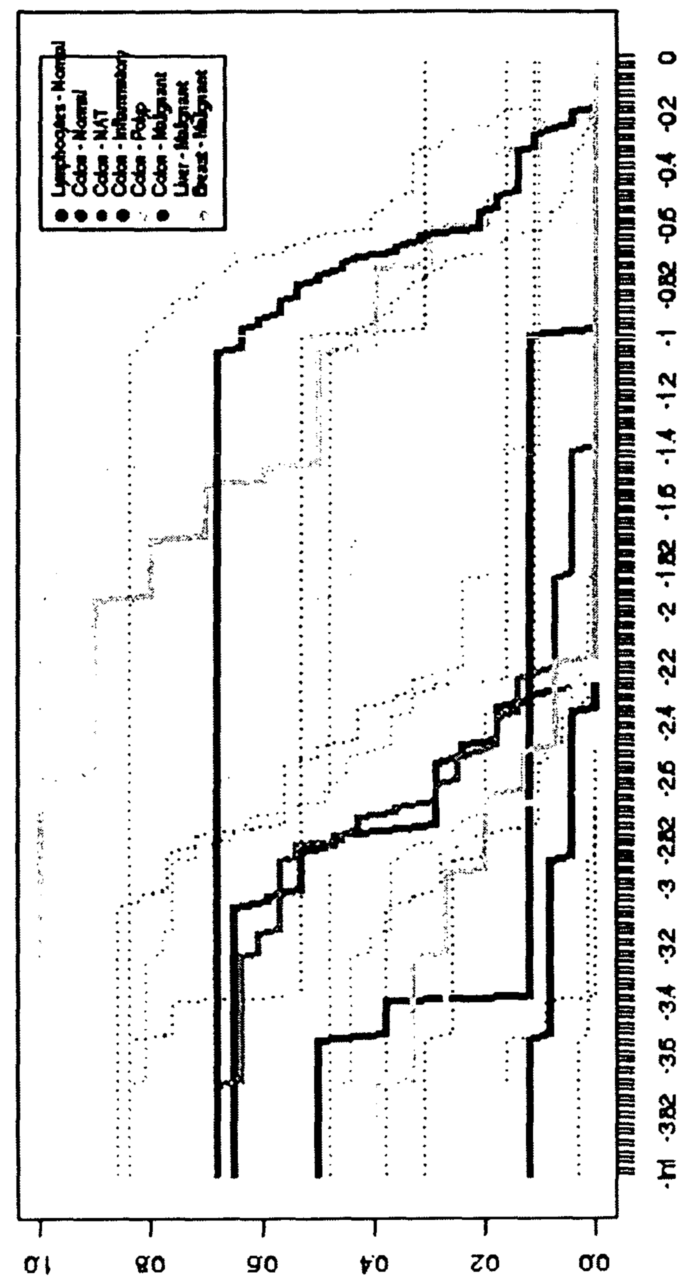
Lymphocytes - Normal
Colon - Normal
Colon - NAT
Colon - Inflammatory
Colon - Polyp
Colon - Malignant
Liver - Malignant
Breast - Malignant

Figure 75

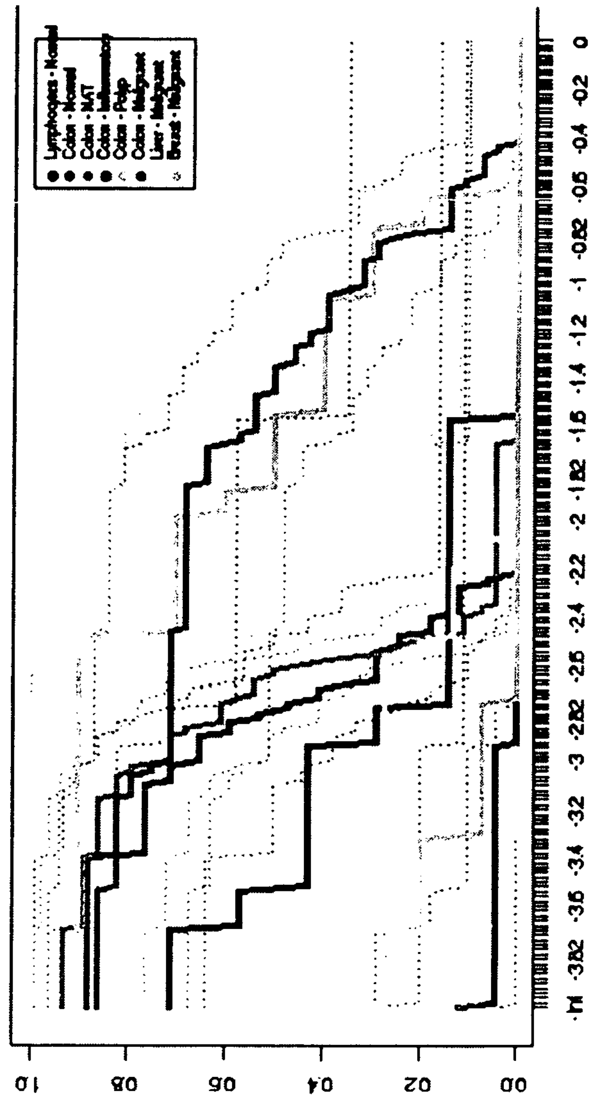

Figure 76

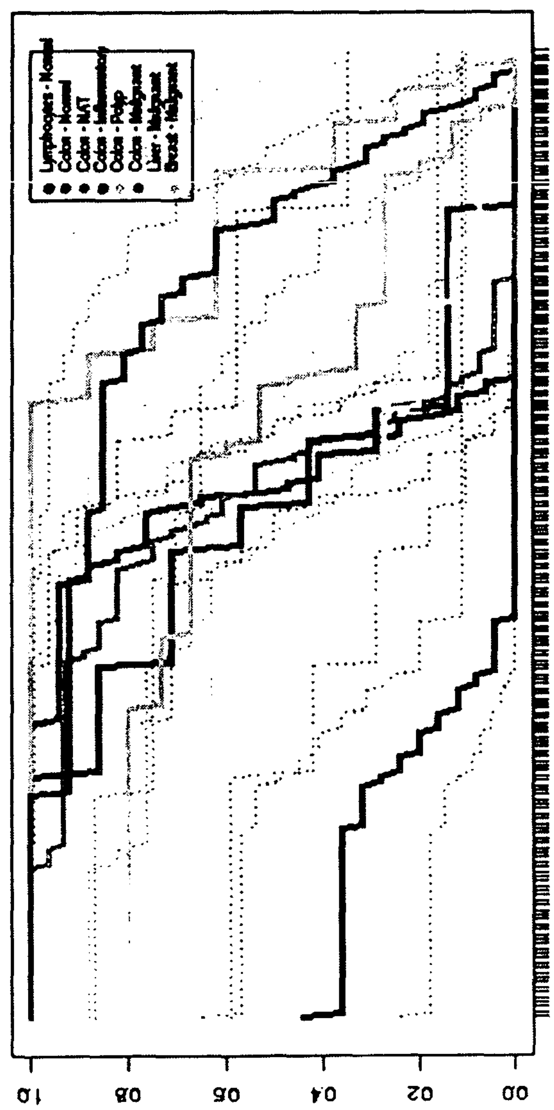

Figure 77

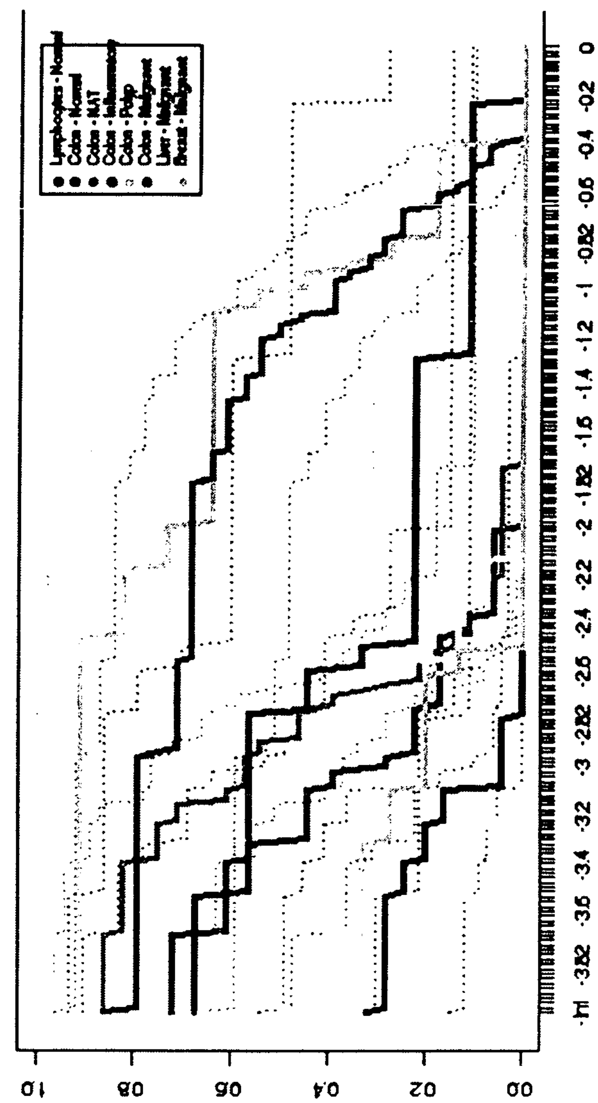
Lymphocytes - Normal
Colon - Normal
Colon - NAT
Colon - Inflammatory
Colon - Polyp
Colon - Malignant
Liver - Malignant
Breast - Malignant

Figure 78

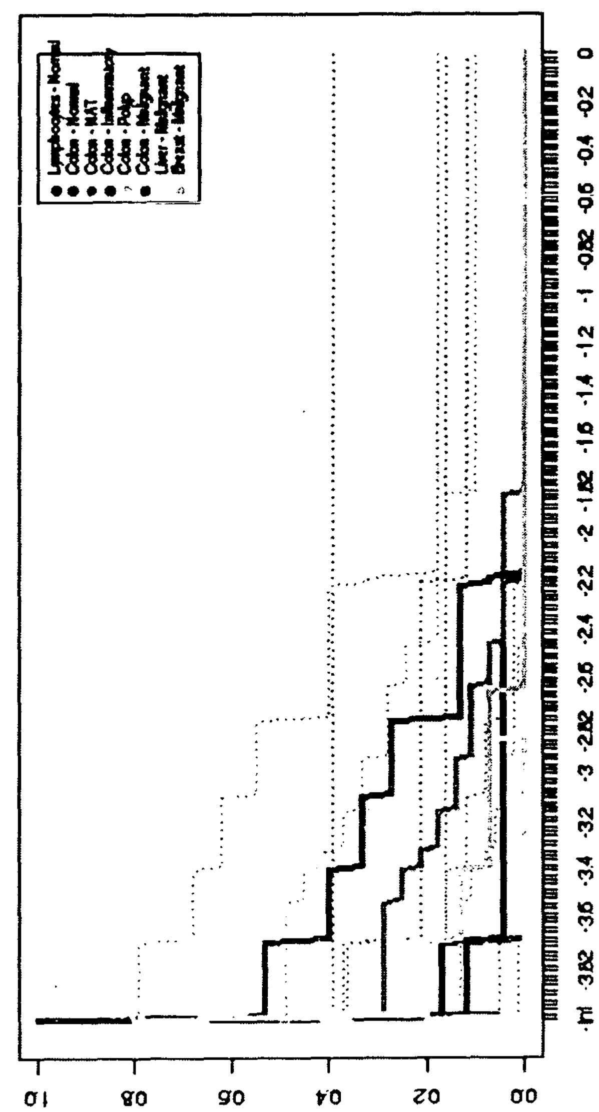

Figure 79

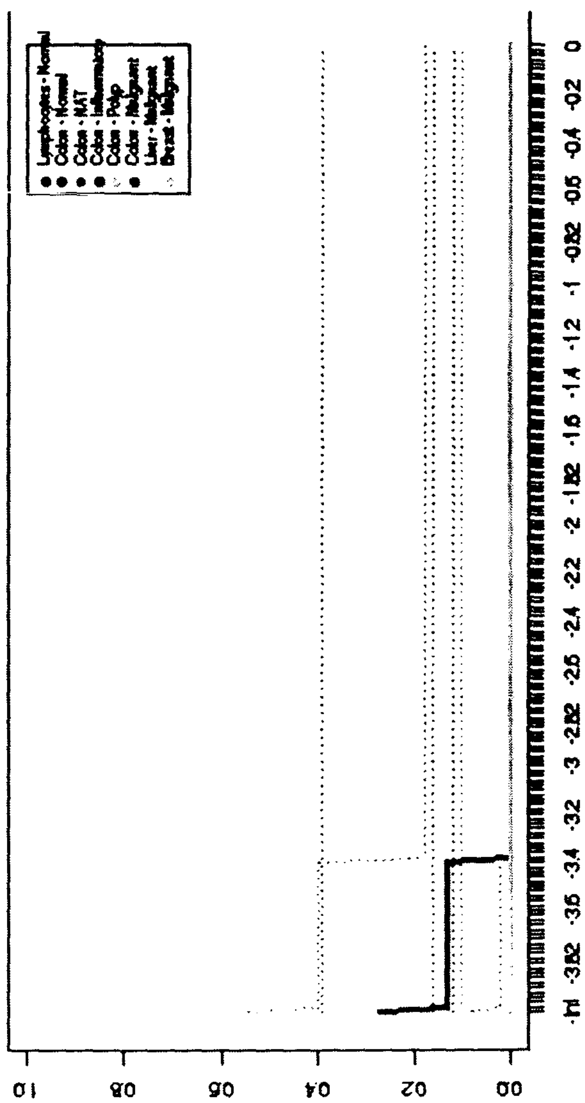
Lymphocytes - Normal
Colon - Normal
Colon - NAT
Colon - Inflammatory
Colon - Polyp
Colon - Malignant
Liver - Malignant
Breast - Malignant

Figure 80

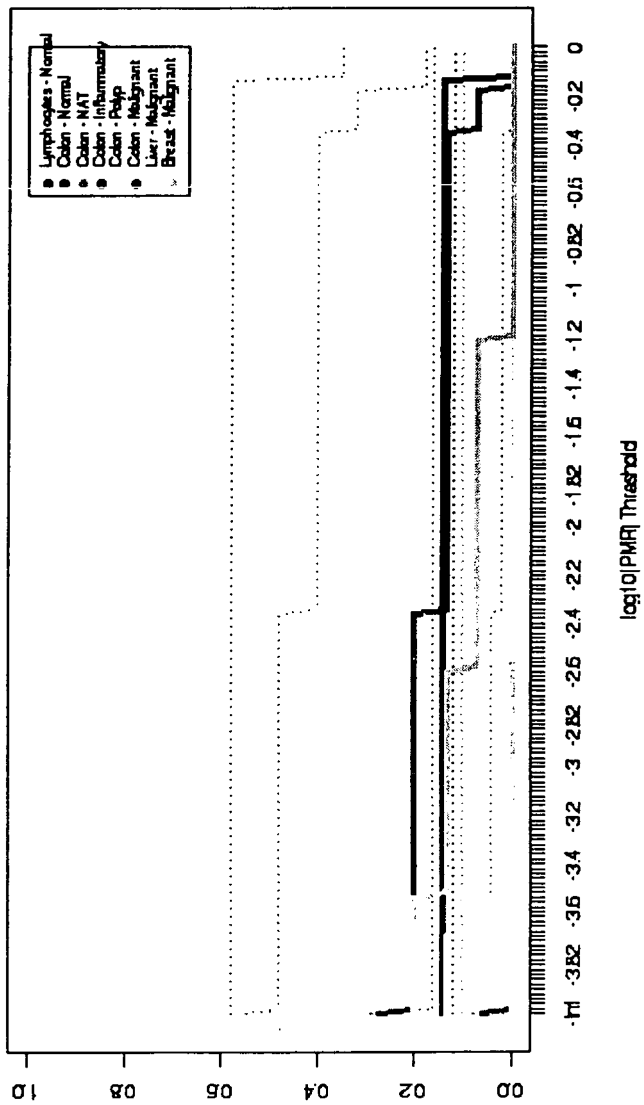
Lymphocytes - Normal
Colon - Normal
Colon - NAT
Colon - Inflammatory
Colon - Polyp
Colon - Malignant
Liver - Malignant
Breast - Malignant
log10(PMR) Threshold

Figure 81

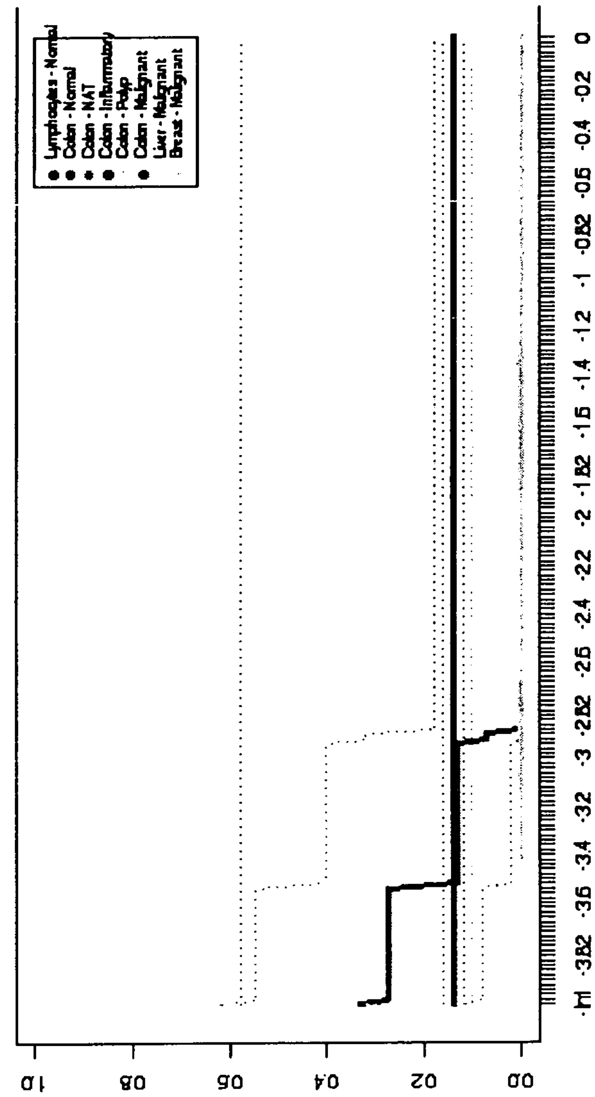
Lymphocytes - Normal
Colon - Normal
Colon - NAT
Colon - Inflammatory
Colon - Polyp
Colon - Malignant
Liver - Malignant
Breast - Malignant

Figure 82

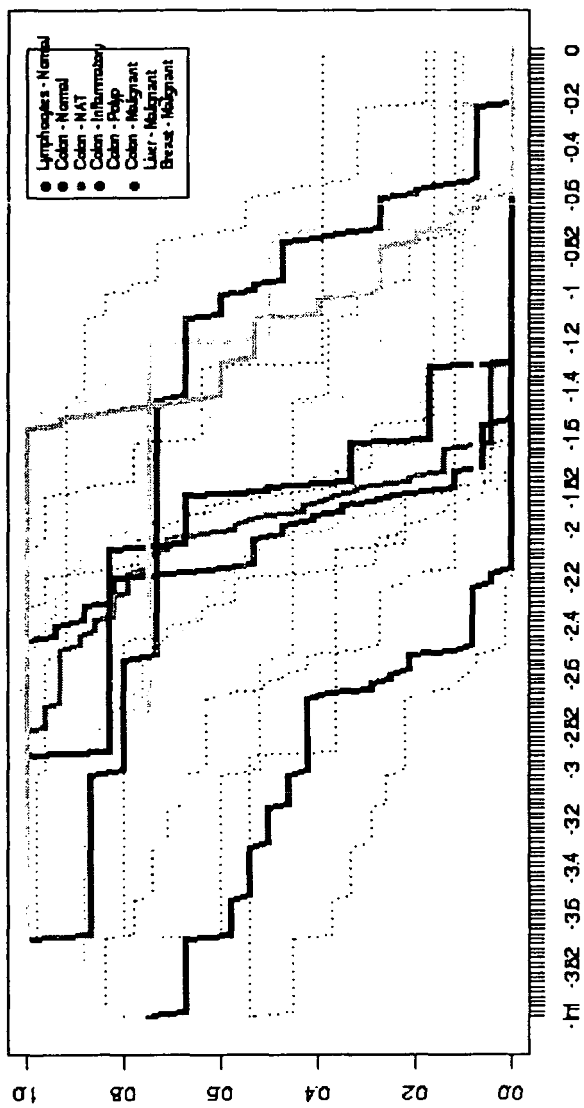
Lymphocytes - Normal
Colon - Normal
Colon - NAT
Colon - Inflammatory
Colon - Polyp
Colon - Malignant
Liver - Malignant
Breast - Malignant
1.0
0.8
0.6
0.4
0.2
0.0
0
-0.2
-0.4
-0.6
-1
-1.2
-1.4
-1.6
-2
-2.2
-2.4
-2.6
-3
-3.2
-3.4
-3.6

Figure 83

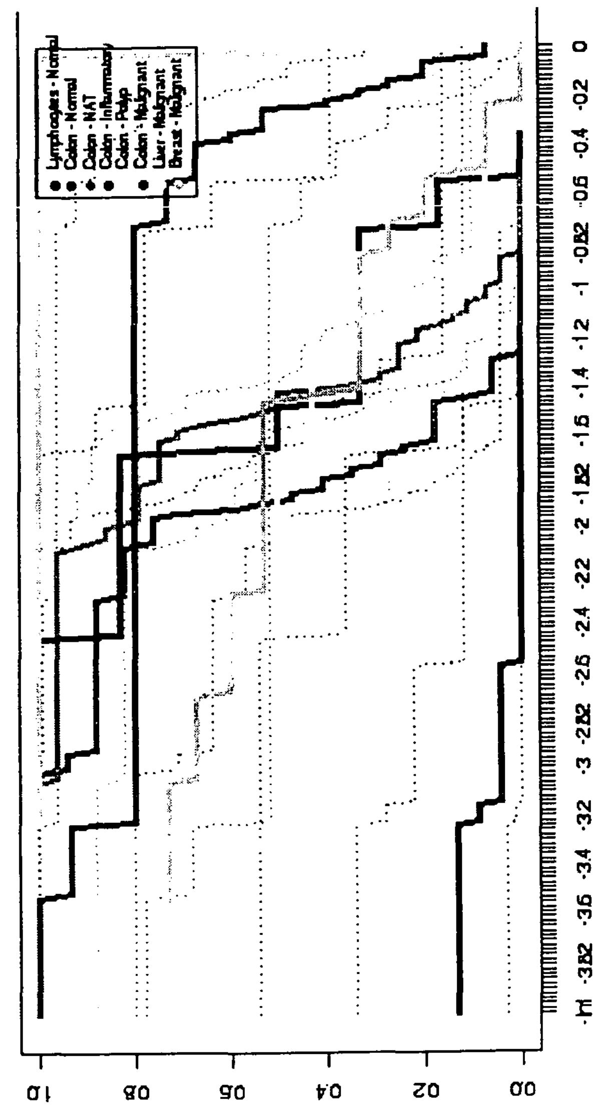
Lymphocytes - Normal
Colon - Normal
Colon - NAT
Colon - Inflammatory
Colon - Polyp
Colon - Malignant
Liver - Malignant
Breast - Malignant

Figure 84

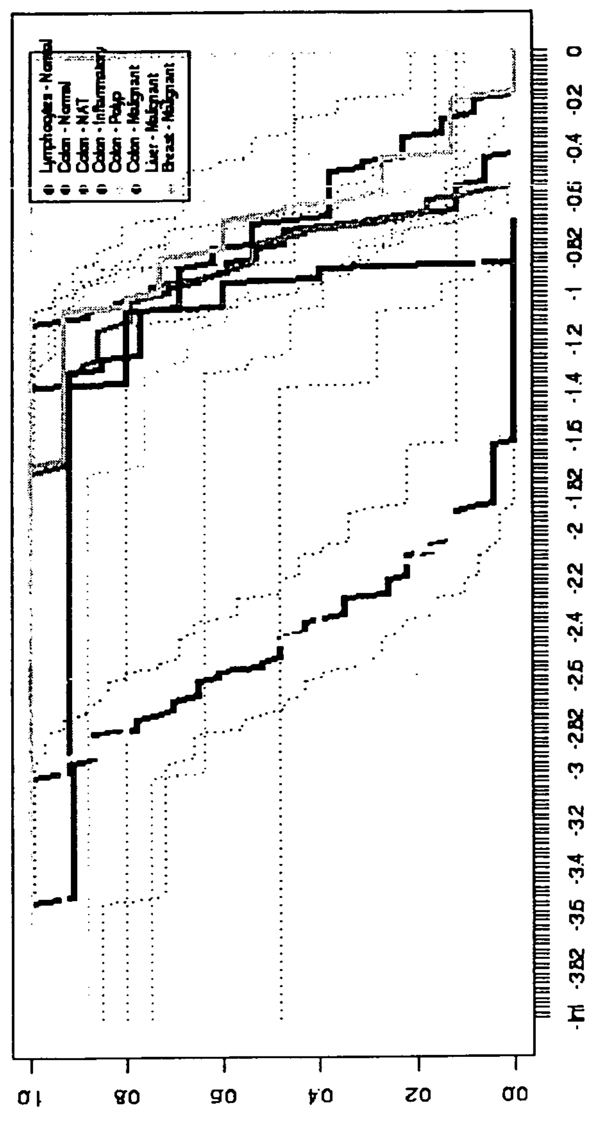
Lymphocytes - Normal
Colon - Normal
Colon - NAT
Colon - Inflammatory
Colon - Polyp
Colon - Malignant
Liver - Malignant
Breast - Malignant

Figure 85

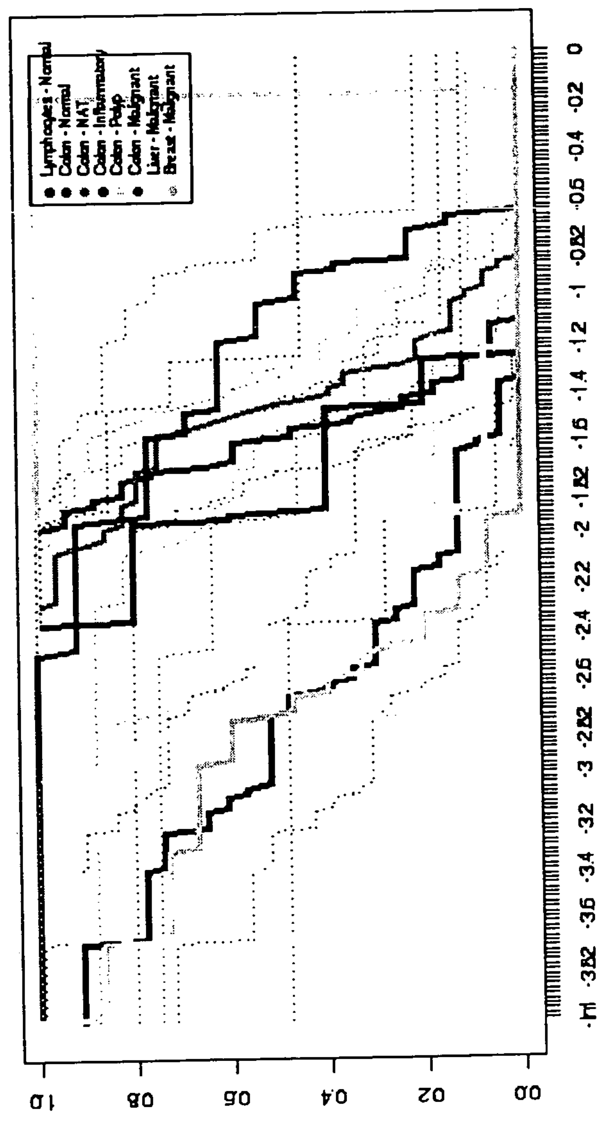
Lymphocytes - Normal
Colon - Normal
Colon - NAT
Colon - Inflammatory
Colon - Polyp
Colon - Malignant
Liver - Malignant
Breast - Malignant

Figure 86

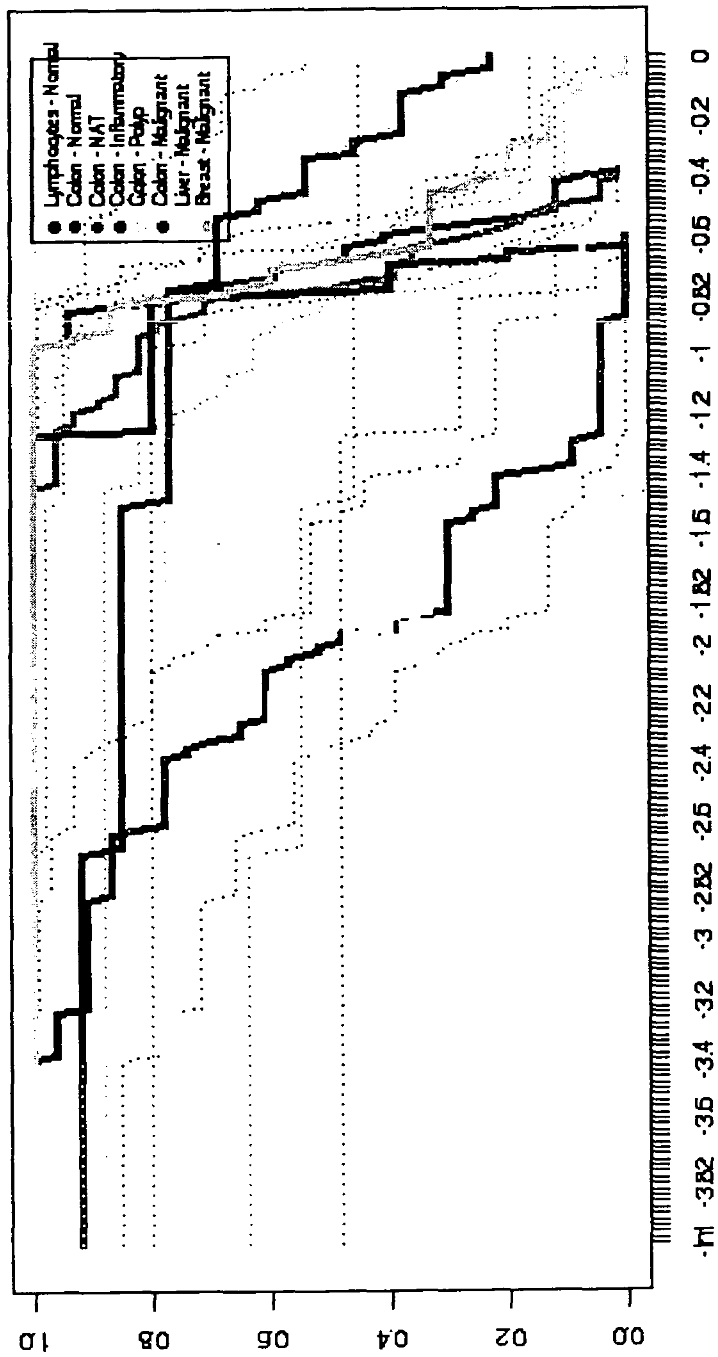
Lymphocytes - Normal
Colon - Normal
Colon - NAT
Colon - Inflammatory
Colon - Polyp
Colon - Malignant
Liver - Malignant
Breast - Malignant

Figure 87

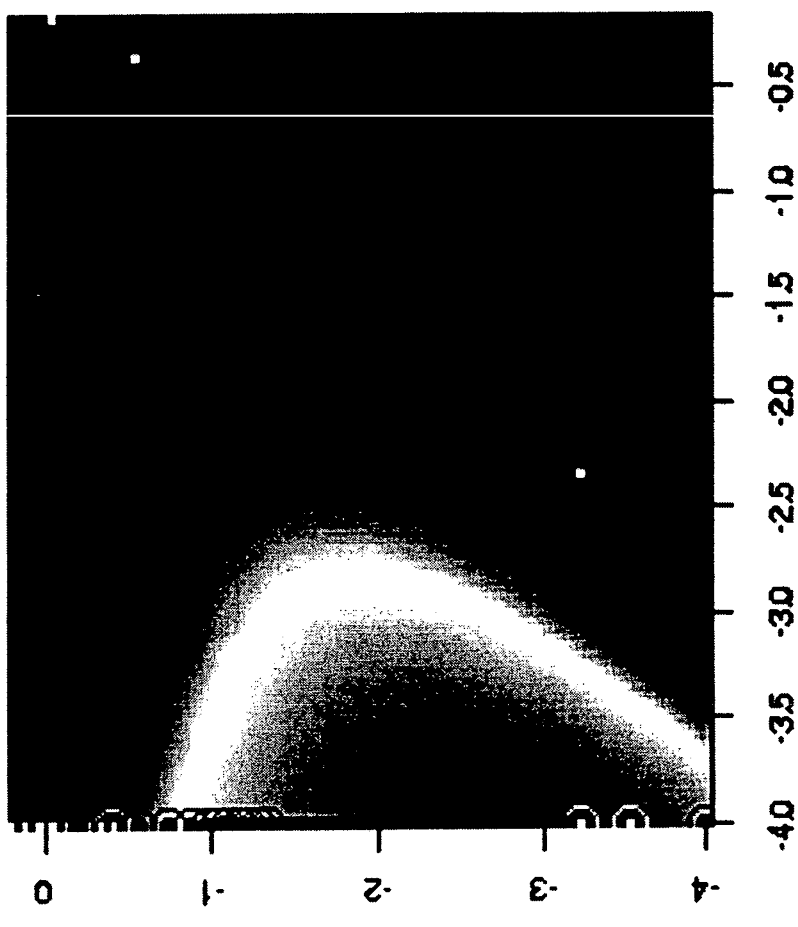

Figure 88

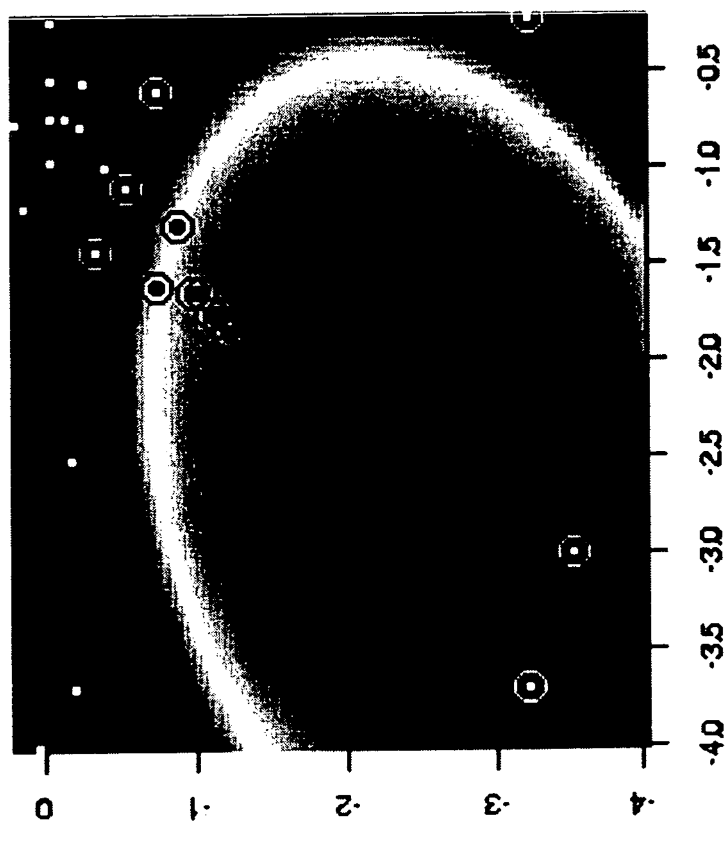

Figure 89

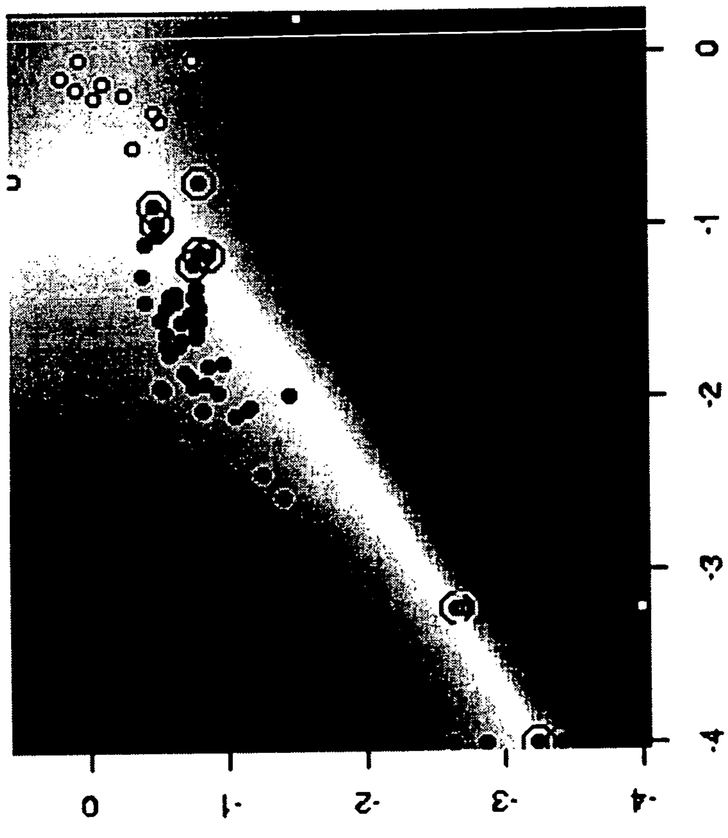

Figure 90

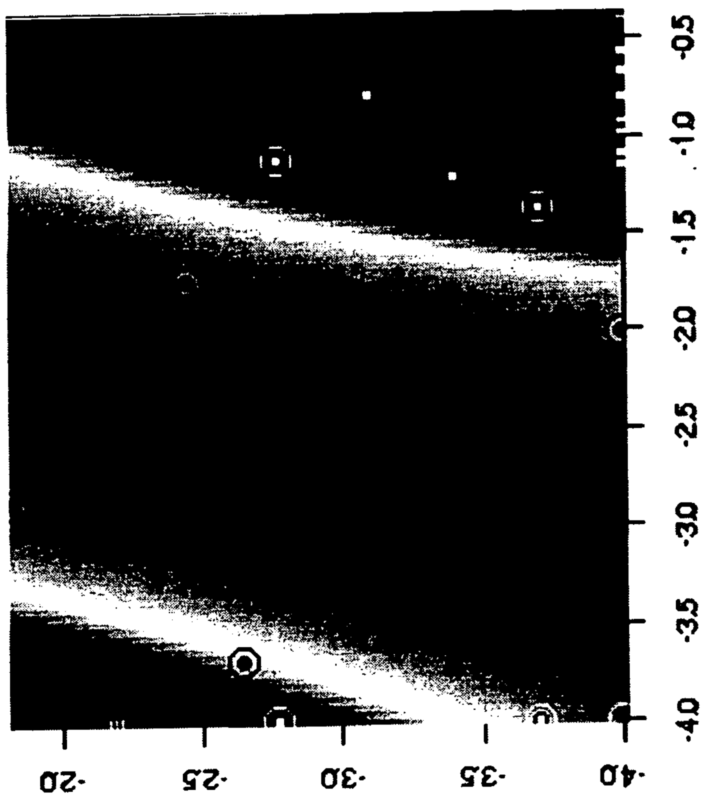

Figure 91

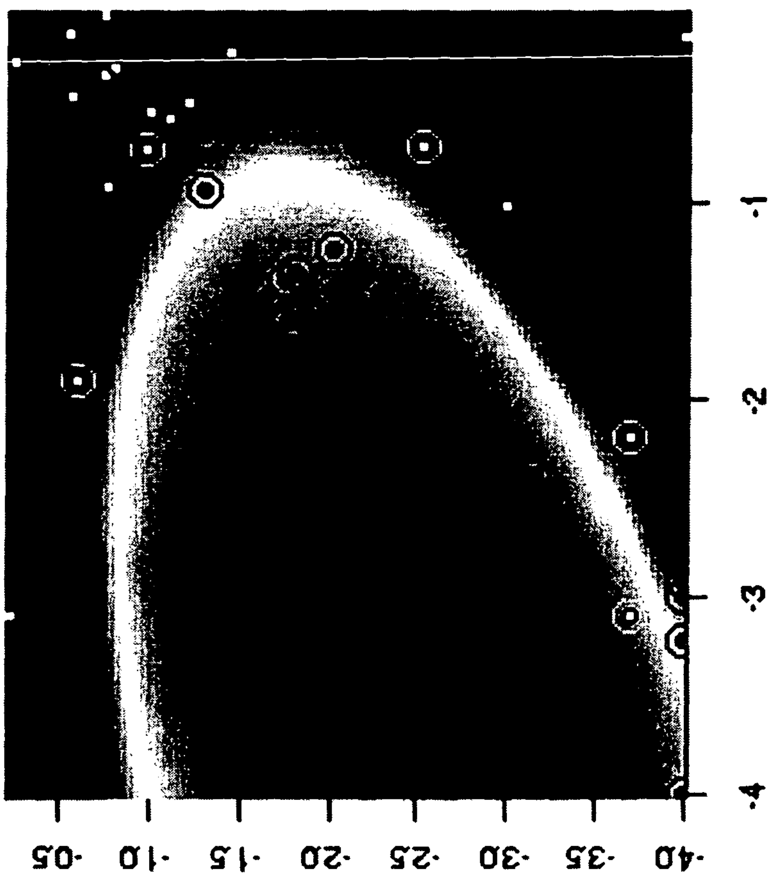

Figure 92

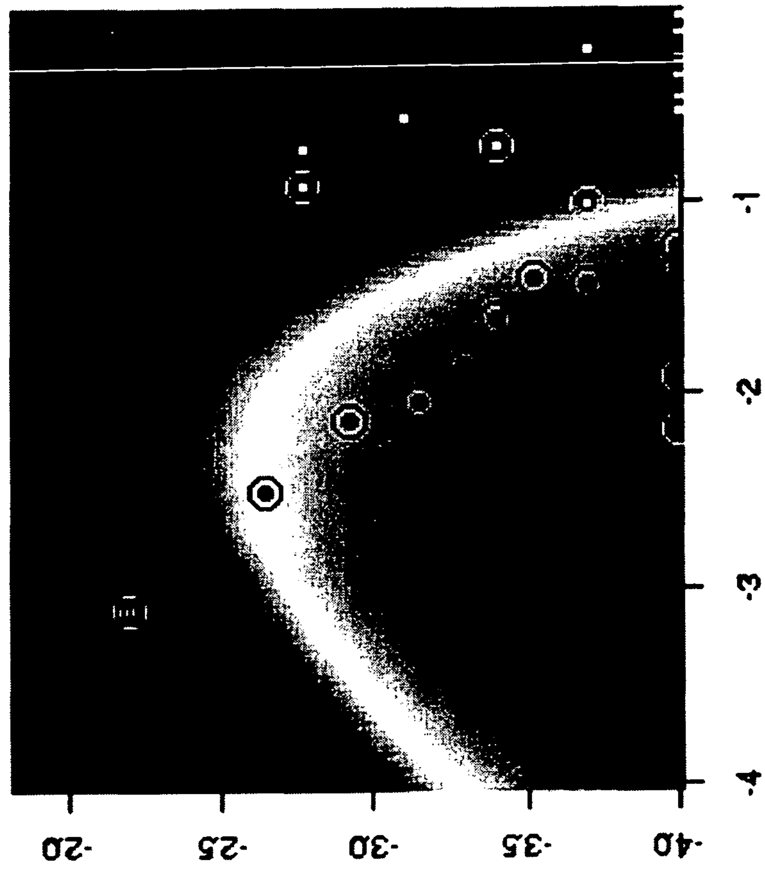

Figure 93

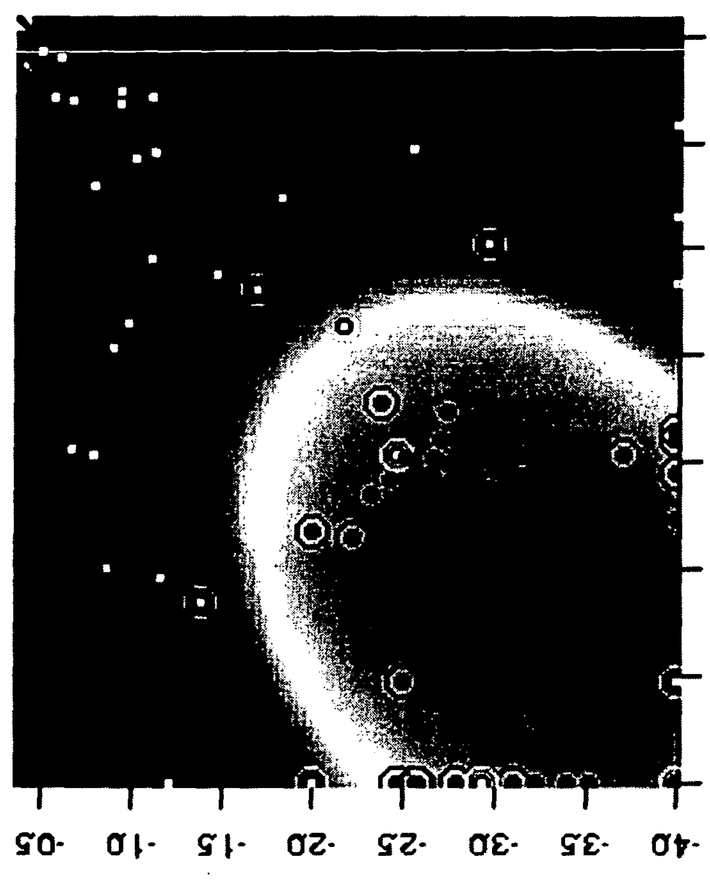

Figure 94

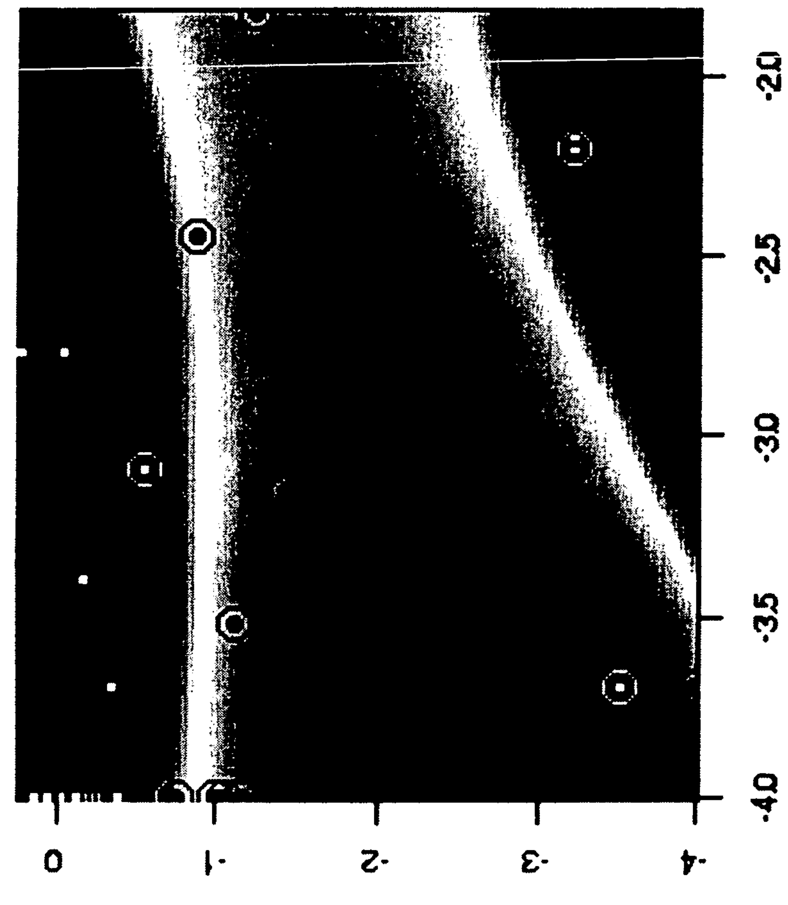

Figure 95

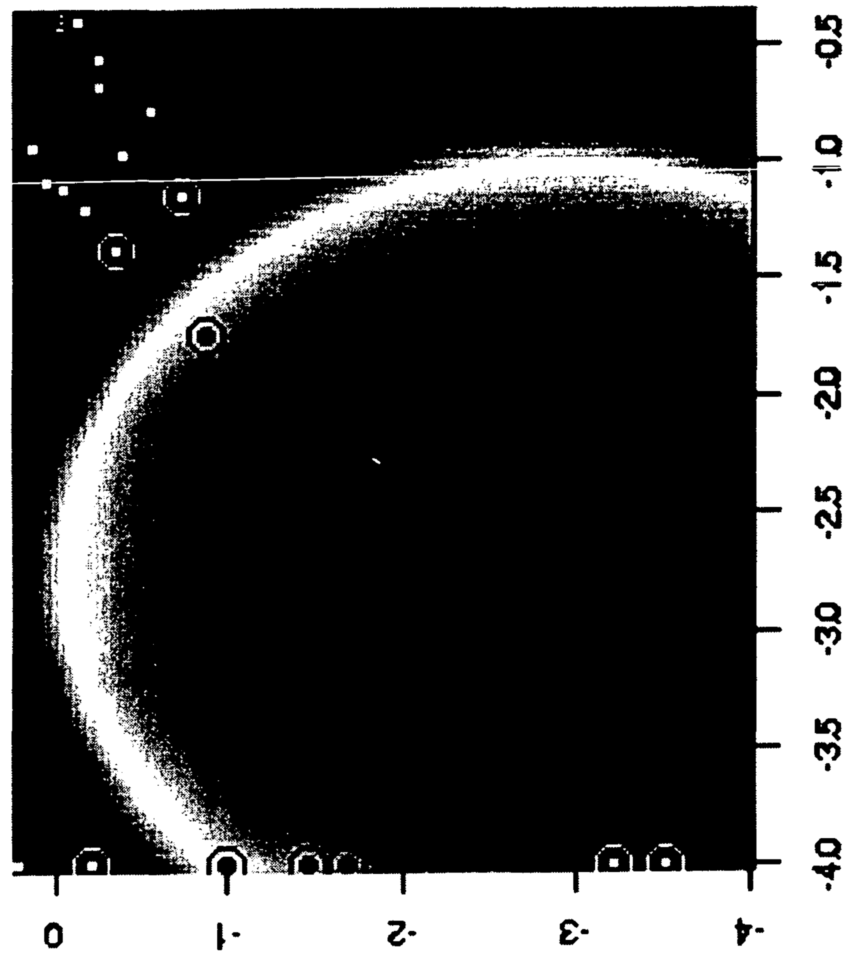

FIGURE 96

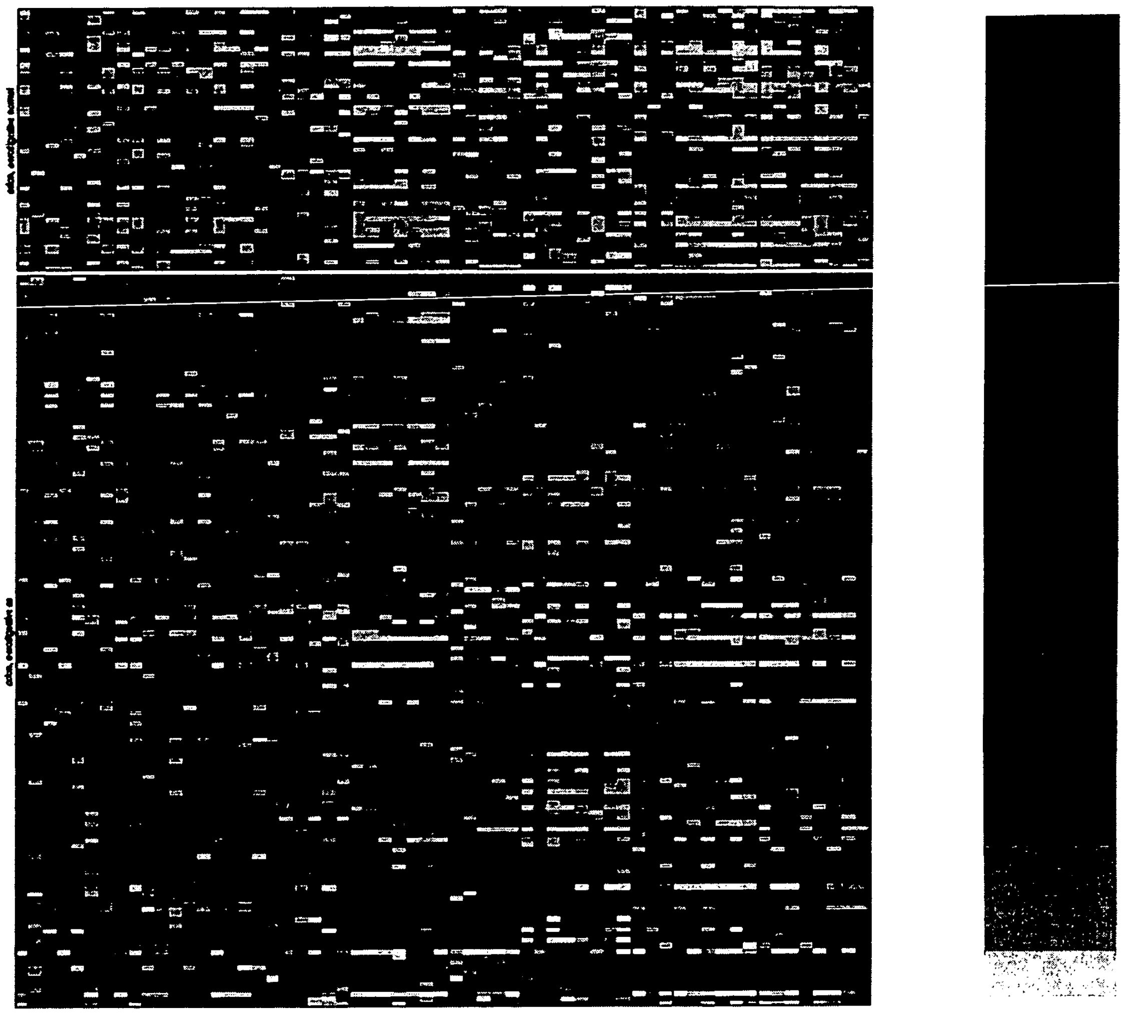

SEQ ID NO: 27
SEQ ID NO: 33
SEQ ID NO: 39
SEQ ID NO: 3
SEQ ID NO: 35
SEQ ID NO: 41
SEQ ID NO: 20

Europäisches Patentamt
European Patent Office
Office européen des brevets

# EUROPEAN SEARCH REPORT

Application Number
EP 10 17 4640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/18632 A2 (EPIGENOMICS AG [DE]; OLEK ALEXANDER [DE]; PIEPENBROCK CHRISTIAN [DE];) 7 March 2002 (2002-03-07) | 11-14 | INV. C12Q1/68 C07H21/00 |
| A | * the whole document * | 1-10, 15-17 | |
| X | -& DATABASE Geneseq [Online] 12 July 2002 (2002-07-12), "Oligonucleotide for detecting cytosine methylation SEQ ID NO 30421.", XP002638045, retrieved from EBI accession no. GSN:ABQ43830 Database accession no. ABQ43830 * the whole document * | 11-14 | |
| X | -& DATABASE Geneseq [Online] 12 July 2002 (2002-07-12), "Oligonucleotide for detecting cytosine methylation SEQ ID NO 32249.", XP002638046, retrieved from EBI accession no. GSN:ABQ45658 Database accession no. ABQ45658 * the whole document * | 11-14 | TECHNICAL FIELDS SEARCHED (IPC) C12Q |
| X | -& DATABASE Geneseq [Online] 12 July 2002 (2002-07-12), "Oligonucleotide for detecting cytosine methylation SEQ ID NO 30417.", XP002638047, retrieved from EBI accession no. GSN:ABQ43826 Database accession no. ABQ43826 * the whole document * ----- | 11-14 | |

-/--

The present search report has been drawn up for all claims

| Place of search | Date of completion of the search | Examiner |
|---|---|---|
| Munich | 23 May 2011 | Bradbrook, Derek |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 17 4640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE Geneseq [Online]<br>4 April 2002 (2002-04-04), "Phosphorothioate substituted RNA/DNA hybrid oligonucleotide SEQ ID NO: 7.", XP002638044, retrieved from EBI accession no. GSN:ABL35101 Database accession no. ABL35101<br>* the whole document *<br>----- | 11-14 | |
| X | TOYOTA M ET AL: "CpG island methylator phenotype in colorectal cancer", PROCEEDINGS OF THE NATIONAL ACADEMY OF SCIENCES OF USA, NATIONAL ACADEMY OF SCIENCE. WASHINGTON, US, vol. 96, July 1999 (1999-07), pages 8681-8686, XP002307651, ISSN: 0027-8424<br>* the whole document *<br>----- | 1-10, 15-17 | |
| X | RASHID ASIF ET AL: "CpG island methylation in colorectal adenomas", AMERICAN JOURNAL OF PATHOLOGY, vol. 159, no. 3, September 2001 (2001-09), pages 1129-1135, XP002328021, ISSN: 0002-9440<br>* the whole document *<br>----- | 1-10, 15-17 | TECHNICAL FIELDS SEARCHED (IPC) |
| X | WO 02/24056 A (MARKOWITZ SANFORD ; UNIV CASE WESTERN RESERVE (US); GRADY WILLIAM (US)) 28 March 2002 (2002-03-28)<br>* the whole document *<br>-----<br>-/-- | 1-10, 15-17 | |

The present search report has been drawn up for all claims

| Place of search | Date of completion of the search | Examiner |
|---|---|---|
| Munich | 23 May 2011 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 17 4640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN RIJNSOEVER M ET AL: "Characterisation of colorectal cancers showing hypermethylation at multiple CpG islands.", GUT, vol. 51, no. 6, December 2002 (2002-12), pages 797-802, XP002328020, ISSN: 0017-5749 * the whole document * ----- | 1-10, 15-17 | |
| X | WO 03/014388 A (TAUBERT HEIKE ; DISTLER JUERGEN (DE); EPIGENOMICS AG (DE); MODEL FABIA) 20 February 2003 (2003-02-20) * page 8, line 32 - page 20, line 31 * * page 23, line 1 - page 24, line 8; claims 1-40; table 1 * ----- | 1-10, 15-17 | |
| T | LOFTON-DAY CATHERINE ET AL: "DNA methylation biomarkers for blood-based colorectal cancer screening", CLINICAL CHEMISTRY, AMERICAN ASSOCIATION FOR CLINICAL CHEMISTRY, WASHINGTON, DC, vol. 54, no. 2, 1 February 2008 (2008-02-01), pages 414-423, XP009107287, ISSN: 0009-9147, DOI: DOI:10.1373/CLINCHEM.2007.095992 * the whole document * ----- | | TECHNICAL FIELDS SEARCHED (IPC) |

The present search report has been drawn up for all claims

| Place of search | Date of completion of the search | Examiner |
|---|---|---|
| Munich | 23 May 2011 | Bradbrook, Derek |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 4640

This annex lists the patent family members relating to the patent documents cited in the above-mentioned European search report. The members are as contained in the European Patent Office EDP file on
The European Patent Office is in no way liable for these particulars which are merely given for the purpose of information.

23-05-2011

| Patent document cited in search report | Publication date | Patent family member(s) | Publication date |
|---|---|---|---|
| WO 0218632 A2 | 07-03-2002 | AT 382711 T | 15-01-2008 |
| | | AU 1218802 A | 13-03-2002 |
| | | AU 2002212188 B2 | 28-02-2008 |
| | | CA 2420840 A1 | 27-02-2003 |
| | | DK 1423528 T3 | 13-05-2008 |
| | | EP 1423528 A2 | 02-06-2004 |
| | | ES 2299520 T3 | 01-06-2008 |
| | | IL 154663 A | 24-12-2009 |
| | | IS 6705 A | 31-01-2003 |
| | | JP 2004516821 T | 10-06-2004 |
| | | MX PA03001834 A | 01-08-2003 |
| | | NZ 524229 A | 27-01-2006 |
| | | US 2004234960 A1 | 25-11-2004 |
| WO 0224056 A | 28-03-2002 | AU 2432802 A | 02-04-2002 |
| | | US 2005153351 A1 | 14-07-2005 |
| | | US 2003068620 A1 | 10-04-2003 |
| WO 03014388 A | 20-02-2003 | CA 2455161 A1 | 20-02-2003 |
| | | DE 10139283 A1 | 13-03-2003 |
| | | EP 1421220 A2 | 26-05-2004 |
| | | JP 2005525783 T | 02-09-2005 |
| | | US 2005064410 A1 | 24-03-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 67906203 A
- US 60313803 A
- US 60249403 A
- EP 04090175 A
- EP 04090072 A
- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B
- US 6265171 B, Herman
- US 6331393 B

### Non-patent literature cited in the description


- **LIPSHUTZ, R. J. et al.** *Nature Genetics,* 1999, vol. 21, 20-24
- **BOWTELL, D. D. L.** *Nature genetics suppl.,* 1999, vol. 21, 25-32
- **SABBIONI et al.** *Molecular Diagnosis,* 2003, vol. 7, 201-207
- **YOUNG J ; BIDEN KG ; SIMMS LA ; HUGGARD P ; KARAMATIC R ; EYRE HJ ; SUTHERLAND GR ; HERATH N ; BARKER M ; ANDERSON GJ.** HPP1: a transmembrane protein-encoding gene commonly methylated in colorectal polyps and cancers. *Proc Natl Acad Sci USA,* 2001, vol. 98, 265-270
- **J.P. EGAN.** Signal Detection Theory and ROC Analysis. Academic Press, 1975
- **GONZALGO et al.** *Cancer Research,* 1997, vol. 57, 594-599
- **EADS et al.** *Cancer Res.,* 1999, vol. 59, 2302-2306
- **GONZALGO ; JONES.** *Nucleic Acids Res.,* 1997, vol. 25, 2529-2531
- **HERMAN et al.** *Proc. Natl. Acad. Sci. USA,* 1996, vol. 93, 9821-9826
- **XIONG ; LAIRD.** *Nucleic Acids Res.,* 1997, vol. 25, 2532-2534
- **TOYOTA et al.** *Cancer Res.,* 1999, vol. 59, 2307-12
- **SAMBROOK et al.** Molecular Cloning, A Laboratory Manual. Cold Spring Harbor Press, 1989
- Current Protocols in Molecular Biology. John Wiley & Sons, 1995
- **OLEK A et al.** A modified and improved method for bisulfite based cytosine methylation analysis. *Nucleic Acids Res.,* 1996, vol. 24, 5064-6
- **REIN, T. et al.** *Nucleic Acids Res.,* 1998, vol. 26, 2255
- **ZESCHNIGK M et al.** *Eur J Hum Genet.,* 1997, vol. 5, 94-98
- **OLEK ; WALTER.** *Nat Genet.,* 1997, vol. 17, 275-6
- **GONZALGO ; JONES.** *Nucleic Acids Res.,* 1997, vol. 25, 2529-31
- **XIONG ; LAIRD.** *Nucleic Acids Res.,* 1997, vol. 25, 2532-4
- **GRIGG ; CLARK.** *Bioessays,* 1994, vol. 16, 431-6
- **ZESCHNIGK M et al.** *Hum Mol Genet.,* 1997, vol. 6, 387-95
- **FEIL R et al.** *Nucleic Acids Res.,* 1994, vol. 22, 695
- **MARTIN V et al.** *Gene,* 1995, vol. 157, 261-4
- **FROMMER et al.** *Proc. Natl. Acad. Sci. USA,* 1992, vol. 89, 1827-1831
- **SADRI ; HORNSBY.** *Nucl. Acids Res.,* 1996, vol. 24, 5058-5059
- **SAMBROOK et al.** Molecular Cloning: A Laboratory Manual. 1989
- **BELYAVSKY et al.** *Nucl Acid Res,* 1989, vol. 17, 2919-2932
- **KRUG ; BERGER.** Methods in Enzymology. Academic Press, 1987, vol. 152, 316-325
- Basic and Clinical Immunology. Appleton & Lange, 1991, 217-262

- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **KROL et al.** *BioTechniques,* 1988, vol. 6, 958-976
- **ZON.** *Pharm. Res.,* 1988, vol. 5, 539-549
- *Nature Genetics,* January 1999, vol. 21
- **YU et al.** *BioTechniques,* 1997, vol. 23, 714-720
- **KARAS ; HILLENKAMP.** *Anal Chem.,* 1988, vol. 60, 2299-301
- **GUT ; BECK.** *Current Innovations and Future Trends,* 1995, vol. 1, 147-57
- **GUT ; BECK.** *Nucleic Acids Res.,* 1995, vol. 23, 1367-73
- **HEID et al.** *Genome Res.,* 1996, vol. 6, 986-994
- **SANGER F. et al.** *Proc Natl Acad Sci USA,* 1997, vol. 74, 5463-5467
- **IRIZARRY RA ; HOBBS B ; COLLIN F ; BEAZER-BARCLAY YD ; ANTONELLIS KJ ; SCHERF U ; SPEED TP.** Exploration, normalization, and summaries of high density oligonucleotide array probe level data. *Biostatistics,* April 2003, vol. 4 (2), 249-64
- **GAUTIER L ; COPE L ; BOLSTAD BM ; IRIZARRY RA.** affy--analysis of Affymetrix GeneChip data at the probe level. *Bioinformatics,* 12 February 2004, vol. 20 (3), 307-150